# EUROPEAN PATENT SPECIFICATION

(11) **EP 1 623 008 B1**
(45) Date of publication and mention of the grant of the patent: **30.07.2014**
(21) Application number: 04758405.7
(22) Date of filing: 26.03.2004
(51) Int. Cl.: A01H 9/00

(54) **PUFA POLYKETIDE SYNTHASE SYSTEMS AND USES THEREOF**
PUFA-POLYKETIDSYNTHASE-SYSTEME UND VERWENDUNGEN DAVON
SYSTEMES DE POLYCETIDES SYNTHASE D'ACIDE GRAS POLYINSATURE ET LEURS UTILISATIONS

(30) Priority: 26.03.2003 US 457979 P
(43) Date of publication of application: 08.02.2006
(73) Proprietor: DSM IP Assets B.V., 6411 TE Heerlen (NL)
(72) Inventor: METZ, James G., Longmont, CO 80501 (US); WEAVER, Craig A., Boulder, CO 80301 (US); BARCLAY, William R., Boulder, CO 80303 (US); FLATT, James H., Longmont, CO 80503 (US)
(74) Representative: Mallalieu, Catherine Louise
(86) International application number: PCT/US2004/009323
(87) International publication number: WO 2004/087879

(56) References cited:
- WO-A-00/42195
- WO-A-02/083870
- US-A- 5 908 622
- US-A- 6 140 486
- US-A1- 2002 138 874
- US-A1- 2002 156 254
- US-A1- 2002 194 641
- FAN K W ET AL: "Eicosapentaenoic and docosahexaenoic acids production by and okara-utilizing potential of thraustochytrids" JOURNAL OF INDUSTRIAL MICROBIOLOGY AND BIOTECHNOLOGY, BASINGSTOKE, GB, vol. 27, no. 4, 1 October 2001 (2001-10-01), pages 199-202, XP002393382 ISSN: 1367-5435
- METZ J G ET AL: "Production of polyunsaturated fatty acids by polyketide synthases in both prokaryotes and eukaryotes" SCIENCE, AMERICAN ASSOCIATION FOR THE ADVANCEMENT OF SCIENCE, US, WASHINGTON, DC, vol. 293, 13 July 2001 (2001-07-13), pages 290-293, XP002956819 ISSN: 0036-8075
- NAPIER J A: "Plumbing the depths of PUFA biosynthesis: a novel polyketide synthase-like pathway from marine organisms" TRENDS IN PLANT SCIENCE, ELSEVIER SCIENCE, OXFORD, GB, vol. 7, no. 2, 1 February 2002 (2002-02-01), pages 51-54, XP002956820 ISSN: 1360-1385
- QIU X ET AL: "Identification of a Delta 4 fatty acid desaturase from Thraustochytrium sp. involved in the biosynthesis of docosahexanoic acid by heterologous expression in Saccharomyces cerevisiae and Brassica juncea." THE JOURNAL OF BIOLOGICAL CHEMISTRY 24 AUG 2001, vol. 276, no. 34, 24 August 2001 (2001-08-24), pages 31561-31566, XP002522023 ISSN: 0021-9258
- METZ ET AL.: 'Production of polyunsaturated fatty acids by polyketide synthase in both prokaryotes and eukaryotes' SCIENCE vol. 293, 13 July 2001, pages 290 - 293, XP002956819
- QUI ET AL.: 'Identification of a delta4 fatty acid desaturase from Thraustochytrium sp. involved in the biosynthesis' J. BIOL. CHEM. vol. 276, no. 34, 24 August 2001, pages 31561 - 31566, XP002522023
- YOKOCHI ET AL.: 'Optimization of docosahexaenoic acid production' APPL. MICROBIOL. BIOTECHNOL. vol. 49, 1998, pages 72 - 76, XP002928890
- WATANABE ET AL.: 'Fatty acid synthesis of an eicosapentaenoic acid-producing bacterium: de novo synthesis, chain elongation, and desaturation system' J. BIOCHEM. vol. 122, 1997, pages 467 - 473, XP008107154
- NAKAHARA ET AL.: 'Production of docosahexaenoic and docosapentaenoic acids by Schizochytrium sp. isolated from Yap islands' J. AM. OIL CHEM. SOC. vol. 73, no. 11, 1996, pages 1421 - 1426, XP000891671
- WEETE ET AL.: 'Lipids and ultrastructure of Thrauchytrium sp. ATCC26185' AM. OIL CHEM. SOC. vol. 32, no. 8, 1997, pages 839 - 845, XP009077836
- SINGH ET AL.: 'Microbial production of docosahexaenoic acid (DHA, C22:6)' ADV. APPL. MICROBIOL. vol. 45, 1997, pages 271 - 312, XP008046097

## Description

### Field of the Invention

This invention relates to polyunsaturated fatty acid (PUFA) polyketide synthase (PKS) systems from microorganisms, including eukaryotic organisms, such as Thraustochytrid microorganisms. More particularly, this invention relates to nucleic acids encoding non-bacterial PUFA PKS systems, to non-bacterial PUFA PKS systems, to genetically modified organisms comprising non-bacterial PUFA PKS systems, and to methods of making and using the non-bacterial PUFA PKS systems disclosed herein. This invention also relates to genetically modified microorganisms and methods to efficiently produce lipids (triacylglyerols (TAG), as well as membrane-associated phospholipids (PL)) enriched in various polyunsaturated fatty acids (PUFAs) and particularly, eicosapentaenoic acid (C20:5, ω-3; EPA) by manipulation of a PUFA polyketide synthase (PKS) system.

### Background of the Invention

Polyketide synthase (PKS) systems are generally known in the art as enzyme complexes derived from fatty acid synthase (FAS) systems, but which are often highly modified to produce specialized products that typically show little resemblance to fatty acids. It has now been shown, however, that polyketide synthase systems exist in marine bacteria and certain microalgae that are capable of synthesizing PUFAs from malonyl-CoA. The PKS pathways for PUFA synthesis in *Shewanella* and another marine bacteria, *Vibrio marinus,* are described in detail in U.S. Patent No. 6,140,486. The PKS pathways for PUFA synthesis in the eukaryotic Thraustochytrid, *Schizochytrium* is described in detail in U.S. Patent 6,566,583. Finally, the PKS pathways for PUFA synthesis in eukaryotes such as members of Thraustochytriales, including the complete structural description of the PUFA PKS pathway in *Schizochytrium* and the identification of the PUFA PKS pathway in *Thraustochytrium,* including details regarding uses of these pathways, are described in detail in U.S. Patent Application Publication No. 20020194641, published December 19, 2002 (corresponding to U.S. Patent Application Serial No. 10/124,800, filed April 16, 2002).

Researchers have attempted to exploit polyketide synthase (PKS) systems that have been described in the literature as falling into one of three basic types, typically referred to as: Type II, Type I and modular. The Type II system is characterized by separable proteins, each of which carries out a distinct enzymatic reaction. The enzymes work in concert to produce the end product and each individual enzyme of the system typically participates several times in the production of the end product. This type of system operates in a manner analogous to the fatty acid synthase (FAS) systems found in plants and bacteria. Type I PKS systems are similar to the Type II system in that the enzymes are used in an iterative fashion to produce the end product. The Type I differs from Type II in that enzymatic activities, instead ofbeing associated with separable proteins, occur as domains of larger proteins. This system is analogous to the Type I FAS systems found in animals and fungi.

In contrast to the Type I and II systems, in modular PKS systems, each enzyme domain is used only once in the production of the end product. The domains are found in very large proteins and the product of each reaction is passed on to another domain in the PKS protein. Additionally, in all of the PKS systems described above, if a carbon-carbon double bond is incorporated into the end product, it is always in the *trans* configuration.

In the Type I and Type II PKS systems described above, the same set of reactions is carried out in each cycle until the end product is obtained. There is no allowance for the introduction of unique reactions during the biosynthetic procedure. The modular PKS systems require huge proteins that do not utilize the economy of iterative reactions (i.e., a distinct domain is required for each reaction). Additionally, as stated above, carbon-carbon double bonds are introduced in the *trans* configuration in all of the previously described PKS systems.

Polyunsaturated fatty acids (PUFAs) are critical components of membrane lipids in most eukaryotes (Lauritzen et al., Prog. Lipid Res. 40 1 (2001); McConn et al., Plant J. 15, 521 (1998)) and are precursors of certain hormones and signaling molecules (Heller et al., Drugs 55, 487 (1998); Creelman et al., Annu. Rev. Plant Physiol. Plant Mol. Biol. 48, 355 (1997)). Known pathways of PUFA synthesis involve the processing of saturated 16:0 or 18:0 fatty acids (the abbreviation X:Y indicates an acyl group containing X carbon atoms and Y double bonds (usually *cis* in PUFAs); double-bond positions of PUFAs are indicated relative to the methyl carbon of the fatty acid chain (ω3 or ω6) with systematic methylene interruption of the double bonds) derived from fatty acid synthase (FAS) by elongation and aerobic desaturation reactions (Sprecher, Curr. Opin. Clin. Nutr. Metab. Care 2, 135 (1999); Parker-Barnes et al., Proc. Natl. Acad. Sci. USA 97, 8284 (2000); Shanklin et al., Annu. Rev. Plant Physiol. Plant Nol. Biol. 49, 611 (1998)). Starting from acetyl-CoA, the synthesis of docosahexaenoic acid (DHA) requires approximately 30 distinct enzyme activities and nearly 70 reactions including the four repetitive steps of the fatty acid synthesis cycle. Polyketide synthases (PKSs) carry out some of the same reactions as FAS (Hopwood et al., Annu. Rev. Genet. 24, 37 (1990); Bentley et al., Annu. Rev. Microbiol. 53, 411 (1999)) and use the same small protein (or domain), acyl carrier protein (ACP), as a covalent attachment site for the growing carbon chain. However, in these enzyme systems, the complete cycle of reduction, dehydration and reduction seen in FAS is often abbreviated so that a highly derivatized carbon chain is produced, typically containing many keto- and hydroxy-groups as well as carbon-carbon double bonds in the *trans* configuration. The linear products of PKSs are often cyclized to form complex biochemicals that include antibiotics and many other secondary products (Hopwood et al., (1990) *supra;* Bentley et al., (1999), *supra*; Keating et al., Curr. Opin. Chem. Biol. 3, 598 (1999)).

Very long chain PUFAs such as docosahexaenoic acid (DHA; 22:6ω3) and eicosapentaenoic acid (EPA; 20:5ω3) have been reported from several species of marine bacteria, including *Shewanella* sp (Nichols et al., Curr. Op. Biotechnol. 10, 240 (1999); Yazawa, Lipids 31, S (1996); DeLong et al., Appl. Environ. Microbiol. 51, 730 (1986)). Analysis of a genomic fragment (cloned as plasmid pEPA) from *Shewanella* sp. strain SCRC2738 led to the identification of five open reading frames (Orfs), totaling 20 Kb, that are necessary and sufficient for EPA production in *E. coli* (Yazawa, (1996), *supra*). Several of the predicted protein domains were homologues of FAS enzymes, while other regions showed no homology to proteins of known function. At least 11 regions within the five Orfs were identifiable as putative enzyme domains (See Metz et al., Science 293:290-293 (2001)). When compared with sequences in the gene databases, seven of these were more strongly related to PKS proteins than to FAS proteins. Included in this group were domains putatively encoding malonyl-CoA:ACP acyltransferase (MAT), β-ketoacyl-ACP synthase (KS), β-ketoacyl-ACP reductase (KR), acyltransferase (AT), phosphopantetheine transferase, chain length (or chain initiation) factor (CLF) and a highly unusual cluster of six ACP domains (i.e., the presence of more than two clustered ACP domains had not previously been reported in PKS or FAS sequences). It is likely that the PKS pathway for PUFA synthesis that has been identified in *Shewanella* is widespread in marine bacteria. Genes with high homology to the *Shewanella* gene cluster have been identified in *Photobacterium profundum* (Allen et al., Appli. Environ. Microbiol. 65:1710 (1999)) and in *Moritella marina* (*Vibrio marinus*) (see U.S. Patent No. 6,140,486, *ibid.,* and Tanaka et al., Biotechnol. Lett. 21:939 (1999)).

Polyunsaturated fatty acids (PUFAs) are considered to be useful for nutritional, pharmaceutical, industrial, and other purposes. An expansive supply of PUFAs from natural sources and from chemical synthesis are not sufficient for commercial needs. A major current source for PUFAs is from marine fish; however, fish stocks are declining, and this may not be a sustainable resource. Additionally, contamination, both heavy metal and toxic organic molecules, is a serious issue with oil derived from marine fish. Vegetable oils derived from oil seed crops are relatively inexpensive and do not have the contamination issues associated with fish oils. However, the PUFAs found in commercially developed plant oils are typically limited to linoleic acid (eighteen carbons with 2 double bonds, in the delta 9 and 12 positions - 18:2 delta 9,12) and linolenic acid (18:3 delta 9,12,15). In the conventional pathway for PUFA synthesis, medium chain-length saturated fatty acids (products of a fatty acid synthase (FAS) system) are modified by a series of elongation and desaturation reactions. Because a number of separate desaturase and elongase enzymes are required for fatty acid synthesis from linoleic and linolenic acids to produce the more saturated and longer chain PUFAs, engineering plant host cells for the expression of PUFAs such as EPA and docosahexaenoic acid (DHA) may require expression of several separate enzymes to achieve synthesis. Additionally, for production of useable quantities of such PUFAs, additional engineering efforts may be required, for example, engineering the down regulation of enzymes that compete for substrate, engineering of higher enzyme activities such as by mutagenesis or targeting of enzymes to plastid organelles. Therefore it is of interest to obtain genetic material involved in PUFA biosynthesis from species that naturally produce these fatty acids and to express the isolated material alone or in combination in a heterologous system which can be manipulated to allow production of commercial quantities of PUFAs.

The discovery of a PUFA PKS system in marine bacteria such as *Shewanella* and *Vibrio marinus* (see U.S. Patent No. 6,140,486, *ibid.)* provides a resource for new methods of commercial PUFA production. However, these marine bacteria have limitations which may ultimately restrict their usefulness on a commercial level. First, although U.S. Patent No. 6,140,486 discloses that these marine bacteria PUFA PKS systems can be used to genetically modify plants, the marine bacteria naturally live and grow in cold marine environments and the enzyme systems of these bacteria do not function well above 22°C. In contrast, many crop plants, which are attractive targets for genetic manipulation using the PUFA PKS system, have normal growth conditions at temperatures above 22°C and ranging to higher than 40°C. Therefore, the PUFA PKS systems from these marine bacteria are not predicted to be readily adaptable to plant expression under normal growth conditions. Additionally, the known marine bacteria PUFA PKS systems do not directly produce triacylglyerols (TAG), whereas direct production of TAG would be desirable because TAG are a lipid storage product, and as a result, can be accumulated at very high levels in cells, as opposed to a "structural" lipid product (e.g. phospholipids), which can generally only accumulate at low levels.

With regard to the production of eicosapentaenoic acid (EPA) in particular, researchers have tried to produce EPA with microbes by growing them in both photosynthetic and heterotrophic cultures. They have also used both classical and directed genetic approaches in attempts to increase the productively of the organisms under culture conditions. Other researchers have attempted to produce EPA in oil-seed crop plants by introduction of genes encoding various desaturase and elongase enzymes.

Researchers have attempted to use cultures of red microalgae (Monodus), diatoms (e.g. *Phaeodactylum*)*,* other microalgae and fungi (e.g. *Mortierella* cultivated at low temperatures). However, in all cases, productivity was low compared to existing commercial microbial production systems for other long chain PUFAs such as DHA. In many cases, the EPA occurred primarily in the phospholipids (PL) rather than the triacylglycerols (TAG). Since productivity of microalgae under heterotrophic growth conditions can be much higher than under phototrophic conditions, researchers have attempted, and achieved, trophic conversion by introduction of genes encoding specific sugar transporters. However, even with the newly acquired heterotrophic capability, productivity in terms of oil remained relatively low.

Efforts to produce EPA in oil-seed crop plants by modification of the endogenous fatty acid biosynthesis pathway have only yielded plants with very low levels of the PUFA in their oils. As discussed above, several marine bacteria have been shown to produce PUFAs (EPA as well as DHA). However, these bacteria do not produce TAG and the EPA is found primarily in the PL membranes. The levels of EPA produced as well as the growth characteristics of these particular marine bacteria (discussed above) limit their utility for commercial production of EPA.

Therefore, there is a need in the art for other PUFA PKS systems having greater flexibility for commercial use, and for a biological system that efficiently produces quantities of lipids (PL and TAG) enriched in desired PUFAs, such as EPA, in a commercially useful production process.

### Summary of the Invention

One embodiment of the present invention relates to an isolated nucleic acid molecule comprising a nucleic acid sequence selected from the group consisting of:
a) a nucleic acid sequence encoding an amino acid sequence selected from the group consisting of: SEQ ID NO:62, SEQ ID NO:64, SEQ ID NO:66, SEQ ID NO:68 and biologically active fragments thereof, wherein the biologically active fragments exhibit a biological activity selected from the group consisting of enoyl-ACP reductase (ER) activity and β-hydroxyacyl-ACP dehydratase (DH) activity;
b) a nucleic acid sequence encoding an amino acid sequence that is at least about 70% identical to an amino acid sequence selected from the group consisting of: SEQ ID NO:62 and SEQ ID NO:66, wherein the amino acid sequence has a biological activity selected from the group consisting of enoyl-ACP reductase (ER) activity and β-hydroxyacyl-ACP dehydratase (DH) activity;
c) a nucleic acid sequence encoding an amino acid sequence that is at least about 80% identical to an amino acid sequence selected from the group consisting of: SEQ ID NO:64 and SEQ ID NO:68, wherein the amino acid sequence has a biological activity selected from the group consisting of enoyl-ACP reductase (ER) activity and β-hydroxyacyl-ACP dehydratase (DH) activity; and
d) a nucleic-acid sequence that is fully complementary to the nucleic acid sequence of (a), (b) or (c).

In one embodiment the nucleic acid sequence encoding an amino acid sequence : is preferably at least about 80% identical, and more preferably at least about 90% identical, to an amino acid sequence selected from the group consisting of: SEQ ID NO:62 and SEQ ID NO:66.

In one embodiment the nucleic acid sequence encoding an amino acid sequence is preferably at least about 90% identical, to an amino acid sequence selected from the group consisting of SEQ

ID No: 64 and SEQ ID NO:6% In one aspect, the nucleic acid sequence encodes an amino acid sequence selected from: SEQ ID NO:62, SEQ ID NO:64, SEQ ID NO:66 and SEQ ID NO:68, and biologically active fragments thereof. In one aspect, the nucleic acid sequence is selected from the group consisting of SEQ ID NO:61, SEQ ID NO:63, SEQ ID NO: 65, and SEQ ID NO:67.

Another embodiment of the present invention relates to a recombinant nucleic acid molecule comprising any of the above-described nucleic acid molecules, operatively linked to at least one transcription control sequence.

Yet another embodiment of the present invention relates to a recombinant cell transfected with any of the above-described recombinant nucleic acid molecules.

Another embodiment of the present invention relates to a genetically modified microorganism, wherein the microorganism expresses a PKS system comprising at least one biologically active domain of a polyunsaturated fatty acid (PUFA) polyketide synthase (PKS) system, wherein the at least one domain of the PUFA PKS system comprises an amino acid sequence selected from the group consisting of:
a) an amino acid sequence selected from the group consisting of: SEQ ID NO:62, SEQ ID NO:64, SEQ ID NO:66, SEQ ID NO:68 and biologically active fragments thereof, wherein the biologically active fragments exhibit a biological activity selected from the group consisting of enoyl-ACP reductase (ER) activity and β-hydroxyacyl-ACP dehydratase (DH) activity;
b) an amino acid sequence that is at least about 70% identical to an amino acid sequence selected from the group consisting of: SEQ ID NO:62 and SEQ ID NO:66, wherein the amino acid sequence has a biological activity selected from the group consisting of enoyl- . ACP reductase (ER) activity and β-hydroxyacyl-ACP dehydratase (DH) activity; and
c) an amino acid sequence that is at least about 80% identical to an amino acid sequence selected from the group consisting of: SEQ ID NO:64 and SEQ ID NO:68, wherein the amino acid sequence has a biological activity selected from the group consisting of enoyl-ACP reductase (ER) activity and β-hydroxyacyl-ACP dehydratase (DH) activity; wherein the microorganism is genetically modified by transfection with a recombinant nucleic acid molecule encoding the at least one domain of a polyunsaturated fatty acid (PUFA) polyketide synthase (PKS) system.

In one embodiment the amino acid sequence is preferably at least about 80% identical, and more preferably at least about 90% identical, to an amino acid sequence selected from the group consisting of: SEQ ID NO:62 and SEQ ID NO:66. In one embodiment the amino acid sequence is preferably at least about 90% identical, to an amino acid sequence selected form the group consisting of: SEQ ID NO:64 and SEQ ID NO:68. The microorganism is genetically modified to affect the activity of the PKS system. The microorganism can include a Thraustochytrid, such as a *Schizochytrium.* In one embodiment, such a microorganism has been further genetically modified to recombinantly express at least one nucleic acid molecule encoding at least one biologically active domain from a PKS system selected from the group consisting of a bacterial PUFA PKS system, a Type I PKS system, a Type II PKS system, a modular PKS system, and a non-bacterial PUFA. PKS system. The non-bacterial PUFA PKS system can include a Thranstochytrid PUFA PKS system and in one aspect, a *Schizochytrium* PUFA PKS system.

In another embodiment, the microorganism endogenously expresses a PKS system comprising the at least one domain of the PUFA PKS system, and wherein the genetic modification is in a nucleic acid sequence encoding at least one domain of the PUFA PKS system. In another aspect, such a microorganism has been further genetically modified to recombinantly express at least one nucleic acid molecule encoding at least one biologically active domain from a PKS system selected from the group consisting of: a bacterial PUFA PKS system, a Type I PKS system, a Type II PKS system, a modular PKS system, and a non-bacterial PUFA PKS system (e.g., a Thraustochytrid PUFA PKS system, such as a *Schizochytrium* PUFA PKS system).

In another aspect, the microorganism endogenously expresses a PUFA PKS system comprising the at least one biologically active domain of a PUFA PKS system, and wherein the genetic modification comprises expression of a recombinant nucleic acid molecule selected from the group consisting of a recombinant nucleic acid molecule encoding at least one biologically active domain from a second PKS system and a recombinant nucleic acid molecule encoding a protein that affects the activity of the endogenous PUFA PKS system. The biologically active domain from a second PKS system can include, but is not limited to: (a) a domain of a polyunsaturated fatty acid (PUFA) polyketide synthase (PKS) system from a Thraustochytrid microorganism; (b) a domain of a PUFA PKS system from a microorganism identified by the following method: (i) selecting a microorganism that produces at least one PUFA; and, (ii) identifying a microorganism from (i) that has an ability to produce increased PUFAs under dissolved oxygen conditions of less than about 5% of saturation in the fermentation medium, as compared to production of PUFAs by the microorganism under dissolved oxygen conditions of greater than about 5% of saturation in the fermentation medium; (c) a domain comprising an amino acid sequence selected from the group consisting of: SEQ ID NO:2, SEQ ID NO:4, SEQ ID NO:6, SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32, and biologically active fragments thereof; and (d) a domain comprising an amino acid sequence that is at least about 60% identical, and more preferably at least about 70% identical, and more preferably at least about 80% identical, and more preferably at least about 90% identical, to the amino acid sequence of (c), wherein the amino acid sequence has a biological activity of at least one domain of a polyunsaturated fatty acid (PUFA) polyketide synthase (PKS) system. In one aspect, recombinant nucleic acid molecule encodes a phosphopantetheine transferase. In one aspect, the second PKS system is selected from the group consisting of: a bacterial PUFA Yet another embodiment of the present invention relates to a genetically modified plant, wherein the plant has been genetically modified to recombinantly express a PKS system comprising at least one biologically active domain of a polyunsaturated fatty acid (PUFA) polyketide synthase (PKS) system, wherein the domain comprises an amino acid sequence selected from the group consisting of:
a) an amino acid sequence selected from the group consisting of: SEQ ID NO:62, SEQ ID NO:64, SEQ ID NO:66, SEQ ID NO:68 and biologically active fragments thereof, wherein the biologically active fragments exhibit a biological activity selected from the group consisting of enoyl-ACP reductase (ER) activity and β-hydroxyacyl-ACP dehydratase (DH) activity;
b) an amino acid sequence that is at least about 70% identical to an amino acid sequence selected from the group consisting of: SEQ ID NO:62 and SEQ ID NO:66, wherein the amino acid sequence has a biological activity selected from the group consisting of enoyl-ACP reductase (ER) activity and β-hydroxyacyl-ACP dehydratase (DH) activity; and
c) an amino acid sequence that is at least about 80% identical to an amino acid sequence selected from the group consisting of: SEQ ID NO:64 and SEQ ID NO:68, wherein the amino acid sequence has a biological activity selected from the group consisting of enoyl-ACP reductase (ER) activity and β-hydroxyacyl-ACP dehydratase (DH) activity.

In one embodiment the amino acid sequence is preferably at least about 80% identical, and more preferably at least about 90% identical to an amino acid sequence selected from the group consisting of SEQ ID NO:62 and SEQ ID NO:66. In one embodiment the amino acid sequence is preferably at least about 90% 5' identical, to an amino acid sequence selected from the group consisting of SEQ ID NO:6 4 and SEQ ID NO:68.

In one embodiment, the plant has been further genetically modified to recombinantly express at least one nucleic acid molecule encoding at least one biologically active domain from a PKS system selected from the group consisting of a bacterial PUFA PKS system, a I Type I PKS System, a Type II PKS system, a modular PKS system, and a non bacterial PUFA PKS system (e.g., a Thraustochytrid PUFA PKS system, such as a *Schizochytrium* PUFA PKS system).

Yet another aspect of the present invention relates to a method to produce a bioactive molecule that is produced by a polyketide-synthase, system, comprising growing or culturing under conditions effective to produce the bioactive molecule in the genetically modified organism of the invention or the genetically modified plant of the invention.

In one embodiment, the genetic modification changes at least one product produced by the endogenous PKS system, as compared to an organism wherein the PUFA PKS system has not been genetically modified.

In another embodiment, the organism endogenously expresses a PKS system comprising the at least one biologically active domain of the PUFA PKS system, and the genetic modification comprises transfection of the organism with a recombinant nucleic acid molecule selected from the group consisting of: a.recombinant nucleic acid molecule encoding at least one biologically active domain from a second PKS system and a recombinant nucleic acid molecule encoding a protein that affects the activity of the PUFA PKS system. In one embodiment, the genetic modification changes at least one product produced by the endogenous PKS system, as compared to an organism that has not been genetically modified to affect PUFA production.

In another embodiment the organism is genetically modified by transfection with a recombinant nucleic acid molecule encoding the at least one domain of the polyunsaturated fatty acid (PUFA) polyketide synthase (PKS) system.

In another embodiment, the organism produces a polyunsatured fatty acid (PUFA) profile that differs from the naturally occurring organism without a genetic modification.

In another embodiment, the organism endogenously expresses a non-bacterial PUFA PKS system, and wherein the genetic modification comprises substitution of a domain from a different PKS system for a nucleic acid sequence encoding at least one domain of the non-bacterial PUFA PKS system,

In yet another embodiment, the organism endogenously expresses a non-bacterial PUFA PKS system that has been modified by transfecting the organism with a recombinant nucleic acid molecule encoding a protein that regulates the chain length of fatty acids produced by the PUFA PKS system.

In another embodiment, the bioactive molecule is selected from: an anti-inflammatory formulation, a chemotherapeutic agent, an active excipient, an osteoporosis drug, an anti-depressant, an anti-convulsant, an anti-*Heliobactor pylori* drug, a drug for treatment of neurodegenerative disease, a drug for treatment of degenerative liver disease, an antibiotic, and/or a cholesterol lowering formulation. In one embodiment, the bioactive molecule am antibiotic. In another embodiment, the bioactive molecule is a polyunsaturated fatty acid (PUPA). In yet another embodiment, the bioactive molecule is a molecule including carbon-carbon double bonds in the *cis* configuration. In one embodiment, the bioactive molecule is a molecule including a double bond at every third carbon. In one aspect, the organism is a microorganism. In another aspect, the organism is a plant

Another aspect of the present invention relates to a method to produce a plant that has a polyunsaturated fatty acid (PUFA) profile that differs from the naturally occurring plant, comprising genetically modifying cells of the plant to express a PKS system comprising at least one recombinant nucleic acid molecule comprising a nucleic acid sequence encoding at least one biologically active domain of a PUFA PKS system, wherein the at least one domain of the PUFA PKS system is as previously defined.

Another embodiment of the present invention relates to a method to modify an endproduct containing at least one fatty acid, comprising adding to the endproduct an oil produced by a recombinant host cell that expresses at least one recombinant nucleic acid molecule comprising a nucleic acid sequence encoding at least one biologically active domain of a PUFA PKS system, wherein the at least one domain of a PUFA PKS system comprises an amino acid sequence as previously defined.

In one embodiment, the endproduct is selected from: a dietary supplement, a food product, a pharmaceutical formulation, a humanized animal milk, and an infant formula. In one embodiment, the pharmaceutical formulation is selected from the group consisting of an anti-inflammatory formulation, a chemotherapeutic agent, active excipient, an osteopogosas drug, an anti-depressant, an anti-convalsant, an anti- *Heliobactor pylori* drug, a drug for treatment of neurodegenerative disease, a drug for treatment of degenerative liver disease, an antibiotic, and a cholesterol lowering formulation. In one embodiment, the endproduct is used to treat a condition selected from the group consisting of: chronic inflammation, acute inflammation, gastrointestinal disorder, cancer, cachexia, cardiac restenosis, neurodegenerative disorder, degenerative disorder of the liver, blood lipid disorder, osteoporosis, osteoarthritis, autoimmune disease, preeclampsia, preterm birth, age related maculopathy, pulmonary disorder, and peroxisomal disorder.

Yet another aspect of the present invention relates to a method to produce a humanized animal milk, comprising genetically modifying milk-producing cells of a milk-producing animal with at least one recombinant nucleic acid molecule comprising a nucleic acid sequence encoding at least one biologically active domain of a PUFA PKS system wherein the at least one domain of the PUFAPKS system comprises an amino acid sequence as previously defined.

Another aspect of the present invention relates to a genetically modified Thraustochytrid microorganism, wherein the, microorganism has an endogenous polyunsaturated fatty acid (PUFA) polyketide synthase (PKS) system, and wherein the endogenous PUFA PKS system has been genetically modified by deleting at least one nucleic acid sequence that encodes at least one domain of the endogenous PUFA PKS system and inserting a nucleic acid sequence as defined in any one of Claims 1 to 3, wherein the expression profile of a polyunsaturated fatty acid (PUFA) by the geneticallymodified Thraustochytrid microorganism is altered as compared to the Thraustochytrid microorganism in the absence of the genetic modification.

In one aspect, the endogenous PUFA PKS system has been modified by mutagenesis of a nucleic acid sequence that encodes at least one domain of the endogenous PUFA PKS system. In one aspect, the modification is produced by targeted mutagenesis. In another aspect, the modification is produced by classical mutagenesis and screening.

In another aspect, the endogenous PUFA PKS system has been modified by deleting at least one nucleic acid sequence that encodes at least one domain of the endogenous PUFA PKS system and inserting therefore a nucleic acid sequence encoding a homologue of the endogenous domain to alter the PUFA production profile of the Thraustochytrid microorganism, wherein the homologue has a biological activity of at least one domain of a PKS system. In one aspect, the homologue of the endogenous domain comprises a modification, as compared to the endogenous domain, selected from the group consisting of at least one deletion, insertion or substitution that results in an alteration of PUFA production profile by the microorganism. In another aspect, the amino acid sequence of the homologue is at least about 60% identical, and more preferably about 70% identical, and more preferably about 80% identical, and more preferably about 90% identical to the amino acid sequence of the endogenous domain. In one aspect, homologue of the endogenous domain is a domain from a PUFA PKS system of another Thraustochytrid microorganism.

In another aspect, the endogenous PUFA PKS system has been modified by deleting at least one nucleic acid sequence that encodes at least one domain of the endogenous PUFA PKS system and inserting therefore a nucleic acid sequence encoding at least one domain of a PKS system from a different microorganism. In one aspect, the nucleic acid sequence encoding at least one domain of a PKS system from a different microorganism is from a bacterial PUFA PKS system. For example, the different microorganism can be a marine bacteria having a PUFA PKS system that naturally produces PUFAs at a temperature of about 25°C or greater. In one aspect, the marine bacteria is selected from the group consisting of *Shewanella olleyana* and *Shewanella japonica.* In one aspect, the domain of a PKS system from a different microorganism is from a PKS system selected from the group consisting of: a Type I PKS system, a Type II PKS system, a modular PKS system, and a PUFA PKS system from a different Thraustochytrid microorganism.

In any of the above aspects, the domain of the endogenous PUFA PKS system can include, but is not limited to, a domain having a biological activity of at least one of the

In one embodiment, the PUFA production profile is altered to initiate, increase or decrease production of eicosapentaenoic acid (EPA) by the microorganism. In another embodiment, the PUFA production profile is altered to initiate, increase or decrease production of docosahexaenoic acid (DHA) by the microorganism. In another embodiment, the PUFA production profile is altered to initiate, increase or decrease production of one or both isomers of docosapentaenoic acid (DPA) by the microorganism. In another embodiment, the PUFA production profile is altered to initiate, increase or decrease production of arachidonic acid (ARA) by the microorganism. In another embodiment, the Thraustochytrid is from a genus selected from the group consisting of *Schizochytrium, Thraustochytrium,* and *Japonochytrium.* In another embodiment, the Thraustochytrid is from the genus *Schizochytrium.* In another embodiment, the Thraustochytrid is from a *Schizochytrium* species selected from the group consisting of:
*Schizochytrium aggregatum, Schizochytrium limacinum,* and *Schizochytrium minutum.* In another embodiment, the Thraustochytrid is from the genus *Thraustochytrium* of SEQ ID NO:20, SEQ ID NO:22, SEQ ID NO:28 and SEQ ID NO:30 of the endogenous PUFA PKS system. In one aspect, the *Schizochytrium* has been modified by deleting at least one nucleic acid sequence that encodes at least one domain of the endogenous PUFA PKS system and inserting therefore a nucleic acid sequence encoding at least one domain of a PKS system from a non-*Schizochytrium* microorganism. In one aspect, the non-*Schizochytrium* microorganism grows and produces PUFAs at temperature of at least about 15°C, and more preferably at least about 20°C, and more preferably at least about 25°C, and more preferably at least about 30°C, and more preferably between about 20°C and about 40°C. In one aspect, the nucleic acid sequence encoding at least one domain of a PKS system from a non-*Schizochytrium* microorganism is from a bacterial PUFA PKS system. In one aspect, the bacterial PUFA PKS system is from a bacterium selected from the group consisting of *Shewanella olleyana* and *Shewanella japonica.* In another aspect, the nucleic acid sequence encoding at least one domain of a PKS system is selected from the group consisting of a Type I PKS system, a Type II PKS system, a modular PKS system, and a non-bacterial PUFA PKS system (e.g., a eukaryotic PUFA PKS system, such as a Thraustochytrid PUFA PKS system).

Another embodiment of the present invention relates to a genetically modified *Schizochytrium* that produces increased amounts of docosahexaenoic acid (DHA) as compared to a non-genetically modified *Schizochytrium,* wherein the *Schizochytrium* has an endogenous polyunsaturated fatty acid (PUFA) polyketide synthase (PKS) system comprising a genetic modification in at least one nucleic sequence that encodes at least one domain of the endogenous PUFA PKS system that results in increased the production of DHA by the *Schizochytrium.* In one aspect, at least one domain of the endogenous PUFA PKS system has been modified by substitution for at least one domain of a PUFA PKS system from *Thraustochytrium.* In one aspect, the ratio of DHA to DPA produced by the *Schizochytrium* is increased as compared to a non-genetically modified *Schizochytrium.*

Another embodiment of the present invention relates to a method to produce lipids enriched for at least one selected polyunsaturated fatty acid (PUFA), comprising culturing under conditions effective to produce the lipids a genetically modified Thraustochytrid microorganism as described above or a genetically modified *Schizochytrium* as described above. In one aspect, the selected PUFA is eicosapentaenoic acid (EPA).

### Brief Description of the Figures

Fig. 1 is a graphical representation of the domain structure of the *Schizochytrium* PUFA PKS system.
Fig. 2 shows a comparison of domains of PUFA PKS systems from *Schizochytrium* and *shewanella.*

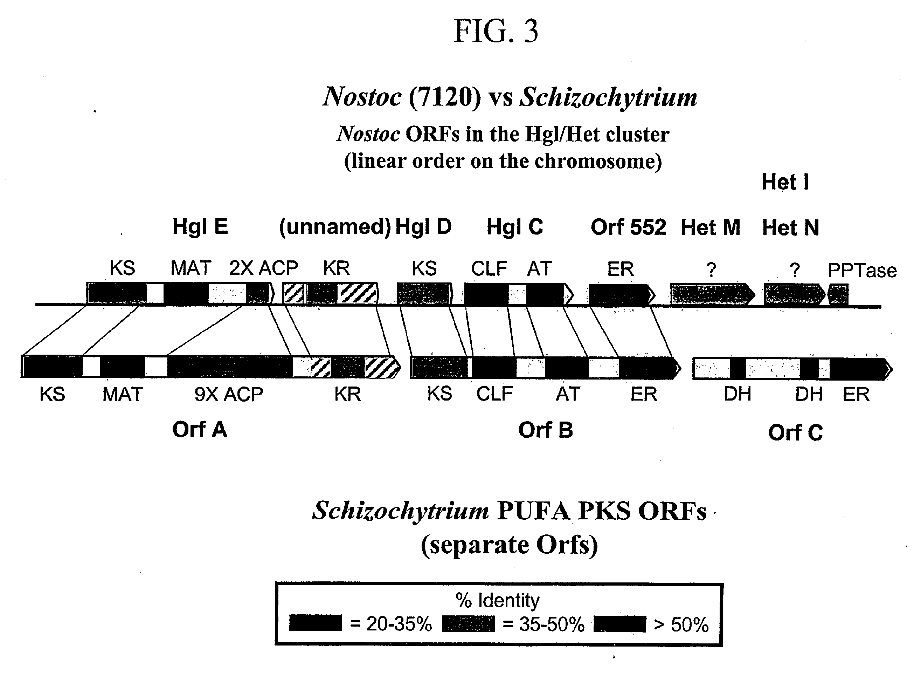

Fig. 3 shows a comparison of domains of PUFA PKS systems from *Schizochytrium* and a relaxed PKS system from *Nostoc* whose product is a long chain fatty acid that does not contain any double bonds.

### Detailed Description of the Invention

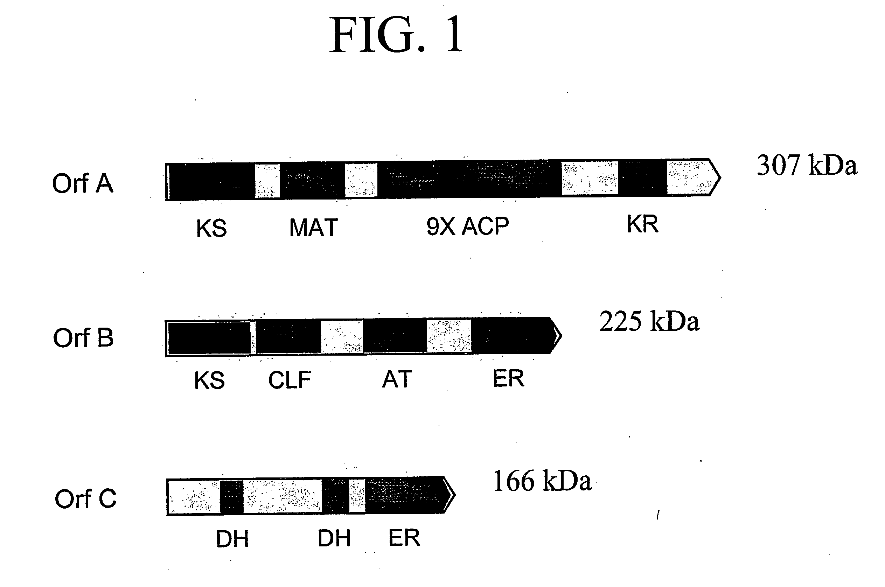

The present invention generally relates to polyunsaturated fatty acid (PUFA) polyketide synthase (PKS) systems, to genetically modified organisms comprising such PUFA PKS systems, to methods of making and using such systems for the production of products of interest, including bioactive molecules and particularly, PUFAs, such as DHA, DPA and EPA. As used herein, a PUFA PKS system generally has the following identifying features: (1) it produces PUFAs as a natural product of the system; and (2) it comprises several multifunctional proteins assembled into a complex that conducts both *iterative* processing of the fatty acid chain as well *non-iterative* processing, including *trans-cis* isomerization and enoyl reduction reactions in selected cycles (See Fig. 1, for example). Reference to a PUFA PKS system refers collectively to all of the genes and their encoded products that work in a complex to produce PUFAs in an organism. Therefore, the PUFA PKS system refers specifically to a PKS system for which the natural products are PUFAs.

More specifically, first, a PUFA PKS system that forms the basis of this invention produces polyunsaturated fatty acids (PUFAs) as products (i.e., an organism that endogenously (naturally) contains such a PKS system makes PUFAs using this system). The PUFAs referred to herein are preferably polyunsaturated fatty acids with a carbon chain length of at least 16 carbons, and more preferably at least 18 carbons, and more preferably at least 20 carbons, and more preferably 22 or more carbons, with at least 3 or more double bonds, and preferably 4 or more, and more preferably 5 or more, and even more preferably 6 or more double bonds, wherein all double bonds are in the *cis* configuration. It is an object of the present invention to find or create via genetic manipulation or manipulation of the endproduct, PKS systems which produce polyunsaturated fatty acids of desired chain length and with desired numbers of double bonds. Examples of PUFAs include, but are not limited to, DHA (docosahexaenoic acid (C22:6, ω-3)), ARA (eicosatetraenoic acid or arachidonic acid (C20:4, n-6)), DPA (docosapentaenoic acid (C22:5, ω-6 or ω-3)), and EPA (eicosapentaenoic acid (C20:5, ω-3)).

Second, the PUFA PKS system described herein incorporates both iterative and non-iterative reactions, which distinguish the system from previously described PKS systems (e.g., type I, type II or modular). More particularly, the PUFA PKS system described herein contains domains that appear to function during each cycle as well as those which appear to function during only some of the cycles. A key aspect of this functionality may be related to the domains showing homology to the bacterial Fab-A enzymes. For example, the Fab-A enzyme of *E*. *coli* has been shown to possess two enzymatic activities. It possesses a dehydration activity in which a water molecule (H₂O) is abstracted from a carbon chain containing a hydroxy group, leaving a *trans* double bond in that carbon chain. In addition, it has an isomerase activity in which the *trans* double bond is converted to the *cis* configuration. This isomerization is accomplished in conjunction with a migration of the double bond position to adjacent carbons. In PKS (and FAS) systems, the main carbon chain is extended in 2 carbon increments. One can therefore predict the number of extension reactions required to produce the PUFA products of these PKS systems. For example, to produce DHA (C22:6, all *cis)* requires 10 extension reactions. Since there are only 6 double bonds in the end product, it means that during some of the reaction cycles, a double bond is retained (as a *cis* isomer), and in others, the double bond is reduced prior to the next extension.

Before the discovery of a PUFA PKS system in marine bacteria (see U.S. Patent No. 6,140,486), PKS systems were not known to possess this combination of iterative and selective enzymatic reactions, and they were not thought of as being able to produce carbon-carbon double bonds in the *cis* configuration. However, the PUFA PKS system described by the present invention has the capacity to introduce *cis* double bonds and the capacity to vary the reaction sequence in the cycle.

The present inventors propose to use these features of the PUFA PKS system to produce a range of bioactive molecules that could not be produced by the previously described (Type II, Type I and modular) PKS systems. These bioactive molecules include, but are not limited to, polyunsaturated fatty acids (PUFAs), antibiotics or other bioactive compounds, many of which will be discussed below. For example, using the knowledge of the PUFA PKS gene structures described herein, any of a number of methods can be used to alter the PUFA PKS genes, or combine portions of these genes with other synthesis systems, including other PKS systems, such that new products are produced. The inherent ability of this particular type of system to do both iterative and selective reactions will enable this system to yield products that would not be found if similar methods were applied to other types of PKS systems.

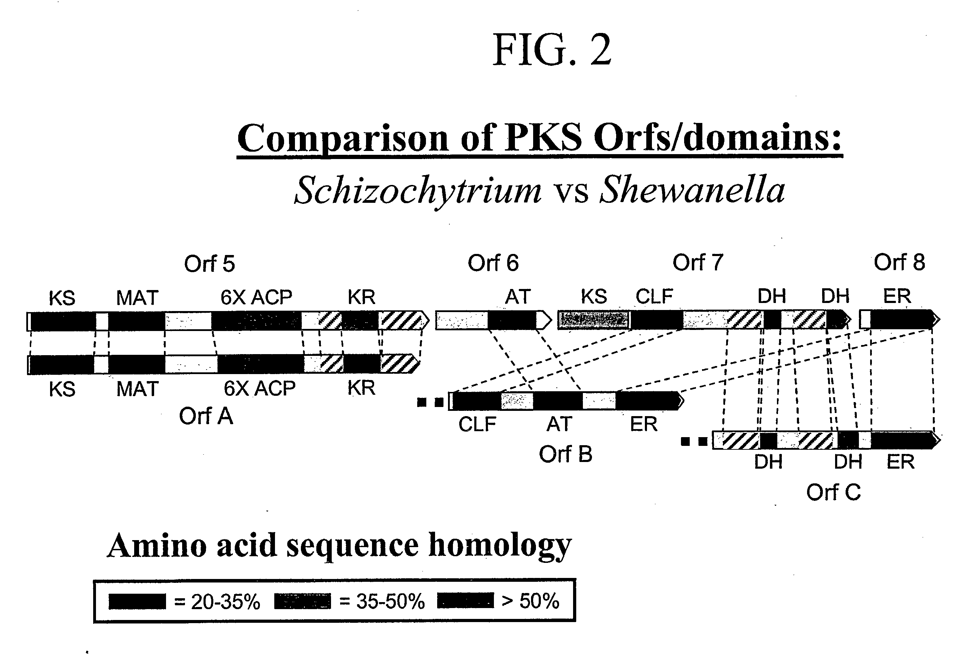

Much of the structure of the PKS system for PUFA synthesis in the eukaryotic Thraustochytrid, *Schizochytrium* has been described in detail in U.S. Patent No. 6,566,583. Complete sequencing of cDNA and genomic clones in *Schizochytrium* by the present inventors allowed the identification of the full-length genomic sequence of each of OrfA, OrfB and OrfC and the complete identification of the specific domains in these *Schizochytrium* Orfs with homology to those in *Shewanella* (see Fig. 2 and U.S. Patent Application Serial No. 10/124,800, *supra).* In U.S. Patent Application Serial No. 10/124,800, the inventors also identified a *Thraustochytrium* species as meeting the criteria for having a PUFA PKS system and then demonstrated that this organism was likely to contain genes with homology to *Schizochytrium* PUFA PKS genes by Southern blot analysis. However, the isolation and determination of the structure of such genes and the domain organization of the genes was not described in U.S. Patent Application Serial No. 10/124,800. In the present invention, the inventors have now cloned and sequenced the full-length genomic sequence of homologous open reading frames (Orfs) in this Thraustochytrid of the genus *Thraustochytrium* (specifically, *Thraustochytrium* sp. 23B (ATCC 20892)), and have identified the domains comprising the PUFA PKS system in this *Thraustochytrium.* Therefore, the present invention solves the above-mentioned problem of providing additional PUFA PKS systems that have the flexibility for commercial use. The *Thraustochytrium* PUFA PKS system is described in detail below.

The present invention also solves the above-identified problem for production of commercially valuable lipids enriched in a desired PUFA, such as EPA, by the present inventors' development of genetically modified microorganisms and methods for efficiently producing lipids (triacylglyerols (TAG) as well as membrane-associated phospholipids (PL)) enriched in PUFAs by manipulation of the polyketide synthase-like system that produces PUFAs in eukaryotes, including members of the order Thraustochytriales such as *Schizochytrium* and *Thraustochytrium.* Specifically, and by way of example, the present inventors describe herein a strain of *Schizochytrium* that has previously been optimized for commercial production of oils enriched in PUFA, primarily docosahexaenoic acid (DHA; C22:6 n-3) and docosapentaenoic acid (DPA; C22:5 n-6), and that will now be genetically modified such that EPA (C20:5 n-3) production (or other PUFA production) replaces the DHA production, without sacrificing the oil productivity characteristics of the organism. In addition, the present inventors describe herein the genetic modification of *Schizochytrium* with PUFA PKS genes from *Thraustochytrium* to improve the DHA production by the *Schizochytrium* organism, specifically by altering the ratio of DHA to DPA produced by the microorganism through the modification of the PUFA PKS system. These are only a few examples of the technology encompassed by the invention, as the concepts of the invention can readily be applied to other production organisms and other desired PUFAs as described in detail below.

In one embodiment, a PUFA PKS system according to the present invention comprises at least the following biologically active domains: (a) at least two enoyl-ACP reductase (ER) domains; (b) at least six acyl carrier protein (ACP) domains; (c) at least two β-ketoacyl-ACP synthase (KS) domains; (d) at least one acyltransferase (AT) domain; (e) at least one β-ketoacyl-ACP reductase (KR) domain; (f) at least two FabA-like β-hydroxyacyl-ACP dehydrase (DH) domains; (g) at least one chain length factor (CLF) domain; and (h) at least one malonyl-CoA:ACP acyltransferase (MAT) domain. The functions of these domains are generally individually known in the art and will be described in detail below with regard to the PUFA PKS system of the present invention.

In another embodiment, the PUFA PKS system comprises at least the following biologically active domains: (a) at least one enoyl-ACP reductase (ER) domain; (b) multiple acyl carrier protein (ACP) domains (at least from one to four, and preferably at least five, and more preferably at least six, and even more preferably seven, eight, nine, or more than nine); (c) at least two β-ketoacyl-ACP synthase (KS) domains; (d) at least one acyltransferase (AT) domain; (e) at least one β-ketoacyl-ACP reductase (KR) domain; (f) at least two FabA-like β-hydroxyacyl-ACP dehydrase (DH) domains; (g) at least one chain length factor (CLF) domain; and (h) at least one malonyl-CoA:ACP acyltransferase (MAT) domain. Preferably, such a PUFA PKS system is a non-bacterial PUFA-PKS system.

In one embodiment, a PUFA PKS system of the present invention is a non-bacterial PUFA PKS system. In other words, in one embodiment, the PUFA PKS system of the present invention is isolated from an organism that is not a bacterium, or is a homologue of, or derived from, a PUFA PKS system from an organism that is not a bacterium, such as a eukaryote or an archaebacterium. Eukaryotes are separated from prokaryotes based on the degree of differentiation of the cells, with eukaryotes having more highly differentiated cells and prokaryotes having less differentiated cells. In general, prokaryotes do not possess a nuclear membrane, do not exhibit mitosis during cell division, have only one chromosome, their cytoplasm contains 70S ribosomes, they do not possess any mitochondria, endoplasmic reticulum, chloroplasts, lysosomes or Golgi apparatus, their flagella (if present) consists of a single fibril. In contrast, eukaryotes have a nuclear membrane, they do exhibit mitosis during cell division, they have many chromosomes, their cytoplasm contains 80S ribosomes, they do possess mitochondria, endoplasmic reticulum, chloroplasts (in algae), lysosomes and Golgi apparatus, and their flagella (if present) consists of many fibrils. In general, bacteria are prokaryotes, while algae, fungi, protist, protozoa and higher plants are eukaryotes.

The PUFA PKS systems of the marine bacteria (e.g., *Shewanella* sp. strain SCRC2738 and *Vibrio marinus)* are not the basis of the present invention, although the present invention does contemplate the use of domains from these bacterial PUFA PKS systems *in conjunction* with domains from the non-bacterial PUFA PKS systems of the present invention. In addition, the present invention does contemplate the isolation and use of PUFA PKS gene sets (and proteins and domains encoded thereby) isolated from other bacteria (e.g. *Shewanella olleyana* and *Shewanella japonica)* that will be particularly suitable for use as sources of PUFA PKS genes for modifying or combining with the non-bacterial PUFA PKS genes described herein to produce hybrid constructs and genetically modified microorganisms and plants. For example, according to the present invention, genetically modified organisms can be produced which incorporate non-bacterial PUFA PKS functional domains with bacterial PUFA PKS functional domains, as well as PKS functional domains or proteins from other PKS systems (type I, type II, modular) or FAS systems. As discussed in more detail below, PUFA PKS genes from two species of *Shewanella,* namely *Shewanella olleyana* or *Shewanella japonica,* are exemplary bacterial genes that are preferred for use in genetically modified microorganisms, plants, and methods of the invention. PUFA PKS systems (genes and the proteins and domains encoded thereby) from such marine bacteria (e.g., *Shewanella olleyana* or *Shewanella japonica)* are encompassed by the present invention as novel PUFA PKS sequences.

According to the present invention, the terms/phrases "Thraustochytrid", "Thraustochytriales microorganism" and "microorganism ofthe order Thraustochytriales" can be used interchangeably and refer to any members of the order Thraustochytriales, which includes both the family Thraustochytriaceae and the family Labyrinthulaceae. The terms "Labyrinthulid" and "Labyrinthulaceae" are used herein to specifically refer to members of the family Labyrinthulaceae. To specifically reference Thraustochytrids that are members of the family Thraustochytriaceae, the term "Thraustochytriaceae" is used herein. Thus, for the present invention, members of the Labyrinthulids are considered to be included in the Thraustochytrids.

Developments have resulted in frequent revision of the taxonomy of the Thraustochytrids. Taxonomic theorists generally place Thraustochytrids with the algae or algae-like protists. However, because of taxonomic uncertainty, it would be best for the purposes of the present invention to consider the strains described in the present invention as Thraustochytrids to include the following organisms: Order: Thraustochytriales; Family: Thraustochytriaceae (Genera: *Thraustochytrium, Schizochytrium" Japonochytrium, Aplanochytrium,* or *Elina)* or Labyrinthulaceae (Genera *Labyrinthula, Labyrinthuloides,* or *Labyrinthomyxa).* Also, the following genera are sometimes included in either family Thraustochytriaceae or Labyrinthulaceae: *Althornia, Corallochytrium, Diplophyrys, and Pyrrhosorus),* and for the purposes of this invention are encompassed by reference to a Thraustochytrid or a member ofthe order Thraustochytriales. It is recognized that at the time of this invention, revision in the taxonomy of Thraustochytrids places the genus *Labyrinthuloides* in the family of Labyrinthulaceae and confirms the placement of the two families Thraustochytriaceae and Labyrinthulaceae within the Stramenopile lineage. It is noted that the Labyrinthulaceae are sometimes commonly called labyrinthulids or labyrinthula, or labyrinthuloides and the Thraustochytriaceae are commonly called thraustochytrids, although, as discussed above, for the purposes of clarity of this invention, reference to Thraustochytrids encompasses any member of the order Thraustochytriales and/or includes members of both Thraustochytriaceae and Labyrinthulaceae. Recent taxonomic changes are summarized below.

Strains of certain unicellular microorganisms disclosed herein are members of the order Thraustochytriales. Thraustochytrids are marine eukaryotes with an evolving taxonomic history. Problems with the taxonomic placement of the Thraustochytrids have been reviewed by Moss (1986), Bahnweb and Jackle (1986) and Chamberlain and Moss (1988).

For convenience purposes, the Thraustochytrids were first placed by taxonomists with other colorless zoosporic eukaryotes in the Phycomycetes (algae-like fungi). The name Phycomycetes, however, was eventually dropped from taxonomic status, and the Thraustochytrids were retained in the Oomycetes (the biflagellate zoosporic fungi). It was initially assumed that the Oomycetes were related to the heterokont algae, and eventually a wide range of ultrastructural and biochemical studies, summarized by Barr (Barr, 1981, Biosystems 14:359-370) supported this assumption. The Oomycetes were in fact accepted by Leedale (Leedale, 1974, Taxon 23:261-270) and other phycologists as part of the heterokont algae. However, as a matter of convenience resulting from their heterotrophic nature, the Oomycetes and Thraustochytrids have been largely studied by mycologists (scientists who study fungi) rather than phycologists (scientists who study algae).

From another taxonomic perspective, evolutionary biologists have developed two general schools of thought as to how eukaryotes evolved. One theory proposes an exogenous origin of membrane-bound organelles through a series of endosymbioses (Margulis, 1970, Origin of Eukaryotic Cells. Yale University Press, New Haven); e.g., mitochondria were derived from bacterial endosymbionts, chloroplasts from cyanophytes, and flagella from spirochaetes. The other theory suggests a gradual evolution of the membrane-bound organelles from the non-membrane-bounded systems of the prokaryote ancestor via an autogenous process (Cavalier-Smith, 1975, Nature (Lond.) 256:462-468). Both groups of evolutionary biologists however, have removed the Oomycetes and Thraustochytrids from the fungi and place them either with the chromophyte algae in the kingdom Chromophyta (Cavalier-Smith, 1981, BioSysterns 14:461-481) (this kingdom has been more recently expanded to include other protists and members of this kingdom are now called Stramenopiles) or with all algae in the kingdom Protoctista (Margulis and Sagen, 1985, Biosystems 18:141-147).

With the development of electron microscopy, studies on the ultrastructure of the zoospores of two genera of Thraustochytrids, *Thraustochytrium* and *Schizochytrium,* (Perkins, 1976, pp. 279-312 in "Recent Advances in Aquatic Mycology" (ed. E.B.G. Jones), John Wiley & Sons, New York; Kazama, 1980, Can. J. Bot. 58:2434-2446; Barr, 1981, Biosystems 14:359-370) have provided good evidence that the Thraustochytriaceae are only distantly related to the Oomycetes. Additionally, genetic data representing a correspondence analysis (a form of multivariate statistics) of 5-S ribosomal RNA sequences indicate that Thraustochytriales are clearly a unique group of eukaryotes, completely separate from the fungi, and most closely related to the red and brown algae, and to members of the Oomycetes (Mannella, et al., 1987, Mol. Evol. 24:228-235). Most taxonomists have agreed to remove the Thraustochytrids from the Oomycetes (Bartnicki-Garcia, 1987, pp. 389-403 in "Evolutionary Biology of the Fungi" (eds. Rayner, A.D.M., Brasier, C.M. & Moore, D.), Cambridge University Press, Cambridge).

In summary, employing the taxonomic system of Cavalier-Smith (Cavalier-Smith, 1981, BioSystems 14:461-481,1983; Cavalier-Smith,1993, Microbiol Rev. 57:953-994), the Thraustochytrids are classified with the chromophyte algae in the kingdom Chromophyta (Stramenopiles). This taxonomic placement has been more recently reaffirmed by Cavalier-Smith et al. using the 18s rRNA signatures of the Heterokonta to demonstrate that Thraustochytrids are chromists not Fungi (Cavalier-Smith et al.,1994, Phil. Tran. Roy. Soc. London Series BioSciences 346:387-397). This places the Thraustochytrids in a completely different kingdom from the fungi, which are all placed in the kingdom Eufungi.

Currently, there are 71 distinct groups of eukaryotic organisms (Patterson 1999) and within these groups four major lineages have been identified with some confidence: (1) Alveolates, (2) Stramenopiles, (3) a Land Plant-green algae-Rhodophyte_Glaucophyte ("plant") clade and (4) an Opisthokont clade (Fungi and Animals). Formerly these four maj or lineages would have been labeled Kingdoms but use of the "kingdom" concept is no longer considered useful by some researchers.

As noted by Armstrong, Stramenopile refers to three-parted tubular hairs, and most members of this lineage have flagella bearing such hairs. Motile cells of the Stramenopiles (unicellular organisms, sperm, zoopores) are asymmetrical having two laterally inserted flagella, one long, bearing three-parted tubular hairs that reverse the thrust of the flagellum, and one short and smooth. Formerly, when the group was less broad, the Stramenopiles were called Kingdom Chromista or the heterokont (=different flagella) algae because those groups consisted of the Brown Algae or Phaeophytes, along with the yellow-green Algae, Golden-brown Algae, Eustigmatophytes and Diatoms. Subsequently some heterotrophic, fungal-like organisms, the water molds, and labyrinthulids (slime net amoebas), were found to possess similar motile cells, so a group name referring to photosynthetic pigments or algae became inappropriate. Currently, two of the families within the Stramenopile lineage are the Labyrinthulaceae and the Thraustochytriaceae. Historically, there have been numerous classification strategies for these unique microorganisms and they are often classified under the same order (i.e., Thraustochytriales). Relationships of the members in these groups are still developing. Porter and Leander have developed data based on 18S small subunit ribosomal DNA indicating the thraustochytrid-labyrinthulid clade in monophyletic. However, the clade is supported by two branches; the first contains three species of *Thraustochytrium* and *Ulkenia profunda,* and the second includes three species of *Labyrinthula,* two species of *Labyrinthuloides* and *Schizochytrium aggregatum.*

The taxonomic placement of the Thraustochytrids as used in the present invention is therefore summarized below:
Kingdom: Chromophyta (Stramenopiles)
Phylum: Heterokonta
Order: Thraustochytriales (Thraustochytrids)
Family: Thraustochytriaceae or Labyrinthulaceae
Genera: *Thraustochytrium, Schizochytrium, Japonochytrium, Aplanochytrium, Elina, Labyrinthula, Labyrinthuloides,* or *Labyrinthulomyxa*

Some early taxonomists separated a few original members of the genus *Thraustochytrium* (those with an amoeboid life stage) into a separate genus called *Ulkenia.* However it is now known that most, if not all, Thraustochytrids (including *Thraustochytrium* and *Schizochytrium*), exhibit amoeboid stages and as such, *Ulkenia* is not considered by some to be a valid genus. As used herein, the genus *Thraustochytrium* will include *Ulkenia.*

Despite the uncertainty of taxonomic placement within higher classifications of Phylum and Kingdom, the Thraustochytrids remain a distinctive and characteristic grouping whose members remain classifiable within the order Thraustochytriales.

*Schizochytrium* is a Thraustochytrid marine microorganism that accumulates large quantities oftriacylglycerols rich in DHA and docosapentaenoic acid (DPA; 22:5 ω-6); e.g., 30% DHA + DPA by dry weight (Barclay et al., J. Appl. Playcol. 6, 123 (1994)). In eukaryotes that synthesize 20- and 22-carbon PUFAs by an elongation/desaturation pathway, the pools of 18-, 20- and 22-carbon intermediates are relatively large so that *in vivo* labeling experiments using [¹⁴C]-acetate reveal clear precursor-product kinetics for the predicted intermediates (Gellerman et al., Biochim. Biophys. Acta 573:23 (1979)). Furthermore, radiolabeled intermediates provided exogenously to such organisms are converted to the final PUFA products. The present inventors have shown that [1-¹⁴C]-acetate was rapidly taken up by *Schizochytrium* cells and incorporated into fatty acids, but at the shortest labeling time (1 min), DHA contained 31 % of the label recovered in fatty acids, and this percentage remained essentiallyunchanged during the 10-15 min of [¹⁴C]-acetate incorporation and the subsequent 24 hours of culture growth. Similarly, DPA represented 10% of the label throughout the experiment. There is no evidence for a precursor-product relationship between 16- or 18-carbon fatty acids and the 22-carbon polyunsaturated fatty acids. These results are consistent with rapid synthesis of DHA from [¹⁴C]-acetate involving very small (possibly enzyme-bound) pools of intermediates. A cell-free homogenate derived from *Schizochytrium* cultures incorporated [1-¹⁴C]-malonyl-CoA into DHA, DPA, and saturated fatty acids. The same biosynthetic activities were retained by a 100,000xg supernatant fraction but were not present in the membrane pellet. Thus, DHA and DPA synthesis in *Schizochytrium* does not involve membrane-bound desaturases or fatty acid elongation enzymes like those described for other eukaryotes (Parker-Barnes et al., 2000, *supra;* Shanklin et al., 1998, *supra*)*.* These fractionation data contrast with those obtained from the *Shewanella* enzymes (See Metz et al., 2001, *supra)* and may indicate use of a different (soluble) acyl acceptor molecule, such as CoA, by the *Schizochytrium* enzyme. It is expected that *Thraustochytrium* will have a similar biochemistry.

In U.S. Patent No. 6,566,583, a cDNA library from *Schizochytrium* was constructed and approximately 8500 random clones (ESTs) were sequenced. Sequences that exhibited homology to 8 of the 11 domains of the *Shewanella* PKS genes shown in Fig. 2 were all identified at frequencies of 0.2-0.5%. In U.S. Patent No. 6,566,583, several cDNA clones from *Schizochytrium* showing homology to the *Shewanella* PKS genes were sequenced, and various clones were assembled into nucleic acid sequences representing two partial open reading frames and one complete open reading frame.

Further sequencing of cDNA and genomic clones by the present inventors allowed the identification of the full-length genomic sequence of each of OrfA, OrfB and OrfC in *Schizochytrium* and the complete identification of the domains in *Schizochytrium* with homology to those in *Shewanella* (see Fig. 2). These genes are described in detail in U.S. Patent Application Serial No. 10/124,800, *supra* and are described in some detail below.

The present inventors have now identified, cloned, and sequenced the full-length genomic sequence of homologous Orfs in a Thraustochytrid of the genus *Thraustochytrium* (specifically, *Thraustochytrium* sp. 23B (ATCC 20892)) and have identified the domains comprising the PUFA PKS system in this *Thraustochytrium.*

Based on the comparison of the domains of the PUFA PKS system of *Schizochytrium* with the domains of the PUFA PKS system of *Shewanella,* clearly, the *Schizochytrium* genome encodes proteins that are highly similar to the proteins in *Shewanella* that are capable of catalyzing EPA synthesis. The proteins in *Schizochytrium* constitute a PUFA PKS system that catalyzes DHA and DPA synthesis. Simple modification of the reaction scheme identified for *Shewanella* will allow for DHA synthesis in *Schizochytrium.* The homology between the prokaryotic *Shewanella* and eukaryotic *Schizochytrium* genes suggests that the PUFA PKS has undergone lateral gene transfer.

A similar comparison can be made for *Thraustochytrium.* In all cases, comparison of the *Thraustochytrium* 23B *(Th.* 23B) PUFA PKS proteins or domains to other known sequences revealed that the closest match was one of the *Schizochytrium* PUFA PKS proteins (OrfA, B or C, or a domain therefrom) as described in U.S. Patent Application Serial No. 10/124,800, *supra.* The next closest matches in all cases were to one of the PUFA PKS proteins from marine bacteria *(Shewanella* SCRC-2738, *Shewanella oneidensis, Photobacter profundum* and *Moritella marina)* or from a related system found in nitrogen fixing cyanobacteria (e.g., *Nostoc punctiforme* and *Nostoc* sp. PCC 7120). The products of the cyanobacterial enzyme systems lack double bonds and the proteins lack domains related to the DH domains implicated in *cis* double bond formation (i.e., the FabA related DH domains).

According to the present invention, the phrase "open reading frame" is denoted by the abbreviation "Orf'. It is noted that the protein encoded by an open reading frame can also be denoted in all upper case letters as "ORF" and a nucleic acid sequence for an open reading frame can also be denoted in all lower case letters as "orf", but for the sake of consistency, the spelling "Orf" is preferentially used herein to describe either the nucleic acid sequence or the protein encoded thereby. It will be obvious from the context of the usage of the term whether a protein or nucleic acid sequence is referenced.

### Schizochytrium PUFA PKS

Fig. 1 is a graphical representation of the three open reading frames from the *Schizochytrium* PUFA PKS system, and includes the domain structure of this PUFA PKS system. As described in detail in U.S. Patent Application Serial No. 10/124,800, the domain structure of each open reading frame is as follows:

### Open Reading Frame A (OrfA):

The complete nucleotide sequence for OrfA is represented herein as SEQ ID NO: 1. OrfA is a 8730 nucleotide sequence (not including the stop codon) which encodes a 2910 amino acid sequence, represented herein as SEQ ID NO:2. Within OrfA are twelve domains: (a) one β-ketoacyl-ACP synthase (KS) domain; (b) one malonyl-CoA:ACP acyltransferase (MAT) domain; (c) nine acyl carrier protein (ACP) domains; and (d) one β-ketoacyl-ACP reductase (KR) domain. The nucleotide sequence for OrfA has been deposited with GenBank as Accession No. AF378327 (amino acid sequence Accession No. AAK728879).
The first domain in *Schizochytrium* OrfA is a β-ketoacyl-ACP synthase (KS) domain, also referred to herein as OrfA-KS. This domain is contained within the nucleotide sequence spanning from a starting point of between about positions 1 and 40 of SEQ ID NO:1 (OrfA) to an ending point of between about positions 1428 and 1500 of SEQ ID NO:1. The nucleotide sequence containing the sequence encoding the OrfA-KS domain is represented herein as SEQ ID NO:7 (positions 1-1500 of SEQ ID NO:1). The amino acid sequence containing the KS domain spans from a starting point of between about positions 1 and 14 of SEQ ID NO:2 (OrfA) to an ending point of between about positions 476 and 500 of SEQ ID NO:2. The amino acid sequence containing the OrfA-KS domain is represented herein as SEQ ID NO:8 (positions 1-500 of SEQ ID NO:2). It is noted that the OrfA-KS domain contains an active site motif: DXAC* (*acyl binding site C₂₁₅).

According to the present invention, a domain or protein having β-ketoacyl-ACP synthase (KS) biological activity (function) is characterized as the enzyme that carries out the initial step of the FAS (and PKS) elongation reaction cycle. The term "β-ketoacyl-ACP synthase" can be used interchangeably with the terms "3-keto acyl-ACP synthase", "β-keto acyl-ACP synthase", and "keto-acyl ACP synthase", and similar derivatives. The acyl group destined for elongation is linked to a cysteine residue at the active site of the enzyme by a thioester bond. In the multi-step reaction, the acyl-enzyme undergoes condensation with malonyl-ACP to form -ketoacyl-ACP, CO₂ and free enzyme. The KS plays a key role in the elongation cycle and in many systems has been shown to possess greater substrate specificity than other enzymes of the reaction cycle. For example, *E*. *coli* has three distinct KS enzymes - each with its own particular role in the physiology of the organism (Magnuson et al., Microbiol. Rev. 57, 522 (1993)). The two KS domains ofthe PUFA-PKS systems could have distinct roles in the PUFA biosynthetic reaction sequence.

As a class of enzymes, KS's have been well characterized. The sequences of many verified KS genes are known, the active site motifs have been identified and the crystal structures of several have been determined. Proteins (or domains of proteins) can be readily identified as belonging to the KS family of enzymes by homology to known KS sequences.

The second domain in OrfA is a malonyl-CoA:ACP acyltransferase (MAT) domain, also referred to herein as OrfA-MAT. This domain is contained within the nucleotide sequence spanning from a starting point of between about positions 1723 and 1798 of SEQ ID NO:1 (OrfA) to an ending point of between about positions 2805 and 3000 of SEQ ID NO:1. The nucleotide sequence containing the sequence encoding the OrfA-MAT domain is represented herein as SEQ ID NO:9 (positions 1723-3000 of SEQ ID NO:1). The amino acid sequence containing the MAT domain spans from a starting point of between about positions 575 and 600 of SEQ ID NO:2 (OrfA) to an ending point of between about positions 935 and 1000 of SEQ ID NO:2. The amino acid sequence containing the OrfA-MAT domain is represented herein as SEQ ID NO:10 (positions 575-1000 of SEQ ID NO:2). It is noted that the OrfA-MAT domain contains an active site motif: GHS*XG (*acyl binding site S₇₀₆), represented herein as SEQ ID NO:11.

According to the present invention, a domain or protein having malonyl-CoA:ACP acyltransferase (MAT) biological activity (function) is characterized as one that transfers the malonyl moiety from malonyl-CoA to ACP. The term "malonyl-CoA:ACP acyltransferase" can be used interchangeably with "malonyl acyltransferase" and similar derivatives. In addition to the active site motif (GxSxG), these enzymes possess an extended motif (R and Q amino acids in key positions) that identifies them as MAT enzymes (in contrast to the AT domain of Schizochytrium Orf B). In some PKS systems (but not the PUFA PKS domain) MAT domains will preferentially load methyl- or ethyl- malonate on to the ACP group (from the corresponding CoA ester), thereby introducing branches into the linear carbon chain. MAT domains can be recognized by their homology to known MAT sequences and by their extended motif structure.

Domains 3-11 of OrfA are nine tandem acyl carrier protein (ACP) domains, also referred to herein as OrfA-ACP (the first domain in the sequence is OrfA-ACP1, the second domain is OrfA-ACP2, the third domain is OrfA-ACP3, etc.). The first ACP domain, OrfA-ACP1, is contained within the nucleotide sequence spanning from about position 3343 to about position 3600 of SEQ ID NO:1 (OrfA). The nucleotide sequence containing the sequence encoding the OrfA-ACP1 domain is represented herein as SEQ ID NO:12 (positions 3343-3600 of SEQ ID NO:1). The amino acid sequence containing the first ACP domain spans from about position 1115 to about position 1200 of SEQ ID NO:2. The amino acid sequence containing the OrfA-ACP1 domain is represented herein as SEQ ID NO:13 (positions 1115-1200 of SEQ ID NO:2). It is noted that the OrfA-ACP1 domain contains an active site motif: LGIDS* (*pantetheine binding motif S₁₁₅₇), represented herein by SEQ ID NO:14.

The nucleotide and amino acid sequences of all nine ACP domains are highly conserved and therefore, the sequence for each domain is not represented herein by an individual sequence identifier. However, based on the information disclosed herein, one of skill in the art can readily determine the sequence containing each of the other eight ACP domains (see discussion below).

All nine ACP domains together span a region of OrfA of from about position 3283 to about position 6288 of SEQ ID NO:1, which corresponds to amino acid positions of from about 1095 to about 2096 of SEQ ID NO:2. The nucleotide sequence for the entire ACP region containing all nine domains is represented herein as SEQ ID NO:16. The region represented by SEQ ID NO:16 includes the linker segments between individual ACP domains. The repeat interval for the nine domains is approximately every 330 nucleotides of SEQ ID NO:16 (the actual number of amino acids measured between adjacent active site serines ranges from 104 to 116 amino acids). Each of the nine ACP domains contains a pantetheine binding motif LGIDS* (represented herein by SEQ ID NO:14), wherein S* is the pantetheine binding site serine (S). The pantetheine binding site serine (S) is located near the center of each ACP domain sequence. At each end of the ACP domain region and between each ACP domain is a region that is highly enriched for proline (P) and alanine (A), which is believed to be a linker region. For example, between ACP domains 1 and 2 is the sequence: APAPVKAAAPAAPVASAPAPA, represented herein as SEQ ID NO:15. The locations of the active site serine residues (i.e., the pantetheine binding site) for each of the nine ACP domains, with respect to the amino acid sequence of SEQ ID NO:2, are as follows: ACP1 = S₁₁₅₇; ACP2 = S₁₂₆₆; ACP3 = S₁₃₇₇; ACP4 = S₁₄₈₈; ACP5 = S₁₆₀₄; ACP6 = S₁₇₁₅; ACP7 = S₁₈₁₉; ACP8 = S₁₉₃₀, and ACP9 = S₂₀₃₄. Given that the average size of an ACP domain is about 85 amino acids, excluding the linker, and about 110 amino acids including the linker, with the active site serine being approximately in the center of the domain, one of skill in the art can readily determine the positions of each of the nine ACP domains in OrfA.

According to the present invention, a domain or protein having acyl carrier protein (ACP) biological activity (function) is characterized as being small polypeptides (typically, 80 to 100 amino acids long), that function as carriers for growing fatty acyl chains via a thioester linkage to a covalently bound co-factor of the protein. They occur as separate units or as domains within larger proteins. ACPs are converted from inactive apo-forms to functional holo-forms by transfer of the phosphopantetheinyl moeity of CoA to a highly conserved serine residue of the ACP. Acyl groups are attached to ACP by a thioester linkage at the free terminus of the phosphopantetheinyl moiety. ACPs can be identified by labeling with radioactive pantetheine and by sequence homology to known ACPs. The presence of variations of the above mentioned motif (LGIDS*) is also a signature of an ACP.

Domain 12 in OrfA is a β-ketoacyl-ACP reductase (KR) domain, also referred to herein as OrfA-KR. This domain is contained within the nucleotide sequence spanning from a starting point of about position 6598 of SEQ ID NO:1 to an ending point of about position 8730 of SEQ ID NO:1. The nucleotide sequence containing the sequence encoding the OrfA-KR domain is represented herein as SEQ ID NO:17 (positions 6598-8730 of SEQ ID NO:1). The amino acid sequence containing the KR domain spans from a starting point of about position 2200 of SEQ ID NO:2 (OrfA) to an ending point of about position 2910 of SEQ ID NO:2. The amino acid sequence containing the OrfA-KR domain is represented herein as SEQ ID NO:18 (positions 2200-2910 of SEQ ID NO:2). Within the KR domain is a core region with homology to short chain aldehyde-dehydrogenases (KR is a member of this family). This core region spans from about position 7198 to about position 7500 of SEQ ID NO:1, which corresponds to amino acid positions 2400-2500 of SEQ ID NO:2.

According to the present invention, a domain or protein having β-ketoacyl-ACP reductase (KR) activity is characterized as one that catalyzes the pyridine-nucleotide-dependent reduction of 3-ketoacyl forms of ACP. The term "β-ketoacyl-ACP reductase" can be used interchangeably with the terms "ketoreductase", "3-ketoacyl-ACP reductase", "ketoacyl ACP reductase" and similar derivatives of the term. It is the first reductive step in the *de novo* fatty acid biosynthesis elongation cycle and a reaction often performed in polyketide biosynthesis. Significant sequence similarity is observed with one family of enoyl-ACP reductases (ER), the other reductase of FAS (but not the ER family present in the PUFA PKS system), and the short-chain alcohol dehydrogenase family. Pfam analysis ofthe PUFA PKS region indicated above reveals the homology to the short-chain alcohol dehydrogenase family in the core region. Blast analysis of the same region reveals matches in the core area to known KR enzymes as well as an extended region of homology to domains from the other characterized PUFA PKS systems.

### Oven Reading Frame B (OrfB):

The complete nucleotide sequence for OrfB is represented herein as SEQ ID NO:3. OrfB is a 6177 nucleotide sequence (not including the stop codon) which encodes a 2059 amino acid sequence, represented herein as SEQ ID NO:4. Within OrfB are four domains: (a) one β-ketoacyl-ACP synthase (KS) domain; (b) one chain length factor (CLF) domain; (c) one acyltransferase (AT) domain; and, (d) one enoyl-ACP reductase (ER) domain. The nucleotide sequence for OrfB has been deposited with GenBank as Accession No. AF378328 (amino acid sequence Accession No. AAK728880).

The first domain in OrfB is a β-ketoacyl-ACP synthase (KS) domain, also referred to herein as OrfB-KS. This domain is contained within the nucleotide sequence spanning from a starting point of between about positions 1 and 43 of SEQ ID NO:3 (OrfB) to an ending point of between about positions 1332 and 1350 of SEQ ID NO:3. The nucleotide sequence containing the sequence encoding the OrfB-KS domain is represented herein as SEQ ID NO:19 (positions 1-1350 of SEQ ID NO:3). The amino acid sequence containing the KS domain spans from a starting point of between about positions 1 and 15 of SEQ ID NO:4 (OrfB) to an ending point of between about positions 444 and 450 of SEQ ID NO:4. The amino acid sequence containing the OrfB-KS domain is represented herein as SEQ ID NO:20 (positions 1-450 of SEQ ID NO:4). It is noted that the OrfB-KS domain contains an active site motif: DXAC* (*acyl binding site C₁₉₆). KS biological activity and methods of identifying proteins or domains having such activity is described above.

The second domain in OrfB is a chain length factor (CLF) domain, also referred to herein as OrfB-CLF. This domain is contained within the nucleotide sequence spanning from a starting point of between about positions 1378 and 1402 of SEQ ID NO:3 (OrfB) to an ending point of between about positions 2682 and 2700 of SEQ ID NO:3. The nucleotide sequence containing the sequence encoding the OrfB-CLF domain is represented herein as SEQ ID NO:21 (positions 1378-2700 of SEQ ID NO:3). The amino acid sequence containing the CLF domain spans from a starting point of between about positions 460 and 468 of SEQ ID NO:4 (OrfB) to an ending point of between about positions 894 and 900 of SEQ ID NO:4. The amino acid sequence containing the OrfB-CLF domain is represented herein as SEQ ID NO:22 (positions 460-900 of SEQ ID NO:4). It is noted that the OrfB-CLF domain contains a KS active site motif without the acyl-binding cysteine.

According to the present invention, a domain or protein is referred to as a chain length factor (CLF) based on the following rationale. The CLF was originally described as characteristic of Type II (dissociated enzymes) PKS systems and was hypothesized to play a role in determining the number of elongation cycles, and hence the chain length, of the end product. CLF amino acid sequences show homology to KS domains (and are thought to form heterodimers with a KS protein), but they lack the active site cysteine. CLF's role in PKS systems is currently controversial. New evidence (C. Bisang et al., *Nature* 401, 502 (1999)) suggests a role in priming (providing the initial acyl group to be elongated) the PKS systems. In this role the CLF domain is thought to decarboxylate malonate (as malonyl-ACP), thus forming an acetate group that can be transferred to the KS active site. This acetate therefore acts as the 'priming' molecule that can undergo the initial elongation (condensation) reaction. Homologues of the Type II CLF have been identified as 'loading' domains in some modular PKS systems. A domain with the sequence features of the CLF is found in all currently identified PUFA PKS systems and in each case is found as part of a multidomain protein.

The third domain in OrfB is an AT domain, also referred to herein as OrfB-AT. This domain is contained within the nucleotide sequence spanning from a starting point of between about positions 2701 and 3598 of SEQ ID NO:3 (OrfB) to an ending point of between about positions 3975 and 4200 of SEQ ID NO:3. The nucleotide sequence containing the sequence encoding the OrfB-AT domain is represented herein as SEQ ID NO:23 (positions 2701-4200 of SEQ ID NO:3). The amino acid sequence containing the AT domain spans from a starting point of between about positions 901 and 1200 of SEQ ID NO:4 (OrfB) to an ending point of between about positions 1325 and 1400 of SEQ ID NO:4. The amino acid sequence containing the OrfB-AT domain is represented herein as SEQ ID NO:24 (positions 901-1400 of SEQ ID NO:4). It is noted that the OrfB-AT domain contains an active site motif of GxS*xG (*acyl binding site S₁₁₄₀) that is characteristic of acyltransferse (AT) proteins.

An "acyltransferase" or "AT" refers to a general class of enzymes that can carry out a number of distinct acyl transfer reactions. The term "acyltransferase" can be used interchangeably with the term "acyl transferase". The *Schizochytrium* domain shows good homology to a domain present in all of the other PUFA PKS systems currently examined and very weak homology to some acyltransferases whose specific functions have been identified (e.g. to malonyl-CoA:ACP acyltransferase, MAT). In spite of the weak homology to MAT, this AT domain is not believed to function as a MAT because it does not possess an extended motif structure characteristic of such enzymes (see MAT domain description, above). For the purposes of this disclosure, the functions of the AT domain in a PUFA PKS system include, but are not limited to: transfer of the fatty acyl group from the OrfA ACP domain(s) to water (i.e. a thioesterase - releasing the fatty acyl group as a free fatty acid), transfer of a fatty acyl group to an acceptor such as CoA, transfer of the acyl group among the various ACP domains, or transfer of the fatty acyl group to a lipophilic acceptor molecule (e.g. to lysophosphadic acid).

The fourth domain in OrfB is an ER domain, also referred to herein as OrfB-ER. This domain is contained within the nucleotide sequence spanning from a starting point of about position 4648 of SEQ ID NO:3 (OrfB) to an ending point of about position 6177 of SEQ ID NO:3. The nucleotide sequence containing the sequence encoding the OrfB-ER domain is represented herein as SEQ ID NO:25 (positions 4648-6177 of SEQ ID NO:3). The amino acid sequence containing the ER domain spans from a starting point of about position 1550 of SEQ ID NO:4 (OrfB) to an ending point of about position 2059 of SEQ ID NO:4. The amino acid sequence containing the OrfB-ER domain is represented herein as SEQ ID NO:26 (positions 1550-2059 of SEQ ID NO:4).

According to the present invention, this domain has enoyl-ACP reductase (ER) biological activity. According to the present invention, the term "enoyl-ACP reductase" can be used interchangeably with "enoyl reductase", "enoyl ACP-reductase" and "enoyl acyl-ACP reductase". The ER enzyme reduces the trans-double bond (introduced by the DH activity) in the fatty acyl-ACP, resulting in fully saturating those carbons. The ER domain in the PUFA-PKS shows homology to a newly characterized family of ER enzymes (Heath et al., Nature 406, 145 (2000)). Heath and Rock identified this new class of ER enzymes by cloning a gene of interest from *Streptococcus pneumoniae,* purifying a protein expressed from that gene, and showing that it had ER activity in an *in vitro* assay. The sequence of the Schizochytrium ER domain of OrfB shows homology to the S. *pneumoniae* ER protein. All of the PUFA PKS systems currently examined contain at least one domain with very high sequence homology to the *Schizochytrium* ER domain. The *Schizochytrium* PUFA PKS system contains two ER domains (one on OrfB and one on OrfC).

### Open Reading Frame C (OrfC):

The complete nucleotide sequence for OrfC is represented herein as SEQ ID NO:5. OrfC is a 4509 nucleotide sequence (not including the stop codon) which encodes a 1503 amino acid sequence, represented herein as SEQ ID NO:6. Within OrfC are three domains: (a) two FabA-like β-hydroxyacyl-ACP dehydrase (DH) domains; and (b) one enoyl-ACP reductase (ER) domain. The nucleotide sequence for OrfC has been deposited with GenBank as Accession No. AF378329 (amino acid sequence Accession No. AAK728881).

The first domain in OrfC is a DH domain, also referred to herein as OrfC-DH1. This is one of two DH domains in OrfC, and therefore is designated DH1. This domain is contained within the nucleotide sequence spanning from a starting point of between about positions 1 and 778 of SEQ ID NO:5 (OrfC) to an ending point of between about positions 1233 and 1350 of SEQ ID NO:5. The nucleotide sequence containing the sequence encoding the OrfC-DH1 domain is represented herein as SEQ ID NO:27 (positions 1-1350 of SEQ ID NO:5). The amino acid sequence containing the DH1 domain spans from a starting point of between about positions 1 and 260 of SEQ ID NO:6 (OrfC) to an ending point of between about positions 411 and 450 of SEQ ID NO:6. The amino acid sequence containing the OrfC-DH1 domain is represented herein as SEQ ID NO:28 (positions 1-450 of SEQ ID NO:6).

According to the present invention, this domain has FabA-like β-hydroxyacyl-ACP dehydrase (DH) biological activity. The term "FabA-like β-hydroxyacyl-ACP dehydrase" can be used interchangeably with the terms "FabA-like β-hydroxy acyl-ACP dehydrase", "β-hydroxyacyl-ACP dehydrase", "dehydrase" and similar derivatives. The characteristics of both the DH domains (see below for DH 2) in the PUFA PKS systems have been described in the preceding sections. This class of enzyme removes HOH from a β-ketoacyl-ACP and leaves a *trans* double bond in the carbon chain. The DH domains of the PUFA PKS systems show homology to bacterial DH enzymes associated with their FAS systems (rather than to the DH domains of other PKS systems). A subset of bacterial DH's, the FabA-like DH's, possesses *cis-trans* isomerase activity (Heath et al., J. Biol. Chem., 271, 27795 (1996)). It is the homologies to the FabA-like DH's that indicate that one or both of the DH domains is responsible for insertion of the cis double bonds in the PUFA PKS products.

The second domain in OrfC is a DH domain, also referred to herein as OrfC-DH2. This is the second of two DH domains in OrfC, and therefore is designated DH2. This domain is contained within the nucleotide sequence spanning from a starting point of between about positions 1351 and 2437 of SEQ ID NO:5 (OrfC) to an ending point of between about positions 2607 and 2850 of SEQ ID NO:5. The nucleotide sequence containing the sequence encoding the OrfC-DH2 domain is represented herein as SEQ ID NO:29 (positions 1351-2850 of SEQ ID NO:5). The amino acid sequence containing the DH2 domain spans from a starting point of between about positions 451 and 813 of SEQ ID NO:6 (OrfC) to an ending point of between about positions 869 and 950 of SEQ ID NO:6. The amino acid sequence containing the OrfC-DH2 domain is represented herein as SEQ ID NO:30 (positions 451-950 of SEQ ID NO:6). DH biological activity has been described above.

The third domain in OrfC is an ER domain, also referred to herein as OrfC-ER. This domain is contained within the nucleotide sequence spanning from a starting point of about position 2998 of SEQ ID NO:5 (OrfC) to an ending point of about position 4509 of SEQ ID NO:5. The nucleotide sequence containing the sequence encoding the OrfC-ER domain is represented herein as SEQ ID NO:31 (positions 2998-4509 of SEQ ID NO:5). The amino acid sequence containing the ER domain spans from a starting point of about position 1000 of SEQ ID NO:6 (OrfC) to an ending point of about position 1502 of SEQ ID NO:6. The amino acid sequence containing the OrfC-ER domain is represented herein as SEQ ID NO:32 (positions 1000-1502 of SEQ ID NO:6). ER biological activity has been described above.

### Thraustochytrium 23B PUFA PKS

### Th. 23B Open Reading Frame A (OrfA):

The complete nucleotide sequence for *Th.* 23B OrfA is represented herein as SEQ ID NO:38. SEQ ID NO:38 encodes the following domains in *Th.* 23B OrfA: (a) one β-ketoacyl-ACP synthase (KS) domain; (b) one malonyl-CoA:ACP acyltransferase (MAT) domain; (c) eight acyl carrier protein (ACP) domains; and (d) one β-ketoacyl-ACP reductase (KR) domain. This domain organization is the same as is present in *Schizochytrium* Orf A (SEQ ID NO:1) with the exception that the *Th.* 23B Orf A has 8 adjacent ACP domains, while *Schizochytrium* Orf A has 9 adjacent ACP domains. *Th.* 23B OrfA is a 8433 nucleotide sequence (not including the stop codon) which encodes a 2811 amino acid sequence, represented herein as SEQ ID NO:39. The *Th.* 23B OrfA amino acid sequence (SEQ ID NO:39) was compared with known sequences in a standard BLAST search (BLAST parameters: Blastp, low complexity filter Off, program - BLOSUM62,Gap cost - Existence: 11, Extension 1; (BLAST described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402, incorporated herein by reference in its entirety))). At the amino acid level, the sequences with the greatest degree of homology to *Th.* 23B OrfA was *Schizochytrium* Orf A (gb AAK72879.1) (SEQ ID NO:2). The alignment extends over the entire query but is broken into 2 pieces (due to the difference in numbers of ACP repeats). SEQ ID NO:39 first aligns at positions 6 through 1985 (including 8 ACP domains) with SEQ ID NO:2 and shows a sequence identity to SEQ ID NO:2 of 54% over 2017 amino acids. SEQ ID NO:39 also aligns at positions 980 through 2811 with SEQ ID NO:2 and shows a sequence identity to SEQ ID NO:2 of 43% over 1861 amino acids. In this second alignment, the match is evident for the *Th.* 23B 8X ACPs in the regions of the conserved pantetheine attachment site motif, but is very poor over the 1 st *Schizochytrium* ACP domain (i.e., there is not a 9^{th} ACP domain in the *Th.* 23B query sequence, but the Blastp output under theses conditions attempts to align them anyway). SEQ ID NO:39 shows the next closest identity with sequences from *Shewanella oneidensis* (Accession No. NP_717214) and *Photobacter profundum* (Accession No. AAL01060).

The first domain in *Th.* 23B OrfA is a KS domain, also referred to herein as *Th.* 23B OrfA-KS. KS domain function has been described in detail above. This domain is contained within the nucleotide sequence spanning from about position 1 to about position 1500 of SEQ ID NO:38, represented herein as SEQ ID NO:40. The amino acid sequence containing the *Th.* 23B KS domain is a region of SEQ ID NO:39 spanning from about position 1 to about position 500 of SEQ ID NO:39, represented herein as SEQ ID NO:41. This region of SEQ ID NO:39 has a Pfam match to FabB (β-ketoacyl-ACP synthase) spanning from position 1 to about position 450 of SEQ ID NO:39 (also positions 1 to about 450 of SEQ ID NO:41). It is noted that the *Th.* 23B OrfA-KS domain contains an active site motif: DXAC* (*acyl binding site C₂₀₇). Also, a characteristic motif at the end of the *Th.* 23B KS region, GFGG, is present in positions 453-456 of SEQ ID NO:39 (also positions 453-456 of SEQ ID NO:41). The amino acid sequence spanning positions 1-500 of SEQ ID NO:39 is about 79% identical to *Schizochytrium* OrfA (SEQ ID NO:2) over 496 amino acids. The amino acid sequence spanning positions 1-450 of SEQ ID NO:39 is about 81% identical to *Schizochytrium* OrfA (SEQ ID NO:2) over 446 amino acids.

The second domain in *Th.* 23B OrfA is a MAT domain, also referred to herein as *Th.* 23B OrfA-MAT. MAT domain function has been described in detail above. This domain is contained within the nucleotide sequence spanning from between about position 1503 and about position 3000 of SEQ ID NO:38, represented herein as SEQ ID NO:42. The amino acid sequence containing the *Th.* 23B MAT domain is a region of SEQ ID NO:39 spanning from about position 501 to about position 1000, represented herein by SEQ ID NO:43. This region of SEQ ID NO:39 has a Pfam match to FabD (malonyl-CoA:ACP acyltransferase) spanning from about position 580 to about position 900 of SEQ ID NO:39 (positions 80-400 of SEQ ID NO:43). It is noted that the *Th.* 23B OrfA-MAT domain contains an active site motif: GHS*XG (*acyl binding site S₆₉₇), represented by positions 695-699 of SEQ ID NO:39. The amino acid sequence spanning positions 501-1000 of SEQ ID NO:39 is about 46% identical to *Schizochytrium* OrfA (SEQ ID NO:2) over 481 amino acids. The amino acid sequence spanning positions 580-900 of SEQ ID NO:39 is about 50% identical to *Schizochytrium* OrfA (SEQ ID NO:2) over 333 amino acids.

Domains 3-10 of *Th.* 23B OrfA are eight tandem ACP domains, also referred to herein as *Th.* 23B OrfA-ACP (the first domain in the sequence is OrfA-ACP1, the second domain is OrfA-ACP2, the third domain is OrfA-ACP3, etc.). The function of ACP domains has been described in detail above. The first *Th.* 23B ACP domain, *Th.* 23B OrfA-ACP1, is contained within the nucleotide sequence spanning from about position 3205 to about position 3555 of SEQ ID NO:38 (OrfA), represented herein as SEQ ID NO:44. The amino acid sequence containing the first *Th.* 23B ACP domain is a region of SEQ ID NO:39 spanning from about position 1069 to about position 1185 of SEQ ID NO:39, represented herein by SEQ ID NO:45. The amino acid sequence spanning positions 1069-1185 of SEQ ID NO:39 is about 65% identical to *Schizochytrium* OrfA (SEQ ID NO:2) over 85 amino acids. *Th.* 23B OrfA-ACP1 has a similar identity to any one of the nine ACP domains in *Schizochytrium* OrfA.

The eight ACP domains in *Th.* 23B OrfA are adjacent to one another and can be identified by the presence of the phosphopantetheine binding site motif, LGXDS* (represented by SEQ ID NO:46), wherein the S* is the phosphopantetheine attachment site. The amino acid position of each of the eight S* sites, with reference to SEQ ID NO:39, are 1128 (ACP1),1244 (ACP2), 1360 (ACP3), 1476 (ACP4), 1592 (ACP5), 1708 (ACP6),1824 (ACP7) and 1940 (ACP8). The nucleotide and amino acid sequences of all eight *Th.* 23B ACP domains are highly conserved and therefore, the sequence for each domain is not represented herein by an individual sequence identifier. However, based on the information disclosed herein, one of skill in the art can readily determine the sequence containing each of the other seven ACP domains in SEQ ID NO:38 and SEQ ID NO:39.

All eight *Th.* 23B ACP domains together span a region of *Th.* 23B OrfA of from about position 3205 to about postion 5994 of SEQ ID NO:38, which corresponds to amino acid positions of from about 1069 to about 1998 of SEQ ID NO:39. The nucleotide sequence for the entire ACP region containing all eight domains is represented herein as SEQ ID NO:47. SEQ ID NO:47 encodes an amino acid sequence represented herein by SEQ ID NO:48. SEQ ID NO:48 includes the linker segments between individual ACP domains. The repeat interval for the eight domains is approximately every 116 amino acids of SEQ ID NO:48, and each domain can be considered to consist of about 116 amino acids centered on the active site motif (described above). It is noted that the linker regions between the nine adjacent ACP domains in OrfA in *Schizochytrium* are highly enriched in proline and alanine residues, while the linker regions between the eight adjacent ACP domains in OrfA of *Thraustochytrium* are highly enriched in serine residues (and not proline or alanine residues).

The last domain in *Th.* 23B OrfA is a KR domain, also referred to herein as *Th.* 23B OrfA-KR. KR domain function has been discussed in detail above. This domain is contained within the nucleotide sequence spanning from between about position 6001 to about position 8433 of SEQ ID NO:38, represented herein by SEQ ID NO:49. The amino acid sequence containing the *Th.* 23B KR domain is a region of SEQ ID NO:39 spanning from about position 2001 to about position 2811 of SEQ ID NO:39, represented herein by SEQ ID NO:50. This region of SEQ ID NO:39 has a Pfam match to FabG (β-ketoacyl-ACP reductase) spanning from about position 2300 to about 2550 of SEQ ID NO:39 (positions 300-550 of SEQ ID NO:50). The amino acid sequence spanning positions 2001-2811 of SEQ ID NO:39 is about 40% identical to *Schizochytrium* OrfA (SEQ ID NO:2) over 831 amino acids. The amino acid sequence spanning positions 2300-2550 of SEQ ID NO:39 is about 51% identical to *Schizochytrium* OrfA (SEQ ID NO:2) over 235 amino acids.

### Th. 23B Open Reading Frame B (OrfB):

The complete nucleotide sequence for *Th.* 23B OrfB is represented herein as SEQ ID NO:51. SEQ ID NO:51 encodes the following domains in *Th.* 23B OrfB: (a) one β-ketoacyl-ACP synthase (KS) domain; (b) one chain length factor (CLF) domain; (c) one acyltransferase (AT) domain; and, (d) one enoyl-ACP reductase (ER) domain. This domain organization is the same as in *Schizochytrium* Orf B (SEQ ID NO:3) with the exception that the linker region between the AT and ER domains of the *Schizochytrium* protein is longer than that of *Th.* 23B by about 50-60 amino acids. Also, this linker region in *Schizochytrium* has a specific area that is highly enriched in serine residues (it contains 15 adjacent serine residues, in addition to other serines in the region), whereas the corresponding linker region in *Th.* 23B OrfB is not enriched in serine residues. This difference in the AT/ER linker region most likely accounts for a break in the alignment between *Schizochytrium* OrfB and *Th.* 23B OrfB at the start of this region.

*Th.* 23B OrfB is a 5805 nucleotide sequence (not including the stop codon) which encodes a 1935 amino acid sequence, represented herein as SEQ ID NO:52. The *Th.* 23B OrfB amino acid sequence (SEQ ID NO:52) was compared with known sequences in a standard BLAST search (BLAST parameters: Blastp, low complexity filter Off, program - BLOSUM62,Gap cost - Existence: 11, Extension 1; (BLAST described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402, incorporated herein by reference in its entirety))). At the amino acid level, the sequences with the greatest degree of homology to *Th.* 23B OrfB were *Schizochytrium* Orf B (gb AAK72880.1) (SEQ ID NO:4), over most of OrfB; and *Schizochytrium* OrfC (gb AAK728881.1) (SEQ ID NO:6), over the last domain (the alignment is broken into 2 pieces, as mentioned above). SEQ ID NO:52 first aligns at positions 10 through about 1479 (including the KS, CLF and AT domains) with SEQ ID NO:4 and shows a sequence identity to SEQ ID NO:4 of 52% over 1483 amino acids. SEQ ID NO:52 also aligns at positions 1491 through 1935 (including the ER domain) with SEQ ID NO:6 and shows a sequence identity to SEQ ID NO:4 of 64% over 448 amino acids.

The first domain in the *Th.* 23B OrfB is a KS domain, also referred to herein as *Th.* 23B OrfB-KS. KS domain function has been described in detail above. This domain is contained within the nucleotide sequence spanning from between about position 1 and about postion 1500 of SEQ ID NO:51 (*Th.* 23B OrfB), represented herein as SEQ ID NO:53. The amino acid sequence containing the *Th.* 23B KS domain is a region of SEQ ID NO: 52 spanning from about position 1 to about position 500 of SEQ ID NO:52, represented herein as SEQ ID NO:54. This region of SEQ ID NO:52 has a Pfam match to FabB (β-ketoacyl-ACP synthase) spanning from about position 1 to about position 450 (positions 1-450 of SEQ ID NO:54). It is noted that the *Th.* 23B OrfB-KS domain contains an active site motif: DXAC*, where C* is the site of acyl group attachment and wherein the C* is at position 201 of SEQ ID NO:52. Also, a characteristic motif at the end of the KS region, GFGG is present in amino acid positions 434-437 of SEQ ID NO:52. The amino acid sequence spanning positions 1-500 of SEQ ID NO:52 is about 64% identical to *Schizochytrium* OrfB (SEQ ID NO:4) over 500 amino acids. The amino acid sequence spanning positions 1-450 of SEQ ID NO:52 is about 67% identical to *Schizochytrium* OrfB (SEQ ID NO:4) over 442 amino acids.

The second domain in *Th.* 23B OrfB is a CLF domain, also referred to herein as *Th.* 23B OrfB-CLF. CLF domain function has been described in detail above. This domain is contained within the nucleotide sequence spanning from between about position 1501 and about position 3000 of SEQ ID NO:51 (OrfB), represented herein as SEQ ID NO:55. The amino acid sequence containing the CLF domain is a region of SEQ ID NO: 52 spanning from about position 501 to about position 1000 of SEQ ID NO:52, represented herein as SEQ ID NO:56. This region of SEQ ID NO:52 has a Pfam match to FabB (β-ketoacyl-ACP synthase) spanning from about position 550 to about position 910 (positions 50-410 of SEQ ID NO:56). Although CLF has homology to KS proteins, it lacks an active site cysteine to which the acyl group is attached in KS proteins. The amino acid sequence spanning positions 501-1000 of SEQ ID NO:52 is about 49% identical to *Schizochytrium* OrfB (SEQ ID NO:4) over 517 amino acids. The amino acid sequence spanning positions 550-910 of SEQ ID NO:52 is about 54% identical to *Schizochytrium* OrfB (SEQ ID NO:4) over 360 amino acids.

The third domain in *Th.* 23B OrfB is an AT domain, also referred to herein as *Th.* 23B OrfB-AT. AT domain function has been described in detail above. This domain is contained within the nucleotide sequence spanning from between about position 3001 and about position 4500 of SEQ ID NO:51 (*Th.* 23B OrfB), represented herein as SEQ ID NO:58. The amino acid sequence containing the *Th.* 23B AT domain is a region of SEQ ID NO: 52 spanning from about position 1001 to about position 1500 of SEQ ID NO:52, represented herein as SEQ ID NO:58. This region of SEQ ID NO:52 has a Pfam match to FabD (malonyl-CoA:ACP acyltransferase) spanning from about position 1100 to about position 1375 (positions 100-375 of SEQ ID NO:58). Although this AT domain of the PUFA synthases has homology to MAT proteins, it lacks the extended motif of the MAT (key arginine and glutamine residues) and it is not thought to be involved in malonyl-CoA transfers. The GXS*XG motif of acyltransferases is present, with the S* being the site of acyl attachment and located at position 1123 with respect to SEQ ID NO:52. The amino acid sequence spanning positions 1001-1500 of SEQ ID NO:52 is about 44% identical to *Schizochytrium* OrfB (SEQ ID NO:4) over 459 amino acids. The amino acid sequence spanning positions 1100-1375 of SEQ ID NO:52 is about 45% identical to *Schizochytrium* OrfB (SEQ ID NO:4) over 283 amino acids.

The fourth domain in *Th.* 23B OrfB is an ER domain, also referred to herein as *Th.* 23B OrfB-ER. ER domain function has been described in detail above. This domain is contained within the nucleotide sequence spanning from between about position 4501 and about position 5805 of SEQ ID NO:51 (OrfB), represented herein as SEQ ID NO:59. The amino acid sequence containing the *Th.* 23B ER domain is a region of SEQ ID NO: 52 spanning from about position 1501 to about position 1935 of SEQ ID NO:52, represented herein as SEQ ID NO:60. This region of SEQ ID NO:52 has a Pfam match to a family of dioxygenases related to 2-nitropropane dioxygenases spanning from about position 1501 to about position 1810 (positions 1-310 of SEQ ID NO:60). That this domain functions as an ER can be further predicted due to homology to a newly characterized ER enzyme from *Streptococcus pneumoniae.* The amino acid sequence spanning positions 1501-1935 of SEQ ID NO:52 is about 66% identical to *Schizochytrium* OrfB (SEQ ID NO:4) over 433 amino acids. The amino acid sequence spanning positions 1501-1810 of SEQ ID NO:52 is about 70% identical to *Schizochytrium* OrfB (SEQ ID NO:4) over 305 amino acids.

### Th. 23B Open Reading Frame C (OrfC):

The complete nucleotide sequence for *Th.* 23B OrfC is represented herein as SEQ ID NO:61. SEQ ID NO:61 encodes the following domains in *Th.* 23B OrfC: (a) two FabA-like β-hydroxyacyl-ACP dehydrase (DH) domains, both with homology to the FabA protein (an enzyme that catalyzes the synthesis of *trans*-2-decenoyl-ACP and the reversible isomerization of this product to *cis*-3-decenoyl-ACP); and (b) one enoyl-ACP reductase (ER) domain with high homology to the ER domain of *Schizochytrium* OrfB. This domain organization is the same as in *Schizochytrium* Orf C (SEQ ID NO:5).

*Th.* 23B OrfC is a 4410 nucleotide sequence (not including the stop codon) which encodes a 1470 amino acid sequence, represented herein as SEQ ID NO:62. The *Th.* 23B OrfC amino acid sequence (SEQ ID NO:62) was compared with known sequences in a standard BLAST search (BLAST parameters: Blastp, low complexity filter Off, program - BLOSUM62,Gap cost - Existence: 11, Extension 1; (BLAST described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402, incorporated herein by reference in its entirety))). At the amino acid level, the sequences with the greatest degree of homology to *Th.* 23B OrfC was *Schizochytrium* OrfC (gb AAK728881.1) (SEQ ID NO:6). SEQ ID NO:52 is 66% identical to *Schizochytrium* OrfC (SEQ ID NO:6).

The first domain in *Th.* 23B OrfC is a DH domain, also referred to herein as *Th.* 23B OrfC-DH1. DH domain function has been described in detail above. This domain is contained within the nucleotide sequence spanning from between about position 1 to about position 1500 of SEQ ID NO:61 (OrfC), represented herein as SEQ ID NO:63. The amino acid sequence containing the *Th.* 23B DH1 domain is a region of SEQ ID NO: 62 spanning from about position 1 to about position 500 of SEQ ID NO:62, represented herein as SEQ ID NO:64. This region of SEQ ID NO:62 has a Pfam match to FabA, as mentioned above, spanning from about position 275 to about position 400 (positions 275-400 of SEQ ID NO:64). The amino acid sequence spanning positions 1-500 of SEQ ID NO:62 is about 66% identical to *Schizochytrium* OrfC (SEQ ID NO:6) over 526 amino acids. The amino acid sequence spanning positions 275-400 of SEQ ID NO:62 is about 81% identical to *Schizochytrium* OrfC (SEQ ID NO:6) over 126 amino acids.

The second domain in *Th.* 23B OrfC is also a DH domain, also referred to herein as *Th.* 23B OrfC-DH2. This is the second of two DH domains in OrfC, and therefore is designated DH2. This domain is contained within the nucleotide sequence spanning from between about position 1501 to about 3000 of SEQ ID NO:61 (OrfC), represented herein as SEQ ID NO:65. The amino acid sequence containing the *Th.* 23B DH2 domain is a region of SEQ ID NO: 62 spanning from about position 501 to about position 1000 of SEQ ID NO:62, represented herein as SEQ ID NO:66. This region of SEQ ID NO:62 has a Pfam match to FabA, as mentioned above, spanning from about position 800 to about position 925 (positions 300-425 of SEQ ID NO:66). The amino acid sequence spanning positions 501-1000 of SEQ ID NO:62 is about 56% identical to *Schizochytrium* OrfC (SEQ ID NO:6) over 518 amino acids. The amino acid sequence spanning positions 800-925 of SEQ ID NO:62 is about 58% identical to *Schizochytrium* OrfC (SEQ ID NO:6) over 124 amino acids.

The third domain in *Th.* 23B OrfC is an ER domain, also referred to herein as *Th.* 23B OrfC-ER. ER domain function has been described in detail above. This domain is contained within the nucleotide sequence spanning from between about position 3001 to about position 4410 of SEQ ID NO:61 (OrfC), represented herein as SEQ ID NO:67. The amino acid sequence containing the *Th.* 23B ER domain is a region of SEQ ID NO: 62 spanning from about position 1001 to about position 1470 of SEQ ID NO:62, represented herein as SEQ ID NO:68. This region of SEQ ID NO:62 has a Pfam match to the dioxygenases related to 2-nitropropane dioxygenases, as mentioned above, spanning from about position 1025 to about position 1320 (positions 25-320 of SEQ ID NO:68). This domain function as an ER can also be predicted due to homology to a newly characterized ER enzyme from *Streptococcus pneumoniae.* The amino acid sequence spanning positions 1001-1470 of SEQ ID NO:62 is about 75% identical to *Schizochytrium* OrfB (SEQ ID NO:4) over 474 amino acids. The amino acid sequence spanning positions 1025-1320 of SEQ ID NO:62 is about 81 % identical to *Schizochytrium* OrfB (SEQ ID NO:4) over 296 amino acids.

One embodiment of the present invention relates to an isolated protein or domain from a non-bacterial PUFA PKS system, a homologue thereof, and/or a fragment thereof. Also included in the invention are isolated nucleic acid molecules encoding any of the proteins, domains or peptides described herein (discussed in detail below). According to the present invention, an isolated protein or peptide, such as a protein or peptide from a PUFA PKS system, is a protein or a fragment thereof (including a polypeptide or peptide) that has been removed from its natural milieu (i.e., that has been subject to human manipulation) and can include purified proteins, partially purified proteins, recombinantly produced proteins, and synthetically produced proteins, for example. As such, "isolated" does not reflect the extent to which the protein has been purified. Preferably, an isolated protein of the present invention is produced recombinantly. An isolated peptide can be produced synthetically (e.g., chemically, such as by peptide synthesis) or recombinantly. In addition, and by way of example, a "*Thraustochytrium* PUFA PKS protein" refers to a PUFA PKS protein (generally including a homologue of a naturally occurring PUFA PKS protein) from a *Thraustochytrium* microorganism, or to a PUFA PKS protein that has been otherwise produced from the knowledge of the structure (e.g., sequence), and perhaps the function, of a naturally occurring PUFA PKS protein from *Thraustochytrium.* In other words, general reference to a *Thraustochytrium* PUFA PKS protein includes any PUFA PKS protein that has substantially similar structure and function of a naturally occurring PUFA PKS protein from *Thraustochytrium* or that is a biologically active (i.e., has biological activity) homologue of a naturally occurring PUFA PKS protein from *Thraustochytrium* as described in detail herein. As such, a *Thraustochytrium* PUFA PKS protein can include purified, partially purified, recombinant, mutated/modified and synthetic proteins. The same description applies to reference to other proteins or peptides described herein, such as the PUFA PKS proteins and domains from *Schizochytrium* or from other microorganisms.

According to the present invention, the terms "modification" and "mutation" can be used interchangeably, particularly with regard to the modifications/mutations to the primary amino acid sequences of a protein or peptide (or nucleic acid sequences) described herein. The term "modification" can also be used to describe post-translational modifications to a protein or peptide including, but not limited to, methylation, farnesylation, carboxymethylation, geranyl geranylation, glycosylation, phosphorylation, acetylation, myristoylation, prenylation, palmitation, and/or amidation. Modifications can also include, for example, complexing a protein or peptide with another compound. Such modifications can be considered to be mutations, for example, if the modification is different than the post-translational modification that occurs in the natural, wild-type protein or peptide.

As used herein, the term "homologue" is used to refer to a protein or peptide which differs from a naturally occurring protein or peptide (i.e., the "prototype" or "wild-type" protein) by one or more minor modifications or mutations to the naturally occurring protein or peptide, but which maintains the overall basic protein and side chain structure of the naturally occurring form (i.e., such that the homologue is identifiable as being related to the wild-type protein). Such changes include, but are not limited to: changes in one or a few amino acid side chains; changes one or a few amino acids, including deletions (e.g., a truncated version of the protein or peptide) insertions and/or substitutions; changes in stereochemistry of one or a few atoms; and/or minor derivatizations, including but not limited to: methylation, famesylation, geranyl geranylation, glycosylation, carboxymethylation, phosphorylation, acetylation, myristoylation, prenylation, palmitation, and/or amidation. A homologue can have either enhanced, decreased, or substantially similar properties as compared to the naturally occurring protein or peptide. Preferred homologues of a PUFA PKS protein or domain are described in detail below. It is noted that homologues can include synthetically produced homologues, naturally occurring allelic variants of a given protein or domain, or homologous sequences from organisms other than the organism from which the reference sequence was derived.

Conservative substitutions typically include substitutions within the following groups: glycine and alanine; valine, isoleucine and leucine; aspartic acid, glutamic acid, asparagine, and glutamine; serine and threonine; lysine and arginine; and phenylalanine and tyrosine. Substitutions may also be made on the basis of conserved hydrophobicity or hydrophilicity (Kyte and Doolittle, J. Mol. Biol. (1982) 157: 105-132), or on the basis of the ability to assume similar polypeptide secondary structure (Chou and Fasman, Adv. Enzymol. (1978) 47: 45-148, 1978).

Homologues can be the result of natural allelic variation or natural mutation. A naturally occurring allelic variant of a nucleic acid encoding a protein is a gene that occurs at essentially the same locus (or loci) in the genome as the gene which encodes such protein, but which, due to natural variations caused by, for example, mutation or recombination, has a similar but not identical sequence. Allelic variants typically encode proteins having similar activity to that of the protein encoded by the gene to which they are being compared. One class of allelic variants can encode the same protein but have different nucleic acid sequences due to the degeneracy of the genetic code. Allelic variants can also comprise alterations in the 5' or 3' untranslated regions of the gene (e.g., in regulatory control regions). Allelic variants are well known to those skilled in the art.

Homologues can be produced using techniques known in the art for the production of proteins including, but not limited to, direct modifications to the isolated, naturally occurring protein, direct protein synthesis, or modifications to the nucleic acid sequence encoding the protein using, for example, classic or recombinant DNA techniques to effect random or targeted mutagenesis.

Modifications or mutations in protein homologues, as compared to the wild-type protein, either increase, decrease, or do not substantially change, the basic biological activity of the homologue as compared to the naturally occurring (wild-type) protein. In general, the biological activity or biological action of a protein refers to any function(s) exhibited or performed by the protein that is ascribed to the naturally occurring form of the protein as measured or observed in *vivo* (i.e., in the natural physiological environment of the protein) or *in vitro* (i.e., under laboratory conditions). Biological activities of PUFA PKS systems and the individual proteins/domains that make up a PUFA PKS system have been described in detail elsewhere herein. Modifications of a protein, such as in a homologue or mimetic (discussed below), may result in proteins having the same biological activity as the naturally occurring protein, or in proteins having decreased or increased biological activity as compared to the naturally occurring protein. Modifications which result in a decrease in protein expression or a decrease in the activity of the protein, can be referred to as inactivation (complete or partial), down-regulation, or decreased action (or activity) of a protein. Similarly, modifications which result in an increase in protein expression or an increase in the activity of the protein, can be referred to as amplification, overproduction, activation, enhancement, up-regulation or increased action (or activity) of a protein. It is noted that general reference to a homologue having the biological activity of the wild-type protein does not necessarily mean that the homologue has identical biological activity as the wild-type protein, particularly with regard to the level of biological activity. Rather, a homologue can perform the same biological activity as the wild-type protein, but at a reduced or increased level of activity as compared to the wild-type protein. A functional domain of a PUFA PKS system is a domain (i.e., a domain can be a portion of a protein) that is capable of performing a biological function (i.e., has biological activity).

Methods of detecting and measuring PUFA PKS protein or domain biological activity include, but are not limited to, measurement of transcription of a PUFA PKS protein or domain, measurement of translation of a PUFA PKS protein or domain, measurement of posttranslational modification of a PUFA PKS protein or domain, measurement of enzymatic activity of a PUFA PKS protein or domain, and/or measurement production of one or more products of a PUFA PKS system (e.g., PUFA production). It is noted that an isolated protein of the present invention (including a homologue) is not necessarily required to have the biological activity of the wild-type protein. For example, a PUFA PKS protein or domain can be a truncated, mutated or inactive protein, for example. Such proteins are useful in screening assays, for example, or for other purposes such as antibody production. In a preferred embodiment, the isolated proteins of the present invention have biological activity that is similar to that of the wild-type protein (although not necessarily equivalent, as discussed above).

Methods to measure protein expression levels generally include, but are not limited to: Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorption/ionization time-of-flight (MALDI-TOF) mass spectrometry, microcytometry, microarray, microscopy, fluorescence activated cell sorting (FACS), and flow cytometry, as well as assays based on a property of the protein including but not limited to enzymatic activity or interaction with other protein partners. Binding assays are also well known in the art. For example, a BIAcore machine can be used to determine the binding constant of a complex between two proteins. The dissociation constant for the complex can be determined by monitoring changes in the refractive index with respect to time as buffer is passed over the chip (O'Shannessy et al. Anal. Biochem. 212:457-468 (1993); Schuster et al., Nature 365:343-347 (1993)). Other suitable assays for measuring the binding of one protein to another include, for example, immunoassays such as enzyme linked immunoabsorbent assays (ELISA) and radioimmunoassays (RIA); or determination of binding by monitoring the change in the spectroscopic or optical properties of the proteins through fluorescence, UV absorption, circular dichrosim, or nuclear magnetic resonance (NMR).

In one embodiment, the present invention relates to an isolated protein comprising an amino acid sequence selected from the group consisting of: (a) an amino acid sequence selected from the group consisting of: SEQ ID NO:39, SEQ ID NO:52, SEQ ID NO:62, and biologically active fragments thereof; (b) an amino acid sequence selected from the group consisting of: SEQ ID NO:41, SEQ ID NO:43, SEQ ID NO:45, SEQ ID NO:48, SEQ ID NO:50, SEQ ID NO:54, SEQ ID NO:56, SEQ ID NO:58, SEQ ID NO:60, SEQ ID NO:64, SEQ ID NO:66, SEQ ID NO:68 and biologically active fragments thereof; (c) an amino acid sequence that is at least about 60% identical to at least 500 consecutive amino acids of the amino acid sequence of (a), wherein the amino acid sequence has a biological activity of at least one domain of a polyunsaturated fatty acid (PUFA) polyketide synthase (PKS) system; and/or (d) an amino acid sequence that is at least about 60% identical to the amino acid sequence of (b), wherein the amino acid sequence has a biological activity of at least one domain of a polyunsaturated fatty acid (PUFA) polyketide synthase (PKS) system. In a further embodiment, an amino acid sequence including the active site domains or other functional motifs described above for several of the PUFA PKS domains are encompassed by the invention. In one embodiment, the amino acid sequence described above does not include any of the following amino acid sequences: SEQ ID NO:2, SEQ ID NO:4, SEQ ID NO:6, SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32.

In one aspect of the invention, a PUFA PKS protein or domain encompassed by the present invention, including a homologue of a particular PUFA PKS protein or domain described herein, comprises an amino acid sequence that is at least about 60% identical to at least 500 consecutive amino acids of an amino acid sequence chosen from: SEQ ID NO:39, SEQ ID NO:52, or SEQ ID NO:62, wherein the amino acid sequence has a biological activity of at least one domain of a PUFA PKS system. In a further aspect, the amino acid sequence of the protein is at least about 60% identical to at least about 600 consecutive amino acids, and more preferably to at least about 700 consecutive amino acids, and more preferably to at least about 800 consecutive amino acids, and more preferably to at least about 900 consecutive amino acids, and more preferably to at least about 1000 consecutive amino acids, and more preferably to at least about 1100 consecutive amino acids, and more preferably to at least about 1200 consecutive amino acids, and more preferably to at least about 1300 consecutive amino acids, and more preferably to at least about 1400 consecutive amino acids of any of SEQ ID NO:39, SEQ ID NO:52, or SEQ ID NO:62, or to the full length of SEQ ID NO:62. In a further aspect, the amino acid sequence of the protein is at least about 60% identical to at least about 1500 consecutive amino acids, and more preferably to at least about 1600 consecutive amino acids, and more preferably to at least about 1700 consecutive amino acids, and more preferably to at least about 1800 consecutive amino acids, and more preferably to at least about 1900 consecutive amino acids, of any of SEQ ID NO:39 or SEQ ID NO:52, or to the full length of SEQ ID NO:52. In a further aspect, the amino acid sequence of the protein is at least about 60% identical to at least about 2000 consecutive amino acids, and more preferably to at least about 2100 consecutive amino acids, and more preferably to at least about 2200 consecutive amino acids, and more preferably to at least about 2300 consecutive amino acids, and more preferably to at least about 2400 consecutive amino acids, and more preferably to at least about 2500 consecutive amino acids, and more preferably to at least about 2600 consecutive amino acids, and more preferably to at least about 2700 consecutive amino acids, and more preferably to at least about 2800 consecutive amino acids, and even more preferably, to the full length of SEQ ID NO:39. In one embodiment, the amino acid sequence described above does not include any of the following amino acid sequences: SEQ ID NO:2, SEQ ID NO:4, SEQ ID NO:6, SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32.

In another aspect, a PUFA PKS protein or domain encompassed by the present invention, including homologues as described above, comprises an amino acid sequence that is at least about 65% identical, and more preferably at least about 70% identical, and more preferably at least about 75% identical, and more preferably at least about 80% identical, and more preferably at least about 85% identical, and more preferably at least about 90% identical, and more preferably at least about 95% identical, and more preferably at least about 96% identical, and more preferably at least about 97% identical, and more preferably at least about 98% identical, and more preferably at least about 99% identical to an amino acid sequence chosen from: SEQ ID NO:39, SEQ ID NO:52, or SEQ ID NO:62, over any of the consecutive amino acid lengths described in the paragraph above, wherein the amino acid sequence has a biological activity of at least one domain of a PUFA PKS system. In one embodiment, the amino acid sequence described above does not include any of the following amino acid sequences: SEQ ID NO:2, SEQ ID NO:4, SEQ ID NO:6, SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32.

In one aspect of the invention, a PUFA PKS protein or domain encompassed by the present invention, including a homologue as described above, comprises an amino acid sequence that is at least about 60% identical to an amino acid sequence chosen from: SEQ ID NO:39, SEQ ID NO:41, SEQ ID NO:43, SEQ ID NO:45, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, wherein the amino acid sequence has a biological activity of at least one domain of a PUFA PKS system. In a further aspect, the amino acid sequence of the protein is at least about 65% identical, and more preferably at least about 70% identical, and more preferably at least about 75% identical, and more preferably at least about 80% identical, and more preferably at least about 85% identical, and more preferably at least about 90% identical, and more preferably at least about 95% identical, and more preferably at least about 96% identical, and more preferably at least about 97% identical, and more preferably at least about 98% identical, and more preferably at least about 99% identical to an amino acid sequence chosen from: SEQ ID NO:39, SEQ ID NO:41, SEQ ID NO:43, SEQ ID NO:45, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, wherein the amino acid sequence has a biological activity of at least one domain of a PUFA PKS system. In one embodiment, the amino acid sequence described above does not include any of the following amino acid sequences: SEQ ID NO:2, SEQ ID NO:4, SEQ ID NO:6, SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32.

In another aspect, a PUFA PKS protein or domain encompassed by the present invention, including a homologue as described above, comprises an amino acid sequence that is at least about 50% identical to an amino acid sequence chosen from: SEQ ID NO:39, SEQ ID NO:43, SEQ ID NO:50, SEQ ID NO:52, and SEQ ID NO:58, wherein the amino acid sequence has a biological activity of at least one domain of a PUFA PKS system. In another aspect, the amino acid sequence of the protein is at least about 55% identical, and more preferably at least about 60% identical, to an amino acid sequence chosen from: SEQ ID NO:39, SEQ ID NO:43, SEQ ID NO:50, SEQ ID NO:52, SEQ ID NO:56 and SEQ ID NO:58, wherein the amino acid sequence has a biological activity of at least one domain of a PUFA PKS system. In a further aspect, the amino acid sequence of the protein is at least about 65% identical to an amino acid sequence chosen from SEQ ID NO:39, SEQ ID NO:43, SEQ ID NO:50, SEQ ID NO:52, SEQ ID NO:54, SEQ ID NO:56 and SEQ ID NO:58, wherein the amino acid sequence has a biological activity of at least one domain of a PUFA PKS system. In another aspect, the amino acid sequence of the protein is at least about 70% identical, and more preferably at least about 75% identical, to an amino acid sequence chosen from: SEQ ID NO:39, SEQ ID NO:43, SEQ ID NO:45, SEQ ID NO:48, SEQ ID NO:50, SEQ ID NO:52, SEQ ID NO:54, SEQ ID NO:56, SEQ ID NO:58, SEQ ID NO:60, SEQ ID NO:62, and SEQ ID NO:64, wherein the amino acid sequence has a biological activity of at least one domain of a PUFA PKS system. In another aspect, the amino acid sequence of the protein is at least about 80% identical, and more preferably at least about 85% identical, and more preferably at least about 90% identical, and more preferably at least about 95% identical, and more preferably at least about 96% identical, and more preferably at least about 97% identical, and more preferably at least about 98% identical, and more preferably at least about 99% identical, to an amino acid sequence chosen from: SEQ ID NO:39, SEQ ID NO:41, SEQ ID NO:43, SEQ ID NO:45, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, wherein the amino acid sequence has a biological activity of at least one domain of a PUFA PKS system. In one embodiment, the amino acid sequence described above does not include any of the following amino acid sequences: SEQ ID NO:2, SEQ ID NO:4, SEQ ID NO:6, SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32.

In a preferred embodiment an isolated protein or domain of the present invention comprises, consists essentially of, or consists of, an amino acid sequence chosen from: SEQ ID NO:39, SEQ ID NO:41, SEQ ID NO:43, SEQ ID NO:45, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, or anybiologically active fragments thereof, including any fragments that have a biological activity of at least one domain of a PUFA PKS system.

In one aspect of the present invention, the following *Schizochytrium* proteins and domains are useful in one or more embodiments of the present invention, all of which have been previously described in detail in U.S. Patent Application Serial No. 10/124,800, *supra.* In one aspect of the invention, a PUFA PKS protein or domain useful in the present invention comprises an amino acid sequence that is at least about 60% identical to at least 500 consecutive amino acids of an amino acid sequence chosen from: SEQ ID NO:2, SEQ ID NO:4, and SEQ ID NO:6; wherein the amino acid sequence has a biological activity of at least one domain of a PUFA PKS system. In a further aspect, the amino acid sequence of the protein is at least about 60% identical to at least about 600 consecutive amino acids, and more preferably to at least about 700 consecutive amino acids, and more preferably to at least about 800 consecutive amino acids, and more preferably to at least about 900 consecutive amino acids, and more preferably to at least about 1000 consecutive amino acids, and more preferably to at least about 1100 consecutive amino acids, and more preferably to at least about 1200 consecutive amino acids, and more preferably to at least about 1300 consecutive amino acids, and more preferably to at least about 1400 consecutive amino acids, and more preferably to at least about 1500 consecutive amino acids of any of SEQ ID NO:2, SEQ ID NO:4 and SEQ ID NO:6, or to the full length of SEQ ID NO:6. In a further aspect, the amino acid sequence of the protein is at least about 60% identical to at least about 1600 consecutive amino acids, and more preferably to at least about 1700 consecutive amino acids, and more preferably to at least about 1800 consecutive amino acids, and more preferably to at least about 1900 consecutive amino acids, and more preferably to at least about 2000 consecutive amino acids of any of SEQ ID NO:2 or SEQ ID NO:4, or to the full length of SEQ ID NO:4. In a further aspect, the amino acid sequence of the protein is at least about 60% identical to at least about 2100 consecutive amino acids, and more preferably to at least about 2200 consecutive amino acids, and more preferably to at least about 2300 consecutive amino acids, and more preferably to at least about 2400 consecutive amino acids, and more preferably to at least about 2500 consecutive amino acids, and more preferably to at least about 2600 consecutive amino acids, and more preferably to at least about 2700 consecutive amino acids, and more preferably to at least about 2800 consecutive amino acids, and even more preferably, to the full length of SEQ ID NO:2.

In another aspect, a PUFA PKS protein or domain useful in one or more embodiments of the present invention comprises an amino acid sequence that is at least about 65% identical, and more preferably at least about 70% identical, and more preferably at least about 75% identical, and more preferably at least about 80% identical, and more preferably at least about 85% identical, and more preferably at least about 90% identical, and more preferably at least about 95% identical, and more preferably at least about 96% identical, and more preferably at least about 97% identical, and more preferably at least about 98% identical, and more preferably at least about 99% identical to an amino acid sequence chosen from: SEQ ID NO:2, SEQ ID NO:4, or SEQ ID NO:6, over any of the consecutive amino acid lengths described in the paragraph above, wherein the amino acid sequence has a biological activity of at least one domain of a PUFA PKS system.

In another aspect of the invention, a PUFA PKS protein or domain useful in one or more embodiments of the present invention comprises an amino acid sequence that is at least about 60% identical to an amino acid sequence chosen from: SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, or SEQ ID NO:32, wherein the amino acid sequence has a biological activity of at least one domain of a PUFA PKS system. In a further aspect, the amino acid sequence of the protein is at least about 65% identical, and more preferably at least about 70% identical, and more preferably at least about 75% identical, and more preferably at least about 80% identical, and more preferably at least about 85% identical, and more preferably at least about 90% identical, and more preferably at least about 95% identical, and more preferably at least about 96% identical, and more preferably at least about 97% identical, and more preferably at least about 98% identical, and more preferably at least about 99% identical to an amino acid sequence chosen from: SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32, wherein the amino acid sequence has a biological activity of at least one domain of a PUFA PKS system.

In yet another aspect of the invention, a PUFA PKS protein or domain useful in one or more embodiments of the present invention comprises, consists essentially of, or consists of, an amino acid sequence chosen from: SEQ ID NO:2, SEQ ID NO:4, SEQ ID NO:6, SEQ ID NO:8, SEQ ID NO:10, SEQ ID NO:13, SEQ ID NO:18, SEQ ID NO:20, SEQ ID NO:22, SEQ ID NO:24, SEQ ID NO:26, SEQ ID NO:28, SEQ ID NO:30, SEQ ID NO:32 or any biologically active fragments thereof, including any fragments that have a biological activity of at least one domain of a PUFA PKS system.

According to the present invention, the term "contiguous" or "consecutive", with regard to nucleic acid or amino acid sequences described herein, means to be connected in an unbroken sequence. For example, for a first sequence to comprise 30 contiguous (or consecutive) amino acids of a second sequence, means that the first sequence includes an unbroken sequence of 30 amino acid residues that is 100% identical to an unbroken sequence of 30 amino acid residues in the second sequence. Similarly, for a first sequence to have "100% identity" with a second sequence means that the first sequence exactly matches the second sequence with no gaps between nucleotides or amino acids.

As used herein, unless otherwise specified, reference to a percent (%) identity refers to an evaluation of homology which is performed using: (1) a BLAST 2.0 Basic BLAST homology search using blastp for amino acid searches, blastn for nucleic acid searches, and blastX for nucleic acid searches and searches of translated amino acids in all 6 open reading frames, all with standard default parameters, wherein the query sequence is filtered for low complexity regions by default (described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402, incorporated herein by reference in its entirety); (2) a BLAST 2 alignment (using the parameters described below); (3) and/or PSI-BLAST with the standard default parameters (Position-Specific Iterated BLAST). It is noted that due to some differences in the standard parameters between BLAST 2.0 Basic BLAST and BLAST 2, two specific sequences might be recognized as having significant homologyusing the BLAST 2 program, whereas a search performed in BLAST 2.0 Basic BLAST using one of the sequences as the query sequence may not identify the second sequence in the top matches. In addition, PSI-BLAST provides an automated, easy-to-use version of a "profile" search, which is a sensitive way to look for sequence homologues. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using any one of these programs.

Two specific sequences can be aligned to one another using BLAST 2 sequence as described in Tatusova and Madden, (1999), "Blast 2 sequences - a new tool for comparing protein and nucleotide sequences", FEMS Microbiol Lett. 174, 247, incorporated herein by reference in its entirety. BLAST 2 sequence alignment is performed in blastp or blastn using the BLAST 2.0 algorithm to perform a Gapped BLAST search (BLAST 2.0) between the two sequences allowing for the introduction of gaps (deletions and insertions) in the resulting alignment. For purposes of clarity herein, a BLAST 2 sequence alignment is performed using the standard default parameters as follows.
For blastn, using 0 BLOSUM62 matrix:
Reward for match = 1
Penalty for mismatch = -2
Open gap (5) and extension gap (2) penalties
gap x_dropoff (50) expect (10) word size (11) filter (on)
For blastp, using 0 BLOSUM62 matrix:
Open gap (11) and extension gap (1) penalties
gap x_dropoff (50) expect (10) word size (3) filter (on).

According to the present invention, an amino acid sequence that has a biological activity of at least one domain of a PUFA PKS system is an amino acid sequence that has the biological activity of at least one domain of the PUFA PKS system described in detail herein, as previously exemplified by the *Schizochytrium* PUFA PKS system or as additionally exemplified herein by the *Thraustochytrium* PUFA PKS system. The biological activities of the various domains within the *Schizochytrium* or *Thraustochytrium* PUFA PKS systems have been described in detail above. Therefore, an isolated protein useful in the present invention can include the translation product of any PUFA PKS open reading frame, any PUFA PKS domain, biologically active fragment thereof, or any homologue of a naturally occurring PUFA PKS open reading frame product or domain which has biological activity.

In another embodiment of the invention, an amino acid sequence having the biological activity of at least one domain of a PUFA PKS system of the present invention includes an amino acid sequence that is sufficiently similar to a naturally occurring PUFA PKS protein or polypeptide that a nucleic acid sequence encoding the amino acid sequence is capable of hybridizing under moderate, high, or very high stringency conditions (described below) to (i.e., with) a nucleic acid molecule encoding the naturally occurring PUFA PKS protein or polypeptide (i.e., to the complement of the nucleic acid strand encoding the naturally occurring PUFA PKS protein or polypeptide). Preferably, an amino acid sequence having the biological activity of at least one domain of a PUFA PKS system of the present invention is encoded by a nucleic acid sequence that hybridizes under moderate, high or very high stringency conditions to the complement of a nucleic acid sequence that encodes any of the above-described amino acid sequences for a PUFA PKS protein or domain. Methods to deduce a complementary sequence are known to those skilled in the art. It should be noted that since amino acid sequencing and nucleic acid sequencing technologies are not entirely error-free, the sequences presented herein, at best, represent apparent sequences of PUFA PKS domains and proteins of the present invention.

As used herein, hybridization conditions refer to standard hybridization conditions under which nucleic acid molecules are used to identify similar nucleic acid molecules. Such standard conditions are disclosed, for example, in Sambrook et al., Molecular Cloning: A Laboratory Manual, Cold Spring Harbor Labs Press, 1989. Sambrook et al., ibid., is incorporated by reference herein in its entirety (see specifically, pages 9.31-9.62). In addition, formulae to calculate the appropriate hybridization and wash conditions to achieve hybridization permitting varying degrees of mismatch of nucleotides are disclosed, for example, in Meinkoth et al., 1984, Anal. Biochem. 138, 267-284; Meinkoth et al., *ibid*., is incorporated by reference herein in its entirety.

More particularly, moderate stringency hybridization and washing conditions, as referred to herein, refer to conditions which permit isolation of nucleic acid molecules having at least about 70% nucleic acid sequence identity with the nucleic acid molecule being used to probe in the hybridization reaction (i.e., conditions permitting about 30% or less mismatch of nucleotides). High stringency hybridization and washing conditions, as referred to herein, refer to conditions which permit isolation of nucleic acid molecules having at least about 80% nucleic acid sequence identity with the nucleic acid molecule being used to probe in the hybridization reaction (i.e., conditions permitting about 20% or less mismatch of nucleotides). Very high stringency hybridization and washing conditions, as referred to herein, refer to conditions which permit isolation of nucleic acid molecules having at least about 90% nucleic acid sequence identity with the nucleic acid molecule being used to probe in the hybridization reaction (i.e., conditions permitting about 10% or less mismatch of nucleotides). As discussed above, one of skill in the art can use the formulae in Meinkoth et al., *ibid.* to calculate the appropriate hybridization and wash conditions to achieve these particular levels of nucleotide mismatch. Such conditions will vary, depending on whether DNA:RNA or DNA:DNA hybrids are being formed. Calculated melting temperatures for DNA:DNA hybrids are 10°C less than for DNA:RNA hybrids. In particular embodiments, stringent hybridization conditions for DNA:DNA hybrids include hybridization at an ionic strength of 6X SSC (0.9 M Na⁺) at a temperature of between about 20°C and about 35°C (lower stringency), more preferably, between about 28°C and about 40°C (more stringent), and even more preferably, between about 35°C and about 45°C (even more stringent), with appropriate wash conditions. In particular embodiments, stringent hybridization conditions for DNA:RNA hybrids include hybridization at an ionic strength of 6X SSC (0.9 M Na⁺) at a temperature of between about 30°C and about 45°C, more preferably, between about 38°C and about 50°C, and even more preferably, between about 45°C and about 55°C, with similarly stringent wash conditions. These values are based on calculations of a melting temperature for molecules larger than about 100 nucleotides, 0% formamide and a G + C content of about 40%. Alternatively, Tₘ can be calculated empirically as set forth in Sambrook et al., *supra,* pages 9.31 to 9.62. In general, the wash conditions should be as stringent as possible, and should be appropriate for the chosen hybridization conditions. For example, hybridization conditions can include a combination of salt and temperature conditions that are approximately 20-25°C below the calculated Tₘ of a particular hybrid, and wash conditions typically include a combination of salt and temperature conditions that are approximately 12-20°C below the calculated Tₘ of the particular hybrid. One example of hybridization conditions suitable for use with DNA:DNA hybrids includes a 2-24 hour hybridization in 6X SSC (50% formamide) at about 42°C, followed by washing steps that include one or more washes at room temperature in about 2X SSC, followed by additional washes at higher temperatures and lower ionic strength (e.g., at least one wash as about 37°C in about O.1X-0.5X SSC, followed by at least one wash at about 68°C in about 0.1X-0.5X SSC).

The present invention also includes a fusion protein that includes any PUFA PKS protein or domain or any homologue or fragment thereof attached to one or more fusion segments. Suitable fusion segments for use with the present invention include, but are not limited to, segments that can: enhance a protein's stability; provide other desirable biological activity; and/or assist with the purification of the protein (e.g., by affinity chromatography). A suitable fusion segment can be a domain of any size that has the desired function (e.g., imparts increased stability, solubility, biological activity; and/or simplifies purification of a protein). Fusion segments can be joined to amino and/or carboxyl termini of the protein and can be susceptible to cleavage in order to enable straight-forward recovery of the desired protein. Fusion proteins are preferably produced by culturing a recombinant cell transfected with a fusion nucleic acid molecule that encodes a protein including the fusion segment attached to either the carboxyl and/or amino terminal end of the protein of the invention as discussed above.

In one embodiment of the present invention, any of the above-described PUFA PKS amino acid sequences, as well as homologues of such sequences, can be produced with from at least one, and up to about 20, additional heterologous amino acids flanking each of the C-and/or N-terminal end of the given amino acid sequence. The resulting protein or polypeptide can be referred to as "consisting essentially of" a given amino acid sequence. According to the present invention, the heterologous amino acids are a sequence of amino acids that are not naturally found (i.e., not found in nature, *in vivo*) flanking the given amino acid sequence or which would not be encoded by the nucleotides that flank the naturally occurring nucleic acid sequence encoding the given amino acid sequence as it occurs in the gene, if such nucleotides in the naturally occurring sequence were translated using standard codon usage for the organism from which the given amino acid sequence is derived. Similarly, the phrase "consisting essentially of", when used with reference to a nucleic acid sequence herein, refers to a nucleic acid sequence encoding a given amino acid sequence that can be flanked by from at least one, and up to as many as about 60, additional heterologous nucleotides at each of the 5' and/or the 3' end of the nucleic acid sequence encoding the given amino acid sequence. The heterologous nucleotides are not naturally found (i.e., not found in nature, *in vivo*) flanking the nucleic acid sequence encoding the given amino acid sequence as it occurs in the natural gene.

The minimum size of a protein or domain and/or a homologue or fragment thereof of the present invention is, in one aspect, a size sufficient to have the requisite biological activity, or sufficient to serve as an antigen for the generation of an antibody or as a target in an *in vitro* assay. In one embodiment, a protein of the present invention is at least about 8 amino acids in length (e.g., suitable for an antibody epitope or as a detectable peptide in an assay), or at least about 25 amino acids in length, or at least about 50 amino acids in length, or at least about 100 amino acids in length, or at least about 150 amino acids in length, or at least about 200 amino acids in length, or at least about 250 amino acids in length, or at least about 300 amino acids in length, or at least about 350 amino acids in length, or at least about 400 amino acids in length, or at least about 450 amino acids in length, or at least about 500 amino acids in length, or at least about 750 amino acids in length, and so on, in any length between 8 amino acids and up to the full length of a protein or domain of the invention or longer, in whole integers (e.g., 8, 9, 10,...25, 26,...500, 501,...1234, 1235,...). There is no limit, other than a practical limit, on the maximum size of such a protein in that the protein can include a portion of a PUFA PKS protein, domain, or biologically active or useful fragment thereof, or a full-length PUFA PKS protein or domain, plus additional sequence (e.g., a fusion protein sequence), if desired.

Further embodiments of the present invention include isolated nucleic acid molecules comprising, consisting essentially of, or consisting of nucleic acid sequences that encode any of the above-identified proteins or domains, including a homologue or fragment thereof, as well as nucleic acid sequences that are fully complementary thereto. In accordance with the present invention, an isolated nucleic acid molecule is a nucleic acid molecule that has been removed from its natural milieu (i.e., that has been subject to human manipulation), its natural milieu being the genome or chromosome in which the nucleic acid molecule is found in nature. As such, "isolated" does not necessarily reflect the extent to which the nucleic acid molecule has been purified, but indicates that the molecule does not include an entire genome or an entire chromosome in which the nucleic acid molecule is found in nature. An isolated nucleic acid molecule can include a gene. An isolated nucleic acid molecule that includes a gene is not a fragment of a chromosome that includes such gene, but rather includes the coding region and regulatory regions associated with the gene, but no additional genes naturally found on the same chromosome. An isolated nucleic acid molecule can also include a specified nucleic acid sequence flanked by (i.e., at the 5' and/or the 3' end of the sequence) additional nucleic acids that do not normally flank the specified nucleic acid sequence in nature (i.e., heterologous sequences). Isolated nucleic acid molecule can include DNA, RNA (e.g., mRNA), or derivatives of either DNA or RNA (e.g., cDNA). Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding a protein or domain of a protein.

Preferably, an isolated nucleic acid molecule of the present invention is produced using recombinant DNA technology (e.g., polymerase chain reaction (PCR) amplification, cloning) or chemical synthesis. Isolated nucleic acid molecules include natural nucleic acid molecules and homologues thereof, including, but not limited to, natural allelic variants and modified nucleic acid molecules in which nucleotides have been inserted, deleted, substituted, and/or inverted in such a manner that such modifications provide the desired effect on PUFA PKS system biological activity as described herein. Protein homologues (e.g., proteins encoded by nucleic acid homologues) have been discussed in detail above.

A nucleic acid molecule homologue can be produced using a number of methods known to those skilled in the art (see, for example, Sambrook et al., Molecular Cloning: A Laboratory Manual, Cold Spring Harbor Labs Press, 1989). For example, nucleic acid molecules can be modified using a variety of techniques including, but not limited to, classic mutagenesis techniques and recombinant DNA techniques, such as site-directed mutagenesis, chemical treatment of a nucleic acid molecule to induce mutations, restriction enzyme cleavage of a nucleic acid fragment, ligation of nucleic acid fragments, PCR amplification and/or mutagenesis of selected regions of a nucleic acid sequence, synthesis of oligonucleotide mixtures and ligation of mixture groups to "build" a mixture of nucleic acid molecules and combinations thereof. Nucleic acid molecule homologues can be selected from a mixture of modified nucleic acids by screening for the function of the protein encoded by the nucleic acid and/or by hybridization with a wild-type gene.

The minimum size of a nucleic acid molecule of the present invention is a size sufficient to form a probe or oligonucleotide primer that is capable of forming a stable hybrid. (e.g., under moderate, high or very high stringency conditions) with the complementary sequence of a nucleic acid molecule useful in the present invention, or of a size sufficient to encode an amino acid sequence having a biological activity of at least one domain of a PUFA PKS system according to the present invention. As such, the size of the nucleic acid molecule encoding such a protein can be dependent on nucleic acid composition and percent homology or identity between the nucleic acid molecule and complementary sequence as well as upon hybridization conditions *per se* (e.g., temperature, salt concentration, and formamide concentration). The minimal size of a nucleic acid molecule that is used as an oligonucleotide primer or as a probe is typically at least about 12 to about 15 nucleotides in length if the nucleic acid molecules are GC-rich and at least about 15 to about 18 bases in length if they are AT-rich. There is no limit, other than a practical limit, on the maximal size of a nucleic acid molecule of the present invention, in that the nucleic acid molecule can include a sequence sufficient to encode a biologically active fragment of a domain of a PUFA PKS system, an entire domain of a PUFA PKS system, several domains within an open reading frame (Orf) of a PUFA PKS system, an entire Orf of a PUFA PKS system, or more than one Orf of a PUFA PKS system.

In one embodiment of the present invention, an isolated nucleic acid molecule comprises, consists essentially of, or consists of a nucleic acid sequence encoding any of the above-described amino acid sequences, including any of the amino acid sequences, or homologues thereof, from a *Schizochytrium* or *Thraustochytrium* described herein. In one aspect, the nucleic acid sequence is selected from the group of: SEQ ID NO:1, SEQ ID NO:3, SEQ ID NO:5, SEQ ID NO:7, SEQ ID NO:9, SEQ ID NO:12, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:38, SEQ ID NO:40, SEQ ID NO:42, SEQ ID NO:44, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, or SEQ ID NO:67, or homologues (including sequences that are at least about 50%, 55%, 60%, 65%, 70%, 75%, 80%, 85%, 90%, 95%, 96%, 97%, 98%, or 99% identical to such sequences), or fragments thereof, or any complementary sequences thereof.

Another embodiment of the present invention includes a recombinant nucleic acid molecule comprising a recombinant vector and a nucleic acid sequence encoding protein or peptide having a biological activity of at least one domain (or homologue or fragment thereof) of a PUFA PKS system as described herein. Such nucleic acid sequences are described in detail above. According to the present invention, a recombinant vector is an engineered (i.e., artificially produced) nucleic acid molecule that is used as a tool for manipulating a nucleic acid sequence of choice and for introducing such a nucleic acid sequence into a host cell. The recombinant vector is therefore suitable for use in cloning, sequencing, and/or otherwise manipulating the nucleic acid sequence of choice, such as by expressing and/or delivering the nucleic acid sequence of choice into a host cell to form a recombinant cell. Such a vector typically contains heterologous nucleic acid sequences, that is nucleic acid sequences that are not naturally found adjacent to nucleic acid sequence to be cloned or delivered, although the vector can also contain regulatory nucleic acid sequences (e.g., promoters, untranslated regions) which are naturally found adjacent to nucleic acid molecules of the present invention or which are useful for expression of the nucleic acid molecules of the present invention (discussed in detail below). The vector can be either RNA or DNA, either prokaryotic or eukaryotic, and typically is a plasmid. The vector can be maintained as an extrachromosomal element (e.g., a plasmid) or it can be integrated into the chromosome of a recombinant organism (e.g., a microbe or a plant). The entire vector can remain in place within a host cell, or under certain conditions, the plasmid DNA can be deleted, leaving behind the nucleic acid molecule of the present invention. The integrated nucleic acid molecule can be under chromosomal promoter control, under native or plasmid promoter control, or under a combination of several promoter controls. Single or multiple copies of the nucleic acid molecule can be integrated into the chromosome. A recombinant vector of the present invention can contain at least one selectable marker.

In one embodiment, a recombinant vector used in a recombinant nucleic acid molecule of the present invention is an expression vector. As used herein, the phrase "expression vector" is used to refer to a vector that is suitable for production of an encoded product (e.g., a protein of interest). In this embodiment, a nucleic acid sequence encoding the product to be produced (e.g., a PUFA PKS domain) is inserted into the recombinant vector to produce a recombinant nucleic acid molecule. The nucleic acid sequence encoding the protein to be produced is inserted into the vector in a manner that operatively links the nucleic acid sequence to regulatory sequences in the vector which enable the transcription and translation of the nucleic acid sequence within the recombinant host cell.

In another embodiment, a recombinant vector used in a recombinant nucleic acid molecule of the present invention is a targeting vector. As used herein, the phrase "targeting vector" is used to refer to a vector that is used to deliver a particular nucleic acid molecule into a recombinant host cell, wherein the nucleic acid molecule is used to delete or inactivate an endogenous gene within the host cell or microorganism (i.e., used for targeted gene disruption or knock-out technology). Such a vector may also be known in the art as a "knock-out" vector. In one aspect of this embodiment, a portion of the vector, but more typically, the nucleic acid molecule inserted into the vector (i.e., the insert), has a nucleic acid sequence that is homologous to a nucleic acid sequence of a target gene in the host cell (i.e., a gene which is targeted to be deleted or inactivated). The nucleic acid sequence of the vector insert is designed to bind to the target gene such that the target gene and the insert undergo homologous recombination, whereby the endogenous target gene is deleted, inactivated or attenuated (i.e., by at least a portion of the endogenous target gene being mutated or deleted). The use of this type of recombinant vector to replace an endogenous *Schizochytrium* gene with a recombinant gene is described in the Examples section, and the general technique for genetic transformation ofThraustochytrids is described in detail in U.S. Patent Application Serial No. 10/124,807, published as U.S. Patent Application Publication No. 20030166207, published September 4, 2003.

Typically, a recombinant nucleic acid molecule includes at least one nucleic acid molecule of the present invention operatively linked to one or more expression control sequences. As used herein, the phrase "recombinant molecule" or "recombinant nucleic acid molecule" primarily refers to a nucleic acid molecule or nucleic acid sequence operatively linked to a expression control sequence, but can be used interchangeably with the phrase "nucleic acid molecule", when such nucleic acid molecule is a recombinant molecule as discussed herein. According to the present invention, the phrase "operatively linked" refers to linking a nucleic acid molecule to an expression control sequence (e.g., a transcription control sequence and/or a translation control sequence) in a manner such that the molecule is able to be expressed when transfected (i.e., transformed, transduced, transfected, conjugated or conduced) into a host cell. Transcription control sequences are sequences which control the initiation, elongation, or termination of transcription. Particularly important transcription control sequences are those which control transcription initiation, such as promoter, enhancer, operator and repressor sequences. Suitable transcription control sequences include any transcription control sequence that can function in a host cell or organism into which the recombinant nucleic acid molecule is to be introduced.

Recombinant nucleic acid molecules of the present invention can also contain additional regulatory sequences, such as translation regulatory sequences, origins of replication, and other regulatory sequences that are compatible with the recombinant cell. In one embodiment, a recombinant molecule of the present invention, including those which are integrated into the host cell chromosome, also contains secretory signals (i.e., signal segment nucleic acid sequences) to enable an expressed protein to be secreted from the cell that produces the protein. Suitable signal segments include a signal segment that is naturally associated with the protein to be expressed or any heterologous signal segment capable of directing the secretion of the protein according to the present invention. In another embodiment, a recombinant molecule of the present invention comprises a leader sequence to enable an expressed protein to be delivered to and inserted into the membrane of a host cell. Suitable leader sequences include a leader sequence that is naturally associated with the protein, or any heterologous leader sequence capable of directing the delivery and insertion of the protein to the membrane of a cell.

The present inventors have found that the *Schizochytrium* PUFA PKS Orfs A and B are closely linked in the genome and region between the Orfs has been sequenced. The Orfs are oriented in opposite directions and 4244 base pairs separate the start (ATG) codons (i.e. they are arranged as follows: 3'OrfA5' - 4244 bp - 5'OrfB3'). Examination of the 4244 bp intergenic region did not reveal any obvious Orfs (no significant matches were found on a BlastX search). Both Orfs A and B are highly expressed in *Schizochytrium,* at least during the time of oil production, implying that active promoter elements are embedded in this intergenic region. These genetic elements are believed to have utility as a bi-directional promoter sequence for transgenic applications. For example, in a preferred embodiment, one could clone this region, place any genes of interest at each end and introduce the construct into *Schizochytrium* (or some other host in which the promoters can be shown to function). It is predicted that the regulatory elements, under the appropriate conditions, would provide for coordinated, high level expression of the two introduced genes. The complete nucleotide sequence for the regulatory region containing *Schizochytrium* PUFA PKS regulatory elements (e.g., a promoter) is represented herein as SEQ ID NO:36.

In a similar manner, OrfC is highly expressed in *Schizochytrium* during the time of oil production and regulatory elements are expected to reside in the region upstream of its start codon. A region of genomic DNA upstream of OrfC has been cloned and sequenced and is represented herein as (SEQ ID NO:37). This sequence contains the 3886 nt immediately upstream of the OrfC start codon. Examination of this region did not reveal any obvious Orfs (i.e., no significant matches were found on a BlastX search). It is believed that regulatory elements contained in this region, under the appropriate conditions, will provide for high-level expression of a gene placed behind them. Additionally, under the appropriate conditions, the level of expression may be coordinated with genes under control of the A - B intergenic region (SEQ ID NO:36).

Therefore, in one embodiment, a recombinant nucleic acid molecule useful in the present invention, as disclosed herein, can include a PUFA PKS regulatory region contained within SEQ ID NO:36 and/or SEQ ID NO:37. Such a regulatory region can include any portion (fragment) of SEQ ID NO:36 and/or SEQ ID NO:37 that has at least basal PUFA PKS transcriptional activity.

One or more recombinant molecules of the present invention can be used to produce an encoded product (e.g., a PUFA PKS domain, protein, or system) of the present invention. In one embodiment, an encoded product is produced by expressing a nucleic acid molecule as described herein under conditions effective to produce the protein. A preferred method to produce an encoded protein is by transfecting a host cell with one or more recombinant molecules to form a recombinant cell. Suitable host cells to transfect include, but are not limited to, any bacterial, fungal (e.g., yeast), insect, plant or animal cell that can be transfected. In one embodiment of the invention, a preferred host cell is a Thraustochytrid host cell (described in detail below) or a plant host cell. Host cells can be either untransfected cells or cells that are already transfected with at least one other recombinant nucleic acid molecule.

According to the present invention, the term "transfection" is used to refer to any method by which an exogenous nucleic acid molecule (i.e., a recombinant nucleic acid molecule) can be inserted into a cell. The term "transformation" can be used interchangeably with the term "transfection" when such term is used to refer to the introduction of nucleic acid molecules into microbial cells, such as algae, bacteria and yeast, or into plants. In microbial systems, the term "transformation" is used to describe an inherited change due to the acquisition of exogenous nucleic acids by the microorganism or plant and is essentially synonymous with the term "transfection." However, in animal cells, transformation has acquired a second meaning which can refer to changes in the growth properties of cells in culture after they become cancerous, for example. Therefore, to avoid confusion, the term "transfection" is preferably used with regard to the introduction of exogenous nucleic acids into animal cells, and the term "transfection" will be used herein to generally encompass transfection of animal cells, and transformation of microbial cells or plant cells, to the extent that the terms pertain to the introduction of exogenous nucleic acids into a cell. Therefore, transfection techniques include, but are not limited to, transformation, particle bombardment, diffusion, active transport, bath sonication, electroporation, microinjection, lipofection, adsorption, infection and protoplast fusion.

It will be appreciated by one skilled in the art that use of recombinant DNA technologies can improve control of expression of transfected nucleic acid molecules by manipulating, for example, the number of copies of the nucleic acid molecules within the host cell, the efficiency with which those nucleic acid molecules are transcribed, the efficiency with which the resultant transcripts are translated, and the efficiency of post-translational modifications. Additionally, the promoter sequence might be genetically engineered to improve the level of expression as compared to the native promoter. Recombinant techniques useful for controlling the expression of nucleic acid molecules include, but are not limited to, integration of the nucleic acid molecules into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Dalgamo sequences), modification of nucleic acid molecules to correspond to the codon usage of the host cell, and deletion of sequences that destabilize transcripts.

General discussion above with regard to recombinant nucleic acid molecules and transfection of host cells is intended to be applied to any recombinant nucleic acid molecule discussed herein, including those encoding any amino acid sequence having a biological activity of at least one domain from a PUFA PKS, those encoding amino acid sequences from other PKS systems, and those encoding other proteins or domains.

Polyunsaturated fatty acids (PUFAs) are essential membrane components in higher eukaryotes and the precursors of many lipid-derived signaling molecules. The PUFA PKS system of the present invention uses pathways for PUFA synthesis that do not require desaturation and elongation of saturated fatty acids. The pathways catalyzed by PUFA PKSs that are distinct from previously recognized PKSs in both structure and mechanism. Generation of *cis* double bonds is suggested to involve position-specific isomerases; these enzymes are believed to be useful in the production of new families of antibiotics.

To produce significantly high yields of one or more desired polyunsaturated fatty acids or other bioactive molecules, an organism, preferably a microorganism or a plant, and most preferably a Thraustochytrid microorganism, can be genetically modified to alter the activity and particularly, the end product, of the PUFA PKS system in the microorganism or plant.

Therefore, one embodiment of the present invention relates to a genetically modified microorganism, wherein the microorganism expresses a PKS system comprising at least one biologically active domain of a polyunsaturated fatty acid (PUFA) polyketide synthase (PKS) system. The domain of the PUFA PKS system can include any of the domains, including homologues thereof, for PUFA PKS systems as described above (e.g., for *Schizochytrium* and *Thraustochytrium*), and can also include any domain of a PUFA PKS system from any other non-bacterial microorganism, including any eukaryotic microorganism, including any Thraustochytrid microorganism or any domain of a PUFA PKS system from a microorganism identified by a screening method as described in U.S. Patent Application Serial No. 10/124,800, *supra.* The genetic modification affects the activity of the PKS system in the organism. The screening process described in U.S. Patent Application Serial No. 10/124,800 includes the steps of: (a) selecting a microorganism that produces at least one PUFA; and, (b) identifying a microorganism from (a) that has an ability to produce increased PUFAs under dissolved oxygen conditions of less than about 5% of saturation in the fermentation medium, as compared to production of PUFAs by the microorganism under dissolved oxygen conditions of greater than about 5% of saturation, and preferably about 10%, and more preferably about 15%, and more preferably about 20% of saturation in the fermentation medium.

In one aspect, such an organism can endogenously contain and express a PUFA PKS system, and the genetic modification can be a genetic modification of one or more of the functional domains of the endogenous PUFA PKS system, whereby the modification has some effect on the activity of the PUFA PKS system. In another aspect, such an organism can endogenously contain and express a PUFA PKS system, and the genetic modification can be an introduction of at least one exogenous nucleic acid sequence (e.g., a recombinant nucleic acid molecule), wherein the exogenous nucleic acid sequence encodes at least one biologically active domain or protein from a second PKS system and/or a protein that affects the activity of the PUFA PKS system (e.g., a phosphopantetheinyl transferases (PPTase), discussed below). In yet another aspect, the organism does not necessarily endogenously (naturally) contain a PUFA PKS system, but is genetically modified to introduce at least one recombinant nucleic acid molecule encoding an amino acid sequence having the biological activity of at least one domain of a PUFA PKS system. In this aspect, PUFA PKS activity is affected by introducing or increasing PUFA PKS activity in the organism. Various embodiments associated with each of these aspects will be discussed in greater detail below.

It is to be understood that a genetic modification of a PUFA PKS system or an organism comprising a PUFA PKS system can involve the modification of at least one domain of a PUFA PKS system (including a portion of a domain), more than one or several domains of a PUFA PKS system (including adjacent domains, non-contiguous domains, or domains on different proteins in the PUFA PKS system), entire proteins of the PUFA PKS system, and the entire PUFA PKS system (e.g., all of the proteins encoded by the PUFA PKS genes). As such, modifications can include a small modification to a single domain of an endogenous PUFA PKS system; to substitution, deletion or addition to one or more domains or proteins of a given PUFA PKS system; up to replacement of the entire PUFA PKS system in an organism with the PUFA PKS system from a different organism. One of skill in the art will understand that any genetic modification to a PUFA PKS system is encompassed by the invention.

As used herein, a genetically modified microorganism can include a genetically modified bacterium, protist, microalgae, fungus, or other microbe, and particularly, any of the genera of the order Thraustochytriales (e.g., a Thraustochytrid) described herein (e.g., *Schizochytrium, Thraustochytrium, Japonochytrium, Labyrinthula, Labyrinthuloides,* etc.). Such a genetically modified microorganism has a genome which is modified (i.e., mutated or changed) from its normal (i.e., wild-type or naturally occurring) form such that the desired result is achieved (i.e., increased or modified PUFA PKS activity and/or production of a desired product using the PKS system). Genetic modification of a microorganism can be accomplished using classical strain development and/or molecular genetic techniques. Such techniques known in the art and are generally disclosed for microorganisms, for example, in Sambrook et al., 1989, Molecular Cloning: A Laboratory Manual, Cold Spring Harbor Labs Press. The reference Sambrook et al., *ibid*., is incorporated by reference herein in its entirety. A genetically modified microorganism can include a microorganism in which nucleic acid molecules have been inserted, deleted or modified (i.e., mutated; e.g., by insertion, deletion, substitution, and/or inversion of nucleotides), in such a manner that such modifications provide the desired effect within the microorganism.

Preferred microorganism host cells to modify according to the present invention include, but are not limited to, any bacteria, protist, microalga, fungus, or protozoa. In one aspect, preferred microorganisms to genetically modify include, but are not limited to, any microorganism of the order Thraustochytriales, including any microorganism in the families Thraustochytriaceae and Labyrinthulaceae. Particularly preferred host cells for use in the present invention could include microorganisms from a genus including, but not limited to: *Thraustochytrium, , Japonochytrium, Aplanochytrium, Elina* and *Schizochytrium* within the Thraustochytriaceae and *Labyrinthula, Labyrinthuloides,* and *Labyrinthomyxa* within the Labyrinthulaceae. Preferred species within these genera include, but are not limited to: any species within *Labyrinthula,* including *Labrinthula* sp., *Labyrinthula algeriensis, Labyrinthula cienkowskii, Labyrinthula chattonii, Labyrinthula coenocystis, Labyrinthula macrocystis, Labyrinthula macrocystis atlantica, Labyrinthula macrocystis macrocystis, Labyrinthula magnifica, Labyrinthula minuta, Labyriznthula roscoffensis, Labyrinthula valkanovii, Labyrinthula vitellina, Labyrinthula vitellina pacifica, Labyrinthula vitellina vitellina, Labyrinthula zopfii;* any *Labyrinthuloides* species, including *Labyrinthuloides* sp., *Labyrinthuloides minuta, Labyrinthuloides schizochytrops*; any *Labyrinthomyxa* species, including *Labyrinthomyxa* sp., *Labyrinthomyxa pohlia, Labyrinthomyxa sauvageaui,* any *Aplanochytrium* species, including *Aplanochytrium* sp. and *Aplanochytrium kerguelensis*; any *Elina* species, including *Elina* sp., *Elina marisalba, Elina sinorifica*; any *Japanochytrium* species, including *Japanochytrium* sp., *Japanochytrium marinum*; any *Schizochytrium* species, including *Schizochytrium* sp., *Schizochytrium aggregatum, Schizochytrium limacinum, Schizochytrium minutum, Schizochytrium octosporum;* and *any Thraustochytrium* species, including *Thraustochytrium* sp., *Thraustochytrium aggregatum, Thraustochytrium arudimentale, Thraustochytrium aureum, Thraustochytrium benthicola, Thraustochytrium globosum, Thraustochytrium kinnei, Thraustochytrium motivum, Thraustochytrium pachydermum, Thraustochytrium proliferum, Thraustochytrium roseum, Thraustochytrium striatum, Ulkenia* sp., *Ulkenia minuta, Ulkenia profunda, Ulkenia radiate, Ulkenia sarkariana,* and *Ulkenia visurgensis.* Particularly preferred species within these genera include, but are not limited to: any *Schizochytrium* species, including *Schizochytrium aggregatum, Schizochytrium limacinum, Schizochytrium minutum*; any *Thraustochytrium* species (including former *Ulkenia* species such as *U. vistirgensis, U. amoeboida, U. sarkariana, U. profunda, U. radiata, U. minuta* and *Ulkenia sp.* BP-5601), and including *Thraustochytrium striatum, Thraustochytrium aureum, Thraustochytrium roseum*; and any *Japonochytrium* species. Particularly preferred strains ofThraustochytriales include, but are not limited to: *Schizochytrium sp.* (S31)(ATCC 20888); *Schizochytrium sp.* (S8)(ATCC 20889); *Schizochytrium sp.* (LC-RM)(ATCC 18915); *Schizochytrium sp.* (SR21); *Schizochytrium aggregatum* (Goldstein et Belsky)(ATCC 28209); *Schizochytrium limacinum* (Honda et Yokochi)(IFO 32693); *Thraustochytrium sp.* (23B)(ATCC 20891); *Thraustochytrium striatum* (Schneider)(ATCC 24473); *Thraustochytrium aureum* (Goldstein)(ATCC 34304); *Thraustochytrium roseum* (Goldstein)(ATCC 28210); and *Japonochytrium sp.* (L1)(ATCC 28207). Other examples of suitable host microorganisms for genetic modification include, but are not limited to, yeast including *Saccharomyces cerevisiae, Saccharomyces carlsbergensis,* or other yeast such as *Candida, Kluyveromyces,* or other fungi, for example, filamentous fungi such as *Aspergillus, Neurospora, Penicillium,* etc. Bacterial cells also may be used as hosts. These include, but are not limited to, *Escherichia coli,* which can be useful in fermentation processes. Alternatively, and only by way of example, a host such as a *Lactobacillus* species or *Bacillus* species can be used as a host.

Another embodiment of the present invention relates to a genetically modified plant, wherein the plant has been genetically modified to recombinantly express a PKS system comprising at least one biologically active domain of a polyunsaturated fatty acid (PUFA) polyketide synthase (PKS) system. The domain of the PUFA PKS system can include any of the domains, including homologues thereof, for PUFA PKS systems as described above (e.g., for *Schizochytrium* and/or *Thraustochytrium*), and can also include any domain of a PUFA PKS system from any non-bacterial microorganism (including any eukaryotic microorganism and any other Thraustochytrid microorganism) or any domain of a PUFA, PKS system from a microorganism identified by a screening method as described in U.S. Patent Application Serial No. 10/124,800, *supra.* The plant can also be further modified with at least one domain or biologically active fragment thereof of another PKS system, including, but not limited to, bacterial PUFA PKS or PKS systems, Type I PKS systems, Type II PKS systems, modular PKS systems, and/or any non-bacterial PUFA PKS system (e.g., eukaryotic, Thraustochytrid, Thraustochytriaceae or Labyrinthulaceae, *Schizochytrium,* etc.).

As used herein, a genetically modified plant can include any genetically modified plant including higher plants and particularly, any consumable plants or plants useful for producing a desired bioactive molecule of the present invention. Such a genetically modified plant has a genome which is modified (i.e., mutated or changed) from its normal (i.e., wild-type or naturally occurring) form such that the desired result is achieved (i.e., increased or modified PUFA PKS activity and/or production of a desired product using the PKS system). Genetic modification of a plant can be accomplished using classical strain development and/or molecular genetic techniques. Methods for producing a transgenic plant, wherein a recombinant nucleic acid molecule encoding a desired amino acid sequence is incorporated into the genome of the plant, are known in the art. A preferred plant to genetically modify according to the present invention is preferably a plant suitable for consumption by animals, including humans.

Preferred plants to genetically modify according to the present invention (i.e., plant host cells) include, but are not limited to any higher plants, and particularly consumable plants, including crop plants and especially plants used for their oils. Such plants can include, for example: canola, soybeans, rapeseed, linseed, corn, safflowers, sunflowers and tobacco. Other preferred plants include those plants that are known to produce compounds used as pharmaceutical agents, flavoring agents, neutraceutical agents, functional food ingredients or cosmetically active agents or plants that are genetically engineered to produce these compounds/agents.

According to the present invention, a genetically modified microorganism or plant includes a microorganism or plant that has been modified using recombinant technology or by classical mutagenesis and screening techniques. As used herein, genetic modifications which result in a decrease in gene expression, in the function of the gene, or in the function of the gene product (i.e., the protein encoded by the gene) can be referred to as inactivation (complete or partial), deletion, interruption, blockage or down-regulation of a gene. For example, a genetic modification in a gene which results in a decrease in the function of the protein encoded by such gene, can be the result of a complete deletion of the gene (i.e., the gene does not exist, and therefore the protein does not exist), a mutation in the gene which results in incomplete or no translation of the protein (e.g., the protein is not expressed), or a mutation in the gene which decreases or abolishes the natural function of the protein (e.g., a protein is expressed which has decreased or no enzymatic activity or action). Genetic modifications that result in an increase in gene expression or function can be referred to as amplification, overproduction, overexpression, activation, enhancement, addition, or up-regulation of a gene.

The genetic modification of a microorganism or plant according to the present invention preferably affects the activity of the PKS system expressed by the microorganism or plant, whether the PKS system is endogenous and genetically modified, endogenous with the introduction of recombinant nucleic acid molecules into the organism (with the option of modifying the endogenous system or not), or provided completely by recombinant technology. To alter the PUFA production profile of a PUFA PKS system or organism expressing such system includes causing any detectable or measurable change in the production of any one or more PUFAs by the host microorganism or plant as compared to in the absence of the genetic modification (i.e., as compared to the unmodified, wild-type microorganism or plant or the microorganism or plant that is unmodified at least with respect to PUFA synthesis - i.e., the organism might have other modifications not related to PUFA synthesis). To affect the activity of a PKS system includes any genetic modification that causes any detectable or measurable change or modification in the PKS system expressed by the organism as compared to in the absence of the genetic modification. A detectable change or modification in the PKS system can include, but is not limited to: a change or modification (introduction of, increase or decrease) of the expression and/or biological activity of any one or more of the domains in a modified PUFA PKS system as compared to the endogenous PUFA PKS system in the absence of genetic modification, the introduction of PKS system activity into an organism such that the organism now has measurable/detectable PKS system activity (i.e., the organism did not contain a PKS system prior to the genetic modification), the introduction into the organism of a functional domain from a different PKS system than a PKS system endogenously expressed by the organism such that the PKS system activity is modified (e.g., a bacterial PUFA PKS domain or a type I PKS domain is introduced into an organism that endogenously expresses a non-bacterial PUFA PKS system), a change in the amount of a bioactive molecule (e.g., a PUFA) produced by the PKS system (e.g., the system produces more (increased amount) or less (decreased amount) of a given product as compared to in the absence of the genetic modification), a change in the type of a bioactive molecule (e.g., a change in the type of PUFA) produced by the PKS system (e.g., the system produces an additional or different PUFA, a new or different product, or a variant of a PUFA or other product that is naturally produced by the system), and/or a change in the ratio of multiple bioactive molecules produced by the PKS system (e.g., the system produces a different ratio of one PUFA to another PUFA, produces a completely different lipid profile as compared to in the absence of the genetic modification, or places various PUFAs in different positions in a triacylglycerol as compared to the natural configuration). Such a genetic modification includes any type of genetic modification and specifically includes modifications made by recombinant technology and by classical mutagenesis.

It should be noted that reference to increasing the activity of a functional domain or protein in a PUFA PKS system refers to any genetic modification in the organism containing the domain or protein (or into which the domain or protein is to be introduced) which results in increased functionality of the domain or protein system and can include higher activity of the domain or protein (e.g., specific activity *or in vivo* enzymatic activity), reduced inhibition or degradation of the domain or protein system, and overexpression of the domain or protein. For example, gene copy number can be increased, expression levels can be increased by use of a promoter that gives higher levels of expression than that of the native promoter, or a gene can be altered by genetic engineering or classical mutagenesis to increase the activity of the domain or protein encoded by the gene.

Similarly, reference to decreasing the activity of a functional domain or protein in a PUFA PKS system refers to any genetic modification in the organism containing such domain or protein (or into which the domain or protein is to be introduced) which results in decreased functionality of the domain or protein and includes decreased activity of the domain or protein, increased inhibition or degradation of the domain or protein and a reduction or elimination of expression of the domain or protein. For example, the action of domain or protein of the present invention can be decreased by blocking or reducing the production of the domain or protein, "knocking out" the gene or portion thereof encoding the domain or protein, reducing domain or protein activity, or inhibiting the activity of the domain or protein. Blocking or reducing the production of a domain or protein can include placing the gene encoding the domain or protein under the control of a promoter that requires the presence of an inducing compound in the growth medium. By establishing conditions such that the inducer becomes depleted from the medium, the expression of the gene encoding the domain or protein (and therefore, of protein synthesis) could be turned off. The present inventors demonstrate the ability to delete (knock out) targeted genes in a Thraustochytrid microorganism in the Examples section. Blocking or reducing the activity of domain or protein could also include using an excision technology approach similar to that described in U.S. Patent No. 4,743,546, incorporated herein by reference. To use this approach, the gene encoding the protein of interest is cloned between specific genetic sequences that allow specific, controlled excision of the gene from the genome. Excision could be prompted by, for example, a shift in the cultivation temperature of the culture, as in U.S. Patent No. 4,743,546, or by some other physical or nutritional signal.

In one embodiment of the present invention, a genetic modification includes a modification of a nucleic acid sequence encoding an amino acid sequence that has a biological activity of at least one domain of a non-bacterial PUFA PKS system as described herein (e.g., a domain, more than one domain, a protein, or the entire PUFA PKS system, of an endogenous PUFA PKS system of a Thraustochytrid host). Such a modification can be made to an amino acid sequence within an endogenously (naturally) expressed non-bacterial PUFA PKS system, whereby a microorganism that naturally contains such a system is genetically modified by, for example, classical mutagenesis and selection techniques and/or molecular genetic techniques, include genetic engineering techniques. Genetic engineering techniques can include, for example, using a targeting recombinant vector to delete a portion of an endogenous gene (demonstrated in the Examples), or to replace a portion of an endogenous gene with a heterologous sequence (demonstrated in the Examples). Examples of heterologous sequences that could be introduced into a host genome include sequences encoding at least one functional domain from another PKS system, such as a different non-bacterial PUFA PKS system (e.g., from a eukaryote, including another Thraustochytrid), a bacterial PUFA PKS system, a type I PKS system, a type II PKS system, or a modular PKS system. A heterologous sequence can also include an entire PUFA PKS system (e.g., all genes associated with the PUFA PKS system) that is used to replace the entire endogenous PUFA PKS system (e.g., all genes of the endogenous PUFA PKS system) in a host. A heterologous sequence can also include a sequence encoding a modified functional domain (a homologue) of a natural domain from a PUFA PKS system of a host Thraustochytrid (e.g., a nucleic acid sequence encoding a modified domain from OrfB of a *Schizochytrium,* wherein the modified domain will, when used to replace the naturally occurring domain expressed in the *Schizochytrium,* alter,the PUFA production profile by the *Schizochytrium*). Other heterologous sequences to introduce into the genome of a host includes a sequence encoding a protein or functional domain that is not a domain of a PKS system, but which will affect the activity of the endogenous PKS system. For example, one could introduce into the host genome a nucleic acid molecule encoding a phosphopantetheinyl transferase (discussed below). Specific modifications that could be made to an endogenous PUFA PKS system are discussed in detail herein.

In another aspect of this embodiment of the invention, the genetic modification can include: (1) the introduction of a recombinant nucleic acid molecule encoding an amino acid sequence having a biological activity of at least one domain of a PUFA PKS system; and/or (2) the introduction of a recombinant nucleic acid molecule encoding a protein or functional domain that affects the activity of a PUFA PKS system, into a host. The host can include: (1) a host cell that does not express any PKS system, wherein all functional domains of a PKS system are introduced into the host cell, and wherein at least one functional domain is from a non-bacterial PUFA PKS system; (2) a host cell that expresses a PKS system (endogenous or recombinant) having at least one functional domain of a non-bacterial PUFA PKS system, wherein the introduced recombinant nucleic acid molecule can encode at least one additional non-bacterial PUFA PKS domain function or another protein or domain that affects the activity of the host PKS system; and (3) a host cell that expresses a PKS system (endogenous or recombinant) which does not necessarily include a domain function from a non-bacterial PUFA PKS, and wherein the introduced recombinant nucleic acid molecule includes a nucleic acid sequence encoding at least one functional domain of a non-bacterial PUFA PKS system. In other words, the present invention intends to encompass any genetically modified organism (e.g., microorganism or plant), wherein the organism comprises at least one non-bacterial PUFA PKS domain function (either endogenously or introduced by recombinant modification), and wherein the genetic modification has a measurable effect on the non-bacterial PUFA PKS domain function or on the PKS system when the organism comprises a functional PKS system.

The present invention encompasses many possible non-bacterial and bacterial microorganisms as either possible host cells for the PUFA PKS systems described herein and/or as sources for additional genetic material encoding PUFA PKS system proteins and domains for use in the genetic modifications and methods described herein. For example, microbial organisms with a PUFA PKS system similar to that found in *Schizochytrium,* such as the *Thraustochytrium* microorganism discovered by the present inventors and described in Example 1, can be readily identified/isolated/screened by methods to identify other non-bacterial microorganisms that have a polyunsaturated fatty acid (PUFA) polyketide synthase (PKS) system that are described in detail in U.S. Patent Application Publication No. 20020194641, *supra* (corresponding to U.S. Patent Application Serial No. 10/124,800).

Locations for collection of the preferred types of microbes for screening for a PUFA PKS system according to the present invention include any of the following: low oxygen environments (or locations near these types of low oxygen environments including in the guts of animals including invertebrates that consume microbes or microbe-containing foods (including types of filter feeding organisms), low or non-oxygen containing aquatic habitats (including freshwater, saline and marine), and especially at-or near-low oxygen environments (regions) in the oceans. The microbial strains would preferably not be obligate anaerobes but be adapted to live in both aerobic and low or anoxic environments. Soil environments containing both aerobic and low oxygen or anoxic environments would also excellent environments to find these organisms in and especially in these types of soil in aquatic habitats or temporary aquatic habitats.

A particularly preferred non-bacterial microbial strain to screen for use as a host and/or a source of PUFA PKS genes according to the present invention would be a strain (selected from the group consisting of algae, fungi (including yeast), protozoa or protists) that, during a portion of its life cycle, is capable of consuming whole bacterial cells (bacterivory) by mechanisms such as phagocytosis, phagotrophic or endocytic capability and/or has a stage of its life cycle in which it exists as an amoeboid stage or naked protoplast. This method of nutrition would greatly increase the potential for transfer of a bacterial PKS system into a eukaryotic cell if a mistake occurred and the bacterial cell (or its DNA) did not get digested and instead are functionally incorporated into the eukaryotic cell.

Included in the present invention as sources of PUFA PKS genes (and proteins and domains encoded thereby) are any Thraustochytrids other than those specifically described herein that contain a PUFA PKS system. Such Thraustochytrids include, but are not limited to, but are not limited to, any microorganism of the order Thraustochytriales, including any microorganism in the families Thraustochytriaceae and Labyrinthulaceae, which further comprise a genus including, but not limited to: *Thraustochytrium,* , *Japonochytrium, Aplanochytrium, Elina* and *Schizochytrium* within the Thraustochytriaceae and *Labyrinthula, Labyrinthuloides,* and *Labyrinthomyxa* within the Labyrinthulaceae. Preferred species within these genera include, but are not limited to: any species within *Labyrinthula,* including *Labrinthula* sp., *Labyrinthula algeriensis, Labyrinthula cienkowskii, Labyrinthula chattonii, Labyrinthula coenocystis, Labyrinthula macrocystis, Labyrinthula macrocystis atlantica, Labyrinthula macrocystis macrocystis, Labyrinthula magnifica, Labyrinthula minuta, Labyrinthula roscoffensis, Labyrinthula valkanovii, Labyrinthula vitellina, Labyrinthula vitellina pacifica, Labyrinthula vitellina vitellina, Labyrinthula zopfii*; any *Labyrinthuloides* species, including *Labyrinthuloides* sp., *Labyrinthuloides minuta, Labyrinthuloides schizochytrops*; any *Labyrinthomyxa* species, including *Labyrinthomyxa* sp., *Labyrinthomyxa pohlia, Labyrinthomyxa sauvageaui,* any *Aplanochytrium* species, including *Aplanochytrium* sp. and *Aplanochytrium kerguelensis*; any *Elina* species, including *Elina* sp., *Elina marisalba, Elina sinorifica*; any *Japanochytrium* species, including *Japanochytrium* sp., *Japanochytrium marinum*; any *Schizochytrium* species, including *Schizochytrium* sp., *Schizochytrium aggregatum, Schizochytrium limacinum, Schizochytrium minutum, Schizochytrium octosporum*; and any *Thraustochytrium* species, including *Thraustochytrium* sp., *Thraustochytrium aggregatum, Thraustochytrium arudimentale, Thraustochytrium aureum, Thraustochytrium benthicola, Thraustochytrium globosum, Thraustochytrium kinnei, Thraustochytrium motivum, Thraustochytrium pachydermum, Thraustochytrium proliferum, Thraustochytrium roseum, Thraustochytrium striatum, Ulkenia* sp., *Ulkenia minuta, Ulkenia profunda, Ulkenia radiate, Ulkenia sarkariana,* and *Ulkenia visurgensis.*

It is noted that, without being bound by theory, the present inventors consider *Labyrinthula* and other Labyrinthulaceae as sources of PUFA PKS genes because the Labyrinthulaceae are closely related to the Thraustochytriaceae which are known to possess PUFA PKS genes, the Labyrinthulaceae are known to be bactivorous/phagocytotic, and some members of the Labyrinthulaceae have fatty acid/PUFA profiles consistent with having a PUFA PKS system.

Strains of microbes (other than the members of the Thraustochytrids) capable of bacterivory (especially by phagocytosis or endocytosis) can be found in the following microbial classes (including but not limited to example genera):
In the algae and algae-like microbes (including Stramenopiles): of the class Euglenophyceae (for example *genera Euglena, and Peranema*), the class Chrysophyceae (for example the genus *Ochromonas*), the class Dinobryaceae (for example the genera *Dinobryon, Platychrysis,* and *Chrysochromulina*), the Dinophyceae (including the genera *Crypthecodinium, Gymnodinium, Peridinium, Ceratium, Gyrodinium,* and *Oxyrrhis*)*,* the class Cryptophyceae (for example the genera *Cryptomonas,* and *Rhodomonas*), the class Xanthophyceae (for example the genus *Olisthodiscus*) (and including forms of algae in which an amoeboid stage occurs as in the flagellates Rhizochloridaceae, and zoospores/gametes of *Aphanochaete pascheri, Bumilleria stigeoclonium* and *Vaucheria geminata*)*,* the class Eustigmatophyceae, and the class Prymnesiopyceae (including the genera Prymnesium and Diacronema).
In the Stramenopiles including the: Proteromonads, Opalines, Developayella, Diplophorys, Labyrinthulids, Thraustochytrids, Bicosecids, Oomycetes, Hypochytridiomycetes, Commation, Reticulosphaera, Pelagomonas, Pelapococcus, Ollicola, Aureococcus, Parmales, Raphidiophytes, Synurids, Rhizochromulinaales, Pedinellales, Dictyochales, Chrysomeridales, Sarcinochrysidales, Hydrurales, Hibberdiales, and Chromulinales.
In the Fungi: Class Myxomycetes (form myxamoebae) -- slime molds, class Acrasieae including the orders Acrasiceae (for example the genus Sappinia), class Guttulinaceae (for example the genera *Guttulinopsis, and Guttulina*), class Dictysteliaceae (for example the genera *Acrasis, Dictyostelium, Polysphondylium, and Coenonia*), and class Phycomyceae including the orders Chytridiales, Ancylistales, Blastocladiales, Monoblepharidales, Saprolegniales, Peronosporales, Mucorales, and Entomophthorales.
In the Protozoa: Protozoa strains with life stages capable of bacterivory (including byphageocytosis) can be selected from the types classified as ciliates, flagellates or amoebae. Protozoan ciliates include the groups: Chonotrichs, Colpodids, Cyrtophores, Haptorids, Karyorelicts, Oligohymenophora, Polyhymenophora (spirotrichs), Prostomes and Suctoria. Protozoan flagellates include the Biosoecids, Bodonids, Cercomonads, Chrysophytes (for example the genera Anthophysa, Chrysamoemba, Chrysosphaerella, Dendromonas, Dinobryon, Mallomonas, Ochromonas, Paraphysomonas, Poterioochromonas, Spumella, Syncrypta, Synura, and Uroglena), Collar flagellates, Cryptophytes (for example the genera Chilomonas, Cryptomonas, Cyanomonas, and Goniomonas), Dinoflagellates, Diplomonads, Euglenoids, Heterolobosea, Pedinellids, Pelobionts, Phalansteriids, Pseudodendromonads, Spongomonads and Volvocales (and other flagellates including the unassigned flagellate genera ofArtodiscus, Clautriavia, Helkesimastix, Kathablepharis and Multicilia). Amoeboid protozoans include the groups: Actinophryids, Centrohelids, Desmothoricids, Diplophryids, Eumamoebae, Heterolobosea, Leptomyxids, Nucleariid filose amoebae, Pelebionts, Testate amoebae and Vampyrellids (and including the unassigned amoebid genera Gymnophrys, Biomyxa, Microcometes, Reticulomyxa, Belonocystis, Elaeorhanis, Allelogromia, Gromia or Lieberkuhnia). The protozoan orders include the following: Percolomonadeae, Heterolobosea, Lyromonadea, Pseudociliata, Trichomonadea, Hypermastigea, Heteromiteae, Telonemea, Cyathobodonea, Ebridea, Pyytomyxea, Opalinea, Kinetomonadea, Hemimastigea, ,Protostelea, Myxagastrea, Dictyostelea, Choanomonadea, Apicomonadea, Eogregarinea, Neogregarinea, Coelotrolphea, Eucoccidea, Haemosporea, Piroplasmea, Spirotrichea, Prostomatea, Litostomatea, Phyllopharyngea, Nassophorea, Oligohymenophorea, Colpodea, Karyorelicta, Nucleohelea, Centrohelea, Acantharea, Sticholonchea, Polycystinea, Phaeodarea, Lobosea, Filosea, Athalamea, Monothalamea, Polythalamea, Xenophyophorea, Schizocladea, Holosea, Entamoebea, Myxosporea, Actinomyxea, Halosporea, Paramyxea, Rhombozoa and Orthonectea.

A preferred embodiment of the present invention includes strains of the microorganisms listed above that have been collected from one of the preferred habitats listed above.

In some embodiments of this method of the present invention, PUFA PKS systems from bacteria, including genes and portions thereof (encoding entire PUFA PKS systems, proteins thereof and/or domains thereof) can be used to genetically modify other PUFA PKS systems (e.g., any non-bacterial PUFA PKS system) and/or microorganisms containing the same (or vice versa) in the embodiments of the invention. In one aspect, novel PUFA PKS systems can be identified in bacteria that are expected to be particularly useful for creating genetically modified microorganisms (e.g., genetically modified Thraustochytrids) and/or novel hybrid constructs encoding PUFA PKS systems for use in the methods and genetically modified microorganisms and plants of the present invention. In one aspect, bacteria that may be particularly useful in the embodiments of the present invention have PUFA PKS systems, wherein the PUFA PKS system is capable of producing PUFAs at temperatures exceeding about 20°C, preferably exceeding about 25°C and even more preferably exceeding about 30°C. As described previously herein, the marine bacteria, *Shewanella* and *Vibrio marinus,* described in U.S. Patent No. 6,140,486, do not produce PUFAs at higher temperatures, which limits the usefulness of PUFA PKS systems derived from these bacteria, particularly in plant applications under field conditions. Therefore, in one embodiment, the screening method of the present invention can be used to identify bacteria that have a PUFA PKS system, wherein the bacteria are capable of growth and PUFA production at higher temperatures (e.g., above about 15°C, 20°C, 25°C, or 30°C or even higher). However, even if the bacteria sources do not grow well and/or produce PUFAs at the higher temperatures, the present invention encompasses the identification, isolation and use of the PUFA PKS systems (genes and proteins/domains encoded thereby), wherein the *PUFA PKS systems* from the bacteria have enzymatic/biological activity at temperatures above about 15°C, 20°C, 25°C, or 30°C or even higher. In one aspect of this embodiment, inhibitors of eukaryotic growth such as nystatin (antifungal) or cycloheximide (inhibitor of eukaryotic protein synthesis) can be added to agar plates used to culture/select initial strains from water samples/soil samples collected from the types of habitats/niches such as marine or estuarian habits, or any other habitat where such bacteria can be found. This process would help select for enrichment of bacterial strains without (or minimal) contamination of eukaryotic strains. This selection process, in combination with culturing the plates at elevated temperatures (e.g. 30°C), and then selecting strains that produce at least one PUFA would initially identify candidate bacterial strains with a PUFA PKS system that is operative at elevated temperatures (as opposed to those bacterial strains in the prior art which only exhibit PUFA production at temperatures less than about 20°C and more preferably below about 5°C).

However, even in bacteria that do not grow well (or at all) at higher temperatures, or that do not produce at least one PUFA at higher temperatures, such strains can be identified and selected as comprising a PUFA PKS system by the identification of the ability of the bacterium to produce PUFAs under any conditions and/or by screening the genome of the bacterium for genes that are homologous to other known PUFA PKS genes from bacteria or non-bacterial organisms (e.g., see Example 7). To evaluate PUFA PKS function at higher temperatures for genes from any bacterial source, one can produce cell-free extracts and test for PUFA production at various temperatures, followed by selection of microorganisms that contain PUFA PKS genes that have enzymatic/biological activity at higher temperature ranges (e.g., 15°C, 20°C, 25°C, or 30°C or even higher).

Suitable bacteria to use as hosts for genetic modification include any bacterial strain as discussed above. Particularly suitable bacteria to use as a *source* of PUFA PKS genes (and proteins and domains encoded thereby) for the production of genetically modified sequences and organisms according to the present invention include any bacterium that comprises a PUFA PKS system. Such bacteria are typically isolated from marine or estuarian habitats and can be readily identified by their ability to product PUFAs and/or by the presence of one or more genes having homology to known PUFA PKS genes in the organism. Such bacteria can include, but are not limited to, bacteria of the genera *Shewanella* and *Vibrio.* Preferred bacteria for use in the present invention include those with PUFA PKS systems that are biologically active at higher temperatures (e.g., above about 15°C, 20°C, 25°C, or 30°C or even higher). The present inventors have identified two exemplary bacteria (e.g. *Shewanella olleyana* and *Shewanella japonica;* see Examples 7 and 8) that will be particularly suitable for use as sources of PUFA PKS genes, and others can be readily identified or are known to comprise PUFA PKS genes and may be useful in an embodiment of the present invention (e.g., *Shewanella gelidimarina*)*.*

Furthermore, it is recognized that not all bacterial or non-bacterial microorganisms can be readily cultured from natural habitats. However, genetic characteristics of such un-culturable microorganisms can be evaluated by isolating genes from DNA prepared *en mass* from mixed or crude environmental samples. Particularly suitable to the present invention, PUFA PKS genes derived from un-culturable microorganisms can be isolated from environmental DNA samples by degenerate PCR using primers designed to generally match regions of high similarity in known PUFA PKS genes (e.g., see Example 7). Alternatively, whole DNA fragments can be cloned directly from purified environmental DNA by any of several methods known to the art. Sequence of the DNA fragments thus obtained can reveal homologs to known genes such as PUFA PKS genes. Homologs of OrfB and OrfC (referring to the domain structure of *Schizochytrium* and *Thraustochytrium,* for example) may be particularly useful in defining the PUFA PKS end product. Whole coding regions of PUFA PKS genes can then be expressed in host organisms (such as *Escherichia coli* or yeast) in combination with each other or with known PUFA PKS gene or gene fragment combinations to evaluate their effect on PUFA production. As described above, activity in cell-free extracts can be used to determine function at desired temperatures. Isolated PUFA PKS genes can also be transformed directly into appropriate *Schizochytrium* or other suitable strains to measure function. PUFA PKS system-encoding constructs identified or produced in such a manner, including hybrid constructs, can also be used to transform other organisms, such as plants.

Therefore, using the non-bacterial PUFA PKS systems of the present invention, which, for example, makes use of genes from Thraustochytrid PUFA PKS systems, as well as PUFA PKS systems and PKS systems from bacteria, gene mixing can be used to extend the range of PUFA products to include EPA, DHA, ARA, GLA, SDA and others (described in detail below), as well as to produce a wide variety of bioactive molecules, including antibiotics, other pharmaceutical compounds, and other desirable products. The method to obtain these bioactive molecules includes not only the mixing of genes from various organisms but also various methods of genetically modifying the non-bacterial PUFA PKS genes disclosed herein. Knowledge of the genetic basis and domain structure of the non-bacterial PUFA PKS system of the present invention provides a basis for designing novel genetically modified organisms which produce a variety of bioactive molecules. Although mixing and modification of any PKS domains and related genes are contemplated by the present inventors, by way of example, various possible manipulations of the PUFA-PKS system are discussed below with regard to genetic modification and bioactive molecule production.

Accordingly, encompassed by the present invention are methods to genetically modify microbial or plant cells by: genetically modifying at least one nucleic acid sequence in the organism that encodes an amino acid sequence having the biological activity of at least one functional domain of a non-bacterial PUFA PKS system according to the present invention, and/or expressing at least one recombinant nucleic acid molecule comprising a nucleic acid sequence encoding such amino acid sequence. Various embodiments of such sequences, methods to genetically modify an organism, and specific modifications have been described in detail above. Typically, the method is used to produce a particular genetically modified organism that produces a particular bioactive molecule or molecules.

One embodiment of the present invention relates to a genetically modified Thraustochytrid microorganism, wherein the microorganism has an endogenous polyunsaturated fatty acid (PUFA) polyketide synthase (PKS) system, and wherein the endogenous PUFA PKS system has been genetically modified to alter the expression profile of a polyunsaturated fatty acid (PUFA) by the microorganism as compared to the Thraustochytrid microorganism in the absence of the modification. Thraustochytrid microorganisms useful as host organisms in the present invention endogenously contain and express a PUFA PKS system. The genetic modification can be a genetic modification of one or more of the functional domains of the endogenous PUFA PKS system, whereby the modification alters the PUFA production profile of the endogenous PUFA PKS system. In addition, or as an alternative, the genetic modification can be an introduction of at least one exogenous nucleic acid sequence (e.g., a recombinant nucleic acid molecule) to the microorganism, wherein the exogenous nucleic acid sequence encodes at least one biologically active domain or protein from a second PKS system and/or a protein that affects the activity of the PUFA PKS system (e.g., a phosphopantetheinyl transferases (PPTase)). The second PKS system can be any PKS system, including other PUFA PKS systems and including homologues of genes from the Thraustochytrid PUFA PKS system to be genetically modified.

This embodiment of the invention is particularly useful for the production of commercially valuable lipids enriched in a desired PUFA, such as EPA, via the present inventors' development of genetically modified microorganisms and methods for efficiently producing lipids (triacylglyerols (TAG) as well as membrane-associated phospholipids (PL)) enriched in PUFAs.

This particular embodiment of the present invention is derived in part from the following knowledge: (1) utilization of the inherent TAG production capabilities of selected microorganisms, and particularly, of Thraustochytrids, such as the commercially developed *Schizochytrium* strain described herein; (2) the present inventors' detailed understanding of PUFA PKS biosynthetic pathways (i.e., PUFA PKS systems) in eukaryotes and in particular, in members of the order Thraustochytriales; and, (3) utilization of a homologous genetic recombination system in *Schizochytrium.* Based on the inventors' knowledge of the systems involved, the same general approach may be exploited to produce PUFAs other than EPA.

In one embodiment of the invention, the endogenous Thraustochytrid PUFA PKS genes, such as the *Schizochytrium* genes encoding PUFA PKS enzymes that normally produce DHA and DPA, are modified by random or targeted mutagenesis, replaced with genes from other organisms that encode homologous PKS proteins (e.g., from bacteria or other sources), or replaced with genetically modified *Schizochytrium, Thraustochytrium* or other Thraustochytrid PUFA PKS genes. The product of the enzymes encoded by these introduced and/or modified genes can be EPA, for example, or it could be some other related molecule, including other PUFAs. One feature of this method is the utilization of endogenous components of Thraustochytrid PUFA synthesis and accumulation machinery that is essential for efficient production and incorporation of the PUFA into PL and TAG. In particular, this embodiment of the invention is directed to the modification of the type of PUFA produced by the organism, while retaining the high oil productivity of the parent strain.

Although some of the following discussion uses the organism *Schizochytrium* as an exemplary host organism, any Thraustochytrid can be modified according to the present invention, including members of the genera *Thraustochytrium, Labyrinthuloides,* and *Japonochytrium.* For example, the genes encoding the PUFA PKS system for a species of *Thraustochytrium* have been identified (see Example 6), and this organism can also serve as a host organism for genetic modification using the methods described herein, although it is more likely that the *Thraustochytrium* PKS genes will be used to modify the endogenous PUFA PKS genes of another Thraustochytrid, such as *Schizochytrium.* Furthermore, using methods for screening organisms as set forth in U.S. Application Serial No. 10/124,800, *supra,* one can identify other organisms useful in the present method and all such organisms are encompassed herein.

This embodiment of the present invention can be illustrated as follows. By way of example, based on the present inventors' current understanding of PUFA synthesis and accumulation in *Schizochytrium,* the overall biochemical process can be divided into three parts.

First, the PUFAs that accumulate in *Schizochytrium* oil (DHA and DPA) are the product of a PUFA PKS system as discussed above. The PUFA PKS system in *Schizochytrium* converts malonyl-CoA into the end product PUFA without release of significant amounts of intermediate compounds. In *Schizochytrium,* three genes have been identified (Orfs A, B and C; also represented by SEQ ID NO:1, SEQ ID NO:3 and SEQ ID NO:5, respectively) that encode all of the enzymatic domains known to be required for actual synthesis of PUFAs. Similar sets of genes (encoding proteins containing homologous sets of enzymatic domains) have been cloned and characterized from several other non-eukaryotic organisms that produce PUFAs, namely, several strains of marine bacteria. In addition, the present inventors have identified and now sequenced PUFA PKS genes in at least one other marine protist (Thraustochytrium strain 23B) (described in detail below).

The PUFA products of marine bacteria include EPA (e.g., produced by *Shewanella* SRC2738 and *Photobacter profundum*) as well as DHA (*Vibrio marinus,* now known as *Moritella marina*) (described in U.S. Patent No. 6,140,486, *supra*; and in U.S. Patent No. 6,566,583*, supra).* It is an embodiment of the invention that any PUFA PKS gene set could be envisioned to substitute for the *Schizochytrium* genes described in the example herein, as long as the physiological growth requirements of the production organism (e.g., *Schizochytrium*) in fermentation conditions were satisfied. In particular, the PUFA-producing bacterial strains described above grow only at relatively low temperatures (typically less than 20°C) which further indicates that their PUFA PKS gene products will not function at standard growth temperatures for *Schizochytrium* (25-30°C). However, the inventors have recently identified at least two other marine bacteria that grow and produce EPA at standard growth temperatures for *Schizochytrium* and other Thraustochytrids (see Example 7). These alternate marine bacteria have been shown to possess PUFA-PKS-like genes that will serve as material for modification of *Schizochytrium* and other Thraustochytrids by methods described herein. It will be apparent to those skilled in the art from this disclosure that other currently unstudied or unidentified PUFA-producing bacteria could also contain PUFA PKS genes useful for modification of Thraustochytrids.

Second, in addition to the genes that encode the enzymes directly involved in PUFA synthesis, an "accessory" enzyme is required. The gene encodes a phosphopantetheine transferase (PPTase) that activates the acyl-carrier protein (ACP) domains present in the PUFA PKS complex. Activation of the ACP domains by addition of this co-factor is required for the PUFA PKS enzyme complex to function. All of the ACP domains of the PUFA PKS systems identified so far show a high degree of amino acid sequence conservation and, without being bound by theory, the present inventors believe that the PPTase of *Schizochytrium* and other Thraustochytrids will recognize and activate ACP domains from other PUFA PKS systems. As proof of principle that heterologous PPTases and PUFA PKS genes can function together to produce a PUFA product, the present inventors demonstrate herein the use of two different heterologous PPTases with the PUFA PKS genes from *Schizochytrium* to produce a PUFA in a bacterial host cell.

Third, in *Schizochytrium,* the products of the PUFA PKS system are efficiently channeled into both the phospholipids (PL) and triacylglycerols (TAG). The present inventors' data suggest that the PUFA is transferred from the ACP domains of the PKS complex to coenzyme A (CoA). As in other eukaryotic organisms, this acyl-CoA would then serve as the substrate for the various acyl-transferases that form the PL and TAG molecules. In contrast, the data indicate that in bacteria, transfer to CoA does not occur; rather, there is a direct transfer from the ACP domains ofthe PKS complex to the acyl-transferases that form PL. The enzymatic system in *Schizochytrium* that transfers PUFA from ACP to CoA clearly can recognize both DHA and DPA and therefore, the present inventors believe that it is predictable that any PUFA product of the PUFA PKS system (as attached to the PUFA PKS ACP domains) will serve as a substrate.

Therefore, in one embodiment of the present invention, the present inventors propose to alter the genes encoding the components of the PUFA PKS enzyme complex (part 1) while utilizing the endogenous PPTase from *Schizochytrium* or another Thraustochytrid host (part 2) and PUFA-ACP to PUFA-CoA transferase activity and TAG / PL synthesis systems (or other endogenous PUFA ACP to TAG/PL mechanism) (part 3). These methods of the present invention are supported by experimental data, some of which are presented in the Examples section in detail.

First, the present inventors have found that the PUFA PKS system can be transferred between organisms, and that some parts are interchangeable. More particularly, it has been previously shown that the PUFA PKS pathways of the marine bacteria, *Shewanella* SCR2738 (Yazawa, 1996, Lipids 31:S297-300) and *Vibrio marinus* (along with the PPTase from *Shewanella*) (U.S. Patent No. 6,140,486), can be successfully transferred to a heterologous host (i.e., to *E. coli*)*.* Additionally, the degree of structural homology between the subunits of the PUFA PKS enzymes from these two organisms (*Shewanella* SCRC2738 and *Vibrio marinus*) is such that it has been possible to mix and match genes from the two systems (U.S. Patent No. 6,140,486, *supra).* The PUFA end product of the mixed sets of genes varied depending on the origins of the specific gene homologues. At least one open reading frame (*Shewanella's* Orf 7 and its *Vibrio marinus* homologue; see Fig. 13 of U.S. Patent No. 6,140,486; note that the nomenclature for this Orf has changed; it is labeled as Orf 8 in the patent, but was submitted to Genbank as Orf 7, and is now referred to by its GenBank designation) could be associated with determination of whether DHA or EPA would be the product of the composite system. The functional domains of all of the PUFA PKS enzymes identified so far show sequence homology to one another. Similarly, these data indicated that PUFA PKS systems, including those from the marine bacteria, can be transferred to, and will function in, *Schizochytrium* and other Thraustochytrids.

The present inventors have now expressed the PUFA PKS genes (Orfs A, B and C) from *Schizochytrium* in an E. *coli* host and have demonstrated that the cells made DHA and DPA in about the same ratio as the endogenous production of these PUFAs in *Schizochytrium* (see Example 2). Therefore, it has been demonstrated that the recombinant *Schizochytrium* PUFA PKS genes encode a functional PUFA synthesis system. Additionally, all or portions of the *Thraustochytrium* 23B OrfA and OrfC genes have been shown to function in *Schizochytrium* (see Example 6).

Second, the present inventors have previously found that PPTases can activate heterologous PUFA PKS ACP domains. Production of DHA in E. *coli* transformed with the PUFA PKS genes from *Vibrio marinus* occurred only when an appropriate PPTase gene (in this case, from Shewanella SCRC2738) was also present (see U.S. Patent No. 6,140,486, *supra).* This demonstrated that the *Shewanella* PPTase was able to activate the *Vibrio* PUFA PKS ACP domains. Additionally, the present inventors have now demonstrated the activation (pantetheinylation) of ACP domains from *Schizochytrium* Orf A using a PPTase (sfp) from *Bacillus subtilus* (see Example 2). The present inventors have also demonstrated activation (pantetheinylation) of ACP domains from *Schizochytrium* Orf A by a PPTase called Het I from Nostoc (see Example 2). The HetI enzyme was additionally used as the PPTase in the experiments discussed above for the production of DHA and DPA in *E*. *coli* using the recombinant *Schizochytrium* PUFA PKS genes (Example 2).

Third, data indicate that DHA-CoA and DPA-CoA may be metabolic intermediates in the *Schizochytrium* TAG and PL synthesis pathway. Published biochemical data suggest that in bacteria, the newly synthesized PUFAs are transferred directly from the PUFA PKS ACP domains to the phospholipid synthesis enzymes. In contrast, the present inventors' data indicate that in *Schizochytrium,* a eukaryotic organism, there maybe an intermediate between the PUFA on the PUFA PKS ACP domains and the target TAG and PL molecules. The typical carrier of fatty acids in the eukaryotic cytoplasm is CoA. The inventors examined extracts of *Schizochytrium* cells and found significant levels of compounds that co-migrated during HPLC fractionation with authentic standards of DHA-CoA, DPA-CoA, 16:0-CoA and 18:1-CoA. The identity of the putative DHA-CoA and DPA-CoA peaks were confirmed using mass spectroscopy. In contrast, the inventors were not able to detect DHA-CoA in extracts of *Vibrio marinus,* again suggesting that a different mechanism exists in bacteria for transfer of the PUFA to its final target (e.g., direct transfer to PL). The data indicate a mechanism likely exists in *Schizochytrium* for transfer of the newly synthesized PUFA to CoA (probably via a direct transfer from the ACP to CoA). Both TAG and PL synthesis enzymes could then access this PUFA-CoA. The observation that both DHA and DPA CoA are produced suggests that the enzymatic transfer machinery may recognize a range of PUFAs.

Fourth, the present inventors have now created knockouts of Orf A, Orf B, and Orf C in *Schizochytrium* (see Example 3). The knockout strategy relies on the homologous recombination that has been demonstrated to occur in *Schizochytrium* (see U.S. Patent Application Serial No.10/124,807, *supra*)*.* Several strategies can be employed in the design of knockout constructs. The specific strategy used to inactivate these three genes utilized insertion of a Zeocin™ resistance gene coupled to a tubulin promoter (derived from pMON50000, see U.S. Patent Application Serial No. 10/124,807) into a cloned portion of the Orf. The new construct containing the interrupted coding region was then used for the transformation of wild type *Schizochytrium* cells via particle bombardment (see U.S. Patent Application Serial No. 10/124,807). Bombarded cells were spread on plates containing both Zeocin™ and a supply of PUFA (see below). Colonies that grew on these plates were then streaked onto Zeocin™ plates that were not supplemented with PUFAs. Those colonies that required PUFA supplementation for growth were candidates for having had the PUFA PKS Orf inactivated via homologous recombination. In all three cases, this presumption was confirmed by rescuing the knockout by transforming the cells with a full-length genomic DNA clones of the respective *Schizochytrium* Orfs. Furthermore, in some cases, it was found that the Zeocin™ resistance gene had been removed (see Example 5), indicating that the introduced functional gene had integrated into the original site by double homologous recombination (i.e. deleting the resistance marker). One key to the success of this strategy was supplementation of the growth medium with PUFAs. In the present case, an effective means of supplementation was found to be sequestration of the PUFA by mixing with partially methylated beta-cyclodextrin prior to adding to the growth medium (see Example 5). Together, these experiments demonstrate the principle that one of skill in the art, given the guidance provided herein, can inactivate one or more of the PUFA PKS genes in a PUFA PKS-containing microorganism such as *Schizochytrium,* and create a PUFA auxotroph which can then be used for further genetic modification (e.g., by introducing other PKS genes) according to the present invention (e.g., to alter the fatty acid profile of the recombinant organism).

One important element of the genetic modification of the organisms of the present invention is the ability to directly transform a Thraustochytrid genome. In U.S. Application Serial No. 10/124,807, *supra,* transformation of *Schizochytrium* via single crossover homologous recombination and targeted gene replacement via double crossover homologous recombination were demonstrated. As discussed above, the present inventors have now used this technique for homologous recombination to inactivate Orf A, Orf B and OrfC of the PUFA-PKA system in *Schizochytrium.* The resulting mutants are dependent on supplementation of the media with PUFA. Several markers of transformation, promoter elements for high level expression of introduced genes and methods for delivery of exogenous genetic material have been developed and are available. Therefore, the tools are in place for knocking out endogenous PUFA PKS genes in Thraustochytrids and other eukaryotes having similar PUFA PKS systems and replacing them with genes from other organisms (or with modified *Schizochytrium* genes) as proposed above.

In one approach for production of EPA-rich TAG, the PUFA PKS system of *Schizochytrium* can be altered by the addition of heterologous genes encoding a PUFA PKS system whose product is EPA. It is anticipated that the endogenous PPTase will activate the ACP domains of that heterologous PUFA PKS system. Additionally, it is anticipated that the EPA will be converted to EPA-CoA and will readily be incorporated into *Schizochytrium* TAG and PL membranes. In one modification of this approach, techniques can be used to modify the relevant domains of the endogenous *Schizochytrium* system (either by introduction of specific regions of heterologous genes or by mutagenesis of the *Schizochytrium* genes themselves) such that its end product is EPA rather than DHA and DPA. This is an exemplary approach, as this technology can be applied to the production of other PUFA end products and to any eukaryotic microorganism that comprises a PUFA PKS system and that has the ability to efficiently channel the products of the PUFA PKS system into both the phospholipids (PL) and triacylglycerols (TAG). In particular, the invention is applicable to any Thraustochytrid microorganism or any other eukaryote that has an endogenous PUFA PKS system, which is described in detail below by way of example. In addition, the invention is applicable to any suitable host organism, into which the modified genetic material for production of various PUFA profiles as described herein can be transformed. For example, in the Examples, the PUFA PKS system from *Schizochytrium* is transformed into an E. *coli.* Such a transformed organism could then be further modified to alter the PUFA production profile using the methods described herein.

The present invention can make use of genes and nucleic acid sequences which encode proteins or domains from PKS systems other than the PUFA PKS system described herein and in U.S. Patent Application Serial No. 10/124,800, and include genes and nucleic acid sequences from bacterial and non-bacterial PKS systems, including PKS systems of Type II, Type I and modular, described above. Organisms which express each of these types of PKS systems are known in the art and can serve as sources for nucleic acids useful in the genetic modification process of the present invention.

In a preferred embodiment, genes and nucleic acid sequences which encode proteins or domains from PKS systems other than the PUFA PKS system or from other PUFA PKS systems are isolated or derived from organisms which have preferred growth characteristics for production of PUFAs. In particular, it is desirable to be able to culture the genetically modified Thraustochytrid microorganism at temperatures greater than about 15°C, greater than 20°C, greater than 25°C, greater than 30°C, greater than 35°C, greater than 40°C, or in one embodiment, at any temperature between about 20°C and 40°C. Therefore, PKS proteins or domains having functional enzymatic activity at these temperatures are preferred. For example, the present inventors describe herein the use of PKS genes from *Shewanella olleyana* or *Shewanella japonica,* which are marine bacteria that naturally produce EPA and grow at temperatures up to 30°C and 35°C, respectively (see Example 7). PKS proteins or domains from these organisms are examples of proteins and domains that can be mixed with Thraustochytrid PUFA PKS proteins and domains as described herein to produce a genetically modified organism that has a specifically designed or modified PUFA production profile.

In another preferred embodiment, the genes and nucleic acid sequences that encode proteins or domains from a PUFA PKS system that produces one fatty acid profile are used to modify another PUFA PKS system and thereby alter the fatty acid profile of the host. For example, *Thraustochytrium* 23B (ATCC 20892) is significantly different from *Schizochytrium* sp. (ATCC 20888) in its fatty acid profile. *Thraustochytrium* 23B can have DHA:DPA(n-6) ratios as high as 40:1 compared to only 2-3:1 in *Schizochytrium* (ATCC 20888). *Thraustochytrium* 23B can also have higher levels of C20:5(n-3). However, *Schizochytrium* (ATCC 20888) is an excellent oil producer as compared to *Thraustochytrium* 23B. *Schizochytrium* accumulates large quantities of triacylglycerols rich in DHA and docosapentaenoic acid (DPA; 22:5ω6); e.g., 30% DHA + DPA by dry weight. Therefore, the present inventors describe herein the modification of the *Schizochytrium* endogenous PUFA PKS system with *Thraustochytrium* 23B PUFA PKS genes to create a genetically modified *Schizochytrium* with a DHA:DPA profile more similar to *Thraustochytrium* 23B (i.e., a "super-DHA-producer" *Schizochytrium,* wherein the production capabilities of the *Schizochytrium* combine with the DHA:DPA ratio of *Thraustochytrium*)*.*

Therefore, the present invention makes use of genes from Thraustochytrid PUFA PKS systems, and further utilizes gene mixing to extend and/or alter the range of PUFA products to include EPA, DHA, DPA, ARA, GLA, SDA and others. The method to obtain these altered PUFA production profiles includes not only the mixing of genes from various organisms into the Thrasustochytrid PUFA PKS genes, but also various methods of genetically modifying the endogenous Thraustochytrid PUFA PKS genes disclosed herein. Knowledge of the genetic basis and domain structure of the Thraustochytrid PUFA PKS system of the present invention (e.g., described in detail for *Schizochytrium* above) provides a basis for designing novel genetically modified organisms which produce a variety of PUFA profiles. Novel PUFA PKS constructs prepared in microorganisms such as a Thraustochytrid can be isolated and used to transform plants to impart similar PUFA production properties onto the plants.

Any one or more of the endogenous Thraustochytrid PUFA PKS domains can be altered or replaced according to the present invention, provided that the modification produces the desired result (i.e., alteration of the PUFA production profile of the microorganism). Particularly preferred domains to alter or replace include, but are not limited to, any of the domains corresponding to the domains in *Schizochytrium* OrfB or OrfC (β-keto acyl-ACP synthase (KS), acyltransferase (AT), FabA-like β-hydroxy acyl-ACP dehydrase (DH), chain length factor (CLF), enoyl ACP-reductase (ER), an enzyme that catalyzes the synthesis of *trans*-2-acyl-ACP, an enzyme that catalyzes the reversible isomerization of *trans*-2-acyl-ACP to *cis*-3-acyl-ACP, and an enzyme that catalyzes the elongation of *cis*-3-acyl-ACP to *cis*-5-β-keto-acyl-ACP). In one embodiment, preferred domains to alter or replace include, but are not limited to, β-keto acyl-ACP synthase (KS), FabA-like β-hydroxy acyl-ACP dehydrase (DH), and chain length factor (CLF).

In one aspect of the invention, Thraustochytrid PUFA-PKS PUFA production is altered by modifying the CLF (chain length factor) domain. This domain is characteristic of Type II (dissociated enzymes) PKS systems. Its amino acid sequence shows homology to KS (keto synthase pairs) domains, but it lacks the active site cysteine. CLF may function to determine the number of elongation cycles, and hence the chain length, of the end product. In this embodiment of the invention, using the current state of knowledge of FAS and PKS synthesis, a rational strategy for production of ARA by directed modification of the non-bacterial PUFA-PKS system is provided. There is controversy in the literature concerning the function of the CLF in PKS systems (Bisang et al., Nature 401, 502 (1999); Yi et al., J. Am. Chem. Soc. 125, 12708 (2003)) and it is realized that other domains may be involved in determination of the chain length of the end product. However, it is significant that *Schizochytrium* produces both DHA (C22:6,ω-3) and DPA (C22:5, ω-6). In the PUFA-PKS system the *cis* double bonds are introduced during synthesis of the growing carbon chain. Since placement of the ω-3 and ω-6 double bonds occurs early in the synthesis of the molecules, one would not expect that they would affect subsequent end-product chain length determination. Thus, without being bound by theory, the present inventors believe that introduction of a factor (e.g. CLF) that directs synthesis of C20 units (instead of C22 units) into the *Schizochytrium* PUFA-PKS system will result in the production of EPA (C20:5, ω-3) and ARA (C20:4, ω-6). For example, in heterologous systems, one could exploit the CLF by directly substituting a CLF from an EPA producing system (such as one from *Photobacterium,* or preferably from a microorganism with the preferred growth requirements as described below) into the *Schizochytrium* gene set. The fatty acids of the resulting transformants can then be analyzed for alterations in profiles to identify the transformants producing EPA and/or ARA.

By way of example, in this aspect of the invention, one could construct a clone with the CLF of OrfB replaced with a CLF from a C20 PUFA-PKS system. A marker gene could be inserted downstream ofthe coding region. More specifically, one can use the homologous recombination system for transformation of Thraustochytrids as described herein and in detail in U.S. Patent Application Serial No. 10/124,807, *supra.* One can then transform the wild type Thraustochytrid cells (e.g., *Schizochytrium* cells), select for the marker phenotype, and then screen for those that had incorporated the new CLF. Again, one would analyze these transformants for any effects on fatty acid profiles to identify transformants producing EPA and/or ARA. If some factor other than those associated with the CLF is found to influence the chain length of the end product, a similar strategy could be employed to alter those factors.

In another aspect of the invention, modification or substitution of the β-hydroxyacyl-ACP dehydrase/keto synthase pairs is contemplated. During *cis*-vaccenic acid (C18:1, Δ11) synthesis in *E. coli,* creation of the *cis* double bond is believed to depend on a specific DH enzyme, β-hydroxyacyl-ACP dehydrase, the product of the *fabA* gene. This enzyme removes HOH from a β-keto acyl-ACP and leaves a *trans* double bond in the carbon chain. A subset of DH's, FabA-like, possess *cis-trans* isomerase activity (Heath et al.,1996, *supra).* A novel aspect of bacterial and non-bacterial PUFA-PKS systems is the presence of two FabA-like DH domains. Without being bound by theory, the present inventors believe that one or both of these DH domains will possess *cis-trans* isomerase activity (manipulation of the DH domains is discussed in greater detail below).

Another aspect of the unsaturated fatty acid synthesis in *E*. *coli* is the requirement for a particular KS enzyme, β-ketoacyl-ACP synthase, the product of the *fabB* gene. This is the enzyme that carries out condensation of a fatty acid, linked to a cysteine residue at the active site (by a thio-ester bond), with a malonyl-ACP. In the multi-step reaction, CO₂ is released and the linear chain is extended by two carbons. It is believed that only this KS can extend a carbon chain that contains a double bond. This extension occurs only when the double bond is in the *cis* configuration; if it is in the *trans* configuration, the double bond is reduced by enoyl-ACP reductase (ER) prior to elongation (Heath et al., 1996, *supra).* All of the PUFA-PKS systems characterized so far have two KS domains, one of which shows greater homology to the FabB-like KS of *E*. *coli* than the other. Again, without being bound by theory, the present inventors believe that in PUFA-PKS systems, the specificities and interactions of the DH (FabA-like) and KS (FabB-like) enzymatic domains determine the number and placement of *cis* double bonds in the end products. Because the number of 2-carbon elongation reactions is greater than the number of double bonds present in the PUFA-PKS end products, it can be determined that in some extension cycles complete reduction occurs. Thus the DH and KS domains can be used as targets for alteration of the DHA/DPA ratio or ratios of other long chain fatty acids. These can be modified and/or evaluated by introduction of homologous domains from other systems or by mutagenesis of these gene fragments.

In another embodiment, the ER (enoyl-ACP reductase - an enzyme which reduces the trans-double bond in the fatty acyl-ACP resulting in fully saturated carbons) domains can be modified or substituted to change the type of product made by the PKS system. For example, the present inventors know that *Schizochytrium* PUFA-PKS system differs from the previously described bacterial systems in that it has two (rather than one) ER domains. Without being bound by theory, the present inventors believe these ER domains can strongly influence the resulting PKS production product. The resulting PKS product could be changed by separately knocking out the individual domains or by modifying their nucleotide sequence or by substitution of ER domains from other organisms.

In another aspect of the invention, substitution of one of the DH (FabA-like) domains of the PUFA-PKS system for a DH domain that does not posses isomerization activity is contemplated, potentially creating a molecule with a mix of *cis-* and *trans-* double bonds. The current products of the *Schizochytrium* PUFA PKS system are DHA and DPA (C22:5 ω6). If one manipulated the system to produce C20 fatty acids, one would expect the products to be EPA and ARA (C20:4 ω6). This could provide a new source for ARA. One could also substitute domains from related PUFA-PKS systems that produced a different DHA to DPA ratio - for example by using genes from *Thraustochytrium* 23B (the PUFA PKS system of which is identified in U.S. Patent Application Serial No. 10/124,800, *supra).*

Additionally, in one embodiment, one of the ER domains is altered in the Thraustochytrid PUPA PKS system (e.g. by removing or inactivating) to alter the end product profile. Similar strategies could be attempted in a directed manner for each of the distinct domains of the PUFA-PKS proteins using more or less sophisticated approaches. Of course one would not be limited to the manipulation of single domains. Finally, one could extend the approach by mixing domains from the PUFA-PKS system and other PKS or FAS systems (e.g., type I, type II, modular) to create an entire range of new PUFA end products.

It is recognized that many genetic alterations, either random or directed, which one may introduce into a native (endogenous, natural) PKS system, will result in an inactivation of enzymatic functions. Therefore, in order to test for the effects of genetic manipulation of a Thraustochytrid PUFA PKS system in a controlled environment, one could first use a recombinant system in another host, such as *E. coli,* to manipulate various aspects of the system and evaluate the results. For example, the FabB- strain of *E. coli* is incapable of synthesizing unsaturated fatty acids and requires supplementation of the medium with fatty acids that can substitute for its normal unsaturated fatty acids in order to grow (see Metz et al., 2001, *supra).* However, this requirement (for supplementation of the medium) can be removed when the strain is transformed with a functional PUFA-PKS system (i.e. one that produces a PUFA product in the *E. coli* host - see (Metz et al., 2001, *supra,* Figure 2A). The transformed FabB- strain now requires a functional PUFA-PKS system (to produce the unsaturated fatty acids) for growth without supplementation. The key element in this example is that production of a wide range of unsaturated fatty acid will suffice (even unsaturated fatty acid substitutes such as branched chain fatty acids). Therefore, in another preferred embodiment of the invention, one could create a large number of mutations in one or more of the PUFA PKS genes disclosed herein, and then transform the appropriately modified FabB- strain (e.g. create mutations in an expression construct containing an ER domain and transform a FabB- strain having the other essential domains on a separate plasmid - or integrated into the chromosome) and select only for those transformants that grow without supplementation of the medium (i.e., that still possessed an ability to produce a molecule that could complement the FabB- defect).

One test system for genetic modification of a PUFA PKS is exemplified in the Examples section. Briefly, a host microorganism such as *E*. *coli* is transformed with genes encoding a PUFA PKS system including all or a portion of a Thraustochytrid PUFA PKS system (e.g., Orfs A, B and C of *Schizochytrium*) and a gene encoding a phosphopantetheinyl transferases (PPTase), which is required for the attachment of a phosphopantetheine cofactor to produce the active, holo-ACP in the PKS system. The genes encoding the PKS system can be genetically engineered to introduce one or more modifications to the Thraustochytrid PUFA PKS genes and/or to introduce nucleic acids encoding domains from other PKS systems into the Thraustochytrid genes (including genes from non-Thraustochytrid microorganisms and genes from different Thraustochytrid microorganisms). The PUFA PKS system can be expressed in the *E*. *coli* and the PUFA production profile measured. In this manner, potential genetic modifications can be evaluated prior to manipulation of the Thraustochytrid PUFA production organism.

The present invention includes the manipulation of endogenous nucleic acid molecules and/or the use of isolated nucleic acid molecules comprising a nucleic acid sequence from a Thraustochytrid PUFA PKS system or a homologue thereof. In one aspect, the present invention relates to the modification and/or use of a nucleic acid molecule comprising a nucleic acid sequence encoding a domain from a PUFA PKS system having a biological activity of at least one of the following proteins: malonyl-CoA:ACP acyltransferase (MAT), β-keto acyl-ACP synthase (KS), ketoreductase (KR), acyltransferase (AT), FabA-like β-hydroxy acyl-ACP dehydrase (DH), phosphopantetheine transferase, chain length factor (CLF), acyl carrier protein (ACP), enoyl ACP-reductase (ER), an enzyme that catalyzes the synthesis of *trans*-2-acyl-ACP, an enzyme that catalyzes the reversible isomerization of *trans*-2-acyl-ACP to *cis*-3-acyl-ACP, and/or an enzyme that catalyzes the elongation of *cis*-3-acyl-ACP to *cis*-5-β-keto-acyl-ACP. Preferred domains to modify in order to alter the PUFA production profile of a host Thraustochytrid have been discussed previously herein.

The genetic modification of a Thraustochytrid microorganism according to the present invention preferably affects the type, amounts, and/or activity of the PUFAs produced by the microorganism, whether the endogenous PUFA PKS system is genetically modified and/or whether recombinant nucleic acid molecules are introduced into the organism. According to the present invention, to affect an activity of a PUFA PKS system, such as to affect the PUFA production profile, includes any genetic modification in the PUFA PKS system or genes that interact with the PUFA PKS system that causes any detectable or measurable change or modification in any biological activity the PUFA PKS system expressed by the organism as compared to in the absence ofthe genetic modification. According to the present invention, the phrases "PUFA profile", "PUFA expression profile" and "PUFA production profile" can be used interchangeably and describe the overall profile of PUFAs expressed/produced by a microorganism. The PUFA expression profile can include the types of PUFAs expressed by the microorganism, as well as the absolute and relative amounts of the PUFAs produced. Therefore, a PUFA profile can be described in terms of the ratios of PUFAs to one another as produced by the microorganism, in terms of the types of PUFAs produced by the microorganism, and/or in terms of the types and absolute or relative amounts of PUFAs produced by the microorganism.

As discussed above, while the host microorganism can include any eukaryotic microorganism with an endogenous PUFA PKS system and the ability to efficiently channel the products of the PUFA PKS system into both the phospholipids (PL) and triacylglycerols (TAG), the preferred host microorganism is any member of the order Thraustochytriales, including the families Thraustochytriaceae and Labyrinthulaceae. Particularly preferred host cells for use in the present invention could include microorganisms from a genus including, but not limited to: *Thraustochytrium, Japonochytrium, Aplanochytrium, Elina,* and *Schizochytrium* within the Thraustochytriaceae, and *Labyrinthula, Labyrinthuloides,* and *Labyrinthomyxa* within the Labyrinthulaceae. Preferred species within these genera include, but are not limited to: any species within *Labyrinthula,* including *Labrinthula* sp., *Labyrinthula algeriensis, Labyrinthula cienkowskii, Labyrinthula chattonii, Labyrinthula coenocystis, Labyrinthula macrocystis, Labyrinthulamacrocystis atlantica, Labyrinthula macrocystis macrocystis, Labyrinthula magnifica, Labyrinthula minuta, Labyrinthula roscoffensis, Labyrinthula valkanovii, Labyrinthula vitellina, Labyrinthula vitellinapacifica, Labyrinzthula vitellina vitellina, Labyrinthula zopfii*; any *Labyrinthuloides* species, including *Labyrinthuloides* sp., *Labyrinthuloides minuta, Labyrinthuloides schizochytrops*; any *Labyrinthomyxa* species, including *Labyrinthomyxa* sp., *Labyrinthomyxa pohlia, Labyrinthomyxa sauvageaui,* any *Aplanochytrium* species, including *Aplanochytrium* sp. and *Aplanochytrium kerguelensis*; any *Elina* species, including *Elina* sp., *Elina marisalba, Elina sinorifica*; any *Japanochytrium* species, including *Japanochytrium* sp., *Japanochytrium marinum*; any *Schizochytrium* species, including *Schizochytrium* sp., *Schizochytrium aggregatum, Schizochytrium limacinum, Schizochytrium minutum, Schizochytrium octosporum*; and any *Thraustochytrium* species, including *Thraustochytrium* sp., *Thraustochytrium aggregatum, Thraustochytrium arudinzentale, Thraustochytrium aureum, Thraustochytrium benthicola, Thraustochytrium globosum, Thraustochytrium kinnei, Thraustochytrium motivum, Thraustochytrium pachydernum, Thraustochytrium proliferum, Thraustochytrium roseum, Thraustochytrium striatum, Ulkenia* sp., *Ulkenia minuta, Ulkenia profunda, Ulkenia radiate, Ulkenia sarkariana,* and *Ulkenia visurgensis.* Particularly preferred species within these genera include, but are not limited to: any *Schizochytrium* species, including *Schizochytrium aggregatum, Schizochytrium limacinum, Schizochytrium minutum*; any *Thraustochytrium* species (including former *Ulkenia* species such as *U. visurgensis, U. amoeboida, U. sarkariana, U. profunda, U. radiata, U. minuta* and *Ulkenia sp.* BP-5601), and including *Thraustochytrium striatum, Thraustochytrium aureum, Thraustochytrium roseum*; and any *Japonochytrium* species. Particularly preferred strains ofThraustochytriales include, but are not limited to: *Schizochytrium sp.* (S31)(ATCC 20888); *Schizochytrium sp.* (S8)(ATCC 20889); *Schizochytrium sp.* (LC-RM)(ATCC 18915); *Schizochytrium sp.* (SR21); *Schizochytrium aggregatum* (Goldstein et Belsky)(ATCC *28209); Schizochytrium limacinum* (Honda et Yokochi)(IFO 32693); *Thraustochytrium sp.* (23B)(ATCC 20891); *Thraustochytrium striatum* (Schneider)(ATCC 24473); *Thraustochytrium aureum* (Goldstein)(ATCC 34304); *Thraustochytrium roseum* (Goldstein)(ATCC 28210); and *Japonochytrium sp.* (L1)(ATCC 28207).

In one embodiment of the present invention, it is contemplated that a mutagenesis program could be combined with a selective screening process to obtain a Thraustochytrid microorganism with the PUFA production profile of interest. The mutagenesis methods could include, but are not limited to: chemical mutagenesis, gene shuffling, switching regions of the genes encoding specific enzymatic domains, or mutagenesis restricted to specific regions of those genes, as well as other methods.

For example, high throughput mutagenesis methods could be used to influence or optimize production of the desired PUFA profile. Once an effective model system has been developed, one could modify these genes in a high throughput manner. Utilization of these technologies can be envisioned on two levels. First, if a sufficiently selective screen for production of a product of interest (e.g., EPA) can be devised, it could be used to attempt to alter the system to produce this product (e.g., in lieu of, or in concert with, other strategies such as those discussed above). Additionally, if the strategies outlined above resulted in a set of genes that did produce the PUFA profile of interest, the high throughput technologies could then be used to optimize the system. For example, if the introduced domain only functioned at relatively low temperatures, selection methods could be devised to permit removing that limitation.

In one embodiment of the present invention, a genetically modified Thraustochytrid microorganism has an enhanced ability to synthesize desired PUFAs and/or has a newly introduced ability to synthesize a different profile of PUFAs. According to the present invention, "an enhanced ability to synthesize" a product refers to any enhancement, or up-regulation, in a pathway related to the synthesis of the product such that the microorganism produces an increased amount of the product (including any production of a product where there was none before) as compared to the wild-type microorganism, cultured or grown, under the same conditions. Methods to produce such genetically modified organisms have been described in detail above.

As described above, in one embodiment of the present invention, a genetically modified microorganism or plant includes a microorganism or plant which has an enhanced ability to synthesize desired bioactive molecules (products) or which has a newly introduced ability to synthesize specific products (e.g., to synthesize a specific antibiotic). According to the present invention, "an enhanced ability to synthesize" a product refers to any enhancement, or up-regulation, in a pathway related to the synthesis of the product such that the microorganism or plant produces an increased amount of the product (including any production of a product where there was none before) as compared to the wild-type microorganism or plant, cultured or grown, under the same conditions. Methods to produce such genetically modified organisms have been described in detail above.

One embodiment of the present invention is a method to produce desired bioactive molecules (also referred to as products or compounds) by growing or culturing a genetically modified microorganism or plant of the present invention (described in detail above). Such a method includes the step of culturing in a fermentation medium or growing in a suitable environment, such as soil, a microorganism or plant, respectively, that has a genetic modification as described previously herein and in accordance with the present invention. Preferred host cells for genetic modification related to the PUFA PKS system of the invention are described above.

One embodiment of the present invention is a method to produce desired PUFAs by culturing a genetically modified Thraustochytrid microorganism of the present invention (described in detail above). Such a method includes the step of culturing in a fermentation medium and under conditions effective to produce the PUFA(s) a Thraustochytrid microorganism that has a genetic modification as described previously herein and in accordance with the present invention. An appropriate, or effective, medium refers to any medium in which a genetically modified microorganism of the present invention, including Thraustochytrids and other microorganisms, when cultured, is capable of producing the desired PUFA product(s). Such a medium is typically an aqueous medium comprising assimilable carbon, nitrogen and phosphate sources. Such a medium can also include appropriate salts, minerals, metals and other nutrients. Any microorganisms of the present invention can be cultured in conventional fermentation bioreactors. The microorganisms can be cultured by any fermentation process which includes, but is not limited to, batch, fed-batch, cell recycle, and continuous fermentation. Preferred growth conditions for Thraustochytrid microorganisms according to the present invention are well known in the art and are described in detail, for example, in U.S. Patent No. 5,130,242, U.S. Patent No. 5,340,742, and U.S. Patent No. 5,698,244, each of which is incorporated herein by reference in its entirety.

In one embodiment, the genetically modified microorganism is cultured at a temperature of greater than about 15 °C, and in another embodiment, greater than about 20°C, and in another embodiment, greater than about 25°C, and in another embodiment, greater than about 30°C, and in another embodiment, greater than about 35°C, and in another embodiment, greater than about 40°C, and in one embodiment, at any temperature between about 20°C and 40°C.

The desired PUFA(s) and/or other bioactive molecules produced by the genetically modified microorganism can be recovered from the fermentation medium using conventional separation and purification techniques. For example, the fermentation medium can be filtered or centrifuged to remove microorganisms, cell debris and other particulate matter, and the product can be recovered from the cell-free supernatant by conventional methods, such as, for example, ion exchange, chromatography, extraction, solvent extraction, phase separation, membrane separation, electrodialysis, reverse osmosis, distillation, chemical derivatization and crystallization. Alternatively, microorganisms producing the PUFA(s), or extracts and various fractions thereof, can be used without removal of the microorganism components from the product.

Preferably, a genetically modified Thraustochytrid microorganism of the invention produces one or more polyunsaturated fatty acids including, but not limited to, EPA (C20:5, ω-3), DHA (C22:6, ω-3), DPA (C22:5, ω-6), ARA (C20:4, ω-6), GLA (C18:3, n-6), and SDA (C18:4, n-3)). In one preferred embodiment, a *Schizochytrium* that, in wild-type form, produces high levels of DHA and DPA, is genetically modified according to the invention to produce high levels of EPA. As discussed above, one advantage of using genetically modified Thraustochytrid microorganisms to produce PUFAs is that the PUFAs are directly incorporated into both the phospholipids (PL) and triacylglycerides (TAG).

Preferably; PUFAs are produced in an amount that is greater than about 5% of the dry weight of the microorganism, and in one aspect, in an amount that is greater than 6%, and in another aspect, in an amount that is greater than 7%, and in another aspect, in an amount that is greater than 8%, and in another aspect, in an amount that is greater than 9%, and in another aspect, in an amount that is greater than 10%, and so on in whole integer percentages, up to greater than 90% dry weight of the microorganism (e.g., 15%, 20%, 30%, 40%, 50%, and any percentage in between).

In the method for production of desired bioactive compounds of the present invention, a genetically modified plant is cultured in a fermentation medium or grown in a suitable medium such as soil. An appropriate, or effective, fermentation medium has been discussed in detail above. A suitable growth medium for higher plants includes any growth medium for plants, including, but not limited to, soil, sand, any other particulate media that support root growth (e.g. vermiculite, perlite, etc.) or hydroponic culture, as well as suitable light, water and nutritional supplements which optimize the growth of the higher plant. The genetically modified plants of the present invention are engineered to produce significant quantities of the desired product through the activity of the PKS system that is genetically modified according to the present invention. The compounds can be recovered through purification processes which extract the compounds from the plant. In a preferred embodiment, the compound is recovered by harvesting the plant. In this embodiment, the plant can be consumed in its natural state or further processed into consumable products.

Many genetic modifications useful for producing bioactive molecules will be apparent to those of skill in the art, given the present disclosure, and various other modifications have been discussed previously herein. The present invention contemplates any genetic modification related to a PUFA PKS system as described herein which results in the production of a desired bioactive molecule.

Bioactive molecules, according to the present invention, include any molecules (compounds, products, etc.) that have a biological activity, and that can be produced by a PKS system that comprises at least one amino acid sequence having a biological activity of at least one functional domain of a non-bacterial PUFA PKS system as described herein. Such bioactive molecules can include, but are not limited to: a polyunsaturated fatty acid (PUFA), an anti-inflammatory formulation, a chemotherapeutic agent, an active excipient, an osteoporosis drug, an anti-depressant, an anti-convulsant, an anti*-Heliobactor pylori* drug, a drug for treatment of neurodegenerative disease, a drug for treatment of degenerative liver disease, an antibiotic, and a cholesterol lowering formulation. One advantage of the non-bacterial PUFA PKS system of the present invention is the ability of such a system to introduce carbon-carbon double bonds in the *cis* configuration, and molecules including a double bond at every third carbon. This ability can be utilized to produce a variety of compounds.

Preferably, bioactive compounds of interest are produced by the genetically modified microorganism in an amount that is greater than about 0.05%, and preferably greater than about 0.1 %, and more preferably greater than about 0.25%, and more preferably greater than about 0.5%, and more preferably greater than about 0.75%, and more preferably greater than about 1%, and more preferably greater than about 2.5%, and more preferably greater than about 5%, and more preferably greater than about 10%, and more preferably greater than about 15%, and even more preferably greater than about 20% of the dry weight of the microorganism. For lipid compounds, preferably, such compounds are produced in an amount that is greater than about 5% of the dry weight of the microorganism. For other bioactive compounds, such as antibiotics or compounds that are synthesized in smaller amounts, those strains possessing such compounds at of the dry weight of the microorganism are identified as predictably containing a novel PKS system of the type described above. In some embodiments, particular bioactive molecules (compounds) are secreted by the microorganism, rather than accumulating. Therefore, such bioactive molecules are generally recovered from the culture medium and the concentration of molecule produced will vary depending on the microorganism and the size of the culture.

One embodiment ofthe present invention relates to a method to modify an endproduct containing at least one fatty acid, comprising adding to the endproduct an oil produced by a recombinant host cell that expresses at least one recombinant nucleic acid molecule comprising a nucleic acid sequence encoding at least one biologically active domain of a PUFA PKS system. The PUFA PKS system includes any suitable bacterial or non-bacterial PUFA PKS system described herein, including the PUFA PKS systems from *Thraustochytrium* and *Schizochytrium,* or any PUFA PKS system from bacteria that normally (i.e., under normal or natural conditions) are capable of growing and producing PUFAs at temperatures above 22°C, such as *Shewanella olleyana* or *Shewanella japonica.*

Preferably, the endproduct is selected from the group consisting of a food, a dietary supplement, a pharmaceutical formulation, a humanized animal milk, and an infant formula. Suitable pharmaceutical formulations include, but are not limited to, an anti-inflammatory formulation, a chemotherapeutic agent, an active excipient, an osteoporosis drug, an anti-depressant, an anti-convulsant, an anti-*Heliobactor pylori* drug, a drug for treatment of neurodegenerative disease, a drug for treatment of degenerative liver disease, an antibiotic, and a cholesterol lowering formulation, In one embodiment, the endproduct is used to treat a condition selected from the group consisting of: chronic inflammation, acute inflammation, gastrointestinal disorder, cancer, cachexia, cardiac restenosis, neurodegenerative disorder, degenerative disorder of the liver, blood lipid disorder, osteoporosis, osteoarthritis, autoimmune disease, preeclampsia, preterm birth, age related maculopathy, pulmonary disorder, and peroxisomal disorder.

Suitable food products include, but are not limited to, fine bakery wares, bread and rolls, breakfast cereals, processed and unprocessed cheese, condiments (ketchup, mayonnaise, etc.), dairy products (milk, yogurt); puddings and gelatin desserts, carbonated drinks, teas, powdered beverage mixes, processed fish products, fruit-based drinks, chewing gum, hard confectionery, frozen dairy products, processed meat products, nut and nut-based spreads, pasta, processed poultry products, gravies and sauces, potato chips and other chips or crisps, chocolate and other confectionery, soups and soup mixes, soya based products (milks, drinks, creams, whiteners), vegetable oil-based spreads, and vegetable-based drinks.

Yet another embodiment of the present invention relates to a method to produce a humanized animal milk. This method includes the steps of genetically modifying milk-producing cells of a milk-producing animal with at least one recombinant nucleic acid molecule comprising a nucleic acid sequence encoding at least one biologically active domain of a PUFA PKS system as described herein.

Methods to genetically modify a host cell and to produce a genetically modified non-human, milk-producing animal, are known in the art. Examples of host animals to modify include cattle, sheep, pigs, goats, yaks, etc., which are amenable to genetic manipulation and cloning for rapid expansion of a transgene expressing population. For animals, PKS-like transgenes can be adapted for expression in target organelles, tissues and body fluids through modification of the gene regulatory regions. Of particular interest is the production of PUFAs in the breast milk of the host animal.

The following examples are provided for the purpose of illustration and are not intended to limit the scope of the present invention.

### Examples

### Example 1

The following example, from U.S. Patent Application No. 10/124,800, describes the use of the screening process of the present invention to identify other non-bacterial organisms comprising a PUFA PKS system according to the present invention.

*Thraustochytrium* sp. 23B (ATCC 20892) was cultured as described in detail herein.

A frozen vial of *Thraustochytrium* sp. 23B (ATCC 20892) was used to inoculate a 250 mL shake flask containing 50 mL of RCA medium. The culture was shaken on a shaker table (200 rpm) for 72 hr at 25°C. RCA medium contains the following:

| RCA Medium | |
|---|---|
| Deionized water | 1000 mL |
| Reef Crystals® sea salts | 40 g/L |
| Glucose | 20 g/L |
| Monosodium glutamate (MSG) | 20 g/L |
| Yeast extract | 1 g/L |
| PII metals* | 5 mL/L |
| Vitamin mix* | 1 mL/L |
| pH | 7.0 |

| | |
|---|---|
| *PII metal mix and vitamin mix are same as those outlined in U.S. Patent No. 5,130,742, incorporated herein by reference in its entirety. | |

25 mL of the 72 hr old culture was then used to inoculate another 250 mL shake flask containing 50 mL of low nitrogen RCA medium (10 g/L MSG instead of 20 g/L) and the other 25 mL of culture was used to inoculate a 250 mL shake flask containing 175 mL of low-nitrogen RCA medium. The two flasks were then placed on a shaker table (200 rpm) for 72 hr at 25°C. The cells were then harvested via centrifugation and dried by lyophilization. The dried cells were analyzed for fat content and fatty acid profile and content using standard gas chromatograph procedures.

The screening results for *Thraustochytrium* 23B under low oxygen conditions relative to high oxygen conditions were as follows:

| | |
|---|---|
| Did DHA as % FAME increase? | Yes (38->44%) |
| C14:0 + C16:0 + C16:1 greater than about 40% TFA? | Yes (44%) |
| No C18:3(n-3) or C18:3(n-6)? | Yes (0%) |
| Did fat content increase? | Yes (2-fold increase) |
| Did DHA (or other HUFF content increase)? | Yes (2.3-fold increase) |

The results, especially the significant increase in DHA content (as % FAME) under low oxygen conditions, conditions, strongly indicates the presence of a PUFA producing PKS system in this strain of *Thraustochytrium.*

In order to provide additional data confirming the presence of a PUFA PKS system, a Southern blot of *Thraustochytrium* 23B was conducted using PKS probes from *Schizochytrium* strain 20888, a strain which has already been determined to contain a PUFA producing PKS system (i.e., SEQ ID Nos:1-32 described above). Fragments of *Thraustochytrium* 23B genomic DNA which are homologous to hybridization probes from PKS PUFA synthesis genes were detected using the Southern blot technique. *Thraustochytrium* 23B genomic DNA was digested with either *Cla*I or *Kpn*I restriction endonucleases, separated by agarose gel electrophoresis (0.7% agarose, in standard tris-acetate-EDTA buffer), and blotted to a Schleicher &Schuell Nytran Supercharge membrane by capillary transfer. Two digoxigenin labeled hybridization probes were used - one specific for the enoyl-ACP reductase (ER) region of *Schizochytrium* PKS Orf B (nucleotides 5012-5511 of Orf B; SEQ ID NO.3), and the other specific for a conserved region at the beginning of *Schizochytrium* PKS Orf C (nucleotides 76-549 of OrfC; SEQ ID NO:5).

The OrfB-ER probe detected an approximately 13kb ClaI fragment and an approximately 3.6 kb KpnI fragment in the *Thraustochytrium* 23B genomic DNA. The OrfC probe detected an approximately 7.5 kb ClaI fragment and an approximately 4.6 kb KpnI fragment in the *Thraustochytrium* 23B genomic DNA.

Finally, a recombinant genomic library, consisting of DNA fragments from *Thraustochytrium* 23B genomic DNA inserted into vector lambda FIX II (Stratagene), was screened using digoxigenin labeled probes corresponding to the following segments of *Schizochytrium* 20888 PUFA-PKS genes: nucleotides 7385-7879 ofOrfA (SEQ ID NO:1), nucleotides 5012-5511 of Orf B (SEQ ID NO:3), and nucleotides 76-549 of Orf C (SEQ ID NO:5). Each of these probes detected positive plaques from the *Thraustochytrium* 23B library, indicating extensive homology between the *Schizochytrium* PUFA-PKS genes and the genes of *Thraustochytrium* 23B.

These results demonstrate that *Thraustochytrium* 23B genomic DNA contains sequences that are homologous to PKS genes from *Schizochytrium* 20888.

### Example 2

The following example demonstrates that *Schizochytrium* Orfs A, B and C encode a functional DHA/DPA synthesis enzyme via functional expression in E. *coli.*

### General preparation of E. coli transformants

The three genes encoding the *Schizochytrium* PUFA PKS system that produces DHA and DPA in *Schizochytrium* (Orfs A, B & C; SEQ ID NO:1, SEQ ID NO:3 and SEQ ID NO:5, respectively) were cloned into a single *E. coli* expression vector (derived from pET2 1 c (Novagen)). The genes are transcribed as a single message (by the T7 RNA-polymerase), and a ribosome-binding site cloned in front of each of the genes initiates translation. Modification of the Orf B coding sequence was needed to obtain production of a full-length Orf B protein in E. *coli* (see below). An accessory gene, encoding a PPTase (see below) was cloned into a second plasmid (derived from pACYC184, New England Biolabs).

### OrfB

The Orf B gene is predicted to encode a protein with a mass of ~224 kDa. Initial attempts at expression of the gene in E. *coli* resulted in accumulation of a protein with an apparent molecular mass of ~165 kDa (as judged by comparison to proteins of known mass during SDS-PAGE). Examination of the Orf B nucleotide sequence revealed a region containing 15 sequential serine codons - all of them being the TCT codon. The genetic code contains 6 different serine codons, and three of these are used frequently in E. *coli.* The present inventors used four overlapping oligonucleotides in combination with a polymerase chain reaction protocol to resynthesize a small portion of the OrfB gene (a ~195 base pair, BspHI to SacII restriction enzyme fragment) that contained the serine codon repeat region. In the synthetic OrfB fragment, a random mixture of the 3 serine codons commonly used by *E. coli* was used, and some other potentially problematic codons were changed as well (i.e., other codons rarely used by *E. coli).* The BspHI to SacII fragment present in the original Orf B was replaced by the resynthesized fragment (to yield OrfB*) and the modified gene was cloned into the relevant expression vectors. The modified OrfB * still encodes the amino acid sequence of SEQ ID NO:4. Expression of the modified OrfB* clone in E. *coli* resulted in the appearance of a ~224 kDa protein, indicating that the full-length product of OrfB was produced. The sequence of the resynthesized OrfB* BspHI to SacII fragment is shown in SEQ ID NO:80. Referring to SEQ ID NO:80, the nucleotide sequence of the resynthesized BspHI to SacII region of Orf B is shown. The BspHI restriction site and the SacII restriction site are identified. The BspHI site starts at nucleotide 4415 of the Orf B CDS (SEQ ID NO:3) (note: there are a total of three BspHI sites in the Orf B CDS, while the SacII site is unique). The sequence ofthe unmodified OrfB CDS is given in GenBank Accession number AF378328 and in SEQ ID NO:3.

### PPTase

The ACP domains of the Orf A protein (SEQ ID NO:2 in *Schizochytrium*) must be activated by addition of phosphopantetheine group in order to function. The enzymes that catalyze this general type of reaction are called phosphopantetheine transferases (PPTases). *E. coli* contains two endogenous PPTases, but it was anticipated that they would not recognize the Orf A ACP domains from *Schizochytrium.* This was confirmed by expressing Orfs A, B* (see above) and C in E. *coli* without an additional PPTase. In this transformant, no DHA production was detected. The inventors tested two heterologous PPTases in the E. *coli* PUFA PKS expression system: (1) sfp (derived from *Bacillus subtilis*) and (2) Het I (from the cyanobacterium *Nostoc* strain 7120).

The sfp PPTase has been well characterized and is widely used due to its ability to recognize a broad range of substrates. Based on published sequence information (Nakana, et al., 1992, Molecular and General Genetics 232: 313-321), an expression vector for sfp was built by cloning the coding region, along with defined up- and downstream flanking DNA sequences, into a pACYC-184 cloning vector. The oligonucleotides:
CGGGGTACCCGGGAGCCGCCTTGGCTTTGT (forward; SEQ ID NO:73); and
AAACTGCAGCCCGGGTCCAGCTGGCAGGCACCCTG (reverse; SEQ ID NO:74),
were used to amplify the region of interest from genomic *B. subtilus* DNA. Convenient restriction enzyme sites were included in the oligonucleotides to facilitate cloning in an intermediate, high copy number vector and finally into the EcoRV site of pACYC184 to create the plasmid: pBR301. Examination of extracts of E. *coli* transformed with this plasmid revealed the presence of a novel protein with the mobility expected for sfp. Co-expression of the sfp construct in cells expressing the Orf A, B*, C proteins, under certain conditions, resulted in DHA production. This experiment demonstrated that sfp was able to activate the *Schizochytrium* Orf A ACP domains. In addition, the regulatory elements associated with the sfp gene were used to create an expression cassette into which other genes could be inserted. Specifically, the sfp coding region (along with three nucleotides immediately upstream of the ATG) in pBR301 was replaced with a 53 base pair section of DNA designed so that it contains several unique (for this construct) restriction enzyme sites. The initial restriction enzyme site in this region is NdeI (CATATG; SEQ ID NO:79). The ATG sequence embedded in this site is utilized as the initiation methionine codon for introduced genes. The additional restriction sites (BglLL, NotI, SmaI, PmelI, HindIII, SpeI and XhoI) were included to facilitate the cloning process. The functionality of this expression vector cassette was tested by using PCR to generate a version of sfp with a NdeI site at the 5' end and an XhoI site ate the 3' end. This fragment was cloned into the expression cassette and transferred into E. *coli* along with the Orf A, B* and C expression vector. Under appropriate conditions, these cells accumulated DHA, demonstrating that a functional sfp had been produced.

To the present inventors' knowledge, Het I has not been tested previously in a heterologous situation. Het I is present in a cluster of genes in Nostoc known to be responsible for the synthesis of long chain hydroxy-fatty acids that are a component of a glyco-lipid layer present in heterocysts of that organism. The present inventors, without being bound by theory, believe that Het I activates the ACP domains of a protein, Hgl E, present in that cluster. The two ACP domains of Hgl E have a high degree of sequence homology to the ACP domains found in *Schizochytrium* Orf A. The endogenous start codon of Het I has not been identified (there is no methionine present in the putative protein). There are several potential alternative start codons (e.g., TTG and ATT) near the 5' end of the open reading frame. The sequence of the region of Nostoc DNA encoding the HetI gene is shown in SEQ ID NO:81. SEQ ID NO:82 represents the amino acid sequence encoded by SEQ ID NO:81. Referring to SEQ ID NO:81, limit to the upstream coding region indicated by the inframe nonsense triplet (TAA) at positions 1-3 of SEQ ID NO:81 and ends with the stop codon (TGA) at positions 715-717 of SEQ ID NO:81. No methionine codons (ATG) are present in the sequence. Potential alternative initiation codons are: 3 TTG codons (positions 4-6, 7-9 and 49-51 of SEQ ID NO:81), ATT (positions 76-78 of SEQ ID NO:81) and GTG (positions 235-237 of SEQ ID NO:81). A HetI expression construct was made by using PCR to replace the furthest 5' potential alternative start codon (TTG) with a methionine codon (ATG, as part of the above described NdeI restriction enzyme recognition site), and introducing an XhoI site at the 3' end of the coding sequence. The modified HetI coding sequence was then inserted into the NdeI and XhoI sites of the pACYC184 vector construct containing the sfp regulatory elements. Expression of this Het I construct in E. *coli* resulted in the appearance of a new protein of the size expected from the sequence data. Co-expression of Het I with *Schizochytium* Orfs A, B*, C in E. *coli* under several conditions resulted in the accumulation of DHA and DPA in those cells. In all of the experiments in which sfp and Het I were compared, more DHA and DPA accumulated in the cells containing the Het I construct than in cells containing the sfp construct.

### Production of DHA and DPA in E. coli transformants

The two plasmids encoding: (1) the *Schizochytrium* PUFA PKS genes (Orfs A, B* and C) and (2) the PPTase (from sfp or from Het I) were transformed into *E. coli* strain BL21 which contains an inducible T7 RNA polymerase gene. Synthesis of the *Schizochytrium* proteins was induced by addition of IPTG to the medium, while PPTase expression was controlled by a separate regulatory element (see above). Cells were grown under various defined conditions and using either of the two heterologous PPTase genes. The cells were harvested and the fatty acids were converted to methyl-esters (FAME) and analyzed using gas-liquid chromatography.

Under several conditions, DHA and DPA were detected in *E. coli* cells expressing the *Schizochytrium* PUFA PKS genes, plus either of the two heterologous PPTases. No DHA or DPA was detected in FAMEs prepared from control cells (i.e., cells transformed with a plasmid lacking one of the Orfs). The ratio of DHA to DPA observed in *E. coli* approximates that of the endogenous DHA and DPA production observed in *Schizochytrium.* The highest level of PUFA (DHA plus DPA), representing ∼17% of the total FAME, was found in cells grown at 32°C in 765 medium (recipe available from the American Type Culture Collection) supplemented with 10% (by weight) glycerol. Note that PUFA accumulation was also observed when cells were grown in Luria Broth supplemented with 5 or 10 % glycerol, and when grown at 20°C. Selection for the presence of the respective plasmids was maintained by inclusion of the appropriate antibiotics during the growth and IPTG (to a final concentration of 0.5 mM) was used to induce expression of Orfs A, B* and C.

Fig. 4 shows an example chromatogram from gas-liquid chromatographic analysis of FAMEs derived from control cells and from cells expressing the *Schizochytrium* PUFA PKS genes plus a PPTase (in this case Het I). Identity of the labeled FAMEs has been confirmed using mass spectroscopy.

### Example 3

The following example shows demonstrates that genes encoding the *Schizochytrium* PUFA PKS enzyme complex can be selectively inactivated (knocked out), and that it is a lethal phenotype unless the medium is supplemented with polyunsaturated fatty acids.

Homologous recombination has been demonstrated in *Schizochytrium* (see copending U.S. Patent Application Serial No. 10/124,807, incorporated herein by reference in its entirety). A plasmid designed to inactivate *Schizochytrium* Orf A (SEQ ID NO:1) was made by inserting a Zeocin™ resistance marker into the Sma I site of a clone containing the Orf A coding sequence. The Zeocin™ resistance marker was obtained from the plasmid pMON50000 - expression of the Zeocin™ resistance gene is driven by a *Schizochytrium* derived tubulin promoter element (see U.S. Patent Application Serial No. 10/124,807, *ibid.*)*.* The knock-out construct thus consists of: 5' *Schizochytrium* Orf A coding sequence, the tub-Zeocin™ resistance element and 3' *Schizochytrium* Orf A coding sequence, all cloned into pBluescript II SK (+) vector (Stratagene).

The plasmid was introduced into *Schizochytrium* cells by particle bombardment and transformants were selected on plates containing Zeocin™ and supplemented with polyunsaturated fatty acids (PUFA) (see Example 4). Colonies that grew on the Zeocin™ plus PUFA plates were tested for ability to grow on plates without the PUFA supplementation and several were found that required the PUFA. These PUFA auxotrophs are putative Orf A knockouts. Northern blot analysis ofRNA extracted from several of these mutants confirmed that a full-length Orf A message was not produced in these mutants.

These experiments demonstrate that a *Schizochytrium* gene (e.g., Orf A) can be inactivated via homologous recombination, that inactivation of Orf A results in a lethal phenotype, and that those mutants can be rescued by supplementation of the media with PUFA.

Similar sets of experiments directed to the inactivation of *Schizochytrium* Orf B (SEQ ID NO:3) and Orf C (SEQ ID NO:5) have yielded similar results. That is, Orf B and Orf C can be individually inactivated by homologous recombination and those cells require PUFA supplementation for growth.

### Example 4

The following example shows that PUFA auxotrophs can be maintained on medium supplemented with EPA, demonstrating that EPA can substitute for DHA in *Schizochytrium.*

As indicated in Example 3, *Schizochytrium* cells in which the PUFA PKS complex has been inactivated required supplementation with PUFA to survive. Aside from demonstrating that *Schizochytrium* is dependent on the products of this system for growth, this experimental system permits the testing of various fatty acids for their ability to rescue the mutants. It was discovered that the mutant cells (in which any of the three genes have been inactivated) grew as well on media supplemented with EPA as they did on media supplemented with DHA. This result indicates that, if the endogenous PUFA PKS complex which produces DHA were replaced with one whose product was EPA, the cells would be viable. Additionally, these mutant cells could be rescued by supplementation with either ARA or GLA, demonstrating the feasibility of producing genetically modified *Schizochytrium* that produce these products. It is noted that a preferred method for supplementation with PUFAs involves combining the free fatty acids with partially methylated beta-cyclodextrin prior to addition of the PUFAs to the medium.

### Example 5

The following example shows that inactivated PUFA genes can be replaced at the same site with active forms of the genes in order to restore PUFA synthesis.

Double homologous recombination at the acetolactate synthase gene site has been demonstrated in *Schizochytrium* (see U.S. Patent Application Serial No. 10/124,807, *supra*). The present inventors tested this concept for replacement of the *Schizochytrium* PUFA PKS genes by transformation of a *Schizochytrium* Orf A knockout strain (described in Example 2) with a full-length *Schizochytrium* Orf A genomic clone. The transformants were selected by their ability to grow on media without supplemental PUFAs. These PUFA prototrophs were then tested for resistance to Zeocin™ and several were found that were sensitive to the antibiotic. These results indicate that the introduced *Schizochytrium* Orf A has replaced the Zeocin™ resistance gene in the knockout strain via double homologous recombination. This experiment demonstrates the proof of concept for gene replacement within the PUFA PKS genes. Similar experiments for *Schizochytrium* Orf B and Orf C knock-outs have given identical results.

### Example 6

This example shows that all or some portions of the *Thraustochytrium* 23B PUFA PKS genes can function in *Schizochytrium.*

As described in U.S Patent Application Serial No. 10/124,800 *(supra),* the DHA-producing protist *Thraustochytrium* 23B (*Th.* 23B) has been shown to contain orfA, orfB, and orfC homologs. Complete genomic clones of the three *Th.* 23B genes were used to transform the *Schizochytrium* strain containing the cognate orf "knock-out". Direct selection for complemented transformants was carried out in the absence of PUFA supplementation. By this method, it was shown that the *Th.* 23B orfA and orfC genes could complement the *Schizochytrium* orfA and orfC knock-out strains, respectively, to PUFA prototrophy. Complemented transformants were found that either retained or lost Zeocin™ resistance (the marker inserted into the *Schizochytrium* genes thereby defining the knock-outs). The Zeocin™-resistant complemented transformants are likely to have arisen by a single cross-over integration of the entire *Thraustochytrium* gene into the *Schizochytrium* genome outside of the respective orf region. This result suggests that the entire *Thraustochytrium* gene is functioning in *Schizochytrium.* The Zeocin™-sensitive complemented transformants are likely to have arisen by double cross-over events in which portions (or conceivably all) of the *Thraustochytrium* genes functionally replaced the cognate regions of the *Schizochytrium* genes that had contained the disruptive Zeocin™ resistance marker. This result suggests that a fraction of the *Thraustochytrium* gene is functioning in *Schizochytrium.*

### Example 7

The following example shows that certain EPA-producing bacteria contain PUFA PKS-like genes that appear to be suitable for modification of *Schizochytrium.*

Two EPA-producing marine bacterial strains of the genus *Shewanella* have been shown to grow at temperatures typical *of Schizochytrium* fermentations and to possess PUFA PKS-like genes. *Shewanella olleyana* (Australian Collection of Antarctic Microorganisms (ACAM) strain number 644; Skerratt et al., Int. J. Syst. Evol. Microbiol 52, 2101 (2002)) produces EPA and grows up to 30°C. *Shewanella japonica* (American Type Culture Collection (ATCC) strain number BAA-316; Ivanova et al., Int. J. Syst. Evol. Microbiol. 51, 1027 (2001)) produces EPA and grows up to 35°C.

To identify and isolate the PUFA-PKS genes from these bacterial strains, degenerate PCR primer pairs for the KS-MAT region of bacterial orf5/pfaA genes and the DH-DH region of bacterial orf7/pfaC genes were designed based on published gene sequences for *Shewanella* SCRC-273 8, *Shewanella oneidensis* MR-1; *Shewanella* sp. GA-22; *Photobacter profundum,* and *Moritella marina* (see discussion above). Specifically, the primers and PCR conditions were designed as follows:
Primers for the KS/AT region; based on the following published sequences: *Shewanella* sp. SCRC-2738; *Shewanella oneidensis* MR-1; *Photobacter profundum*; *Moritella marina*:

| | |
|---|---|
| **prRZ23** | GGYATGMTGRTTGGTGAAGG (forward; SEQ ID NO:69) |
| **prRZ24** | TRTTSASRTAYTGYGAACCTTG (reverse; SEQ ID NO:70) |

Primers for the DH region; based on the following published sequences: *Shewanella* sp. GA-22; *Shewanella* sp. SCRC-2738; *Photobacter profundum*; *Moritella marina*:

| | |
|---|---|
| **prRZ28** | ATGKCNGAAGGTTGTGGCCA (forward; SEQ ID NO:71) |
| **prRZ29** | CCWGARATRAAGCCRTTDGGTTG (reverse; SEQ ID NO:72) |

The PCR conditions (with bacterial chromosomal DNA as templates) were as follows:
Reaction Mixture:
0.2 µM dNTPs
0.1 µM each primer
8% DMSO
250 ng chromosomal DNA
2.5U Herculase® DNA polymerase (Stratagene)
1X Herculase® buffer
50µL total volume

PCR Protocol: (1) 98°C for 3 min.; (2) 98°C for 40 sec.; (3) 56°C for 30 sec.; (4) 72°C for 90 sec.; (5) Repeat steps 2-4 for 29 cycles; (6) 72°C for 10 min.; (7) Hold at 6°C.

For both primer pairs, PCR gave distinct products with expected sizes using chromosomal DNA templates from either *Shewanella olleyana* or *Shewanella japonica.* The four respective PCR products were cloned into pCR-BLUNT II-TOPO (Invitrogen) and insert sequences were determined using the M13 forward and reverse primers. In all cases, the DNA sequences thus obtained were highly homologous to known bacterial PUFA PKS gene regions.

The DNA sequences obtained from the bacterial PCR products were compared with known sequences and with PUFA PKS genes from *Schizochytrium* ATCC 20888 in a standard Blastx search (BLAST parameters: Low Complexity filter: On; Matrix: BLOSUM62; Word Size: 3; Gap Costs: Existance11, Extension 1 (BLAST described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402, incorporated herein by reference in its entirety)).

At the amino acid level, the sequences with the greatest degree of homology to the *Shewanella olleyana* ACAM644 ketoacyl synthase/acyl transferase (KS-AT) deduced amino acid sequence encoded by SEQ ID NO:76 were: *Photobacter profundum* pfaA (identity = 70%; positives = 81%); *Shewanella oneidensis* MR-1 "multi-domain β-ketoacyl synthase" (identity = 66%; positives = 77%); and *Moritella marina* ORF8 (identity = 56%; positives = 71%). The *Schizochytrium* sp. ATCC20888 orfA was 41% identical and 56% positive to the deduced amino acid sequence encoded by SEQ ID NO:76.

At the amino acid level, the sequences with the greatest degree of homology to the *Shewanella japonica* ATCC BAA-316 ketoacyl synthase/acyl transferase (KS-AT) deduced amino acid sequence encoded by SEQ ID NO:78 were: *Shewanella oneidensis* MR-1 "multi-domain β-ketoacyl synthase" (identity = 67%; positives = 79%); *Shewanella* sp. SCRC-2738 orf5 (identity = 69%; positives = 77%); and *Moritella marina* ORF8 (identity = 56%; positives = 70%). The *Schizochytrium* sp. ATCC20888 orfA was 41% identical and 55% positive to the deduced amino acid sequence encoded by SEQ ID NO:78.

At the amino acid level, the sequences with the greatest degree of homology to the *Shewanella olleyana* ACAM644 dehydrogenase (DH) deduced amino acid sequence encoded by SEQ ID NO:75 were: *Shewanella* sp. SCRC-2738 orf7 (identity = 77%; positives = 86%); *Photobacter profundum* pfaC (identity=72%; positives = 81%); and *Shewanella oneidensis* MR-1 "multi-domain β-ketoacyl synthase" (identity = 75%; positives = 83%). The *Schizochytrium* sp. ATCC20888 orfC was 26% identical and 42% positive to the deduced amino acid sequence encoded by SEQ ID NO:75.

At the amino acid level, the sequences with the greatest degree of homology to the *Shewanella japonica* ATCC BAA-316 dehydrogenase (DH) deduced amino acid sequence encoded by SEQ ID NO:77 were: *Shewanella* sp. SCRC-2738 orf7 (identity= 77%; positives = 86%); *Photobacter profundum* pfaC (identity = 73%; positives = 83%) and *Shewanella oneidensis* MR-1 "multi-domain β-ketoacyl synthase" (identity = 74%; positives = 81%). The *Schizochytrium* sp. ATCC20888 orfC was 27% identical and 42% positive to the deduced amino acid sequence encoded by SEQ ID NO:77.

It is expected that the PUFA PKS gene sets from these two *Shewanella* strains will provide beneficial sources of whole genes or individual domains for the modification of *Schizochytrium* PUFA production. PUFA PKS genes and the proteins and domains encoded thereby from either of *Shewanella olleyana* or *Shewanella japonica* are explicitly encompassed by the present invention.

### Example 8

This example demonstrates how the bacterial PUFA PKS gene fragments described in Example 7 can be used to modify PUFA production in *Schizochytrium.*

All presently-known examples of PUFA PKS genes from bacteria exist as four closely linked genes that contain the same domains as in the three-gene *Schizochytrium* set. It is anticipated that the PUFA PKS genes from *Shewanella olleyana* and *Shewanella japonica* will likewise be found in this tightly clustered arrangement. The homologous regions identified in Example 7 are used to isolate the PUFA PKS gene clusters from clone banks of *Sh. olleyana* and *Sh. japonica* DNAs. Clone banks can be constructed in bacteriophage lambda vectors, cosmid vectors, bacterial artificial chromosome ("BAC") vectors, or by other methods known in the art. Desired clones containing bacterial PUFA PKS genes can be identified by colony or plaque hybridization (as described in Example 1) using probes generated by PCR of the partial gene sequences of Example 7 employing primers designed from these sequences. The complete DNA sequence of the new bacterial PUFA PKS gene sets are then used to design vectors for transformation *of Schizochytrium* strains defective in the endogenous PUFA PKS genes (e.g., see Examples 3, 5, and 6). Whole bacterial genes (coding sequences) may be used to replace whole *Schizochytrium* genes (coding sequences), thus utilizing the *Schizochytrium* gene expression regions, and the fourth bacterial gene may be targeted to a different location within the genome. Alternatively, individual bacterial PUFA PKS functional domains may be "swapped" or exchanged with the analogous *Schizochytrium* domains by similar techniques of homologous recombination. It is understood that the sequence of the bacterial PUFA PKS genes or domains may have to be modified to accommodate details *of Schizochytrium* codon usage, but this is within the ability of those of skill in the art.

Each publication cited or discussed herein is incorporated herein by reference in its entirety.

## Claims

1. An isolated nucleic acid molecule comprising a nucleic acid sequence selected from the group consisting of:
a) a nucleic acid sequence encoding an amino acid sequence selected from the group consisting of: SEQ ID NO:62, SEQ ID NO:64, SEQ ID NO:66, SEQ ID NO:68 and biologically active fragments thereof, wherein the biologically active fragments exhibit a biological activity selected from the group consisting of enoyl-ACP reductase (ER) activity and β-hydroxyacyl-ACP dehydratase (DH) activity;
b) a nucleic acid sequence encoding an amino acid sequence that is at least about 70% identical to an amino acid sequence selected from the group consisting of: SEQ ID NO:62 and SEQ ID NO:66, wherein the amino acid sequence has a biological activity selected from the group consisting of enoyl-ACP reductase (ER) activity and β-hydroxyacyl-ACP dehydratase (DH) activity;
c) a nucleic acid sequence encoding an amino acid sequence that is at least about 80% identical to an amino acid sequence selected from the group consisting of: SEQ ID NO:64 and SEQ ID NO:68, wherein the amino acid sequence has a biological activity selected from the group consisting of enoyl-ACP reductase (ER) activity and β-hydroxyacyl-ACP dehydratase (DH) activity; and
d) a nucleic acid sequence that is fully complementary to the nucleic acid sequence of (a), (b) or (c).

2. The isolated nucleic acid molecule of claim 1, wherein the nucleic acid sequence encodes an amino acid sequence that is at least 80% identical to the amino acid sequence set forth in b).

3. The isolated nucleic acid molecule of claim 1, wherein the nucleic acid sequence encodes an amino acid sequence that is at least 90% identical to the amino acid sequence set forth in b) or c).

4. The isolated nucleic acid molecule of Claim 1, wherein the nucleic acid sequence is selected from the group consisting of: SEQ ID NO:61, SEQ ID NO:63, SEQ ID NO:65, and SEQ ID NO:67.

5. A recombinant nucleic acid molecule comprising the nucleic acid molecule of any preceding Claim, operatively linked to at least one transcription control sequence.

6. A recombinant cell comprising the recombinant nucleic acid molecule of Claim 5.

7. A genetically modified microorganism, wherein the microorganism expresses a PKS system comprising at least one biologically active domain of a polyunsaturated fatty acid (PUFA) polyketide synthase (PKS) system, wherein the at least one domain of the PUFA PKS system comprises an amino acid sequence selected from the group consisting of:
a) an amino acid sequence selected from the group consisting of: SEQ ID NO:62, SEQ ID NO:64, SEQ ID NO:66, SEQ ID NO:68 and biologically active fragments thereof, wherein the biologically active fragments exhibit a biological activity selected from the group consisting of enoyl-ACP reductase (ER) activity and β-hydroxyacyl-ACP dehydratase (DH) activity;
b) an amino acid sequence that is at least about 70% identical to an amino acid sequence selected from the group consisting of: SEQ ID NO:62 and SEQ ID NO:66, wherein the amino acid sequence has a biological activity selected from the group consisting of enoyl-ACP reductase (ER) activity and β-hydroxyacyl-ACP dehydratase (DH) activity; and
c) an amino acid sequence that is at least about 80% identical to an amino acid sequence selected from the group consisting of: SEQ ID NO:64 and SEQ ID NO:68, wherein the amino acid sequence has a biological activity selected from the group consisting of enoyl-ACP reductase (ER) activity and β-hydroxyacyl-ACP dehydratase (DH) activity;
wherein the microorganism is genetically modified by transfection with a recombinant nucleic acid molecule encoding the at least one domain of a polyunsaturated fatty acid (PUFA) polyketide synthase (PKS) system.

8. The genetically modified microorganism of Claim 7, wherein the amino acid sequence is at least 80% identical to the amino acid sequence set forth in b).

9. The genetically modified microorganism of Claim 7, wherein the amino acid sequence is at least 90% identical to the amino acid sequence set forth in b) or c).

10. The genetically modified microorganism of any one of Claims 7 to 9, wherein the microorganism is a Thraustochytrid.

11. The genetically modified microorganism of any one of Claims 7 to 9, wherein the microorganism is a Schizochytrium.

12. A genetically modified plant, wherein the plant has been genetically modified to recombinantly express a PKS system comprising at least one biologically active domain of a polyunsaturated fatty acid (PUFA) polyketide synthase (PKS) system, wherein the domain comprises an amino acid sequence selected from the group consisting of:
a) an amino acid sequence selected from the group consisting of: SEQ ID NO:62, SEQ ID NO:64, SEQ ID NO:66, SEQ ID NO:68 and biologically active fragments thereof, wherein the biologically active fragments exhibit a biological activity selected from the group consisting of enoyl-ACP reductase (ER) activity and β-hydroxyacyl-ACP dehydratase (DH) activity;
b) an amino acid sequence that is at least about 70% identical to an amino acid sequence selected from the group consisting of: SEQ ID NO:62 and SEQ ID NO:66, wherein the amino acid sequence has a biological activity selected from the group consisting of enoyl-ACP reductase (ER) activity and β-hydroxyacyl-ACP dehydratase (DH) activity; and
c) an amino acid sequence that is at least about 80% identical to an amino acid sequence selected from the group consisting of: SEQ ID NO:64 and SEQ ID NO:68, wherein the amino acid sequence has a biological activity selected from the group consisting of enoyl-ACP reductase (ER) activity and β-hydroxyacyl-ACP dehydratase (DH) activity.

13. The genetically modified plant of Claim 12, wherein the amino acid sequence is at least 80% identical to the amino acid sequence set forth in b).

14. The genetically modified plant of Claim 12, wherein the amino acid sequence is at least 90% identical to the amino acid sequence set forth in b) or c).

15. The genetically modified microorganism of any one of Claims 7 to 9, or the genetically modified plant of any one of Claims 12 to 14, wherein the microorganism or plant has been further genetically modified to recombinantly express at least one nucleic acid molecule encoding at least one biologically active domain from a PKS system selected from the group consisting of: a bacterial PUFA PKS system, a Type I PKS system, a Type II PKS system, a modular PKS system, and a non-bacterial PUFA PKS system.

16. The genetically modified microorganism or plant of Claim 15, wherein the non-bacterial PUFA PKS system is a Thraustochytrid PUFA PKS system.

17. The genetically modified microorganism or plant of Claim 16, wherein the Thraustochytrid PUFA PKS system is a Schizochytrium PUFA PKS system.

18. A method to produce a bioactive molecule that is produced by a polyketide synthase system, comprising growing or culturing under conditions effective to produce the bioactive molecule in the genetically modified microorganism of any one of Claims 7 to 9 or the genetically modified plant of any one of Claims 12 to 14.

19. The method of Claim 18, wherein the genetically modified microorganism or genetically modified plant produces a polyunsaturated fatty acid (PUFA) profile that differs from the naturally occurring microorganism or plant without a genetic modification.

20. The method of Claim 18, wherein the microorganism endogenously expresses a non-bacterial PUFA PKS system, and wherein the genetic modification comprises substitution of a domain from a different PKS system for a nucleic acid sequence encoding at least one domain of the non-bacterial PUFA PKS system.

21. The method of Claim 18, wherein the bioactive molecule is selected from the group consisting of: an anti-inflammatory formulation, a chemotherapeutic agent, an active excipient, an osteoporosis drug, an anti-depressant, an anti-convulsant, an anti-Heliobactor pylori drug, a drug for treatment of neurodegenerative disease, a drug for treatment of degenerative liver disease, an antibiotic, a polyunsaturated fatty acid (PUFA), and a cholesterol lowering formulation.

22. The method of Claim 18, wherein the bioactive molecule is a molecule including carbon-carbon double bonds in the *cis* configuration or a molecule including a double bond at every third carbon.

23. A method to produce a plant that has a polyunsaturated fatty acid (PUFA) profile that differs from the naturally occurring plant, comprising genetically modifying cells of the plant by transformation to express a PKS system comprising at least one recombinant nucleic acid molecule comprising a nucleic acid sequence encoding at least one biologically active domain of a PUFA PKS system, wherein the at least one domain of the PUFA PKS system comprises an amino acid sequence as defined in any one of Claims 7 to 9.

24. A method to modify an endproduct containing at least one fatty acid, comprising adding to the endproduct an oil produced by a recombinant host cell that expresses at least one recombinant nucleic acid molecule comprising a nucleic acid sequence encoding at least one biologically active domain of a PUFA PKS system, wherein the at least one domain of a PUFA PKS system comprises an amino acid sequence as defined in any one of Claims 7 to 9.

25. The method of Claim 24, wherein the endproduct is selected from the group consisting of a dietary supplement, a food product, a pharmaceutical formulation, a humanized animal milk, and an infant formula.

26. The method of Claim 25, wherein the pharmaceutical formulation is selected from the group consisting of an anti-inflammatory formulation, a chemotherapeutic agent, an active excipient, an osteoporosis drug, an anti-depressant, an anti-convulsant, an anti- Heliobactor pylori drug, a drug for treatment of neurodegenerative disease, a drug for treatment of degenerative liver disease, an antibiotic, and a cholesterol lowering formulation.

27. The method of Claim 24, wherein the endproduct is used to treat a condition selected from the group consisting of: chronic inflammation, acute inflammation, gastrointestinal disorder, cancer, cachexia, cardiac restenosis, neurodegenerative disorder, degenerative disorder of the liver, blood lipid disorder, osteoporosis, osteoarthritis, autoimmune disease, preeclampsia, preterm birth, age related maculopathy, pulmonary disorder, and peroxisomal disorder.

28. A method to produce a humanized animal milk, comprising genetically modifying milk-producing cells of a milk-producing animal with at least one recombinant nucleic acid molecule comprising a nucleic acid sequence encoding at least one biologically active domain of a PUFA PKS system, wherein the at least one domain of the PUFA PKS system comprises an amino acid sequence as defined in any one of Claims 7 to 9.

29. A genetically modified Thraustochytrid microorganism, wherein the microorganism has an endogenous polyunsaturated fatty acid (PUFA) polyketide synthase (PKS) system, and wherein the endogenous PUFA PKS system has been genetically modified by deleting at least one nucleic acid sequence that encodes at least one domain of the endogenous PUFA PKS system and inserting a nucleic acid sequence as defined in any one of Claims 1 to 3,
wherein the expression profile of a polyunsaturated fatty acid (PUFA) by the genetically modified Thraustochytrid microorganism is altered as compared to the Thraustochytrid microorganism in the absence of the genetic modification.

30. The genetically modified Thraustochytrid microorganism of Claim 29, wherein the PUFA production profile is altered to initiate, increase or decrease production by the microorganism of a fatty acid selected from the group consisting of: eicosapentaenoic acid (EPA), docosahexaenoic acid (DHA), one or both isomers of docosapentaenoic acid (DPA), and arachidonic acid (ARA).

31. The genetically modified Thraustochytrid microorganism of Claim 29, wherein the Thraustochytrid is from a genus selected from the group consisting of *Schizochytrium, Thraustochytrium,* and *Japonochytrium.*

32. The genetically modified Thraustochytrid microorganism of Claim 29, wherein the Thraustochytrid is from a Schizochytrium species selected from the group consisting of: *Schizochytrium aggregatum, Schizochytrium limacinum,* and *Schizochytrium minutum.*

## Patentansprüche

1. Isoliertes Nukleinsäuremolekül, umfassend eine aus der aus:
a) einer für eine aus der aus SEQ ID NO:62, SEQ ID NO:64, SEQ ID NO:66, SEQ ID NO:68 sowie biologisch aktiven Fragmenten davon bestehenden Gruppe ausgewählte Aminosäuresequenz codierenden Nukleinsäuresequenz, wobei die biologisch aktiven Fragmente eine aus der aus Enoyl-ACP-Reduktase-(ER)-Aktivität und β-Hydroxyacyl-ACP-Dehydratase-(DH)-Aktivität bestehenden Gruppe ausgewählte biologische Aktivität zeigen;
b) einer für eine Aminosäuresequenz, die zu wenigstens etwa 70% mit einer aus der aus SEQ ID NO:62 und SEQ ID NO:66 bestehenden Gruppe ausgewählten Aminosäuresequenz identisch ist, codierenden Nukleinsäuresequenz, wobei die Aminosäuresequenz eine aus der aus Enoyl-ACP-Reduktase(ER)-Aktivität und β-Hydroxyacyl-ACP-Dehydratase(DH)-Aktivität bestehenden Gruppe ausgewählte biologische Aktivität aufweist;
c) einer für eine Aminosäuresequenz, die zu wenigstens etwa 80% mit einer aus der aus SEQ ID NO:64 und SEQ ID NO:68 bestehenden Gruppe ausgewählten Aminosäuresequenz identisch ist, codierenden Nukleinsäuresequenz, wobei die Aminosäuresequenz eine aus der aus Enoyl-ACP-Reduktase(ER)-Aktivität und β-Hydroxyacyl-ACP-Dehydratase(DH)-Aktivität bestehenden Gruppe ausgewählte biologische Aktivität aufweist; und
d) einer Nukleinsäuresequenz, die zu der Nukleinsäuresequenz unter (a), (b) oder (c) vollständig komplementär ist,
bestehenden Gruppe ausgewählte Nukleinsäuresequenz.

2. Isoliertes Nukleinsäuremolekül nach Anspruch 1, wobei die Nukleinsäuresequenz für eine Aminosäuresequenz codiert, die zu wenigstens 80% mit der Aminosäuresequenz gemäß b) identisch ist.

3. Isoliertes Nukleinsäuremolekül nach Anspruch 1, wobei die Nukleinsäuresequenz für eine Aminosäuresequenz codiert, die zu wenigstens 90% mit der Aminosäuresequenz gemäß b) oder c) identisch ist.

4. Isoliertes Nukleinsäuremolekül nach Anspruch 1, wobei die Nukleinsäuresequenz aus der aus SEQ ID NO:61, SEQ ID NO:63, SEQ ID NO:65 und SEQ ID NO:67 bestehenden Gruppe ausgewählt ist.

5. Rekombinantes Nukleinsäuremolekül, umfassend das Nukleinsäuremolekül nach einem vorhergehenden Anspruch in operativer Verknüpfung mit wenigstens einer Transkriptionskontrollsequenz.

6. Rekombinante Zelle, umfassend das rekombinante Nukleinsäuremolekül nach Anspruch 5.

7. Gentechnisch veränderter Mikroorganismus, wobei der Mikroorganismus ein wenigstens eine biologisch aktive Domäne eines PUFA(Polyunsaturated Fatty Acid)-Polyketidsynthase(PKS)-Systems umfassendes PKS-System exprimiert, wobei die wenigstens eine Domäne des PUFA-PKS-Systems eine aus der aus:
a) einer aus der aus SEQ ID NO:62, SEQ ID NO:64, SEQ ID NO:66, SEQ ID NO:68 sowie biologisch aktiven Fragmenten davon bestehenden Gruppe ausgewählten Aminosäuresequenz, wobei die biologisch aktiven Fragmente eine aus der aus Enoyl-ACP-Reduktase(ER)-Aktivität und β-Hydroxyacyl-ACP-Dehydratase(DH)-Aktivität bestehenden Gruppe ausgewählte biologische Aktivität zeigen;
b) einer Aminosäuresequenz, die zu wenigstens etwa 70% mit einer aus der aus SEQ ID NO:62 und SEQ ID NO:66 bestehenden Gruppe ausgewählten Aminosäuresequenz identisch ist, wobei die Aminosäuresequenz eine aus der aus Enoyl-ACP-Reduktase(ER)-Aktivität und β-Hydroxyacyl-ACP-Dehydratase(DH)-Aktivität bestehenden Gruppe ausgewählte biologische Aktivität aufweist; und
c) einer Aminosäuresequenz, die zu wenigstens etwa 80% mit einer aus der aus SEQ ID NO:64 und SEQ ID NO:68 bestehenden Gruppe ausgewählten Aminosäuresequenz identisch ist, wobei die Aminosäuresequenz eine aus der aus Enoyl-ACP-Reduktase(ER)-Aktivität und β-Hydroxyacyl-ACP-Dehydratase(DH)-Aktivität bestehenden Gruppe ausgewählte biologische Aktivität aufweist, bestehenden Gruppe ausgewählte Aminosäuresequenz umfasst;
wobei der Mikroorganismus durch Transfektion mit einem für die wenigstens eine Domäne eines PUFA-(Polyunsaturated Fatty Acid)-Polyketidsynthase-(PKS)-Systems codierenden rekombinanten Nukleinsäuremolekül gentechnisch verändert ist.

8. Gentechnisch veränderter Mikroorganismus nach Anspruch 7, wobei die Aminosäuresequenz zu wenigstens 80% mit der Aminosäuresequenz gemäß b) identisch ist.

9. Gentechnisch veränderter Mikroorganismus nach Anspruch 7, wobei die Aminosäuresequenz zu wenigstens 90% mit der Aminosäuresequenz gemäß b) oder c) identisch ist.

10. Gentechnisch veränderter Mikroorganismus nach einem der Ansprüche 7 bis 9, wobei der Mikroorganismus zu den Thraustochytriacae gehört.

11. Gentechnisch veränderter Mikroorganismus nach einem der Ansprüche 7 bis 9, wobei es sich bei dem Mikroorganismus um ein Schizochytrium handelt.

12. Gentechnisch veränderte Pflanze, wobei die Pflanze gentechnisch verändert wurde, so dass ein wenigstens eine biologisch aktive Domäne eines PUFA-(Polyunsaturated Fatty Acid)-Polyketidsynthase-(PKS)-Systems umfassendes PKS-System rekombinant exprimiert wird, wobei die Domäne eine aus der aus:
a) einer aus der aus SEQ ID NO:62, SEQ ID NO:64, SEQ ID NO:66, SEQ ID NO:68 sowie biologisch aktiven Fragmenten davon bestehenden Gruppe ausgewählten Aminosäuresequenz, wobei die biologisch aktiven Fragmente eine aus der aus Enoyl-ACP-Reduktase(ER)-Aktivität und β-Hydroxyacyl-ACP-Dehydratase(DH)-Aktivität bestehenden Gruppe ausgewählte biologische Aktivität zeigen;
b) einer Aminosäuresequenz, die zu wenigstens etwa 70% mit einer aus der aus SEQ ID NO:62 und SEQ ID NO:66 bestehenden Gruppe ausgewählten Aminosäuresequenz identisch ist, wobei die Aminosäuresequenz eine aus der aus Enoyl-ACP-Reduktase(ER)-Aktivität und β-Hydroxyacyl-ACP-Dehydratase(DH)-Aktivität bestehenden Gruppe ausgewählte biologische Aktivität aufweist; und
c) einer Aminosäuresequenz, die zu wenigstens etwa 80% mit einer aus der aus SEQ ID NO:64 und SEQ ID NO:68 bestehenden Gruppe ausgewählten Aminosäuresequenz identisch ist, wobei die Aminosäuresequenz eine aus der aus Enoyl-ACP-Reduktase(ER)-Aktivität und β-Hydroxyacyl-ACP-Dehydratase(DH)-Aktivität bestehenden Gruppe ausgewählte biologische Aktivität aufweist,
bestehenden Gruppe ausgewählte Aminosäuresequenz umfasst.

13. Gentechnisch veränderte Pflanze nach Anspruch 12, wobei die Aminosäuresequenz zu wenigstens 80% mit der Aminosäuresequenz gemäß b) identisch ist.

14. Gentechnisch veränderte Pflanze nach Anspruch 12, wobei die Aminosäuresequenz zu wenigstens 90% mit der Aminosäuresequenz gemäß b) oder c) identisch ist.

15. Gentechnisch veränderter Mikroorganismus nach einem der Ansprüche 7 bis 9 oder gentechnisch veränderte Pflanze nach einem der Ansprüche 12 bis 14, wobei der Mikroorganismus bzw. die Pflanze weiter gentechnisch verändert wurde, so dass wenigstens ein für wenigstens eine biologisch aktive Domäne aus einem aus der aus einem bakteriellen PUFA-PKS-System, einem Typ-I-PKS-System, einem Typ-II-PKS-System, einem modularen PKS-System und einem nicht bakteriellen PUFA-PKS-System bestehenden Gruppe ausgewählten PKS-System codierendes Nukleinsäuremolekül rekombinant exprimiert wird.

16. Gentechnisch veränderter Mikroorganismus oder gentechnisch veränderte Pflanze nach Anspruch 15, wobei es sich bei dem nicht bakteriellen PUFA-PKS-System um ein Thraustochytriacae-PUFA-PKS-System handelt.

17. Gentechnisch veränderter Mikroorganismus oder gentechnisch veränderte Pflanze nach Anspruch 16, wobei es sich bei dem Thraustochytriacae-PUFA-PKS-System um ein Schizochytrium-PUFA-PKS-System handelt.

18. Verfahren zur Herstellung eines bioaktiven Moleküls, das von einem Polyketidsynthase-System produziert wird, umfassend das Anziehen oder Kultivieren unter für die Produktion des bioaktiven Moleküls in dem gentechnisch veränderten Mikroorganismus nach einem der Ansprüche 7 bis 9 oder der gentechnisch veränderten Pflanze nach einem der Ansprüche 12 bis 14 wirksamen Bedingungen.

19. Verfahren nach Anspruch 18, wobei der gentechnisch veränderte Mikroorganismus oder die gentechnisch veränderte Pflanze ein PUFA(Polyunsaturated Fatty Acid)-Profil produziert, das sich von dem natürlich vorkommenden Mikroorganismus bzw. der natürlich vorkommenden Pflanze ohne eine gentechnische Veränderung unterscheidet.

20. Verfahren nach Anspruch 18, wobei der Mikroorganismus endogen ein nicht bakterielles PUFA-PKS-System exprimiert und wobei die gentechnische Veränderung den Austausch einer für wenigstens eine Domäne des nicht bakteriellen PUFA-PKS-Systems codierenden Nukleinsäuresequenz gegen eine Domäne aus einem anderen PKS-System umfasst.

21. Verfahren nach Anspruch 18, wobei das bioaktive Molekül aus der aus einer entzündungshemmenden Formulierung, einem Chemotherapeutikum, einem aktiven Hilfsstoff, einem Osteoporose-Arzneistoff, einem Antidepressivum, einem Antikonvulsivum, einem Arzneistoff gegen Heliobacter pylori, einem Arzneistoff zur Behandlung von neurodegenerativer Krankheit, einem Arzneistoff zur Behandlung von degenerativer Leberkrankheit, einem Antibiotikum, einer mehrfach ungesättigten Fettsäure (Poly-unsaturated Fatty Acid, PUFA) und einer cholesterinsenkenden Formulierung bestehenden Gruppe ausgewählt ist.

22. Verfahren nach Anspruch 18, wobei es sich bei dem bioaktiven Molekül um ein Kohlenstoff-KohlenstoffDoppelbindungen in der cis-Konfiguration enthaltendes Molekül oder ein eine Doppelbindung an jedem dritten Kohlenstoffatom enthaltendes Molekül handelt.

23. Verfahren zur Herstellung einer Pflanze, die ein PUFA(Polyunsaturated Fatty Acid)-Profil aufweist, das sich von der natürlich vorkommenden Pflanze unterscheidet, wobei man bei dem Verfahren Zellen der Pflanze durch Transformation gentechnisch verändert, so dass ein wenigstens ein eine für wenigstens eine biologisch aktive Domäne eines PUFA-PKS-Systems codierende Nukleinsäuresequenz umfassendes rekombinantes Nukleinsäuremolekül umfassendes PKS-System exprimiert wird, wobei die wenigstens eine Domäne des PUFA-PKS-Systems eine Aminosäuresequenz mit der in einem der Ansprüche 7 bis 9 angegebenen Bedeutung umfasst.

24. Verfahren zur Veränderung eines wenigstens eine Fettsäure enthaltenden Endprodukts, wobei man zu dem Endprodukt ein von einer rekombinanten Wirtszelle, die wenigstens ein eine für wenigstens eine biologisch aktive Domäne eines PUFA-PKS-Systems codierende Nukleinsäuresequenz umfassendes rekombinantes Nukleinsäuremolekül exprimiert, produziertes Öl gibt, wobei die wenigstens eine Domäne eines PUFA-PKS-Systems eine Aminosäuresequenz mit der in einem der Ansprüche 7 bis 9 angegebenen Bedeutung umfasst.

25. Verfahren nach Anspruch 24, wobei das Endprodukt aus der aus einem Nahrungsergänzungsmittel, einem Lebensmittelprodukt, einer pharmazeutischen Formulierung, einer humanisierten Tiermilch und einer Säuglingsnahrung bestehenden Gruppe ausgewählt ist.

26. Verfahren nach Anspruch 25, wobei die pharmazeutische Formulierung aus der aus einer entzündungshemmenden Formulierung, einem Chemotherapeutikum, einem aktiven Hilfsstoff, einem Osteoporose-Arzneistoff, einem Antidepressivum, einem Antikonvulsivum, einem Arzneistoff gegen Heliobacter pylori, einem Arzneistoff zur Behandlung von neurodegenerativer Krankheit, einem Arzneistoff zur Behandlung von degenerativer Leberkrankheit, einem Antibiotikum und einer cholesterinsenkenden Formulierung bestehenden Gruppe ausgewählt ist.

27. Verfahren nach Anspruch 24, wobei das Endprodukt zur Behandlung eines aus der aus chronischer Entzündung, akuter Entzündung, Magen-Darm-Erkrankung, Krebs, Kachexie, Herz-Restenose, neurodegenerativer Erkrankung, degenerativer Erkrankung der Leber, Blutfettstörung, Osteoporose, Osteoarthritis, Autoimmunkrankheit, Präeklampsie, Frühgeburt, altersbedingter Makulopathie, Lungenerkrankung und peroxisomaler Erkrankung bestehenden Gruppe ausgewählten Leidens verwendet wird.

28. Verfahren zur Herstellung einer humanisierten Tiermilch, bei dem man milchproduzierende Zellen eines milchproduzierenden Tiers mit wenigstens einem eine für wenigstens eine biologisch aktive Domäne eines PUFA-PKS-Systems codierende Nukleinsäuresequenz umfassenden rekombinanten Nukleinsäuremolekül gentechnisch verändert, wobei die wenigstens eine Domäne des PUFA-PKS-Systems eine Aminosäuresequenz mit der in einem der Ansprüche 7 bis 9 angegebenen Bedeutung umfasst.

29. Gentechnisch veränderter Thraustochytriacae-Mikroorganismus, wobei der Mikroorganismus ein endogenes PUFA(Polyunsaturated Fatty Acid)-Polyketidsynthase(PKS)-System aufweist und wobei das endogene PUFA-PKS-System gentechnisch verändert wurde, indem wenigstens eine Nukleinsäuresequenz, die für wenigstens eine Domäne des endogenen PUFA-PKS-Systems codiert, deletiert und eine Nukleinsäuresequenz mit der in einem der Ansprüche 1 bis 3 angegebenen Bedeutung inseriert wurde,
wobei das Expressionsprofil einer mehrfach ungesättigten Fettsäure (PUFA) durch den gentechnisch veränderten Thraustochytriacae-Mikroorganismus im Vergleich zu dem Thraustochytriacae-Mikroorganismus in Abwesenheit der genetischen Veränderung geändert ist.

30. Gentechnisch veränderter Thraustochytriacae-Mikroorganismus nach Anspruch 29, wobei das PUFA-Produktionsprofil geändert ist, um die Produktion einer aus der aus Eicosapentaensäure (EPA), Docosahexaensäure (DHA), einem oder beiden Isomeren der Docosapentaensäure (DPA) und Arachidonsäure (ARA) bestehenden Gruppe ausgewählten Fettsäure durch den Mikroorganismus zu starten, zu steigern oder zu senken.

31. Gentechnisch veränderter Thraustochytriacae-Mikroorganismus nach Anspruch 29, wobei der Thraustochytriacae-Mikroorganismus aus einer aus der aus *Schizochytrium, Thraustochytrium* und *Japonochytrium* bestehenden Gruppe ausgewählten Gattung stammt.

32. Gentechnisch veränderter Thraustochytriacae-Mikroorganismus nach Anspruch 29, wobei der Thraustochytriacae-Mikroorganismus aus einer aus der aus *Schizochytrium aggregatum, Schizochytrium limacinum* und *Schizochytrium minutum* bestehenden Gruppe ausgewählten Schizochytrium-Spezies stammt.

## Revendications

1. Molécule d'acide nucléique isolée comprenant une séquence d'acide nucléique choisie dans le groupe constitué de :
a) une séquence d'acide nucléique codant pour une séquence d'acides aminés choisie dans le groupe constitué de : SEQ ID NO: 62, SEQ ID NO: 64, SEQ ID NO: 66, SEQ ID NO: 68 et des fragments biologiquement actifs de celles-ci, les fragments biologiquement actifs présentant une activité biologique choisie dans le groupe constitué de l'activité énoyl-ACP réductase (ER) et l'activité β-hydroxyacyl-ACP déshydratase (DH) activité ;
b) une séquence d'acide nucléique codant pour une séquence d'acides aminés qui est au moins environ 70 % identique à une séquence d'acides aminés choisie dans le groupe constitué de : SEQ ID NO: 62 et SEQ ID NO: 66, la séquence d'acides aminés ayant une activité biologique choisie dans le groupe constitué de l'activité énoyl-ACP réductase (ER) et l'activité β-hydroxyacyl-ACP déshydratase (DH) ;
c) une séquence d'acide nucléique codant pour une séquence d'acides aminés qui est au moins environ 80 % identique à une séquence d'acides aminés choisie dans le groupe constitué de : SEQ ID NO: 64 et SEQ ID NO: 68, la séquence d'acides aminés ayant une activité biologique choisie dans le groupe constitué de l'activité énoyl-ACP réductase (ER) et l'activité β-hydroxyacyl-ACP déshydratase (DH) ; et
d) une séquence d'acide nucléique qui est totalement complémentaire de la séquence d'acide nucléique de (a), (b) ou (c).

2. Molécule d'acide nucléique isolée de la revendication 1, dans laquelle la séquence d'acide nucléique code pour une séquence d'acides aminés qui est au moins 80 % identique à la séquence d'acides aminés décrite dans b).

3. Molécule d'acide nucléique isolée de la revendication 1, dans laquelle la séquence d'acide nucléique code pour une séquence d'acides aminés qui est au moins 90 % identique à la séquence d'acides aminés décrite dans b) ou c).

4. Molécule d'acide nucléique isolée de la revendication 1, dans laquelle la séquence d'acide nucléique est choisie dans le groupe constitué de : SEQ ID NO: 61, SEQ ID NO: 63, SEQ ID NO: 65, et SEQ ID NO: 67.

5. Molécule d'acide nucléique recombinante comprenant la molécule d'acide nucléique de l'une quelconque des revendications précédentes, fonctionnellement liée à au moins une séquence de contrôle de transcription.

6. Cellule recombinante comprenant la molécule d'acide nucléique recombinante de la revendication 5.

7. Micro-organisme génétiquement modifié, le micro-organisme exprimant un système PKS comprenant au moins un domaine biologiquement actif d'un système acide gras polyinsaturé (PUFA) polycétide synthase (PKS), l'au moins un domaine du système PUFA PKS comprenant une séquence d'acides aminés choisie dans le groupe constitué de :
a) une séquence d'acides aminés choisie dans le groupe constitué de : SEQ ID NO: 62, SEQ ID NO: 64, SEQ ID NO: 66, SEQ ID NO: 68 et des fragments biologiquement actifs de celles-ci, les fragments biologiquement actifs présentant une activité biologique choisie dans le groupe constitué de l'activité énoyl-ACP réductase (ER) et l'activité β-hydroxyacyl-ACP déshydratase (DH) ;
b) une séquence d'acides aminés qui est au moins environ 70 % identique à une séquence d'acides aminés choisie dans le groupe constitué de : SEQ ID NO: 62 et SEQ ID NO: 66, la séquence d'acides aminés ayant une activité biologique choisie dans le groupe constitué de l'activité énoyl-ACP réductase (ER) et L'activité β-hydroxyacyl-ACP déshydratase (DH) ; et
c) une séquence d'acides aminés qui est au moins environ 80 % identique à une séquence d'acides aminés choisie dans le groupe constitué de : SEQ ID NO: 64 et SEQ ID NO: 68, la séquence d'acides aminés ayant une activité biologique choisie dans le groupe constitué de l'activité énoyl-ACP réductase (ER) et l'activité β-hydroxyacyl-ACP déshydratase (DH) ;
le micro-organisme étant génétiquement modifié par transfection avec une molécule d'acide nucléique recombinante codant pour l'au moins un domaine d'un système acide gras polyinsaturé (PUFA) polycétide synthase (PKS).

8. Micro-organisme génétiquement modifié de la revendication 7, dans lequel la séquence d'acides aminés est au moins 80 % identique à la séquence d'acides aminés décrite dans b).

9. Micro-organisme génétiquement modifié de la revendication 7, dans lequel la séquence d'acides aminés est au moins 90 % identique à la séquence d'acides aminés décrite dans b) ou c).

10. Micro-organisme génétiquement modifié de l'une quelconque des revendications 7 à 9, le micro-organisme étant un thraustochytride.

11. Micro-organisme génétiquement modifié de l'une quelconque des revendications 7 à 9, le micro-organisme étant un Schizochytrium.

12. Plante génétiquement modifiée, la plante ayant été génétiquement modifiée pour exprimer de façon recombinante un système PKS comprenant au moins un domaine biologiquement actif d'un système acide gras polyinsaturé (PUFA) polycétide synthase (PKS), le domaine comprenant une séquence d'acides aminés choisie dans le groupe constitué de :
a) une séquence d'acides aminés choisie dans le groupe constitué de : SEQ ID NO: 62, SEQ ID NO: 64, SEQ ID NO: 66, SEQ ID NO: 68 et des fragments biologiquement actifs de ceux-ci, les fragments biologiquement actifs présentant une activité biologique choisie dans le groupe constitué de l'activité énoyl-ACP réductase (ER) et l'activité β-hydroxyacyl-ACP déshydratase (DH) ;
b) une séquence d'acides aminés qui est au moins environ 70 % identique à une séquence d'acides aminés choisie dans le groupe constitué de : SEQ ID NO: 62 et SEQ ID NO: 66, la séquence d'acides aminés ayant une activité biologique choisie dans le groupe constitué de l'activité énoyl-ACP réductase (ER) et l'activité β-hydroxyacyl-ACP déshydratase (DH) ; et
c) une séquence d'acides aminés qui est au moins environ 80 % identique à une séquence d'acides aminés choisie dans le groupe constitué de : SEQ ID NO: 64 et SEQ ID NO: 68, la séquence d'acides aminés ayant une activité biologique choisie dans le groupe constitué de l'activité énoyl-ACP réductase (ER) et L'activité β-hydroxyacyl-ACP déshydratase (DH).

13. Plante génétiquement modifiée de la revendication 12, la séquence d'acides aminés étant au moins 80 % identique à la séquence d'acides aminés décrite dans b).

14. Plante génétiquement modifiée de la revendication 12, la séquence d'acides aminés étant au moins 90 % identique à la séquence d'acides aminés décrite dans b) ou c).

15. Micro-organisme génétiquement modifié de l'une quelconque des revendications 7 à 9, ou la plante génétiquement modifiée de l'une quelconque des revendications 12 à 14, le micro-organisme ou la plante ayant été en outre génétiquement modifié pour exprimer de façon recombinante au moins une molécule d'acide nucléique codant pour au moins un domaine biologiquement actif d'un système PKS choisi dans le groupe constitué de : un système PUFA PKS bactérien, un système PKS de type I, un système PKS de type II, un système PKS modulaire, et un système PUFA PKS non bactérien.

16. Micro-organisme ou plante génétiquement modifié de la revendication 15, le système PUFA PKS non bactérien étant un système PUFA PKS de thraustochytride.

17. Micro-organisme ou plante génétiquement modifié de la revendication 16, dans lequel le système PUFA PKS de thraustochytride est un système PUFA PKS de Schizochytrium.

18. Procédé pour produire une molécule bioactive qui est produite par un système de polycétide synthase, comprenant la croissance ou la culture dans des conditions efficaces pour produire la molécule bioactive dans le micro-organisme génétiquement modifié de l'une quelconque des revendications 7 à 9 ou la plante génétiquement modifiée de l'une quelconque des revendications 12 à 14.

19. Procédé de la revendication 18, dans lequel le micro-organisme génétiquement modifié ou la plante génétiquement modifiée produit un profil d'acide gras polyinsaturé (PUFA) qui diffère du micro-organisme ou de la plante d'origine naturelle sans modification génétique.

20. Procédé de la revendication 18, dans lequel le micro-organisme exprime de façon endogène un système PUFA PKS non bactérien, et dans lequel la modification génétique comprend la substitution d'un domaine d'un système PKS différent pour une séquence d'acide nucléique codant pour au moins un domaine du système PUFA PKS non bactérien.

21. Procédé de la revendication 18, dans lequel la molécule bioactive est choisie dans le groupe constitué de : une formulation anti-inflammatoire, un agent chimiothérapeutique, un excipient actif, un médicament contre l'ostéoporose, un antidépresseur, un anticonvulsivant, un médicament anti-Heliobacter pylori, un médicament pour le traitement d'une maladie neurodégénérative, un médicament pour le traitement d'une maladie hépatique dégénérative, un antibiotique, un acide gras polyinsaturé (PUFA), et une formulation hypocholestérolémiante.

22. Procédé de la revendication 18, dans lequel la molécule bioactive est une molécule comprenant des doubles liaisons carbone-carbone en configuration cis ou une molécule comprenant une double liaison à un carbone sur trois.

23. Procédé pour produire une plante qui a un profil d'acide gras polyinsaturé (PUFA) qui diffère de la plante d'origine naturelle, comprenant la modification génétique de cellules par transformation de la plante pour exprimer un système PKS comprenant au moins une molécule d'acide nucléique recombinante comprenant une séquence d'acide nucléique codant pour au moins un domaine biologiquement actif d'un système PUFA PKS, l'au moins un domaine du système PUFA PKS comprenant une séquence d'acides aminés telle que définie dans l'une quelconque des revendications 7 à 9.

24. Procédé pour modifier un produit final contenant au moins un acide gras, comprenant l'ajout au produit final d'une huile produite par une cellule hôte recombinante qui exprime au moins une molécule d'acide nucléique recombinante comprenant une séquence d'acide nucléique codant pour au moins un domaine biologiquement actif d'un système PUFA PKS, l'au moins un domaine d'un système PUFA PKS comprenant une séquence d'acides aminés telle que définie dans l'une quelconque des revendications 7 à 9.

25. Procédé de la revendication 24, dans lequel le produit final est choisi dans le groupe constitué d'un supplément alimentaire, un produit alimentaire, une formulation pharmaceutique, un lait animal humanisé, et une formule pour nourrisson.

26. Procédé de la revendication 25,
dans lequel la formulation pharmaceutique est choisie dans le groupe constitué d'une formulation anti-inflammatoire, un agent chimiothérapeutique, un excipient actif, un médicament contre l'ostéoporose, un antidépresseur, un anticonvulsivant, un médicament anti-Heliobacter pylori, un médicament pour le traitement d'une maladie neurodégénérative, un médicament pour le traitement d'une maladie hépatique dégénérative, un antibiotique, et une formulation hypocholestérolémiante.

27. Procédé de la revendication 24, dans lequel le produit final est utilisé pour traiter une affection choisie dans le groupe constitué de : une inflammation chronique, une inflammation aiguë, un trouble gastrointestinal, un cancer, la cachexie, la resténose cardiaque, un trouble neurodégénératif, un trouble neurodégénératif hépatique, un trouble lipidémique, l'ostéoporose, l'arthrose, une maladie auto-immune, la pré-éclampsie, un accouchement prématuré, la maculopathie liée à l'âge, un trouble pulmonaire, et un trouble lié aux peroxisomes.

28. Procédé pour produire un lait animal humanisé, comprenant la modification génétique de cellules productrices de lait d'un animal producteur de lait avec au moins une molécule d'acide nucléique recombinante comprenant une séquence d'acide nucléique codant pour au moins un domaine biologiquement actif d'un système PUFA PKS, l'au moins un domaine du système PUFA PKS comprenant une séquence d'acides aminés telle que définie dans l'une quelconque des revendications 7 à 9.

29. Micro-organisme thraustochytride génétiquement modifié, le micro-organisme ayant un système acide gras polyinsaturé (PUFA) polycétide synthase (PKS) endogène, et le système PUFA PKS endogène ayant été génétiquement modifié par délétion d'au moins une séquence d'acide nucléique qui code pour au moins un domaine du système PUFA PKS endogène et insertion d'une séquence d'acide nucléique telle que définie dans l'une quelconque des revendications 1 à 3,
dans lequel le profil d'expression d'un acide gras polyinsaturé (PUFA) par le micro-organisme thraustochytride génétiquement modifié est modifié par rapport au micro-organisme thraustochytride en l'absence de la modification génétique.

30. Micro-organisme thraustochytride génétiquement modifié de la revendication 29, dans lequel le profil de production de PUFA est modifié de manière à initier, augmenter ou diminuer la production par le micro-organisme d'un acide gras choisi dans le groupe constitué de : l'acide eicosapentaénoïque (EPA), l'acide docosahexaénoïque (DHA), l'un ou les deux isomères de l'acide docosapentaénoïque (DPA), et l'acide arachidonique (ARA).

31. Micro-organisme thraustochytride génétiquement modifié de la revendication 29, le thraustochytride étant d'un genre choisi dans le groupe constitué de *Schizochytrium, Thraustochytrium,* et *Japonochytrium.*

32. Micro-organisme thraustochytride génétiquement modifié de la revendication 29, le thraustochytride étant d'une espèce de *Schizochytrium* choisie dans le groupe constitué de : *Schizochytrium aggregatum, Schizochytrium limacinum,* et *Schizochytrium minutum.*


### SEQUENCE LISTING

<110> Metz, James Weaver, Craig Barclay, William Flatt, James
<120> PUFA Polyketide Synthase Systems and Uses Thereof of Lipids
<130> 2997-49-pct
<150> 10/124,800
   <151> 2002-04-16
<150> 60/457,979
   <151> 2003-03-26
<160> 82
<170> PatentIn version 3.2
<210> 1
   <211> 8730
   <212> DNA
   <213> Schizochytrium sp.
<220>
   <221> CDS
   <222> (1)..(8730)
<400> 1
   atg gcg gcc cgt ctg cag gag caa aag gga ggc gag atg gat acc cgc        48
   Met Ala Ala Arg Leu Gln Glu Gln Lys Gly Gly Glu Met Asp Thr Arg
   1               5                   10                  15
   att gcc atc atc ggc atg tcg gcc atc ctc ccc tgc ggc acg acc gtg        96
   Ile Ala Ile Ile Gly Met Ser Ala Ile Leu Pro Cys Gly Thr Thr Val
               20                  25                  30
   cgc gag tcg tgg gag acc atc cgc gcc ggc atc gac tgc ctg tcg gat       144
   Arg Glu Ser Trp Glu Thr Ile Arg Ala Gly Ile Asp Cys Leu Ser Asp
           35                  40                  45
   ctc ccc gag gac cgc gtc gac gtg acg gcg tac ttt gac ccc gtc aag       192
   Leu Pro Glu Asp Arg Val Asp Val Thr Ala Tyr Phe Asp Pro Val Lys
       50                  55                  60
   acc acc aag gac aag atc tac tgc aag cgc ggt ggc ttc att ccc gag       240
   Thr Thr Lys Asp Lys Ile Tyr Cys Lys Arg Gly Gly Phe Ile Pro Glu
   65                  70                  75                  80
   tac gac ttt gac gcc cgc gag ttc gga ctc aac atg ttc cag atg gag       288
   Tyr Asp Phe Asp Ala Arg Glu Phe Gly Leu Asn Met Phe Gln Met Glu
                   85                  90                  95
   gac tcg gac gca aac cag acc atc tcg ctt ctc aag gtc aag gag gcc       336
   Asp Ser Asp Ala Asn Gln Thr Ile Ser Leu Leu Lys Val Lys Glu Ala
               100                 105                 110
   ctc cag gac gcc ggc atc gac gcc ctc ggc aag gaa aag aag aac atc       384
   Leu Gln Asp Ala Gly Ile Asp Ala Leu Gly Lys Glu Lys Lys Asn Ile
           115                 120                 125
   ggc tgc gtg ctc ggc att ggc ggc ggc caa aag tcc agc cac gag ttc       432
   Gly Cys Val Leu Gly Ile Gly Gly Gly Gln Lys Ser Ser His Glu Phe
       130                 135                 140
   tac tcg cgc ctt aat tat gtt gtc gtg gag aag gtc ctc cgc aag atg       480
   Tyr Ser Arg Leu Asn Tyr Val Val Val Glu Lys Val Leu Arg Lys Met
   145                 150                 155                 160
   ggc atg ccc gag gag gac gtc aag gtc gcc gtc gaa aag tac aag gcc       528
   Gly Met Pro Glu Glu Asp Val Lys Val Ala Val Glu Lys Tyr Lys Ala
                   165                 170                 175
   aac ttc ccc gag tgg cgc ctc gac tcc ttc cct ggc ttc ctc ggc aac      576
   Asn Phe Pro Glu Trp Arg Leu Asp Ser Phe Pro Gly Phe Leu Gly Asn
               180                 185                 190
   gtc acc gcc ggt cgc tgc acc aac acc ttc aac ctc gac ggc atg aac      624
   Val Thr Ala Gly Arg Cys Thr Asn Thr Phe Asn Leu Asp Gly Met Asn
           195                 200                 205
   tgc gtt gtc gac gcc gca tgc gcc tcg tcc ctc atc gcc gtc aag gtc      672
   Cys Val Val Asp Ala Ala Cys Ala Ser Ser Leu Ile Ala Val Lys Val
       210                 215                 220
   gcc atc gac gag ctg ctc tac ggt gac tgc gac atg atg gtc acc ggt      720
   Ala Ile Asp Glu Leu Leu Tyr Gly Asp Cys Asp Met Met Val Thr Gly
   225                 230                 235                 240
   gcc acc tgc acg gat aac tcc atc ggc atg tac atg gcc ttc tcc aag      768
   Ala Thr Cys Thr Asp Asn Ser Ile Gly Met Tyr Met Ala Phe Ser Lys
                   245                 250                 255
   acc ccc gtg ttc tcc acg gac ccc agc gtg cgc gcc tac gac gaa aag      816
   Thr Pro Val Phe Ser Thr Asp Pro Ser Val Arg Ala Tyr Asp Glu Lys
               260                 265                 270
   aca aag ggc atg ctc atc ggc gag ggc tcc gcc atg ctc gtc ctc aag      864
   Thr Lys Gly Met Leu Ile Gly Glu Gly Ser Ala Met Leu Val Leu Lys
           275                 280                 285
   cgc tac gcc gac gcc gtc cgc gac ggc gat gag atc cac gct gtt att      912
   Arg Tyr Ala Asp Ala Val Arg Asp Gly Asp Glu Ile His Ala Val Ile
       290                 295                 300
   cgc ggc tgc gcc tcc tcc agt gat ggc aag gcc gcc ggc atc tac acg      960
   Arg Gly Cys Ala Ser Ser Ser Asp Gly Lys Ala Ala Gly Ile Tyr Thr
   305                 310                 315                 320
   ccc acc att tcg ggc cag gag gag gcc ctc cgc cgc gcc tac aac cgc     1008
   Pro Thr Ile Ser Gly Gln Glu Glu Ala Leu Arg Arg Ala Tyr Asn Arg
                   325                 330                 335
   gcc tgt gtc gac ccg gcc acc gtc act ctc gtc gag ggt cac ggc acc     1056
   Ala Cys Val Asp Pro Ala Thr Val Thr Leu Val Glu Gly His Gly Thr
               340                 345                 350
   ggt act ccc gtt ggc gac cgc atc gag ctc acc gcc ttg cgc aac ctc     1104
   Gly Thr Pro Val Gly Asp Arg Ile Glu Leu Thr Ala Leu Arg Asn Leu
           355                 360                 365
   ttt gac aag gcc tac ggc gag ggc aac acc gaa aag gtc gct gtg ggc     1152
   Phe Asp Lys Ala Tyr Gly Glu Gly Asn Thr Glu Lys Val Ala Val Gly
       370                 375                 380
   agc atc aag tcc agc atc ggc cat ctc aag gcc gtc gcc ggt ctc gcc     1200
   Ser Ile Lys Ser Ser Ile Gly His Leu Lys Ala Val Ala Gly Leu Ala
   385                 390                 395                 400
   ggt atg atc aag gtc atc atg gcg ctc aag cac aag act ctc ccg ggc     1248
   Gly Met Ile Lys Val Ile Met Ala Leu Lys His Lys Thr Leu Pro Gly
                   405                 410                 415
   acc atc aac gtc gac aac cca ccc aac ctc tac gac aac acg ccc atc     1296
   Thr Ile Asn Val Asp Asn Pro Pro Asn Leu Tyr Asp Asn Thr Pro Ile
               420                 425                 430
   aac gag tcc tcg ctc tac att aac acc atg aac cgc ccc tgg ttc ccg     1344
   Asn Glu Ser Ser Leu Tyr Ile Asn Thr Met Asn Arg Pro Trp Phe Pro
           435                 440                 445
   ccc cct ggt gtg ccc cgc cgc gcc ggc att tcg agc ttt ggc ttt ggt      1392
   Pro Pro Gly Val Pro Arg Arg Ala Gly Ile Ser Ser Phe Gly Phe Gly
       450                 455                 460
   ggc gcc aac tac cac gcc gtc ctc gag gag gcc gag ccc gag cac acg      1440
   Gly Ala Asn Tyr His Ala Val Leu Glu Glu Ala Glu Pro Glu His Thr
   465                 470                 475                 480
   acc gcg tac cgc ctc aac aag cgc ccg cag ccc gtg ctc atg atg gcc      1488
   Thr Ala Tyr Arg Leu Asn Lys Arg Pro Gln Pro Val Leu Met Met Ala
                   485                 490                 495
   gcc acg ccc gcg gcc ctc cag tcg ctc tgc gag gcc cag ctc aag gag      1536
   Ala Thr Pro Ala Ala Leu Gln Ser Leu Cys Glu Ala Gln Leu Lys Glu
               500                 505                 510
   ttc gag gcc gcc atc aag gag aac gag acc gtc aag aac acc gcc tac      1584
   Phe Glu Ala Ala Ile Lys Glu Asn Glu Thr Val Lys Asn Thr Ala Tyr
           515                 520                 525
   atc aag tgc gtc aag ttc ggc gag cag ttc aaa ttc cct ggc tcc atc      1632
   Ile Lys Cys Val Lys Phe Gly Glu Gln Phe Lys Phe Pro Gly Ser Ile
       530                 535                 540
   ccg gcc aca aac gcg cgc ctc ggc ttc ctc gtc aag gat gct gag gat      1680
   Pro Ala Thr Asn Ala Arg Leu Gly Phe Leu Val Lys Asp Ala Glu Asp
   545                 550                 555                 560
   gcc tgc tcc acc ctc cgt gcc atc tgc gcc caa ttc gcc aag gat gtc      1728
   Ala Cys Ser Thr Leu Arg Ala Ile Cys Ala Gln Phe Ala Lys Asp Val
                   565                 570                 575
   acc aag gag gcc tgg cgc ctc ccc cgc gag ggc gtc agc ttc cgc gcc      1776
   Thr Lys Glu Ala Trp Arg Leu Pro Arg Glu Gly Val Ser Phe Arg Ala
               580                 585                 590
   aag ggc atc gcc acc aac ggc gct gtc gcc gcg ctc ttc tcc ggc cag      1824
   Lys Gly Ile Ala Thr Asn Gly Ala Val Ala Ala Leu Phe Ser Gly Gln
           595                 600                 605
   ggc gcg cag tac acg cac atg ttt agc gag gtg gcc atg aac tgg ccc      1872
   Gly Ala Gln Tyr Thr His Met Phe Ser Glu Val Ala Met Asn Trp Pro
       610                 615                 620
   cag ttc cgc cag agc att gcc gcc atg gac gcc gcc cag tcc aag gtc      1920
   Gln Phe Arg Gln Ser Ile Ala Ala Met Asp Ala Ala Gln Ser Lys Val
   625                 630                 635                 640
   gct gga agc gac aag gac ttt gag cgc gtc tcc cag gtc ctc tac ccg      1968
   Ala Gly Ser Asp Lys Asp Phe Glu Arg Val Ser Gln Val Leu Tyr Pro
                   645                 650                 655
   cgc aag ccg tac gag cgt gag ccc gag cag aac ccc aag aag atc tcc      2016
   Arg Lys Pro Tyr Glu Arg Glu Pro Glu Gln Asn Pro Lys Lys Ile Ser
               660                 665                 670
   ctc acc gcc tac tcg cag ccc tcg acc ctg gcc tgc gct ctc ggt gcc      2064
   Leu Thr Ala Tyr Ser Gln Pro Ser Thr Leu Ala Cys Ala Leu Gly Ala
           675                 680                 685
   ttt gag atc ttc aag gag gcc ggc ttc acc ccg gac ttt gcc gcc ggc      2112
   Phe Glu Ile Phe Lys Glu Ala Gly Phe Thr Pro Asp Phe Ala Ala Gly
       690                 695                 700
   cat tcg ctc ggt gag ttc gcc gcc ctc tac gcc gcg ggc tgc gtc gac      2160
   His Ser Leu Gly Glu Phe Ala Ala Leu Tyr Ala Ala Gly Cys Val Asp
   705                 710                 715                 720
   cgc gac gag ctc ttt gag ctt gtc tgc cgc cgc gcc cgc atc atg ggc      2208
   Arg Asp Glu Leu Phe Glu Leu Val Cys Arg Arg Ala Arg Ile Met Gly
                   725                 730                 735
   ggc aag gac gca ccg gcc acc ccc aag gga tgc atg gcc gcc gtc att      2256
   Gly Lys Asp Ala Pro Ala Thr Pro Lys Gly Cys Met Ala Ala Val Ile
               740                 745                 750
   ggc ccc aac gcc gag aac atc aag gtc cag gcc gcc aac gtc tgg ctc      2304
   Gly Pro Asn Ala Glu Asn Ile Lys Val Gln Ala Ala Asn Val Trp Leu
           755                 760                 765
   ggc aac tcc aac tcg cct tcg cag acc gtc atc acc ggc tcc gtc gaa      2352
   Gly Asn Ser Asn Ser Pro Ser Gln Thr Val Ile Thr Gly Ser Val Glu
       770                 775                 780
   ggt atc cag gcc gag agc gcc cgc ctc cag aag gag ggc ttc cgc gtc      2400
   Gly Ile Gln Ala Glu Ser Ala Arg Leu Gln Lys Glu Gly Phe Arg Val
   785                 790                 795                 800
   gtg cct ctt gcc tgc gag agc gcc ttc cac tcg ccc cag atg gag aac      2448
   Val Pro Leu Ala Cys Glu Ser Ala Phe His Ser Pro Gln Met Glu Asn
                   805                 810                 815
   gcc tcg tcg gcc ttc aag gac gtc atc tcc aag gtc tcc ttc cgc acc      2496
   Ala Ser Ser Ala Phe Lys Asp Val Ile Ser Lys Val Ser Phe Arg Thr
               820                 825                 830
   ccc aag gcc gag acc aag ctc ttc agc aac gtc tct ggc gag acc tac      2544
   Pro Lys Ala Glu Thr Lys Leu Phe Ser Asn Val Ser Gly Glu Thr Tyr
           835                 840                 845
   ccc acg gac gcc cgc gag atg ctt acg cag cac atg acc agc agc gtc      2592
   Pro Thr Asp Ala Arg Glu Met Leu Thr Gln His Met Thr Ser Ser Val
       850                 855                 860
   aag ttc ctc acc cag gtc cgc aac atg cac cag gcc ggt gcg cgc atc      2640
   Lys Phe Leu Thr Gln Val Arg Asn Met His Gln Ala Gly Ala Arg Ile
   865                 870                 875                 880
   ttt gtc gag ttc gga ccc aag cag gtg ctc tcc aag ctt gtc tcc gag      2688
   Phe Val Glu Phe Gly Pro Lys Gln Val Leu Ser Lys Leu Val Ser Glu
                   885                 890                 895
   acc ctc aag gat gac ccc tcg gtt gtc acc gtc tct gtc aac ccg gcc      2736
   Thr Leu Lys Asp Asp Pro Ser Val Val Thr Val Ser Val Asn Pro Ala
               900                 905                 910
   tcg ggc acg gat tcg gac atc cag ctc cgc gac gcg gcc gtc cag ctc      2784
   Ser Gly Thr Asp Ser Asp Ile Gln Leu Arg Asp Ala Ala Val Gln Leu
           915                 920                 925
   gtt gtc gct ggc gtc aac ctt cag ggc ttt gac aag tgg gac gcc ccc      2832
   Val Val Ala Gly Val Asn Leu Gln Gly Phe Asp Lys Trp Asp Ala Pro
       930                 935                 940
   gat gcc acc cgc atg cag gcc atc aag aag aag cgc act acc ctc cgc      2880
   Asp Ala Thr Arg Met Gln Ala Ile Lys Lys Lys Arg Thr Thr Leu Arg
   945                 950                 955                 960
   ctt tcg gcc gcc acc tac gtc tcg gac aag acc aag aag gtc cgc gac      2928
   Leu Ser Ala Ala Thr Tyr Val Ser Asp Lys Thr Lys Lys Val Arg Asp
                   965                 970                 975
   gcc gcc atg aac gat ggc cgc tgc gtc acc tac ctc aag ggc gcc gca      2976
   Ala Ala Met Asn Asp Gly Arg Cys Val Thr Tyr Leu Lys Gly Ala Ala
               980                 985                 990
   ccg ctc atc aag gcc ccg gag ccc  gtt gtc gac gag gcc  gcc aag cgc    3024
   Pro Leu Ile Lys Ala Pro Glu Pro  Val Val Asp Glu Ala  Ala Lys Arg
           995                 1000                 1005
   gag gcc  gag cgt ctc cag aag  gag ctt cag gat gcc  cag cgc cag      3069
   Glu Ala  Glu Arg Leu Gln Lys  Glu Leu Gln Asp Ala  Gln Arg Gln
       1010                 1015                 1020
   ctc gac  gac gcc aag cgc gcc  gcc gcc gag gcc aac  tcc aag ctc      3114
   Leu Asp  Asp Ala Lys Arg Ala  Ala Ala Glu Ala Asn  Ser Lys Leu
       1025                 1030                 1035
   gcc gct  gcc aag gag gag gcc  aag acc gcc gct gct  tcg gcc aag      3159
   Ala Ala  Ala Lys Glu Glu Ala  Lys Thr Ala Ala Ala  Ser Ala Lys
       1040                 1045                 1050
   ccc gca  gtt gac act gct gtt  gtc gaa aag cat cgt  gcc atc ctc      3204
   Pro Ala  Val Asp Thr Ala Val  Val Glu Lys His Arg  Ala Ile Leu
       1055                 1060                 1065
   aag tcc  atg ctc gcg gag ctc  gat ggc tac gga tcg  gtc gac gct      3249
   Lys Ser  Met Leu Ala Glu Leu  Asp Gly Tyr Gly Ser  Val Asp Ala
       1070                 1075                 1080
   tct tcc  ctc cag cag cag cag  cag cag cag acg gcc  ccc gcc ccg      3294
   Ser Ser  Leu Gln Gln Gln Gln  Gln Gln Gln Thr Ala  Pro Ala Pro
       1085                 1090                 1095
   gtc aag  gct gct gcg cct gcc  gcc ccc gtt gcc tcg  gcc cct gcc      3339
   Val Lys  Ala Ala Ala Pro Ala  Ala Pro Val Ala Ser  Ala Pro Ala
       1100                 1105                 1110
   ccg gct  gtc tcg aac gag ctt  ctt gag aag gcc gag  act gtc gtc      3384
   Pro Ala  Val Ser Asn Glu Leu  Leu Glu Lys Ala Glu  Thr Val Val
       1115                 1120                 1125
   atg gag  gtc ctc gcc gcc aag  acc ggc tac gag acc  gac atg atc      3429
   Met Glu  Val Leu Ala Ala Lys  Thr Gly Tyr Glu Thr  Asp Met Ile
       1130                 1135                 1140
   gag gct  gac atg gag ctc gag  acc gag ctc ggc att  gac tcc atc      3474
   Glu Ala  Asp Met Glu Leu Glu  Thr Glu Leu Gly Ile  Asp Ser Ile
       1145                 1150                 1155
   aag cgt  gtc gag atc ctc tcc  gag gtc cag gcc atg  ctc aat gtc      3519
   Lys Arg  Val Glu Ile Leu Ser  Glu Val Gln Ala Met  Leu Asn Val
       1160                 1165                 1170
   gag gcc  aag gat gtc gat gcc  ctc agc cgc act cgc  act gtt ggt      3564
   Glu Ala  Lys Asp Val Asp Ala  Leu Ser Arg Thr Arg  Thr Val Gly
       1175                 1180                 1185
   gag gtt  gtc aac gcc atg aag  gcc gag atc gct ggc  agc tct gcc      3609
   Glu Val  Val Asn Ala Met Lys  Ala Glu Ile Ala Gly  Ser Ser Ala
       1190                 1195                 1200
   ccg gcg  cct gct gcc gct gct  ccg gct ccg gcc aag  gct gcc cct      3654
   Pro Ala  Pro Ala Ala Ala Ala  Pro Ala Pro Ala Lys  Ala Ala Pro
       1205                 1210                 1215
   gcc gcc  gct gcg cct gct gtc  tcg aac gag ctt ctc  gag aag gcc      3699
   Ala Ala  Ala Ala Pro Ala Val  Ser Asn Glu Leu Leu  Glu Lys Ala
       1220                 1225                 1230
   gag acc  gtc gtc atg gag gtc  ctc gcc gcc aag act  ggc tac gag      3744
   Glu Thr  Val Val Met Glu Val  Leu Ala Ala Lys Thr  Gly Tyr Glu
       1235                 1240                 1245
   act gac  atg atc gag tcc gac  atg gag ctc gag act  gag ctc ggc      3789
   Thr Asp  Met Ile Glu Ser Asp  Met Glu Leu Glu Thr  Glu Leu Gly
       1250                 1255                 1260
   att gac  tcc atc aag cgt gtc  gag atc ctc tcc gag  gtt cag gcc      3834
   Ile Asp  Ser Ile Lys Arg Val  Glu Ile Leu Ser Glu  Val Gln Ala
       1265                 1270                 1275
   atg ctc  aac gtc gag gcc aag  gac gtc gac gct ctc  agc cgc act      3879
   Met Leu  Asn Val Glu Ala Lys  Asp Val Asp Ala Leu  Ser Arg Thr
       1280                 1285                 1290
   cgc act  gtg ggt gag gtc gtc  aac gcc atg aag gct  gag atc gct       3924
   Arg Thr  Val Gly Glu Val Val  Asn Ala Met Lys Ala  Glu Ile Ala
       1295                 1300                 1305
   ggt ggc  tct gcc ccg gcg cct  gcc gcc gct gcc cca  ggt ccg gct       3969
   Gly Gly  Ser Ala Pro Ala Pro  Ala Ala Ala Ala Pro  Gly Pro Ala
       1310                 1315                 1320
   gct gcc  gcc cct gcg cct gcc  gcc gcc gcc cct gct  gtc tcg aac       4014
   Ala Ala  Ala Pro Ala Pro Ala  Ala Ala Ala Pro Ala  Val Ser Asn
       1325                 1330                 1335
   gag ctt  ctt gag aag gcc gag  acc gtc gtc atg gag  gtc ctc gcc       4059
   Glu Leu  Leu Glu Lys Ala Glu  Thr Val Val Met Glu  Val Leu Ala
       1340                 1345                 1350
   gcc aag  act ggc tac gag act  gac atg atc gag tcc  gac atg gag       4104
   Ala Lys  Thr Gly Tyr Glu Thr  Asp Met Ile Glu Ser  Asp Met Glu
       1355                 1360                 1365
   ctc gag  acc gag ctc ggc att  gac tcc atc aag cgt  gtc gag att       4149
   Leu Glu  Thr Glu Leu Gly Ile  Asp Ser Ile Lys Arg  Val Glu Ile
       1370                 1375                 1380
   ctc tcc  gag gtc cag gcc atg  ctc aac gtc gag gcc  aag gac gtc       4194
   Leu Ser  Glu Val Gln Ala Met  Leu Asn Val Glu Ala  Lys Asp Val
       1385                 1390                 1395
   gac gct  ctc agc cgc acc cgc  act gtt ggc gag gtc  gtc gat gcc       4239
   Asp Ala  Leu Ser Arg Thr Arg  Thr Val Gly Glu Val  Val Asp Ala
       1400                 1405                 1410
   atg aag  gcc gag atc gct ggt  ggc tct gcc ccg gcg  cct gcc gcc       4284
   Met Lys  Ala Glu Ile Ala Gly  Gly Ser Ala Pro Ala  Pro Ala Ala
       1415                 1420                 1425
   gct gct  cct gct ccg gct gct  gcc gcc cct gcg cct  gcc gcc cct       4329
   Ala Ala  Pro Ala Pro Ala Ala  Ala Ala Pro Ala Pro  Ala Ala Pro
       1430                 1435                 1440
   gcg cct  gct gtc tcg agc gag  ctt ctc gag aag gcc  gag act gtc       4374
   Ala Pro  Ala Val Ser Ser Glu  Leu Leu Glu Lys Ala  Glu Thr Val
       1445                 1450                 1455
   gtc atg  gag gtc ctc gcc gcc  aag act ggc tac gag  act gac atg       4419
   Val Met  Glu Val Leu Ala Ala  Lys Thr Gly Tyr Glu  Thr Asp Met
       1460                 1465                 1470
   atc gag  tcc gac atg gag ctc  gag acc gag ctc ggc  att gac tcc       4464
   Ile Glu  Ser Asp Met Glu Leu  Glu Thr Glu Leu Gly  Ile Asp Ser
       1475                 1480                 1485
   atc aag  cgt gtc gag att ctc  tcc gag gtc cag gcc  atg ctc aac       4509
   Ile Lys  Arg Val Glu Ile Leu  Ser Glu Val Gln Ala  Met Leu Asn
       1490                 1495                 1500
   gtc gag  gcc aag gac gtc gac  gct ctc agc cgc acc  cgc act gtt       4554
   Val Glu  Ala Lys Asp Val Asp  Ala Leu Ser Arg Thr  Arg Thr Val
       1505                 1510                 1515
   ggc gag  gtc gtc gat gcc atg  aag gcc gag atc gct  ggt ggc tct       4599
   Gly Glu  Val Val Asp Ala Met  Lys Ala Glu Ile Ala  Gly Gly Ser
       1520                 1525                 1530
   gcc ccg  gcg cct gcc gcc gct  gct cct gct ccg gct  gct gcc gcc       4644
   Ala Pro  Ala Pro Ala Ala Ala  Ala Pro Ala Pro Ala  Ala Ala Ala
       1535                 1540                 1545
   cct gcg  cct gcc gcc cct gcg  cct gcc gcc cct gcg  cct gct gtc       4689
   Pro Ala  Pro Ala Ala Pro Ala  Pro Ala Ala Pro Ala  Pro Ala Val
       1550                 1555                 1560
   tcg agc  gag ctt ctc gag aag  gcc gag act gtc gtc  atg gag gtc      4734
   Ser Ser  Glu Leu Leu Glu Lys  Ala Glu Thr Val Val  Met Glu Val
       1565                 1570                 1575
   ctc gcc  gcc aag act ggc tac  gag act gac atg att  gag tcc gac      4779
   Leu Ala  Ala Lys Thr Gly Tyr  Glu Thr Asp Met Ile  Glu Ser Asp
       1580                 1585                 1590
   atg gag  ctc gag acc gag ctc  ggc att gac tcc atc  aag cgt gtc      4824
   Met Glu  Leu Glu Thr Glu Leu  Gly Ile Asp Ser Ile  Lys Arg Val
       1595                 1600                 1605
   gag att  ctc tcc gag gtt cag  gcc atg ctc aac gtc  gag gcc aag      4869
   Glu Ile  Leu Ser Glu Val Gln  Ala Met Leu Asn Val  Glu Ala Lys
       1610                 1615                 1620
   gac gtc  gac gct ctc agc cgc  act cgc act gtt ggt  gag gtc gtc      4914
   Asp Val  Asp Ala Leu Ser Arg  Thr Arg Thr Val Gly  Glu Val Val
       1625                 1630                 1635
   gat gcc  atg aag gct gag atc  gct ggc agc tcc gcc  tcg gcg cct      4959
   Asp Ala  Met Lys Ala Glu Ile  Ala Gly Ser Ser Ala  Ser Ala Pro
       1640                 1645                 1650
   gcc gcc  gct gct cct gct ccg  gct gct gcc gct cct  gcg ccc gct      5004
   Ala Ala  Ala Ala Pro Ala Pro  Ala Ala Ala Ala Pro  Ala Pro Ala
       1655                 1660                 1665
   gcc gcc  gcc cct gct gtc tcg  aac gag ctt ctc gag  aaa gcc gag      5049
   Ala Ala  Ala Pro Ala Val Ser  Asn Glu Leu Leu Glu  Lys Ala Glu
       1670                 1675                 1680
   act gtc  gtc atg gag gtc ctc  gcc gcc aag act ggc  tac gag act      5094
   Thr Val  Val Met Glu Val Leu  Ala Ala Lys Thr Gly  Tyr Glu Thr
       1685                 1690                 1695
   gac atg  atc gag tcc gac atg  gag ctc gag act gag  ctc ggc att      5139
   Asp Met  Ile Glu Ser Asp Met  Glu Leu Glu Thr Glu  Leu Gly Ile
       1700                 1705                 1710
   gac tcc  atc aag cgt gtc gag  atc ctc tcc gag gtt  cag gcc atg      5184
   Asp Ser  Ile Lys Arg Val Glu  Ile Leu Ser Glu Val  Gln Ala Met
       1715                 1720                 1725
   ctc aac  gtc gag gcc aag gac  gtc gat gcc ctc agc  cgc acc cgc      5229
   Leu Asn  Val Glu Ala Lys Asp  Val Asp Ala Leu Ser  Arg Thr Arg
       1730                 1735                 1740
   act gtt  ggc gag gtt gtc gat  gcc atg aag gcc gag  atc gct ggt      5274
   Thr Val  Gly Glu Val Val Asp  Ala Met Lys Ala Glu  Ile Ala Gly
       1745                 1750                 1755
   ggc tct  gcc ccg gcg cct gcc  gcc gct gcc cct gct  ccg gct gcc      5319
   Gly Ser  Ala Pro Ala Pro Ala  Ala Ala Ala Pro Ala  Pro Ala Ala
       1760                 1765                 1770
   gcc gcc  cct gct gtc tcg aac  gag ctt ctc gag aag  gcc gag act      5364
   Ala Ala  Pro Ala Val Ser Asn  Glu Leu Leu Glu Lys  Ala Glu Thr
       1775                 1780                 1785
   gtc gtc  atg gag gtc ctc gcc  gcc aag act ggc tac  gag acc gac      5409
   Val Val  Met Glu Val Leu Ala  Ala Lys Thr Gly Tyr  Glu Thr Asp
       1790                 1795                 1800
   atg atc  gag tcc gac atg gag  ctc gag acc gag ctc  ggc att gac      5454
   Met Ile  Glu Ser Asp Met Glu  Leu Glu Thr Glu Leu  Gly Ile Asp
       1805                 1810                 1815
   tcc atc  aag cgt gtc gag att  ctc tcc gag gtt cag  gcc atg ctc      5499
   Ser Ile  Lys Arg Val Glu Ile  Leu Ser Glu Val Gln  Ala Met Leu
       1820                 1825                 1830
   aac gtc  gag gcc aag gac gtc  gat gct ctc agc cgc  act cgc act      5544
   Asn Val  Glu Ala Lys Asp Val  Asp Ala Leu Ser Arg  Thr Arg Thr
       1835                1840                1845
   gtt ggc  gag gtc gtc gat gcc  atg aag gct gag atc  gcc ggc agc      5589
   Val Gly  Glu Val Val Asp Ala  Met Lys Ala Glu Ile  Ala Gly Ser
       1850                1855                1860
   tcc gcc  ccg gcg cct gcc gcc  gct gct cct gct ccg  gct gct gcc      5634
   Ser Ala  Pro Ala Pro Ala Ala  Ala Ala Pro Ala Pro  Ala Ala Ala
       1865                1870                1875
   gct cct  gcg ccc gct gcc gct  gcc cct gct gtc tcg  agc gag ctt      5679
   Ala Pro  Ala Pro Ala Ala Ala  Ala Pro Ala Val Ser  Ser Glu Leu
       1880                1885                1890
   ctc gag  aag gcc gag acc gtc  gtc atg gag gtc ctc  gcc gcc aag      5724
   Leu Glu  Lys Ala Glu Thr Val  Val Met Glu Val Leu  Ala Ala Lys
       1895                1900                1905
   act ggc  tac gag act gac atg  att gag tcc gac atg  gag ctc gag      5769
   Thr Gly  Tyr Glu Thr Asp Met  Ile Glu Ser Asp Met  Glu Leu Glu
       1910                1915                1920
   act gag  ctc ggc att gac tcc  atc aag cgt gtc gag  atc ctc tcc      5814
   Thr Glu  Leu Gly Ile Asp Ser  Ile Lys Arg Val Glu  Ile Leu Ser
       1925                1930                1935
   gag gtt  cag gcc atg ctc aac  gtc gag gcc aag gac  gtc gat gcc      5859
   Glu Val  Gln Ala Met Leu Asn  Val Glu Ala Lys Asp  Val Asp Ala
       1940                1945                1950
   ctc agc  cgc acc cgc act gtt  ggc gag gtt gtc gat  gcc atg aag      5904
   Leu Ser  Arg Thr Arg Thr Val  Gly Glu Val Val Asp  Ala Met Lys
       1955                1960                1965
   gcc gag  atc gct ggt ggc tct  gcc ccg gcg cct gcc  gcc gct gcc      5949
   Ala Glu  Ile Ala Gly Gly Ser  Ala Pro Ala Pro Ala  Ala Ala Ala
       1970                1975                1980
   cct gct  ccg gct gcc gcc gcc  cct gct gtc tcg aac  gag ctt ctt      5994
   Pro Ala  Pro Ala Ala Ala Ala  Pro Ala Val Ser Asn  Glu Leu Leu
       1985                1990                1995
   gag aag  gcc gag acc gtc gtc  atg gag gtc ctc gcc  gcc aag act      6039
   Glu Lys  Ala Glu Thr Val Val  Met Glu Val Leu Ala  Ala Lys Thr
       2000                2005                2010
   ggc tac  gag acc gac atg atc  gag tcc gac atg gag  ctc gag acc      6084
   Gly Tyr  Glu Thr Asp Met Ile  Glu Ser Asp Met Glu  Leu Glu Thr
       2015                2020                2025
   gag ctc  ggc att gac tcc atc  aag cgt gtc gag att  ctc tcc gag      6129
   Glu Leu  Gly Ile Asp Ser Ile  Lys Arg Val Glu Ile  Leu Ser Glu
       2030                2035                2040
   gtt cag  gcc atg ctc aac gtc  gag gcc aag gac gtc  gac gct ctc      6174
   Val Gln  Ala Met Leu Asn Val  Glu Ala Lys Asp Val  Asp Ala Leu
       2045                2050                2055
   agc cgc  act cgc act gtt ggc  gag gtc gtc gat gcc  atg aag gct      6219
   Ser Arg  Thr Arg Thr Val Gly  Glu Val Val Asp Ala  Met Lys Ala
       2060                2065                2070
   gag atc  gct ggt ggc tct gcc  ccg gcg cct gcc gcc  gct gct cct      6264
   Glu Ile  Ala Gly Gly Ser Ala  Pro Ala Pro Ala Ala  Ala Ala Pro
       2075                2080                2085
   gcc tcg  gct ggc gcc gcg cct  gcg gtc aag att gac  tcg gtc cac      6309
   Ala Ser  Ala Gly Ala Ala Pro  Ala Val Lys Ile Asp  Ser Val His
       2090                2095                2100
   ggc gct  gac tgt gat gat ctt  tcc ctg atg cac gcc  aag gtg gtt     6354
   Gly Ala  Asp Cys Asp Asp Leu  Ser Leu Met His Ala  Lys Val Val
       2105                 2110                 2115
   gac atc  cgc cgc ccg gac gag  ctc atc ctg gag cgc  ccc gag aac     6399
   Asp Ile  Arg Arg Pro Asp Glu  Leu Ile Leu Glu Arg  Pro Glu Asn
       2120                 2125                 2130
   cgc ccc  gtt ctc gtt gtc gat  gac ggc agc gag ctc  acc ctc gcc     6444
   Arg Pro  Val Leu Val Val Asp  Asp Gly Ser Glu Leu  Thr Leu Ala
       2135                 2140                 2145
   ctg gtc  cgc gtc ctc ggc gcc  tgc gcc gtt gtc ctg  acc ttt gag     6489
   Leu Val  Arg Val Leu Gly Ala  Cys Ala Val Val Leu  Thr Phe Glu
       2150                 2155                 2160
   ggt ctc  cag ctc gct cag cgc  gct ggt gcc gct gcc  atc cgc cac     6534
   Gly Leu  Gln Leu Ala Gln Arg  Ala Gly Ala Ala Ala  Ile Arg His
       2165                 2170                 2175
   gtg ctc  gcc aag gat ctt tcc  gcg gag agc gcc gag  aag gcc atc     6579
   Val Leu  Ala Lys Asp Leu Ser  Ala Glu Ser Ala Glu  Lys Ala Ile
       2180                 2185                 2190
   aag gag  gcc gag cag cgc ttt  ggc gct ctc ggc ggc  ttc atc tcg     6624
   Lys Glu  Ala Glu Gln Arg Phe  Gly Ala Leu Gly Gly  Phe Ile Ser
       2195                 2200                 2205
   cag cag  gcg gag cgc ttc gag  ccc gcc gaa atc ctc  ggc ttc acg     6669
   Gln Gln  Ala Glu Arg Phe Glu  Pro Ala Glu Ile Leu  Gly Phe Thr
       2210                 2215                 2220
   ctc atg  tgc gcc aag ttc gcc  aag gct tcc ctc tgc  acg gct gtg     6714
   Leu Met  Cys Ala Lys Phe Ala  Lys Ala Ser Leu Cys  Thr Ala Val
       2225                 2230                 2235
   gct ggc  ggc cgc ccg gcc ttt  atc ggt gtg gcg cgc  ctt gac ggc     6759
   Ala Gly  Gly Arg Pro Ala Phe  Ile Gly Val Ala Arg  Leu Asp Gly
       2240                 2245                 2250
   cgc ctc  gga ttc act tcg cag  ggc act tct gac gcg  ctc aag cgt     6804
   Arg Leu  Gly Phe Thr Ser Gln  Gly Thr Ser Asp Ala  Leu Lys Arg
       2255                 2260                 2265
   gcc cag  cgt ggt gcc atc ttt  ggc ctc tgc aag acc  atc ggc ctc     6849
   Ala Gln  Arg Gly Ala Ile Phe  Gly Leu Cys Lys Thr  Ile Gly Leu
       2270                 2275                 2280
   gag tgg  tcc gag tct gac gtc  ttt tcc cgc ggc gtg  gac att gct     6894
   Glu Trp  Ser Glu Ser Asp Val  Phe Ser Arg Gly Val  Asp Ile Ala
       2285                 2290                 2295
   cag ggc  atg cac ccc gag gat  gcc gcc gtg gcg att  gtg cgc gag     6939
   Gln Gly  Met His Pro Glu Asp  Ala Ala Val Ala Ile  Val Arg Glu
       2300                 2305                 2310
   atg gcg  tgc gct gac att cgc  att cgc gag gtc ggc  att ggc gca     6984
   Met Ala  Cys Ala Asp Ile Arg  Ile Arg Glu Val Gly  Ile Gly Ala
       2315                 2320                 2325
   aac cag  cag cgc tgc acg atc  cgt gcc gcc aag ctc  gag acc ggc     7029
   Asn Gln  Gln Arg Cys Thr Ile  Arg Ala Ala Lys Leu  Glu Thr Gly
       2330                 2335                 2340
   aac ccg  cag cgc cag atc gcc  aag gac gac gtg ctg  ctc gtt tct     7074
   Asn Pro  Gln Arg Gln Ile Ala  Lys Asp Asp Val Leu  Leu Val Ser
       2345                 2350                 2355
   ggc ggc  gct cgc ggc atc acg  cct ctt tgc atc cgg  gag atc acg     7119
   Gly Gly  Ala Arg Gly Ile Thr  Pro Leu Cys Ile Arg  Glu Ile Thr
       2360                 2365                 2370
   cgc cag  atc gcg ggc ggc aag  tac att ctg ctt ggc  cgc agc aag      7164
   Arg Gln  Ile Ala Gly Gly Lys  Tyr Ile Leu Leu Gly  Arg Ser Lys
       2375                 2380                 2385
   gtc tct  gcg agc gaa ccg gca  tgg tgc gct ggc atc  act gac gag      7209
   Val Ser  Ala Ser Glu Pro Ala  Trp Cys Ala Gly Ile  Thr Asp Glu
       2390                 2395                 2400
   aag gct  gtg caa aag gct gct  acc cag gag ctc aag  cgc gcc ttt      7254
   Lys Ala  Val Gln Lys Ala Ala  Thr Gln Glu Leu Lys  Arg Ala Phe
       2405                 2410                 2415
   agc gct  ggc gag ggc ccc aag  ccc acg ccc cgc gct  gtc act aag      7299
   Ser Ala  Gly Glu Gly Pro Lys  Pro Thr Pro Arg Ala  Val Thr Lys
       2420                 2425                 2430
   ctt gtg  ggc tct gtt ctt ggc  gct cgc gag gtg cgc  agc tct att      7344
   Leu Val  Gly Ser Val Leu Gly  Ala Arg Glu Val Arg  Ser Ser Ile
       2435                 2440                 2445
   gct gcg  att gaa gcg ctc ggc  ggc aag gcc atc tac  tcg tcg tgc      7389
   Ala Ala  Ile Glu Ala Leu Gly  Gly Lys Ala Ile Tyr  Ser Ser Cys
       2450                 2455                 2460
   gac gtg  aac tct gcc gcc gac  gtg gcc aag gcc gtg  cgc gat gcc      7434
   Asp Val  Asn Ser Ala Ala Asp  Val Ala Lys Ala Val  Arg Asp Ala
       2465                 2470                 2475
   gag tcc  cag ctc ggt gcc cgc  gtc tcg ggc atc gtt  cat gcc tcg      7479
   Glu Ser  Gln Leu Gly Ala Arg  Val Ser Gly Ile Val  His Ala Ser
       2480                 2485                 2490
   ggc gtg  ctc cgc gac cgt ctc  atc gag aag aag ctc  ccc gac gag      7524
   Gly Val  Leu Arg Asp Arg Leu  Ile Glu Lys Lys Leu  Pro Asp Glu
       2495                 2500                 2505
   ttc gac  gcc gtc ttt ggc acc  aag gtc acc ggt ctc  gag aac ctc      7569
   Phe Asp  Ala Val Phe Gly Thr  Lys Val Thr Gly Leu  Glu Asn Leu
       2510                 2515                 2520
   ctc gcc  gcc gtc gac cgc gcc  aac ctc aag cac atg  gtc ctc ttc      7614
   Leu Ala  Ala Val Asp Arg Ala  Asn Leu Lys His Met  Val Leu Phe
       2525                 2530                 2535
   agc tcg  ctc gcc ggc ttc cac  ggc aac gtc ggc cag  tct gac tac      7659
   Ser Ser  Leu Ala Gly Phe His  Gly Asn Val Gly Gln  Ser Asp Tyr
       2540                 2545                 2550
   gcc atg  gcc aac gag gcc ctt  aac aag atg ggc ctc  gag ctc gcc      7704
   Ala Met  Ala Asn Glu Ala Leu  Asn Lys Met Gly Leu  Glu Leu Ala
       2555                 2560                 2565
   aag gac  gtc tcg gtc aag tcg  atc tgc ttc ggt ccc  tgg gac ggt      7749
   Lys Asp  Val Ser Val Lys Ser  Ile Cys Phe Gly Pro  Trp Asp Gly
       2570                 2575                 2580
   ggc atg  gtg acg ccg cag ctc  aag aag cag ttc cag  gag atg ggc      7794
   Gly Met  Val Thr Pro Gln Leu  Lys Lys Gln Phe Gln  Glu Met Gly
       2585                 2590                 2595
   gtg cag  atc atc ccc cgc gag  ggc ggc gct gat acc  gtg gcg cgc      7839
   Val Gln  Ile Ile Pro Arg Glu  Gly Gly Ala Asp Thr  Val Ala Arg
       2600                 2605                 2610
   atc gtg  ctc ggc tcc tcg ccg  gct gag atc ctt gtc  ggc aac tgg      7884
   Ile Val  Leu Gly Ser Ser Pro  Ala Glu Ile Leu Val  Gly Asn Trp
       2615                 2620                 2625
   cgc acc  ccg tcc aag aag gtc  ggc tcg gac acc atc  acc ctg cac      7929
   Arg Thr  Pro Ser Lys Lys Val  Gly Ser Asp Thr Ile  Thr Leu His
       2630                 2635                 2640
   cgc aag att tcc gcc aag tcc aac ccc ttc ctc gag gac cac gtc      7974
   Arg Lys Ile Ser Ala Lys Ser Asn Pro Phe Leu Glu Asp His Val
       2645                2650                2655
   atc cag ggc cgc cgc gtg ctg ccc atg acg ctg gcc att ggc tcg      8019
   Ile Gln Gly Arg Arg Val Leu Pro Met Thr Leu Ala Ile Gly Ser
       2660                2665                2670
   ctc gcg gag acc tgc ctc ggc ctc ttc ccc ggc tac tcg ctc tgg      8064
   Leu Ala Glu Thr Cys Leu Gly Leu Phe Pro Gly Tyr Ser Leu Trp
       2675                2680                2685
   gcc att gac gac gcc cag ctc ttc aag ggt gtc act gtc gac ggc      8109
   Ala Ile Asp Asp Ala Gln Leu Phe Lys Gly Val Thr Val Asp Gly
       2690                2695                2700
   gac gtc aac tgc gag gtg acc ctc acc ccg tcg acg gcg ccc tcg      8154
   Asp Val Asn Cys Glu Val Thr Leu Thr Pro Ser Thr Ala Pro Ser
       2705                2710                2715
   ggc cgc gtc aac gtc cag gcc acg ctc aag acc ttt tcc agc ggc      8199
   Gly Arg Val Asn Val Gln Ala Thr Leu Lys Thr Phe Ser Ser Gly
       2720                2725                2730
   aag ctg gtc ccg gcc tac cgc gcc gtc atc gtg ctc tcc aac cag      8244
   Lys Leu Val Pro Ala Tyr Arg Ala Val Ile Val Leu Ser Asn Gln
       2735                2740                2745
   ggc gcg ccc ccg gcc aac gcc acc atg cag ccg ccc tcg ctc gat      8289
   Gly Ala Pro Pro Ala Asn Ala Thr Met Gln Pro Pro Ser Leu Asp
       2750                2755                2760
   gcc gat ccg gcg ctc cag ggc tcc gtc tac gac ggc aag acc ctc      8334
   Ala Asp Pro Ala Leu Gln Gly Ser Val Tyr Asp Gly Lys Thr Leu
       2765                2770                2775
   ttc cac ggc ccg gcc ttc cgc ggc atc gat gac gtg ctc tcg tgc      8379
   Phe His Gly Pro Ala Phe Arg Gly Ile Asp Asp Val Leu Ser Cys
       2780                2785                2790
   acc aag agc cag ctt gtg gcc aag tgc agc gct gtc ccc ggc tcc      8424
   Thr Lys Ser Gln Leu Val Ala Lys Cys Ser Ala Val Pro Gly Ser
       2795                2800                2805
   gac gcc gct cgc ggc gag ttt gcc acg gac act gac gcc cat gac      8469
   Asp Ala Ala Arg Gly Glu Phe Ala Thr Asp Thr Asp Ala His Asp
       2810                2815                2820
   ccc ttc gtg aac gac ctg gcc ttt cag gcc atg ctc gtc tgg gtg      8514
   Pro Phe Val Asn Asp Leu Ala Phe Gln Ala Met Leu Val Trp Val
       2825                2830                2835
   cgc cgc acg ctc ggc cag gct gcg ctc ccc aac tcg atc cag cgc      8559
   Arg Arg Thr Leu Gly Gln Ala Ala Leu Pro Asn Ser Ile Gln Arg
       2840                2845                2850
   atc gtc cag cac cgc ccg gtc ccg cag gac aag ccc ttc tac att      8604
   Ile Val Gln His Arg Pro Val Pro Gln Asp Lys Pro Phe Tyr Ile
       2855                2860                2865
   acc ctc cgc tcc aac cag tcg ggc ggt cac tcc cag cac aag cac      8649
   Thr Leu Arg Ser Asn Gln Ser Gly Gly His Ser Gln His Lys His
       2870                2875                2880
   gcc ctt cag ttc cac aac gag cag ggc gat ctc ttc att gat gtc      8694
   Ala Leu Gln Phe His Asn Glu Gln Gly Asp Leu Phe Ile Asp Val
       2885                2890                2895
   cag gct tcg gtc atc gcc acg gac agc ctt gcc ttc                  8730
   Gln Ala Ser Val Ile Ala Thr Asp Ser Leu Ala Phe
       2900                2905                2910
<210> 2
   <211> 2910
   <212> PRT
   <213> Schizochytrium sp.
<400> 2
   Met Ala Ala Arg Leu Gln Glu Gln Lys Gly Gly Glu Met Asp Thr Arg
   1               5                   10                  15
   Ile Ala Ile Ile Gly Met Ser Ala Ile Leu Pro Cys Gly Thr Thr Val
               20                  25                  30
   Arg Glu Ser Trp Glu Thr Ile Arg Ala Gly Ile Asp Cys Leu Ser Asp
           35                  40                  45
   Leu Pro Glu Asp Arg Val Asp Val Thr Ala Tyr Phe Asp Pro Val Lys
       50                  55                  60
   Thr Thr Lys Asp Lys Ile Tyr Cys Lys Arg Gly Gly Phe Ile Pro Glu
   65                  70                  75                  80
   Tyr Asp Phe Asp Ala Arg Glu Phe Gly Leu Asn Met Phe Gln Met Glu
                   85                  90                  95
   Asp Ser Asp Ala Asn Gln Thr Ile Ser Leu Leu Lys Val Lys Glu Ala
               100                 105                 110
   Leu Gln Asp Ala Gly Ile Asp Ala Leu Gly Lys Glu Lys Lys Asn Ile
           115                 120                 125
   Gly Cys Val Leu Gly Ile Gly Gly Gly Gln Lys Ser Ser His Glu Phe
       130                 135                 140
   Tyr Ser Arg Leu Asn Tyr Val Val Val Glu Lys Val Leu Arg Lys Met
   145                 150                 155                 160
   Gly Met Pro Glu Glu Asp Val Lys Val Ala Val Glu Lys Tyr Lys Ala
                   165                 170                 175
   Asn Phe Pro Glu Trp Arg Leu Asp Ser Phe Pro Gly Phe Leu Gly Asn
               180                 185                 190
   Val Thr Ala Gly Arg Cys Thr Asn Thr Phe Asn Leu Asp Gly Met Asn
           195                 200                 205
   Cys Val Val Asp Ala Ala Cys Ala Ser Ser Leu Ile Ala Val Lys Val
       210                 215                 220
   Ala Ile Asp Glu Leu Leu Tyr Gly Asp Cys Asp Met Met Val Thr Gly
   225                 230                 235                 240
   Ala Thr Cys Thr Asp Asn Ser Ile Gly Met Tyr Met Ala Phe Ser Lys
                   245                 250                 255
   Thr Pro Val Phe Ser Thr Asp Pro Ser Val Arg Ala Tyr Asp Glu Lys
               260                 265                 270
   Thr Lys Gly Met Leu Ile Gly Glu Gly Ser Ala Met Leu Val Leu Lys
           275                 280                 285
   Arg Tyr Ala Asp Ala Val Arg Asp Gly Asp Glu Ile His Ala Val Ile
       290                 295                 300
   Arg Gly Cys Ala Ser Ser Ser Asp Gly Lys Ala Ala Gly Ile Tyr Thr
   305                 310                 315                 320
   Pro Thr Ile Ser Gly Gln Glu Glu Ala Leu Arg Arg Ala Tyr Asn Arg
                   325                 330                 335
   Ala Cys Val Asp Pro Ala Thr Val Thr Leu Val Glu Gly His Gly Thr
               340                 345                 350
   Gly Thr Pro Val Gly Asp Arg Ile Glu Leu Thr Ala Leu Arg Asn Leu
           355                 360                 365
   Phe Asp Lys Ala Tyr Gly Glu Gly Asn Thr Glu Lys Val Ala Val Gly
       370                 375                 380
   Ser Ile Lys Ser Ser Ile Gly His Leu Lys Ala Val Ala Gly Leu Ala
   385                 390                 395                 400
   Gly Met Ile Lys Val Ile Met Ala Leu Lys His Lys Thr Leu Pro Gly
                   405                 410                 415
   Thr Ile Asn Val Asp Asn Pro Pro Asn Leu Tyr Asp Asn Thr Pro Ile
               420                 425                 430
   Asn Glu Ser Ser Leu Tyr Ile Asn Thr Met Asn Arg Pro Trp Phe Pro
           435                 440                 445
   Pro Pro Gly Val Pro Arg Arg Ala Gly Ile Ser Ser Phe Gly Phe Gly
       450                 455                 460
   Gly Ala Asn Tyr His Ala Val Leu Glu Glu Ala Glu Pro Glu His Thr
   465                 470                 475                 480
   Thr Ala Tyr Arg Leu Asn Lys Arg Pro Gln Pro Val Leu Met Met Ala
                   485                 490                 495
   Ala Thr Pro Ala Ala Leu Gln Ser Leu Cys Glu Ala Gln Leu Lys Glu
               500                 505                 510
   Phe Glu Ala Ala Ile Lys Glu Asn Glu Thr Val Lys Asn Thr Ala Tyr
           515                 520                 525
   Ile Lys Cys Val Lys Phe Gly Glu Gln Phe Lys Phe Pro Gly Ser Ile
       530                 535                 540
   Pro Ala Thr Asn Ala Arg Leu Gly Phe Leu Val Lys Asp Ala Glu Asp
   545 550 555 560
   Ala Cys Ser Thr Leu Arg Ala Ile Cys Ala Gln Phe Ala Lys Asp Val
   565 570 575
   Thr Lys Glu Ala Trp Arg Leu Pro Arg Glu Gly Val Ser Phe Arg Ala
   580 585 590
   Lys Gly Ile Ala Thr Asn Gly Ala Val Ala Ala Leu Phe Ser Gly Gln
   595 600 605
   Gly Ala Gln Tyr Thr His Met Phe Ser Glu Val Ala Met Asn Trp Pro
   610 615 620
   Gln Phe Arg Gln Ser Ile Ala Ala Met Asp Ala Ala Gln Ser Lys Val
   625 630 635 640
   Ala Gly Ser Asp Lys Asp Phe Glu Arg Val Ser Gln Val Leu Tyr Pro
   645 650 655
   Arg Lys Pro Tyr Glu Arg Glu Pro Glu Gln Asn Pro Lys Lys Ile Ser
   660 665 670
   Leu Thr Ala Tyr Ser Gln Pro Ser Thr Leu Ala Cys Ala Leu Gly Ala
   675 680 685
   Phe Glu Ile Phe Lys Glu Ala Gly Phe Thr Pro Asp Phe Ala Ala Gly
   690 695 700
   His Ser Leu Gly Glu Phe Ala Ala Leu Tyr Ala Ala Gly Cys Val Asp
   705 710 715 720
   Arg Asp Glu Leu Phe Glu Leu Val Cys Arg Arg Ala Arg Ile Met Gly
   725 730 735
   Gly Lys Asp Ala Pro Ala Thr Pro Lys Gly Cys Met Ala Ala Val Ile
   740 745 750
   Gly Pro Asn Ala Glu Asn Ile Lys Val Gln Ala Ala Asn Val Trp Leu
   755 760 765
   Gly Asn Ser Asn Ser Pro Ser Gln Thr Val Ile Thr Gly Ser Val Glu
   770 775 780
   Gly Ile Gln Ala Glu Ser Ala Arg Leu Gln Lys Glu Gly Phe Arg Val
   785 790 795 800
   Val Pro Leu Ala Cys Glu Ser Ala Phe His Ser Pro Gln Met Glu Asn
   805 810 815
   Ala Ser Ser Ala Phe Lys Asp Val Ile Ser Lys Val Ser Phe Arg Thr
   820 825 830
   Pro Lys Ala Glu Thr Lys Leu Phe Ser Asn Val Ser Gly Glu Thr Tyr
   835 840 845
   Pro Thr Asp Ala Arg Glu Met Leu Thr Gln His Met Thr Ser Ser Val
   850 855 860
   Lys Phe Leu Thr Gln Val Arg Asn Met His Gln Ala Gly Ala Arg Ile
   865 870 875 880
   Phe Val Glu Phe Gly Pro Lys Gln Val Leu Ser Lys Leu Val Ser Glu
   885 890 895
   Thr Leu Lys Asp Asp Pro Ser Val Val Thr Val Ser Val Asn Pro Ala
   900 905 910
   Ser Gly Thr Asp Ser Asp Ile Gln Leu Arg Asp Ala Ala Val Gln Leu
   915 920 925
   Val Val Ala Gly Val Asn Leu Gln Gly Phe Asp Lys Trp Asp Ala Pro
   930 935 940
   Asp Ala Thr Arg Met Gln Ala Ile Lys Lys Lys Arg Thr Thr Leu Arg
   945 950 955 960
   Leu Ser Ala Ala Thr Tyr Val Ser Asp Lys Thr Lys Lys Val Arg Asp
   965 970 975
   Ala Ala Met Asn Asp Gly Arg Cys Val Thr Tyr Leu Lys Gly Ala Ala
   980 985 990
   Pro Leu Ile Lys Ala Pro Glu Pro Val Val Asp Glu Ala Ala Lys Arg
   995 1000 1005
   Glu Ala Glu Arg Leu Gln Lys Glu Leu Gln Asp Ala Gln Arg Gln
   1010 1015 1020
   Leu Asp Asp Ala Lys Arg Ala Ala Ala Glu Ala Asn Ser Lys Leu
   1025 1030 1035
   Ala Ala Ala Lys Glu Glu Ala Lys Thr Ala Ala Ala Ser Ala Lys
   1040 1045 1050
   Pro Ala Val Asp Thr Ala Val Val Glu Lys His Arg Ala Ile Leu
   1055 1060 1065
   Lys Ser Met Leu Ala Glu Leu Asp Gly Tyr Gly Ser Val Asp Ala
   1070 1075 1080
   Ser Ser Leu Gln Gln Gln Gln Gln Gln Gln Thr Ala Pro Ala Pro
   1085 1090 1095
   Val Lys Ala Ala Ala Pro Ala Ala Pro Val Ala Ser Ala Pro Ala
   1100 1105 1110
   Pro Ala Val Ser Asn Glu Leu Leu Glu Lys Ala Glu Thr Val Val
   1115 1120 1125
   Met Glu Val Leu Ala Ala Lys Thr Gly Tyr Glu Thr Asp Met Ile
   1130 1135 1140
   Glu Ala Asp Met Glu Leu Glu Thr Glu Leu Gly Ile Asp Ser Ile
   1145 1150 1155
   Lys Arg Val Glu Ile Leu Ser Glu Val Gln Ala Met Leu Asn Val
   1160 1165 1170
   Glu Ala Lys Asp Val Asp Ala Leu Ser Arg Thr Arg Thr Val Gly
   1175 1180 1185
   Glu Val Val Asn Ala Met Lys Ala Glu Ile Ala Gly Ser Ser Ala
   1190 1195 1200
   Pro Ala Pro Ala Ala Ala Ala Pro Ala Pro Ala Lys Ala Ala Pro
   1205 1210 1215
   Ala Ala Ala Ala Pro Ala Val Ser Asn Glu Leu Leu Glu Lys Ala
   1220 1225 1230
   Glu Thr Val Val Met Glu Val Leu Ala Ala Lys Thr Gly Tyr Glu
   1235 1240 1245
   Thr Asp Met Ile Glu Ser Asp Met Glu Leu Glu Thr Glu Leu Gly
   1250 1255 1260
   Ile Asp Ser Ile Lys Arg Val Glu Ile Leu Ser Glu Val Gln Ala
   1265 1270 1275
   Met Leu Asn Val Glu Ala Lys Asp Val Asp Ala Leu Ser Arg Thr
   1280 1285 1290
   Arg Thr Val Gly Glu Val Val Asn Ala Met Lys Ala Glu Ile Ala
   1295 1300 1305
   Gly Gly Ser Ala Pro Ala Pro Ala Ala Ala Ala Pro Gly Pro Ala
   1310 1315 1320
   Ala Ala Ala Pro Ala Pro Ala Ala Ala Ala Pro Ala Val Ser Asn
   1325 1330 1335
   Glu Leu Leu Glu Lys Ala Glu Thr Val Val Met Glu Val Leu Ala
   1340 1345 1350
   Ala Lys Thr Gly Tyr Glu Thr Asp Met Ile Glu Ser Asp Met Glu
   1355 1360 1365
   Leu Glu Thr Glu Leu Gly Ile Asp Ser Ile Lys Arg Val Glu Ile
   1370 1375 1380
   Leu Ser Glu Val Gln Ala Met Leu Asn Val Glu Ala Lys Asp Val
   1385 1390 1395
   Asp Ala Leu Ser Arg Thr Arg Thr Val Gly Glu Val Val Asp Ala
   1400 1405 1410
   Met Lys Ala Glu Ile Ala Gly Gly Ser Ala Pro Ala Pro Ala Ala
   1415 1420 1425
   Ala Ala Pro Ala Pro Ala Ala Ala Ala Pro Ala Pro Ala Ala Pro
   1430 1435 1440
   Ala Pro Ala Val Ser Ser Glu Leu Leu Glu Lys Ala Glu Thr Val
   1445 1450 1455
   Val Met Glu Val Leu Ala Ala Lys Thr Gly Tyr Glu Thr Asp Met
   1460 1465 1470
   Ile Glu Ser Asp Met Glu Leu Glu Thr Glu Leu Gly Ile Asp Ser
   1475 1480 1485
   Ile Lys Arg Val Glu Ile Leu Ser Glu Val Gln Ala Met Leu Asn
   1490 1495 1500
   Val Glu Ala Lys Asp Val Asp Ala Leu Ser Arg Thr Arg Thr Val
   1505 1510 1515
   Gly Glu Val Val Asp Ala Met Lys Ala Glu Ile Ala Gly Gly Ser
   1520 1525 1530
   Ala Pro Ala Pro Ala Ala Ala Ala Pro Ala Pro Ala Ala Ala Ala
   1535 1540 1545
   Pro Ala Pro Ala Ala Pro Ala Pro Ala Ala Pro Ala Pro Ala Val
   1550 1555 1560
   Ser Ser Glu Leu Leu Glu Lys Ala Glu Thr Val Val Met Glu Val
   1565 1570 1575
   Leu Ala Ala Lys Thr Gly Tyr Glu Thr Asp Met Ile Glu Ser Asp
   1580 1585 1590
   Met Glu Leu Glu Thr Glu Leu Gly Ile Asp Ser Ile Lys Arg Val
   1595 1600 1605
   Glu Ile Leu Ser Glu Val Gln Ala Met Leu Asn Val Glu Ala Lys
   1610 1615 1620
   Asp Val Asp Ala Leu Ser Arg Thr Arg Thr Val Gly Glu Val Val
   1625 1630 1635
   Asp Ala Met Lys Ala Glu Ile Ala Gly Ser Ser Ala Ser Ala Pro
   1640 1645 1650
   Ala Ala Ala Ala Pro Ala Pro Ala Ala Ala Ala Pro Ala Pro Ala
   1655 1660 1665
   Ala Ala Ala Pro Ala Val Ser Asn Glu Leu Leu Glu Lys Ala Glu
   1670 1675 1680
   Thr Val Val Met Glu Val Leu Ala Ala Lys Thr Gly Tyr Glu Thr
   1685 1690 1695
   Asp Met Ile Glu Ser Asp Met Glu Leu Glu Thr Glu Leu Gly Ile
   1700 1705 1710
   Asp Ser Ile Lys Arg Val Glu Ile Leu Ser Glu Val Gln Ala Met
   1715 1720 1725
   Leu Asn Val Glu Ala Lys Asp Val Asp Ala Leu Ser Arg Thr Arg
   1730 1735 1740
   Thr Val Gly Glu Val Val Asp Ala Met Lys Ala Glu Ile Ala Gly
   1745 1750 1755
   Gly Ser Ala Pro Ala Pro Ala Ala Ala Ala Pro Ala Pro Ala Ala
   1760 1765 1770
   Ala Ala Pro Ala Val Ser Asn Glu Leu Leu Glu Lys Ala Glu Thr
   1775 1780 1785
   Val Val Met Glu Val Leu Ala Ala Lys Thr Gly Tyr Glu Thr Asp
   1790 1795 1800
   Met Ile Glu Ser Asp Met Glu Leu Glu Thr Glu Leu Gly Ile Asp
   1805 1810 1815
   Ser Ile Lys Arg Val Glu Ile Leu Ser Glu Val Gln Ala Met Leu
   1820 1825 1830
   Asn Val Glu Ala Lys Asp Val Asp Ala Leu Ser Arg Thr Arg Thr
   1835 1840 1845
   Val Gly Glu Val Val Asp Ala Met Lys Ala Glu Ile Ala Gly Ser
   1850 1855 1860
   Ser Ala Pro Ala Pro Ala Ala Ala Ala Pro Ala Pro Ala Ala Ala
   1865 1870 1875
   Ala Pro Ala Pro Ala Ala Ala Ala Pro Ala Val Ser Ser Glu Leu
   1880 1885 1890
   Leu Glu Lys Ala Glu Thr Val Val Met Glu Val Leu Ala Ala Lys
   1895 1900 1905
   Thr Gly Tyr Glu Thr Asp Met Ile Glu Ser Asp Met Glu Leu Glu
   1910 1915 1920
   Thr Glu Leu Gly Ile Asp Ser Ile Lys Arg Val Glu Ile Leu Ser
   1925 1930 1935
   Glu Val Gln Ala Met Leu Asn Val Glu Ala Lys Asp Val Asp Ala
   1940 1945 1950
   Leu Ser Arg Thr Arg Thr Val Gly Glu Val Val Asp Ala Met Lys
   1955 1960 1965
   Ala Glu Ile Ala Gly Gly Ser Ala Pro Ala Pro Ala Ala Ala Ala
   1970 1975 1980
   Pro Ala Pro Ala Ala Ala Ala Pro Ala Val Ser Asn Glu Leu Leu
   1985 1990 1995
   Glu Lys Ala Glu Thr Val Val Met Glu Val Leu Ala Ala Lys Thr
   2000 2005 2010
   Gly Tyr Glu Thr Asp Met Ile Glu Ser Asp Met Glu Leu Glu Thr
   2015 2020 2025
   Glu Leu Gly Ile Asp Ser Ile Lys Arg Val Glu Ile Leu Ser Glu
   2030 2035 2040
   Val Gln Ala Met Leu Asn Val Glu Ala Lys Asp Val Asp Ala Leu
   2045 2050 2055
   Ser Arg Thr Arg Thr Val Gly Glu Val Val Asp Ala Met Lys Ala
   2060 2065 2070
   Glu Ile Ala Gly Gly Ser Ala Pro Ala Pro Ala Ala Ala Ala Pro
   2075 2080 2085
   Ala Ser Ala Gly Ala Ala Pro Ala Val Lys Ile Asp Ser Val His
   2090 2095 2100
   Gly Ala Asp Cys Asp Asp Leu Ser Leu Met His Ala Lys Val Val
   2105 2110 2115
   Asp Ile Arg Arg Pro Asp Glu Leu Ile Leu Glu Arg Pro Glu Asn
   2120 2125 2130
   Arg Pro Val Leu Val Val Asp Asp Gly Ser Glu Leu Thr Leu Ala
   2135 2140 2145
   Leu Val Arg Val Leu Gly Ala Cys Ala Val Val Leu Thr Phe Glu
   2150 2155 2160
   Gly Leu Gln Leu Ala Gln Arg Ala Gly Ala Ala Ala Ile Arg His
   2165 2170 2175
   Val Leu Ala Lys Asp Leu Ser Ala Glu Ser Ala Glu Lys Ala Ile
   2180 2185 2190
   Lys Glu Ala Glu Gln Arg Phe Gly Ala Leu Gly Gly Phe Ile Ser
   2195 2200 2205
   Gln Gln Ala Glu Arg Phe Glu Pro Ala Glu Ile Leu Gly Phe Thr
   2210 2215 2220
   Leu Met Cys Ala Lys Phe Ala Lys Ala Ser Leu Cys Thr Ala Val
   2225 2230 2235
   Ala Gly Gly Arg Pro Ala Phe Ile Gly Val Ala Arg Leu Asp Gly
   2240 2245 2250
   Arg Leu Gly Phe Thr Ser Gln Gly Thr Ser Asp Ala Leu Lys Arg
   2255 2260 2265
   Ala Gln Arg Gly Ala Ile Phe Gly Leu Cys Lys Thr Ile Gly Leu
   2270 2275 2280
   Glu Trp Ser Glu Ser Asp Val Phe Ser Arg Gly Val Asp Ile Ala
   2285 2290 2295
   Gln Gly Met His Pro Glu Asp Ala Ala Val Ala Ile Val Arg Glu
   2300 2305 2310
   Met Ala Cys Ala Asp Ile Arg Ile Arg Glu Val Gly Ile Gly Ala
   2315 2320 2325
   Asn Gln Gln Arg Cys Thr Ile Arg Ala Ala Lys Leu Glu Thr Gly
   2330 2335 2340
   Asn Pro Gln Arg Gln Ile Ala Lys Asp Asp Val Leu Leu Val Ser
   2345 2350 2355
   Gly Gly Ala Arg Gly Ile Thr Pro Leu Cys Ile Arg Glu Ile Thr
   2360 2365 2370
   Arg Gln Ile Ala Gly Gly Lys Tyr Ile Leu Leu Gly Arg Ser Lys
   2375 2380 2385
   Val Ser Ala Ser Glu Pro Ala Trp Cys Ala Gly Ile Thr Asp Glu
   2390 2395 2400
   Lys Ala Val Gln Lys Ala Ala Thr Gln Glu Leu Lys Arg Ala Phe
   2405 2410 2415
   Ser Ala Gly Glu Gly Pro Lys Pro Thr Pro Arg Ala Val Thr Lys
   2420 2425 2430
   Leu Val Gly Ser Val Leu Gly Ala Arg Glu Val Arg Ser Ser Ile
   2435 2440 2445
   Ala Ala Ile Glu Ala Leu Gly Gly Lys Ala Ile Tyr Ser Ser Cys
   2450 2455 2460
   Asp Val Asn Ser Ala Ala Asp Val Ala Lys Ala Val Arg Asp Ala
   2465 2470 2475
   Glu Ser Gln Leu Gly Ala Arg Val Ser Gly Ile Val His Ala Ser
   2480 2485 2490
   Gly Val Leu Arg Asp Arg Leu Ile Glu Lys Lys Leu Pro Asp Glu
   2495 2500 2505
   Phe Asp Ala Val Phe Gly Thr Lys Val Thr Gly Leu Glu Asn Leu
   2510 2515 2520
   Leu Ala Ala Val Asp Arg Ala Asn Leu Lys His Met Val Leu Phe
   2525 2530 2535
   Ser Ser Leu Ala Gly Phe His Gly Asn Val Gly Gln Ser Asp Tyr
   2540 2545 2550
   Ala Met Ala Asn Glu Ala Leu Asn Lys Met Gly Leu Glu Leu Ala
   2555 2560 2565
   Lys Asp Val Ser Val Lys Ser Ile Cys Phe Gly Pro Trp Asp Gly
   2570 2575 2580
   Gly Met Val Thr Pro Gln Leu Lys Lys Gln Phe Gln Glu Met Gly
   2585 2590 2595
   Val Gln Ile Ile Pro Arg Glu Gly Gly Ala Asp Thr Val Ala Arg
   2600 2605 2610
   Ile Val Leu Gly Ser Ser Pro Ala Glu Ile Leu Val Gly Asn Trp
   2615 2620 2625
   Arg Thr Pro Ser Lys Lys Val Gly Ser Asp Thr Ile Thr Leu His
   2630 2635 2640
   Arg Lys Ile Ser Ala Lys Ser Asn Pro Phe Leu Glu Asp His Val
   2645 2650 2655
   Ile Gln Gly Arg Arg Val Leu Pro Met Thr Leu Ala Ile Gly Ser
   2660 2665 2670
   Leu Ala Glu Thr Cys Leu Gly Leu Phe Pro Gly Tyr Ser Leu Trp
   2675 2680 2685
   Ala Ile Asp Asp Ala Gln Leu Phe Lys Gly Val Thr Val Asp Gly
   2690 2695 2700
   Asp Val Asn Cys Glu Val Thr Leu Thr Pro Ser Thr Ala Pro Ser
   2705 2710 2715
   Gly Arg Val Asn Val Gln Ala Thr Leu Lys Thr Phe Ser Ser Gly
   2720 2725 2730
   Lys Leu Val Pro Ala Tyr Arg Ala Val Ile Val Leu Ser Asn Gln
   2735 2740 2745
   Gly Ala Pro Pro Ala Asn Ala Thr Met Gln Pro Pro Ser Leu Asp
   2750 2755 2760
   Ala Asp Pro Ala Leu Gln Gly Ser Val Tyr Asp Gly Lys Thr Leu
   2765 2770 2775
   Phe His Gly Pro Ala Phe Arg Gly Ile Asp Asp Val Leu Ser Cys
   2780 2785 2790
   Thr Lys Ser Gln Leu Val Ala Lys Cys Ser Ala Val Pro Gly Ser
   2795 2800 2805
   Asp Ala Ala Arg Gly Glu Phe Ala Thr Asp Thr Asp Ala His Asp
   2810 2815 2820
   Pro Phe Val Asn Asp Leu Ala Phe Gln Ala Met Leu Val Trp Val
   2825 2830 2835
   Arg Arg Thr Leu Gly Gln Ala Ala Leu Pro Asn Ser Ile Gln Arg
   2840 2845 2850
   Ile Val Gln His Arg Pro Val Pro Gln Asp Lys Pro Phe Tyr Ile
   2855 2860 2865
   Thr Leu Arg Ser Asn Gln Ser Gly Gly His Ser Gln His Lys His
   2870 2875 2880
   Ala Leu Gln Phe His Asn Glu Gln Gly Asp Leu Phe Ile Asp Val
   2885 2890 2895
   Gln Ala Ser Val Ile Ala Thr Asp Ser Leu Ala Phe
   2900 2905 2910
<210> 3
   <211> 6177
   <212> DNA
   <213> Schizochytrium sp.
<220>
   <221> CDS
   <222> (1)..(6177)
<400> 3
   atg gcc gct cgg aat gtg agc gcc gcg cat gag atg cac gat gaa aag        48
   Met Ala Ala Arg Asn Val Ser Ala Ala His Glu Met His Asp Glu Lys
   1               5                   10                  15
   cgc atc gcc gtc gtc ggc atg gcc gtc cag tac gcc gga tgc aaa acc        96
   Arg Ile Ala Val Val Gly Met Ala Val Gln Tyr Ala Gly Cys Lys Thr
               20                  25                  30
   aag gac gag ttc tgg gag gtg ctc atg aac ggc aag gtc gag tcc aag       144
   Lys Asp Glu Phe Trp Glu Val Leu Met Asn Gly Lys Val Glu Ser Lys
           35                  40                  45
   gtg atc agc gac aaa cga ctc ggc tcc aac tac cgc gcc gag cac tac        192
   Val Ile Ser Asp Lys Arg Leu Gly Ser Asn Tyr Arg Ala Glu His Tyr
       50                  55                  60
   aaa gca gag cgc agc aag tat gcc gac acc ttt tgc aac gaa acg tac        240
   Lys Ala Glu Arg Ser Lys Tyr Ala Asp Thr Phe Cys Asn Glu Thr Tyr
   65                  70                  75                  80
   ggc acc ctt gac gag aac gag atc gac aac gag cac gaa ctc ctc ctc        288
   Gly Thr Leu Asp Glu Asn Glu Ile Asp Asn Glu His Glu Leu Leu Leu
                   85                  90                  95
   aac ctc gcc aag cag gca ctc gca gag aca tcc gtc aaa gac tcg aca        336
   Asn Leu Ala Lys Gln Ala Leu Ala Glu Thr Ser Val Lys Asp Ser Thr
               100                 105                 110
   cgc tgc ggc atc gtc agc ggc tgc ctc tcg ttc ccc atg gac aac ctc        384
   Arg Cys Gly Ile Val Ser Gly Cys Leu Ser Phe Pro Met Asp Asn Leu
           115                 120                 125
   cag ggt gaa ctc ctc aac gtg tac caa aac cat gtc gag aaa aag ctc        432
   Gln Gly Glu Leu Leu Asn Val Tyr Gln Asn His Val Glu Lys Lys Leu
       130                 135                 140
   ggg gcc cgc gtc ttc aag gac gcc tcc cat tgg tcc gaa cgc gag cag        480
   Gly Ala Arg Val Phe Lys Asp Ala Ser His Trp Ser Glu Arg Glu Gln
   145                 150                 155                 160
   tcc aac aaa ccc gag gcc ggt gac cgc cgc atc ttc atg gac ccg gcc        528
   Ser Asn Lys Pro Glu Ala Gly Asp Arg Arg Ile Phe Met Asp Pro Ala
                   165                 170                 175
   tcc ttc gtc gcc gaa gaa ctc aac ctc ggc gcc ctt cac tac tcc gtc        576
   Ser Phe Val Ala Glu Glu Leu Asn Leu Gly Ala Leu His Tyr Ser Val
               180                 185                 190
   gac gca gca tgc gcc acg gcg ctc tac gtg ctc cgc ctc gcg cag gat        624
   Asp Ala Ala Cys Ala Thr Ala Leu Tyr Val Leu Arg Leu Ala Gln Asp
           195                 200                 205
   cat ctc gtc tcc ggc gcc gcc gac gtc atg ctc tgc ggt gcc acc tgc        672
   His Leu Val Ser Gly Ala Ala Asp Val Met Leu Cys Gly Ala Thr Cys
       210                 215                 220
   ctg ccg gag ccc ttt ttc atc ctt tcg ggc ttt tcc acc ttc cag gcc        720
   Leu Pro Glu Pro Phe Phe Ile Leu Ser Gly Phe Ser Thr Phe Gln Ala
   225                 230                 235                 240
   atg ccc gtc ggc acg ggc cag aac gtg tcc atg ccg ctg cac aag gac        768
   Met Pro Val Gly Thr Gly Gln Asn Val Ser Met Pro Leu His Lys Asp
                   245                 250                 255
   agc cag ggc ctc acc ccg ggt gag ggc ggc tcc atc atg gtc ctc aag        816
   Ser Gln Gly Leu Thr Pro Gly Glu Gly Gly Ser Ile Met Val Leu Lys
               260                 265                 270
   cgt ctc gat gat gcc atc cgc gac ggc gac cac att tac ggc acc ctt        864
   Arg Leu Asp Asp Ala Ile Arg Asp Gly Asp His Ile Tyr Gly Thr Leu
           275                 280                 285
   ctc ggc gcc aat gtc agc aac tcc ggc aca ggt ctg ccc ctc aag ccc        912
   Leu Gly Ala Asn Val Ser Asn Ser Gly Thr Gly Leu Pro Leu Lys Pro
       290                 295                 300
   ctt ctc ccc agc gag aaa aag tgc ctc atg gac acc tac acg cgc att        960
   Leu Leu Pro Ser Glu Lys Lys Cys Leu Met Asp Thr Tyr Thr Arg Ile
   305                 310                 315                 320
   aac gtg cac ccg cac aag att cag tac gtc gag tgc cac gcc acc ggc       1008
   Asn Val His Pro His Lys Ile Gln Tyr Val Glu Cys His Ala Thr Gly
                   325                 330                 335
   acg ccc cag ggt gat cgt gtg gaa atc gac gcc gtc aag gcc tgc ttt     1056
   Thr Pro Gln Gly Asp Arg Val Glu Ile Asp Ala Val Lys Ala Cys Phe
               340                 345                 350
   gaa ggc aag gtc ccc cgt ttc ggt acc aca aag ggc aac ttt gga cac     1104
   Glu Gly Lys Val Pro Arg Phe Gly Thr Thr Lys Gly Asn Phe Gly His
           355                 360                 365
   acc cts gyc gca gcc ggc ttt gcc ggt atg tgc aag gtc ctc ctc tcc     1152
   Thr Xaa Xaa Ala Ala Gly Phe Ala Gly Met Cys Lys Val Leu Leu Ser
       370                 375                 380
   atg aag cat ggc atc atc ccg ccc acc ccg ggt atc gat gac gag acc     1200
   Met Lys His Gly Ile Ile Pro Pro Thr Pro Gly Ile Asp Asp Glu Thr
   385                 390                 395                 400
   aag atg gac cct ctc gtc gtc tcc ggt gag gcc atc cca tgg cca gag     1248
   Lys Met Asp Pro Leu Val Val Ser Gly Glu Ala Ile Pro Trp Pro Glu
                   405                 410                 415
   acc aac ggc gag ccc aag cgc gcc ggt ctc tcg gcc ttt ggc ttt ggt     1296
   Thr Asn Gly Glu Pro Lys Arg Ala Gly Leu Ser Ala Phe Gly Phe Gly
               420                 425                 430
   ggc acc aac gcc cat gcc gtc ttt gag gag cat gac ccc tcc aac gcc     1344
   Gly Thr Asn Ala His Ala Val Phe Glu Glu His Asp Pro Ser Asn Ala
           435                 440                 445
   gcc tgc acg ggc cac gac tcc att tct gcg ctc tcg gcc cgc tgc ggc     1392
   Ala Cys Thr Gly His Asp Ser Ile Ser Ala Leu Ser Ala Arg Cys Gly
       450                 455                 460
   ggt gaa agc aac atg cgc atc gcc atc act ggt atg gac gcc acc ttt     1440
   Gly Glu Ser Asn Met Arg Ile Ala Ile Thr Gly Met Asp Ala Thr Phe
   465                 470                 475                 480
   ggc gct ctc aag gga ctc gac gcc ttc gag cgc gcc att tac acc ggc     1488
   Gly Ala Leu Lys Gly Leu Asp Ala Phe Glu Arg Ala Ile Tyr Thr Gly
                   485                 490                 495
   gct cac ggt gcc atc cca ctc cca gaa aag cgc tgg cgc ttt ctc ggc     1536
   Ala His Gly Ala Ile Pro Leu Pro Glu Lys Arg Trp Arg Phe Leu Gly
               500                 505                 510
   aag gac aag gac ttt ctt gac ctc tgc ggc gtc aag gcc acc ccg cac     1584
   Lys Asp Lys Asp Phe Leu Asp Leu Cys Gly Val Lys Ala Thr Pro His
           515                 520                 525
   ggc tgc tac att gaa gat gtt gag gtc gac ttc cag cgc ctc cgc acg     1632
   Gly Cys Tyr Ile Glu Asp Val Glu Val Asp Phe Gln Arg Leu Arg Thr
       530                 535                 540
   ccc atg acc cct gaa gac atg ctc ctc cct cag cag ctt ctg gcc gtc     1680
   Pro Met Thr Pro Glu Asp Met Leu Leu Pro Gln Gln Leu Leu Ala Val
   545                 550                 555                 560
   acc acc att gac cgc gcc atc ctc gac tcg gga atg aaa aag ggt ggc     1728
   Thr Thr Ile Asp Arg Ala Ile Leu Asp Ser Gly Met Lys Lys Gly Gly
                   565                 570                 575
   aat gtc gcc gtc ttt gtc ggc ctc ggc acc gac ctc gag ctc tac cgt     1776
   Asn Val Ala Val Phe Val Gly Leu Gly Thr Asp Leu Glu Leu Tyr Arg
               580                 585                 590
   cac cgt gct cgc gtc gct ctc aag gag cgc gtc cgc cct gaa gcc tcc     1824
   His Arg Ala Arg Val Ala Leu Lys Glu Arg Val Arg Pro Glu Ala Ser
           595                 600                 605
   aag aag ctc aat gac atg atg cag tac att aac gac tgc ggc aca tcc     1872
   Lys Lys Leu Asn Asp Met Met Gln Tyr Ile Asn Asp Cys Gly Thr Ser
       610                 615                 620
   aca tcg tac acc tcg tac att ggc aac ctc gtc gcc acg cgc gtc tcg      1920
   Thr Ser Tyr Thr Ser Tyr Ile Gly Asn Leu Val Ala Thr Arg Val Ser
   625                 630                 635                 640
   tcg cag tgg ggc ttc acg ggc ccc tcc ttt acg atc acc gag ggc aac      1968
   Ser Gln Trp Gly Phe Thr Gly Pro Ser Phe Thr Ile Thr Glu Gly Asn
               645                 650                 655
   aac tcc gtc tac cgc tgc gcc gag ctc ggc aag tac ctc ctc gag acc      2016
   Asn Ser Val Tyr Arg Cys Ala Glu Leu Gly Lys Tyr Leu Leu Glu Thr
           660                 665                 670
   ggc gag gtc gat ggc gtc gtc gtt gcg ggt gtc gat ctc tgc ggc agt      2064
   Gly Glu Val Asp Gly Val Val Val Ala Gly Val Asp Leu Cys Gly Ser
       675                 680                 685
   gcc gaa aac ctt tac gtc aag tct cgc cgc ttc aag gtg tcc acc tcc      2112
   Ala Glu Asn Leu Tyr Val Lys Ser Arg Arg Phe Lys Val Ser Thr Ser
       690                 695                 700
   gat acc ccg cgc gcc agc ttt gac gcc gcc gcc gat ggc tac ttt gtc      2160
   Asp Thr Pro Arg Ala Ser Phe Asp Ala Ala Ala Asp Gly Tyr Phe Val
   705                 710                 715                 720
   ggc gag ggc tgc ggt gcc ttt gtg ctc aag cgt gag act agc tgc acc      2208
   Gly Glu Gly Cys Gly Ala Phe Val Leu Lys Arg Glu Thr Ser Cys Thr
               725                 730                 735
   aag gac gac cgt atc tac gct tgc atg gat gcc atc gtc cct ggc aac      2256
   Lys Asp Asp Arg Ile Tyr Ala Cys Met Asp Ala Ile Val Pro Gly Asn
           740                 745                 750
   gtc cct agc gcc tgc ttg cgc gag gcc ctc gac cag gcg cgc gtc aag      2304
   Val Pro Ser Ala Cys Leu Arg Glu Ala Leu Asp Gln Ala Arg Val Lys
       755                 760                 765
   ccg ggc gat atc gag atg ctc gag ctc agc gcc gac tcc gcc cgc cac      2352
   Pro Gly Asp Ile Glu Met Leu Glu Leu Ser Ala Asp Ser Ala Arg His
       770                 775                 780
   ctc aag gac ccg tcc gtc ctg ccc aag gag ctc act gcc gag gag gaa      2400
   Leu Lys Asp Pro Ser Val Leu Pro Lys Glu Leu Thr Ala Glu Glu Glu
   785                 790                 795                 800
   atc ggc ggc ctt cag acg atc ctt cgt gac gat gac aag ctc ccg cgc      2448
   Ile Gly Gly Leu Gln Thr Ile Leu Arg Asp Asp Asp Lys Leu Pro Arg
               805                 810                 815
   aac gtc gca acg ggc agt gtc aag gcc acc gtc ggt gac acc ggt tat      2496
   Asn Val Ala Thr Gly Ser Val Lys Ala Thr Val Gly Asp Thr Gly Tyr
           820                 825                 830
   gcc tct ggt gct gcc agc ctc atc aag gct gcg ctt tgc atc tac aac      2544
   Ala Ser Gly Ala Ala Ser Leu Ile Lys Ala Ala Leu Cys Ile Tyr Asn
       835                 840                 845
   cgc tac ctg ccc agc aac ggc gac gac tgg gat gaa ccc gcc cct gag      2592
   Arg Tyr Leu Pro Ser Asn Gly Asp Asp Trp Asp Glu Pro Ala Pro Glu
       850                 855                 860
   gcg ccc tgg gac agc acc ctc ttt gcg tgc cag acc tcg cgc gct tgg      2640
   Ala Pro Trp Asp Ser Thr Leu Phe Ala Cys Gln Thr Ser Arg Ala Trp
   865                 870                 875                 880
   ctc aag aac cct ggc gag cgt cgc tat gcg gcc gtc tcg ggc gtc tcc      2688
   Leu Lys Asn Pro Gly Glu Arg Arg Tyr Ala Ala Val Ser Gly Val Ser
               885                 890                 895
   gag acg cgc tcg tgc tat tcc gtg ctc ctc tcc gaa gcc gag ggc cac      2736
   Glu Thr Arg Ser Cys Tyr Ser Val Leu Leu Ser Glu Ala Glu Gly His
           900                 905                 910
   tac gag cgc gag aac cgc atc tcg ctc gac gag gag gcg ccc aag ctc     2784
   Tyr Glu Arg Glu Asn Arg Ile Ser Leu Asp Glu Glu Ala Pro Lys Leu
           915                 920                 925
   att gtg ctt cgc gcc gac tcc cac gag gag atc ctt ggt cgc ctc gac     2832
   Ile Val Leu Arg Ala Asp Ser His Glu Glu Ile Leu Gly Arg Leu Asp
       930                 935                 940
   aag atc cgc gag cgc ttc ttg cag ccc acg ggc gcc gcc ccg cgc gag     2880
   Lys Ile Arg Glu Arg Phe Leu Gln Pro Thr Gly Ala Ala Pro Arg Glu
   945                 950                 955                 960
   tcc gag ctc aag gcg cag gcc cgc cgc atc ttc ctc gag ctc ctc ggc     2928
   Ser Glu Leu Lys Ala Gln Ala Arg Arg Ile Phe Leu Glu Leu Leu Gly
                   965                 970                 975
   gag acc ctt gcc cag gat gcc gct tct tca ggc tcg caa aag ccc ctc     2976
   Glu Thr Leu Ala Gln Asp Ala Ala Ser Ser Gly Ser Gln Lys Pro Leu
               980                 985                 990
   gct ctc agc ctc gtc tcc acg ccc  tcc aag ctc cag cgc  gag gtc gag     3024
   Ala Leu Ser Leu Val Ser Thr Pro  Ser Lys Leu Gln Arg  Glu Val Glu
           995                 1000                1005
   ctc gcg  gcc aag ggt atc ccg  cgc tgc ctc aag atg  cgc cgc gat     3069
   Leu Ala  Ala Lys Gly Ile Pro  Arg Cys Leu Lys Met  Arg Arg Asp
       1010                 1015                 1020
   tgg agc  tcc cct gct ggc agc  cgc tac gcg cct gag  ccg ctc gcc     3114
   Trp Ser  Ser Pro Ala Gly Ser  Arg Tyr Ala Pro Glu  Pro Leu Ala
       1025                 1030                 1035
   agc gac  cgc gtc gcc ttc atg  tac ggc gaa ggt cgc  agc cct tac     3159
   Ser Asp  Arg Val Ala Phe Met  Tyr Gly Glu Gly Arg  Ser Pro Tyr
       1040                 1045                 1050
   tac ggc  atc acc caa gac att  cac cgc att tgg ccc  gaa ctc cac     3204
   Tyr Gly  Ile Thr Gln Asp Ile  His Arg Ile Trp Pro  Glu Leu His
       1055                 1060                 1065
   gag gtc  atc aac gaa aag acg  aac cgt ctc tgg gcc  gaa ggc gac     3249
   Glu Val  Ile Asn Glu Lys Thr  Asn Arg Leu Trp Ala  Glu Gly Asp
       1070                 1075                 1080
   cgc tgg  gtc atg ccg cgc gcc  agc ttc aag tcg gag  ctc gag agc     3294
   Arg Trp  Val Met Pro Arg Ala  Ser Phe Lys Ser Glu  Leu Glu Ser
       1085                 1090                 1095
   cag cag  caa gag ttt gat cgc  aac atg att gaa atg  ttc cgt ctt     3339
   Gln Gln  Gln Glu Phe Asp Arg  Asn Met Ile Glu Met  Phe Arg Leu
       1100                 1105                 1110
   gga atc  ctc acc tca att gcc  ttc acc aat ctg gcg  cgc gac gtt     3384
   Gly Ile  Leu Thr Ser Ile Ala  Phe Thr Asn Leu Ala  Arg Asp Val
       1115                 1120                 1125
   ctc aac  atc acg ccc aag gcc  gcc ttt ggc ctc agt  ctt ggc gag     3429
   Leu Asn  Ile Thr Pro Lys Ala  Ala Phe Gly Leu Ser  Leu Gly Glu
       1130                 1135                 1140
   att tcc  atg att ttt gcc ttt  tcc aag aag aac ggt  ctc atc tcc     3474
   Ile Ser  Met Ile Phe Ala Phe  Ser Lys Lys Asn Gly  Leu Ile Ser
       1145                 1150                 1155
   gac cag  ctc acc aag gat ctt  cgc gag tcc gac gtg  tgg aac aag     3519
   Asp Gln  Leu Thr Lys Asp Leu  Arg Glu Ser Asp Val  Trp Asn Lys
       1160                 1165                 1170
   gct ctg  gcc gtt gaa ttt aat  gcg ctg cgc gag gcc  tgg ggc att     3564
   Ala Leu  Ala Val Glu Phe Asn  Ala Leu Arg Glu Ala  Trp Gly Ile
       1175                 1180                 1185
   cca cag agt gtc ccc aag gac gag ttc tgg caa ggc tac att gtg      3609
   Pro Gln Ser Val Pro Lys Asp Glu Phe Trp Gln Gly Tyr Ile Val
       1190                1195                1200
   cgc ggc acc aag cag gat atc gag gcg gcc atc gcc ccg gac agc      3654
   Arg Gly Thr Lys Gln Asp Ile Glu Ala Ala Ile Ala Pro Asp Ser
       1205                1210                1215
   aag tac gtg cgc ctc acc atc atc aat gat gcc aac acc gcc ctc      3699
   Lys Tyr Val Arg Leu Thr Ile Ile Asn Asp Ala Asn Thr Ala Leu
       1220                1225                1230
   att agc ggc aag ccc gac gcc tgc aag gct gcg atc gcg cgt ctc      3744
   Ile Ser Gly Lys Pro Asp Ala Cys Lys Ala Ala Ile Ala Arg Leu
       1235                1240                1245
   ggt ggc aac att cct gcg ctt ccc gtg acc cag ggc atg tgc ggc      3789
   Gly Gly Asn Ile Pro Ala Leu Pro Val Thr Gln Gly Met Cys Gly
       1250                1255                1260
   cac tgc ccc gag gtg gga cct tat acc aag gat atc gcc aag atc      3834
   His Cys Pro Glu Val Gly Pro Tyr Thr Lys Asp Ile Ala Lys Ile
       1265                1270                1275
   cat gcc aac ctt gag ttc ccc gtt gtc gac ggc ctt gac ctc tgg      3879
   His Ala Asn Leu Glu Phe Pro Val Val Asp Gly Leu Asp Leu Trp
       1280                1285                1290
   acc aca atc aac cag aag cgc ctc gtg cca cgc gcc acg ggc gcc      3924
   Thr Thr Ile Asn Gln Lys Arg Leu Val Pro Arg Ala Thr Gly Ala
       1295                1300                1305
   aag gac gaa tgg gcc cct tct tcc ttt ggc gag tac gcc ggc cag      3969
   Lys Asp Glu Trp Ala Pro Ser Ser Phe Gly Glu Tyr Ala Gly Gln
       1310                1315                1320
   ctc tac gag aag cag gct aac ttc ccc caa atc gtc gag acc att      4014
   Leu Tyr Glu Lys Gln Ala Asn Phe Pro Gln Ile Val Glu Thr Ile
       1325                1330                1335
   tac aag caa aac tac gac gtc ttt gtc gag gtt ggg ccc aac aac      4059
   Tyr Lys Gln Asn Tyr Asp Val Phe Val Glu Val Gly Pro Asn Asn
       1340                1345                1350
   cac cgt agc acc gca gtg cgc acc acg ctt ggt ccc cag cgc aac      4104
   His Arg Ser Thr Ala Val Arg Thr Thr Leu Gly Pro Gln Arg Asn
       1355                1360                1365
   cac ctt gct ggc gcc atc gac aag cag aac gag gat gct tgg acg      4149
   His Leu Ala Gly Ala Ile Asp Lys Gln Asn Glu Asp Ala Trp Thr
       1370                1375                1380
   acc atc gtc aag ctt gtg gct tcg ctc aag gcc cac ctt gtt cct      4194
   Thr Ile Val Lys Leu Val Ala Ser Leu Lys Ala His Leu Val Pro
       1385                1390                1395
   ggc gtc acg atc tcg ccg ctg tac cac tcc aag ctt gtg gcg gag      4239
   Gly Val Thr Ile Ser Pro Leu Tyr His Ser Lys Leu Val Ala Glu
       1400                1405                1410
   gct cag gct tgc tac gct gcg ctc tgc aag ggt gaa aag ccc aag      4284
   Ala Gln Ala Cys Tyr Ala Ala Leu Cys Lys Gly Glu Lys Pro Lys
       1415                1420                1425
   aag aac aag ttt gtg cgc aag att cag ctc aac ggt cgc ttc aac      4329
   Lys Asn Lys Phe Val Arg Lys Ile Gln Leu Asn Gly Arg Phe Asn
       1430                1435                1440
   agc aag gcg gac ccc atc tcc tcg gcc gat ctt gcc agc ttt ccg      4374
   Ser Lys Ala Asp Pro Ile Ser Ser Ala Asp Leu Ala Ser Phe Pro
       1445                1450                1455
   cct gcg gac cct gcc att gaa gcc gcc atc tcg agc cgc atc atg      4419
   Pro Ala Asp Pro Ala Ile Glu Ala Ala Ile Ser Ser Arg Ile Met
       1460                1465                1470
   aag cct gtc gct ccc aag ttc tac gcg cgt ctc aac att gac gag      4464
   Lys Pro Val Ala Pro Lys Phe Tyr Ala Arg Leu Asn Ile Asp Glu
       1475                1480                1485
   cag gac gag acc cga gat ccg atc ctc aac aag gac aac gcg ccg      4509
   Gln Asp Glu Thr Arg Asp Pro Ile Leu Asn Lys Asp Asn Ala Pro
       1490                1495                1500
   tct tct tct tct tct tct tct tct tct tct tct tct tct tct tct      4554
   Ser Ser Ser Ser Ser Ser Ser Ser Ser Ser Ser Ser Ser Ser Ser
       1505                1510                1515
   ccg tcg cct gct cct tcg gcc ccc gtg caa aag aag gct gct ccc      4599
   Pro Ser Pro Ala Pro Ser Ala Pro Val Gln Lys Lys Ala Ala Pro
       1520                1525                1530
   gcc gcg gag acc aag gct gtt gct tcg gct gac gca ctt cgc agt      4644
   Ala Ala Glu Thr Lys Ala Val Ala Ser Ala Asp Ala Leu Arg Ser
       1535                1540                1545
   gcc ctg ctc gat ctc gac agt atg ctt gcg ctg agc tct gcc agt      4689
   Ala Leu Leu Asp Leu Asp Ser Met Leu Ala Leu Ser Ser Ala Ser
       1550                1555                1560
   gcc tcc ggc aac ctt gtt gag act gcg cct agc gac gcc tcg gtc      4734
   Ala Ser Gly Asn Leu Val Glu Thr Ala Pro Ser Asp Ala Ser Val
       1565                1570                1575
   att gtg ccg ccc tgc aac att gcg gat ctc ggc agc cgc gcc ttc      4779
   Ile Val Pro Pro Cys Asn Ile Ala Asp Leu Gly Ser Arg Ala Phe
       1580                1585                1590
   atg aaa acg tac ggt gtt tcg gcg cct ctg tac acg ggc gcc atg      4824
   Met Lys Thr Tyr Gly Val Ser Ala Pro Leu Tyr Thr Gly Ala Met
       1595                1600                1605
   gcc aag ggc att gcc tct gcg gac ctc gtc att gcc gcc ggc cgc      4869
   Ala Lys Gly Ile Ala Ser Ala Asp Leu Val Ile Ala Ala Gly Arg
       1610                1615                1620
   cag ggc atc ctt gcg tcc ttt ggc gcc ggc gga ctt ccc atg cag      4914
   Gln Gly Ile Leu Ala Ser Phe Gly Ala Gly Gly Leu Pro Met Gln
       1625                1630                1635
   gtt gtg cgt gag tcc atc gaa aag att cag gcc gcc ctg ccc aat      4959
   Val Val Arg Glu Ser Ile Glu Lys Ile Gln Ala Ala Leu Pro Asn
       1640                1645                1650
   ggc ccg tac gct gtc aac ctt atc cat tct ccc ttt gac agc aac      5004
   Gly Pro Tyr Ala Val Asn Leu Ile His Ser Pro Phe Asp Ser Asn
       1655                1660                1665
   ctc gaa aag ggc aat gtc gat ctc ttc ctc gag aag ggt gtc acc      5049
   Leu Glu Lys Gly Asn Val Asp Leu Phe Leu Glu Lys Gly Val Thr
       1670                1675                1680
   ttt gtc gag gcc tcg gcc ttt atg acg ctc acc ccg cag gtc gtg      5094
   Phe Val Glu Ala Ser Ala Phe Met Thr Leu Thr Pro Gln Val Val
       1685                1690                1695
   cgg tac cgc gcg gct ggc ctc acg cgc aac gcc gac ggc tcg gtc      5139
   Arg Tyr Arg Ala Ala Gly Leu Thr Arg Asn Ala Asp Gly Ser Val
       1700                1705                1710
   aac atc cgc aac cgt atc att ggc aag gtc tcg cgc acc gag ctc      5184
   Asn Ile Arg Asn Arg Ile Ile Gly Lys Val Ser Arg Thr Glu Leu
       1715                1720                1725
   gcc gag atg ttc atg cgt cct gcg ccc gag cac ctt ctt cag aag        5229
   Ala Glu Met Phe Met Arg Pro Ala Pro Glu His Leu Leu Gln Lys
       1730                1735                1740
   ctc att gct tcc ggc gag atc aac cag gag cag gcc gag ctc gcc        5274
   Leu Ile Ala Ser Gly Glu Ile Asn Gln Glu Gln Ala Glu Leu Ala
       1745                1750                1755
   cgc cgt gtt ccc gtc gct gac gac atc gcg gtc gaa gct gac tcg        5319
   Arg Arg Val Pro Val Ala Asp Asp Ile Ala Val Glu Ala Asp Ser
       1760                1765                1770
   ggt ggc cac acc gac aac cgc ccc atc cac gtc att ctg ccc ctc        5364
   Gly Gly His Thr Asp Asn Arg Pro Ile His Val Ile Leu Pro Leu
       1775                1780                1785
   atc atc aac ctt cgc gac cgc ctt cac cgc gag tgc ggc tac ccg        5409
   Ile Ile Asn Leu Arg Asp Arg Leu His Arg Glu Cys Gly Tyr Pro
       1790                1795                1800
   gcc aac ctt cgc gtc cgt gtg ggc gcc ggc ggt ggc att ggg tgc        5454
   Ala Asn Leu Arg Val Arg Val Gly Ala Gly Gly Gly Ile Gly Cys
       1805                1810                1815
   ccc cag gcg gcg ctg gcc acc ttc aac atg ggt gcc tcc ttt att        5499
   Pro Gln Ala Ala Leu Ala Thr Phe Asn Met Gly Ala Ser Phe Ile
       1820                1825                1830
   gtc acc ggc acc gtg aac cag gtc gcc aag cag tcg ggc acg tgc        5544
   Val Thr Gly Thr Val Asn Gln Val Ala Lys Gln Ser Gly Thr Cys
       1835                1840                1845
   gac aat gtg cgc aag cag ctc gcg aag gcc act tac tcg gac gta        5589
   Asp Asn Val Arg Lys Gln Leu Ala Lys Ala Thr Tyr Ser Asp Val
       1850                1855                1860
   tgc atg gcc ccg gct gcc gac atg ttc gag gaa ggc gtc aag ctt        5634
   Cys Met Ala Pro Ala Ala Asp Met Phe Glu Glu Gly Val Lys Leu
       1865                1870                1875
   cag gtc ctc aag aag gga acc atg ttt ccc tcg cgc gcc aac aag        5679
   Gln Val Leu Lys Lys Gly Thr Met Phe Pro Ser Arg Ala Asn Lys
       1880                1885                1890
   ctc tac gag ctc ttt tgc aag tac gac tcg ttc gag tcc atg ccc        5724
   Leu Tyr Glu Leu Phe Cys Lys Tyr Asp Ser Phe Glu Ser Met Pro
       1895                1900                1905
   ccc gca gag ctt gcg cgc gtc gag aag cgc atc ttc agc cgc gcg        5769
   Pro Ala Glu Leu Ala Arg Val Glu Lys Arg Ile Phe Ser Arg Ala
       1910                1915                1920
   ctc gaa gag gtc tgg gac gag acc aaa aac ttt tac att aac cgt        5814
   Leu Glu Glu Val Trp Asp Glu Thr Lys Asn Phe Tyr Ile Asn Arg
       1925                1930                1935
   ctt cac aac ccg gag aag atc cag cgc gcc gag cgc gac ccc aag        5859
   Leu His Asn Pro Glu Lys Ile Gln Arg Ala Glu Arg Asp Pro Lys
       1940                1945                1950
   ctc aag atg tcg ctg tgc ttt cgc tgg tac ctg agc ctg gcg agc        5904
   Leu Lys Met Ser Leu Cys Phe Arg Trp Tyr Leu Ser Leu Ala Ser
       1955                1960                1965
   cgc tgg gcc aac act gga gct tcc gat cgc gtc atg gac tac cag        5949
   Arg Trp Ala Asn Thr Gly Ala Ser Asp Arg Val Met Asp Tyr Gln
       1970                1975                1980
   gtc tgg tgc ggt cct gcc att ggt tcc ttc aac gat ttc atc aag        5994
   Val Trp Cys Gly Pro Ala Ile Gly Ser Phe Asn Asp Phe Ile Lys
       1985                1990                1995
   gga act tac ctt gat ccg gcc gtc gca aac gag tac ccg tgc gtc      6039
   Gly Thr Tyr Leu Asp Pro Ala Val Ala Asn Glu Tyr Pro Cys Val
       2000                2005                2010
   gtt cag att aac aag cag atc ctt cgt gga gcg tgc ttc ttg cgc      6084
   Val Gln Ile Asn Lys Gln Ile Leu Arg Gly Ala Cys Phe Leu Arg
       2015                2020                2025
   cgt ctc gaa att ctg cgc aac gca cgc ctt tcc gat ggc gct gcc      6129
   Arg Leu Glu Ile Leu Arg Asn Ala Arg Leu Ser Asp Gly Ala Ala
       2030                2035                2040
   gct ctt gtg gcc agc atc gat gac aca tac gtc ccg gcc gag aag      6174
   Ala Leu Val Ala Ser Ile Asp Asp Thr Tyr Val Pro Ala Glu Lys
       2045                2050                2055
   ctg                                                              6177
   Leu
<210> 4
   <211> 2059
   <212> PRT
   <213> Schizochytrium sp.
<220>
   <221> misc_feature
   <222> (370)..(370)
   <223> The 'Xaa' at location 370 stands for Leu.
<220>
   <221> misc_feature
   <222> (371)..(371)
   <223> The 'Xaa' at location 371 stands for Ala, or Val.
<400> 4
   Met Ala Ala Arg Asn Val Ser Ala Ala His Glu Met His Asp Glu Lys
   1               5                   10                  15
   Arg Ile Ala Val Val Gly Met Ala Val Gln Tyr Ala Gly Cys Lys Thr
               20                  25                  30
   Lys Asp Glu Phe Trp Glu Val Leu Met Asn Gly Lys Val Glu Ser Lys
           35                  40                  45
   Val Ile Ser Asp Lys Arg Leu Gly Ser Asn Tyr Arg Ala Glu His Tyr
       50                  55                  60
   Lys Ala Glu Arg Ser Lys Tyr Ala Asp Thr Phe Cys Asn Glu Thr Tyr
   65                  70                  75                  80
   Gly Thr Leu Asp Glu Asn Glu Ile Asp Asn Glu His Glu Leu Leu Leu
                   85                  90                  95
   Asn Leu Ala Lys Gln Ala Leu Ala Glu Thr Ser Val Lys Asp Ser Thr
               100                 105                 110
   Arg Cys Gly Ile Val Ser Gly Cys Leu Ser Phe Pro Met Asp Asn Leu
           115                 120                 125
   Gln Gly Glu Leu Leu Asn Val Tyr Gln Asn His Val Glu Lys Lys Leu
   130 135 140
   Gly Ala Arg Val Phe Lys Asp Ala Ser His Trp Ser Glu Arg Glu Gln
   145 150 155 160
   Ser Asn Lys Pro Glu Ala Gly Asp Arg Arg Ile Phe Met Asp Pro Ala
   165 170 175
   Ser Phe Val Ala Glu Glu Leu Asn Leu Gly Ala Leu His Tyr Ser Val
   180 185 190
   Asp Ala Ala Cys Ala Thr Ala Leu Tyr Val Leu Arg Leu Ala Gln Asp
   195 200 205
   His Leu Val Ser Gly Ala Ala Asp Val Met Leu Cys Gly Ala Thr Cys
   210 215 220
   Leu Pro Glu Pro Phe Phe Ile Leu Ser Gly Phe Ser Thr Phe Gln Ala
   225 230 235 240
   Met Pro Val Gly Thr Gly Gln Asn Val Ser Met Pro Leu His Lys Asp
   245 250 255
   Ser Gln Gly Leu Thr Pro Gly Glu Gly Gly Ser Ile Met Val Leu Lys
   260 265 270
   Arg Leu Asp Asp Ala Ile Arg Asp Gly Asp His Ile Tyr Gly Thr Leu
   275 280 285
   Leu Gly Ala Asn Val Ser Asn Ser Gly Thr Gly Leu Pro Leu Lys Pro
   290 295 300
   Leu Leu Pro Ser Glu Lys Lys Cys Leu Met Asp Thr Tyr Thr Arg Ile
   305 310 315 320
   Asn Val His Pro His Lys Ile Gln Tyr Val Glu Cys His Ala Thr Gly
   325 330 335
   Thr Pro Gln Gly Asp Arg Val Glu Ile Asp Ala Val Lys Ala Cys Phe
   340 345 350
   Glu Gly Lys Val Pro Arg Phe Gly Thr Thr Lys Gly Asn Phe Gly His
   355 360 365
   Thr Xaa Xaa Ala Ala Gly Phe Ala Gly Met Cys Lys Val Leu Leu Ser
   370 375 380
   Met Lys His Gly Ile Ile Pro Pro Thr Pro Gly Ile Asp Asp Glu Thr
   385 390 395 400
   Lys Met Asp Pro Leu Val Val Ser Gly Glu Ala Ile Pro Trp Pro Glu
   405 410 415
   Thr Asn Gly Glu Pro Lys Arg Ala Gly Leu Ser Ala Phe Gly Phe Gly
   420 425 430
   Gly Thr Asn Ala His Ala Val Phe Glu Glu His Asp Pro Ser Asn Ala
   435 440 445
   Ala Cys Thr Gly His Asp Ser Ile Ser Ala Leu Ser Ala Arg Cys Gly
   450 455 460
   Gly Glu Ser Asn Met Arg Ile Ala Ile Thr Gly Met Asp Ala Thr Phe
   465 470 475 480
   Gly Ala Leu Lys Gly Leu Asp Ala Phe Glu Arg Ala Ile Tyr Thr Gly
   485 490 495
   Ala His Gly Ala Ile Pro Leu Pro Glu Lys Arg Trp Arg Phe Leu Gly
   500 505 510
   Lys Asp Lys Asp Phe Leu Asp Leu Cys Gly Val Lys Ala Thr Pro His
   515 520 525
   Gly Cys Tyr Ile Glu Asp Val Glu Val Asp Phe Gln Arg Leu Arg Thr
   530 535 540
   Pro Met Thr Pro Glu Asp Met Leu Leu Pro Gln Gln Leu Leu Ala Val
   545 550 555 560
   Thr Thr Ile Asp Arg Ala Ile Leu Asp Ser Gly Met Lys Lys Gly Gly
   565 570 575
   Asn Val Ala Val Phe Val Gly Leu Gly Thr Asp Leu Glu Leu Tyr Arg
   580 585 590
   His Arg Ala Arg Val Ala Leu Lys Glu Arg Val Arg Pro Glu Ala Ser
   595 600 605
   Lys Lys Leu Asn Asp Met Met Gln Tyr Ile Asn Asp Cys Gly Thr Ser
   610 615 620
   Thr Ser Tyr Thr Ser Tyr Ile Gly Asn Leu Val Ala Thr Arg Val Ser
   625 630 635 640
   Ser Gln Trp Gly Phe Thr Gly Pro Ser Phe Thr Ile Thr Glu Gly Asn
   645 650 655
   Asn Ser Val Tyr Arg Cys Ala Glu Leu Gly Lys Tyr Leu Leu Glu Thr
   660 665 670
   Gly Glu Val Asp Gly Val Val Val Ala Gly Val Asp Leu Cys Gly Ser
   675 680 685
   Ala Glu Asn Leu Tyr Val Lys Ser Arg Arg Phe Lys Val Ser Thr Ser
   690 695 700
   Asp Thr Pro Arg Ala Ser Phe Asp Ala Ala Ala Asp Gly Tyr Phe Val
   705 710 715 720
   Gly Glu Gly Cys Gly Ala Phe Val Leu Lys Arg Glu Thr Ser Cys Thr
   725 730 735
   Lys Asp Asp Arg Ile Tyr Ala Cys Met Asp Ala Ile Val Pro Gly Asn
   740 745 750
   Val Pro Ser Ala Cys Leu Arg Glu Ala Leu Asp Gln Ala Arg Val Lys
   755 760 765
   Pro Gly Asp Ile Glu Met Leu Glu Leu Ser Ala Asp Ser Ala Arg His
   770 775 780
   Leu Lys Asp Pro Ser Val Leu Pro Lys Glu Leu Thr Ala Glu Glu Glu
   785 790 795 800
   Ile Gly Gly Leu Gln Thr Ile Leu Arg Asp Asp Asp Lys Leu Pro Arg
   805 810 815
   Asn Val Ala Thr Gly Ser Val Lys Ala Thr Val Gly Asp Thr Gly Tyr
   820 825 830
   Ala Ser Gly Ala Ala Ser Leu Ile Lys Ala Ala Leu Cys Ile Tyr Asn
   835 840 845
   Arg Tyr Leu Pro Ser Asn Gly Asp Asp Trp Asp Glu Pro Ala Pro Glu
   850 855 860
   Ala Pro Trp Asp Ser Thr Leu Phe Ala Cys Gln Thr Ser Arg Ala Trp
   865 870 875 880
   Leu Lys Asn Pro Gly Glu Arg Arg Tyr Ala Ala Val Ser Gly Val Ser
   885 890 895
   Glu Thr Arg Ser Cys Tyr Ser Val Leu Leu Ser Glu Ala Glu Gly His
   900 905 910
   Tyr Glu Arg Glu Asn Arg Ile Ser Leu Asp Glu Glu Ala Pro Lys Leu
   915 920 925
   Ile Val Leu Arg Ala Asp Ser His Glu Glu Ile Leu Gly Arg Leu Asp
   930 935 940
   Lys Ile Arg Glu Arg Phe Leu Gln Pro Thr Gly Ala Ala Pro Arg Glu
   945 950 955 960
   Ser Glu Leu Lys Ala Gln Ala Arg Arg Ile Phe Leu Glu Leu Leu Gly
   965 970 975
   Glu Thr Leu Ala Gln Asp Ala Ala Ser Ser Gly Ser Gln Lys Pro Leu
   980 985 990
   Ala Leu Ser Leu Val Ser Thr Pro Ser Lys Leu Gln Arg Glu Val Glu
   995 1000 1005
   Leu Ala Ala Lys Gly Ile Pro Arg Cys Leu Lys Met Arg Arg Asp
   1010 1015 1020
   Trp Ser Ser Pro Ala Gly Ser Arg Tyr Ala Pro Glu Pro Leu Ala
   1025 1030 1035
   Ser Asp Arg Val Ala Phe Met Tyr Gly Glu Gly Arg Ser Pro Tyr
   1040 1045 1050
   Tyr Gly Ile Thr Gln Asp Ile His Arg Ile Trp Pro Glu Leu His
   1055 1060 1065
   Glu Val Ile Asn Glu Lys Thr Asn Arg Leu Trp Ala Glu Gly Asp
   1070 1075 1080
   Arg Trp Val Met Pro Arg Ala Ser Phe Lys Ser Glu Leu Glu Ser
   1085 1090 1095
   Gln Gln Gln Glu Phe Asp Arg Asn Met Ile Glu Met Phe Arg Leu
   1100 1105 1110
   Gly Ile Leu Thr Ser Ile Ala Phe Thr Asn Leu Ala Arg Asp Val
   1115 1120 1125
   Leu Asn Ile Thr Pro Lys Ala Ala Phe Gly Leu Ser Leu Gly Glu
   1130 1135 1140
   Ile Ser Met Ile Phe Ala Phe Ser Lys Lys Asn Gly Leu Ile Ser
   1145 1150 1155
   Asp Gln Leu Thr Lys Asp Leu Arg Glu Ser Asp Val Trp Asn Lys
   1160 1165 1170
   Ala Leu Ala Val Glu Phe Asn Ala Leu Arg Glu Ala Trp Gly Ile
   1175 1180 1185
   Pro Gln Ser Val Pro Lys Asp Glu Phe Trp Gln Gly Tyr Ile Val
   1190 1195 1200
   Arg Gly Thr Lys Gln Asp Ile Glu Ala Ala Ile Ala Pro Asp Ser
   1205 1210 1215
   Lys Tyr Val Arg Leu Thr Ile Ile Asn Asp Ala Asn Thr Ala Leu
   1220 1225 1230
   Ile Ser Gly Lys Pro Asp Ala Cys Lys Ala Ala Ile Ala Arg Leu
   1235 1240 1245
   Gly Gly Asn Ile Pro Ala Leu Pro Val Thr Gln Gly Met Cys Gly
   1250 1255 1260
   His Cys Pro Glu Val Gly Pro Tyr Thr Lys Asp Ile Ala Lys Ile
   1265 1270 1275
   His Ala Asn Leu Glu Phe Pro Val Val Asp Gly Leu Asp Leu Trp
   1280 1285 1290
   Thr Thr Ile Asn Gln Lys Arg Leu Val Pro Arg Ala Thr Gly Ala
   1295 1300 1305
   Lys Asp Glu Trp Ala Pro Ser Ser Phe Gly Glu Tyr Ala Gly Gln
   1310 1315 1320
   Leu Tyr Glu Lys Gln Ala Asn Phe Pro Gln Ile Val Glu Thr Ile
   1325 1330 1335
   Tyr Lys Gln Asn Tyr Asp Val Phe Val Glu Val Gly Pro Asn Asn
   1340 1345 1350
   His Arg Ser Thr Ala Val Arg Thr Thr Leu Gly Pro Gln Arg Asn
   1355 1360 1365
   His Leu Ala Gly Ala Ile Asp Lys Gln Asn Glu Asp Ala Trp Thr
   1370 1375 1380
   Thr Ile Val Lys Leu Val Ala Ser Leu Lys Ala His Leu Val Pro
   1385 1390 1395
   Gly Val Thr Ile Ser Pro Leu Tyr His Ser Lys Leu Val Ala Glu
   1400 1405 1410
   Ala Gln Ala Cys Tyr Ala Ala Leu Cys Lys Gly Glu Lys Pro Lys
   1415 1420 1425
   Lys Asn Lys Phe Val Arg Lys Ile Gln Leu Asn Gly Arg Phe Asn
   1430 1435 1440
   Ser Lys Ala Asp Pro Ile Ser Ser Ala Asp Leu Ala Ser Phe Pro
   1445 1450 1455
   Pro Ala Asp Pro Ala Ile Glu Ala Ala Ile Ser Ser Arg Ile Met
   1460 1465 1470
   Lys Pro Val Ala Pro Lys Phe Tyr Ala Arg Leu Asn Ile Asp Glu
   1475 1480 1485
   Gln Asp Glu Thr Arg Asp Pro Ile Leu Asn Lys Asp Asn Ala Pro
   1490 1495 1500
   Ser Ser Ser Ser Ser Ser Ser Ser Ser Ser Ser Ser Ser Ser Ser
   1505 1510 1515
   Pro Ser Pro Ala Pro Ser Ala Pro Val Gln Lys Lys Ala Ala Pro
   1520 1525 1530
   Ala Ala Glu Thr Lys Ala Val Ala Ser Ala Asp Ala Leu Arg Ser
   1535 1540 1545
   Ala Leu Leu Asp Leu Asp Ser Met Leu Ala Leu Ser Ser Ala Ser
   1550 1555 1560
   Ala Ser Gly Asn Leu Val Glu Thr Ala Pro Ser Asp Ala Ser Val
   1565 1570 1575
   Ile Val Pro Pro Cys Asn Ile Ala Asp Leu Gly Ser Arg Ala Phe
   1580 1585 1590
   Met Lys Thr Tyr Gly Val Ser Ala Pro Leu Tyr Thr Gly Ala Met
   1595 1600 1605
   Ala Lys Gly Ile Ala Ser Ala Asp Leu Val Ile Ala Ala Gly Arg
   1610 1615 1620
   Gln Gly Ile Leu Ala Ser Phe Gly Ala Gly Gly Leu Pro Met Gln
   1625 1630 1635
   Val Val Arg Glu Ser Ile Glu Lys Ile Gln Ala Ala Leu Pro Asn
   1640 1645 1650
   Gly Pro Tyr Ala Val Asn Leu Ile His Ser Pro Phe Asp Ser Asn
   1655 1660 1665
   Leu Glu Lys Gly Asn Val Asp Leu Phe Leu Glu Lys Gly Val Thr
   1670 1675 1680
   Phe Val Glu Ala Ser Ala Phe Met Thr Leu Thr Pro Gln Val Val
   1685 1690 1695
   Arg Tyr Arg Ala Ala Gly Leu Thr Arg Asn Ala Asp Gly Ser Val
   1700 1705 1710
   Asn Ile Arg Asn Arg Ile Ile Gly Lys Val Ser Arg Thr Glu Leu
   1715 1720 1725
   Ala Glu Met Phe Met Arg Pro Ala Pro Glu His Leu Leu Gln Lys
   1730 1735 1740
   Leu Ile Ala Ser Gly Glu Ile Asn Gln Glu Gln Ala Glu Leu Ala
   1745 1750 1755
   Arg Arg Val Pro Val Ala Asp Asp Ile Ala Val Glu Ala Asp Ser
   1760 1765 1770
   Gly Gly His Thr Asp Asn Arg Pro Ile His Val Ile Leu Pro Leu
   1775 1780 1785
   Ile Ile Asn Leu Arg Asp Arg Leu His Arg Glu Cys Gly Tyr Pro
   1790 1795 1800
   Ala Asn Leu Arg Val Arg Val Gly Ala Gly Gly Gly Ile Gly Cys
   1805 1810 1815
   Pro Gln Ala Ala Leu Ala Thr Phe Asn Met Gly Ala Ser Phe Ile
   1820 1825 1830
   Val Thr Gly Thr Val Asn Gln Val Ala Lys Gln Ser Gly Thr Cys
   1835 1840 1845
   Asp Asn Val Arg Lys Gln Leu Ala Lys Ala Thr Tyr Ser Asp Val
   1850 1855 1860
   Cys Met Ala Pro Ala Ala Asp Met Phe Glu Glu Gly Val Lys Leu
   1865 1870 1875
   Gln Val Leu Lys Lys Gly Thr Met Phe Pro Ser Arg Ala Asn Lys
   1880 1885 1890
   Leu Tyr Glu Leu Phe Cys Lys Tyr Asp Ser Phe Glu Ser Met Pro
   1895 1900 1905
   Pro Ala Glu Leu Ala Arg Val Glu Lys Arg Ile Phe Ser Arg Ala
   1910 1915 1920
   Leu Glu Glu Val Trp Asp Glu Thr Lys Asn Phe Tyr Ile Asn Arg
   1925 1930 1935
   Leu His Asn Pro Glu Lys Ile Gln Arg Ala Glu Arg Asp Pro Lys
   1940 1945 1950
   Leu Lys Met Ser Leu Cys Phe Arg Trp Tyr Leu Ser Leu Ala Ser
   1955 1960 1965
   Arg Trp Ala Asn Thr Gly Ala Ser Asp Arg Val Met Asp Tyr Gln
   1970 1975 1980
   Val Trp Cys Gly Pro Ala Ile Gly Ser Phe Asn Asp Phe Ile Lys
   1985 1990 1995
   Gly Thr Tyr Leu Asp Pro Ala Val Ala Asn Glu Tyr Pro Cys Val
   2000 2005 2010
   Val Gln Ile Asn Lys Gln Ile Leu Arg Gly Ala Cys Phe Leu Arg
   2015 2020 2025
   Arg Leu Glu Ile Leu Arg Asn Ala Arg Leu Ser Asp Gly Ala Ala
   2030 2035 2040
   Ala Leu Val Ala Ser Ile Asp Asp Thr Tyr Val Pro Ala Glu Lys
   2045 2050 2055
   Leu
<210> 5
   <211> 4509
   <212> DNA
   <213> Schizochytrium sp.
<220>
   <221> CDS
   <222> (1)..(4509)
<400> 5
   atg gcg ctc cgt gtc aag acg aac aag aag cca tgc tgg gag atg acc      48
   Met Ala Leu Arg Val Lys Thr Asn Lys Lys Pro Cys Trp Glu Met Thr
   1               5                   10                  15
   aag gag gag ctg acc agc ggc aag acc gag gtg ttc aac tat gag gaa      96
   Lys Glu Glu Leu Thr Ser Gly Lys Thr Glu Val Phe Asn Tyr Glu Glu
               20                  25                  30
   ctc ctc gag ttc gca gag ggc gac atc gcc aag gtc ttc gga ccc gag     144
   Leu Leu Glu Phe Ala Glu Gly Asp Ile Ala Lys Val Phe Gly Pro Glu
           35                  40                  45
   ttc gcc gtc atc gac aag tac ccg cgc cgc gtg cgc ctg ccc gcc cgc     192
   Phe Ala Val Ile Asp Lys Tyr Pro Arg Arg Val Arg Leu Pro Ala Arg
       50                  55                  60
   gag tac ctg ctc gtg acc cgc gtc acc ctc atg gac gcc gag gtc aac     240
   Glu Tyr Leu Leu Val Thr Arg Val Thr Leu Met Asp Ala Glu Val Asn
   65                  70                  75                  80
   aac tac cgc gtc ggc gcc cgc atg gtc acc gag tac gat ctc ccc gtc     288
   Asn Tyr Arg Val Gly Ala Arg Met Val Thr Glu Tyr Asp Leu Pro Val
                   85                  90                  95
   aac gga gag ctc tcc gag ggc gga gac tgc ccc tgg gcc gtc ctg gtc     336
   Asn Gly Glu Leu Ser Glu Gly Gly Asp Cys Pro Trp Ala Val Leu Val
               100                 105                 110
   gag agt ggc cag tgc gat ctc atg ctc atc tcc tac atg ggc att gac     384
   Glu Ser Gly Gln Cys Asp Leu Met Leu Ile Ser Tyr Met Gly Ile Asp
           115                 120                 125
   ttc cag aac cag ggc gac cgc gtc tac cgc ctg ctc aac acc acg ctc     432
   Phe Gln Asn Gln Gly Asp Arg Val Tyr Arg Leu Leu Asn Thr Thr Leu
       130                 135                 140
   acc ttt tac ggc gtg gcc cac gag ggc gag acc ctc gag tac gac att     480
   Thr Phe Tyr Gly Val Ala His Glu Gly Glu Thr Leu Glu Tyr Asp Ile
   145                 150                 155                 160
   cgc gtc acc ggc ttc gcc aag cgt ctc gac ggc ggc atc tcc atg ttc     528
   Arg Val Thr Gly Phe Ala Lys Arg Leu Asp Gly Gly Ile Ser Met Phe
                   165                 170                 175
   ttc ttc gag tac gac tgc tac gtc aac ggc cgc ctc ctc atc gag atg     576
   Phe Phe Glu Tyr Asp Cys Tyr Val Asn Gly Arg Leu Leu Ile Glu Met
               180                 185                 190
   cgc gat ggc tgc gcc ggc ttc ttc acc aac gag gag ctc gac gcc ggc     624
   Arg Asp Gly Cys Ala Gly Phe Phe Thr Asn Glu Glu Leu Asp Ala Gly
           195                 200                 205
   aag ggc gtc gtc ttc acc cgc ggc gac ctc gcc gcc cgc gcc aag atc     672
   Lys Gly Val Val Phe Thr Arg Gly Asp Leu Ala Ala Arg Ala Lys Ile
       210                 215                 220
   cca aag cag gac gtc tcc ccc tac gcc gtc gcc ccc tgc ctc cac aag     720
   Pro Lys Gln Asp Val Ser Pro Tyr Ala Val Ala Pro Cys Leu His Lys
   225                 230                 235                 240
   acc aag ctc aac gaa aag gag atg cag acc ctc gtc gac aag gac tgg      768
   Thr Lys Leu Asn Glu Lys Glu Met Gln Thr Leu Val Asp Lys Asp Trp
                   245                 250                 255
   gca tcc gtc ttt ggc tcc aag aac ggc atg ccg gaa atc aac tac aaa      816
   Ala Ser Val Phe Gly Ser Lys Asn Gly Met Pro Glu Ile Asn Tyr Lys
               260                 265                 270
   ctc tgc gcg cgt aag atg ctc atg att gac cgc gtc acc agc att gac      864
   Leu Cys Ala Arg Lys Met Leu Met Ile Asp Arg Val Thr Ser Ile Asp
           275                 280                 285
   cac aag ggc ggt gtc tac ggc ctc ggt cag ctc gtc ggt gaa aag atc      912
   His Lys Gly Gly Val Tyr Gly Leu Gly Gln Leu Val Gly Glu Lys Ile
       290                 295                 300
   ctc gag cgc gac cac tgg tac ttt ccc tgc cac ttt gtc aag gat cag      960
   Leu Glu Arg Asp His Trp Tyr Phe Pro Cys His Phe Val Lys Asp Gln
   305                 310                 315                 320
   gtc atg gcc gga tcc ctc gtc tcc gac ggc tgc agc cag atg ctc aag     1008
   Val Met Ala Gly Ser Leu Val Ser Asp Gly Cys Ser Gln Met Leu Lys
                   325                 330                 335
   atg tac atg atc tgg ctc ggc ctc cac ctc acc acc gga ccc ttt gac     1056
   Met Tyr Met Ile Trp Leu Gly Leu His Leu Thr Thr Gly Pro Phe Asp
               340                 345                 350
   ttc cgc ccg gtc aac ggc cac ccc aac aag gtc cgc tgc cgc ggc caa     1104
   Phe Arg Pro Val Asn Gly His Pro Asn Lys Val Arg Cys Arg Gly Gln
           355                 360                 365
   atc tcc ccg cac aag ggc aag ctc gtc tac gtc atg gag atc aag gag     1152
   Ile Ser Pro His Lys Gly Lys Leu Val Tyr Val Met Glu Ile Lys Glu
       370                 375                 380
   atg ggc ttc gac gag gac aac gac ccg tac gcc att gcc gac gtc aac     1200
   Met Gly Phe Asp Glu Asp Asn Asp Pro Tyr Ala Ile Ala Asp Val Asn
   385                 390                 395                 400
   atc att gat gtc gac ttc gaa aag ggc cag gac ttt agc ctc gac cgc     1248
   Ile Ile Asp Val Asp Phe Glu Lys Gly Gln Asp Phe Ser Leu Asp Arg
                   405                 410                 415
   atc agc gac tac ggc aag ggc gac ctc aac aag aag atc gtc gtc gac     1296
   Ile Ser Asp Tyr Gly Lys Gly Asp Leu Asn Lys Lys Ile Val Val Asp
               420                 425                 430
   ttt aag ggc atc gct ctc aag atg cag aag cgc tcc acc aac aag aac     1344
   Phe Lys Gly Ile Ala Leu Lys Met Gln Lys Arg Ser Thr Asn Lys Asn
           435                 440                 445
   ccc tcc aag gtt cag ccc gtc ttt gcc aac ggc gcc gcc act gtc ggc     1392
   Pro Ser Lys Val Gln Pro Val Phe Ala Asn Gly Ala Ala Thr Val Gly
       450                 455                 460
   ccc gag gcc tcc aag gct tcc tcc ggc gcc agc gcc agc gcc agc gcc     1440
   Pro Glu Ala Ser Lys Ala Ser Ser Gly Ala Ser Ala Ser Ala Ser Ala
   465                 470                 475                 480
   gcc ccg gcc aag cct gcc ttc agc gcc gat gtt ctt gcg ccc aag ccc     1488
   Ala Pro Ala Lys Pro Ala Phe Ser Ala Asp Val Leu Ala Pro Lys Pro
                   485                 490                 495
   gtt gcc ctt ccc gag cac atc ctc aag ggc gac gcc ctc gcc ccc aag     1536
   Val Ala Leu Pro Glu His Ile Leu Lys Gly Asp Ala Leu Ala Pro Lys
               500                 505                 510
   gag atg tcc tgg cac ccc atg gcc cgc atc ccg ggc aac ccg acg ccc     1584
   Glu Met Ser Trp His Pro Met Ala Arg Ile Pro Gly Asn Pro Thr Pro
           515                 520                 525
   tct ttt gcg ccc tcg gcc tac aag ccg cgc aac atc gcc ttt acg ccc      1632
   Ser Phe Ala Pro Ser Ala Tyr Lys Pro Arg Asn Ile Ala Phe Thr Pro
       530                 535                 540
   ttc ccc ggc aac ccc aac gat aac gac cac acc ccg ggc aag atg ccg      1680
   Phe Pro Gly Asn Pro Asn Asp Asn Asp His Thr Pro Gly Lys Met Pro
   545                 550                 555                 560
   ctc acc tgg ttc aac atg gcc gag ttc atg gcc ggc aag gtc agc atg      1728
   Leu Thr Trp Phe Asn Met Ala Glu Phe Met Ala Gly Lys Val Ser Met
                   565                 570                 575
   tgc ctc ggc ccc gag ttc gcc aag ttc gac gac tcg aac acc agc cgc      1776
   Cys Leu Gly Pro Glu Phe Ala Lys Phe Asp Asp Ser Asn Thr Ser Arg
               580                 585                 590
   agc ccc gct tgg gac ctc gct ctc gtc acc cgc gcc gtg tct gtg tct      1824
   Ser Pro Ala Trp Asp Leu Ala Leu Val Thr Arg Ala Val Ser Val Ser
           595                 600                 605
   gac ctc aag cac gtc aac tac cgc aac atc gac ctc gac ccc tcc aag      1872
   Asp Leu Lys His Val Asn Tyr Arg Asn Ile Asp Leu Asp Pro Ser Lys
       610                 615                 620
   ggt acc atg gtc ggc gag ttc gac tgc ccc gcg gac gcc tgg ttc tac      1920
   Gly Thr Met Val Gly Glu Phe Asp Cys Pro Ala Asp Ala Trp Phe Tyr
   625                 630                 635                 640
   aag ggc gcc tgc aac gat gcc cac atg ccg tac tcg atc ctc atg gag      1968
   Lys Gly Ala Cys Asn Asp Ala His Met Pro Tyr Ser Ile Leu Met Glu
                   645                 650                 655
   atc gcc ctc cag acc tcg ggt gtg ctc acc tcg gtg ctc aag gcg ccc      2016
   Ile Ala Leu Gln Thr Ser Gly Val Leu Thr Ser Val Leu Lys Ala Pro
               660                 665                 670
   ctg acc atg gag aag gac gac atc ctc ttc cgc aac ctc gac gcc aac      2064
   Leu Thr Met Glu Lys Asp Asp Ile Leu Phe Arg Asn Leu Asp Ala Asn
           675                 680                 685
   gcc gag ttc gtg cgc gcc gac ctc gac tac cgc ggc aag act atc cgc      2112
   Ala Glu Phe Val Arg Ala Asp Leu Asp Tyr Arg Gly Lys Thr Ile Arg
       690                 695                 700
   aac gtc acc aag tgc act ggc tac agc atg ctc ggc gag atg ggc gtc      2160
   Asn Val Thr Lys Cys Thr Gly Tyr Ser Met Leu Gly Glu Met Gly Val
   705                 710                 715                 720
   cac cgc ttc acc ttt gag ctc tac gtc gat gat gtg ctc ttt tac aag      2208
   His Arg Phe Thr Phe Glu Leu Tyr Val Asp Asp Val Leu Phe Tyr Lys
                   725                 730                 735
   ggc tcg acc tcg ttc ggc tgg ttc gtg ccc gag gtc ttt gcc gcc cag      2256
   Gly Ser Thr Ser Phe Gly Trp Phe Val Pro Glu Val Phe Ala Ala Gln
               740                 745                 750
   gcc ggc ctc gac aac ggc cgc aag tcg gag ccc tgg ttc att gag aac      2304
   Ala Gly Leu Asp Asn Gly Arg Lys Ser Glu Pro Trp Phe Ile Glu Asn
           755                 760                 765
   aag gtt ccg gcc tcg cag gtc tcc tcc ttt gac gtg cgc ccc aac ggc      2352
   Lys Val Pro Ala Ser Gln Val Ser Ser Phe Asp Val Arg Pro Asn Gly
       770                 775                 780
   agc ggc cgc acc gcc atc ttc gcc aac gcc ccc agc ggc gcc cag ctc      2400
   Ser Gly Arg Thr Ala Ile Phe Ala Asn Ala Pro Ser Gly Ala Gln Leu
   785                 790                 795                 800
   aac cgc cgc acg gac cag ggc cag tac ctc gac gcc gtc gac att gtc      2448
   Asn Arg Arg Thr Asp Gln Gly Gln Tyr Leu Asp Ala Val Asp Ile Val
                   805                 810                 815
   tcc ggc agc ggc aag aag agc ctc ggc tac gcc cac ggt tcc aag acg      2496
   Ser Gly Ser Gly Lys Lys Ser Leu Gly Tyr Ala His Gly Ser Lys Thr
               820                 825                 830
   gtc aac ccg aac gac tgg ttc ttc tcg tgc cac ttt tgg ttt gac tcg      2544
   Val Asn Pro Asn Asp Trp Phe Phe Ser Cys His Phe Trp Phe Asp Ser
           835                 840                 845
   gtc atg ccc gga agt ctc ggt gtc gag tcc atg ttc cag ctc gtc gag      2592
   Val Met Pro Gly Ser Leu Gly Val Glu Ser Met Phe Gln Leu Val Glu
       850                 855                 860
   gcc atc gcc gcc cac gag gat ctc gct ggc aaa gca cgg cat tgc caa      2640
   Ala Ile Ala Ala His Glu Asp Leu Ala Gly Lys Ala Arg His Cys Gln
   865                 870                 875                 880
   ccc cac ctt tgt gca cgc ccc cgg gca aga tca agc tgg aag tac cgc      2688
   Pro His Leu Cys Ala Arg Pro Arg Ala Arg Ser Ser Trp Lys Tyr Arg
                   885                 890                 895
   ggc cag ctc acg ccc aag agc aag aag atg gac tcg gag gtc cac atc      2736
   Gly Gln Leu Thr Pro Lys Ser Lys Lys Met Asp Ser Glu Val His Ile
               900                 905                 910
   gtg tcc gtg gac gcc cac gac ggc gtt gtc gac ctc gtc gcc gac ggc      2784
   Val Ser Val Asp Ala His Asp Gly Val Val Asp Leu Val Ala Asp Gly
           915                 920                 925
   ttc ctc tgg gcc gac agc ctc cgc gtc tac tcg gtg agc aac att cgc      2832
   Phe Leu Trp Ala Asp Ser Leu Arg Val Tyr Ser Val Ser Asn Ile Arg
       930                 935                 940
   gtg cgc atc gcc tcc ggt gag gcc cct gcc gcc gcc tcc tcc gcc gcc      2880
   Val Arg Ile Ala Ser Gly Glu Ala Pro Ala Ala Ala Ser Ser Ala Ala
   945                 950                 955                 960
   tct gtg ggc tcc tcg gct tcg tcc gtc gag cgc acg cgc tcg agc ccc      2928
   Ser Val Gly Ser Ser Ala Ser Ser Val Glu Arg Thr Arg Ser Ser Pro
                   965                 970                 975
   gct gtc gcc tcc ggc ccg gcc cag acc atc gac ctc aag cag ctc aag      2976
   Ala Val Ala Ser Gly Pro Ala Gln Thr Ile Asp Leu Lys Gln Leu Lys
               980                 985                 990
   acc gag ctc ctc gag ctc gat gcc  ccg ctc tac ctc tcg  cag gac ccg    3024
   Thr Glu Leu Leu Glu Leu Asp Ala  Pro Leu Tyr Leu Ser  Gln Asp Pro
           995                 1000                 1005
   acc agc  ggc cag ctc aag aag  cac acc gac gtg gcc  tcc ggc cag      3069
   Thr Ser  Gly Gln Leu Lys Lys  His Thr Asp Val Ala  Ser Gly Gln
       1010                 1015                 1020
   gcc acc  atc gtg cag ccc tgc  acg ctc ggc gac ctc  ggt gac cgc      3114
   Ala Thr  Ile Val Gln Pro Cys  Thr Leu Gly Asp Leu  Gly Asp Arg
       1025                 1030                 1035
   tcc ttc  atg gag acc tac ggc  gtc gtc gcc ccg ctg  tac acg ggc      3159
   Ser Phe  Met Glu Thr Tyr Gly  Val Val Ala Pro Leu  Tyr Thr Gly
       1040                 1045                 1050
   gcc atg  gcc aag ggc att gcc  tcg gcg gac ctc gtc  atc gcc gcc      3204
   Ala Met  Ala Lys Gly Ile Ala  Ser Ala Asp Leu Val  Ile Ala Ala
       1055                 1060                 1065
   ggc aag  cgc aag atc ctc ggc  tcc ttt ggc gcc ggc  ggc ctc ccc      3249
   Gly Lys  Arg Lys Ile Leu Gly  Ser Phe Gly Ala Gly  Gly Leu Pro
       1070                 1075                 1080
   atg cac  cac gtg cgc gcc gcc  ctc gag aag atc cag  gcc gcc ctg      3294
   Met His  His Val Arg Ala Ala  Leu Glu Lys Ile Gln  Ala Ala Leu
       1085                 1090                 1095
   cct cag ggc ccc tac gcc gtc aac ctc atc cac tcg cct ttt gac      3339
   Pro Gln Gly Pro Tyr Ala Val Asn Leu Ile His Ser Pro Phe Asp
       1100                1105                1110
   agc aac ctc gag aag ggc aac gtc gat ctc ttc ctc gag aag ggc      3384
   Ser Asn Leu Glu Lys Gly Asn Val Asp Leu Phe Leu Glu Lys Gly
       1115                1120                1125
   gtc act gtg gtg gag gcc tcg gca ttc atg acc ctc acc ccg cag      3429
   Val Thr Val Val Glu Ala Ser Ala Phe Met Thr Leu Thr Pro Gln
       1130                1135                1140
   gtc gtg cgc tac cgc gcc gcc ggc ctc tcg cgc aac gcc gac ggt      3474
   Val Val Arg Tyr Arg Ala Ala Gly Leu Ser Arg Asn Ala Asp Gly
       1145                1150                1155
   tcg gtc aac atc cgc aac cgc atc atc ggc aag gtc tcg cgc acc      3519
   Ser Val Asn Ile Arg Asn Arg Ile Ile Gly Lys Val Ser Arg Thr
       1160                1165                1170
   gag ctc gcc gag atg ttc atc cgc ccg gcc ccg gag cac ctc ctc      3564
   Glu Leu Ala Glu Met Phe Ile Arg Pro Ala Pro Glu His Leu Leu
       1175                1180                1185
   gag aag ctc atc gcc tcg ggc gag atc acc cag gag cag gcc gag      3609
   Glu Lys Leu Ile Ala Ser Gly Glu Ile Thr Gln Glu Gln Ala Glu
       1190                1195                1200
   ctc gcg cgc cgc gtt ccc gtc gcc gac gat atc gct gtc gag gct      3654
   Leu Ala Arg Arg Val Pro Val Ala Asp Asp Ile Ala Val Glu Ala
       1205                1210                1215
   gac tcg ggc ggc cac acc gac aac cgc ccc atc cac gtc atc ctc      3699
   Asp Ser Gly Gly His Thr Asp Asn Arg Pro Ile His Val Ile Leu
       1220                1225                1230
   ccg ctc atc atc aac ctc cgc aac cgc ctg cac cgc gag tgc ggc      3744
   Pro Leu Ile Ile Asn Leu Arg Asn Arg Leu His Arg Glu Cys Gly
       1235                1240                1245
   tac ccc gcg cac ctc cgc gtc cgc gtt ggc gcc ggc ggt ggc gtc      3789
   Tyr Pro Ala His Leu Arg Val Arg Val Gly Ala Gly Gly Gly Val
       1250                1255                1260
   ggc tgc ccg cag gcc gcc gcc gcc gcg ctc acc atg ggc gcc gcc      3834
   Gly Cys Pro Gln Ala Ala Ala Ala Ala Leu Thr Met Gly Ala Ala
       1265                1270                1275
   ttc atc gtc acc ggc act gtc aac cag gtc gcc aag cag tcc ggc      3879
   Phe Ile Val Thr Gly Thr Val Asn Gln Val Ala Lys Gln Ser Gly
       1280                1285                1290
   acc tgc gac aac gtg cgc aag cag ctc tcg cag gcc acc tac tcg      3924
   Thr Cys Asp Asn Val Arg Lys Gln Leu Ser Gln Ala Thr Tyr Ser
       1295                1300                1305
   gat atc tgc atg gcc ccg gcc gcc gac atg ttc gag gag ggc gtc      3969
   Asp Ile Cys Met Ala Pro Ala Ala Asp Met Phe Glu Glu Gly Val
       1310                1315                1320
   aag ctc cag gtc ctc aag aag gga acc atg ttc ccc tcg cgc gcc      4014
   Lys Leu Gln Val Leu Lys Lys Gly Thr Met Phe Pro Ser Arg Ala
       1325                1330                1335
   aac aag ctc tac gag ctc ttt tgc aag tac gac tcc ttc gac tcc      4059
   Asn Lys Leu Tyr Glu Leu Phe Cys Lys Tyr Asp Ser Phe Asp Ser
       1340                1345                1350
   atg cct cct gcc gag ctc gag cgc atc gag aag cgt atc ttc aag      4104
   Met Pro Pro Ala Glu Leu Glu Arg Ile Glu Lys Arg Ile Phe Lys
       1355                1360                1365
   cgc gca  ctc cag gag gtc tgg  gag gag acc aag gac  ttt tac att      4149
   Arg Ala  Leu Gln Glu Val Trp  Glu Glu Thr Lys Asp  Phe Tyr Ile
       1370                 1375                 1380
   aac ggt  ctc aag aac ccg gag  aag atc cag cgc gcc  gag cac gac      4194
   Asn Gly  Leu Lys Asn Pro Glu  Lys Ile Gln Arg Ala  Glu His Asp
       1385                 1390                 1395
   ccc aag  ctc aag atg tcg ctc  tgc ttc cgc tgg tac  ctt ggt ctt      4239
   Pro Lys  Leu Lys Met Ser Leu  Cys Phe Arg Trp Tyr  Leu Gly Leu
       1400                 1405                 1410
   gcc agc  cgc tgg gcc aac atg  ggc gcc ccg gac cgc  gtc atg gac      4284
   Ala Ser  Arg Trp Ala Asn Met  Gly Ala Pro Asp Arg  Val Met Asp
       1415                 1420                 1425
   tac cag  gtc tgg tgt ggc ccg  gcc att ggc gcc ttc  aac gac ttc      4329
   Tyr Gln  Val Trp Cys Gly Pro  Ala Ile Gly Ala Phe  Asn Asp Phe
       1430                 1435                 1440
   atc aag  ggc acc tac ctc gac  ccc gct gtc tcc aac  gag tac ccc      4374
   Ile Lys  Gly Thr Tyr Leu Asp  Pro Ala Val Ser Asn  Glu Tyr Pro
       1445                 1450                 1455
   tgt gtc  gtc cag atc aac ctg  caa atc ctc cgt ggt  gcc tgc tac      4419
   Cys Val  Val Gln Ile Asn Leu  Gln Ile Leu Arg Gly  Ala Cys Tyr
       1460                 1465                 1470
   ctg cgc  cgt ctc aac gcc ctg  cgc aac gac ccg cgc  att gac ctc      4464
   Leu Arg  Arg Leu Asn Ala Leu  Arg Asn Asp Pro Arg  Ile Asp Leu
       1475                 1480                 1485
   gag acc  gag gat gct gcc ttt  gtc tac gag ccc acc  aac gcg ctc      4509
   Glu Thr  Glu Asp Ala Ala Phe  Val Tyr Glu Pro Thr  Asn Ala Leu
       1490                 1495                 1500
<210> 6
   <211> 1503
   <212> PRT
   <213> Schizochytrium sp.
<400> 6
   Met Ala Leu Arg Val Lys Thr Asn Lys Lys Pro Cys Trp Glu Met Thr
   1               5                   10                  15
   Lys Glu Glu Leu Thr Ser Gly Lys Thr Glu Val Phe Asn Tyr Glu Glu
               20                  25                  30
   Leu Leu Glu Phe Ala Glu Gly Asp Ile Ala Lys Val Phe Gly Pro Glu
           35                  40                  45
   Phe Ala Val Ile Asp Lys Tyr Pro Arg Arg Val Arg Leu Pro Ala Arg
       50                  55                  60
   Glu Tyr Leu Leu Val Thr Arg Val Thr Leu Met Asp Ala Glu Val Asn
   65                  70                  75                  80
   Asn Tyr Arg Val Gly Ala Arg Met Val Thr Glu Tyr Asp Leu Pro Val
                   85                  90                  95
   Asn Gly Glu Leu Ser Glu Gly Gly Asp Cys Pro Trp Ala Val Leu Val
               100                 105                 110
   Glu Ser Gly Gln Cys Asp Leu Met Leu Ile Ser Tyr Met Gly Ile Asp
   115 120 125
   Phe Gln Asn Gln Gly Asp Arg Val Tyr Arg Leu Leu Asn Thr Thr Leu
   130 135 140
   Thr Phe Tyr Gly Val Ala His Glu Gly Glu Thr Leu Glu Tyr Asp Ile
   145 150 155 160
   Arg Val Thr Gly Phe Ala Lys Arg Leu Asp Gly Gly Ile Ser Met Phe
   165 170 175
   Phe Phe Glu Tyr Asp Cys Tyr Val Asn Gly Arg Leu Leu Ile Glu Met
   180 185 190
   Arg Asp Gly Cys Ala Gly Phe Phe Thr Asn Glu Glu Leu Asp Ala Gly
   195 200 205
   Lys Gly Val Val Phe Thr Arg Gly Asp Leu Ala Ala Arg Ala Lys Ile
   210 215 220
   Pro Lys Gln Asp Val Ser Pro Tyr Ala Val Ala Pro Cys Leu His Lys
   225 230 235 240
   Thr Lys Leu Asn Glu Lys Glu Met Gln Thr Leu Val Asp Lys Asp Trp
   245 250 255
   Ala Ser Val Phe Gly Ser Lys Asn Gly Met Pro Glu Ile Asn Tyr Lys
   260 265 270
   Leu Cys Ala Arg Lys Met Leu Met Ile Asp Arg Val Thr Ser Ile Asp
   275 280 285
   His Lys Gly Gly Val Tyr Gly Leu Gly Gln Leu Val Gly Glu Lys Ile
   290 295 300
   Leu Glu Arg Asp His Trp Tyr Phe Pro Cys His Phe Val Lys Asp Gln
   305 310 315 320
   Val Met Ala Gly Ser Leu Val Ser Asp Gly Cys Ser Gln Met Leu Lys
   325 330 335
   Met Tyr Met Ile Trp Leu Gly Leu His Leu Thr Thr Gly Pro Phe Asp
   340 345 350
   Phe Arg Pro Val Asn Gly His Pro Asn Lys Val Arg Cys Arg Gly Gln
   355 360 365
   Ile Ser Pro His Lys Gly Lys Leu Val Tyr Val Met Glu Ile Lys Glu
   370 375 380
   Met Gly Phe Asp Glu Asp Asn Asp Pro Tyr Ala Ile Ala Asp Val Asn
   385 390 395 400
   Ile Ile Asp Val Asp Phe Glu Lys Gly Gln Asp Phe Ser Leu Asp Arg
                   405                 410                 415
   Ile Ser Asp Tyr Gly Lys Gly Asp Leu Asn Lys Lys Ile Val Val Asp
               420                 425                 430
   Phe Lys Gly Ile Ala Leu Lys Met Gln Lys Arg Ser Thr Asn Lys Asn
           435                 440                 445
   Pro Ser Lys Val Gln Pro Val Phe Ala Asn Gly Ala Ala Thr Val Gly
       450                 455                 460
   Pro Glu Ala Ser Lys Ala Ser Ser Gly Ala Ser Ala Ser Ala Ser Ala
   465                 470                 475                 480
   Ala Pro Ala Lys Pro Ala Phe Ser Ala Asp Val Leu Ala Pro Lys Pro
                   485                 490                 495
   Val Ala Leu Pro Glu His Ile Leu Lys Gly Asp Ala Leu Ala Pro Lys
               500                 505                 510
   Glu Met Ser Trp His Pro Met Ala Arg Ile Pro Gly Asn Pro Thr Pro
           515                 520                 525
   Ser Phe Ala Pro Ser Ala Tyr Lys Pro Arg Asn Ile Ala Phe Thr Pro
       530                 535                 540
   Phe Pro Gly Asn Pro Asn Asp Asn Asp His Thr Pro Gly Lys Met Pro
   545                 550                 555                 560
   Leu Thr Trp Phe Asn Met Ala Glu Phe Met Ala Gly Lys Val Ser Met
                   565                 570                 575
   Cys Leu Gly Pro Glu Phe Ala Lys Phe Asp Asp Ser Asn Thr Ser Arg
               580                 585                 590
   Ser Pro Ala Trp Asp Leu Ala Leu Val Thr Arg Ala Val Ser Val Ser
           595                 600                 605
   Asp Leu Lys His Val Asn Tyr Arg Asn Ile Asp Leu Asp Pro Ser Lys
       610                 615                 620
   Gly Thr Met Val Gly Glu Phe Asp Cys Pro Ala Asp Ala Trp Phe Tyr
   625                 630                 635                 640
   Lys Gly Ala Cys Asn Asp Ala His Met Pro Tyr Ser Ile Leu Met Glu
                   645                 650                 655
   Ile Ala Leu Gln Thr Ser Gly Val Leu Thr Ser Val Leu Lys Ala Pro
               660                 665                 670
   Leu Thr Met Glu Lys Asp Asp Ile Leu Phe Arg Asn Leu Asp Ala Asn
           675                 680                 685
   Ala Glu Phe Val Arg Ala Asp Leu Asp Tyr Arg Gly Lys Thr Ile Arg
   690 695 700
   Asn Val Thr Lys Cys Thr Gly Tyr Ser Met Leu Gly Glu Met Gly Val
   705 710 715 720
   His Arg Phe Thr Phe Glu Leu Tyr Val Asp Asp Val Leu Phe Tyr Lys
   725 730 735
   Gly Ser Thr Ser Phe Gly Trp Phe Val Pro Glu Val Phe Ala Ala Gln
   740 745 750
   Ala Gly Leu Asp Asn Gly Arg Lys Ser Glu Pro Trp Phe Ile Glu Asn
   755 760 765
   Lys Val Pro Ala Ser Gln Val Ser Ser Phe Asp Val Arg Pro Asn Gly
   770 775 780
   Ser Gly Arg Thr Ala Ile Phe Ala Asn Ala Pro Ser Gly Ala Gln Leu
   785 790 795 800
   Asn Arg Arg Thr Asp Gln Gly Gln Tyr Leu Asp Ala Val Asp Ile Val
   805 810 815
   Ser Gly Ser Gly Lys Lys Ser Leu Gly Tyr Ala His Gly Ser Lys Thr
   820 825 830
   Val Asn Pro Asn Asp Trp Phe Phe Ser Cys His Phe Trp Phe Asp Ser
   835 840 845
   Val Met Pro Gly Ser Leu Gly Val Glu Ser Met Phe Gln Leu Val Glu
   850 855 860
   Ala Ile Ala Ala His Glu Asp Leu Ala Gly Lys Ala Arg His Cys Gln
   865 870 875 880
   Pro His Leu Cys Ala Arg Pro Arg Ala Arg Ser Ser Trp Lys Tyr Arg
   885 890 895
   Gly Gln Leu Thr Pro Lys Ser Lys Lys Met Asp Ser Glu Val His Ile
   900 905 910
   Val Ser Val Asp Ala His Asp Gly Val Val Asp Leu Val Ala Asp Gly
   915 920 925
   Phe Leu Trp Ala Asp Ser Leu Arg Val Tyr Ser Val Ser Asn Ile Arg
   930 935 940
   Val Arg Ile Ala Ser Gly Glu Ala Pro Ala Ala Ala Ser Ser Ala Ala
   945 950 955 960
   Ser Val Gly Ser Ser Ala Ser Ser Val Glu Arg Thr Arg Ser Ser Pro
   965 970 975
   Ala Val Ala Ser Gly Pro Ala Gln Thr Ile Asp Leu Lys Gln Leu Lys
   980 985 990
   Thr Glu Leu Leu Glu Leu Asp Ala Pro Leu Tyr Leu Ser Gln Asp Pro
   995 1000 1005
   Thr Ser Gly Gln Leu Lys Lys His Thr Asp Val Ala Ser Gly Gln
   1010 1015 1020
   Ala Thr Ile Val Gln Pro Cys Thr Leu Gly Asp Leu Gly Asp Arg
   1025 1030 1035
   Ser Phe Met Glu Thr Tyr Gly Val Val Ala Pro Leu Tyr Thr Gly
   1040 1045 1050
   Ala Met Ala Lys Gly Ile Ala Ser Ala Asp Leu Val Ile Ala Ala
   1055 1060 1065
   Gly Lys Arg Lys Ile Leu Gly Ser Phe Gly Ala Gly Gly Leu Pro
   1070 1075 1080
   Met His His Val Arg Ala Ala Leu Glu Lys Ile Gln Ala Ala Leu
   1085 1090 1095
   Pro Gln Gly Pro Tyr Ala Val Asn Leu Ile His Ser Pro Phe Asp
   1100 1105 1110
   Ser Asn Leu Glu Lys Gly Asn Val Asp Leu Phe Leu Glu Lys Gly
   1115 1120 1125
   Val Thr Val Val Glu Ala Ser Ala Phe Met Thr Leu Thr Pro Gln
   1130 1135 1140
   Val Val Arg Tyr Arg Ala Ala Gly Leu Ser Arg Asn Ala Asp Gly
   1145 1150 1155
   Ser Val Asn Ile Arg Asn Arg Ile Ile Gly Lys Val Ser Arg Thr
   1160 1165 1170
   Glu Leu Ala Glu Met Phe Ile Arg Pro Ala Pro Glu His Leu Leu
   1175 1180 1185
   Glu Lys Leu Ile Ala Ser Gly Glu Ile Thr Gln Glu Gln Ala Glu
   1190 1195 1200
   Leu Ala Arg Arg Val Pro Val Ala Asp Asp Ile Ala Val Glu Ala
   1205 1210 1215
   Asp Ser Gly Gly His Thr Asp Asn Arg Pro Ile His Val Ile Leu
   1220 1225 1230
   Pro Leu Ile Ile Asn Leu Arg Asn Arg Leu His Arg Glu Cys Gly
   1235 1240 1245
   Tyr Pro Ala His Leu Arg Val Arg Val Gly Ala Gly Gly Gly Val
   1250 1255 1260
   Gly Cys Pro Gln Ala Ala Ala Ala Ala Leu Thr Met Gly Ala Ala
   1265 1270 1275
   Phe Ile Val Thr Gly Thr Val Asn Gln Val Ala Lys Gln Ser Gly
   1280 1285 1290
   Thr Cys Asp Asn Val Arg Lys Gln Leu Ser Gln Ala Thr Tyr Ser
   1295 1300 1305
   Asp Ile Cys Met Ala Pro Ala Ala Asp Met Phe Glu Glu Gly Val
   1310 1315 1320
   Lys Leu Gln Val Leu Lys Lys Gly Thr Met Phe Pro Ser Arg Ala
   1325 1330 1335
   Asn Lys Leu Tyr Glu Leu Phe Cys Lys Tyr Asp Ser Phe Asp Ser
   1340 1345 1350
   Met Pro Pro Ala Glu Leu Glu Arg Ile Glu Lys Arg Ile Phe Lys
   1355 1360 1365
   Arg Ala Leu Gln Glu Val Trp Glu Glu Thr Lys Asp Phe Tyr Ile
   1370 1375 1380
   Asn Gly Leu Lys Asn Pro Glu Lys Ile Gln Arg Ala Glu His Asp
   1385 1390 1395
   Pro Lys Leu Lys Met Ser Leu Cys Phe Arg Trp Tyr Leu Gly Leu
   1400 1405 1410
   Ala Ser Arg Trp Ala Asn Met Gly Ala Pro Asp Arg Val Met Asp
   1415 1420 1425
   Tyr Gln Val Trp Cys Gly Pro Ala Ile Gly Ala Phe Asn Asp Phe
   1430 1435 1440
   Ile Lys Gly Thr Tyr Leu Asp Pro Ala Val Ser Asn Glu Tyr Pro
   1445 1450 1455
   Cys Val Val Gln Ile Asn Leu Gln Ile Leu Arg Gly Ala Cys Tyr
   1460 1465 1470
   Leu Arg Arg Leu Asn Ala Leu Arg Asn Asp Pro Arg Ile Asp Leu
   1475 1480 1485
   Glu Thr Glu Asp Ala Ala Phe Val Tyr Glu Pro Thr Asn Ala Leu
   1490 1495 1500
<210> 7
   <211> 600
   <212> DNA
   <213> Schizochytrium sp.
<220>
   <221> CDS
   <222> (1)..(600)
<400> 7
   atg gcg gcc cgt ctg cag gag caa aag gga ggc gag atg gat acc cgc         48
   Met Ala Ala Arg Leu Gln Glu Gln Lys Gly Gly Glu Met Asp Thr Arg
   1               5                   10                  15
   att gcc atc atc ggc atg tcg gcc atc ctc ccc tgc ggc acg acc gtg         96
   Ile Ala Ile Ile Gly Met Ser Ala Ile Leu Pro Cys Gly Thr Thr Val
               20                  25                  30
   cgc gag tcg tgg gag acc atc cgc gcc ggc atc gac tgc ctg tcg gat        144
   Arg Glu Ser Trp Glu Thr Ile Arg Ala Gly Ile Asp Cys Leu Ser Asp
           35                  40                  45
   ctc ccc gag gac cgc gtc gac gtg acg gcg tac ttt gac ccc gtc aag        192
   Leu Pro Glu Asp Arg Val Asp Val Thr Ala Tyr Phe Asp Pro Val Lys
       50                  55                  60
   acc acc aag gac aag atc tac tgc aag cgc ggt ggc ttc att ccc gag        240
   Thr Thr Lys Asp Lys Ile Tyr Cys Lys Arg Gly Gly Phe Ile Pro Glu
   65                  70                  75                  80
   tac gac ttt gac gcc cgc gag ttc gga ctc aac atg ttc cag atg gag        288
   Tyr Asp Phe Asp Ala Arg Glu Phe Gly Leu Asn Met Phe Gln Met Glu
                   85                  90                  95
   gac tcg gac gca aac cag acc atc tcg ctt ctc aag gtc aag gag gcc        336
   Asp Ser Asp Ala Asn Gln Thr Ile Ser Leu Leu Lys Val Lys Glu Ala
               100                 105                 110
   ctc cag gac gcc ggc atc gac gcc ctc ggc aag gaa aag aag aac atc        384
   Leu Gln Asp Ala Gly Ile Asp Ala Leu Gly Lys Glu Lys Lys Asn Ile
           115                 120                 125
   ggc tgc gtg ctc ggc att ggc ggc ggc caa aag tcc agc cac gag ttc        432
   Gly Cys Val Leu Gly Ile Gly Gly Gly Gln Lys Ser Ser His Glu Phe
       130                 135                 140
   tac tcg cgc ctt aat tat gtt gtc gtg gag aag gtc ctc cgc aag atg        480
   Tyr Ser Arg Leu Asn Tyr Val Val Val Glu Lys Val Leu Arg Lys Met
   145                 150                 155                 160
   ggc atg ccc gag gag gac gtc aag gtc gcc gtc gaa aag tac aag gcc        528
   Gly Met Pro Glu Glu Asp Val Lys Val Ala Val Glu Lys Tyr Lys Ala
                   165                 170                 175
   aac ttc ccc gag tgg cgc ctc gac tcc ttc cct ggc ttc ctc ggc aac        576
   Asn Phe Pro Glu Trp Arg Leu Asp Ser Phe Pro Gly Phe Leu Gly Asn
               180                 185                 190
   gtc acc gcc ggt cgc tgc acc aac                                        600
   Val Thr Ala Gly Arg Cys Thr Asn
           195                 200
<210> 8
   <211> 200
   <212> PRT
   <213> Schizochytrium sp.
<400> 8
   Met Ala Ala Arg Leu Gln Glu Gln Lys Gly Gly Glu Met Asp Thr Arg
   1               5                   10                  15
   Ile Ala Ile Ile Gly Met Ser Ala Ile Leu Pro Cys Gly Thr Thr Val
               20                  25                  30
   Arg Glu Ser Trp Glu Thr Ile Arg Ala Gly Ile Asp Cys Leu Ser Asp
           35                  40                  45
   Leu Pro Glu Asp Arg Val Asp Val Thr Ala Tyr Phe Asp Pro Val Lys
       50                  55                  60
   Thr Thr Lys Asp Lys Ile Tyr Cys Lys Arg Gly Gly Phe Ile Pro Glu
   65                  70                  75                  80
   Tyr Asp Phe Asp Ala Arg Glu Phe Gly Leu Asn Met Phe Gln Met Glu
                   85                  90                  95
   Asp Ser Asp Ala Asn Gln Thr Ile Ser Leu Leu Lys Val Lys Glu Ala
               100                 105                 110
   Leu Gln Asp Ala Gly Ile Asp Ala Leu Gly Lys Glu Lys Lys Asn Ile
           115                 120                 125
   Gly Cys Val Leu Gly Ile Gly Gly Gly Gln Lys Ser Ser His Glu Phe
       130                 135                 140
   Tyr Ser Arg Leu Asn Tyr Val Val Val Glu Lys Val Leu Arg Lys Met
   145                 150                 155                 160
   Gly Met Pro Glu Glu Asp Val Lys Val Ala Val Glu Lys Tyr Lys Ala
                   165                 170                 175
   Asn Phe Pro Glu Trp Arg Leu Asp Ser Phe Pro Gly Phe Leu Gly Asn
               180                 185                 190
   Val Thr Ala Gly Arg Cys Thr Asn
           195                 200
<210> 9
   <211> 1278
   <212> DNA
   <213> Schizochytrium sp.
<220>
   <221> CDS
   <222> (1)..(1278)
<400> 9
   gat gtc acc aag gag gcc tgg cgc ctc ccc cgc gag ggc gtc agc ttc        48
   Asp Val Thr Lys Glu Ala Trp Arg Leu Pro Arg Glu Gly Val Ser Phe
   1               5                   10                  15
   cgc gcc aag ggc atc gcc acc aac ggc gct gtc gcc gcg ctc ttc tcc        96
   Arg Ala Lys Gly Ile Ala Thr Asn Gly Ala Val Ala Ala Leu Phe Ser
               20                  25                  30
   ggc cag ggc gcg cag tac acg cac atg ttt agc gag gtg gcc atg aac       144
   Gly Gln Gly Ala Gln Tyr Thr His Met Phe Ser Glu Val Ala Met Asn
           35                  40                  45
   tgg ccc cag ttc cgc cag agc att gcc gcc atg gac gcc gcc cag tcc     192
   Trp Pro Gln Phe Arg Gln Ser Ile Ala Ala Met Asp Ala Ala Gln Ser
       50                  55                  60
   aag gtc gct gga agc gac aag gac ttt gag cgc gtc tcc cag gtc ctc     240
   Lys Val Ala Gly Ser Asp Lys Asp Phe Glu Arg Val Ser Gln Val Leu
   65                  70                  75                  80
   tac ccg cgc aag ccg tac gag cgt gag ccc gag cag aac ccc aag aag     288
   Tyr Pro Arg Lys Pro Tyr Glu Arg Glu Pro Glu Gln Asn Pro Lys Lys
                   85                  90                  95
   atc tcc ctc acc gcc tac tcg cag ccc tcg acc ctg gcc tgc gct ctc     336
   Ile Ser Leu Thr Ala Tyr Ser Gln Pro Ser Thr Leu Ala Cys Ala Leu
               100                 105                 110
   ggt gcc ttt gag atc ttc aag gag gcc ggc ttc acc ccg gac ttt gcc     384
   Gly Ala Phe Glu Ile Phe Lys Glu Ala Gly Phe Thr Pro Asp Phe Ala
           115                 120                 125
   gcc ggc cat tcg ctc ggt gag ttc gcc gcc ctc tac gcc gcg ggc tgc     432
   Ala Gly His Ser Leu Gly Glu Phe Ala Ala Leu Tyr Ala Ala Gly Cys
       130                 135                 140
   gtc gac cgc gac gag ctc ttt gag ctt gtc tgc cgc cgc gcc cgc atc     480
   Val Asp Arg Asp Glu Leu Phe Glu Leu Val Cys Arg Arg Ala Arg Ile
   145                 150                 155                 160
   atg ggc ggc aag gac gca ccg gcc acc ccc aag gga tgc atg gcc gcc     528
   Met Gly Gly Lys Asp Ala Pro Ala Thr Pro Lys Gly Cys Met Ala Ala
                   165                 170                 175
   gtc att ggc ccc aac gcc gag aac atc aag gtc cag gcc gcc aac gtc     576
   Val Ile Gly Pro Asn Ala Glu Asn Ile Lys Val Gln Ala Ala Asn Val
               180                 185                 190
   tgg ctc ggc aac tcc aac tcg cct tcg cag acc gtc atc acc ggc tcc     624
   Trp Leu Gly Asn Ser Asn Ser Pro Ser Gln Thr Val Ile Thr Gly Ser
           195                 200                 205
   gtc gaa ggt atc cag gcc gag agc gcc cgc ctc cag aag gag ggc ttc     672
   Val Glu Gly Ile Gln Ala Glu Ser Ala Arg Leu Gln Lys Glu Gly Phe
       210                 215                 220
   cgc gtc gtg cct ctt gcc tgc gag agc gcc ttc cac tcg ccc cag atg     720
   Arg Val Val Pro Leu Ala Cys Glu Ser Ala Phe His Ser Pro Gln Met
   225                 230                 235                 240
   gag aac gcc tcg tcg gcc ttc aag gac gtc atc tcc aag gtc tcc ttc     768
   Glu Asn Ala Ser Ser Ala Phe Lys Asp Val Ile Ser Lys Val Ser Phe
                   245                 250                 255
   cgc acc ccc aag gcc gag acc aag ctc ttc agc aac gtc tct ggc gag     816
   Arg Thr Pro Lys Ala Glu Thr Lys Leu Phe Ser Asn Val Ser Gly Glu
               260                 265                 270
   acc tac ccc acg gac gcc cgc gag atg ctt acg cag cac atg acc agc     864
   Thr Tyr Pro Thr Asp Ala Arg Glu Met Leu Thr Gln His Met Thr Ser
           275                 280                 285
   agc gtc aag ttc ctc acc cag gtc cgc aac atg cac cag gcc ggt gcg     912
   Ser Val Lys Phe Leu Thr Gln Val Arg Asn Met His Gln Ala Gly Ala
       290                 295                 300
   cgc atc ttt gtc gag ttc gga ccc aag cag gtg ctc tcc aag ctt gtc     960
   Arg Ile Phe Val Glu Phe Gly Pro Lys Gln Val Leu Ser Lys Leu Val
   305                 310                 315                 320
   tcc gag acc ctc aag gat gac ccc tcg gtt gtc acc gtc tct gtc aac     1008
   Ser Glu Thr Leu Lys Asp Asp Pro Ser Val Val Thr Val Ser Val Asn
                   325                 330                 335
   ccg gcc tcg ggc acg gat tcg gac atc cag ctc cgc gac gcg gcc gtc      1056
   Pro Ala Ser Gly Thr Asp Ser Asp Ile Gln Leu Arg Asp Ala Ala Val
               340                 345                 350
   cag ctc gtt gtc gct ggc gtc aac ctt cag ggc ttt gac aag tgg gac      1104
   Gln Leu Val Val Ala Gly Val Asn Leu Gln Gly Phe Asp Lys Trp Asp
           355                 360                 365
   gcc ccc gat gcc acc cgc atg cag gcc atc aag aag aag cgc act acc      1152
   Ala Pro Asp Ala Thr Arg Met Gln Ala Ile Lys Lys Lys Arg Thr Thr
       370                 375                 380
   ctc cgc ctt tcg gcc gcc acc tac gtc tcg gac aag acc aag aag gtc      1200
   Leu Arg Leu Ser Ala Ala Thr Tyr Val Ser Asp Lys Thr Lys Lys Val
   385                 390                 395                 400
   cgc gac gcc gcc atg aac gat ggc cgc tgc gtc acc tac ctc aag ggc      1248
   Arg Asp Ala Ala Met Asn Asp Gly Arg Cys Val Thr Tyr Leu Lys Gly
                   405                 410                 415
   gcc gca ccg ctc atc aag gcc ccg gag ccc                              1278
   Ala Ala Pro Leu Ile Lys Ala Pro Glu Pro
               420                 425
<210> 10
   <211> 426
   <212> PRT
   <213> Schizochytrium sp.
<400> 10
   Asp Val Thr Lys Glu Ala Trp Arg Leu Pro Arg Glu Gly Val Ser Phe
   1 5 10 15
   Arg Ala Lys Gly Ile Ala Thr Asn Gly Ala Val Ala Ala Leu Phe Ser
   20 25 30
   Gly Gln Gly Ala Gln Tyr Thr His Met Phe Ser Glu Val Ala Met Asn
   35 40 45
   Trp Pro Gln Phe Arg Gln Ser Ile Ala Ala Met Asp Ala Ala Gln Ser
   50 55 60
   Lys Val Ala Gly Ser Asp Lys Asp Phe Glu Arg Val Ser Gln Val Leu
   65 70 75 80
   Tyr Pro Arg Lys Pro Tyr Glu Arg Glu Pro Glu Gln Asn Pro Lys Lys
   85 90 95
   Ile Ser Leu Thr Ala Tyr Ser Gln Pro Ser Thr Leu Ala Cys Ala Leu
   100 105 110
   Gly Ala Phe Glu Ile Phe Lys Glu Ala Gly Phe Thr Pro Asp Phe Ala
   115 120 125
   Ala Gly His Ser Leu Gly Glu Phe Ala Ala Leu Tyr Ala Ala Gly Cys
   130 135 140
   Val Asp Arg Asp Glu Leu Phe Glu Leu Val Cys Arg Arg Ala Arg Ile
   145 150 155 160
   Met Gly Gly Lys Asp Ala Pro Ala Thr Pro Lys Gly Cys Met Ala Ala
                   165                 170                 175
   Val Ile Gly Pro Asn Ala Glu Asn Ile Lys Val Gln Ala Ala Asn Val
               180                 185                 190
   Trp Leu Gly Asn Ser Asn Ser Pro Ser Gln Thr Val Ile Thr Gly Ser
           195                 200                 205
   Val Glu Gly Ile Gln Ala Glu Ser Ala Arg Leu Gln Lys Glu Gly Phe
       210                 215                 220
   Arg Val Val Pro Leu Ala Cys Glu Ser Ala Phe His Ser Pro Gln Met
   225                 230                 235                 240
   Glu Asn Ala Ser Ser Ala Phe Lys Asp Val Ile Ser Lys Val Ser Phe
                   245                 250                 255
   Arg Thr Pro Lys Ala Glu Thr Lys Leu Phe Ser Asn Val Ser Gly Glu
               260                 265                 270
   Thr Tyr Pro Thr Asp Ala Arg Glu Met Leu Thr Gln His Met Thr Ser
           275                 280                 285
   Ser Val Lys Phe Leu Thr Gln Val Arg Asn Met His Gln Ala Gly Ala
       290                 295                 300
   Arg Ile Phe Val Glu Phe Gly Pro Lys Gln Val Leu Ser Lys Leu Val
   305                 310                 315                 320
   Ser Glu Thr Leu Lys Asp Asp Pro Ser Val Val Thr Val Ser Val Asn
                   325                 330                 335
   Pro Ala Ser Gly Thr Asp Ser Asp Ile Gln Leu Arg Asp Ala Ala Val
               340                 345                 350
   Gln Leu Val Val Ala Gly Val Asn Leu Gln Gly Phe Asp Lys Trp Asp
           355                 360                 365
   Ala Pro Asp Ala Thr Arg Met Gln Ala Ile Lys Lys Lys Arg Thr Thr
       370                 375                 380
   Leu Arg Leu Ser Ala Ala Thr Tyr Val Ser Asp Lys Thr Lys Lys Val
   385                 390                 395                 400
   Arg Asp Ala Ala Met Asn Asp Gly Arg Cys Val Thr Tyr Leu Lys Gly
                   405                 410                 415
   Ala Ala Pro Leu Ile Lys Ala Pro Glu Pro
               420                 425
<210> 11
   <211> 5
   <212> PRT
   <213> Schizochytrium sp.
<220>
   <221> MISC_FEATURE
   <222> (1)..(5)
   <223> Xaa = any amino acid
<400> 11
   Gly His Ser Xaa Gly
   1               5
<210> 12
   <211> 258
   <212> DNA
   <213> Schizochytrium sp.
<220>
   <221> CDS
   <222> (1)..(258)
<400> 12
   gct gtc tcg aac gag ctt ctt gag aag gcc gag act gtc gtc atg gag      48
   Ala Val Ser Asn Glu Leu Leu Glu Lys Ala Glu Thr Val Val Met Glu
   1               5                   10                  15
   gtc ctc gcc gcc aag acc ggc tac gag acc gac atg atc gag gct gac      96
   Val Leu Ala Ala Lys Thr Gly Tyr Glu Thr Asp Met Ile Glu Ala Asp
               20                  25                  30
   atg gag ctc gag acc gag ctc ggc att gac tcc atc aag cgt gtc gag      144
   Met Glu Leu Glu Thr Glu Leu Gly Ile Asp Ser Ile Lys Arg Val Glu
           35                  40                  45
   atc ctc tcc gag gtc cag gcc atg ctc aat gtc gag gcc aag gat gtc      192
   Ile Leu Ser Glu Val Gln Ala Met Leu Asn Val Glu Ala Lys Asp Val
       50                  55                  60
   gat gcc ctc agc cgc act cgc act gtt ggt gag gtt gtc aac gcc atg      240
   Asp Ala Leu Ser Arg Thr Arg Thr Val Gly Glu Val Val Asn Ala Met
   65                  70                  75                  80
   aag gcc gag atc gct ggc                                              258
   Lys Ala Glu Ile Ala Gly
                   85
<210> 13
   <211> 86
   <212> PRT
   <213> Schizochytrium sp.
<400> 13
   Ala Val Ser Asn Glu Leu Leu Glu Lys Ala Glu Thr Val Val Met Glu
   1               5                   10                  15
   Val Leu Ala Ala Lys Thr Gly Tyr Glu Thr Asp Met Ile Glu Ala Asp
               20                  25                  30
   Met Glu Leu Glu Thr Glu Leu Gly Ile Asp Ser Ile Lys Arg Val Glu
           35                  40                  45
   Ile Leu Ser Glu Val Gln Ala Met Leu Asn Val Glu Ala Lys Asp Val
       50                  55                  60
   Asp Ala Leu Ser Arg Thr Arg Thr Val Gly Glu Val Val Asn Ala Met
   65                  70                  75                  80
   Lys Ala Glu Ile Ala Gly
                   85
<210> 14
   <211> 5
   <212> PRT
   <213> Schizochytrium sp.
<400> 14
   Leu Gly Ile Asp Ser
   1               5
<210> 15
   <211> 21
   <212> PRT
   <213> Schizochytrium sp.
<400> 15
   Ala Pro Ala Pro Val Lys Ala Ala Ala Pro Ala Ala Pro Val Ala Ser
   1               5                   10                  15
   Ala Pro Ala Pro Ala
               20
<210> 16
   <211> 3006
   <212> DNA
   <213> Schizochytrium sp.
<400> 16
   gcccccgccc cggtcaaggc tgctgcgcct gccgcccccg ttgcctcggc ccctgccccg 60
   gctgtctcga acgagcttct tgagaaggcc gagactgtcg tcatggaggt cctcgccgcc 120
   aagaccggct acgagaccga catgatcgag gctgacatgg agctcgagac cgagctcggc 180
   attgactcca tcaagcgtgt cgagatcctc tccgaggtcc aggccatgct caatgtcgag 240
   gccaaggatg tcgatgccct cagccgcact cgcactgttg gtgaggttgt caacgccatg 300
   aaggccgaga tcgctggcag ctctgccccg gcgcctgctg ccgctgctcc ggctccggcc 360
   aaggctgccc ctgccgccgc tgcgcctgct gtctcgaacg agcttctcga gaaggccgag 420
   accgtcgtca tggaggtcct cgccgccaag actggctacg agactgacat gatcgagtcc 480
   gacatggagc tcgagactga gctcggcatt gactccatca agcgtgtcga gatcctctcc 540
   gaggttcagg ccatgctcaa cgtcgaggcc aaggacgtcg acgctctcag ccgcactcgc 600
   actgtgggtg aggtcgtcaa cgccatgaag gctgagatcg ctggtggctc tgccccggcg 660
   cctgccgccg ctgccccagg tccggctgct gccgcccctg cgcctgccgc cgccgcccct 720
   gctgtctcga acgagcttct tgagaaggcc gagaccgtcg tcatggaggt cctcgccgcc 780
   aagactggct acgagactga catgatcgag tccgacatgg agctcgagac cgagctcggc 840
   attgactcca tcaagcgtgt cgagattctc tccgaggtcc aggccatgct caacgtcgag 900
   gccaaggacg tcgacgctct cagccgcacc cgcactgttg gcgaggtcgt cgatgccatg 960
   aaggccgaga tcgctggtgg ctctgccccg gcgcctgccg ccgctgctcc tgctccggct 1020
   gctgccgccc ctgcgcctgc cgcccctgcg cctgctgtct cgagcgagct tctcgagaag 1080
   gccgagactg tcgtcatgga ggtcctcgcc gccaagactg gctacgagac tgacatgatc 1140
   gagtccgaca tggagctcga gaccgagctc ggcattgact ccatcaagcg tgtcgagatt 1200
   ctctccgagg tccaggccat gctcaacgtc gaggccaagg acgtcgacgc tctcagccgc 1260
   acccgcactg ttggcgaggt cgtcgatgcc atgaaggccg agatcgctgg tggctctgcc 1320
   ccggcgcctg ccgccgctgc tcctgctccg gctgctgccg cccctgcgcc tgccgcccct 1380
   gcgcctgccg cccctgcgcc tgctgtctcg agcgagcttc tcgagaaggc cgagactgtc 1440
   gtcatggagg tcctcgccgc caagactggc tacgagactg acatgattga gtccgacatg 1500
   gagctcgaga ccgagctcgg cattgactcc atcaagcgtg tcgagattct ctccgaggtt 1560
   caggccatgc tcaacgtcga ggccaaggac gtcgacgctc tcagccgcac tcgcactgtt 1620
   ggtgaggtcg tcgatgccat gaaggctgag atcgctggca gctccgcctc ggcgcctgcc 1680
   gccgctgctc ctgctccggc tgctgccgct cctgcgcccg ctgccgccgc ccctgctgtc 1740
   tcgaacgagc ttctcgagaa agccgagact gtcgtcatgg aggtcctcgc cgccaagact 1800
   ggctacgaga ctgacatgat cgagtccgac atggagctcg agactgagct cggcattgac 1860
   tccatcaagc gtgtcgagat cctctccgag gttcaggcca tgctcaacgt cgaggccaag 1920
   gacgtcgatg ccctcagccg cacccgcact gttggcgagg ttgtcgatgc catgaaggcc 1980
   gagatcgctg gtggctctgc cccggcgcct gccgccgctg cccctgctcc ggctgccgcc 2040
   gcccctgctg tctcgaacga gcttctcgag aaggccgaga ctgtcgtcat ggaggtcctc 2100
   gccgccaaga ctggctacga gaccgacatg atcgagtccg acatggagct cgagaccgag 2160
   ctcggcattg actccatcaa gcgtgtcgag attctctccg aggttcaggc catgctcaac 2220
   gtcgaggcca aggacgtcga tgctctcagc cgcactcgca ctgttggcga ggtcgtcgat 2280
   gccatgaagg ctgagatcgc cggcagctcc gccccggcgc ctgccgccgc tgctcctgct 2340
   ccggctgctg ccgctcctgc gcccgctgcc gctgcccctg ctgtctcgag cgagcttctc 2400
   gagaaggccg agaccgtcgt catggaggtc ctcgccgcca agactggcta cgagactgac 2460
   atgattgagt ccgacatgga gctcgagact gagctcggca ttgactccat caagcgtgtc 2520
   gagatcctct ccgaggttca ggccatgctc aacgtcgagg ccaaggacgt cgatgccctc 2580
   agccgcaccc gcactgttgg cgaggttgtc gatgccatga aggccgagat cgctggtggc 2640
   tctgccccgg cgcctgccgc cgctgcccct gctccggctg ccgccgcccc tgctgtctcg 2700
   aacgagcttc ttgagaaggc cgagaccgtc gtcatggagg tcctcgccgc caagactggc 2760
   tacgagaccg acatgatcga gtccgacatg gagctcgaga ccgagctcgg cattgactcc 2820
   atcaagcgtg tcgagattct ctccgaggtt caggccatgc tcaacgtcga ggccaaggac 2880
   gtcgacgctc tcagccgcac tcgcactgtt ggcgaggtcg tcgatgccat gaaggctgag 2940
   atcgctggtg gctctgcccc ggcgcctgcc gccgctgctc ctgcctcggc tggcgccgcg 3000
   cctgcg 3006
<210> 17
   <211> 2133
   <212> DNA
   <213> Schizochytrium sp.
<220>
   <221> CDS
   <222> (1)..(2133)
<400> 17
   ttt ggc gct ctc ggc ggc ttc atc tcg cag cag gcg gag cgc ttc gag        48
   Phe Gly Ala Leu Gly Gly Phe Ile Ser Gln Gln Ala Glu Arg Phe Glu
   1               5                   10                  15
   ccc gcc gaa atc ctc ggc ttc acg ctc atg tgc gcc aag ttc gcc aag        96
   Pro Ala Glu Ile Leu Gly Phe Thr Leu Met Cys Ala Lys Phe Ala Lys
               20                  25                  30
   gct tcc ctc tgc acg gct gtg gct ggc ggc cgc ccg gcc ttt atc ggt       144
   Ala Ser Leu Cys Thr Ala Val Ala Gly Gly Arg Pro Ala Phe Ile Gly
           35                  40                  45
   gtg gcg cgc ctt gac ggc cgc ctc gga ttc act tcg cag ggc act tct       192
   Val Ala Arg Leu Asp Gly Arg Leu Gly Phe Thr Ser Gln Gly Thr Ser
       50                  55                  60
   gac gcg ctc aag cgt gcc cag cgt ggt gcc atc ttt ggc ctc tgc aag       240
   Asp Ala Leu Lys Arg Ala Gln Arg Gly Ala Ile Phe Gly Leu Cys Lys
   65                  70                  75                  80
   acc atc ggc ctc gag tgg tcc gag tct gac gtc ttt tcc cgc ggc gtg       288
   Thr Ile Gly Leu Glu Trp Ser Glu Ser Asp Val Phe Ser Arg Gly Val
                   85                  90                  95
   gac att gct cag ggc atg cac ccc gag gat gcc gcc gtg gcg att gtg       336
   Asp Ile Ala Gln Gly Met His Pro Glu Asp Ala Ala Val Ala Ile Val
               100                 105                 110
   cgc gag atg gcg tgc gct gac att cgc att cgc gag gtc ggc att ggc       384
   Arg Glu Met Ala Cys Ala Asp Ile Arg Ile Arg Glu Val Gly Ile Gly
           115                 120                 125
   gca aac cag cag cgc tgc acg atc cgt gcc gcc aag ctc gag acc ggc       432
   Ala Asn Gln Gln Arg Cys Thr Ile Arg Ala Ala Lys Leu Glu Thr Gly
       130                 135                 140
   aac ccg cag cgc cag atc gcc aag gac gac gtg ctg ctc gtt tct ggc       480
   Asn Pro Gln Arg Gln Ile Ala Lys Asp Asp Val Leu Leu Val Ser Gly
   145                 150                 155                 160
   ggc gct cgc ggc atc acg cct ctt tgc atc cgg gag atc acg cgc cag       528
   Gly Ala Arg Gly Ile Thr Pro Leu Cys Ile Arg Glu Ile Thr Arg Gln
                   165                 170                 175
   atc gcg ggc ggc aag tac att ctg ctt ggc cgc agc aag gtc tct gcg       576
   Ile Ala Gly Gly Lys Tyr Ile Leu Leu Gly Arg Ser Lys Val Ser Ala
               180                 185                 190
   agc gaa ccg gca tgg tgc gct ggc atc act gac gag aag gct gtg caa       624
   Ser Glu Pro Ala Trp Cys Ala Gly Ile Thr Asp Glu Lys Ala Val Gln
           195                 200                 205
   aag gct gct acc cag gag ctc aag cgc gcc ttt agc gct ggc gag ggc       672
   Lys Ala Ala Thr Gln Glu Leu Lys Arg Ala Phe Ser Ala Gly Glu Gly
       210                 215                 220
   ccc aag ccc acg ccc cgc gct gtc act aag ctt gtg ggc tct gtt ctt       720
   Pro Lys Pro Thr Pro Arg Ala Val Thr Lys Leu Val Gly Ser Val Leu
   225                 230                 235                 240
   ggc gct cgc gag gtg cgc agc tct att gct gcg att gaa gcg ctc ggc      768
   Gly Ala Arg Glu Val Arg Ser Ser Ile Ala Ala Ile Glu Ala Leu Gly
                   245                 250                 255
   ggc aag gcc atc tac tcg tcg tgc gac gtg aac tct gcc gcc gac gtg      816
   Gly Lys Ala Ile Tyr Ser Ser Cys Asp Val Asn Ser Ala Ala Asp Val
               260                 265                 270
   gcc aag gcc gtg cgc gat gcc gag tcc cag ctc ggt gcc cgc gtc tcg      864
   Ala Lys Ala Val Arg Asp Ala Glu Ser Gln Leu Gly Ala Arg Val Ser
           275                 280                 285
   ggc atc gtt cat gcc tcg ggc gtg ctc cgc gac cgt ctc atc gag aag      912
   Gly Ile Val His Ala Ser Gly Val Leu Arg Asp Arg Leu Ile Glu Lys
       290                 295                 300
   aag ctc ccc gac gag ttc gac gcc gtc ttt ggc acc aag gtc acc ggt      960
   Lys Leu Pro Asp Glu Phe Asp Ala Val Phe Gly Thr Lys Val Thr Gly
   305                 310                 315                 320
   ctc gag aac ctc ctc gcc gcc gtc gac cgc gcc aac ctc aag cac atg     1008
   Leu Glu Asn Leu Leu Ala Ala Val Asp Arg Ala Asn Leu Lys His Met
                   325                 330                 335
   gtc ctc ttc agc tcg ctc gcc ggc ttc cac ggc aac gtc ggc cag tct     1056
   Val Leu Phe Ser Ser Leu Ala Gly Phe His Gly Asn Val Gly Gln Ser
               340                 345                 350
   gac tac gcc atg gcc aac gag gcc ctt aac aag atg ggc ctc gag ctc     1104
   Asp Tyr Ala Met Ala Asn Glu Ala Leu Asn Lys Met Gly Leu Glu Leu
           355                 360                 365
   gcc aag gac gtc tcg gtc aag tcg atc tgc ttc ggt ccc tgg gac ggt     1152
   Ala Lys Asp Val Ser Val Lys Ser Ile Cys Phe Gly Pro Trp Asp Gly
       370                 375                 380
   ggc atg gtg acg ccg cag ctc aag aag cag ttc cag gag atg ggc gtg     1200
   Gly Met Val Thr Pro Gln Leu Lys Lys Gln Phe Gln Glu Met Gly Val
   385                 390                 395                 400
   cag atc atc ccc cgc gag ggc ggc gct gat acc gtg gcg cgc atc gtg     1248
   Gln Ile Ile Pro Arg Glu Gly Gly Ala Asp Thr Val Ala Arg Ile Val
                   405                 410                 415
   ctc ggc tcc tcg ccg gct gag atc ctt gtc ggc aac tgg cgc acc ccg     1296
   Leu Gly Ser Ser Pro Ala Glu Ile Leu Val Gly Asn Trp Arg Thr Pro
               420                 425                 430
   tcc aag aag gtc ggc tcg gac acc atc acc ctg cac cgc aag att tcc     1344
   Ser Lys Lys Val Gly Ser Asp Thr Ile Thr Leu His Arg Lys Ile Ser
           435                 440                 445
   gcc aag tcc aac ccc ttc ctc gag gac cac gtc atc cag ggc cgc cgc     1392
   Ala Lys Ser Asn Pro Phe Leu Glu Asp His Val Ile Gln Gly Arg Arg
       450                 455                 460
   gtg ctg ccc atg acg ctg gcc att ggc tcg ctc gcg gag acc tgc ctc     1440
   Val Leu Pro Met Thr Leu Ala Ile Gly Ser Leu Ala Glu Thr Cys Leu
   465                 470                 475                 480
   ggc ctc ttc ccc ggc tac tcg ctc tgg gcc att gac gac gcc cag ctc     1488
   Gly Leu Phe Pro Gly Tyr Ser Leu Trp Ala Ile Asp Asp Ala Gln Leu
                   485                 490                 495
   ttc aag ggt gtc act gtc gac ggc gac gtc aac tgc gag gtg acc ctc     1536
   Phe Lys Gly Val Thr Val Asp Gly Asp Val Asn Cys Glu Val Thr Leu
               500                 505                 510
   acc ccg tcg acg gcg ccc tcg ggc cgc gtc aac gtc cag gcc acg ctc     1584
   Thr Pro Ser Thr Ala Pro Ser Gly Arg Val Asn Val Gln Ala Thr Leu
           515                 520                 525
   aag acc ttt tcc agc ggc aag ctg gtc ccg gcc tac cgc gcc gtc atc      1632
   Lys Thr Phe Ser Ser Gly Lys Leu Val Pro Ala Tyr Arg Ala Val Ile
       530                 535                 540
   gtg ctc tcc aac cag ggc gcg ccc ccg gcc aac gcc acc atg cag ccg      1680
   Val Leu Ser Asn Gln Gly Ala Pro Pro Ala Asn Ala Thr Met Gln Pro
   545                 550                 555                 560
   ccc tcg ctc gat gcc gat ccg gcg ctc cag ggc tcc gtc tac gac ggc      1728
   Pro Ser Leu Asp Ala Asp Pro Ala Leu Gln Gly Ser Val Tyr Asp Gly
                   565                 570                 575
   aag acc ctc ttc cac ggc ccg gcc ttc cgc ggc atc gat gac gtg ctc      1776
   Lys Thr Leu Phe His Gly Pro Ala Phe Arg Gly Ile Asp Asp Val Leu
               580                 585                 590
   tcg tgc acc aag agc cag ctt gtg gcc aag tgc agc gct gtc ccc ggc      1824
   Ser Cys Thr Lys Ser Gln Leu Val Ala Lys Cys Ser Ala Val Pro Gly
           595                 600                 605
   tcc gac gcc gct cgc ggc gag ttt gcc acg gac act gac gcc cat gac      1872
   Ser Asp Ala Ala Arg Gly Glu Phe Ala Thr Asp Thr Asp Ala His Asp
       610                 615                 620
   ccc ttc gtg aac gac ctg gcc ttt cag gcc atg ctc gtc tgg gtg cgc      1920
   Pro Phe Val Asn Asp Leu Ala Phe Gln Ala Met Leu Val Trp Val Arg
   625                 630                 635                 640
   cgc acg ctc ggc cag gct gcg ctc ccc aac tcg atc cag cgc atc gtc      1968
   Arg Thr Leu Gly Gln Ala Ala Leu Pro Asn Ser Ile Gln Arg Ile Val
                   645                 650                 655
   cag cac cgc ccg gtc ccg cag gac aag ccc ttc tac att acc ctc cgc      2016
   Gln His Arg Pro Val Pro Gln Asp Lys Pro Phe Tyr Ile Thr Leu Arg
               660                 665                 670
   tcc aac cag tcg ggc ggt cac tcc cag cac aag cac gcc ctt cag ttc      2064
   Ser Asn Gln Ser Gly Gly His Ser Gln His Lys His Ala Leu Gln Phe
           675                 680                 685
   cac aac gag cag ggc gat ctc ttc att gat gtc cag gct tcg gtc atc      2112
   His Asn Glu Gln Gly Asp Leu Phe Ile Asp Val Gln Ala Ser Val Ile
       690                 695                 700
   gcc acg gac agc ctt gcc ttc                                          2133
   Ala Thr Asp Ser Leu Ala Phe
   705                 710
<210> 18
   <211> 711
   <212> PRT
   <213> Schizochytrium sp.
<400> 18
   Phe Gly Ala Leu Gly Gly Phe Ile Ser Gln Gln Ala Glu Arg Phe Glu
   1               5                   10                  15
   Pro Ala Glu Ile Leu Gly Phe Thr Leu Met Cys Ala Lys Phe Ala Lys
               20                  25                  30
   Ala Ser Leu Cys Thr Ala Val Ala Gly Gly Arg Pro Ala Phe Ile Gly
           35                  40                  45
   Val Ala Arg Leu Asp Gly Arg Leu Gly Phe Thr Ser Gln Gly Thr Ser
       50                  55                  60
   Asp Ala Leu Lys Arg Ala Gln Arg Gly Ala Ile Phe Gly Leu Cys Lys
   65 70 75 80
   Thr Ile Gly Leu Glu Trp Ser Glu Ser Asp Val Phe Ser Arg Gly Val
   85 90 95
   Asp Ile Ala Gln Gly Met His Pro Glu Asp Ala Ala Val Ala Ile Val
   100 105 110
   Arg Glu Met Ala Cys Ala Asp Ile Arg Ile Arg Glu Val Gly Ile Gly
   115 120 125
   Ala Asn Gln Gln Arg Cys Thr Ile Arg Ala Ala Lys Leu Glu Thr Gly
   130 135 140
   Asn Pro Gln Arg Gln Ile Ala Lys Asp Asp Val Leu Leu Val Ser Gly
   145 150 155 160
   Gly Ala Arg Gly Ile Thr Pro Leu Cys Ile Arg Glu Ile Thr Arg Gln
   165 170 175
   Ile Ala Gly Gly Lys Tyr Ile Leu Leu Gly Arg Ser Lys Val Ser Ala
   180 185 190
   Ser Glu Pro Ala Trp Cys Ala Gly Ile Thr Asp Glu Lys Ala Val Gln
   195 200 205
   Lys Ala Ala Thr Gln Glu Leu Lys Arg Ala Phe Ser Ala Gly Glu Gly
   210 215 220
   Pro Lys Pro Thr Pro Arg Ala Val Thr Lys Leu Val Gly Ser Val Leu
   225 230 235 240
   Gly Ala Arg Glu Val Arg Ser Ser Ile Ala Ala Ile Glu Ala Leu Gly
   245 250 255
   Gly Lys Ala Ile Tyr Ser Ser Cys Asp Val Asn Ser Ala Ala Asp Val
   260 265 270
   Ala Lys Ala Val Arg Asp Ala Glu Ser Gln Leu Gly Ala Arg Val Ser
   275 280 285
   Gly Ile Val His Ala Ser Gly Val Leu Arg Asp Arg Leu Ile Glu Lys
   290 295 300
   Lys Leu Pro Asp Glu Phe Asp Ala Val Phe Gly Thr Lys Val Thr Gly
   305 310 315 320
   Leu Glu Asn Leu Leu Ala Ala Val Asp Arg Ala Asn Leu Lys His Met
   325 330 335
   Val Leu Phe Ser Ser Leu Ala Gly Phe His Gly Asn Val Gly Gln Ser
   340 345 350
   Asp Tyr Ala Met Ala Asn Glu Ala Leu Asn Lys Met Gly Leu Glu Leu
   355 360 365
   Ala Lys Asp Val Ser Val Lys Ser Ile Cys Phe Gly Pro Trp Asp Gly
   370 375 380
   Gly Met Val Thr Pro Gln Leu Lys Lys Gln Phe Gln Glu Met Gly Val
   385 390 395 400
   Gln Ile Ile Pro Arg Glu Gly Gly Ala Asp Thr Val Ala Arg Ile Val
   405 410 415
   Leu Gly Ser Ser Pro Ala Glu Ile Leu Val Gly Asn Trp Arg Thr Pro
   420 425 430
   Ser Lys Lys Val Gly Ser Asp Thr Ile Thr Leu His Arg Lys Ile Ser
   435 440 445
   Ala Lys Ser Asn Pro Phe Leu Glu Asp His Val Ile Gln Gly Arg Arg
   450 455 460
   Val Leu Pro Met Thr Leu Ala Ile Gly Ser Leu Ala Glu Thr Cys Leu
   465 470 475 480
   Gly Leu Phe Pro Gly Tyr Ser Leu Trp Ala Ile Asp Asp Ala Gln Leu
   485 490 495
   Phe Lys Gly Val Thr Val Asp Gly Asp Val Asn Cys Glu Val Thr Leu
   500 505 510
   Thr Pro Ser Thr Ala Pro Ser Gly Arg Val Asn Val Gln Ala Thr Leu
   515 520 525
   Lys Thr Phe Ser Ser Gly Lys Leu Val Pro Ala Tyr Arg Ala Val Ile
   530 535 540
   Val Leu Ser Asn Gln Gly Ala Pro Pro Ala Asn Ala Thr Met Gln Pro
   545 550 555 560
   Pro Ser Leu Asp Ala Asp Pro Ala Leu Gln Gly Ser Val Tyr Asp Gly
   565 570 575
   Lys Thr Leu Phe His Gly Pro Ala Phe Arg Gly Ile Asp Asp Val Leu
   580 585 590
   Ser Cys Thr Lys Ser Gln Leu Val Ala Lys Cys Ser Ala Val Pro Gly
   595 600 605
   Ser Asp Ala Ala Arg Gly Glu Phe Ala Thr Asp Thr Asp Ala His Asp
   610 615 620
   Pro Phe Val Asn Asp Leu Ala Phe Gln Ala Met Leu Val Trp Val Arg
   625 630 635 640
   Arg Thr Leu Gly Gln Ala Ala Leu Pro Asn Ser Ile Gln Arg Ile Val
   645 650 655
   Gln His Arg Pro Val Pro Gln Asp Lys Pro Phe Tyr Ile Thr Leu Arg
   660 665 670
   Ser Asn Gln Ser Gly Gly His Ser Gln His Lys His Ala Leu Gln Phe
   675 680 685
   His Asn Glu Gln Gly Asp Leu Phe Ile Asp Val Gln Ala Ser Val Ile
   690 695 700
   Ala Thr Asp Ser Leu Ala Phe
   705 710
<210> 19
   <211> 1350
   <212> DNA
   <213> Schizochytrium sp.
<220>
   <221> CDS
   <222> (1)..(1350)
<400> 19
   atg gcc gct cgg aat gtg agc gcc gcg cat gag atg cac gat gaa aag      48
   Met Ala Ala Arg Asn Val Ser Ala Ala His Glu Met His Asp Glu Lys
   1               5                   10                  15
   cgc atc gcc gtc gtc ggc atg gcc gtc cag tac gcc gga tgc aaa acc      96
   Arg Ile Ala Val Val Gly Met Ala Val Gln Tyr Ala Gly Cys Lys Thr
               20                  25                  30
   aag gac gag ttc tgg gag gtg ctc atg aac ggc aag gtc gag tcc aag     144
   Lys Asp Glu Phe Trp Glu Val Leu Met Asn Gly Lys Val Glu Ser Lys
           35                  40                  45
   gtg atc agc gac aaa cga ctc ggc tcc aac tac cgc gcc gag cac tac     192
   Val Ile Ser Asp Lys Arg Leu Gly Ser Asn Tyr Arg Ala Glu His Tyr
       50                  55                  60
   aaa gca gag cgc agc aag tat gcc gac acc ttt tgc aac gaa acg tac     240
   Lys Ala Glu Arg Ser Lys Tyr Ala Asp Thr Phe Cys Asn Glu Thr Tyr
   65                  70                  75                  80
   ggc acc ctt gac gag aac gag atc gac aac gag cac gaa ctc ctc ctc     288
   Gly Thr Leu Asp Glu Asn Glu Ile Asp Asn Glu His Glu Leu Leu Leu
                   85                  90                  95
   aac ctc gcc aag cag gca ctc gca gag aca tcc gtc aaa gac tcg aca     336
   Asn Leu Ala Lys Gln Ala Leu Ala Glu Thr Ser Val Lys Asp Ser Thr
               100                 105                 110
   cgc tgc ggc atc gtc agc ggc tgc ctc tcg ttc ccc atg gac aac ctc     384
   Arg Cys Gly Ile Val Ser Gly Cys Leu Ser Phe Pro Met Asp Asn Leu
           115                 120                 125
   cag ggt gaa ctc ctc aac gtg tac caa aac cat gtc gag aaa aag ctc     432
   Gln Gly Glu Leu Leu Asn Val Tyr Gln Asn His Val Glu Lys Lys Leu
       130                 135                 140
   ggg gcc cgc gtc ttc aag gac gcc tcc cat tgg tcc gaa cgc gag cag     480
   Gly Ala Arg Val Phe Lys Asp Ala Ser His Trp Ser Glu Arg Glu Gln
   145                 150                 155                 160
   tcc aac aaa ccc gag gcc ggt gac cgc cgc atc ttc atg gac ccg gcc      528
   Ser Asn Lys Pro Glu Ala Gly Asp Arg Arg Ile Phe Met Asp Pro Ala
                   165                 170                 175
   tcc ttc gtc gcc gaa gaa ctc aac ctc ggc gcc ctt cac tac tcc gtc      576
   Ser Phe Val Ala Glu Glu Leu Asn Leu Gly Ala Leu His Tyr Ser Val
               180                 185                 190
   gac gca gca tgc gcc acg gcg ctc tac gtg ctc cgc ctc gcg cag gat      624
   Asp Ala Ala Cys Ala Thr Ala Leu Tyr Val Leu Arg Leu Ala Gln Asp
           195                 200                 205
   cat ctc gtc tcc ggc gcc gcc gac gtc atg ctc tgc ggt gcc acc tgc      672
   His Leu Val Ser Gly Ala Ala Asp Val Met Leu Cys Gly Ala Thr Cys
       210                 215                 220
   ctg ccg gag ccc ttt ttc atc ctt tcg ggc ttt tcc acc ttc cag gcc      720
   Leu Pro Glu Pro Phe Phe Ile Leu Ser Gly Phe Ser Thr Phe Gln Ala
   225                 230                 235                 240
   atg ccc gtc ggc acg ggc cag aac gtg tcc atg ccg ctg cac aag gac      768
   Met Pro Val Gly Thr Gly Gln Asn Val Ser Met Pro Leu His Lys Asp
                   245                 250                 255
   agc cag ggc ctc acc ccg ggt gag ggc ggc tcc atc atg gtc ctc aag      816
   Ser Gln Gly Leu Thr Pro Gly Glu Gly Gly Ser Ile Met Val Leu Lys
               260                 265                 270
   cgt ctc gat gat gcc atc cgc gac ggc gac cac att tac ggc acc ctt      864
   Arg Leu Asp Asp Ala Ile Arg Asp Gly Asp His Ile Tyr Gly Thr Leu
           275                 280                 285
   ctc ggc gcc aat gtc agc aac tcc ggc aca ggt ctg ccc ctc aag ccc      912
   Leu Gly Ala Asn Val Ser Asn Ser Gly Thr Gly Leu Pro Leu Lys Pro
       290                 295                 300
   ctt ctc ccc agc gag aaa aag tgc ctc atg gac acc tac acg cgc att      960
   Leu Leu Pro Ser Glu Lys Lys Cys Leu Met Asp Thr Tyr Thr Arg Ile
   305                 310                 315                 320
   aac gtg cac ccg cac aag att cag tac gtc gag tgc cac gcc acc ggc     1008
   Asn Val His Pro His Lys Ile Gln Tyr Val Glu Cys His Ala Thr Gly
                   325                 330                 335
   acg ccc cag ggt gat cgt gtg gaa atc gac gcc gtc aag gcc tgc ttt     1056
   Thr Pro Gln Gly Asp Arg Val Glu Ile Asp Ala Val Lys Ala Cys Phe
               340                 345                 350
   gaa ggc aag gtc ccc cgt ttc ggt acc aca aag ggc aac ttt gga cac     1104
   Glu Gly Lys Val Pro Arg Phe Gly Thr Thr Lys Gly Asn Phe Gly His
           355                 360                 365
   acc cts gyc gca gcc ggc ttt gcc ggt atg tgc aag gtc ctc ctc tcc     1152
   Thr Xaa Xaa Ala Ala Gly Phe Ala Gly Met Cys Lys Val Leu Leu Ser
       370                 375                 380
   atg aag cat ggc atc atc ccg ccc acc ccg ggt atc gat gac gag acc     1200
   Met Lys His Gly Ile Ile Pro Pro Thr Pro Gly Ile Asp Asp Glu Thr
   385                 390                 395                 400
   aag atg gac cct ctc gtc gtc tcc ggt gag gcc atc cca tgg cca gag     1248
   Lys Met Asp Pro Leu Val Val Ser Gly Glu Ala Ile Pro Trp Pro Glu
                   405                 410                 415
   acc aac ggc gag ccc aag cgc gcc ggt ctc tcg gcc ttt ggc ttt ggt     1296
   Thr Asn Gly Glu Pro Lys Arg Ala Gly Leu Ser Ala Phe Gly Phe Gly
               420                 425                 430
   ggc acc aac gcc cat gcc gtc ttt gag gag cat gac ccc tcc aac gcc     1344
   Gly Thr Asn Ala His Ala Val Phe Glu Glu His Asp Pro Ser Asn Ala
           435                 440                 445
   gcc tgc                                                                   1350
   Ala Cys
       450
<210> 20
   <211> 450
   <212> PRT
   <213> Schizochytrium sp.
<220>
   <221> misc_feature
   <222> (370)..(370)
   <223> The 'Xaa' at location 370 stands for Leu.
<220>
   <221> misc feature
   <222> (371)..(371)
   <223> The 'Xaa' at location 371 stands for Ala, or Val.
<400> 20
   Met Ala Ala Arg Asn Val Ser Ala Ala His Glu Met His Asp Glu Lys
   1               5                   10                  15
   Arg Ile Ala Val Val Gly Met Ala Val Gln Tyr Ala Gly Cys Lys Thr
               20                  25                  30
   Lys Asp Glu Phe Trp Glu Val Leu Met Asn Gly Lys Val Glu Ser Lys
           35                  40                  45
   Val Ile Ser Asp Lys Arg Leu Gly Ser Asn Tyr Arg Ala Glu His Tyr
       50                  55                  60
   Lys Ala Glu Arg Ser Lys Tyr Ala Asp Thr Phe Cys Asn Glu Thr Tyr
   65                  70                  75                  80
   Gly Thr Leu Asp Glu Asn Glu Ile Asp Asn Glu His Glu Leu Leu Leu
                   85                  90                  95
   Asn Leu Ala Lys Gln Ala Leu Ala Glu Thr Ser Val Lys Asp Ser Thr
               100                 105                 110
   Arg Cys Gly Ile Val Ser Gly Cys Leu Ser Phe Pro Met Asp Asn Leu
           115                 120                 125
   Gln Gly Glu Leu Leu Asn Val Tyr Gln Asn His Val Glu Lys Lys Leu
       130                 135                 140
   Gly Ala Arg Val Phe Lys Asp Ala Ser His Trp Ser Glu Arg Glu Gln
   145                 150                 155                 160
   Ser Asn Lys Pro Glu Ala Gly Asp Arg Arg Ile Phe Met Asp Pro Ala
                   165                 170                 175
   Ser Phe Val Ala Glu Glu Leu Asn Leu Gly Ala Leu His Tyr Ser Val
               180                 185                 190
   Asp Ala Ala Cys Ala Thr Ala Leu Tyr Val Leu Arg Leu Ala Gln Asp
   195 200 205
   His Leu Val Ser Gly Ala Ala Asp Val Met Leu Cys Gly Ala Thr Cys
   210 215 220
   Leu Pro Glu Pro Phe Phe Ile Leu Ser Gly Phe Ser Thr Phe Gln Ala
   225 230 235 240
   Met Pro Val Gly Thr Gly Gln Asn Val Ser Met Pro Leu His Lys Asp
   245 250 255
   Ser Gln Gly Leu Thr Pro Gly Glu Gly Gly Ser Ile Met Val Leu Lys
   260 265 270
   Arg Leu Asp Asp Ala Ile Arg Asp Gly Asp His Ile Tyr Gly Thr Leu
   275 280 285
   Leu Gly Ala Asn Val Ser Asn Ser Gly Thr Gly Leu Pro Leu Lys Pro
   290 295 300
   Leu Leu Pro Ser Glu Lys Lys Cys Leu Met Asp Thr Tyr Thr Arg Ile
   305 310 315 320
   Asn Val His Pro His Lys Ile Gln Tyr Val Glu Cys His Ala Thr Gly
   325 330 335
   Thr Pro Gln Gly Asp Arg Val Glu Ile Asp Ala Val Lys Ala Cys Phe
   340 345 350
   Glu Gly Lys Val Pro Arg Phe Gly Thr Thr Lys Gly Asn Phe Gly His
   355 360 365
   Thr Xaa Xaa Ala Ala Gly Phe Ala Gly Met Cys Lys Val Leu Leu Ser
   370 375 380
   Met Lys His Gly Ile Ile Pro Pro Thr Pro Gly Ile Asp Asp Glu Thr
   385 390 395 400
   Lys Met Asp Pro Leu Val Val Ser Gly Glu Ala Ile Pro Trp Pro Glu
   405 410 415
   Thr Asn Gly Glu Pro Lys Arg Ala Gly Leu Ser Ala Phe Gly Phe Gly
   420 425 430
   Gly Thr Asn Ala His Ala Val Phe Glu Glu His Asp Pro Ser Asn Ala
   435 440 445
   Ala Cys
   450
<210> 21
   <211> 1323
   <212> DNA
   <213> Schizochytrium sp.
<220>
   <221> CDS
   <222> (1)..(1323)
<400> 21
   tcg gcc cgc tgc ggc ggt gaa agc aac atg cgc atc gcc atc act ggt      48
   Ser Ala Arg Cys Gly Gly Glu Ser Asn Met Arg Ile Ala Ile Thr Gly
   1               5                   10                  15
   atg gac gcc acc ttt ggc gct ctc aag gga ctc gac gcc ttc gag cgc      96
   Met Asp Ala Thr Phe Gly Ala Leu Lys Gly Leu Asp Ala Phe Glu Arg
               20                  25                  30
   gcc att tac acc ggc gct cac ggt gcc atc cca ctc cca gaa aag cgc     144
   Ala Ile Tyr Thr Gly Ala His Gly Ala Ile Pro Leu Pro Glu Lys Arg
           35                  40                  45
   tgg cgc ttt ctc ggc aag gac aag gac ttt ctt gac ctc tgc ggc gtc     192
   Trp Arg Phe Leu Gly Lys Asp Lys Asp Phe Leu Asp Leu Cys Gly Val
       50                  55                  60
   aag gcc acc ccg cac ggc tgc tac att gaa gat gtt gag gtc gac ttc     240
   Lys Ala Thr Pro His Gly Cys Tyr Ile Glu Asp Val Glu Val Asp Phe
   65                  70                  75                  80
   cag cgc ctc cgc acg ccc atg acc cct gaa gac atg ctc ctc cct cag     288
   Gln Arg Leu Arg Thr Pro Met Thr Pro Glu Asp Met Leu Leu Pro Gln
                   85                  90                  95
   cag ctt ctg gcc gtc acc acc att gac cgc gcc atc ctc gac tcg gga     336
   Gln Leu Leu Ala Val Thr Thr Ile Asp Arg Ala Ile Leu Asp Ser Gly
               100                 105                 110
   atg aaa aag ggt ggc aat gtc gcc gtc ttt gtc ggc ctc ggc acc gac     384
   Met Lys Lys Gly Gly Asn Val Ala Val Phe Val Gly Leu Gly Thr Asp
           115                 120                 125
   ctc gag ctc tac cgt cac cgt gct cgc gtc gct ctc aag gag cgc gtc     432
   Leu Glu Leu Tyr Arg His Arg Ala Arg Val Ala Leu Lys Glu Arg Val
       130                 135                 140
   cgc cct gaa gcc tcc aag aag ctc aat gac atg atg cag tac att aac     480
   Arg Pro Glu Ala Ser Lys Lys Leu Asn Asp Met Met Gln Tyr Ile Asn
   145                 150                 155                 160
   gac tgc ggc aca tcc aca tcg tac acc tcg tac att ggc aac ctc gtc     528
   Asp Cys Gly Thr Ser Thr Ser Tyr Thr Ser Tyr Ile Gly Asn Leu Val
                   165                 170                 175
   gcc acg cgc gtc tcg tcg cag tgg ggc ttc acg ggc ccc tcc ttt acg     576
   Ala Thr Arg Val Ser Ser Gln Trp Gly Phe Thr Gly Pro Ser Phe Thr
               180                 185                 190
   atc acc gag ggc aac aac tcc gtc tac cgc tgc gcc gag ctc ggc aag     624
   Ile Thr Glu Gly Asn Asn Ser Val Tyr Arg Cys Ala Glu Leu Gly Lys
           195                 200                 205
   tac ctc ctc gag acc ggc gag gtc gat ggc gtc gtc gtt gcg ggt gtc     672
   Tyr Leu Leu Glu Thr Gly Glu Val Asp Gly Val Val Val Ala Gly Val
       210                 215                 220
   gat ctc tgc ggc agt gcc gaa aac ctt tac gtc aag tct cgc cgc ttc     720
   Asp Leu Cys Gly Ser Ala Glu Asn Leu Tyr Val Lys Ser Arg Arg Phe
   225                 230                 235                 240
   aag gtg tcc acc tcc gat acc ccg cgc gcc agc ttt gac gcc gcc gcc     768
   Lys Val Ser Thr Ser Asp Thr Pro Arg Ala Ser Phe Asp Ala Ala Ala
                   245                 250                 255
   gat ggc tac ttt gtc ggc gag ggc tgc ggt gcc ttt gtg ctc aag cgt      816
   Asp Gly Tyr Phe Val Gly Glu Gly Cys Gly Ala Phe Val Leu Lys Arg
               260                 265                 270
   gag act agc tgc acc aag gac gac cgt atc tac gct tgc atg gat gcc      864
   Glu Thr Ser Cys Thr Lys Asp Asp Arg Ile Tyr Ala Cys Met Asp Ala
           275                 280                 285
   atc gtc cct ggc aac gtc cct agc gcc tgc ttg cgc gag gcc ctc gac      912
   Ile Val Pro Gly Asn Val Pro Ser Ala Cys Leu Arg Glu Ala Leu Asp
       290                 295                 300
   cag gcg cgc gtc aag ccg ggc gat atc gag atg ctc gag ctc agc gcc      960
   Gln Ala Arg Val Lys Pro Gly Asp Ile Glu Met Leu Glu Leu Ser Ala
   305                 310                 315                 320
   gac tcc gcc cgc cac ctc aag gac ccg tcc gtc ctg ccc aag gag ctc     1008
   Asp Ser Ala Arg His Leu Lys Asp Pro Ser Val Leu Pro Lys Glu Leu
                   325                 330                 335
   act gcc gag gag gaa atc ggc ggc ctt cag acg atc ctt cgt gac gat     1056
   Thr Ala Glu Glu Glu Ile Gly Gly Leu Gln Thr Ile Leu Arg Asp Asp
               340                 345                 350
   gac aag ctc ccg cgc aac gtc gca acg ggc agt gtc aag gcc acc gtc     1104
   Asp Lys Leu Pro Arg Asn Val Ala Thr Gly Ser Val Lys Ala Thr Val
           355                 360                 365
   ggt gac acc ggt tat gcc tct ggt gct gcc agc ctc atc aag gct gcg     1152
   Gly Asp Thr Gly Tyr Ala Ser Gly Ala Ala Ser Leu Ile Lys Ala Ala
       370                 375                 380
   ctt tgc atc tac aac cgc tac ctg ccc agc aac ggc gac gac tgg gat     1200
   Leu Cys Ile Tyr Asn Arg Tyr Leu Pro Ser Asn Gly Asp Asp Trp Asp
   385                 390                 395                 400
   gaa ccc gcc cct gag gcg ccc tgg gac agc acc ctc ttt gcg tgc cag     1248
   Glu Pro Ala Pro Glu Ala Pro Trp Asp Ser Thr Leu Phe Ala Cys Gln
                   405                 410                 415
   acc tcg cgc gct tgg ctc aag aac cct ggc gag cgt cgc tat gcg gcc     1296
   Thr Ser Arg Ala Trp Leu Lys Asn Pro Gly Glu Arg Arg Tyr Ala Ala
               420                 425                 430
   gtc tcg ggc gtc tcc gag acg cgc tcg                                 1323
   Val Ser Gly Val Ser Glu Thr Arg Ser
           435                 440
<210> 22
   <211> 441
   <212> PRT
   <213> Schizochytrium sp.
<400> 22
   Ser Ala Arg Cys Gly Gly Glu Ser Asn Met Arg Ile Ala Ile Thr Gly
   1               5                   10                  15
   Met Asp Ala Thr Phe Gly Ala Leu Lys Gly Leu Asp Ala Phe Glu Arg
               20                  25                  30
   Ala Ile Tyr Thr Gly Ala His Gly Ala Ile Pro Leu Pro Glu Lys Arg
           35                  40                  45
   Trp Arg Phe Leu Gly Lys Asp Lys Asp Phe Leu Asp Leu Cys Gly Val
       50                  55                  60
   Lys Ala Thr Pro His Gly Cys Tyr Ile Glu Asp Val Glu Val Asp Phe
   65                  70                  75                  80
   Gln Arg Leu Arg Thr Pro Met Thr Pro Glu Asp Met Leu Leu Pro Gln
                   85                  90                  95
   Gln Leu Leu Ala Val Thr Thr Ile Asp Arg Ala Ile Leu Asp Ser Gly
               100                 105                 110
   Met Lys Lys Gly Gly Asn Val Ala Val Phe Val Gly Leu Gly Thr Asp
           115                 120                 125
   Leu Glu Leu Tyr Arg His Arg Ala Arg Val Ala Leu Lys Glu Arg Val
       130                 135                 140
   Arg Pro Glu Ala Ser Lys Lys Leu Asn Asp Met Met Gln Tyr Ile Asn
   145                 150                 155                 160
   Asp Cys Gly Thr Ser Thr Ser Tyr Thr Ser Tyr Ile Gly Asn Leu Val
                   165                 170                 175
   Ala Thr Arg Val Ser Ser Gln Trp Gly Phe Thr Gly Pro Ser Phe Thr
               180                 185                 190
   Ile Thr Glu Gly Asn Asn Ser Val Tyr Arg Cys Ala Glu Leu Gly Lys
           195                 200                 205
   Tyr Leu Leu Glu Thr Gly Glu Val Asp Gly Val Val Val Ala Gly Val
       210                 215                 220
   Asp Leu Cys Gly Ser Ala Glu Asn Leu Tyr Val Lys Ser Arg Arg Phe
   225                 230                 235                 240
   Lys Val Ser Thr Ser Asp Thr Pro Arg Ala Ser Phe Asp Ala Ala Ala
                   245                 250                 255
   Asp Gly Tyr Phe Val Gly Glu Gly Cys Gly Ala Phe Val Leu Lys Arg
               260                 265                 270
   Glu Thr Ser Cys Thr Lys Asp Asp Arg Ile Tyr Ala Cys Met Asp Ala
           275                 280                 285
   Ile Val Pro Gly Asn Val Pro Ser Ala Cys Leu Arg Glu Ala Leu Asp
       290                 295                 300
   Gln Ala Arg Val Lys Pro Gly Asp Ile Glu Met Leu Glu Leu Ser Ala
   305                 310                 315                 320
   Asp Ser Ala Arg His Leu Lys Asp Pro Ser Val Leu Pro Lys Glu Leu
                   325                 330                 335
   Thr Ala Glu Glu Glu Ile Gly Gly Leu Gln Thr Ile Leu Arg Asp Asp
               340                 345                 350
   Asp Lys Leu Pro Arg Asn Val Ala Thr Gly Ser Val Lys Ala Thr Val
   355 360 365
   Gly Asp Thr Gly Tyr Ala Ser Gly Ala Ala Ser Leu Ile Lys Ala Ala
   370 375 380
   Leu Cys Ile Tyr Asn Arg Tyr Leu Pro Ser Asn Gly Asp Asp Trp Asp
   385 390 395 400
   Glu Pro Ala Pro Glu Ala Pro Trp Asp Ser Thr Leu Phe Ala Cys Gln
   405 410 415
   Thr Ser Arg Ala Trp Leu Lys Asn Pro Gly Glu Arg Arg Tyr Ala Ala
   420 425 430
   Val Ser Gly Val Ser Glu Thr Arg Ser
   435 440
<210> 23
   <211> 1500
   <212> DNA
   <213> Schizochytrium sp.
<220>
   <221> CDS
   <222> (1)..(1500)
<400> 23
   tgc tat tcc gtg ctc ctc tcc gaa gcc gag ggc cac tac gag cgc gag      48
   Cys Tyr Ser Val Leu Leu Ser Glu Ala Glu Gly His Tyr Glu Arg Glu
   1               5                   10                  15
   aac cgc atc tcg ctc gac gag gag gcg ccc aag ctc att gtg ctt cgc      96
   Asn Arg Ile Ser Leu Asp Glu Glu Ala Pro Lys Leu Ile Val Leu Arg
               20                  25                  30
   gcc gac tcc cac gag gag atc ctt ggt cgc ctc gac aag atc cgc gag     144
   Ala Asp Ser His Glu Glu Ile Leu Gly Arg Leu Asp Lys Ile Arg Glu
           35                  40                  45
   cgc ttc ttg cag ccc acg ggc gcc gcc ccg cgc gag tcc gag ctc aag     192
   Arg Phe Leu Gln Pro Thr Gly Ala Ala Pro Arg Glu Ser Glu Leu Lys
       50                  55                  60
   gcg cag gcc cgc cgc atc ttc ctc gag ctc ctc ggc gag acc ctt gcc     240
   Ala Gln Ala Arg Arg Ile Phe Leu Glu Leu Leu Gly Glu Thr Leu Ala
   65                  70                  75                  80
   cag gat gcc gct tct tca ggc tcg caa aag ccc ctc gct ctc agc ctc     288
   Gln Asp Ala Ala Ser Ser Gly Ser Gln Lys Pro Leu Ala Leu Ser Leu
                   85                  90                  95
   gtc tcc acg ccc tcc aag ctc cag cgc gag gtc gag ctc gcg gcc aag     336
   Val Ser Thr Pro Ser Lys Leu Gln Arg Glu Val Glu Leu Ala Ala Lys
               100                 105                 110
   ggt atc ccg cgc tgc ctc aag atg cgc cgc gat tgg agc tcc cct gct     384
   Gly Ile Pro Arg Cys Leu Lys Met Arg Arg Asp Trp Ser Ser Pro Ala
           115                 120                 125
   ggc agc cgc tac gcg cct gag ccg ctc gcc agc gac cgc gtc gcc ttc     432
   Gly Ser Arg Tyr Ala Pro Glu Pro Leu Ala Ser Asp Arg Val Ala Phe
       130                 135                 140
   atg tac ggc gaa ggt cgc agc cct tac tac ggc atc acc caa gac att       480
   Met Tyr Gly Glu Gly Arg Ser Pro Tyr Tyr Gly Ile Thr Gln Asp Ile
   145                 150                 155                 160
   cac cgc att tgg ccc gaa ctc cac gag gtc atc aac gaa aag acg aac       528
   His Arg Ile Trp Pro Glu Leu His Glu Val Ile Asn Glu Lys Thr Asn
                   165                 170                 175
   cgt ctc tgg gcc gaa ggc gac cgc tgg gtc atg ccg cgc gcc agc ttc       576
   Arg Leu Trp Ala Glu Gly Asp Arg Trp Val Met Pro Arg Ala Ser Phe
               180                 185                 190
   aag tcg gag ctc gag agc cag cag caa gag ttt gat cgc aac atg att       624
   Lys Ser Glu Leu Glu Ser Gln Gln Gln Glu Phe Asp Arg Asn Met Ile
           195                 200                 205
   gaa atg ttc cgt ctt gga atc ctc acc tca att gcc ttc acc aat ctg       672
   Glu Met Phe Arg Leu Gly Ile Leu Thr Ser Ile Ala Phe Thr Asn Leu
       210                 215                 220
   gcg cgc gac gtt ctc aac atc acg ccc aag gcc gcc ttt ggc ctc agt       720
   Ala Arg Asp Val Leu Asn Ile Thr Pro Lys Ala Ala Phe Gly Leu Ser
   225                 230                 235                 240
   ctt ggc gag att tcc atg att ttt gcc ttt tcc aag aag aac ggt ctc       768
   Leu Gly Glu Ile Ser Met Ile Phe Ala Phe Ser Lys Lys Asn Gly Leu
                   245                 250                 255
   atc tcc gac cag ctc acc aag gat ctt cgc gag tcc gac gtg tgg aac       816
   Ile Ser Asp Gln Leu Thr Lys Asp Leu Arg Glu Ser Asp Val Trp Asn
               260                 265                 270
   aag gct ctg gcc gtt gaa ttt aat gcg ctg cgc gag gcc tgg ggc att       864
   Lys Ala Leu Ala Val Glu Phe Asn Ala Leu Arg Glu Ala Trp Gly Ile
           275                 280                 285
   cca cag agt gtc ccc aag gac gag ttc tgg caa ggc tac att gtg cgc       912
   Pro Gln Ser Val Pro Lys Asp Glu Phe Trp Gln Gly Tyr Ile Val Arg
       290                 295                 300
   ggc acc aag cag gat atc gag gcg gcc atc gcc ccg gac agc aag tac       960
   Gly Thr Lys Gln Asp Ile Glu Ala Ala Ile Ala Pro Asp Ser Lys Tyr
   305                 310                 315                 320
   gtg cgc ctc acc atc atc aat gat gcc aac acc gcc ctc att agc ggc      1008
   Val Arg Leu Thr Ile Ile Asn Asp Ala Asn Thr Ala Leu Ile Ser Gly
                   325                 330                 335
   aag ccc gac gcc tgc aag gct gcg atc gcg cgt ctc ggt ggc aac att      1056
   Lys Pro Asp Ala Cys Lys Ala Ala Ile Ala Arg Leu Gly Gly Asn Ile
               340                 345                 350
   cct gcg ctt ccc gtg acc cag ggc atg tgc ggc cac tgc ccc gag gtg      1104
   Pro Ala Leu Pro Val Thr Gln Gly Met Cys Gly His Cys Pro Glu Val
           355                 360                 365
   gga cct tat acc aag gat atc gcc aag atc cat gcc aac ctt gag ttc      1152
   Gly Pro Tyr Thr Lys Asp Ile Ala Lys Ile His Ala Asn Leu Glu Phe
       370                 375                 380
   ccc gtt gtc gac ggc ctt gac ctc tgg acc aca atc aac cag aag cgc      1200
   Pro Val Val Asp Gly Leu Asp Leu Trp Thr Thr Ile Asn Gln Lys Arg
   385                 390                 395                 400
   ctc gtg cca cgc gcc acg ggc gcc aag gac gaa tgg gcc cct tct tcc      1248
   Leu Val Pro Arg Ala Thr Gly Ala Lys Asp Glu Trp Ala Pro Ser Ser
                   405                 410                 415
   ttt ggc gag tac gcc ggc cag ctc tac gag aag cag gct aac ttc ccc      1296
   Phe Gly Glu Tyr Ala Gly Gln Leu Tyr Glu Lys Gln Ala Asn Phe Pro
               420                 425                 430
   caa atc gtc gag acc att tac aag caa aac tac gac gtc ttt gtc gag        1344
   Gln Ile Val Glu Thr Ile Tyr Lys Gln Asn Tyr Asp Val Phe Val Glu
           435                 440                 445
   gtt ggg ccc aac aac cac cgt agc acc gca gtg cgc acc acg ctt ggt        1392
   Val Gly Pro Asn Asn His Arg Ser Thr Ala Val Arg Thr Thr Leu Gly
       450                 455                 460
   ccc cag cgc aac cac ctt gct ggc gcc atc gac aag cag aac gag gat        1440
   Pro Gln Arg Asn His Leu Ala Gly Ala Ile Asp Lys Gln Asn Glu Asp
   465                 470                 475                 480
   gct tgg acg acc atc gtc aag ctt gtg gct tcg ctc aag gcc cac ctt        1488
   Ala Trp Thr Thr Ile Val Lys Leu Val Ala Ser Leu Lys Ala His Leu
                   485                 490                 495
   gtt cct ggc gtc                                                        1500
   Val Pro Gly Val
               500
<210> 24
   <211> 500
   <212> PRT
   <213> Schizochytrium sp.
<400> 24
   Cys Tyr Ser Val Leu Leu Ser Glu Ala Glu Gly His Tyr Glu Arg Glu
   1               5                   10                  15
   Asn Arg Ile Ser Leu Asp Glu Glu Ala Pro Lys Leu Ile Val Leu Arg
               20                  25                  30
   Ala Asp Ser His Glu Glu Ile Leu Gly Arg Leu Asp Lys Ile Arg Glu
           35                  40                  45
   Arg Phe Leu Gln Pro Thr Gly Ala Ala Pro Arg Glu Ser Glu Leu Lys
       50                  55                  60
   Ala Gln Ala Arg Arg Ile Phe Leu Glu Leu Leu Gly Glu Thr Leu Ala
   65                  70                  75                  80
   Gln Asp Ala Ala Ser Ser Gly Ser Gln Lys Pro Leu Ala Leu Ser Leu
                   85                  90                  95
   Val Ser Thr Pro Ser Lys Leu Gln Arg Glu Val Glu Leu Ala Ala Lys
               100                 105                 110
   Gly Ile Pro Arg Cys Leu Lys Met Arg Arg Asp Trp Ser Ser Pro Ala
           115                 120                 125
   Gly Ser Arg Tyr Ala Pro Glu Pro Leu Ala Ser Asp Arg Val Ala Phe
       130                 135                 140
   Met Tyr Gly Glu Gly Arg Ser Pro Tyr Tyr Gly Ile Thr Gln Asp Ile
   145                 150                 155                 160
   His Arg Ile Trp Pro Glu Leu His Glu Val Ile Asn Glu Lys Thr Asn
                   165                 170                 175
   Arg Leu Trp Ala Glu Gly Asp Arg Trp Val Met Pro Arg Ala Ser Phe
   180 185 190
   Lys Ser Glu Leu Glu Ser Gln Gln Gln Glu Phe Asp Arg Asn Met Ile
   195 200 205
   Glu Met Phe Arg Leu Gly Ile Leu Thr Ser Ile Ala Phe Thr Asn Leu
   210 215 220
   Ala Arg Asp Val Leu Asn Ile Thr Pro Lys Ala Ala Phe Gly Leu Ser
   225 230 235 240
   Leu Gly Glu Ile Ser Met Ile Phe Ala Phe Ser Lys Lys Asn Gly Leu
   245 250 255
   Ile Ser Asp Gln Leu Thr Lys Asp Leu Arg Glu Ser Asp Val Trp Asn
   260 265 270
   Lys Ala Leu Ala Val Glu Phe Asn Ala Leu Arg Glu Ala Trp Gly Ile
   275 280 285
   Pro Gln Ser Val Pro Lys Asp Glu Phe Trp Gln Gly Tyr Ile Val Arg
   290 295 300
   Gly Thr Lys Gln Asp Ile Glu Ala Ala Ile Ala Pro Asp Ser Lys Tyr
   305 310 315 320
   Val Arg Leu Thr Ile Ile Asn Asp Ala Asn Thr Ala Leu Ile Ser Gly
   325 330 335
   Lys Pro Asp Ala Cys Lys Ala Ala Ile Ala Arg Leu Gly Gly Asn Ile
   340 345 350
   Pro Ala Leu Pro Val Thr Gln Gly Met Cys Gly His Cys Pro Glu Val
   355 360 365
   Gly Pro Tyr Thr Lys Asp Ile Ala Lys Ile His Ala Asn Leu Glu Phe
   370 375 380
   Pro Val Val Asp Gly Leu Asp Leu Trp Thr Thr Ile Asn Gln Lys Arg
   385 390 395 400
   Leu Val Pro Arg Ala Thr Gly Ala Lys Asp Glu Trp Ala Pro Ser Ser
   405 410 415
   Phe Gly Glu Tyr Ala Gly Gln Leu Tyr Glu Lys Gln Ala Asn Phe Pro
   420 425 430
   Gln Ile Val Glu Thr Ile Tyr Lys Gln Asn Tyr Asp Val Phe Val Glu
   435 440 445
   Val Gly Pro Asn Asn His Arg Ser Thr Ala Val Arg Thr Thr Leu Gly
   450 455 460
   Pro Gln Arg Asn His Leu Ala Gly Ala Ile Asp Lys Gln Asn Glu Asp
   465 470 475 480
   Ala Trp Thr Thr Ile Val Lys Leu Val Ala Ser Leu Lys Ala His Leu
   485 490 495
   Val Pro Gly Val
   500
<210> 25
   <211> 1530
   <212> DNA
   <213> Schizochytrium sp.
<220>
   <221> CDS
   <222> (1)..(1530)
<400> 25
   ctg ctc gat ctc gac agt atg ctt gcg ctg agc tct gcc agt gcc tcc        48
   Leu Leu Asp Leu Asp Ser Met Leu Ala Leu Ser Ser Ala Ser Ala Ser
   1               5                   10                  15
   ggc aac ctt gtt gag act gcg cct agc gac gcc tcg gtc att gtg ccg        96
   Gly Asn Leu Val Glu Thr Ala Pro Ser Asp Ala Ser Val Ile Val Pro
               20                  25                  30
   ccc tgc aac att gcg gat ctc ggc agc cgc gcc ttc atg aaa acg tac       144
   Pro Cys Asn Ile Ala Asp Leu Gly Ser Arg Ala Phe Met Lys Thr Tyr
           35                  40                  45
   ggt gtt tcg gcg cct ctg tac acg ggc gcc atg gcc aag ggc att gcc       192
   Gly Val Ser Ala Pro Leu Tyr Thr Gly Ala Met Ala Lys Gly Ile Ala
       50                  55                  60
   tct gcg gac ctc gtc att gcc gcc ggc cgc cag ggc atc ctt gcg tcc       240
   Ser Ala Asp Leu Val Ile Ala Ala Gly Arg Gln Gly Ile Leu Ala Ser
   65                  70                  75                  80
   ttt ggc gcc ggc gga ctt ccc atg cag gtt gtg cgt gag tcc atc gaa       288
   Phe Gly Ala Gly Gly Leu Pro Met Gln Val Val Arg Glu Ser Ile Glu
                   85                  90                  95
   aag att cag gcc gcc ctg ccc aat ggc ccg tac gct gtc aac ctt atc       336
   Lys Ile Gln Ala Ala Leu Pro Asn Gly Pro Tyr Ala Val Asn Leu Ile
               100                 105                 110
   cat tct ccc ttt gac agc aac ctc gaa aag ggc aat gtc gat ctc ttc       384
   His Ser Pro Phe Asp Ser Asn Leu Glu Lys Gly Asn Val Asp Leu Phe
           115                 120                 125
   ctc gag aag ggt gtc acc ttt gtc gag gcc tcg gcc ttt atg acg ctc       432
   Leu Glu Lys Gly Val Thr Phe Val Glu Ala Ser Ala Phe Met Thr Leu
       130                 135                 140
   acc ccg cag gtc gtg cgg tac cgc gcg gct ggc ctc acg cgc aac gcc       480
   Thr Pro Gln Val Val Arg Tyr Arg Ala Ala Gly Leu Thr Arg Asn Ala
   145                 150                 155                 160
   gac ggc tcg gtc aac atc cgc aac cgt atc att ggc aag gtc tcg cgc       528
   Asp Gly Ser Val Asn Ile Arg Asn Arg Ile Ile Gly Lys Val Ser Arg
                   165                 170                 175
   acc gag ctc gcc gag atg ttc atg cgt cct gcg ccc gag cac ctt ctt       576
   Thr Glu Leu Ala Glu Met Phe Met Arg Pro Ala Pro Glu His Leu Leu
               180                 185                 190
   cag aag ctc att gct tcc ggc gag atc aac cag gag cag gcc gag ctc      624
   Gln Lys Leu Ile Ala Ser Gly Glu Ile Asn Gln Glu Gln Ala Glu Leu
           195                 200                 205
   gcc cgc cgt gtt ccc gtc gct gac gac atc gcg gtc gaa gct gac tcg      672
   Ala Arg Arg Val Pro Val Ala Asp Asp Ile Ala Val Glu Ala Asp Ser
       210                 215                 220
   ggt ggc cac acc gac aac cgc ccc atc cac gtc att ctg ccc ctc atc      720
   Gly Gly His Thr Asp Asn Arg Pro Ile His Val Ile Leu Pro Leu Ile
   225                 230                 235                 240
   atc aac ctt cgc gac cgc ctt cac cgc gag tgc ggc tac ccg gcc aac      768
   Ile Asn Leu Arg Asp Arg Leu His Arg Glu Cys Gly Tyr Pro Ala Asn
                   245                 250                 255
   ctt cgc gtc cgt gtg ggc gcc ggc ggt ggc att ggg tgc ccc cag gcg      816
   Leu Arg Val Arg Val Gly Ala Gly Gly Gly Ile Gly Cys Pro Gln Ala
               260                 265                 270
   gcg ctg gcc acc ttc aac atg ggt gcc tcc ttt att gtc acc ggc acc      864
   Ala Leu Ala Thr Phe Asn Met Gly Ala Ser Phe Ile Val Thr Gly Thr
           275                 280                 285
   gtg aac cag gtc gcc aag cag tcg ggc acg tgc gac aat gtg cgc aag      912
   Val Asn Gln Val Ala Lys Gln Ser Gly Thr Cys Asp Asn Val Arg Lys
       290                 295                 300
   cag ctc gcg aag gcc act tac tcg gac gta tgc atg gcc ccg gct gcc      960
   Gln Leu Ala Lys Ala Thr Tyr Ser Asp Val Cys Met Ala Pro Ala Ala
   305                 310                 315                 320
   gac atg ttc gag gaa ggc gtc aag ctt cag gtc ctc aag aag gga acc     1008
   Asp Met Phe Glu Glu Gly Val Lys Leu Gln Val Leu Lys Lys Gly Thr
                   325                 330                 335
   atg ttt ccc tcg cgc gcc aac aag ctc tac gag ctc ttt tgc aag tac     1056
   Met Phe Pro Ser Arg Ala Asn Lys Leu Tyr Glu Leu Phe Cys Lys Tyr
               340                 345                 350
   gac tcg ttc gag tcc atg ccc ccc gca gag ctt gcg cgc gtc gag aag     1104
   Asp Ser Phe Glu Ser Met Pro Pro Ala Glu Leu Ala Arg Val Glu Lys
           355                 360                 365
   cgc atc ttc agc cgc gcg ctc gaa gag gtc tgg gac gag acc aaa aac     1152
   Arg Ile Phe Ser Arg Ala Leu Glu Glu Val Trp Asp Glu Thr Lys Asn
       370                 375                 380
   ttt tac att aac cgt ctt cac aac ccg gag aag atc cag cgc gcc gag     1200
   Phe Tyr Ile Asn Arg Leu His Asn Pro Glu Lys Ile Gln Arg Ala Glu
   385                 390                 395                 400
   cgc gac ccc aag ctc aag atg tcg ctg tgc ttt cgc tgg tac ctg agc     1248
   Arg Asp Pro Lys Leu Lys Met Ser Leu Cys Phe Arg Trp Tyr Leu Ser
                   405                 410                 415
   ctg gcg agc cgc tgg gcc aac act gga gct tcc gat cgc gtc atg gac     1296
   Leu Ala Ser Arg Trp Ala Asn Thr Gly Ala Ser Asp Arg Val Met Asp
               420                 425                 430
   tac cag gtc tgg tgc ggt cct gcc att ggt tcc ttc aac gat ttc atc     1344
   Tyr Gln Val Trp Cys Gly Pro Ala Ile Gly Ser Phe Asn Asp Phe Ile
           435                 440                 445
   aag gga act tac ctt gat ccg gcc gtc gca aac gag tac ccg tgc gtc     1392
   Lys Gly Thr Tyr Leu Asp Pro Ala Val Ala Asn Glu Tyr Pro Cys Val
       450                 455                 460
   gtt cag att aac aag cag atc ctt cgt gga gcg tgc ttc ttg cgc cgt     1440
   Val Gln Ile Asn Lys Gln Ile Leu Arg Gly Ala Cys Phe Leu Arg Arg
   465                 470                 475                 480
   ctc gaa att ctg cgc aac gca cgc ctt tcc gat ggc gct gcc gct ctt      1488
   Leu Glu Ile Leu Arg Asn Ala Arg Leu Ser Asp Gly Ala Ala Ala Leu
                   485                 490                 495
   gtg gcc agc atc gat gac aca tac gtc ccg gcc gag aag ctg              1530
   Val Ala Ser Ile Asp Asp Thr Tyr Val Pro Ala Glu Lys Leu
               500                 505                 510
<210> 26
   <211> 510
   <212> PRT
   <213> Schizochytrium sp.
<400> 26
   Leu Leu Asp Leu Asp Ser Met Leu Ala Leu Ser Ser Ala Ser Ala Ser
   1               5                   10                  15
   Gly Asn Leu Val Glu Thr Ala Pro Ser Asp Ala Ser Val Ile Val Pro
               20                  25                  30
   Pro Cys Asn Ile Ala Asp Leu Gly Ser Arg Ala Phe Met Lys Thr Tyr
           35                  40                  45
   Gly Val Ser Ala Pro Leu Tyr Thr Gly Ala Met Ala Lys Gly Ile Ala
       50                  55                  60
   Ser Ala Asp Leu Val Ile Ala Ala Gly Arg Gln Gly Ile Leu Ala Ser
   65                  70                  75                  80
   Phe Gly Ala Gly Gly Leu Pro Met Gln Val Val Arg Glu Ser Ile Glu
                   85                  90                  95
   Lys Ile Gln Ala Ala Leu Pro Asn Gly Pro Tyr Ala Val Asn Leu Ile
               100                 105                 110
   His Ser Pro Phe Asp Ser Asn Leu Glu Lys Gly Asn Val Asp Leu Phe
           115                 120                 125
   Leu Glu Lys Gly Val Thr Phe Val Glu Ala Ser Ala Phe Met Thr Leu
       130                 135                 140
   Thr Pro Gln Val Val Arg Tyr Arg Ala Ala Gly Leu Thr Arg Asn Ala
   145                 150                 155                 160
   Asp Gly Ser Val Asn Ile Arg Asn Arg Ile Ile Gly Lys Val Ser Arg
                   165                 170                 175
   Thr Glu Leu Ala Glu Met Phe Met Arg Pro Ala Pro Glu His Leu Leu
               180                 185                 190
   Gln Lys Leu Ile Ala Ser Gly Glu Ile Asn Gln Glu Gln Ala Glu Leu
           195                 200                 205
   Ala Arg Arg Val Pro Val Ala Asp Asp Ile Ala Val Glu Ala Asp Ser
       210                 215                 220
   Gly Gly His Thr Asp Asn Arg Pro Ile His Val Ile Leu Pro Leu Ile
   225                 230                 235                 240
   Ile Asn Leu Arg Asp Arg Leu His Arg Glu Cys Gly Tyr Pro Ala Asn
                   245                 250                 255
   Leu Arg Val Arg Val Gly Ala Gly Gly Gly Ile Gly Cys Pro Gln Ala
               260                 265                 270
   Ala Leu Ala Thr Phe Asn Met Gly Ala Ser Phe Ile Val Thr Gly Thr
           275                 280                 285
   Val Asn Gln Val Ala Lys Gln Ser Gly Thr Cys Asp Asn Val Arg Lys
       290                 295                 300
   Gln Leu Ala Lys Ala Thr Tyr Ser Asp Val Cys Met Ala Pro Ala Ala
   305                 310                 315                 320
   Asp Met Phe Glu Glu Gly Val Lys Leu Gln Val Leu Lys Lys Gly Thr
                   325                 330                 335
   Met Phe Pro Ser Arg Ala Asn Lys Leu Tyr Glu Leu Phe Cys Lys Tyr
               340                 345                 350
   Asp Ser Phe Glu Ser Met Pro Pro Ala Glu Leu Ala Arg Val Glu Lys
           355                 360                 365
   Arg Ile Phe Ser Arg Ala Leu Glu Glu Val Trp Asp Glu Thr Lys Asn
       370                 375                 380
   Phe Tyr Ile Asn Arg Leu His Asn Pro Glu Lys Ile Gln Arg Ala Glu
   385                 390                 395                 400
   Arg Asp Pro Lys Leu Lys Met Ser Leu Cys Phe Arg Trp Tyr Leu Ser
                   405                 410                 415
   Leu Ala Ser Arg Trp Ala Asn Thr Gly Ala Ser Asp Arg Val Met Asp
               420                 425                 430
   Tyr Gln Val Trp Cys Gly Pro Ala Ile Gly Ser Phe Asn Asp Phe Ile
           435                 440                 445
   Lys Gly Thr Tyr Leu Asp Pro Ala Val Ala Asn Glu Tyr Pro Cys Val
       450                 455                 460
   Val Gln Ile Asn Lys Gln Ile Leu Arg Gly Ala Cys Phe Leu Arg Arg
   465                 470                 475                 480
   Leu Glu Ile Leu Arg Asn Ala Arg Leu Ser Asp Gly Ala Ala Ala Leu
                   485                 490                 495
   Val Ala Ser Ile Asp Asp Thr Tyr Val Pro Ala Glu Lys Leu
               500                 505                 510
<210> 27
   <211> 4512
   <212> DNA
   <213> Schizochytrium sp.
<220>
   <221> CDS
   <222> (1)..(4512)
<400> 27
   atg gcg ctc cgt gtc aag acg aac aag aag cca tgc tgg gag atg acc        48
   Met Ala Leu Arg Val Lys Thr Asn Lys Lys Pro Cys Trp Glu Met Thr
   1               5                   10                  15
   aag gag gag ctg acc agc ggc aag acc gag gtg ttc aac tat gag gaa        96
   Lys Glu Glu Leu Thr Ser Gly Lys Thr Glu Val Phe Asn Tyr Glu Glu
               20                  25                  30
   ctc ctc gag ttc gca gag ggc gac atc gcc aag gtc ttc gga ccc gag       144
   Leu Leu Glu Phe Ala Glu Gly Asp Ile Ala Lys Val Phe Gly Pro Glu
           35                  40                  45
   ttc gcc gtc atc gac aag tac ccg cgc cgc gtg cgc ctg ccc gcc cgc       192
   Phe Ala Val Ile Asp Lys Tyr Pro Arg Arg Val Arg Leu Pro Ala Arg
       50                  55                  60
   gag tac ctg ctc gtg acc cgc gtc acc ctc atg gac gcc gag gtc aac       240
   Glu Tyr Leu Leu Val Thr Arg Val Thr Leu Met Asp Ala Glu Val Asn
   65                  70                  75                  80
   aac tac cgc gtc ggc gcc cgc atg gtc acc gag tac gat ctc ccc gtc       288
   Asn Tyr Arg Val Gly Ala Arg Met Val Thr Glu Tyr Asp Leu Pro Val
                   85                  90                  95
   aac gga gag ctc tcc gag ggc gga gac tgc ccc tgg gcc gtc ctg gtc       336
   Asn Gly Glu Leu Ser Glu Gly Gly Asp Cys Pro Trp Ala Val Leu Val
               100                 105                 110
   gag agt ggc cag tgc gat ctc atg ctc atc tcc tac atg ggc att gac       384
   Glu Ser Gly Gln Cys Asp Leu Met Leu Ile Ser Tyr Met Gly Ile Asp
           115                 120                 125
   ttc cag aac cag ggc gac cgc gtc tac cgc ctg ctc aac acc acg ctc       432
   Phe Gln Asn Gln Gly Asp Arg Val Tyr Arg Leu Leu Asn Thr Thr Leu
       130                 135                 140
   acc ttt tac ggc gtg gcc cac gag ggc gag acc ctc gag tac gac att       480
   Thr Phe Tyr Gly Val Ala His Glu Gly Glu Thr Leu Glu Tyr Asp Ile
   145                 150                 155                 160
   cgc gtc acc ggc ttc gcc aag cgt ctc gac ggc ggc atc tcc atg ttc       528
   Arg Val Thr Gly Phe Ala Lys Arg Leu Asp Gly Gly Ile Ser Met Phe
                   165                 170                 175
   ttc ttc gag tac gac tgc tac gtc aac ggc cgc ctc ctc atc gag atg       576
   Phe Phe Glu Tyr Asp Cys Tyr Val Asn Gly Arg Leu Leu Ile Glu Met
               180                 185                 190
   cgc gat ggc tgc gcc ggc ttc ttc acc aac gag gag ctc gac gcc ggc       624
   Arg Asp Gly Cys Ala Gly Phe Phe Thr Asn Glu Glu Leu Asp Ala Gly
           195                 200                 205
   aag ggc gtc gtc ttc acc cgc ggc gac ctc gcc gcc cgc gcc aag atc       672
   Lys Gly Val Val Phe Thr Arg Gly Asp Leu Ala Ala Arg Ala Lys Ile
       210                 215                 220
   cca aag cag gac gtc tcc ccc tac gcc gtc gcc ccc tgc ctc cac aag       720
   Pro Lys Gln Asp Val Ser Pro Tyr Ala Val Ala Pro Cys Leu His Lys
   225                 230                 235                 240
   acc aag ctc aac gaa aag gag atg cag acc ctc gtc gac aag gac tgg      768
   Thr Lys Leu Asn Glu Lys Glu Met Gln Thr Leu Val Asp Lys Asp Trp
                   245                 250                 255
   gca tcc gtc ttt ggc tcc aag aac ggc atg ccg gaa atc aac tac aaa      816
   Ala Ser Val Phe Gly Ser Lys Asn Gly Met Pro Glu Ile Asn Tyr Lys
               260                 265                 270
   ctc tgc gcg cgt aag atg ctc atg att gac cgc gtc acc agc att gac      864
   Leu Cys Ala Arg Lys Met Leu Met Ile Asp Arg Val Thr Ser Ile Asp
           275                 280                 285
   cac aag ggc ggt gtc tac ggc ctc ggt cag ctc gtc ggt gaa aag atc      912
   His Lys Gly Gly Val Tyr Gly Leu Gly Gln Leu Val Gly Glu Lys Ile
       290                 295                 300
   ctc gag cgc gac cac tgg tac ttt ccc tgc cac ttt gtc aag gat cag      960
   Leu Glu Arg Asp His Trp Tyr Phe Pro Cys His Phe Val Lys Asp Gln
   305                 310                 315                 320
   gtc atg gcc gga tcc ctc gtc tcc gac ggc tgc agc cag atg ctc aag     1008
   Val Met Ala Gly Ser Leu Val Ser Asp Gly Cys Ser Gln Met Leu Lys
                   325                 330                 335
   atg tac atg atc tgg ctc ggc ctc cac ctc acc acc gga ccc ttt gac     1056
   Met Tyr Met Ile Trp Leu Gly Leu His Leu Thr Thr Gly Pro Phe Asp
               340                 345                 350
   ttc cgc ccg gtc aac ggc cac ccc aac aag gtc cgc tgc cgc ggc caa     1104
   Phe Arg Pro Val Asn Gly His Pro Asn Lys Val Arg Cys Arg Gly Gln
           355                 360                 365
   atc tcc ccg cac aag ggc aag ctc gtc tac gtc atg gag atc aag gag     1152
   Ile Ser Pro His Lys Gly Lys Leu Val Tyr Val Met Glu Ile Lys Glu
       370                 375                 380
   atg ggc ttc gac gag gac aac gac ccg tac gcc att gcc gac gtc aac     1200
   Met Gly Phe Asp Glu Asp Asn Asp Pro Tyr Ala Ile Ala Asp Val Asn
   385                 390                 395                 400
   atc att gat gtc gac ttc gaa aag ggc cag gac ttt agc ctc gac cgc     1248
   Ile Ile Asp Val Asp Phe Glu Lys Gly Gln Asp Phe Ser Leu Asp Arg
                   405                 410                 415
   atc agc gac tac ggc aag ggc gac ctc aac aag aag atc gtc gtc gac     1296
   Ile Ser Asp Tyr Gly Lys Gly Asp Leu Asn Lys Lys Ile Val Val Asp
               420                 425                 430
   ttt aag ggc atc gct ctc aag atg cag aag cgc tcc acc aac aag aac     1344
   Phe Lys Gly Ile Ala Leu Lys Met Gln Lys Arg Ser Thr Asn Lys Asn
           435                 440                 445
   ccc tcc aag gtt cag ccc gtc ttt gcc aac ggc gcc gcc act gtc ggc     1392
   Pro Ser Lys Val Gln Pro Val Phe Ala Asn Gly Ala Ala Thr Val Gly
       450                 455                 460
   ccc gag gcc tcc aag gct tcc tcc ggc gcc agc gcc agc gcc agc gcc     1440
   Pro Glu Ala Ser Lys Ala Ser Ser Gly Ala Ser Ala Ser Ala Ser Ala
   465                 470                 475                 480
   gcc ccg gcc aag cct gcc ttc agc gcc gat gtt ctt gcg ccc aag ccc     1488
   Ala Pro Ala Lys Pro Ala Phe Ser Ala Asp Val Leu Ala Pro Lys Pro
                   485                 490                 495
   gtt gcc ctt ccc gag cac atc ctc aag ggc gac gcc ctc gcc ccc aag     1536
   Val Ala Leu Pro Glu His Ile Leu Lys Gly Asp Ala Leu Ala Pro Lys
               500                 505                 510
   gag atg tcc tgg cac ccc atg gcc cgc atc ccg ggc aac ccg acg ccc     1584
   Glu Met Ser Trp His Pro Met Ala Arg Ile Pro Gly Asn Pro Thr Pro
           515                 520                 525
   tct ttt gcg ccc tcg gcc tac aag ccg cgc aac atc gcc ttt acg ccc      1632
   Ser Phe Ala Pro Ser Ala Tyr Lys Pro Arg Asn Ile Ala Phe Thr Pro
       530                 535                 540
   ttc ccc ggc aac ccc aac gat aac gac cac acc ccg ggc aag atg ccg      1680
   Phe Pro Gly Asn Pro Asn Asp Asn Asp His Thr Pro Gly Lys Met Pro
   545                 550                 555                 560
   ctc acc tgg ttc aac atg gcc gag ttc atg gcc ggc aag gtc agc atg      1728
   Leu Thr Trp Phe Asn Met Ala Glu Phe Met Ala Gly Lys Val Ser Met
                   565                 570                 575
   tgc ctc ggc ccc gag ttc gcc aag ttc gac gac tcg aac acc agc cgc      1776
   Cys Leu Gly Pro Glu Phe Ala Lys Phe Asp Asp Ser Asn Thr Ser Arg
               580                 585                 590
   agc ccc gct tgg gac ctc gct ctc gtc acc cgc gcc gtg tct gtg tct      1824
   Ser Pro Ala Trp Asp Leu Ala Leu Val Thr Arg Ala Val Ser Val Ser
           595                 600                 605
   gac ctc aag cac gtc aac tac cgc aac atc gac ctc gac ccc tcc aag      1872
   Asp Leu Lys His Val Asn Tyr Arg Asn Ile Asp Leu Asp Pro Ser Lys
       610                 615                 620
   ggt acc atg gtc ggc gag ttc gac tgc ccc gcg gac gcc tgg ttc tac      1920
   Gly Thr Met Val Gly Glu Phe Asp Cys Pro Ala Asp Ala Trp Phe Tyr
   625                 630                 635                 640
   aag ggc gcc tgc aac gat gcc cac atg ccg tac tcg atc ctc atg gag      1968
   Lys Gly Ala Cys Asn Asp Ala His Met Pro Tyr Ser Ile Leu Met Glu
                   645                 650                 655
   atc gcc ctc cag acc tcg ggt gtg ctc acc tcg gtg ctc aag gcg ccc      2016
   Ile Ala Leu Gln Thr Ser Gly Val Leu Thr Ser Val Leu Lys Ala Pro
               660                 665                 670
   ctg acc atg gag aag gac gac atc ctc ttc cgc aac ctc gac gcc aac      2064
   Leu Thr Met Glu Lys Asp Asp Ile Leu Phe Arg Asn Leu Asp Ala Asn
           675                 680                 685
   gcc gag ttc gtg cgc gcc gac ctc gac tac cgc ggc aag act atc cgc      2112
   Ala Glu Phe Val Arg Ala Asp Leu Asp Tyr Arg Gly Lys Thr Ile Arg
       690                 695                 700
   aac gtc acc aag tgc act ggc tac agc atg ctc ggc gag atg ggc gtc      2160
   Asn Val Thr Lys Cys Thr Gly Tyr Ser Met Leu Gly Glu Met Gly Val
   705                 710                 715                 720
   cac cgc ttc acc ttt gag ctc tac gtc gat gat gtg ctc ttt tac aag      2208
   His Arg Phe Thr Phe Glu Leu Tyr Val Asp Asp Val Leu Phe Tyr Lys
                   725                 730                 735
   ggc tcg acc tcg ttc ggc tgg ttc gtg ccc gag gtc ttt gcc gcc cag      2256
   Gly Ser Thr Ser Phe Gly Trp Phe Val Pro Glu Val Phe Ala Ala Gln
               740                 745                 750
   gcc ggc ctc gac aac ggc cgc aag tcg gag ccc tgg ttc att gag aac      2304
   Ala Gly Leu Asp Asn Gly Arg Lys Ser Glu Pro Trp Phe Ile Glu Asn
           755                 760                 765
   aag gtt ccg gcc tcg cag gtc tcc tcc ttt gac gtg cgc ccc aac ggc      2352
   Lys Val Pro Ala Ser Gln Val Ser Ser Phe Asp Val Arg Pro Asn Gly
       770                 775                 780
   agc ggc cgc acc gcc atc ttc gcc aac gcc ccc agc ggc gcc cag ctc      2400
   Ser Gly Arg Thr Ala Ile Phe Ala Asn Ala Pro Ser Gly Ala Gln Leu
   785                 790                 795                 800
   aac cgc cgc acg gac cag ggc cag tac ctc gac gcc gtc gac att gtc      2448
   Asn Arg Arg Thr Asp Gln Gly Gln Tyr Leu Asp Ala Val Asp Ile Val
                   805                 810                 815
   tcc ggc agc ggc aag aag agc ctc ggc tac gcc cac ggt tcc aag acg      2496
   Ser Gly Ser Gly Lys Lys Ser Leu Gly Tyr Ala His Gly Ser Lys Thr
               820                 825                 830
   gtc aac ccg aac gac tgg ttc ttc tcg tgc cac ttt tgg ttt gac tcg      2544
   Val Asn Pro Asn Asp Trp Phe Phe Ser Cys His Phe Trp Phe Asp Ser
           835                 840                 845
   gtc atg ccc gga agt ctc ggt gtc gag tcc atg ttc cag ctc gtc gag      2592
   Val Met Pro Gly Ser Leu Gly Val Glu Ser Met Phe Gln Leu Val Glu
       850                 855                 860
   gcc atc gcc gcc cac gag gat ctc gct ggc aaa gca cgg cat tgc caa      2640
   Ala Ile Ala Ala His Glu Asp Leu Ala Gly Lys Ala Arg His Cys Gln
   865                 870                 875                 880
   ccc cac ctt tgt gca cgc ccc cgg gca aga tca agc tgg aag tac cgc      2688
   Pro His Leu Cys Ala Arg Pro Arg Ala Arg Ser Ser Trp Lys Tyr Arg
                   885                 890                 895
   ggc cag ctc acg ccc aag agc aag aag atg gac tcg gag gtc cac atc      2736
   Gly Gln Leu Thr Pro Lys Ser Lys Lys Met Asp Ser Glu Val His Ile
               900                 905                 910
   gtg tcc gtg gac gcc cac gac ggc gtt gtc gac ctc gtc gcc gac ggc      2784
   Val Ser Val Asp Ala His Asp Gly Val Val Asp Leu Val Ala Asp Gly
           915                 920                 925
   ttc ctc tgg gcc gac agc ctc cgc gtc tac tcg gtg agc aac att cgc      2832
   Phe Leu Trp Ala Asp Ser Leu Arg Val Tyr Ser Val Ser Asn Ile Arg
       930                 935                 940
   gtg cgc atc gcc tcc ggt gag gcc cct gcc gcc gcc tcc tcc gcc gcc      2880
   Val Arg Ile Ala Ser Gly Glu Ala Pro Ala Ala Ala Ser Ser Ala Ala
   945                 950                 955                 960
   tct gtg ggc tcc tcg gct tcg tcc gtc gag cgc acg cgc tcg agc ccc      2928
   Ser Val Gly Ser Ser Ala Ser Ser Val Glu Arg Thr Arg Ser Ser Pro
                   965                 970                 975
   gct gtc gcc tcc ggc ccg gcc cag acc atc gac ctc aag cag ctc aag      2976
   Ala Val Ala Ser Gly Pro Ala Gln Thr Ile Asp Leu Lys Gln Leu Lys
               980                 985                 990
   acc gag ctc ctc gag ctc gat gcc  ccg ctc tac ctc tcg  cag gac ccg    3024
   Thr Glu Leu Leu Glu Leu Asp Ala  Pro Leu Tyr Leu Ser  Gln Asp Pro
           995                 1000                1005
   acc agc  ggc cag ctc aag aag  cac acc gac gtg gcc  tcc ggc cag      3069
   Thr Ser  Gly Gln Leu Lys Lys  His Thr Asp Val Ala  Ser Gly Gln
       1010                1015                1020
   gcc acc  atc gtg cag ccc tgc  acg ctc ggc gac ctc  ggt gac cgc      3114
   Ala Thr  Ile Val Gln Pro Cys  Thr Leu Gly Asp Leu  Gly Asp Arg
       1025                1030                1035
   tcc ttc  atg gag acc tac ggc  gtc gtc gcc ccg ctg  tac acg ggc      3159
   Ser Phe  Met Glu Thr Tyr Gly  Val Val Ala Pro Leu  Tyr Thr Gly
       1040                1045                1050
   gcc atg  gcc aag ggc att gcc  tcg gcg gac ctc gtc  atc gcc gcc      3204
   Ala Met  Ala Lys Gly Ile Ala  Ser Ala Asp Leu Val  Ile Ala Ala
       1055                1060                1065
   ggc aag  cgc aag atc ctc ggc  tcc ttt ggc gcc ggc  ggc ctc ccc      3249
   Gly Lys  Arg Lys Ile Leu Gly  Ser Phe Gly Ala Gly  Gly Leu Pro
       1070                1075                1080
   atg cac  cac gtg cgc gcc gcc  ctc gag aag atc cag  gcc gcc ctg      3294
   Met His  His Val Arg Ala Ala  Leu Glu Lys Ile Gln  Ala Ala Leu
       1085                1090                1095
   cct cag ggc ccc tac gcc gtc aac ctc atc cac tcg cct ttt gac      3339
   Pro Gln Gly Pro Tyr Ala Val Asn Leu Ile His Ser Pro Phe Asp
       1100                1105                1110
   agc aac ctc gag aag ggc aac gtc gat ctc ttc ctc gag aag ggc      3384
   Ser Asn Leu Glu Lys Gly Asn Val Asp Leu Phe Leu Glu Lys Gly
       1115                1120                1125
   gtc act gtg gtg gag gcc tcg gca ttc atg acc ctc acc ccg cag      3429
   Val Thr Val Val Glu Ala Ser Ala Phe Met Thr Leu Thr Pro Gln
       1130                1135                1140
   gtc gtg cgc tac cgc gcc gcc ggc ctc tcg cgc aac gcc gac ggt      3474
   Val Val Arg Tyr Arg Ala Ala Gly Leu Ser Arg Asn Ala Asp Gly
       1145                1150                1155
   tcg gtc aac atc cgc aac cgc atc atc ggc aag gtc tcg cgc acc      3519
   Ser Val Asn Ile Arg Asn Arg Ile Ile Gly Lys Val Ser Arg Thr
       1160                1165                1170
   gag ctc gcc gag atg ttc atc cgc ccg gcc ccg gag cac ctc ctc      3564
   Glu Leu Ala Glu Met Phe Ile Arg Pro Ala Pro Glu His Leu Leu
       1175                1180                1185
   gag aag ctc atc gcc tcg ggc gag atc acc cag gag cag gcc gag      3609
   Glu Lys Leu Ile Ala Ser Gly Glu Ile Thr Gln Glu Gln Ala Glu
       1190                1195                1200
   ctc gcg cgc cgc gtt ccc gtc gcc gac gat atc gct gtc gag gct      3654
   Leu Ala Arg Arg Val Pro Val Ala Asp Asp Ile Ala Val Glu Ala
       1205                1210                1215
   gac tcg ggc ggc cac acc gac aac cgc ccc atc cac gtc atc ctc      3699
   Asp Ser Gly Gly His Thr Asp Asn Arg Pro Ile His Val Ile Leu
       1220                1225                1230
   ccg ctc atc atc aac ctc cgc aac cgc ctg cac cgc gag tgc ggc      3744
   Pro Leu Ile Ile Asn Leu Arg Asn Arg Leu His Arg Glu Cys Gly
       1235                1240                1245
   tac ccc gcg cac ctc cgc gtc cgc gtt ggc gcc ggc ggt ggc gtc      3789
   Tyr Pro Ala His Leu Arg Val Arg Val Gly Ala Gly Gly Gly Val
       1250                1255                1260
   ggc tgc ccg cag gcc gcc gcc gcc gcg ctc acc atg ggc gcc gcc      3834
   Gly Cys Pro Gln Ala Ala Ala Ala Ala Leu Thr Met Gly Ala Ala
       1265                1270                1275
   ttc atc gtc acc ggc act gtc aac cag gtc gcc aag cag tcc ggc      3879
   Phe Ile Val Thr Gly Thr Val Asn Gln Val Ala Lys Gln Ser Gly
       1280                1285                1290
   acc tgc gac aac gtg cgc aag cag ctc tcg cag gcc acc tac tcg      3924
   Thr Cys Asp Asn Val Arg Lys Gln Leu Ser Gln Ala Thr Tyr Ser
       1295                1300                1305
   gat atc tgc atg gcc ccg gcc gcc gac atg ttc gag gag ggc gtc      3969
   Asp Ile Cys Met Ala Pro Ala Ala Asp Met Phe Glu Glu Gly Val
       1310                1315                1320
   aag ctc cag gtc ctc aag aag gga acc atg ttc ccc tcg cgc gcc      4014
   Lys Leu Gln Val Leu Lys Lys Gly Thr Met Phe Pro Ser Arg Ala
       1325                1330                1335
   aac aag ctc tac gag ctc ttt tgc aag tac gac tcc ttc gac tcc      4059
   Asn Lys Leu Tyr Glu Leu Phe Cys Lys Tyr Asp Ser Phe Asp Ser
       1340                1345                1350
   atg cct cct gcc gag ctc gag cgc atc gag aag cgt atc ttc aag      4104
   Met Pro Pro Ala Glu Leu Glu Arg Ile Glu Lys Arg Ile Phe Lys
       1355                1360                1365
   cgc gca  ctc cag gag gtc tgg  gag gag acc aag gac  ttt tac att      4149
   Arg Ala  Leu Gln Glu Val Trp  Glu Glu Thr Lys Asp  Phe Tyr Ile
       1370                 1375                 1380
   aac ggt  ctc aag aac ccg gag  aag atc cag cgc gcc  gag cac gac      4194
   Asn Gly  Leu Lys Asn Pro Glu  Lys Ile Gln Arg Ala  Glu His Asp
       1385                 1390                 1395
   ccc aag  ctc aag atg tcg ctc  tgc ttc cgc tgg tac  ctt ggt ctt      4239
   Pro Lys  Leu Lys Met Ser Leu  Cys Phe Arg Trp Tyr  Leu Gly Leu
       1400                 1405                 1410
   gcc agc  cgc tgg gcc aac atg  ggc gcc ccg gac cgc  gtc atg gac      4284
   Ala Ser  Arg Trp Ala Asn Met  Gly Ala Pro Asp Arg  Val Met Asp
       1415                 1420                 1425
   tac cag  gtc tgg tgt ggc ccg  gcc att ggc gcc ttc  aac gac ttc      4329
   Tyr Gln  Val Trp Cys Gly Pro  Ala Ile Gly Ala Phe  Asn Asp Phe
       1430                 1435                 1440
   atc aag  ggc acc tac ctc gac  ccc gct gtc tcc aac  gag tac ccc      4374
   Ile Lys  Gly Thr Tyr Leu Asp  Pro Ala Val Ser Asn  Glu Tyr Pro
       1445                 1450                 1455
   tgt gtc  gtc cag atc aac ctg  caa atc ctc cgt ggt  gcc tgc tac      4419
   Cys Val  Val Gln Ile Asn Leu  Gln Ile Leu Arg Gly  Ala Cys Tyr
       1460                 1465                 1470
   ctg cgc  cgt ctc aac gcc ctg  cgc aac gac ccg cgc  att gac ctc      4464
   Leu Arg  Arg Leu Asn Ala Leu  Arg Asn Asp Pro Arg  Ile Asp Leu
       1475                 1480                 1485
   gag acc  gag gat gct gcc ttt  gtc tac gag ccc acc  aac gcg ctc      4509
   Glu Thr  Glu Asp Ala Ala Phe  Val Tyr Glu Pro Thr  Asn Ala Leu
       1490                 1495                 1500
   taa                                                                 4512
<210> 28
   <211> 1503
   <212> PRT
   <213> Schizochytrium sp.
<400> 28
   Met Ala Leu Arg Val Lys Thr Asn Lys Lys Pro Cys Trp Glu Met Thr
   1               5                   10                  15
   Lys Glu Glu Leu Thr Ser Gly Lys Thr Glu Val Phe Asn Tyr Glu Glu
               20                  25                  30
   Leu Leu Glu Phe Ala Glu Gly Asp Ile Ala Lys Val Phe Gly Pro Glu
           35                  40                  45
   Phe Ala Val Ile Asp Lys Tyr Pro Arg Arg Val Arg Leu Pro Ala Arg
       50                  55                  60
   Glu Tyr Leu Leu Val Thr Arg Val Thr Leu Met Asp Ala Glu Val Asn
   65                  70                  75                  80
   Asn Tyr Arg Val Gly Ala Arg Met Val Thr Glu Tyr Asp Leu Pro Val
                   85                  90                  95
   Asn Gly Glu Leu Ser Glu Gly Gly Asp Cys Pro Trp Ala Val Leu Val
   100 105 110
   Glu Ser Gly Gln Cys Asp Leu Met Leu Ile Ser Tyr Met Gly Ile Asp
   115 120 125
   Phe Gln Asn Gln Gly Asp Arg Val Tyr Arg Leu Leu Asn Thr Thr Leu
   130 135 140
   Thr Phe Tyr Gly Val Ala His Glu Gly Glu Thr Leu Glu Tyr Asp Ile
   145 150 155 160
   Arg Val Thr Gly Phe Ala Lys Arg Leu Asp Gly Gly Ile Ser Met Phe
   165 170 175
   Phe Phe Glu Tyr Asp Cys Tyr Val Asn Gly Arg Leu Leu Ile Glu Met
   180 185 190
   Arg Asp Gly Cys Ala Gly Phe Phe Thr Asn Glu Glu Leu Asp Ala Gly
   195 200 205
   Lys Gly Val Val Phe Thr Arg Gly Asp Leu Ala Ala Arg Ala Lys Ile
   210 215 220
   Pro Lys Gln Asp Val Ser Pro Tyr Ala Val Ala Pro Cys Leu His Lys
   225 230 235 240
   Thr Lys Leu Asn Glu Lys Glu Met Gln Thr Leu Val Asp Lys Asp Trp
   245 250 255
   Ala Ser Val Phe Gly Ser Lys Asn Gly Met Pro Glu Ile Asn Tyr Lys
   260 265 270
   Leu Cys Ala Arg Lys Met Leu Met Ile Asp Arg Val Thr Ser Ile Asp
   275 280 285
   His Lys Gly Gly Val Tyr Gly Leu Gly Gln Leu Val Gly Glu Lys Ile
   290 295 300
   Leu Glu Arg Asp His Trp Tyr Phe Pro Cys His Phe Val Lys Asp Gln
   305 310 315 320
   Val Met Ala Gly Ser Leu Val Ser Asp Gly Cys Ser Gln Met Leu Lys
   325 330 335
   Met Tyr Met Ile Trp Leu Gly Leu His Leu Thr Thr Gly Pro Phe Asp
   340 345 350
   Phe Arg Pro Val Asn Gly His Pro Asn Lys Val Arg Cys Arg Gly Gln
   355 360 365
   Ile Ser Pro His Lys Gly Lys Leu Val Tyr Val Met Glu Ile Lys Glu
   370 375 380
   Met Gly Phe Asp Glu Asp Asn Asp Pro Tyr Ala Ile Ala Asp Val Asn
   385 390 395 400
   Ile Ile Asp Val Asp Phe Glu Lys Gly Gln Asp Phe Ser Leu Asp Arg
   405 410 415
   Ile Ser Asp Tyr Gly Lys Gly Asp Leu Asn Lys Lys Ile Val Val Asp
   420 425 430
   Phe Lys Gly Ile Ala Leu Lys Met Gln Lys Arg Ser Thr Asn Lys Asn
   435 440 445
   Pro Ser Lys Val Gln Pro Val Phe Ala Asn Gly Ala Ala Thr Val Gly
   450 455 460
   Pro Glu Ala Ser Lys Ala Ser Ser Gly Ala Ser Ala Ser Ala Ser Ala
   465 470 475 480
   Ala Pro Ala Lys Pro Ala Phe Ser Ala Asp Val Leu Ala Pro Lys Pro
   485 490 495
   Val Ala Leu Pro Glu His Ile Leu Lys Gly Asp Ala Leu Ala Pro Lys
   500 505 510
   Glu Met Ser Trp His Pro Met Ala Arg Ile Pro Gly Asn Pro Thr Pro
   515 520 525
   Ser Phe Ala Pro Ser Ala Tyr Lys Pro Arg Asn Ile Ala Phe Thr Pro
   530 535 540
   Phe Pro Gly Asn Pro Asn Asp Asn Asp His Thr Pro Gly Lys Met Pro
   545 550 555 560
   Leu Thr Trp Phe Asn Met Ala Glu Phe Met Ala Gly Lys Val Ser Met
   565 570 575
   Cys Leu Gly Pro Glu Phe Ala Lys Phe Asp Asp Ser Asn Thr Ser Arg
   580 585 590
   Ser Pro Ala Trp Asp Leu Ala Leu Val Thr Arg Ala Val Ser Val Ser
   595 600 605
   Asp Leu Lys His Val Asn Tyr Arg Asn Ile Asp Leu Asp Pro Ser Lys
   610 615 620
   Gly Thr Met Val Gly Glu Phe Asp Cys Pro Ala Asp Ala Trp Phe Tyr
   625 630 635 640
   Lys Gly Ala Cys Asn Asp Ala His Met Pro Tyr Ser Ile Leu Met Glu
   645 650 655
   Ile Ala Leu Gln Thr Ser Gly Val Leu Thr Ser Val Leu Lys Ala Pro
   660 665 670
   Leu Thr Met Glu Lys Asp Asp Ile Leu Phe Arg Asn Leu Asp Ala Asn
           675                 680                 685
   Ala Glu Phe Val Arg Ala Asp Leu Asp Tyr Arg Gly Lys Thr Ile Arg
       690                 695                 700
   Asn Val Thr Lys Cys Thr Gly Tyr Ser Met Leu Gly Glu Met Gly Val
   705                 710                 715                 720
   His Arg Phe Thr Phe Glu Leu Tyr Val Asp Asp Val Leu Phe Tyr Lys
                   725                 730                 735
   Gly Ser Thr Ser Phe Gly Trp Phe Val Pro Glu Val Phe Ala Ala Gln
               740                 745                 750
   Ala Gly Leu Asp Asn Gly Arg Lys Ser Glu Pro Trp Phe Ile Glu Asn
           755                 760                 765
   Lys Val Pro Ala Ser Gln Val Ser Ser Phe Asp Val Arg Pro Asn Gly
       770                 775                 780
   Ser Gly Arg Thr Ala Ile Phe Ala Asn Ala Pro Ser Gly Ala Gln Leu
   785                 790                 795                 800
   Asn Arg Arg Thr Asp Gln Gly Gln Tyr Leu Asp Ala Val Asp Ile Val
                   805                 810                 815
   Ser Gly Ser Gly Lys Lys Ser Leu Gly Tyr Ala His Gly Ser Lys Thr
               820                 825                 830
   Val Asn Pro Asn Asp Trp Phe Phe Ser Cys His Phe Trp Phe Asp Ser
           835                 840                 845
   Val Met Pro Gly Ser Leu Gly Val Glu Ser Met Phe Gln Leu Val Glu
       850                 855                 860
   Ala Ile Ala Ala His Glu Asp Leu Ala Gly Lys Ala Arg His Cys Gln
   865                 870                 875                 880
   Pro His Leu Cys Ala Arg Pro Arg Ala Arg Ser Ser Trp Lys Tyr Arg
                   885                 890                 895
   Gly Gln Leu Thr Pro Lys Ser Lys Lys Met Asp Ser Glu Val His Ile
               900                 905                 910
   Val Ser Val Asp Ala His Asp Gly Val Val Asp Leu Val Ala Asp Gly
           915                 920                 925
   Phe Leu Trp Ala Asp Ser Leu Arg Val Tyr Ser Val Ser Asn Ile Arg
       930                 935                 940
   Val Arg Ile Ala Ser Gly Glu Ala Pro Ala Ala Ala Ser Ser Ala Ala
   945                 950                 955                 960
   Ser Val Gly Ser Ser Ala Ser Ser Val Glu Arg Thr Arg Ser Ser Pro
   965 970 975
   Ala Val Ala Ser Gly Pro Ala Gln Thr Ile Asp Leu Lys Gln Leu Lys
   980 985 990
   Thr Glu Leu Leu Glu Leu Asp Ala Pro Leu Tyr Leu Ser Gln Asp Pro
   995 1000 1005
   Thr Ser Gly Gln Leu Lys Lys His Thr Asp Val Ala Ser Gly Gln
   1010 1015 1020
   Ala Thr Ile Val Gln Pro Cys Thr Leu Gly Asp Leu Gly Asp Arg
   1025 1030 1035
   Ser Phe Met Glu Thr Tyr Gly Val Val Ala Pro Leu Tyr Thr Gly
   1040 1045 1050
   Ala Met Ala Lys Gly Ile Ala Ser Ala Asp Leu Val Ile Ala Ala
   1055 1060 1065
   Gly Lys Arg Lys Ile Leu Gly Ser Phe Gly Ala Gly Gly Leu Pro
   1070 1075 1080
   Met His His Val Arg Ala Ala Leu Glu Lys Ile Gln Ala Ala Leu
   1085 1090 1095
   Pro Gln Gly Pro Tyr Ala Val Asn Leu Ile His Ser Pro Phe Asp
   1100 1105 1110
   Ser Asn Leu Glu Lys Gly Asn Val Asp Leu Phe Leu Glu Lys Gly
   1115 1120 1125
   Val Thr Val Val Glu Ala Ser Ala Phe Met Thr Leu Thr Pro Gln
   1130 1135 1140
   Val Val Arg Tyr Arg Ala Ala Gly Leu Ser Arg Asn Ala Asp Gly
   1145 1150 1155
   Ser Val Asn Ile Arg Asn Arg Ile Ile Gly Lys Val Ser Arg Thr
   1160 1165 1170
   Glu Leu Ala Glu Met Phe Ile Arg Pro Ala Pro Glu His Leu Leu
   1175 1180 1185
   Glu Lys Leu Ile Ala Ser Gly Glu Ile Thr Gln Glu Gln Ala Glu
   1190 1195 1200
   Leu Ala Arg Arg Val Pro Val Ala Asp Asp Ile Ala Val Glu Ala
   1205 1210 1215
   Asp Ser Gly Gly His Thr Asp Asn Arg Pro Ile His Val Ile Leu
   1220 1225 1230
   Pro Leu Ile Ile Asn Leu Arg Asn Arg Leu His Arg Glu Cys Gly
       1235                1240                1245
   Tyr Pro Ala His Leu Arg Val Arg Val Gly Ala Gly Gly Gly Val
       1250                1255                1260
   Gly Cys Pro Gln Ala Ala Ala Ala Ala Leu Thr Met Gly Ala Ala
       1265                1270                1275
   Phe Ile Val Thr Gly Thr Val Asn Gln Val Ala Lys Gln Ser Gly
       1280                1285                1290
   Thr Cys Asp Asn Val Arg Lys Gln Leu Ser Gln Ala Thr Tyr Ser
       1295                1300                1305
   Asp Ile Cys Met Ala Pro Ala Ala Asp Met Phe Glu Glu Gly Val
       1310                1315                1320
   Lys Leu Gln Val Leu Lys Lys Gly Thr Met Phe Pro Ser Arg Ala
       1325                1330                1335
   Asn Lys Leu Tyr Glu Leu Phe Cys Lys Tyr Asp Ser Phe Asp Ser
       1340                1345                1350
   Met Pro Pro Ala Glu Leu Glu Arg Ile Glu Lys Arg Ile Phe Lys
       1355                1360                1365
   Arg Ala Leu Gln Glu Val Trp Glu Glu Thr Lys Asp Phe Tyr Ile
       1370                1375                1380
   Asn Gly Leu Lys Asn Pro Glu Lys Ile Gln Arg Ala Glu His Asp
       1385                1390                1395
   Pro Lys Leu Lys Met Ser Leu Cys Phe Arg Trp Tyr Leu Gly Leu
       1400                1405                1410
   Ala Ser Arg Trp Ala Asn Met Gly Ala Pro Asp Arg Val Met Asp
       1415                1420                1425
   Tyr Gln Val Trp Cys Gly Pro Ala Ile Gly Ala Phe Asn Asp Phe
       1430                1435                1440
   Ile Lys Gly Thr Tyr Leu Asp Pro Ala Val Ser Asn Glu Tyr Pro
       1445                1450                1455
   Cys Val Val Gln Ile Asn Leu Gln Ile Leu Arg Gly Ala Cys Tyr
       1460                1465                1470
   Leu Arg Arg Leu Asn Ala Leu Arg Asn Asp Pro Arg Ile Asp Leu
       1475                1480                1485
   Glu Thr Glu Asp Ala Ala Phe Val Tyr Glu Pro Thr Asn Ala Leu
       1490                1495                1500
<210> 29
   <211> 1500
   <212> DNA
   <213> Schizochytrium sp.
<220>
   <221> CDS
   <222> (1)..(1500)
<400> 29
   aag gtt cag ccc gtc ttt gcc aac ggc gcc gcc act gtc ggc ccc gag      48
   Lys Val Gln Pro Val Phe Ala Asn Gly Ala Ala Thr Val Gly Pro Glu
   1               5                   10                  15
   gcc tcc aag gct tcc tcc ggc gcc agc gcc agc gcc agc gcc gcc ccg      96
   Ala Ser Lys Ala Ser Ser Gly Ala Ser Ala Ser Ala Ser Ala Ala Pro
               20                  25                  30
   gcc aag cct gcc ttc agc gcc gat gtt ctt gcg ccc aag ccc gtt gcc     144
   Ala Lys Pro Ala Phe Ser Ala Asp Val Leu Ala Pro Lys Pro Val Ala
           35                  40                  45
   ctt ccc gag cac atc ctc aag ggc gac gcc ctc gcc ccc aag gag atg     192
   Leu Pro Glu His Ile Leu Lys Gly Asp Ala Leu Ala Pro Lys Glu Met
       50                  55                  60
   tcc tgg cac ccc atg gcc cgc atc ccg ggc aac ccg acg ccc tct ttt     240
   Ser Trp His Pro Met Ala Arg Ile Pro Gly Asn Pro Thr Pro Ser Phe
   65                  70                  75                  80
   gcg ccc tcg gcc tac aag ccg cgc aac atc gcc ttt acg ccc ttc ccc     288
   Ala Pro Ser Ala Tyr Lys Pro Arg Asn Ile Ala Phe Thr Pro Phe Pro
                   85                  90                  95
   ggc aac ccc aac gat aac gac cac acc ccg ggc aag atg ccg ctc acc     336
   Gly Asn Pro Asn Asp Asn Asp His Thr Pro Gly Lys Met Pro Leu Thr
               100                 105                 110
   tgg ttc aac atg gcc gag ttc atg gcc ggc aag gtc agc atg tgc ctc     384
   Trp Phe Asn Met Ala Glu Phe Met Ala Gly Lys Val Ser Met Cys Leu
           115                 120                 125
   ggc ccc gag ttc gcc aag ttc gac gac tcg aac acc agc cgc agc ccc     432
   Gly Pro Glu Phe Ala Lys Phe Asp Asp Ser Asn Thr Ser Arg Ser Pro
       130                 135                 140
   gct tgg gac ctc gct ctc gtc acc cgc gcc gtg tct gtg tct gac ctc     480
   Ala Trp Asp Leu Ala Leu Val Thr Arg Ala Val Ser Val Ser Asp Leu
   145                 150                 155                 160
   aag cac gtc aac tac cgc aac atc gac ctc gac ccc tcc aag ggt acc     528
   Lys His Val Asn Tyr Arg Asn Ile Asp Leu Asp Pro Ser Lys Gly Thr
                   165                 170                 175
   atg gtc ggc gag ttc gac tgc ccc gcg gac gcc tgg ttc tac aag ggc     576
   Met Val Gly Glu Phe Asp Cys Pro Ala Asp Ala Trp Phe Tyr Lys Gly
               180                 185                 190
   gcc tgc aac gat gcc cac atg ccg tac tcg atc ctc atg gag atc gcc     624
   Ala Cys Asn Asp Ala His Met Pro Tyr Ser Ile Leu Met Glu Ile Ala
           195                 200                 205
   ctc cag acc tcg ggt gtg ctc acc tcg gtg ctc aag gcg ccc ctg acc     672
   Leu Gln Thr Ser Gly Val Leu Thr Ser Val Leu Lys Ala Pro Leu Thr
       210                 215                 220
   atg gag aag gac gac atc ctc ttc cgc aac ctc gac gcc aac gcc gag     720
   Met Glu Lys Asp Asp Ile Leu Phe Arg Asn Leu Asp Ala Asn Ala Glu
   225                 230                 235                 240
   ttc gtg cgc gcc gac ctc gac tac cgc ggc aag act atc cgc aac gtc       768
   Phe Val Arg Ala Asp Leu Asp Tyr Arg Gly Lys Thr Ile Arg Asn Val
                   245                 250                 255
   acc aag tgc act ggc tac agc atg ctc ggc gag atg ggc gtc cac cgc       816
   Thr Lys Cys Thr Gly Tyr Ser Met Leu Gly Glu Met Gly Val His Arg
               260                 265                 270
   ttc acc ttt gag ctc tac gtc gat gat gtg ctc ttt tac aag ggc tcg       864
   Phe Thr Phe Glu Leu Tyr Val Asp Asp Val Leu Phe Tyr Lys Gly Ser
           275                 280                 285
   acc tcg ttc ggc tgg ttc gtg ccc gag gtc ttt gcc gcc cag gcc ggc       912
   Thr Ser Phe Gly Trp Phe Val Pro Glu Val Phe Ala Ala Gln Ala Gly
       290                 295                 300
   ctc gac aac ggc cgc aag tcg gag ccc tgg ttc att gag aac aag gtt       960
   Leu Asp Asn Gly Arg Lys Ser Glu Pro Trp Phe Ile Glu Asn Lys Val
   305                 310                 315                 320
   ccg gcc tcg cag gtc tcc tcc ttt gac gtg cgc ccc aac ggc agc ggc      1008
   Pro Ala Ser Gln Val Ser Ser Phe Asp Val Arg Pro Asn Gly Ser Gly
                   325                 330                 335
   cgc acc gcc atc ttc gcc aac gcc ccc agc ggc gcc cag ctc aac cgc      1056
   Arg Thr Ala Ile Phe Ala Asn Ala Pro Ser Gly Ala Gln Leu Asn Arg
               340                 345                 350
   cgc acg gac cag ggc cag tac ctc gac gcc gtc gac att gtc tcc ggc      1104
   Arg Thr Asp Gln Gly Gln Tyr Leu Asp Ala Val Asp Ile Val Ser Gly
           355                 360                 365
   agc ggc aag aag agc ctc ggc tac gcc cac ggt tcc aag acg gtc aac      1152
   Ser Gly Lys Lys Ser Leu Gly Tyr Ala His Gly Ser Lys Thr Val Asn
       370                 375                 380
   ccg aac gac tgg ttc ttc tcg tgc cac ttt tgg ttt gac tcg gtc atg      1200
   Pro Asn Asp Trp Phe Phe Ser Cys His Phe Trp Phe Asp Ser Val Met
   385                 390                 395                 400
   ccc gga agt ctc ggt gtc gag tcc atg ttc cag ctc gtc gag gcc atc      1248
   Pro Gly Ser Leu Gly Val Glu Ser Met Phe Gln Leu Val Glu Ala Ile
                   405                 410                 415
   gcc gcc cac gag gat ctc gct ggc aaa gca cgg cat tgc caa ccc cac      1296
   Ala Ala His Glu Asp Leu Ala Gly Lys Ala Arg His Cys Gln Pro His
               420                 425                 430
   ctt tgt gca cgc ccc cgg gca aga tca agc tgg aag tac cgc ggc cag      1344
   Leu Cys Ala Arg Pro Arg Ala Arg Ser Ser Trp Lys Tyr Arg Gly Gln
           435                 440                 445
   ctc acg ccc aag agc aag aag atg gac tcg gag gtc cac atc gtg tcc      1392
   Leu Thr Pro Lys Ser Lys Lys Met Asp Ser Glu Val His Ile Val Ser
       450                 455                 460
   gtg gac gcc cac gac ggc gtt gtc gac ctc gtc gcc gac ggc ttc ctc      1440
   Val Asp Ala His Asp Gly Val Val Asp Leu Val Ala Asp Gly Phe Leu
   465                 470                 475                 480
   tgg gcc gac agc ctc cgc gtc tac tcg gtg agc aac att cgc gtg cgc      1488
   Trp Ala Asp Ser Leu Arg Val Tyr Ser Val Ser Asn Ile Arg Val Arg
                   485                 490                 495
   atc gcc tcc ggt                                                      1500
   Ile Ala Ser Gly
               500
<210> 30
   <211> 500
   <212> PRT
   <213> Schizochytrium sp.
<400> 30
   Lys Val Gln Pro Val Phe Ala Asn Gly Ala Ala Thr Val Gly Pro Glu
   1               5                   10                  15
   Ala Ser Lys Ala Ser Ser Gly Ala Ser Ala Ser Ala Ser Ala Ala Pro
               20                  25                  30
   Ala Lys Pro Ala Phe Ser Ala Asp Val Leu Ala Pro Lys Pro Val Ala
           35                  40                  45
   Leu Pro Glu His Ile Leu Lys Gly Asp Ala Leu Ala Pro Lys Glu Met
       50                  55                  60
   Ser Trp His Pro Met Ala Arg Ile Pro Gly Asn Pro Thr Pro Ser Phe
   65                  70                  75                  80
   Ala Pro Ser Ala Tyr Lys Pro Arg Asn Ile Ala Phe Thr Pro Phe Pro
                   85                  90                  95
   Gly Asn Pro Asn Asp Asn Asp His Thr Pro Gly Lys Met Pro Leu Thr
               100                 105                 110
   Trp Phe Asn Met Ala Glu Phe Met Ala Gly Lys Val Ser Met Cys Leu
           115                 120                 125
   Gly Pro Glu Phe Ala Lys Phe Asp Asp Ser Asn Thr Ser Arg Ser Pro
       130                 135                 140
   Ala Trp Asp Leu Ala Leu Val Thr Arg Ala Val Ser Val Ser Asp Leu
   145                 150                 155                 160
   Lys His Val Asn Tyr Arg Asn Ile Asp Leu Asp Pro Ser Lys Gly Thr
                   165                 170                 175
   Met Val Gly Glu Phe Asp Cys Pro Ala Asp Ala Trp Phe Tyr Lys Gly
               180                 185                 190
   Ala Cys Asn Asp Ala His Met Pro Tyr Ser Ile Leu Met Glu Ile Ala
           195                 200                 205
   Leu Gln Thr Ser Gly Val Leu Thr Ser Val Leu Lys Ala Pro Leu Thr
       210                 215                 220
   Met Glu Lys Asp Asp Ile Leu Phe Arg Asn Leu Asp Ala Asn Ala Glu
   225                 230                 235                 240
   Phe Val Arg Ala Asp Leu Asp Tyr Arg Gly Lys Thr Ile Arg Asn Val
                   245                 250                 255
   Thr Lys Cys Thr Gly Tyr Ser Met Leu Gly Glu Met Gly Val His Arg
               260                 265                 270
   Phe Thr Phe Glu Leu Tyr Val Asp Asp Val Leu Phe Tyr Lys Gly Ser
   275 280 285
   Thr Ser Phe Gly Trp Phe Val Pro Glu Val Phe Ala Ala Gln Ala Gly
   290 295 300
   Leu Asp Asn Gly Arg Lys Ser Glu Pro Trp Phe Ile Glu Asn Lys Val
   305 310 315 320
   Pro Ala Ser Gln Val Ser Ser Phe Asp Val Arg Pro Asn Gly Ser Gly
   325 330 335
   Arg Thr Ala Ile Phe Ala Asn Ala Pro Ser Gly Ala Gln Leu Asn Arg
   340 345 350
   Arg Thr Asp Gln Gly Gln Tyr Leu Asp Ala Val Asp Ile Val Ser Gly
   355 360 365
   Ser Gly Lys Lys Ser Leu Gly Tyr Ala His Gly Ser Lys Thr Val Asn
   370 375 380
   Pro Asn Asp Trp Phe Phe Ser Cys His Phe Trp Phe Asp Ser Val Met
   385 390 395 400
   Pro Gly Ser Leu Gly Val Glu Ser Met Phe Gln Leu Val Glu Ala Ile
   405 410 415
   Ala Ala His Glu Asp Leu Ala Gly Lys Ala Arg His Cys Gln Pro His
   420 425 430
   Leu Cys Ala Arg Pro Arg Ala Arg Ser Ser Trp Lys Tyr Arg Gly Gln
   435 440 445
   Leu Thr Pro Lys Ser Lys Lys Met Asp Ser Glu Val His Ile Val Ser
   450 455 460
   Val Asp Ala His Asp Gly Val Val Asp Leu Val Ala Asp Gly Phe Leu
   465 470 475 480
   Trp Ala Asp Ser Leu Arg Val Tyr Ser Val Ser Asn Ile Arg Val Arg
   485 490 495
   Ile Ala Ser Gly
   500
<210> 31
   <211> 1512
   <212> DNA
   <213> Schizochytrium sp.
<220>
   <221> CDS
   <222> (1)..(1512)
<400> 31
   gcc ccg ctc tac ctc tcg cag gac ccg acc agc ggc cag ctc aag aag        48
   Ala Pro Leu Tyr Leu Ser Gln Asp Pro Thr Ser Gly Gln Leu Lys Lys
   1               5                   10                  15
   cac acc gac gtg gcc tcc ggc cag gcc acc atc gtg cag ccc tgc acg        96
   His Thr Asp Val Ala Ser Gly Gln Ala Thr Ile Val Gln Pro Cys Thr
               20                  25                  30
   ctc ggc gac ctc ggt gac cgc tcc ttc atg gag acc tac ggc gtc gtc       144
   Leu Gly Asp Leu Gly Asp Arg Ser Phe Met Glu Thr Tyr Gly Val Val
           35                  40                  45
   gcc ccg ctg tac acg ggc gcc atg gcc aag ggc att gcc tcg gcg gac       192
   Ala Pro Leu Tyr Thr Gly Ala Met Ala Lys Gly Ile Ala Ser Ala Asp
       50                  55                  60
   ctc gtc atc gcc gcc ggc aag cgc aag atc ctc ggc tcc ttt ggc gcc       240
   Leu Val Ile Ala Ala Gly Lys Arg Lys Ile Leu Gly Ser Phe Gly Ala
   65                  70                  75                  80
   ggc ggc ctc ccc atg cac cac gtg cgc gcc gcc ctc gag aag atc cag       288
   Gly Gly Leu Pro Met His His Val Arg Ala Ala Leu Glu Lys Ile Gln
                   85                  90                  95
   gcc gcc ctg cct cag ggc ccc tac gcc gtc aac ctc atc cac tcg cct       336
   Ala Ala Leu Pro Gln Gly Pro Tyr Ala Val Asn Leu Ile His Ser Pro
               100                 105                 110
   ttt gac agc aac ctc gag aag ggc aac gtc gat ctc ttc ctc gag aag       384
   Phe Asp Ser Asn Leu Glu Lys Gly Asn Val Asp Leu Phe Leu Glu Lys
           115                 120                 125
   ggc gtc act gtg gtg gag gcc tcg gca ttc atg acc ctc acc ccg cag       432
   Gly Val Thr Val Val Glu Ala Ser Ala Phe Met Thr Leu Thr Pro Gln
       130                 135                 140
   gtc gtg cgc tac cgc gcc gcc ggc ctc tcg cgc aac gcc gac ggt tcg       480
   Val Val Arg Tyr Arg Ala Ala Gly Leu Ser Arg Asn Ala Asp Gly Ser
   145                 150                 155                 160
   gtc aac atc cgc aac cgc atc atc ggc aag gtc tcg cgc acc gag ctc       528
   Val Asn Ile Arg Asn Arg Ile Ile Gly Lys Val Ser Arg Thr Glu Leu
                   165                 170                 175
   gcc gag atg ttc atc cgc ccg gcc ccg gag cac ctc ctc gag aag ctc       576
   Ala Glu Met Phe Ile Arg Pro Ala Pro Glu His Leu Leu Glu Lys Leu
               180                 185                 190
   atc gcc tcg ggc gag atc acc cag gag cag gcc gag ctc gcg cgc cgc       624
   Ile Ala Ser Gly Glu Ile Thr Gln Glu Gln Ala Glu Leu Ala Arg Arg
           195                 200                 205
   gtt ccc gtc gcc gac gat atc gct gtc gag gct gac tcg ggc ggc cac       672
   Val Pro Val Ala Asp Asp Ile Ala Val Glu Ala Asp Ser Gly Gly His
       210                 215                 220
   acc gac aac cgc ccc atc cac gtc atc ctc ccg ctc atc atc aac ctc       720
   Thr Asp Asn Arg Pro Ile His Val Ile Leu Pro Leu Ile Ile Asn Leu
   225                 230                 235                 240
   cgc aac cgc ctg cac cgc gag tgc ggc tac ccc gcg cac ctc cgc gtc       768
   Arg Asn Arg Leu His Arg Glu Cys Gly Tyr Pro Ala His Leu Arg Val
                   245                 250                 255
   cgc gtt ggc gcc ggc ggt ggc gtc ggc tgc ccg cag gcc gcc gcc gcc       816
   Arg Val Gly Ala Gly Gly Gly Val Gly Cys Pro Gln Ala Ala Ala Ala
               260                 265                 270
   gcg ctc acc atg ggc gcc gcc ttc atc gtc acc ggc act gtc aac cag       864
   Ala Leu Thr Met Gly Ala Ala Phe Ile Val Thr Gly Thr Val Asn Gln
           275                 280                 285
   gtc gcc aag cag tcc ggc acc tgc gac aac gtg cgc aag cag ctc tcg       912
   Val Ala Lys Gln Ser Gly Thr Cys Asp Asn Val Arg Lys Gln Leu Ser
       290                 295                 300
   cag gcc acc tac tcg gat atc tgc atg gcc ccg gcc gcc gac atg ttc       960
   Gln Ala Thr Tyr Ser Asp Ile Cys Met Ala Pro Ala Ala Asp Met Phe
   305                 310                 315                 320
   gag gag ggc gtc aag ctc cag gtc ctc aag aag gga acc atg ttc ccc      1008
   Glu Glu Gly Val Lys Leu Gln Val Leu Lys Lys Gly Thr Met Phe Pro
                   325                 330                 335
   tcg cgc gcc aac aag ctc tac gag ctc ttt tgc aag tac gac tcc ttc      1056
   Ser Arg Ala Asn Lys Leu Tyr Glu Leu Phe Cys Lys Tyr Asp Ser Phe
               340                 345                 350
   gac tcc atg cct cct gcc gag ctc gag cgc atc gag aag cgt atc ttc      1104
   Asp Ser Met Pro Pro Ala Glu Leu Glu Arg Ile Glu Lys Arg Ile Phe
           355                 360                 365
   aag cgc gca ctc cag gag gtc tgg gag gag acc aag gac ttt tac att      1152
   Lys Arg Ala Leu Gln Glu Val Trp Glu Glu Thr Lys Asp Phe Tyr Ile
       370                 375                 380
   aac ggt ctc aag aac ccg gag aag atc cag cgc gcc gag cac gac ccc      1200
   Asn Gly Leu Lys Asn Pro Glu Lys Ile Gln Arg Ala Glu His Asp Pro
   385                 390                 395                 400
   aag ctc aag atg tcg ctc tgc ttc cgc tgg tac ctt ggt ctt gcc agc      1248
   Lys Leu Lys Met Ser Leu Cys Phe Arg Trp Tyr Leu Gly Leu Ala Ser
                   405                 410                 415
   cgc tgg gcc aac atg ggc gcc ccg gac cgc gtc atg gac tac cag gtc      1296
   Arg Trp Ala Asn Met Gly Ala Pro Asp Arg Val Met Asp Tyr Gln Val
               420                 425                 430
   tgg tgt ggc ccg gcc att ggc gcc ttc aac gac ttc atc aag ggc acc      1344
   Trp Cys Gly Pro Ala Ile Gly Ala Phe Asn Asp Phe Ile Lys Gly Thr
           435                 440                 445
   tac ctc gac ccc gct gtc tcc aac gag tac ccc tgt gtc gtc cag atc      1392
   Tyr Leu Asp Pro Ala Val Ser Asn Glu Tyr Pro Cys Val Val Gln Ile
       450                 455                 460
   aac ctg caa atc ctc cgt ggt gcc tgc tac ctg cgc cgt ctc aac gcc      1440
   Asn Leu Gln Ile Leu Arg Gly Ala Cys Tyr Leu Arg Arg Leu Asn Ala
   465                 470                 475                 480
   ctg cgc aac gac ccg cgc att gac ctc gag acc gag gat gct gcc ttt      1488
   Leu Arg Asn Asp Pro Arg Ile Asp Leu Glu Thr Glu Asp Ala Ala Phe
                   485                 490                 495
   gtc tac gag ccc acc aac gcg ctc                                      1512
   Val Tyr Glu Pro Thr Asn Ala Leu
               500
<210> 32
   <211> 504
   <212> PRT
   <213> Schizochytrium sp.
<400> 32
   Ala Pro Leu Tyr Leu Ser Gln Asp Pro Thr Ser Gly Gln Leu Lys Lys
   1               5                   10                  15
   His Thr Asp Val Ala Ser Gly Gln Ala Thr Ile Val Gln Pro Cys Thr
               20                  25                  30
   Leu Gly Asp Leu Gly Asp Arg Ser Phe Met Glu Thr Tyr Gly Val Val
   35 40 45
   Ala Pro Leu Tyr Thr Gly Ala Met Ala Lys Gly Ile Ala Ser Ala Asp
   50 55 60
   Leu Val Ile Ala Ala Gly Lys Arg Lys Ile Leu Gly Ser Phe Gly Ala
   65 70 75 80
   Gly Gly Leu Pro Met His His Val Arg Ala Ala Leu Glu Lys Ile Gln
   85 90 95
   Ala Ala Leu Pro Gln Gly Pro Tyr Ala Val Asn Leu Ile His Ser Pro
   100 105 110
   Phe Asp Ser Asn Leu Glu Lys Gly Asn Val Asp Leu Phe Leu Glu Lys
   115 120 125
   Gly Val Thr Val Val Glu Ala Ser Ala Phe Met Thr Leu Thr Pro Gln
   130 135 140
   Val Val Arg Tyr Arg Ala Ala Gly Leu Ser Arg Asn Ala Asp Gly Ser
   145 150 155 160
   Val Asn Ile Arg Asn Arg Ile Ile Gly Lys Val Ser Arg Thr Glu Leu
   165 170 175
   Ala Glu Met Phe Ile Arg Pro Ala Pro Glu His Leu Leu Glu Lys Leu
   180 185 190
   Ile Ala Ser Gly Glu Ile Thr Gln Glu Gln Ala Glu Leu Ala Arg Arg
   195 200 205
   Val Pro Val Ala Asp Asp Ile Ala Val Glu Ala Asp Ser Gly Gly His
   210 215 220
   Thr Asp Asn Arg Pro Ile His Val Ile Leu Pro Leu Ile Ile Asn Leu
   225 230 235 240
   Arg Asn Arg Leu His Arg Glu Cys Gly Tyr Pro Ala His Leu Arg Val
   245 250 255
   Arg Val Gly Ala Gly Gly Gly Val Gly Cys Pro Gln Ala Ala Ala Ala
   260 265 270
   Ala Leu Thr Met Gly Ala Ala Phe Ile Val Thr Gly Thr Val Asn Gln
   275 280 285
   Val Ala Lys Gln Ser Gly Thr Cys Asp Asn Val Arg Lys Gln Leu Ser
   290 295 300
   Gln Ala Thr Tyr Ser Asp Ile Cys Met Ala Pro Ala Ala Asp Met Phe
   305 310 315 320
   Glu Glu Gly Val Lys Leu Gln Val Leu Lys Lys Gly Thr Met Phe Pro
   325 330 335
   Ser Arg Ala Asn Lys Leu Tyr Glu Leu Phe Cys Lys Tyr Asp Ser Phe
   340 345 350
   Asp Ser Met Pro Pro Ala Glu Leu Glu Arg Ile Glu Lys Arg Ile Phe
   355 360 365
   Lys Arg Ala Leu Gln Glu Val Trp Glu Glu Thr Lys Asp Phe Tyr Ile
   370 375 380
   Asn Gly Leu Lys Asn Pro Glu Lys Ile Gln Arg Ala Glu His Asp Pro
   385 390 395 400
   Lys Leu Lys Met Ser Leu Cys Phe Arg Trp Tyr Leu Gly Leu Ala Ser
   405 410 415
   Arg Trp Ala Asn Met Gly Ala Pro Asp Arg Val Met Asp Tyr Gln Val
   420 425 430
   Trp Cys Gly Pro Ala Ile Gly Ala Phe Asn Asp Phe Ile Lys Gly Thr
   435 440 445
   Tyr Leu Asp Pro Ala Val Ser Asn Glu Tyr Pro Cys Val Val Gln Ile
   450 455 460
   Asn Leu Gln Ile Leu Arg Gly Ala Cys Tyr Leu Arg Arg Leu Asn Ala
   465 470 475 480
   Leu Arg Asn Asp Pro Arg Ile Asp Leu Glu Thr Glu Asp Ala Ala Phe
   485 490 495
   Val Tyr Glu Pro Thr Asn Ala Leu
   500
<210> 33
   <211> 9
   <212> PRT
   <213> Artificial sequence
<220>
   <223> motif
<220>
   <221> MISC_FEATURE
   <222> (2)..(3)
   <223> Xaa = any amino acid
<220>
   <221> MISC_FEATURE
   <222> (6)..(6)
   <223> Xaa = Ala or Ser
<220>
   <221> MISC_FEATURE
   <222> (7)..(8)
   <223> Xaa = any amino acid
<400> 33
   Trp Xaa Xaa Lys Glu Xaa Xaa Xaa Lys
   1               5
<210> 34
   <211> 6
   <212> PRT
   <213> Artificial sequence
<220>
   <223> motif
<220>
   <221> MISC_FEATURE
   <222> (1)..(6)
   <223> Xaa = Ile or Leu or Val
<400> 34
   Phe Asn Xaa Ser His Ser
   1               5
<210> 35
   <211> 5
   <212> PRT
   <213> Artificial sequence
<220>
   <223> motif
<220>
   <221> MISC_FEATURE
   <222> (1)..(5)
   <223> Xaa = Ile or Leu or Val
<400> 35
   Xaa Gly Xaa Asp Xaa
   1               5
<210> 36
   <211> 4244
   <212> DNA
   <213> Schizochytrium sp.
<400> 36
   tttctctctc tcgagctgtt gctgctgctg ctgctgctgc tgcttccttg ctggttctca     60
   cgtccgttcg atcaagcgct cgctcgctcg accgatcggt gcgtgcgtgc gtgcgtgagt    120
   cttgttgcca ggcagccgca ggctgtctgt ctgtttgtgt agttttaccc tcggggttcg    180
   gggtctgcct gcctcccgct cccgcccgcc gccgcccgta tccaccccgc tcgcctccgc    240
   ccatcgggcc tcgcctcctc gcgccgcacg catcgcgcgc atcgcatgca tcatgctgcc    300
   acgcacgggg ggacgcgcgc cccgcgtccc ccgccgccgc cgtcgtcgtc tggcgatgcc    360
   gtcgccgccc tccttccttc cctcgcctcc tcttcctccc gagcccccct gtcttccttc    420
   gcccccgcag cggcgcgcag gaagcgagga gagcggggag gagagaagaa aagaaaagaa    480
   aagaaaagaa aataacagcg ccgtctcgcg cagacgcgcg cggccgcgtg cgaggcggcg    540
   tgatggggct tctcgtggcg cggctgcggc ctggcccggc ctcgcctttg aggtgcaggc    600
   tttgggagag aagagtggga cgcggagaag ataagatggt gccatggcgc aggacggaga 660
   ggttgctgaa acttcttcga gcggcacagg cgatggcgag agaccgacag ctgccggcgc 720
   ggaggggatg gatacctccc gaggctggca tggacgagct ggccgcgcgg atctggctgg 780
   ccgcgcggcg gtgggtccgg aggcgcgagg ttggttttct tcatacctga taccatacgg 840
   tattcattct tcctctccag gaaggaagca agtcacatag agtatcacta gcctaatgat 900
   ggactctatg ttttagggca cgtcggagca gaaggcgcga gcgattcgaa tgcgagcgat 960
   agatacagca cagagacctt gccggcgacg cggatgcagg cgagcacgca cgcaccgcac 1020
   gcacggcagc ggtgcacgcg ctcctcggca gatgcacggt tctgcgccgc gcctttacat 1080
   tttttgattt taggtggtgt gcctgccact ttgaacatca tccacaagtc aacgcagcat 1140
   caagaggcaa gcaagtacat acatccattc gaattcaagt tcaagagacg cagcaacagc 1200
   cgccgctccg ctcaagctgc agctagctgg ctgacagggc tcgctggctg tagtggaaaa 1260
   ttccattcac ttttctgcat ccgcggccag caggcccgta cgcacgttct ctcgtttgtt 1320
   tgttcgttcg tgcgtgcgtg cgtgcgtccc agctgcctgt ctaatctgcc gcgcgatcca 1380
   acgaccctcg gtcgtcgccg caagcgaaac ccgacgccga cctggccaat gccgcaagaa 1440
   tgctaagcgc gcagcaatgc tgagagtaat cttcagccca ccaagtcatt atcgctgccc 1500
   aagtctccat cgcagccaca ttcaggcttt ctctctctct ccctccctct ctttctgccg 1560
   ggagagaagg aaagacccgc cgccgccgcc tctgcgcctg tgacgggctg tccgttgtaa 1620
   gccctcttag acagttccta ggtgccgggc gccgccgcgc ctccgtcgca ggcacacgta 1680
   ggcggccacg ggttcccccc gcaccttcca caccttcttc cccgcagccc ggaccgcgcg 1740
   ccgtctgctt acgcacttcg cgcggccgcc gcccgcgaac ccgagcgcgt gctgtgggcg 1800
   ccgtcttccg gccgcgtcgg aggtcgtccc cgcgccgcgc tactccgggt cctgtgcggt 1860
   acgtacttaa tattaacagt gggacctcgc acaggacctg acggcagcac agacgtcgcc 1920
   gcctcgcatc gctggggacg caggcgaggc atcccggcgc ggccccgcac cggggaggct 1980
   gcggggcggc ctcttccggc cggcggccgc atcaggcgga tgacgcaaga gccctcgcag 2040
   tcgctcgctc gcgggagcgc agcgcggcgc cagcgtggcc aagctcccgc cccttctggc 2100
   tggctgcatg cctgcctgcc tgcctgcctg cgtgcgtgcg tgcgtgcgtg ccttcgtgcg 2160
   tgcctgcctt cgtgcgtgcg tgcgtgagtg cggcggaaga gggatcatgc gaggatcaat 2220
   cacccgccgc acctcgactt ttgaagaagc cgcgatgcga tgcgatgcga tgcgatgcga 2280
   cgcgataccg tgcgaggcta cgaagcgagt ctggccggcc gtcatacaac gcacgttttc 2340
   gagaaggagg gctggcggag gcgtgcatgc cggcgaccat tgcgaacgcg gcgtctcgtg 2400
   gctggcgaag gtgcctggag gatctaacga tcgctgctat gatgctatag ctgtgctgat 2460
   ccccggtcca ttccaccacg tctgtgcctg ccgcctgacc tgcgcttggc tttccttcaa 2520
   gttctcctcc gccgggcctt caggaccgag acgagacctg cagctgcagc tagactcgcg 2580
   ctcgctcgcg gaggattcgc cggccgccgg gccggacggg actcgcgagg tcacacggcc 2640
   gccggcgatc gcgatggctg tgctgacgta ctcgtgcgtg gcagccgtac gtcagcgacg 2700
   ccgcctccgt attgtggatt cgttagttgg ttgttggttg atttgttgat taattttttt 2760
   gttcgtaggc ttggttatag ctaatagttt agtttatact ggtgctcttc ggtgctgatt 2820
   tagctcgact tgggtccaca ccactgcccc tctactgtga atggatcaat ggacgcacga 2880
   cgggccgacg aaagtgcgcg agtgaggtaa cctaagcaac ggcggtcttc agaggggacg 2940
   cacgccctcc gtcgcagtca gtccagacag gcagaaaagc gtcttaggga ccacgcacgc 3000
   acgcacgcac gcacgcacgc ccgcacgcac gctccctccc tcgcgtgcct atttttttag 3060
   gcttccttcc gcacgggcct acctctcgct ccctcgcctc gccgcaccag gcggcagcag 3120
   cgatacctgc cggtgccgcc tccgtcacgc gctcagccgc agctcagccc agccgcgagc 3180
   tagggtttgt tcgtcctgaa ttgtttgatt tgatttgatt tgatttgatc cgatccgatc 3240
   cgatctgatc tgatttgctt tgctttgctt tgtctccctc ccggcgcgga ccaagcgtcc 3300
   gtctgcgcgc cgcagcttcc cttcttctcc cagccctcct tctgctcccg cctctcgcgc 3360
   aagcacgcag cttcgccgcc gcatccggtc ggtcggtcgg tcgatcgacc cgcctgccgc 3420
   tgctgctgtg gccgggcttt tctccatcgg cgactctttc ttctccatac gtcctactac 3480
   gtacatacat actgccggct tcctcctctt ccagcgcggc gacggcggca ggctgcgacg 3540
   tcgtcgccgc cgcgggcgcc gcgcgcgccg ccgccgccgc ccgcgtcgca gggcctcgtc 3600
   gccgccgccg ctccgctccg ctccgaggcc gcgagagggc cgcggcggcg cgatggatgg 3660
   atggatggat ggatggatgg atggattttg ttgatcgatg gcggcgcatg ggcggagatg 3720
   agcgaggacg agcgcgcgag cgcggcagcc ggattcgcag ggcctcgctc gcctcgcgcc 3780
   cgctgccgcg cccgccttgc gagcctgcgc cgcgagcgag cgagcgagcg agcggggctt 3840
   tctttgtctc gcgcgccgct tggcctcgtg tgtcttgtgc ttgcgtagcg ggcgccgcgg 3900
   tggaagatgg ctcattcaat cgacccattc acgcacgcac tccggcgcgc agagaaggcc 3960
   gaggaggagc agcaagcaaa ccaaaagctc tcgcgctcgc ggtctcgggc tcgagcggtc 4020
   tcggagagag agtcttgcgg cgaccaccgg cagcagcagc agcagcagca gcgctgtcga 4080
   gcacgagcac gagcacgagc acgagcacga gcattcgagc aagaggacag acacggttgt 4140
   cagcgcctag ctcgctcgat acagaaagag gcgggttggg cgtaaaaaaa aaggagcacg 4200
   caagccgcca gccagccagc tagctagcca gcctgcctgc caaa 4244
<210> 37
   <211> 3886
   <212> DNA
   <213> Schizochytrium sp.
<220>
   <221> misc_feature
   <222> (1)..(3886)
   <223> n = a, c, g, or t
<400> 37
   gatcttgatt gccaagctct ggattgtcga ttccgatgaa tcgagctctt tgttgtcgag 60
   ctctggcttg ccgagctttc agaaatagac aaaattgccg agttcctgat tgcggggctc 120
   tcgattgcca aggtctggtg gattctcgaa ctctcgattg tcaaaatctt ggtcgtctcg 180
   tcggattctt tcctgatttg ttttgtcaag accttgagat tgtgcaaaac cttgatcgtt 240
   gacaaaccct tgatcgacag cagcctttca tcacgctcag ctcttgtcat tgattatatt 300
   ccccctgaca gccaacacct tgatgcaggg tctcaacctt gatttttgga ggccatcatc 360
   agcatcacgc cccggcactc accctcaaca ttcgacagcc aacgcttttt tttcttcgac 420
   taggatctga gaataaaagc aggtcaccac gaccgtaggc caacgcgaca accatggaaa 480
   taaagtgaca acgaacgact tgcaagttta aatgtaaaga gcagcaattg cccgcccaca 540
   gacaaatgaa agcaggcgcc gagtcttatt tgaggaggtg ggcctgtggc aatgggcgaa 600
   agaaaatcaa ggacaaggag agcaggttac gtaccggtat actggtatac gtacatggat 660
   ggttcttggc aagttgacgg gatgtgtgcg agtgaccgtg gtagttaacg aaagagccgc 720
   aagggcaagg aaagcaagag aatgcagact tttccacagg atggatgggt ccgcagcttg 780
   ccgcatgatg aaacgctgta tttcacctgg cacgtggtgg cgcacgcgcc cacatatgat 840
   cgcggcggcg ggtgtattat acattttccc cctcaggtct actgccatcc ctccatgcgt 900
   cgctcgtgcg aacgacgcaa gcctttcgca tcgtgcagcc tctttctggt aaggcaagag 960
   ctaaacccaa acctaaacga aagaacattt ttacctctct ctctctccca ttggtcgcgt 1020
   gcgctccgcc gctcgctcct cctcctgcca gtgtcgcgcc ctaacttccc ccctccctcc 1080
   ctccctccct ccctccctct ctcctgccac cgcccctctc tccgcgctgc gtgcggtgct 1140
   gccctggacc aatggcatgc tgctgcacgc tcggcggatg acgcaagccg cttcgcaatt 1200
   tccggatcag atctcggcgg ggcgtgcgcc gcggggtcac tgcggacctg ccgcggcccc 1260
   tgcttctttc acatccatca tgtcctccaa acctccgcct cctccacgca cgtacgcacg 1320
   cccgctcgca cgcgcgcact gccgctgcga aagcaagcgc ccgcccgccg cccggcgacg 1380
   ggaaggcggc cgcggtctcc ctccgcggtt gcctcgctcc cgcgcggggc tgggcgggca 1440
   gcagaaggcg ggtggcggcg gcggcttccg tcttcgtcag cggcctacgt cggcggcggc 1500
   gcgcgagact acgcatgccc ttgcgtcatg cgctcgcagg tagccgccgc gggcctagcg 1560
   tttccgctgg cgccgcgcct aagcccccgg cgcgcacggt attgccgcga taccgtacgg 1620
   ccaagaccgc cgcagacgtc ggccctctcg cggccagcca gccagcagcg cagcggagga 1680
   agagcgcgca ggcgcggcgg gagggcggcc gcggagcagc gcagagcggg gcggagcagc 1740
   gcggagcaga acgggcagac tcggagcggg cagggcgggc agagctttgg ggtttaagga 1800
   ccgggttacc ggcgaagtga gcggctgcgg ggagcggctg tgggaggggt gagtacgcaa 1860
   gcacgatgcg agcgagagag agacgctgcc gcgaatcaag aaggtaggcg cgctgcgagg 1920
   cgcggcggcg gagcggagcg agggagaggg agagggagag agagggaggg agacgtcgcc 1980
   gcggcggggc ctggcctggc ctggtttggc ttggtcagcg cggccttgtc cgagcgtgca 2040
   gctggagttg ggtggattca tttggatttt cttttgtttt tgtttttctc tctttcccgg 2100
   aaagtgttgg ccggncggtg ttctttgttt tgatttcttc aaaagttttg gtggttggtt 2160
   ctctctcttg gctctctgtc aggcggtccg gtccacgccc cggcctctcc tctcctctcc 2220
   tctcctctcc tctccgtgcg tatacgtacg tacgtttgta tacgtacata catcccgccc 2280
   gccgtgccgg cgagggtttg ctcagcctgg agcaatgcga tgcgatgcga tgcgatgcga 2340
   cgcgacgcga cgcgagtcac tggttcgcgc tgtggctgtg gcttgcttgc ttacttgctt 2400
   tcgagctctc ccgctttctt ctttccttct cacgccacca ccaacgaaag aagatcggcc 2460
   ccggcacgcc gctgagaagg gctggcggcg atgacggcac gcgcgcccgc tgccacgttg 2520
   gcgctcgctg ctgctgctgc tgctgctgct gctgctgctg ctgctgctgc tgctgcttct 2580
   gcgcgcaggc tttgccacga ggccggcgtg ctggccgctg ccgcttccag tccgcgtgga 2640
   gagatcgaat gagagataaa ctggatggat tcatcgaggg atgaatgaac gatggttgga 2700
   tgcctttttc ctttttcagg tccacagcgg gaagcaggag cgcgtgaatc tgccgccatc 2760
   cgcatacgtc tgcatcgcat cgcatcgcat gcacgcatcg ctcgccggga gccacagacg 2820
   ggcgacaggg cggccagcca gccaggcagc cagccaggca ggcaccagag ggccagagag 2880
   cgcgcctcac gcacgcgccg cagtgcgcgc atcgctcgca gtgcagacct tgattccccg 2940
   cgcggatctc cgcgagcccg aaacgaagag cgccgtacgg gcccatccta gcgtcgcctc 3000
   gcaccgcatc gcatcgcatc gcgttcccta gagagtagta ctcgacgaag gcaccatttc 3060
   cgcgctcctc ttcggcgcga tcgaggcccc cggcgccgcg acgatcgcgg cggccgcggc 3120
   gctggcggcg gccctggcgc tcgcgctggc ggccgccgcg ggcgtctggc cctggcgcgc 3180
   gcgggcgccg caggaggagc ggcagcggct gctcgccgcc agagaagagc gcgccgggcc 3240
   cggggaggga cggggaggag aaggagaagg cgcgcaaggc ggccccgaaa gagaagaccc 3300
   tggacttgaa cgcgaagaag aagaagaagg agaagaagtt gaagaagaag aagaagaagg 3360
   agaggaagtt gaagaagacg aggagcaggc gcgttccaag gcgcgttctc ttccggaggc 3420
   gcgttccagc tgcggcggcg gggcgggctg cggggcgggc gcgggcgcgg gtgcgggcag 3480
   aggggacgcg cgcgcggagg cggagggggc cgagcgggag cccctgctgc tgcggggcgc 3540
   ccgggccgca ggtgtggcgc gcgcgacgac ggaggcgacg acgccagcgg ccgcgacgac 3600
   aaggccggcg gcgtcggcgg gcggaaggcc ccgcgcggag caggggcggg agcaggacaa 3660
   ggcgcaggag caggagcagg gccgggagcg ggagcgggag cgggcggcgg agcccgaggc 3720
   agaacccaat cgagatccag agcgagcaga ggccggccgc gagcccgagc ccgcgccgca 3780
   gatcactagt accgctgcgg aatcacagca gcagcagcag cagcagcagc agcagcagca 3840
   gcagcagcag ccacgagagg gagataaaga aaaagcggca gagacg 3886
<210> 38
   <211> 8436
   <212> DNA
   <213> Thraustochytrium sp.
<220>
   <221> CDS
   <222> (1)..(8433)
<400> 38
   atg aag gac atg gaa gat aga cgg gtc gct att gtg ggc atg tca gct       48
   Met Lys Asp Met Glu Asp Arg Arg Val Ala Ile Val Gly Met Ser Ala
   1               5                   10                  15
   cac ttg cct tgt ggg aca gat gtg aag gaa tca tgg cag gct att cgc       96
   His Leu Pro Cys Gly Thr Asp Val Lys Glu Ser Trp Gln Ala Ile Arg
               20                  25                  30
   gat gga atc gac tgt cta agt gac cta ccc gcg gat cgt ctc gac gtt      144
   Asp Gly Ile Asp Cys Leu Ser Asp Leu Pro Ala Asp Arg Leu Asp Val
           35                  40                  45
   aca gct tac tac aat ccc aac aaa gcc acg aaa gac aag atc tac tgc      192
   Thr Ala Tyr Tyr Asn Pro Asn Lys Ala Thr Lys Asp Lys Ile Tyr Cys
       50                  55                  60
   aaa cgg ggt ggc ttc atc ccg aac tat gac ttc gac ccc cgc gaa ttt      240
   Lys Arg Gly Gly Phe Ile Pro Asn Tyr Asp Phe Asp Pro Arg Glu Phe
   65                  70                  75                  80
   ggg ctc aac atg ttt caa atg gaa gac tct gat gcg aat cag aca ctt      288
   Gly Leu Asn Met Phe Gln Met Glu Asp Ser Asp Ala Asn Gln Thr Leu
                   85                  90                  95
   acc ttg ctc aaa gtc aaa caa gct ctc gaa gat gca agc ata gag cct      336
   Thr Leu Leu Lys Val Lys Gln Ala Leu Glu Asp Ala Ser Ile Glu Pro
               100                 105                 110
   ttc acc aag gag aag aag aac att gga tgt gtt tta ggt att ggt ggg      384
   Phe Thr Lys Glu Lys Lys Asn Ile Gly Cys Val Leu Gly Ile Gly Gly
           115                 120                 125
   ggc caa aag gcg agt cat gag ttc tac tct cgt ctc aac tac gtt gtc      432
   Gly Gln Lys Ala Ser His Glu Phe Tyr Ser Arg Leu Asn Tyr Val Val
       130                 135                 140
   gtt gaa aag gta ctt cgg aaa atg ggt tta cca gat gct gat gtt gaa      480
   Val Glu Lys Val Leu Arg Lys Met Gly Leu Pro Asp Ala Asp Val Glu
   145                 150                 155                 160
   gaa gct gtg gag aaa tac aag gca aat ttt ccc gag tgg cgc cta gac      528
   Glu Ala Val Glu Lys Tyr Lys Ala Asn Phe Pro Glu Trp Arg Leu Asp
                   165                 170                 175
   tct ttc cct ggg ttt ctt ggg aat gta acg gct ggt cgg tgc agt aac      576
   Ser Phe Pro Gly Phe Leu Gly Asn Val Thr Ala Gly Arg Cys Ser Asn
               180                 185                 190
   acc ttc aac atg gaa ggt atg aac tgc gtt gtg gat gct gca tgt gcc      624
   Thr Phe Asn Met Glu Gly Met Asn Cys Val Val Asp Ala Ala Cys Ala
           195                 200                 205
   agt tct cta att gca atc aag gtt gca gtt gaa gag cta ctc ttt ggt      672
   Ser Ser Leu Ile Ala Ile Lys Val Ala Val Glu Glu Leu Leu Phe Gly
       210                 215                 220
   gac tgt gac acc atg att gca ggt gcc acc tgc acg gac aat tca ctt      720
   Asp Cys Asp Thr Met Ile Ala Gly Ala Thr Cys Thr Asp Asn Ser Leu
   225                 230                 235                 240
   ggc atg tac atg gcc ttc tct aaa acg cca gtt ttt tct act gac cca      768
   Gly Met Tyr Met Ala Phe Ser Lys Thr Pro Val Phe Ser Thr Asp Pro
                   245                 250                 255
   agt gtc cgc gcg tat gat gag aaa aca aaa ggg atg cta att gga gaa      816
   Ser Val Arg Ala Tyr Asp Glu Lys Thr Lys Gly Met Leu Ile Gly Glu
               260                 265                 270
   ggt tca gca atg ttc gtt ctt aaa cgc tat gcg gat gcc gta cgt gat      864
   Gly Ser Ala Met Phe Val Leu Lys Arg Tyr Ala Asp Ala Val Arg Asp
           275                 280                 285
   ggc gac aca att cac gcg gtt ctg cgt tct tgc tct tcg tct agt gat      912
   Gly Asp Thr Ile His Ala Val Leu Arg Ser Cys Ser Ser Ser Ser Asp
       290                 295                 300
   gga aaa gcg gca gga att tat act cct act ata tct gga caa gaa gaa      960
   Gly Lys Ala Ala Gly Ile Tyr Thr Pro Thr Ile Ser Gly Gln Glu Glu
   305                 310                 315                 320
   gct ttg cgt cga gcg tat gcc cgt gcg ggg gta tgt cca tct acg atc      1008
   Ala Leu Arg Arg Ala Tyr Ala Arg Ala Gly Val Cys Pro Ser Thr Ile
                   325                 330                 335
   ggg ctt gtt gag ggt cac ggg aca ggg acc cct gtt gga gat cgc att      1056
   Gly Leu Val Glu Gly His Gly Thr Gly Thr Pro Val Gly Asp Arg Ile
               340                 345                 350
   gag tta aca gct ctg cgg aac ttg ttt gac aaa gct ttt ggt agc aag      1104
   Glu Leu Thr Ala Leu Arg Asn Leu Phe Asp Lys Ala Phe Gly Ser Lys
           355                 360                 365
   aag gaa caa ata gca gtt ggc agc ata aag tct cag ata ggt cac ctg      1152
   Lys Glu Gln Ile Ala Val Gly Ser Ile Lys Ser Gln Ile Gly His Leu
       370                 375                 380
   aaa tct gtt gcc ggc ttt gcc ggc ttg gtc aaa gct gtg ctt gcg ctt      1200
   Lys Ser Val Ala Gly Phe Ala Gly Leu Val Lys Ala Val Leu Ala Leu
   385                 390                 395                 400
   aaa cac aaa acg ctc cca ggt tcg att aat gtc gac cag cca cct ttg      1248
   Lys His Lys Thr Leu Pro Gly Ser Ile Asn Val Asp Gln Pro Pro Leu
                   405                 410                 415
   ttg tat gac ggt act caa att caa gac tct tct tta tat atc aac aag      1296
   Leu Tyr Asp Gly Thr Gln Ile Gln Asp Ser Ser Leu Tyr Ile Asn Lys
               420                 425                 430
   aca aat aga cca tgg ttt acg caa aac aag ctt ccg cgt cgg gct ggt      1344
   Thr Asn Arg Pro Trp Phe Thr Gln Asn Lys Leu Pro Arg Arg Ala Gly
           435                 440                 445
   gtc tca agt ttt gga ttt gga ggt gca aac tac cac gcg gtt ctg gaa      1392
   Val Ser Ser Phe Gly Phe Gly Gly Ala Asn Tyr His Ala Val Leu Glu
       450                 455                 460
   gaa ttc gag ccc gag cat gaa aaa cca tac cgc ctc aat act gtt gga      1440
   Glu Phe Glu Pro Glu His Glu Lys Pro Tyr Arg Leu Asn Thr Val Gly
   465                 470                 475                 480
   cat cct gtc ctc ttg tac gct ccg tct gtg gaa gcc ctc aaa gta ctt      1488
   His Pro Val Leu Leu Tyr Ala Pro Ser Val Glu Ala Leu Lys Val Leu
                   485                 490                 495
   tgc aac gac cag ctt gcg gag ctc aca att gca ttg gaa gag gca aaa      1536
   Cys Asn Asp Gln Leu Ala Glu Leu Thr Ile Ala Leu Glu Glu Ala Lys
               500                 505                 510
   aca cat aaa aat gtt gac aaa gtt tgt ggc tac aag ttt att gac gaa      1584
   Thr His Lys Asn Val Asp Lys Val Cys Gly Tyr Lys Phe Ile Asp Glu
           515                 520                 525
   ttt cag ctc caa gga agc tgt cct cca gaa aat ccg aga gta gga ttt      1632
   Phe Gln Leu Gln Gly Ser Cys Pro Pro Glu Asn Pro Arg Val Gly Phe
       530                 535                 540
   tta gca aca ctg cct act tca aat atc att gtc gcg ctt aag gca att      1680
   Leu Ala Thr Leu Pro Thr Ser Asn Ile Ile Val Ala Leu Lys Ala Ile
   545                 550                 555                 560
   ctc gcg cag ctt gat gca aaa cca gat gcg aag aaa tgg gat ttg cct      1728
   Leu Ala Gln Leu Asp Ala Lys Pro Asp Ala Lys Lys Trp Asp Leu Pro
                   565                 570                 575
   cat aaa aag gct ttt ggg gct acc ttc gca tcg tct tca gtg aaa ggc      1776
   His Lys Lys Ala Phe Gly Ala Thr Phe Ala Ser Ser Ser Val Lys Gly
               580                 585                 590
   tct gtt gct gcg ctc ttc gca gga cag ggt acc cag tac tta aac atg      1824
   Ser Val Ala Ala Leu Phe Ala Gly Gln Gly Thr Gln Tyr Leu Asn Met
           595                 600                 605
   ttc tct gat gtg gca atg aac tgg cca ccg ttc cgt gac agc att gtc      1872
   Phe Ser Asp Val Ala Met Asn Trp Pro Pro Phe Arg Asp Ser Ile Val
       610                 615                 620
   gca atg gaa gaa gct caa act gag gta ttt gag ggc caa gtt gaa cca      1920
   Ala Met Glu Glu Ala Gln Thr Glu Val Phe Glu Gly Gln Val Glu Pro
   625                 630                 635                 640
   att agc aaa gtt ctg ttt cca cga gag cgc tat gca tcc gaa agt gaa      1968
   Ile Ser Lys Val Leu Phe Pro Arg Glu Arg Tyr Ala Ser Glu Ser Glu
                   645                 650                 655
   cag ggg aat gaa ctt ctt tgc tta aca gag tac tct cag cca act acg      2016
   Gln Gly Asn Glu Leu Leu Cys Leu Thr Glu Tyr Ser Gln Pro Thr Thr
               660                 665                 670
   ata gca gcc gca gta ggg gcc ttc gat att ttc aaa gcg gct ggc ttt      2064
   Ile Ala Ala Ala Val Gly Ala Phe Asp Ile Phe Lys Ala Ala Gly Phe
           675                 680                 685
   aag cca gac atg gtt gga ggg cat tca ctt ggc gaa ttt gct gct ttg      2112
   Lys Pro Asp Met Val Gly Gly His Ser Leu Gly Glu Phe Ala Ala Leu
       690                 695                 700
   tac gcg gct ggg tcc att tcg cgt gac gac ctg tac aag ctt gtg tgc      2160
   Tyr Ala Ala Gly Ser Ile Ser Arg Asp Asp Leu Tyr Lys Leu Val Cys
   705                 710                 715                 720
   aaa cgg gca aag gca atg gcg aac gct agt gac gga gct atg gca gca      2208
   Lys Arg Ala Lys Ala Met Ala Asn Ala Ser Asp Gly Ala Met Ala Ala
                   725                 730                 735
   gtg att ggc cca gat gca cgt cta gtt acg cca caa aat agt gac gtt      2256
   Val Ile Gly Pro Asp Ala Arg Leu Val Thr Pro Gln Asn Ser Asp Val
               740                 745                 750
   tat gtc gca aac ttc aac tcc gca act caa gta gtc atc agt ggc act      2304
   Tyr Val Ala Asn Phe Asn Ser Ala Thr Gln Val Val Ile Ser Gly Thr
           755                 760                 765
   gtt caa ggt gtg aaa gaa gag tcg aaa ttg ctc att tca aag ggg ttc      2352
   Val Gln Gly Val Lys Glu Glu Ser Lys Leu Leu Ile Ser Lys Gly Phe
       770                 775                 780
   cgc gta ctg cca ctt aaa tgc cag ggc gcc ttc cat tct cct ttg atg      2400
   Arg Val Leu Pro Leu Lys Cys Gln Gly Ala Phe His Ser Pro Leu Met
   785                 790                 795                 800
   ggg cct tct gag gat agt ttc aaa tca ctt gtg gag act tgt acc atc      2448
   Gly Pro Ser Glu Asp Ser Phe Lys Ser Leu Val Glu Thr Cys Thr Ile
                   805                 810                 815
   tcg ccg cca aaa aat gtg aaa ttc ttt tgc aat gtt agt ggc aag gaa      2496
   Ser Pro Pro Lys Asn Val Lys Phe Phe Cys Asn Val Ser Gly Lys Glu
               820                 825                 830
   agc cca aac cca aaa cag acc ctc aag tca cac atg acg tct agc gtt      2544
   Ser Pro Asn Pro Lys Gln Thr Leu Lys Ser His Met Thr Ser Ser Val
           835                 840                 845
   cag ttc gag gag cag att cgt aac atg tac gat gcc gga gca cgt gtt      2592
   Gln Phe Glu Glu Gln Ile Arg Asn Met Tyr Asp Ala Gly Ala Arg Val
       850                 855                 860
   ttt ctg gag ttt gga ccc cgc caa gtc ctt gca aag ctt atc gcg gaa      2640
   Phe Leu Glu Phe Gly Pro Arg Gln Val Leu Ala Lys Leu Ile Ala Glu
   865                 870                 875                 880
   atg ttt ccc tcg tgt aca gct atc agc gtt aac ccc gcg agc agt ggt      2688
   Met Phe Pro Ser Cys Thr Ala Ile Ser Val Asn Pro Ala Ser Ser Gly
                   885                 890                 895
   gac agt gac gtg caa ctc cgc ctc gcc gcc gta aaa ttc gcg gtc tcg      2736
   Asp Ser Asp Val Gln Leu Arg Leu Ala Ala Val Lys Phe Ala Val Ser
               900                 905                 910
   ggt gca gcc ctt agc acc ttt gat cca tgg gag tat cgc aag cca caa      2784
   Gly Ala Ala Leu Ser Thr Phe Asp Pro Trp Glu Tyr Arg Lys Pro Gln
           915                 920                 925
   gat ctt ctt att cga aaa cca cga aaa act gcc ctt gtt cta tca gca      2832
   Asp Leu Leu Ile Arg Lys Pro Arg Lys Thr Ala Leu Val Leu Ser Ala
       930                 935                 940
   gca aca tat gtt tcc cca aag act ctt gca gaa cgt aaa aag gct atg      2880
   Ala Thr Tyr Val Ser Pro Lys Thr Leu Ala Glu Arg Lys Lys Ala Met
   945                 950                 955                 960
   gaa gat atc aag cta gta tcc att aca cca aga gat agt atg gta tca      2928
   Glu Asp Ile Lys Leu Val Ser Ile Thr Pro Arg Asp Ser Met Val Ser
                   965                 970                 975
   att gga aaa atc gcg caa gaa gta cgg aca gct aaa cag cct tta gaa      2976
   Ile Gly Lys Ile Ala Gln Glu Val Arg Thr Ala Lys Gln Pro Leu Glu
               980                 985                 990
   acc gaa att cga aga ctc aac aaa  gaa tta gaa cat ctc  aag aga gag    3024
   Thr Glu Ile Arg Arg Leu Asn Lys  Glu Leu Glu His Leu  Lys Arg Glu
           995                 1000                 1005
   cta gca  gca gcc aaa gcg agt  gtc aag tct gca tca  aaa agc tct      3069
   Leu Ala  Ala Ala Lys Ala Ser  Val Lys Ser Ala Ser  Lys Ser Ser
       1010                 1015                 1020
   aaa gag  cga tct gtc cta tca  aag cac cgc gct ttg  ctt caa aac      3114
   Lys Glu  Arg Ser Val Leu Ser  Lys His Arg Ala Leu  Leu Gln Asn
       1025                 1030                 1035
   att ttg  caa gac tac gat gat  ctt cgt gtg gtg cca  ttc gct gtt      3159
   Ile Leu  Gln Asp Tyr Asp Asp  Leu Arg Val Val Pro  Phe Ala Val
       1040                 1045                 1050
   cgt tct  gtt gca gtg gac aac  acc gcg ccg tat gct  gac caa gtt      3204
   Arg Ser  Val Ala Val Asp Asn  Thr Ala Pro Tyr Ala  Asp Gln Val
       1055                 1060                 1065
   tcg acc  cca gcg tca gag cgg  tcg gct tca ccg ctt  ttc gag aaa      3249
   Ser Thr  Pro Ala Ser Glu Arg  Ser Ala Ser Pro Leu  Phe Glu Lys
       1070                 1075                 1080
   cgc agt  tcg gtt tcg tca gca  cgc ctc gct gaa gct  gaa gcc gcg      3294
   Arg Ser  Ser Val Ser Ser Ala  Arg Leu Ala Glu Ala  Glu Ala Ala
       1085                 1090                 1095
   gta ctg  agc gtt ctc gca gac  aag aca ggc tac gac  agc tca atg      3339
   Val Leu  Ser Val Leu Ala Asp  Lys Thr Gly Tyr Asp  Ser Ser Met
       1100                 1105                 1110
   atc gag  atg gac atg gac ctg  gag agt gag ctt ggc  gtt gat agc      3384
   Ile Glu  Met Asp Met Asp Leu  Glu Ser Glu Leu Gly  Val Asp Ser
       1115                 1120                 1125
   atc aaa  cgc gtg gag atc atg  agc gag gtt caa acg  ctg ctc agc      3429
   Ile Lys  Arg Val Glu Ile Met  Ser Glu Val Gln Thr  Leu Leu Ser
       1130                 1135                 1140
   gtg gaa  gtc tcc gac gtt gac  gct ctg tca aga acc  aag act gtt      3474
   Val Glu  Val Ser Asp Val Asp  Ala Leu Ser Arg Thr  Lys Thr Val
       1145                 1150                 1155
   ggc gac  gtc atc gag gcg atg  aag ctg gaa ctc ggt  gga ccc caa      3519
   Gly Asp  Val Ile Glu Ala Met  Lys Leu Glu Leu Gly  Gly Pro Gln
       1160                 1165                 1170
   ggc cag  act ttg acc gcg gaa  tcg atc cgt cag cca  ccg gtg tcc      3564
   Gly Gln  Thr Leu Thr Ala Glu  Ser Ile Arg Gln Pro  Pro Val Ser
       1175                 1180                 1185
   gag cct  gct gta ccg acc tca  tcg tca agc agt att  gct aat gtt      3609
   Glu Pro  Ala Val Pro Thr Ser  Ser Ser Ser Ser Ile  Ala Asn Val
       1190                 1195                 1200
   tcg tca  gca cgc ctc gct gaa  gct gaa gct gcg gta  ctg agc gtt      3654
   Ser Ser  Ala Arg Leu Ala Glu  Ala Glu Ala Ala Val  Leu Ser Val
       1205                 1210                 1215
   ctc gca  gac aag aca ggc tac  gac agc tca atg atc  gag atg gac      3699
   Leu Ala  Asp Lys Thr Gly Tyr  Asp Ser Ser Met Ile  Glu Met Asp
       1220                 1225                 1230
   atg gac  ctg gag agc gag ctt  ggc gtt gat agc atc  aaa cgc gtg      3744
   Met Asp  Leu Glu Ser Glu Leu  Gly Val Asp Ser Ile  Lys Arg Val
       1235                 1240                 1245
   gag atc  atg agc gag gtt caa  acg ctg ctc agc gtg  gaa gtc tcc      3789
   Glu Ile  Met Ser Glu Val Gln  Thr Leu Leu Ser Val  Glu Val Ser
       1250                 1255                 1260
   gac gtt  gac gct ctg tca aga  act aag act gtt ggc  gac gtc atc      3834
   Asp Val  Asp Ala Leu Ser Arg  Thr Lys Thr Val Gly  Asp Val Ile
       1265                 1270                 1275
   gag gcg  atg aag ctg gaa ctc  ggt gga ccc caa ggc  cag act ttg      3879
   Glu Ala  Met Lys Leu Glu Leu  Gly Gly Pro Gln Gly  Gln Thr Leu
       1280                 1285                 1290
   acc gcg  gaa tcg atc cgt cag  cca ccg gtg tct gag  cct gct gta      3924
   Thr Ala  Glu Ser Ile Arg Gln  Pro Pro Val Ser Glu  Pro Ala Val
       1295                 1300                 1305
   ccg acc  tca tcg tca agc agt  att gct aat gtt tcg  tca gca cgc      3969
   Pro Thr  Ser Ser Ser Ser Ser  Ile Ala Asn Val Ser  Ser Ala Arg
       1310                 1315                 1320
   ctc gct  gaa gct gaa gcg gcg  gta ctg agc gtt ctc  gca gac aag      4014
   Leu Ala  Glu Ala Glu Ala Ala  Val Leu Ser Val Leu  Ala Asp Lys
       1325                 1330                 1335
   aca ggc  tac gac agc tca atg  atc gag atg gac atg  gac ctg gag      4059
   Thr Gly  Tyr Asp Ser Ser Met  Ile Glu Met Asp Met  Asp Leu Glu
       1340                 1345                 1350
   agc gag  ctt ggc gtc gac agc  atc aaa cgc gtg gag  atc atg agc      4104
   Ser Glu  Leu Gly Val Asp Ser  Ile Lys Arg Val Glu  Ile Met Ser
       1355                 1360                 1365
   gag gtt  caa acg ctg ctc agc  gtg gaa gtc tcc gac  gtt gac gct      4149
   Glu Val  Gln Thr Leu Leu Ser  Val Glu Val Ser Asp  Val Asp Ala
       1370                 1375                 1380
   ctg tca  aga acc aag act gtt  ggc gac gtc atc gag  gcg atg aag      4194
   Leu Ser  Arg Thr Lys Thr Val  Gly Asp Val Ile Glu  Ala Met Lys
       1385                 1390                 1395
   ctg gaa  ctc ggt gga ccc caa  ggc cag act ttg acc  gcg gaa tcg      4239
   Leu Glu  Leu Gly Gly Pro Gln  Gly Gln Thr Leu Thr  Ala Glu Ser
       1400                 1405                 1410
   atc cgt  cag cca ccg gtg tcc  gag cct gct gta ccg  acc tca tcg      4284
   Ile Arg  Gln Pro Pro Val Ser  Glu Pro Ala Val Pro  Thr Ser Ser
       1415                 1420                 1425
   tca agc  agt att gct aat gtt  ttg tca gca cgc ctc  gct gaa gct      4329
   Ser Ser  Ser Ile Ala Asn Val  Leu Ser Ala Arg Leu  Ala Glu Ala
       1430                 1435                 1440
   gaa gcc gcg gta ctg agc gtt ctc gca gac aag aca ggc tac gac    4374
   Glu Ala Ala Val Leu Ser Val Leu Ala Asp Lys Thr Gly Tyr Asp
       1445                1450                1455
   agc tca atg atc gag atg gac atg gac ctg gag agc gag ctt ggc    4419
   Ser Ser Met Ile Glu Met Asp Met Asp Leu Glu Ser Glu Leu Gly
       1460                1465                1470
   gtt gat agc atc aaa cgc gtg gag atc atg agc gag gtt caa acg    4464
   Val Asp Ser Ile Lys Arg Val Glu Ile Met Ser Glu Val Gln Thr
       1475                1480                1485
   ttg ctc agc gtg gaa gtc tcc gac gtt gac gct ctg tca aga acc    4509
   Leu Leu Ser Val Glu Val Ser Asp Val Asp Ala Leu Ser Arg Thr
       1490                1495                1500
   aag act gtt ggc gac gtc atc gag gcg atg aag ctg gaa ctc ggt    4554
   Lys Thr Val Gly Asp Val Ile Glu Ala Met Lys Leu Glu Leu Gly
       1505                1510                1515
   gga ccc caa ggc cag act ttg acc gcg gaa tcg atc cgt cag cca    4599
   Gly Pro Gln Gly Gln Thr Leu Thr Ala Glu Ser Ile Arg Gln Pro
       1520                1525                1530
   ccg gtg tct gag cct gct gta ccg acc tca tcg tca agc agt att    4644
   Pro Val Ser Glu Pro Ala Val Pro Thr Ser Ser Ser Ser Ser Ile
       1535                1540                1545
   gct aat gtt tcg tca gca cgc ctc gct gaa gct gaa gcc gcg gta    4689
   Ala Asn Val Ser Ser Ala Arg Leu Ala Glu Ala Glu Ala Ala Val
       1550                1555                1560
   ctg agc gtt ctc gca gac aag aca ggc tac gac agc tca atg atc    4734
   Leu Ser Val Leu Ala Asp Lys Thr Gly Tyr Asp Ser Ser Met Ile
       1565                1570                1575
   gag atg gac atg gac ctg gag agt gag ctt ggc gtc gac agc atc    4779
   Glu Met Asp Met Asp Leu Glu Ser Glu Leu Gly Val Asp Ser Ile
       1580                1585                1590
   aaa cgc gtg gag atc atg agc gag gtt caa acg ctg ctc agc gtg    4824
   Lys Arg Val Glu Ile Met Ser Glu Val Gln Thr Leu Leu Ser Val
       1595                1600                1605
   gaa gtc tcc gac gtt gac gct ctg tca aga acc aag act gtt ggc    4869
   Glu Val Ser Asp Val Asp Ala Leu Ser Arg Thr Lys Thr Val Gly
       1610                1615                1620
   gac gtc atc gag gcg atg aag ctg gaa ctc ggt gga ccc caa ggc    4914
   Asp Val Ile Glu Ala Met Lys Leu Glu Leu Gly Gly Pro Gln Gly
       1625                1630                1635
   cag act ttg acc tct gaa ccg atc cat cag cca cca gtg tcc gag    4959
   Gln Thr Leu Thr Ser Glu Pro Ile His Gln Pro Pro Val Ser Glu
       1640                1645                1650
   cct gct gta ccg acc tca tcg tca agc agt att gct aat gtt tct    5004
   Pro Ala Val Pro Thr Ser Ser Ser Ser Ser Ile Ala Asn Val Ser
       1655                1660                1665
   tca gca cgc ctc gct gaa gct gaa gcc gcg gta ctg agc gtt ctc    5049
   Ser Ala Arg Leu Ala Glu Ala Glu Ala Ala Val Leu Ser Val Leu
       1670                1675                1680
   gca gac aag aca ggc tac gac agc tca atg atc gag atg gac atg    5094
   Ala Asp Lys Thr Gly Tyr Asp Ser Ser Met Ile Glu Met Asp Met
       1685                1690                1695
   gac ctg gag agc gag ctt ggc gtt gat agc atc aaa cgc gtg gaa    5139
   Asp Leu Glu Ser Glu Leu Gly Val Asp Ser Ile Lys Arg Val Glu
       1700                1705                1710
   atc atg  agc gag gtt caa acg  ctg ctc agc gtg gaa  gtc tcc gac       5184
   Ile Met  Ser Glu Val Gln Thr  Leu Leu Ser Val Glu  Val Ser Asp
       1715                 1720                 1725
   gtt gac  gct ctg tca aga acc  aag act gtt ggc gac  gtc atc gag       5229
   Val Asp  Ala Leu Ser Arg Thr  Lys Thr Val Gly Asp  Val Ile Glu
       1730                 1735                 1740
   gcg atg  aag atg gaa ctc ggt  gga ccc caa ggc cag  act ttg acc       5274
   Ala Met  Lys Met Glu Leu Gly  Gly Pro Gln Gly Gln  Thr Leu Thr
       1745                 1750                 1755
   gcg gaa  tcg atc cgt cag cca  ccg gtg tct gag cct  gct gta ccg       5319
   Ala Glu  Ser Ile Arg Gln Pro  Pro Val Ser Glu Pro  Ala Val Pro
       1760                 1765                 1770
   acc tca  tcg tca agc agt att  gct aat gtt tcg tca  gca cgc ctc       5364
   Thr Ser  Ser Ser Ser Ser Ile  Ala Asn Val Ser Ser  Ala Arg Leu
       1775                 1780                 1785
   gct gaa  gct gaa gcg gcg gta  ctg agc gtt ctc gca  gac aag aca       5409
   Ala Glu  Ala Glu Ala Ala Val  Leu Ser Val Leu Ala  Asp Lys Thr
       1790                 1795                 1800
   ggc tac  gac agc tca atg atc  gag atg gac atg gac  ctg gag agc       5454
   Gly Tyr  Asp Ser Ser Met Ile  Glu Met Asp Met Asp  Leu Glu Ser
       1805                 1810                 1815
   gag ctt  ggc gtt gat agc atc  aaa cgc gtg gag atc  atg agc gag       5499
   Glu Leu  Gly Val Asp Ser Ile  Lys Arg Val Glu Ile  Met Ser Glu
       1820                 1825                 1830
   gtt caa  gcg ctg ctc agc gtg  gaa gtc tcc gac gtt  gac gct ctg       5544
   Val Gln  Ala Leu Leu Ser Val  Glu Val Ser Asp Val  Asp Ala Leu
       1835                 1840                 1845
   tca aga  acc aag act gtt ggc  gac gtc atc gag gcg  atg aag atg       5589
   Ser Arg  Thr Lys Thr Val Gly  Asp Val Ile Glu Ala  Met Lys Met
       1850                 1855                 1860
   gaa ctc  ggt gga ccc caa ggc  cag act ttg acc gca  gaa tcg atc       5634
   Glu Leu  Gly Gly Pro Gln Gly  Gln Thr Leu Thr Ala  Glu Ser Ile
       1865                 1870                 1875
   cgt gag  cca ccg gtg tct gag  cct gct gta ccg acc  tca tcg tca       5679
   Arg Glu  Pro Pro Val Ser Glu  Pro Ala Val Pro Thr  Ser Ser Ser
       1880                 1885                 1890
   agt agt  atc gct aat gtt tct  tca gct cgc ctc gct  gaa gct gaa       5724
   Ser Ser  Ile Ala Asn Val Ser  Ser Ala Arg Leu Ala  Glu Ala Glu
       1895                 1900                 1905
   gcc gcg  gta ctg agc gtt ctc  gca gac aag aca ggc  tac gac agc       5769
   Ala Ala  Val Leu Ser Val Leu  Ala Asp Lys Thr Gly  Tyr Asp Ser
       1910                 1915                 1920
   tca atg  atc gag atg gac atg  gac ctg gag agt gag  ctt ggc gtc       5814
   Ser Met  Ile Glu Met Asp Met  Asp Leu Glu Ser Glu  Leu Gly Val
       1925                 1930                 1935
   gac agc  atc aaa cgc gtg gag  atc atg agc gag gtt  caa acg ttg       5859
   Asp Ser  Ile Lys Arg Val Glu  Ile Met Ser Glu Val  Gln Thr Leu
       1940                 1945                 1950
   ctc agc  gtg gaa gtc tcc gac  gtt gac gct ctg tca  aga acc aag       5904
   Leu Ser  Val Glu Val Ser Asp  Val Asp Ala Leu Ser  Arg Thr Lys
       1955                 1960                 1965
   act gtt  ggc gac gtc atc gag  gcg atg aag ctg gaa  ctt ggg gaa       5949
   Thr Val  Gly Asp Val Ile Glu  Ala Met Lys Leu Glu  Leu Gly Glu
       1970                 1975                 1980
   tca tca  agt att gag act ctc  aat tgt acc gag gtt  gag cac acg       5994
   Ser Ser  Ser Ile Glu Thr Leu  Asn Cys Thr Glu Val  Glu His Thr
       1985                 1990                 1995
   agc tac  aaa agt gtc aag gct  tca ggg tgt gag aat  gta gat acc       6039
   Ser Tyr  Lys Ser Val Lys Ala  Ser Gly Cys Glu Asn  Val Asp Thr
       2000                 2005                 2010
   cgt ttc  gct aag gtt gta caa  atc tcg ctt cct agc  aag ctg aaa       6084
   Arg Phe  Ala Lys Val Val Gln  Ile Ser Leu Pro Ser  Lys Leu Lys
       2015                 2020                 2025
   tcc act  gtg tcg cac gat cga  cct gta att gtt gta  gat gat gga       6129
   Ser Thr  Val Ser His Asp Arg  Pro Val Ile Val Val  Asp Asp Gly
       2030                 2035                 2040
   acg ccc  tta acc acg gag ctt  tgt aaa att ctt ggg  ggt aat att       6174
   Thr Pro  Leu Thr Thr Glu Leu  Cys Lys Ile Leu Gly  Gly Asn Ile
       2045                 2050                 2055
   gtg gtt  ctc tct tat caa ggg  aag ccc gct ggt cca  cgg gga gtc       6219
   Val Val  Leu Ser Tyr Gln Gly  Lys Pro Ala Gly Pro  Arg Gly Val
       2060                 2065                 2070
   gag gtg  cca gat ctt tcc gag  gaa gcc cta att caa  gct ctt gca       6264
   Glu Val  Pro Asp Leu Ser Glu  Glu Ala Leu Ile Gln  Ala Leu Ala
       2075                 2080                 2085
   ttg att  cgg tct aca tat gga  gtt cca att ggt ttt  att tgt cag       6309
   Leu Ile  Arg Ser Thr Tyr Gly  Val Pro Ile Gly Phe  Ile Cys Gln
       2090                 2095                 2100
   caa gtg  tct aat gtg agc acc  aag gca cag ctt tgt  tgg gca ctc       6354
   Gln Val  Ser Asn Val Ser Thr  Lys Ala Gln Leu Cys  Trp Ala Leu
       2105                 2110                 2115
   ctc gca  gcg aag cat ctc aag  aag gat ttg aat gct  gtc tta ccc       6399
   Leu Ala  Ala Lys His Leu Lys  Lys Asp Leu Asn Ala  Val Leu Pro
       2120                 2125                 2130
   gat tca  aga tcc ttc ttc gtc  gga gtt gta cgc ttg  aac ggg aaa       6444
   Asp Ser  Arg Ser Phe Phe Val  Gly Val Val Arg Leu  Asn Gly Lys
       2135                 2140                 2145
   ctt gga  act ttc gaa aac atc  agc gac ttc tct aaa  ttt gat ttg       6489
   Leu Gly  Thr Phe Glu Asn Ile  Ser Asp Phe Ser Lys  Phe Asp Leu
       2150                 2155                 2160
   acg aaa  gcc cta gat tac gga  cag cgt ggt tct ctc  tta ggc ctg       6534
   Thr Lys  Ala Leu Asp Tyr Gly  Gln Arg Gly Ser Leu  Leu Gly Leu
       2165                 2170                 2175
   tgc aag  tca cta gac tta gaa  tgg gaa cag gtg ttt  tgc cgt gga       6579
   Cys Lys  Ser Leu Asp Leu Glu  Trp Glu Gln Val Phe  Cys Arg Gly
       2180                 2185                 2190
   ata gat  ctt gcg tgt gat ctt  atg cca ctc cag gcc  gca agg ata       6624
   Ile Asp  Leu Ala Cys Asp Leu  Met Pro Leu Gln Ala  Ala Arg Ile
       2195                 2200                 2205
   ctc aga  aat gag ctt cag tgt  ccc aat atg cgc ctt  cgc gag gtt       6669
   Leu Arg  Asn Glu Leu Gln Cys  Pro Asn Met Arg Leu  Arg Glu Val
       2210                 2215                 2220
   ggg tac  gat att tct ggc gcc  agg tac acc att tca  acc gat gac       6714
   Gly Tyr  Asp Ile Ser Gly Ala  Arg Tyr Thr Ile Ser  Thr Asp Asp
       2225                 2230                 2235
   ctg cta  tgt gga ccc tcg aag  gct aaa gta gag gcc  gca gac ttg       6759
   Leu Leu  Cys Gly Pro Ser Lys  Ala Lys Val Glu Ala  Ala Asp Leu
       2240                 2245                 2250
   ttt ctt  gtg aca ggt ggc gca  cga ggt att aca cct  cat tgt gtt      6804
   Phe Leu  Val Thr Gly Gly Ala  Arg Gly Ile Thr Pro  His Cys Val
       2255                 2260                 2265
   cgt gag  att gca agt cga tcc  ccc gga acc aca ttt  gtg ctg gtt      6849
   Arg Glu  Ile Ala Ser Arg Ser  Pro Gly Thr Thr Phe  Val Leu Val
       2270                 2275                 2280
   gga aga  agc gaa atg tcc gac  gag cct gac tgg gct  gtt ggc cac      6894
   Gly Arg  Ser Glu Met Ser Asp  Glu Pro Asp Trp Ala  Val Gly His
       2285                 2290                 2295
   tac aat  aaa gac ctg gac caa  agc aca atg aaa cac  ttg aaa gca      6939
   Tyr Asn  Lys Asp Leu Asp Gln  Ser Thr Met Lys His  Leu Lys Ala
       2300                 2305                 2310
   acg cat  gct gct gga ggg gta  aaa cct acg cct aaa  gca cat cgt      6984
   Thr His  Ala Ala Gly Gly Val  Lys Pro Thr Pro Lys  Ala His Arg
       2315                 2320                 2325
   gca ctt  gtg aac agg gtc act  ggc tca cgg gag gta  cga gaa tct      7029
   Ala Leu  Val Asn Arg Val Thr  Gly Ser Arg Glu Val  Arg Glu Ser
       2330                 2335                 2340
   ctt aga  gca atc cag gag gca  ggg gca aat gtc gaa  tat atc gcc      7074
   Leu Arg  Ala Ile Gln Glu Ala  Gly Ala Asn Val Glu  Tyr Ile Ala
       2345                 2350                 2355
   tgt gat  gtt tcg gat gaa aac  aag gtc cgc caa ctt  gtg caa aga      7119
   Cys Asp  Val Ser Asp Glu Asn  Lys Val Arg Gln Leu  Val Gln Arg
       2360                 2365                 2370
   gtg gag  caa aag tat ggc tgt  gaa ata act ggg att  tgg cat gca      7164
   Val Glu  Gln Lys Tyr Gly Cys  Glu Ile Thr Gly Ile  Trp His Ala
       2375                 2380                 2385
   agc ggg  gtt ctt cgt gac aaa  ctt gtc gag caa aag  act aca gac      7209
   Ser Gly  Val Leu Arg Asp Lys  Leu Val Glu Gln Lys  Thr Thr Asp
       2390                 2395                 2400
   gac ttt  gag gca gtt ttt ggg  acc aag gtg act ggc  ctt gta aac      7254
   Asp Phe  Glu Ala Val Phe Gly  Thr Lys Val Thr Gly  Leu Val Asn
       2405                 2410                 2415
   atc gtg  tca caa gtc aat atg  tct aag cta cga cac  ttc atc ctc      7299
   Ile Val  Ser Gln Val Asn Met  Ser Lys Leu Arg His  Phe Ile Leu
       2420                 2425                 2430
   ttc agt  tct ttg gct gga ttt  cat ggg aac aag ggc  caa acg gat      7344
   Phe Ser  Ser Leu Ala Gly Phe  His Gly Asn Lys Gly  Gln Thr Asp
       2435                 2440                 2445
   tat gca  att gct aat gaa gcc  ttg aac aaa atc gcg  cat act ctc      7389
   Tyr Ala  Ile Ala Asn Glu Ala  Leu Asn Lys Ile Ala  His Thr Leu
       2450                 2455                 2460
   tca gcg  ttt ttg ccc aaa ctg  aat gca aag gtg cta  gac ttc ggt      7434
   Ser Ala  Phe Leu Pro Lys Leu  Asn Ala Lys Val Leu  Asp Phe Gly
       2465                 2470                 2475
   ccg tgg  gta ggt tca gga atg  gta acc gaa aca ctt  gag aag cat      7479
   Pro Trp  Val Gly Ser Gly Met  Val Thr Glu Thr Leu  Glu Lys His
       2480                 2485                 2490
   ttt aaa  gct atg ggg gtt cag  act att cct ctc gag  cca gga gca      7524
   Phe Lys  Ala Met Gly Val Gln  Thr Ile Pro Leu Glu  Pro Gly Ala
       2495                 2500                 2505
   cgg act  gtt gcg caa atc att  ttg gca agt tcg cca  ccg caa tcg      7569
   Arg Thr  Val Ala Gln Ile Ile  Leu Ala Ser Ser Pro  Pro Gln Ser
       2510                 2515                 2520
   ctt ttg  ggg aac tgg ggc ttt  cca gcc acc aaa ccg  cta caa cgc      7614
   Leu Leu  Gly Asn Trp Gly Phe  Pro Ala Thr Lys Pro  Leu Gln Arg
       2525                 2530                 2535
   tct aat  gta gtc acg ggc aca  ctc tct ccg gaa gag  ata gaa ttc      7659
   Ser Asn  Val Val Thr Gly Thr  Leu Ser Pro Glu Glu  Ile Glu Phe
       2540                 2545                 2550
   atc gca  gac cac aaa att caa  ggc cgc aag gtg ctt  ccc atg atg      7704
   Ile Ala  Asp His Lys Ile Gln  Gly Arg Lys Val Leu  Pro Met Met
       2555                 2560                 2565
   gct gca  atc ggg ttc atg gcc  tct att gcg gaa gga  ctc tac ccg      7749
   Ala Ala  Ile Gly Phe Met Ala  Ser Ile Ala Glu Gly  Leu Tyr Pro
       2570                 2575                 2580
   ggg tac  aat ctg caa ggc gtg  gaa aat gct cag ctc  ttt caa ggc      7794
   Gly Tyr  Asn Leu Gln Gly Val  Glu Asn Ala Gln Leu  Phe Gln Gly
       2585                 2590                 2595
   ttg act  atc aac caa gag aca  aaa ttt caa atc act  ctc att gag      7839
   Leu Thr  Ile Asn Gln Glu Thr  Lys Phe Gln Ile Thr  Leu Ile Glu
       2600                 2605                 2610
   gag cac  aac tct gag gaa aac  ctg gat gtc ctg aca  tcc ctt ggt      7884
   Glu His  Asn Ser Glu Glu Asn  Leu Asp Val Leu Thr  Ser Leu Gly
       2615                 2620                 2625
   gta atg  ttg gaa agc ggg aag  gtg ctt ccc gct tac  cga tgt gtt      7929
   Val Met  Leu Glu Ser Gly Lys  Val Leu Pro Ala Tyr  Arg Cys Val
       2630                 2635                 2640
   gta tgc  ttg aat aca acc cag  cag cag ccc aag cta  tct cca aaa      7974
   Val Cys  Leu Asn Thr Thr Gln  Gln Gln Pro Lys Leu  Ser Pro Lys
       2645                 2650                 2655
   att ctt  aac ttg gaa gtt gac  cct gca tgc gag gtt  aac ccc tat      8019
   Ile Leu  Asn Leu Glu Val Asp  Pro Ala Cys Glu Val  Asn Pro Tyr
       2660                 2665                 2670
   gat gga  aag tcg ttg ttc cac  ggt ccg ctt ttg caa  ttc gtt caa      8064
   Asp Gly  Lys Ser Leu Phe His  Gly Pro Leu Leu Gln  Phe Val Gln
       2675                 2680                 2685
   caa gtg  ttg cac tca agt acc  aaa ggc ctc gtt gcc  aag tgc cgc      8109
   Gln Val  Leu His Ser Ser Thr  Lys Gly Leu Val Ala  Lys Cys Arg
       2690                 2695                 2700
   gcg ctt  cca atc aaa gaa gcc  atc cga ggg cca ttt  atc aag caa      8154
   Ala Leu  Pro Ile Lys Glu Ala  Ile Arg Gly Pro Phe  Ile Lys Gln
       2705                 2710                 2715
   aca ctc  cat gat cca att cta  gac gac gtc att ttt  cag cta atg      8199
   Thr Leu  His Asp Pro Ile Leu  Asp Asp Val Ile Phe  Gln Leu Met
       2720                 2725                 2730
   ctc gtg  tgg tgt cgt aat gct  cta gga agt gca tcg  cta ccc aac      8244
   Leu Val  Trp Cys Arg Asn Ala  Leu Gly Ser Ala Ser  Leu Pro Asn
       2735                 2740                 2745
   aga att  gaa aag atg tca tac  ttt ggg aat gtc tca  gaa ggt agc      8289
   Arg Ile  Glu Lys Met Ser Tyr  Phe Gly Asn Val Ser  Glu Gly Ser
       2750                 2755                 2760
   act ttc  ttt gcc tca gtt aca  cct gtg gga cca aga  gta cca aag      8334
   Thr Phe  Phe Ala Ser Val Thr  Pro Val Gly Pro Arg  Val Pro Lys
       2765                 2770                 2775
   gat ccc  gtg atc aaa atg cag  ttt ctt ctc caa gat  gaa tcc ggc      8379
   Asp Pro  Val Ile Lys Met Gln  Phe Leu Leu Gln Asp  Glu Ser Gly
       2780                 2785                 2790
   aac aca  ttt tca tcg ggg gag  ggc tcg gtt gtg ctt  agt gac gaa      8424
   Asn Thr  Phe Ser Ser Gly Glu  Gly Ser Val Val Leu  Ser Asp Glu
       2795                  2800                 2805
   ctc gtc  ttt tga                                                    8436
   Leu Val  Phe
       2810
<210> 39
   <211> 2811
   <212> PRT
   <213> Thraustochytrium sp.
<400> 39
   Met Lys Asp Met Glu Asp Arg Arg Val Ala Ile Val Gly Met Ser Ala
   1               5                   10                  15
   His Leu Pro Cys Gly Thr Asp Val Lys Glu Ser Trp Gln Ala Ile Arg
               20                  25                  30
   Asp Gly Ile Asp Cys Leu Ser Asp Leu Pro Ala Asp Arg Leu Asp Val
           35                  40                  45
   Thr Ala Tyr Tyr Asn Pro Asn Lys Ala Thr Lys Asp Lys Ile Tyr Cys
       50                  55                  60
   Lys Arg Gly Gly Phe Ile Pro Asn Tyr Asp Phe Asp Pro Arg Glu Phe
   65                  70                  75                  80
   Gly Leu Asn Met Phe Gln Met Glu Asp Ser Asp Ala Asn Gln Thr Leu
                   85                  90                  95
   Thr Leu Leu Lys Val Lys Gln Ala Leu Glu Asp Ala Ser Ile Glu Pro
               100                 105                 110
   Phe Thr Lys Glu Lys Lys Asn Ile Gly Cys Val Leu Gly Ile Gly Gly
           115                 120                 125
   Gly Gln Lys Ala Ser His Glu Phe Tyr Ser Arg Leu Asn Tyr Val Val
       130                 135                 140
   Val Glu Lys Val Leu Arg Lys Met Gly Leu Pro Asp Ala Asp Val Glu
   145                 150                 155                 160
   Glu Ala Val Glu Lys Tyr Lys Ala Asn Phe Pro Glu Trp Arg Leu Asp
                   165                 170                 175
   Ser Phe Pro Gly Phe Leu Gly Asn Val Thr Ala Gly Arg Cys Ser Asn
               180                 185                 190
   Thr Phe Asn Met Glu Gly Met Asn Cys Val Val Asp Ala Ala Cys Ala
           195                 200                 205
   Ser Ser Leu Ile Ala Ile Lys Val Ala Val Glu Glu Leu Leu Phe Gly
       210                 215                 220
   Asp Cys Asp Thr Met Ile Ala Gly Ala Thr Cys Thr Asp Asn Ser Leu
   225                 230                 235                 240
   Gly Met Tyr Met Ala Phe Ser Lys Thr Pro Val Phe Ser Thr Asp Pro
                   245                 250                 255
   Ser Val Arg Ala Tyr Asp Glu Lys Thr Lys Gly Met Leu Ile Gly Glu
               260                 265                 270
   Gly Ser Ala Met Phe Val Leu Lys Arg Tyr Ala Asp Ala Val Arg Asp
           275                 280                 285
   Gly Asp Thr Ile His Ala Val Leu Arg Ser Cys Ser Ser Ser Ser Asp
       290                 295                 300
   Gly Lys Ala Ala Gly Ile Tyr Thr Pro Thr Ile Ser Gly Gln Glu Glu
   305                 310                 315                 320
   Ala Leu Arg Arg Ala Tyr Ala Arg Ala Gly Val Cys Pro Ser Thr Ile
                   325                 330                 335
   Gly Leu Val Glu Gly His Gly Thr Gly Thr Pro Val Gly Asp Arg Ile
               340                 345                 350
   Glu Leu Thr Ala Leu Arg Asn Leu Phe Asp Lys Ala Phe Gly Ser Lys
           355                 360                 365
   Lys Glu Gln Ile Ala Val Gly Ser Ile Lys Ser Gln Ile Gly His Leu
       370                 375                 380
   Lys Ser Val Ala Gly Phe Ala Gly Leu Val Lys Ala Val Leu Ala Leu
   385                 390                 395                 400
   Lys His Lys Thr Leu Pro Gly Ser Ile Asn Val Asp Gln Pro Pro Leu
                   405                 410                 415
   Leu Tyr Asp Gly Thr Gln Ile Gln Asp Ser Ser Leu Tyr Ile Asn Lys
               420                 425                 430
   Thr Asn Arg Pro Trp Phe Thr Gln Asn Lys Leu Pro Arg Arg Ala Gly
           435                 440                 445
   Val Ser Ser Phe Gly Phe Gly Gly Ala Asn Tyr His Ala Val Leu Glu
       450                 455                 460
   Glu Phe Glu Pro Glu His Glu Lys Pro Tyr Arg Leu Asn Thr Val Gly
   465                 470                 475                 480
   His Pro Val Leu Leu Tyr Ala Pro Ser Val Glu Ala Leu Lys Val Leu
                   485                 490                 495
   Cys Asn Asp Gln Leu Ala Glu Leu Thr Ile Ala Leu Glu Glu Ala Lys
               500                 505                 510
   Thr His Lys Asn Val Asp Lys Val Cys Gly Tyr Lys Phe Ile Asp Glu
           515                 520                 525
   Phe Gln Leu Gln Gly Ser Cys Pro Pro Glu Asn Pro Arg Val Gly Phe
       530                 535                 540
   Leu Ala Thr Leu Pro Thr Ser Asn Ile Ile Val Ala Leu Lys Ala Ile
   545                 550                 555                 560
   Leu Ala Gln Leu Asp Ala Lys Pro Asp Ala Lys Lys Trp Asp Leu Pro
                   565                 570                 575
   His Lys Lys Ala Phe Gly Ala Thr Phe Ala Ser Ser Ser Val Lys Gly
               580                 585                 590
   Ser Val Ala Ala Leu Phe Ala Gly Gln Gly Thr Gln Tyr Leu Asn Met
           595                 600                 605
   Phe Ser Asp Val Ala Met Asn Trp Pro Pro Phe Arg Asp Ser Ile Val
       610                 615                 620
   Ala Met Glu Glu Ala Gln Thr Glu Val Phe Glu Gly Gln Val Glu Pro
   625                 630                 635                 640
   Ile Ser Lys Val Leu Phe Pro Arg Glu Arg Tyr Ala Ser Glu Ser Glu
                   645                 650                 655
   Gln Gly Asn Glu Leu Leu Cys Leu Thr Glu Tyr Ser Gln Pro Thr Thr
               660                 665                 670
   Ile Ala Ala Ala Val Gly Ala Phe Asp Ile Phe Lys Ala Ala Gly Phe
           675                 680                 685
   Lys Pro Asp Met Val Gly Gly His Ser Leu Gly Glu Phe Ala Ala Leu
       690                 695                 700
   Tyr Ala Ala Gly Ser Ile Ser Arg Asp Asp Leu Tyr Lys Leu Val Cys
   705                 710                 715                 720
   Lys Arg Ala Lys Ala Met Ala Asn Ala Ser Asp Gly Ala Met Ala Ala
                   725                 730                 735
   Val Ile Gly Pro Asp Ala Arg Leu Val Thr Pro Gln Asn Ser Asp Val
               740                 745                 750
   Tyr Val Ala Asn Phe Asn Ser Ala Thr Gln Val Val Ile Ser Gly Thr
           755                 760                 765
   Val Gln Gly Val Lys Glu Glu Ser Lys Leu Leu Ile Ser Lys Gly Phe
       770                 775                 780
   Arg Val Leu Pro Leu Lys Cys Gln Gly Ala Phe His Ser Pro Leu Met
   785                 790                 795                 800
   Gly Pro Ser Glu Asp Ser Phe Lys Ser Leu Val Glu Thr Cys Thr Ile
   805 810 815
   Ser Pro Pro Lys Asn Val Lys Phe Phe Cys Asn Val Ser Gly Lys Glu
   820 825 830
   Ser Pro Asn Pro Lys Gln Thr Leu Lys Ser His Met Thr Ser Ser Val
   835 840 845
   Gln Phe Glu Glu Gln Ile Arg Asn Met Tyr Asp Ala Gly Ala Arg Val
   850 855 860
   Phe Leu Glu Phe Gly Pro Arg Gln Val Leu Ala Lys Leu Ile Ala Glu
   865 870 875 880
   Met Phe Pro Ser Cys Thr Ala Ile Ser Val Asn Pro Ala Ser Ser Gly
   885 890 895
   Asp Ser Asp Val Gln Leu Arg Leu Ala Ala Val Lys Phe Ala Val Ser
   900 905 910
   Gly Ala Ala Leu Ser Thr Phe Asp Pro Trp Glu Tyr Arg Lys Pro Gln
   915 920 925
   Asp Leu Leu Ile Arg Lys Pro Arg Lys Thr Ala Leu Val Leu Ser Ala
   930 935 940
   Ala Thr Tyr Val Ser Pro Lys Thr Leu Ala Glu Arg Lys Lys Ala Met
   945 950 955 960
   Glu Asp Ile Lys Leu Val Ser Ile Thr Pro Arg Asp Ser Met Val Ser
   965 970 975
   Ile Gly Lys Ile Ala Gln Glu Val Arg Thr Ala Lys Gln Pro Leu Glu
   980 985 990
   Thr Glu Ile Arg Arg Leu Asn Lys Glu Leu Glu His Leu Lys Arg Glu
   995 1000 1005
   Leu Ala Ala Ala Lys Ala Ser Val Lys Ser Ala Ser Lys Ser Ser
   1010 1015 1020
   Lys Glu Arg Ser Val Leu Ser Lys His Arg Ala Leu Leu Gln Asn
   1025 1030 1035
   Ile Leu Gln Asp Tyr Asp Asp Leu Arg Val Val Pro Phe Ala Val
   1040 1045 1050
   Arg Ser Val Ala Val Asp Asn Thr Ala Pro Tyr Ala Asp Gln Val
   1055 1060 1065
   Ser Thr Pro Ala Ser Glu Arg Ser Ala Ser Pro Leu Phe Glu Lys
   1070 1075 1080
   Arg Ser Ser Val Ser Ser Ala Arg Leu Ala Glu Ala Glu Ala Ala
   1085 1090 1095
   Val Leu Ser Val Leu Ala Asp Lys Thr Gly Tyr Asp Ser Ser Met
   1100 1105 1110
   Ile Glu Met Asp Met Asp Leu Glu Ser Glu Leu Gly Val Asp Ser
   1115 1120 1125
   Ile Lys Arg Val Glu Ile Met Ser Glu Val Gln Thr Leu Leu Ser
   1130 1135 1140
   Val Glu Val Ser Asp Val Asp Ala Leu Ser Arg Thr Lys Thr Val
   1145 1150 1155
   Gly Asp Val Ile Glu Ala Met Lys Leu Glu Leu Gly Gly Pro Gln
   1160 1165 1170
   Gly Gln Thr Leu Thr Ala Glu Ser Ile Arg Gln Pro Pro Val Ser
   1175 1180 1185
   Glu Pro Ala Val Pro Thr Ser Ser Ser Ser Ser Ile Ala Asn Val
   1190 1195 1200
   Ser Ser Ala Arg Leu Ala Glu Ala Glu Ala Ala Val Leu Ser Val
   1205 1210 1215
   Leu Ala Asp Lys Thr Gly Tyr Asp Ser Ser Met Ile Glu Met Asp
   1220 1225 1230
   Met Asp Leu Glu Ser Glu Leu Gly Val Asp Ser Ile Lys Arg Val
   1235 1240 1245
   Glu Ile Met Ser Glu Val Gln Thr Leu Leu Ser Val Glu Val Ser
   1250 1255 1260
   Asp Val Asp Ala Leu Ser Arg Thr Lys Thr Val Gly Asp Val Ile
   1265 1270 1275
   Glu Ala Met Lys Leu Glu Leu Gly Gly Pro Gln Gly Gln Thr Leu
   1280 1285 1290
   Thr Ala Glu Ser Ile Arg Gln Pro Pro Val Ser Glu Pro Ala Val
   1295 1300 1305
   Pro Thr Ser Ser Ser Ser Ser Ile Ala Asn Val Ser Ser Ala Arg
   1310 1315 1320
   Leu Ala Glu Ala Glu Ala Ala Val Leu Ser Val Leu Ala Asp Lys
   1325 1330 1335
   Thr Gly Tyr Asp Ser Ser Met Ile Glu Met Asp Met Asp Leu Glu
   1340 1345 1350
   Ser Glu Leu Gly Val Asp Ser Ile Lys Arg Val Glu Ile Met Ser
   1355 1360 1365
   Glu Val Gln Thr Leu Leu Ser Val Glu Val Ser Asp Val Asp Ala
   1370 1375 1380
   Leu Ser Arg Thr Lys Thr Val Gly Asp Val Ile Glu Ala Met Lys
   1385 1390 1395
   Leu Glu Leu Gly Gly Pro Gln Gly Gln Thr Leu Thr Ala Glu Ser
   1400 1405 1410
   Ile Arg Gln Pro Pro Val Ser Glu Pro Ala Val Pro Thr Ser Ser
   1415 1420 1425
   Ser Ser Ser Ile Ala Asn Val Leu Ser Ala Arg Leu Ala Glu Ala
   1430 1435 1440
   Glu Ala Ala Val Leu Ser Val Leu Ala Asp Lys Thr Gly Tyr Asp
   1445 1450 1455
   Ser Ser Met Ile Glu Met Asp Met Asp Leu Glu Ser Glu Leu Gly
   1460 1465 1470
   Val Asp Ser Ile Lys Arg Val Glu Ile Met Ser Glu Val Gln Thr
   1475 1480 1485
   Leu Leu Ser Val Glu Val Ser Asp Val Asp Ala Leu Ser Arg Thr
   1490 1495 1500
   Lys Thr Val Gly Asp Val Ile Glu Ala Met Lys Leu Glu Leu Gly
   1505 1510 1515
   Gly Pro Gln Gly Gln Thr Leu Thr Ala Glu Ser Ile Arg Gln Pro
   1520 1525 1530
   Pro Val Ser Glu Pro Ala Val Pro Thr Ser Ser Ser Ser Ser Ile
   1535 1540 1545
   Ala Asn Val Ser Ser Ala Arg Leu Ala Glu Ala Glu Ala Ala Val
   1550 1555 1560
   Leu Ser Val Leu Ala Asp Lys Thr Gly Tyr Asp Ser Ser Met Ile
   1565 1570 1575
   Glu Met Asp Met Asp Leu Glu Ser Glu Leu Gly Val Asp Ser Ile
   1580 1585 1590
   Lys Arg Val Glu Ile Met Ser Glu Val Gln Thr Leu Leu Ser Val
   1595 1600 1605
   Glu Val Ser Asp Val Asp Ala Leu Ser Arg Thr Lys Thr Val Gly
   1610 1615 1620
   Asp Val Ile Glu Ala Met Lys Leu Glu Leu Gly Gly Pro Gln Gly
   1625 1630 1635
   Gln Thr Leu Thr Ser Glu Pro Ile His Gln Pro Pro Val Ser Glu
   1640 1645 1650
   Pro Ala Val Pro Thr Ser Ser Ser Ser Ser Ile Ala Asn Val Ser
   1655 1660 1665
   Ser Ala Arg Leu Ala Glu Ala Glu Ala Ala Val Leu Ser Val Leu
   1670 1675 1680
   Ala Asp Lys Thr Gly Tyr Asp Ser Ser Met Ile Glu Met Asp Met
   1685 1690 1695
   Asp Leu Glu Ser Glu Leu Gly Val Asp Ser Ile Lys Arg Val Glu
   1700 1705 1710
   Ile Met Ser Glu Val Gln Thr Leu Leu Ser Val Glu Val Ser Asp
   1715 1720 1725
   Val Asp Ala Leu Ser Arg Thr Lys Thr Val Gly Asp Val Ile Glu
   1730 1735 1740
   Ala Met Lys Met Glu Leu Gly Gly Pro Gln Gly Gln Thr Leu Thr
   1745 1750 1755
   Ala Glu Ser Ile Arg Gln Pro Pro Val Ser Glu Pro Ala Val Pro
   1760 1765 1770
   Thr Ser Ser Ser Ser Ser Ile Ala Asn Val Ser Ser Ala Arg Leu
   1775 1780 1785
   Ala Glu Ala Glu Ala Ala Val Leu Ser Val Leu Ala Asp Lys Thr
   1790 1795 1800
   Gly Tyr Asp Ser Ser Met Ile Glu Met Asp Met Asp Leu Glu Ser
   1805 1810 1815
   Glu Leu Gly Val Asp Ser Ile Lys Arg Val Glu Ile Met Ser Glu
   1820 1825 1830
   Val Gln Ala Leu Leu Ser Val Glu Val Ser Asp Val Asp Ala Leu
   1835 1840 1845
   Ser Arg Thr Lys Thr Val Gly Asp Val Ile Glu Ala Met Lys Met
   1850 1855 1860
   Glu Leu Gly Gly Pro Gln Gly Gln Thr Leu Thr Ala Glu Ser Ile
   1865 1870 1875
   Arg Glu Pro Pro Val Ser Glu Pro Ala Val Pro Thr Ser Ser Ser
   1880 1885 1890
   Ser Ser Ile Ala Asn Val Ser Ser Ala Arg Leu Ala Glu Ala Glu
   1895 1900 1905
   Ala Ala Val Leu Ser Val Leu Ala Asp Lys Thr Gly Tyr Asp Ser
   1910 1915 1920
   Ser Met Ile Glu Met Asp Met Asp Leu Glu Ser Glu Leu Gly Val
   1925 1930 1935
   Asp Ser Ile Lys Arg Val Glu Ile Met Ser Glu Val Gln Thr Leu
   1940 1945 1950
   Leu Ser Val Glu Val Ser Asp Val Asp Ala Leu Ser Arg Thr Lys
   1955 1960 1965
   Thr Val Gly Asp Val Ile Glu Ala Met Lys Leu Glu Leu Gly Glu
   1970 1975 1980
   Ser Ser Ser Ile Glu Thr Leu Asn Cys Thr Glu Val Glu His Thr
   1985 1990 1995
   Ser Tyr Lys Ser Val Lys Ala Ser Gly Cys Glu Asn Val Asp Thr
   2000 2005 2010
   Arg Phe Ala Lys Val Val Gln Ile Ser Leu Pro Ser Lys Leu Lys
   2015 2020 2025
   Ser Thr Val Ser His Asp Arg Pro Val Ile Val Val Asp Asp Gly
   2030 2035 2040
   Thr Pro Leu Thr Thr Glu Leu Cys Lys Ile Leu Gly Gly Asn Ile
   2045 2050 2055
   Val Val Leu Ser Tyr Gln Gly Lys Pro Ala Gly Pro Arg Gly Val
   2060 2065 2070
   Glu Val Pro Asp Leu Ser Glu Glu Ala Leu Ile Gln Ala Leu Ala
   2075 2080 2085
   Leu Ile Arg Ser Thr Tyr Gly Val Pro Ile Gly Phe Ile Cys Gln
   2090 2095 2100
   Gln Val Ser Asn Val Ser Thr Lys Ala Gln Leu Cys Trp Ala Leu
   2105 2110 2115
   Leu Ala Ala Lys His Leu Lys Lys Asp Leu Asn Ala Val Leu Pro
   2120 2125 2130
   Asp Ser Arg Ser Phe Phe Val Gly Val Val Arg Leu Asn Gly Lys
   2135 2140 2145
   Leu Gly Thr Phe Glu Asn Ile Ser Asp Phe Ser Lys Phe Asp Leu
   2150 2155 2160
   Thr Lys Ala Leu Asp Tyr Gly Gln Arg Gly Ser Leu Leu Gly Leu
   2165 2170 2175
   Cys Lys Ser Leu Asp Leu Glu Trp Glu Gln Val Phe Cys Arg Gly
   2180 2185 2190
   Ile Asp Leu Ala Cys Asp Leu Met Pro Leu Gln Ala Ala Arg Ile
   2195 2200 2205
   Leu Arg Asn Glu Leu Gln Cys Pro Asn Met Arg Leu Arg Glu Val
   2210 2215 2220
   Gly Tyr Asp Ile Ser Gly Ala Arg Tyr Thr Ile Ser Thr Asp Asp
   2225 2230 2235
   Leu Leu Cys Gly Pro Ser Lys Ala Lys Val Glu Ala Ala Asp Leu
   2240 2245 2250
   Phe Leu Val Thr Gly Gly Ala Arg Gly Ile Thr Pro His Cys Val
   2255 2260 2265
   Arg Glu Ile Ala Ser Arg Ser Pro Gly Thr Thr Phe Val Leu Val
   2270 2275 2280
   Gly Arg Ser Glu Met Ser Asp Glu Pro Asp Trp Ala Val Gly His
   2285 2290 2295
   Tyr Asn Lys Asp Leu Asp Gln Ser Thr Met Lys His Leu Lys Ala
   2300 2305 2310
   Thr His Ala Ala Gly Gly Val Lys Pro Thr Pro Lys Ala His Arg
   2315 2320 2325
   Ala Leu Val Asn Arg Val Thr Gly Ser Arg Glu Val Arg Glu Ser
   2330 2335 2340
   Leu Arg Ala Ile Gln Glu Ala Gly Ala Asn Val Glu Tyr Ile Ala
   2345 2350 2355
   Cys Asp Val Ser Asp Glu Asn Lys Val Arg Gln Leu Val Gln Arg
   2360 2365 2370
   Val Glu Gln Lys Tyr Gly Cys Glu Ile Thr Gly Ile Trp His Ala
   2375 2380 2385
   Ser Gly Val Leu Arg Asp Lys Leu Val Glu Gln Lys Thr Thr Asp
   2390 2395 2400
   Asp Phe Glu Ala Val Phe Gly Thr Lys Val Thr Gly Leu Val Asn
   2405 2410 2415
   Ile Val Ser Gln Val Asn Met Ser Lys Leu Arg His Phe Ile Leu
   2420 2425 2430
   Phe Ser Ser Leu Ala Gly Phe His Gly Asn Lys Gly Gln Thr Asp
   2435 2440 2445
   Tyr Ala Ile Ala Asn Glu Ala Leu Asn Lys Ile Ala His Thr Leu
   2450 2455 2460
   Ser Ala Phe Leu Pro Lys Leu Asn Ala Lys Val Leu Asp Phe Gly
   2465 2470 2475
   Pro Trp Val Gly Ser Gly Met Val Thr Glu Thr Leu Glu Lys His
   2480 2485 2490
   Phe Lys Ala Met Gly Val Gln Thr Ile Pro Leu Glu Pro Gly Ala
   2495 2500 2505
   Arg Thr Val Ala Gln Ile Ile Leu Ala Ser Ser Pro Pro Gln Ser
   2510 2515 2520
   Leu Leu Gly Asn Trp Gly Phe Pro Ala Thr Lys Pro Leu Gln Arg
   2525 2530 2535
   Ser Asn Val Val Thr Gly Thr Leu Ser Pro Glu Glu Ile Glu Phe
   2540 2545 2550
   Ile Ala Asp His Lys Ile Gln Gly Arg Lys Val Leu Pro Met Met
   2555 2560 2565
   Ala Ala Ile Gly Phe Met Ala Ser Ile Ala Glu Gly Leu Tyr Pro
   2570 2575 2580
   Gly Tyr Asn Leu Gln Gly Val Glu Asn Ala Gln Leu Phe Gln Gly
   2585 2590 2595
   Leu Thr Ile Asn Gln Glu Thr Lys Phe Gln Ile Thr Leu Ile Glu
   2600 2605 2610
   Glu His Asn Ser Glu Glu Asn Leu Asp Val Leu Thr Ser Leu Gly
   2615 2620 2625
   Val Met Leu Glu Ser Gly Lys Val Leu Pro Ala Tyr Arg Cys Val
   2630 2635 2640
   Val Cys Leu Asn Thr Thr Gln Gln Gln Pro Lys Leu Ser Pro Lys
   2645 2650 2655
   Ile Leu Asn Leu Glu Val Asp Pro Ala Cys Glu Val Asn Pro Tyr
   2660 2665 2670
   Asp Gly Lys Ser Leu Phe His Gly Pro Leu Leu Gln Phe Val Gln
   2675 2680 2685
   Gln Val Leu His Ser Ser Thr Lys Gly Leu Val Ala Lys Cys Arg
   2690 2695 2700
   Ala Leu  Pro Ile Lys Glu Ala  Ile Arg Gly Pro Phe  Ile Lys Gln
       2705                 2710                 2715
   Thr Leu  His Asp Pro Ile Leu  Asp Asp Val Ile Phe  Gln Leu Met
       2720                 2725                 2730
   Leu Val  Trp Cys Arg Asn Ala  Leu Gly Ser Ala Ser  Leu Pro Asn
       2735                 2740                 2745
   Arg Ile  Glu Lys Met Ser Tyr  Phe Gly Asn Val Ser  Glu Gly Ser
       2750                 2755                 2760
   Thr Phe  Phe Ala Ser Val Thr  Pro Val Gly Pro Arg  Val Pro Lys
       2765                 2770                 2775
   Asp Pro  Val Ile Lys Met Gln  Phe Leu Leu Gln Asp  Glu Ser Gly
       2780                 2785                 2790
   Asn Thr  Phe Ser Ser Gly Glu  Gly Ser Val Val Leu  Ser Asp Glu
       2795                 2800                 2805
   Leu Val  Phe
       2810
<210> 40
   <211> 1500
   <212> DNA
   <213> Thraustochytrium sp.
<220>
   <221> CDS
   <222> (1)..(1500)
<400> 40
   atg aag gac atg gaa gat aga cgg gtc gct att gtg ggc atg tca gct        48
   Met Lys Asp Met Glu Asp Arg Arg Val Ala Ile Val Gly Met Ser Ala
   1               5                   10                  15
   cac ttg cct tgt ggg aca gat gtg aag gaa tca tgg cag gct att cgc        96
   His Leu Pro Cys Gly Thr Asp Val Lys Glu Ser Trp Gln Ala Ile Arg
               20                  25                  30
   gat gga atc gac tgt cta agt gac cta ccc gcg gat cgt ctc gac gtt       144
   Asp Gly Ile Asp Cys Leu Ser Asp Leu Pro Ala Asp Arg Leu Asp Val
           35                  40                  45
   aca gct tac tac aat ccc aac aaa gcc acg aaa gac aag atc tac tgc       192
   Thr Ala Tyr Tyr Asn Pro Asn Lys Ala Thr Lys Asp Lys Ile Tyr Cys
       50                  55                  60
   aaa cgg ggt ggc ttc atc ccg aac tat gac ttc gac ccc cgc gaa ttt       240
   Lys Arg Gly Gly Phe Ile Pro Asn Tyr Asp Phe Asp Pro Arg Glu Phe
   65                  70                  75                  80
   ggg ctc aac atg ttt caa atg gaa gac tct gat gcg aat cag aca ctt       288
   Gly Leu Asn Met Phe Gln Met Glu Asp Ser Asp Ala Asn Gln Thr Leu
                   85                  90                  95
   acc ttg ctc aaa gtc aaa caa gct ctc gaa gat gca agc ata gag cct       336
   Thr Leu Leu Lys Val Lys Gln Ala Leu Glu Asp Ala Ser Ile Glu Pro
               100                 105                 110
   ttc acc aag gag aag aag aac att gga tgt gtt tta ggt att ggt ggg      384
   Phe Thr Lys Glu Lys Lys Asn Ile Gly Cys Val Leu Gly Ile Gly Gly
           115                 120                 125
   ggc caa aag gcg agt cat gag ttc tac tct cgt ctc aac tac gtt gtc      432
   Gly Gln Lys Ala Ser His Glu Phe Tyr Ser Arg Leu Asn Tyr Val Val
       130                 135                 140
   gtt gaa aag gta ctt cgg aaa atg ggt tta cca gat gct gat gtt gaa      480
   Val Glu Lys Val Leu Arg Lys Met Gly Leu Pro Asp Ala Asp Val Glu
   145                 150                 155                 160
   gaa gct gtg gag aaa tac aag gca aat ttt ccc gag tgg cgc cta gac      528
   Glu Ala Val Glu Lys Tyr Lys Ala Asn Phe Pro Glu Trp Arg Leu Asp
                   165                 170                 175
   tct ttc cct ggg ttt ctt ggg aat gta acg gct ggt cgg tgc agt aac      576
   Ser Phe Pro Gly Phe Leu Gly Asn Val Thr Ala Gly Arg Cys Ser Asn
               180                 185                 190
   acc ttc aac atg gaa ggt atg aac tgc gtt gtg gat gct gca tgt gcc      624
   Thr Phe Asn Met Glu Gly Met Asn Cys Val Val Asp Ala Ala Cys Ala
           195                 200                 205
   agt tct cta att gca atc aag gtt gca gtt gaa gag cta ctc ttt ggt      672
   Ser Ser Leu Ile Ala Ile Lys Val Ala Val Glu Glu Leu Leu Phe Gly
       210                 215                 220
   gac tgt gac acc atg att gca ggt gcc acc tgc acg gac aat tca ctt      720
   Asp Cys Asp Thr Met Ile Ala Gly Ala Thr Cys Thr Asp Asn Ser Leu
   225                 230                 235                 240
   ggc atg tac atg gcc ttc tct aaa acg cca gtt ttt tct act gac cca      768
   Gly Met Tyr Met Ala Phe Ser Lys Thr Pro Val Phe Ser Thr Asp Pro
                   245                 250                 255
   agt gtc cgc gcg tat gat gag aaa aca aaa ggg atg cta att gga gaa      816
   Ser Val Arg Ala Tyr Asp Glu Lys Thr Lys Gly Met Leu Ile Gly Glu
               260                 265                 270
   ggt tca gca atg ttc gtt ctt aaa cgc tat gcg gat gcc gta cgt gat      864
   Gly Ser Ala Met Phe Val Leu Lys Arg Tyr Ala Asp Ala Val Arg Asp
           275                 280                 285
   ggc gac aca att cac gcg gtt ctg cgt tct tgc tct tcg tct agt gat      912
   Gly Asp Thr Ile His Ala Val Leu Arg Ser Cys Ser Ser Ser Ser Asp
       290                 295                 300
   gga aaa gcg gca gga att tat act cct act ata tct gga caa gaa gaa      960
   Gly Lys Ala Ala Gly Ile Tyr Thr Pro Thr Ile Ser Gly Gln Glu Glu
   305                 310                 315                 320
   gct ttg cgt cga gcg tat gcc cgt gcg ggg gta tgt cca tct acg atc     1008
   Ala Leu Arg Arg Ala Tyr Ala Arg Ala Gly Val Cys Pro Ser Thr Ile
                   325                 330                 335
   ggg ctt gtt gag ggt cac ggg aca ggg acc cct gtt gga gat cgc att     1056
   Gly Leu Val Glu Gly His Gly Thr Gly Thr Pro Val Gly Asp Arg Ile
               340                 345                 350
   gag tta aca gct ctg cgg aac ttg ttt gac aaa gct ttt ggt agc aag     1104
   Glu Leu Thr Ala Leu Arg Asn Leu Phe Asp Lys Ala Phe Gly Ser Lys
           355                 360                 365
   aag gaa caa ata gca gtt ggc agc ata aag tct cag ata ggt cac ctg     1152
   Lys Glu Gln Ile Ala Val Gly Ser Ile Lys Ser Gln Ile Gly His Leu
       370                 375                 380
   aaa tct gtt gcc ggc ttt gcc ggc ttg gtc aaa gct gtg ctt gcg ctt     1200
   Lys Ser Val Ala Gly Phe Ala Gly Leu Val Lys Ala Val Leu Ala Leu
   385                 390                 395                 400
   aaa cac aaa acg ctc cca ggt tcg att aat gtc gac cag cca cct ttg      1248
   Lys His Lys Thr Leu Pro Gly Ser Ile Asn Val Asp Gln Pro Pro Leu
                   405                     410                     415
   ttg tat gac ggt act caa att caa gac tct tct tta tat atc aac aag      1296
   Leu Tyr Asp Gly Thr Gln Ile Gln Asp Ser Ser Leu Tyr Ile Asn Lys
               420                     425                     430
   aca aat aga cca tgg ttt acg caa aac aag ctt ccg cgt cgg gct ggt      1344
   Thr Asn Arg Pro Trp Phe Thr Gln Asn Lys Leu Pro Arg Arg Ala Gly
           435                     440                     445
   gtc tca agt ttt gga ttt gga ggt gca aac tac cac gcg gtt ctg gaa      1392
   Val Ser Ser Phe Gly Phe Gly Gly Ala Asn Tyr His Ala Val Leu Glu
       450                     455                     460
   gaa ttc gag ccc gag cat gaa aaa cca tac cgc ctc aat act gtt gga      1440
   Glu Phe Glu Pro Glu His Glu Lys Pro Tyr Arg Leu Asn Thr Val Gly
   465                     470                     475                     480
   cat cct gtc ctc ttg tac gct ccg tct gtg gaa gcc ctc aaa gta ctt      1488
   His Pro Val Leu Leu Tyr Ala Pro Ser Val Glu Ala Leu Lys Val Leu
                   485                     490                     495
   tgc aac gac cag                                                      1500
   Cys Asn Asp Gln
               500
<210> 41
   <211> 500
   <212> PRT
   <213> Thraustochytrium sp.
<400> 41
   Met Lys Asp Met Glu Asp Arg Arg Val Ala Ile Val Gly Met Ser Ala
   1               5                   10                  15
   His Leu Pro Cys Gly Thr Asp Val Lys Glu Ser Trp Gln Ala Ile Arg
               20                  25                  30
   Asp Gly Ile Asp Cys Leu Ser Asp Leu Pro Ala Asp Arg Leu Asp Val
           35                  40                  45
   Thr Ala Tyr Tyr Asn Pro Asn Lys Ala Thr Lys Asp Lys Ile Tyr Cys
       50                  55                  60
   Lys Arg Gly Gly Phe Ile Pro Asn Tyr Asp Phe Asp Pro Arg Glu Phe
   65                  70                  75                  80
   Gly Leu Asn Met Phe Gln Met Glu Asp Ser Asp Ala Asn Gln Thr Leu
                   85                  90                  95
   Thr Leu Leu Lys Val Lys Gln Ala Leu Glu Asp Ala Ser Ile Glu Pro
               100                 105                 110
   Phe Thr Lys Glu Lys Lys Asn Ile Gly Cys Val Leu Gly Ile Gly Gly
           115                 120                 125
   Gly Gln Lys Ala Ser His Glu Phe Tyr Ser Arg Leu Asn Tyr Val Val
       130                 135                 140
   Val Glu Lys Val Leu Arg Lys Met Gly Leu Pro Asp Ala Asp Val Glu
   145 150 155 160
   Glu Ala Val Glu Lys Tyr Lys Ala Asn Phe Pro Glu Trp Arg Leu Asp
   165 170 175
   Ser Phe Pro Gly Phe Leu Gly Asn Val Thr Ala Gly Arg Cys Ser Asn
   180 185 190
   Thr Phe Asn Met Glu Gly Met Asn Cys Val Val Asp Ala Ala Cys Ala
   195 200 205
   Ser Ser Leu Ile Ala Ile Lys Val Ala Val Glu Glu Leu Leu Phe Gly
   210 215 220
   Asp Cys Asp Thr Met Ile Ala Gly Ala Thr Cys Thr Asp Asn Ser Leu
   225 230 235 240
   Gly Met Tyr Met Ala Phe Ser Lys Thr Pro Val Phe Ser Thr Asp Pro
   245 250 255
   Ser Val Arg Ala Tyr Asp Glu Lys Thr Lys Gly Met Leu Ile Gly Glu
   260 265 270
   Gly Ser Ala Met Phe Val Leu Lys Arg Tyr Ala Asp Ala Val Arg Asp
   275 280 285
   Gly Asp Thr Ile His Ala Val Leu Arg Ser Cys Ser Ser Ser Ser Asp
   290 295 300
   Gly Lys Ala Ala Gly Ile Tyr Thr Pro Thr Ile Ser Gly Gln Glu Glu
   305 310 315 320
   Ala Leu Arg Arg Ala Tyr Ala Arg Ala Gly Val Cys Pro Ser Thr Ile
   325 330 335
   Gly Leu Val Glu Gly His Gly Thr Gly Thr Pro Val Gly Asp Arg Ile
   340 345 350
   Glu Leu Thr Ala Leu Arg Asn Leu Phe Asp Lys Ala Phe Gly Ser Lys
   355 360 365
   Lys Glu Gln Ile Ala Val Gly Ser Ile Lys Ser Gln Ile Gly His Leu
   370 375 380
   Lys Ser Val Ala Gly Phe Ala Gly Leu Val Lys Ala Val Leu Ala Leu
   385 390 395 400
   Lys His Lys Thr Leu Pro Gly Ser Ile Asn Val Asp Gln Pro Pro Leu
   405 410 415
   Leu Tyr Asp Gly Thr Gln Ile Gln Asp Ser Ser Leu Tyr Ile Asn Lys
   420 425 430
   Thr Asn Arg Pro Trp Phe Thr Gln Asn Lys Leu Pro Arg Arg Ala Gly
           435                     440                     445
   Val Ser Ser Phe Gly Phe Gly Gly Ala Asn Tyr His Ala Val Leu Glu
       450                     455                     460
   Glu Phe Glu Pro Glu His Glu Lys Pro Tyr Arg Leu Asn Thr Val Gly
   465                     470                     475                 480
   His Pro Val Leu Leu Tyr Ala Pro Ser Val Glu Ala Leu Lys Val Leu
                   485                     490                     495
   Cys Asn Asp Gln
               500
<210> 42
   <211> 1500
   <212> DNA
   <213> Thraustochytrium sp.
<220>
   <221> CDS
   <222> (1)..(1500)
<400> 42
   ctt gcg gag ctc aca att gca ttg gaa gag gca aaa aca cat aaa aat      48
   Leu Ala Glu Leu Thr Ile Ala Leu Glu Glu Ala Lys Thr His Lys Asn
   1               5                   10                  15
   gtt gac aaa gtt tgt ggc tac aag ttt att gac gaa ttt cag ctc caa      96
   Val Asp Lys Val Cys Gly Tyr Lys Phe Ile Asp Glu Phe Gln Leu Gln
               20                  25                  30
   gga agc tgt cct cca gaa aat ccg aga gta gga ttt tta gca aca ctg     144
   Gly Ser Cys Pro Pro Glu Asn Pro Arg Val Gly Phe Leu Ala Thr Leu
           35                  40                  45
   cct act tca aat atc att gtc gcg ctt aag gca att ctc gcg cag ctt     192
   Pro Thr Ser Asn Ile Ile Val Ala Leu Lys Ala Ile Leu Ala Gln Leu
       50                  55                  60
   gat gca aaa cca gat gcg aag aaa tgg gat ttg cct cat aaa aag gct     240
   Asp Ala Lys Pro Asp Ala Lys Lys Trp Asp Leu Pro His Lys Lys Ala
   65                  70                  75                  80
   ttt ggg gct acc ttc gca tcg tct tca gtg aaa ggc tct gtt gct gcg     288
   Phe Gly Ala Thr Phe Ala Ser Ser Ser Val Lys Gly Ser Val Ala Ala
                   85                  90                  95
   ctc ttc gca gga cag ggt acc cag tac tta aac atg ttc tct gat gtg     336
   Leu Phe Ala Gly Gln Gly Thr Gln Tyr Leu Asn Met Phe Ser Asp Val
               100                 105                 110
   gca atg aac tgg cca ccg ttc cgt gac agc att gtc gca atg gaa gaa     384
   Ala Met Asn Trp Pro Pro Phe Arg Asp Ser Ile Val Ala Met Glu Glu
           115                 120                 125
   gct caa act gag gta ttt gag ggc caa gtt gaa cca att agc aaa gtt     432
   Ala Gln Thr Glu Val Phe Glu Gly Gln Val Glu Pro Ile Ser Lys Val
       130                 135                 140
   ctg ttt cca cga gag cgc tat gca tcc gaa agt gaa cag ggg aat gaa     480
   Leu Phe Pro Arg Glu Arg Tyr Ala Ser Glu Ser Glu Gln Gly Asn Glu
   145                 150                 155                 160
   ctt ctt tgc tta aca gag tac tct cag cca act acg ata gca gcc gca       528
   Leu Leu Cys Leu Thr Glu Tyr Ser Gln Pro Thr Thr Ile Ala Ala Ala
                   165                 170                 175
   gta ggg gcc ttc gat att ttc aaa gcg gct ggc ttt aag cca gac atg       576
   Val Gly Ala Phe Asp Ile Phe Lys Ala Ala Gly Phe Lys Pro Asp Met
               180                 185                 190
   gtt gga ggg cat tca ctt ggc gaa ttt gct gct ttg tac gcg gct ggg       624
   Val Gly Gly His Ser Leu Gly Glu Phe Ala Ala Leu Tyr Ala Ala Gly
           195                 200                 205
   tcc att tcg cgt gac gac ctg tac aag ctt gtg tgc aaa cgg gca aag       672
   Ser Ile Ser Arg Asp Asp Leu Tyr Lys Leu Val Cys Lys Arg Ala Lys
       210                 215                 220
   gca atg gcg aac gct agt gac gga gct atg gca gca gtg att ggc cca       720
   Ala Met Ala Asn Ala Ser Asp Gly Ala Met Ala Ala Val Ile Gly Pro
   225                 230                 235                 240
   gat gca cgt cta gtt acg cca caa aat agt gac gtt tat gtc gca aac       768
   Asp Ala Arg Leu Val Thr Pro Gln Asn Ser Asp Val Tyr Val Ala Asn
                   245                 250                 255
   ttc aac tcc gca act caa gta gtc atc agt ggc act gtt caa ggt gtg       816
   Phe Asn Ser Ala Thr Gln Val Val Ile Ser Gly Thr Val Gln Gly Val
               260                 265                 270
   aaa gaa gag tcg aaa ttg ctc att tca aag ggg ttc cgc gta ctg cca       864
   Lys Glu Glu Ser Lys Leu Leu Ile Ser Lys Gly Phe Arg Val Leu Pro
           275                 280                 285
   ctt aaa tgc cag ggc gcc ttc cat tct cct ttg atg ggg cct tct gag       912
   Leu Lys Cys Gln Gly Ala Phe His Ser Pro Leu Met Gly Pro Ser Glu
       290                 295                 300
   gat agt ttc aaa tca ctt gtg gag act tgt acc atc tcg ccg cca aaa       960
   Asp Ser Phe Lys Ser Leu Val Glu Thr Cys Thr Ile Ser Pro Pro Lys
   305                 310                 315                 320
   aat gtg aaa ttc ttt tgc aat gtt agt ggc aag gaa agc cca aac cca      1008
   Asn Val Lys Phe Phe Cys Asn Val Ser Gly Lys Glu Ser Pro Asn Pro
                   325                 330                 335
   aaa cag acc ctc aag tca cac atg acg tct agc gtt cag ttc gag gag      1056
   Lys Gln Thr Leu Lys Ser His Met Thr Ser Ser Val Gln Phe Glu Glu
               340                 345                 350
   cag att cgt aac atg tac gat gcc gga gca cgt gtt ttt ctg gag ttt      1104
   Gln Ile Arg Asn Met Tyr Asp Ala Gly Ala Arg Val Phe Leu Glu Phe
           355                 360                 365
   gga ccc cgc caa gtc ctt gca aag ctt atc gcg gaa atg ttt ccc tcg      1152
   Gly Pro Arg Gln Val Leu Ala Lys Leu Ile Ala Glu Met Phe Pro Ser
       370                 375                 380
   tgt aca gct atc agc gtt aac ccc gcg agc agt ggt gac agt gac gtg      1200
   Cys Thr Ala Ile Ser Val Asn Pro Ala Ser Ser Gly Asp Ser Asp Val
   385                 390                 395                 400
   caa ctc cgc ctc gcc gcc gta aaa ttc gcg gtc tcg ggt gca gcc ctt      1248
   Gln Leu Arg Leu Ala Ala Val Lys Phe Ala Val Ser Gly Ala Ala Leu
                   405                 410                 415
   agc acc ttt gat cca tgg gag tat cgc aag cca caa gat ctt ctt att      1296
   Ser Thr Phe Asp Pro Trp Glu Tyr Arg Lys Pro Gln Asp Leu Leu Ile
               420                 425                 430
   cga aaa cca cga aaa act gcc ctt gtt cta tca gca gca aca tat gtt      1344
   Arg Lys Pro Arg Lys Thr Ala Leu Val Leu Ser Ala Ala Thr Tyr Val
           435                 440                 445
   tcc cca aag act ctt gca gaa cgt aaa aag gct atg gaa gat atc aag      1392
   Ser Pro Lys Thr Leu Ala Glu Arg Lys Lys Ala Met Glu Asp Ile Lys
       450                 455                 460
   cta gta tcc att aca cca aga gat agt atg gta tca att gga aaa atc      1440
   Leu Val Ser Ile Thr Pro Arg Asp Ser Met Val Ser Ile Gly Lys Ile
   465                 470                 475                 480
   gcg caa gaa gta cgg aca gct aaa cag cct tta gaa acc gaa att cga      1488
   Ala Gln Glu Val Arg Thr Ala Lys Gln Pro Leu Glu Thr Glu Ile Arg
                   485                 490                 495
   aga ctc aac aaa                                                      1500
   Arg Leu Asn Lys
               500
<210> 43
   <211> 500
   <212> PRT
   <213> Thraustochytrium sp.
<400> 43
   Leu Ala Glu Leu Thr Ile Ala Leu Glu Glu Ala Lys Thr His Lys Asn
   1 5 10 15
   Val Asp Lys Val Cys Gly Tyr Lys Phe Ile Asp Glu Phe Gln Leu Gln
   20 25 30
   Gly Ser Cys Pro Pro Glu Asn Pro Arg Val Gly Phe Leu Ala Thr Leu
   35 40 45
   Pro Thr Ser Asn Ile Ile Val Ala Leu Lys Ala Ile Leu Ala Gln Leu
   50 55 60
   Asp Ala Lys Pro Asp Ala Lys Lys Trp Asp Leu Pro His Lys Lys Ala
   65 70 75 80
   Phe Gly Ala Thr Phe Ala Ser Ser Ser Val Lys Gly Ser Val Ala Ala
   85 90 95
   Leu Phe Ala Gly Gln Gly Thr Gln Tyr Leu Asn Met Phe Ser Asp Val
   100 105 110
   Ala Met Asn Trp Pro Pro Phe Arg Asp Ser Ile Val Ala Met Glu Glu
   115 120 125
   Ala Gln Thr Glu Val Phe Glu Gly Gln Val Glu Pro Ile Ser Lys Val
   130 135 140
   Leu Phe Pro Arg Glu Arg Tyr Ala Ser Glu Ser Glu Gln Gly Asn Glu
   145 150 155 160
   Leu Leu Cys Leu Thr Glu Tyr Ser Gln Pro Thr Thr Ile Ala Ala Ala
   165 170 175
   Val Gly Ala Phe Asp Ile Phe Lys Ala Ala Gly Phe Lys Pro Asp Met
   180 185 190
   Val Gly Gly His Ser Leu Gly Glu Phe Ala Ala Leu Tyr Ala Ala Gly
           195                 200                 205
   Ser Ile Ser Arg Asp Asp Leu Tyr Lys Leu Val Cys Lys Arg Ala Lys
       210                 215                 220
   Ala Met Ala Asn Ala Ser Asp Gly Ala Met Ala Ala Val Ile Gly Pro
   225                 230                 235                 240
   Asp Ala Arg Leu Val Thr Pro Gln Asn Ser Asp Val Tyr Val Ala Asn
                   245                 250                 255
   Phe Asn Ser Ala Thr Gln Val Val Ile Ser Gly Thr Val Gln Gly Val
               260                 265                 270
   Lys Glu Glu Ser Lys Leu Leu Ile Ser Lys Gly Phe Arg Val Leu Pro
           275                 280                 285
   Leu Lys Cys Gln Gly Ala Phe His Ser Pro Leu Met Gly Pro Ser Glu
       290                 295                 300
   Asp Ser Phe Lys Ser Leu Val Glu Thr Cys Thr Ile Ser Pro Pro Lys
   305                 310                 315                 320
   Asn Val Lys Phe Phe Cys Asn Val Ser Gly Lys Glu Ser Pro Asn Pro
                   325                 330                 335
   Lys Gln Thr Leu Lys Ser His Met Thr Ser Ser Val Gln Phe Glu Glu
               340                 345                 350
   Gln Ile Arg Asn Met Tyr Asp Ala Gly Ala Arg Val Phe Leu Glu Phe
           355                 360                 365
   Gly Pro Arg Gln Val Leu Ala Lys Leu Ile Ala Glu Met Phe Pro Ser
       370                 375                 380
   Cys Thr Ala Ile Ser Val Asn Pro Ala Ser Ser Gly Asp Ser Asp Val
   385                 390                 395                 400
   Gln Leu Arg Leu Ala Ala Val Lys Phe Ala Val Ser Gly Ala Ala Leu
                   405                 410                 415
   Ser Thr Phe Asp Pro Trp Glu Tyr Arg Lys Pro Gln Asp Leu Leu Ile
               420                 425                 430
   Arg Lys Pro Arg Lys Thr Ala Leu Val Leu Ser Ala Ala Thr Tyr Val
           435                 440                 445
   Ser Pro Lys Thr Leu Ala Glu Arg Lys Lys Ala Met Glu Asp Ile Lys
       450                 455                 460
   Leu Val Ser Ile Thr Pro Arg Asp Ser Met Val Ser Ile Gly Lys Ile
   465                 470                 475                 480
   Ala Gln Glu Val Arg Thr Ala Lys Gln Pro Leu Glu Thr Glu Ile Arg
                   485                 490                 495
   Arg Leu Asn Lys
               500
<210> 44
   <211> 351
   <212> DNA
   <213> Thraustochytrium sp.
<220>
   <221> CDS
   <222> (1)..(351)
<400> 44
   tcg acc cca gcg tca gag cgg tcg gct tca ccg ctt ttc gag aaa cgc      48
   Ser Thr Pro Ala Ser Glu Arg Ser Ala Ser Pro Leu Phe Glu Lys Arg
   1               5                   10                  15
   agt tcg gtt tcg tca gca cgc ctc gct gaa gct gaa gcc gcg gta ctg      96
   Ser Ser Val Ser Ser Ala Arg Leu Ala Glu Ala Glu Ala Ala Val Leu
               20                  25                  30
   agc gtt ctc gca gac aag aca ggc tac gac agc tca atg atc gag atg     144
   Ser Val Leu Ala Asp Lys Thr Gly Tyr Asp Ser Ser Met Ile Glu Met
           35                  40                  45
   gac atg gac ctg gag agt gag ctt ggc gtt gat agc atc aaa cgc gtg     192
   Asp Met Asp Leu Glu Ser Glu Leu Gly Val Asp Ser Ile Lys Arg Val
       50                  55                  60
   gag atc atg agc gag gtt caa acg ctg ctc agc gtg gaa gtc tcc gac     240
   Glu Ile Met Ser Glu Val Gln Thr Leu Leu Ser Val Glu Val Ser Asp
   65                  70                  75                  80
   gtt gac gct ctg tca aga acc aag act gtt ggc gac gtc atc gag gcg     288
   Val Asp Ala Leu Ser Arg Thr Lys Thr Val Gly Asp Val Ile Glu Ala
                   85                  90                  95
   atg aag ctg gaa ctc ggt gga ccc caa ggc cag act ttg acc gcg gaa     336
   Met Lys Leu Glu Leu Gly Gly Pro Gln Gly Gln Thr Leu Thr Ala Glu
               100                 105                 110
   tcg atc cgt cag cca                                                 351
   Ser Ile Arg Gln Pro
           115
<210> 45
   <211> 117
   <212> PRT
   <213> Thraustochytrium sp.
<400> 45
   Ser Thr Pro Ala Ser Glu Arg Ser Ala Ser Pro Leu Phe Glu Lys Arg
   1               5                   10                  15
   Ser Ser Val Ser Ser Ala Arg Leu Ala Glu Ala Glu Ala Ala Val Leu
               20                  25                  30
   Ser Val Leu Ala Asp Lys Thr Gly Tyr Asp Ser Ser Met Ile Glu Met
           35                  40                  45
   Asp Met Asp Leu Glu Ser Glu Leu Gly Val Asp Ser Ile Lys Arg Val
       50                  55                  60
   Glu Ile Met Ser Glu Val Gln Thr Leu Leu Ser Val Glu Val Ser Asp
   65                  70                  75                  80
   Val Asp Ala Leu Ser Arg Thr Lys Thr Val Gly Asp Val Ile Glu Ala
                   85                  90                  95
   Met Lys Leu Glu Leu Gly Gly Pro Gln Gly Gln Thr Leu Thr Ala Glu
               100                 105                 110
   Ser Ile Arg Gln Pro
           115
<210> 46
   <211> 5
   <212> PRT
   <213> Thraustochytrium sp.
<220>
   <221> MISC_FEATURE
   <222> (1)..(5)
   <223> Xaa = any amino acid
<400> 46
   Leu Gly Xaa Asp Ser
   1               5
<210> 47
   <211> 2790
   <212> DNA
   <213> Thraustochytrium sp.
<220>
   <221> CDS
   <222> (1)..(2790)
<400> 47
   tcg acc cca gcg tca gag cgg tcg gct tca ccg ctt ttc gag aaa cgc        48
   Ser Thr Pro Ala Ser Glu Arg Ser Ala Ser Pro Leu Phe Glu Lys Arg
   1               5                   10                  15
   agt tcg gtt tcg tca gca cgc ctc gct gaa gct gaa gcc gcg gta ctg        96
   Ser Ser Val Ser Ser Ala Arg Leu Ala Glu Ala Glu Ala Ala Val Leu
               20                  25                  30
   agc gtt ctc gca gac aag aca ggc tac gac agc tca atg atc gag atg       144
   Ser Val Leu Ala Asp Lys Thr Gly Tyr Asp Ser Ser Met Ile Glu Met
           35                  40                  45
   gac atg gac ctg gag agt gag ctt ggc gtt gat agc atc aaa cgc gtg       192
   Asp Met Asp Leu Glu Ser Glu Leu Gly Val Asp Ser Ile Lys Arg Val
       50                  55                  60
   gag atc atg agc gag gtt caa acg ctg ctc agc gtg gaa gtc tcc gac       240
   Glu Ile Met Ser Glu Val Gln Thr Leu Leu Ser Val Glu Val Ser Asp
   65                  70                  75                  80
   gtt gac gct ctg tca aga acc aag act gtt ggc gac gtc atc gag gcg       288
   Val Asp Ala Leu Ser Arg Thr Lys Thr Val Gly Asp Val Ile Glu Ala
                   85                  90                  95
   atg aag ctg gaa ctc ggt gga ccc caa ggc cag act ttg acc gcg gaa      336
   Met Lys Leu Glu Leu Gly Gly Pro Gln Gly Gln Thr Leu Thr Ala Glu
               100                 105                 110
   tcg atc cgt cag cca ccg gtg tcc gag cct gct gta ccg acc tca tcg      384
   Ser Ile Arg Gln Pro Pro Val Ser Glu Pro Ala Val Pro Thr Ser Ser
           115                 120                 125
   tca agc agt att gct aat gtt tcg tca gca cgc ctc gct gaa gct gaa      432
   Ser Ser Ser Ile Ala Asn Val Ser Ser Ala Arg Leu Ala Glu Ala Glu
       130                 135                 140
   gct gcg gta ctg agc gtt ctc gca gac aag aca ggc tac gac agc tca      480
   Ala Ala Val Leu Ser Val Leu Ala Asp Lys Thr Gly Tyr Asp Ser Ser
   145                 150                 155                 160
   atg atc gag atg gac atg gac ctg gag agc gag ctt ggc gtt gat agc      528
   Met Ile Glu Met Asp Met Asp Leu Glu Ser Glu Leu Gly Val Asp Ser
                   165                 170                 175
   atc aaa cgc gtg gag atc atg agc gag gtt caa acg ctg ctc agc gtg      576
   Ile Lys Arg Val Glu Ile Met Ser Glu Val Gln Thr Leu Leu Ser Val
               180                 185                 190
   gaa gtc tcc gac gtt gac gct ctg tca aga act aag act gtt ggc gac      624
   Glu Val Ser Asp Val Asp Ala Leu Ser Arg Thr Lys Thr Val Gly Asp
           195                 200                 205
   gtc atc gag gcg atg aag ctg gaa ctc ggt gga ccc caa ggc cag act      672
   Val Ile Glu Ala Met Lys Leu Glu Leu Gly Gly Pro Gln Gly Gln Thr
       210                 215                 220
   ttg acc gcg gaa tcg atc cgt cag cca ccg gtg tct gag cct gct gta      720
   Leu Thr Ala Glu Ser Ile Arg Gln Pro Pro Val Ser Glu Pro Ala Val
   225                 230                 235                 240
   ccg acc tca tcg tca agc agt att gct aat gtt tcg tca gca cgc ctc      768
   Pro Thr Ser Ser Ser Ser Ser Ile Ala Asn Val Ser Ser Ala Arg Leu
                   245                 250                 255
   gct gaa gct gaa gcg gcg gta ctg agc gtt ctc gca gac aag aca ggc      816
   Ala Glu Ala Glu Ala Ala Val Leu Ser Val Leu Ala Asp Lys Thr Gly
               260                 265                 270
   tac gac agc tca atg atc gag atg gac atg gac ctg gag agc gag ctt      864
   Tyr Asp Ser Ser Met Ile Glu Met Asp Met Asp Leu Glu Ser Glu Leu
           275                 280                 285
   ggc gtc gac agc atc aaa cgc gtg gag atc atg agc gag gtt caa acg      912
   Gly Val Asp Ser Ile Lys Arg Val Glu Ile Met Ser Glu Val Gln Thr
       290                 295                 300
   ctg ctc agc gtg gaa gtc tcc gac gtt gac gct ctg tca aga acc aag      960
   Leu Leu Ser Val Glu Val Ser Asp Val Asp Ala Leu Ser Arg Thr Lys
   305                 310                 315                 320
   act gtt ggc gac gtc atc gag gcg atg aag ctg gaa ctc ggt gga ccc     1008
   Thr Val Gly Asp Val Ile Glu Ala Met Lys Leu Glu Leu Gly Gly Pro
                   325                 330                 335
   caa ggc cag act ttg acc gcg gaa tcg atc cgt cag cca ccg gtg tcc     1056
   Gln Gly Gln Thr Leu Thr Ala Glu Ser Ile Arg Gln Pro Pro Val Ser
               340                 345                 350
   gag cct gct gta ccg acc tca tcg tca agc agt att gct aat gtt ttg     1104
   Glu Pro Ala Val Pro Thr Ser Ser Ser Ser Ser Ile Ala Asn Val Leu
           355                 360                 365
   tca gca cgc ctc gct gaa gct gaa gcc gcg gta ctg agc gtt ctc gca     1152
   Ser Ala Arg Leu Ala Glu Ala Glu Ala Ala Val Leu Ser Val Leu Ala
       370                 375                 380
   gac aag aca ggc tac gac agc tca atg atc gag atg gac atg gac ctg      1200
   Asp Lys Thr Gly Tyr Asp Ser Ser Met Ile Glu Met Asp Met Asp Leu
   385                 390                 395                 400
   gag agc gag ctt ggc gtt gat agc atc aaa cgc gtg gag atc atg agc      1248
   Glu Ser Glu Leu Gly Val Asp Ser Ile Lys Arg Val Glu Ile Met Ser
                   405                 410                 415
   gag gtt caa acg ttg ctc agc gtg gaa gtc tcc gac gtt gac gct ctg      1296
   Glu Val Gln Thr Leu Leu Ser Val Glu Val Ser Asp Val Asp Ala Leu
               420                 425                 430
   tca aga acc aag act gtt ggc gac gtc atc gag gcg atg aag ctg gaa      1344
   Ser Arg Thr Lys Thr Val Gly Asp Val Ile Glu Ala Met Lys Leu Glu
           435                 440                 445
   ctc ggt gga ccc caa ggc cag act ttg acc gcg gaa tcg atc cgt cag      1392
   Leu Gly Gly Pro Gln Gly Gln Thr Leu Thr Ala Glu Ser Ile Arg Gln
       450                 455                 460
   cca ccg gtg tct gag cct gct gta ccg acc tca tcg tca agc agt att      1440
   Pro Pro Val Ser Glu Pro Ala Val Pro Thr Ser Ser Ser Ser Ser Ile
   465                 470                 475                 480
   gct aat gtt tcg tca gca cgc ctc gct gaa gct gaa gcc gcg gta ctg      1488
   Ala Asn Val Ser Ser Ala Arg Leu Ala Glu Ala Glu Ala Ala Val Leu
                   485                 490                 495
   agc gtt ctc gca gac aag aca ggc tac gac agc tca atg atc gag atg      1536
   Ser Val Leu Ala Asp Lys Thr Gly Tyr Asp Ser Ser Met Ile Glu Met
               500                 505                 510
   gac atg gac ctg gag agt gag ctt ggc gtc gac agc atc aaa cgc gtg      1584
   Asp Met Asp Leu Glu Ser Glu Leu Gly Val Asp Ser Ile Lys Arg Val
           515                 520                 525
   gag atc atg agc gag gtt caa acg ctg ctc agc gtg gaa gtc tcc gac      1632
   Glu Ile Met Ser Glu Val Gln Thr Leu Leu Ser Val Glu Val Ser Asp
       530                 535                 540
   gtt gac gct ctg tca aga acc aag act gtt ggc gac gtc atc gag gcg      1680
   Val Asp Ala Leu Ser Arg Thr Lys Thr Val Gly Asp Val Ile Glu Ala
   545                 550                 555                 560
   atg aag ctg gaa ctc ggt gga ccc caa ggc cag act ttg acc tct gaa      1728
   Met Lys Leu Glu Leu Gly Gly Pro Gln Gly Gln Thr Leu Thr Ser Glu
                   565                 570                 575
   ccg atc cat cag cca cca gtg tcc gag cct gct gta ccg acc tca tcg      1776
   Pro Ile His Gln Pro Pro Val Ser Glu Pro Ala Val Pro Thr Ser Ser
               580                 585                 590
   tca agc agt att gct aat gtt tct tca gca cgc ctc gct gaa gct gaa      1824
   Ser Ser Ser Ile Ala Asn Val Ser Ser Ala Arg Leu Ala Glu Ala Glu
           595                 600                 605
   gcc gcg gta ctg agc gtt ctc gca gac aag aca ggc tac gac agc tca      1872
   Ala Ala Val Leu Ser Val Leu Ala Asp Lys Thr Gly Tyr Asp Ser Ser
       610                 615                 620
   atg atc gag atg gac atg gac ctg gag agc gag ctt ggc gtt gat agc      1920
   Met Ile Glu Met Asp Met Asp Leu Glu Ser Glu Leu Gly Val Asp Ser
   625                 630                 635                 640
   atc aaa cgc gtg gaa atc atg agc gag gtt caa acg ctg ctc agc gtg      1968
   Ile Lys Arg Val Glu Ile Met Ser Glu Val Gln Thr Leu Leu Ser Val
                   645                 650                 655
   gaa gtc tcc gac gtt gac gct ctg tca aga acc aag act gtt ggc gac      2016
   Glu Val Ser Asp Val Asp Ala Leu Ser Arg Thr Lys Thr Val Gly Asp
               660                 665                 670
   gtc atc gag gcg atg aag atg gaa ctc ggt gga ccc caa ggc cag act      2064
   Val Ile Glu Ala Met Lys Met Glu Leu Gly Gly Pro Gln Gly Gln Thr
           675                 680                 685
   ttg acc gcg gaa tcg atc cgt cag cca ccg gtg tct gag cct gct gta      2112
   Leu Thr Ala Glu Ser Ile Arg Gln Pro Pro Val Ser Glu Pro Ala Val
       690                 695                 700
   ccg acc tca tcg tca agc agt att gct aat gtt tcg tca gca cgc ctc      2160
   Pro Thr Ser Ser Ser Ser Ser Ile Ala Asn Val Ser Ser Ala Arg Leu
   705                 710                 715                 720
   gct gaa gct gaa gcg gcg gta ctg agc gtt ctc gca gac aag aca ggc      2208
   Ala Glu Ala Glu Ala Ala Val Leu Ser Val Leu Ala Asp Lys Thr Gly
                   725                 730                 735
   tac gac agc tca atg atc gag atg gac atg gac ctg gag agc gag ctt      2256
   Tyr Asp Ser Ser Met Ile Glu Met Asp Met Asp Leu Glu Ser Glu Leu
               740                 745                 750
   ggc gtt gat agc atc aaa cgc gtg gag atc atg agc gag gtt caa gcg      2304
   Gly Val Asp Ser Ile Lys Arg Val Glu Ile Met Ser Glu Val Gln Ala
           755                 760                 765
   ctg ctc agc gtg gaa gtc tcc gac gtt gac gct ctg tca aga acc aag      2352
   Leu Leu Ser Val Glu Val Ser Asp Val Asp Ala Leu Ser Arg Thr Lys
       770                 775                 780
   act gtt ggc gac gtc atc gag gcg atg aag atg gaa ctc ggt gga ccc      2400
   Thr Val Gly Asp Val Ile Glu Ala Met Lys Met Glu Leu Gly Gly Pro
   785                 790                 795                 800
   caa ggc cag act ttg acc gca gaa tcg atc cgt gag cca ccg gtg tct      2448
   Gln Gly Gln Thr Leu Thr Ala Glu Ser Ile Arg Glu Pro Pro Val Ser
                   805                 810                 815
   gag cct gct gta ccg acc tca tcg tca agt agt atc gct aat gtt tct      2496
   Glu Pro Ala Val Pro Thr Ser Ser Ser Ser Ser Ile Ala Asn Val Ser
               820                 825                 830
   tca gct cgc ctc gct gaa gct gaa gcc gcg gta ctg agc gtt ctc gca      2544
   Ser Ala Arg Leu Ala Glu Ala Glu Ala Ala Val Leu Ser Val Leu Ala
           835                 840                 845
   gac aag aca ggc tac gac agc tca atg atc gag atg gac atg gac ctg      2592
   Asp Lys Thr Gly Tyr Asp Ser Ser Met Ile Glu Met Asp Met Asp Leu
       850                 855                 860
   gag agt gag ctt ggc gtc gac agc atc aaa cgc gtg gag atc atg agc      2640
   Glu Ser Glu Leu Gly Val Asp Ser Ile Lys Arg Val Glu Ile Met Ser
   865                 870                 875                 880
   gag gtt caa acg ttg ctc agc gtg gaa gtc tcc gac gtt gac gct ctg      2688
   Glu Val Gln Thr Leu Leu Ser Val Glu Val Ser Asp Val Asp Ala Leu
                   885                 890                 895
   tca aga acc aag act gtt ggc gac gtc atc gag gcg atg aag ctg gaa      2736
   Ser Arg Thr Lys Thr Val Gly Asp Val Ile Glu Ala Met Lys Leu Glu
               900                 905                 910
   ctt ggg gaa tca tca agt att gag act ctc aat tgt acc gag gtt gag      2784
   Leu Gly Glu Ser Ser Ser Ile Glu Thr Leu Asn Cys Thr Glu Val Glu
           915                 920                 925
   cac acg                                                              2790
   His Thr
       930
<210> 48
   <211> 930
   <212> PRT
   <213> Thraustochytrium sp.
<400> 48
   Ser Thr Pro Ala Ser Glu Arg Ser Ala Ser Pro Leu Phe Glu Lys Arg
   1 5 10 15
   Ser Ser Val Ser Ser Ala Arg Leu Ala Glu Ala Glu Ala Ala Val Leu
   20 25 30
   Ser Val Leu Ala Asp Lys Thr Gly Tyr Asp Ser Ser Met Ile Glu Met
   35 40 45
   Asp Met Asp Leu Glu Ser Glu Leu Gly Val Asp Ser Ile Lys Arg Val
   50 55 60
   Glu Ile Met Ser Glu Val Gln Thr Leu Leu Ser Val Glu Val Ser Asp
   65 70 75 80
   Val Asp Ala Leu Ser Arg Thr Lys Thr Val Gly Asp Val Ile Glu Ala
   85 90 95
   Met Lys Leu Glu Leu Gly Gly Pro Gln Gly Gln Thr Leu Thr Ala Glu
   100 105 110
   Ser Ile Arg Gln Pro Pro Val Ser Glu Pro Ala Val Pro Thr Ser Ser
   115 120 125
   Ser Ser Ser Ile Ala Asn Val Ser Ser Ala Arg Leu Ala Glu Ala Glu
   130 135 140
   Ala Ala Val Leu Ser Val Leu Ala Asp Lys Thr Gly Tyr Asp Ser Ser
   145 150 155 160
   Met Ile Glu Met Asp Met Asp Leu Glu Ser Glu Leu Gly Val Asp Ser
   165 170 175
   Ile Lys Arg Val Glu Ile Met Ser Glu Val Gln Thr Leu Leu Ser Val
   180 185 190
   Glu Val Ser Asp Val Asp Ala Leu Ser Arg Thr Lys Thr Val Gly Asp
   195 200 205
   Val Ile Glu Ala Met Lys Leu Glu Leu Gly Gly Pro Gln Gly Gln Thr
   210 215 220
   Leu Thr Ala Glu Ser Ile Arg Gln Pro Pro Val Ser Glu Pro Ala Val
   225 230 235 240
   Pro Thr Ser Ser Ser Ser Ser Ile Ala Asn Val Ser Ser Ala Arg Leu
   245 250 255
   Ala Glu Ala Glu Ala Ala Val Leu Ser Val Leu Ala Asp Lys Thr Gly
   260 265 270
   Tyr Asp Ser Ser Met Ile Glu Met Asp Met Asp Leu Glu Ser Glu Leu
   275 280 285
   Gly Val Asp Ser Ile Lys Arg Val Glu Ile Met Ser Glu Val Gln Thr
   290 295 300
   Leu Leu Ser Val Glu Val Ser Asp Val Asp Ala Leu Ser Arg Thr Lys
   305 310 315 320
   Thr Val Gly Asp Val Ile Glu Ala Met Lys Leu Glu Leu Gly Gly Pro
   325 330 335
   Gln Gly Gln Thr Leu Thr Ala Glu Ser Ile Arg Gln Pro Pro Val Ser
   340 345 350
   Glu Pro Ala Val Pro Thr Ser Ser Ser Ser Ser Ile Ala Asn Val Leu
   355 360 365
   Ser Ala Arg Leu Ala Glu Ala Glu Ala Ala Val Leu Ser Val Leu Ala
   370 375 380
   Asp Lys Thr Gly Tyr Asp Ser Ser Met Ile Glu Met Asp Met Asp Leu
   385 390 395 400
   Glu Ser Glu Leu Gly Val Asp Ser Ile Lys Arg Val Glu Ile Met Ser
   405 410 415
   Glu Val Gln Thr Leu Leu Ser Val Glu Val Ser Asp Val Asp Ala Leu
   420 425 430
   Ser Arg Thr Lys Thr Val Gly Asp Val Ile Glu Ala Met Lys Leu Glu
   435 440 445
   Leu Gly Gly Pro Gln Gly Gln Thr Leu Thr Ala Glu Ser Ile Arg Gln
   450 455 460
   Pro Pro Val Ser Glu Pro Ala Val Pro Thr Ser Ser Ser Ser Ser Ile
   465 470 475 480
   Ala Asn Val Ser Ser Ala Arg Leu Ala Glu Ala Glu Ala Ala Val Leu
   485 490 495
   Ser Val Leu Ala Asp Lys Thr Gly Tyr Asp Ser Ser Met Ile Glu Met
   500 505 510
   Asp Met Asp Leu Glu Ser Glu Leu Gly Val Asp Ser Ile Lys Arg Val
   515 520 525
   Glu Ile Met Ser Glu Val Gln Thr Leu Leu Ser Val Glu Val Ser Asp
   530 535 540
   Val Asp Ala Leu Ser Arg Thr Lys Thr Val Gly Asp Val Ile Glu Ala
   545 550 555 560
   Met Lys Leu Glu Leu Gly Gly Pro Gln Gly Gln Thr Leu Thr Ser Glu
   565 570 575
   Pro Ile His Gln Pro Pro Val Ser Glu Pro Ala Val Pro Thr Ser Ser
   580 585 590
   Ser Ser Ser Ile Ala Asn Val Ser Ser Ala Arg Leu Ala Glu Ala Glu
   595 600 605
   Ala Ala Val Leu Ser Val Leu Ala Asp Lys Thr Gly Tyr Asp Ser Ser
   610 615 620
   Met Ile Glu Met Asp Met Asp Leu Glu Ser Glu Leu Gly Val Asp Ser
   625 630 635 640
   Ile Lys Arg Val Glu Ile Met Ser Glu Val Gln Thr Leu Leu Ser Val
   645 650 655
   Glu Val Ser Asp Val Asp Ala Leu Ser Arg Thr Lys Thr Val Gly Asp
   660 665 670
   Val Ile Glu Ala Met Lys Met Glu Leu Gly Gly Pro Gln Gly Gln Thr
   675 680 685
   Leu Thr Ala Glu Ser Ile Arg Gln Pro Pro Val Ser Glu Pro Ala Val
   690 695 700
   Pro Thr Ser Ser Ser Ser Ser Ile Ala Asn Val Ser Ser Ala Arg Leu
   705 710 715 720
   Ala Glu Ala Glu Ala Ala Val Leu Ser Val Leu Ala Asp Lys Thr Gly
   725 730 735
   Tyr Asp Ser Ser Met Ile Glu Met Asp Met Asp Leu Glu Ser Glu Leu
   740 745 750
   Gly Val Asp Ser Ile Lys Arg Val Glu Ile Met Ser Glu Val Gln Ala
   755 760 765
   Leu Leu Ser Val Glu Val Ser Asp Val Asp Ala Leu Ser Arg Thr Lys
   770 775 780
   Thr Val Gly Asp Val Ile Glu Ala Met Lys Met Glu Leu Gly Gly Pro
   785 790 795 800
   Gln Gly Gln Thr Leu Thr Ala Glu Ser Ile Arg Glu Pro Pro Val Ser
   805 810 815
   Glu Pro Ala Val Pro Thr Ser Ser Ser Ser Ser Ile Ala Asn Val Ser
   820 825 830
   Ser Ala Arg Leu Ala Glu Ala Glu Ala Ala Val Leu Ser Val Leu Ala
   835 840 845
   Asp Lys Thr Gly Tyr Asp Ser Ser Met Ile Glu Met Asp Met Asp Leu
   850 855 860
   Glu Ser Glu Leu Gly Val Asp Ser Ile Lys Arg Val Glu Ile Met Ser
   865 870 875 880
   Glu Val Gln Thr Leu Leu Ser Val Glu Val Ser Asp Val Asp Ala Leu
   885 890 895
   Ser Arg Thr Lys Thr Val Gly Asp Val Ile Glu Ala Met Lys Leu Glu
   900 905 910
   Leu Gly Glu Ser Ser Ser Ile Glu Thr Leu Asn Cys Thr Glu Val Glu
   915 920 925
   His Thr
   930
<210> 49
   <211> 2433
   <212> DNA
   <213> Thraustochytrium sp.
<220>
   <221> CDS
   <222> (1)..(2433)
<400> 49
   aaa agt gtc aag gct tca ggg tgt gag aat gta gat acc cgt ttc gct       48
   Lys Ser Val Lys Ala Ser Gly Cys Glu Asn Val Asp Thr Arg Phe Ala
   1               5                   10                  15
   aag gtt gta caa atc tcg ctt cct agc aag ctg aaa tcc act gtg tcg       96
   Lys Val Val Gln Ile Ser Leu Pro Ser Lys Leu Lys Ser Thr Val Ser
               20                  25                  30
   cac gat cga cct gta att gtt gta gat gat gga acg ccc tta acc acg      144
   His Asp Arg Pro Val Ile Val Val Asp Asp Gly Thr Pro Leu Thr Thr
           35                  40                  45
   gag ctt tgt aaa att ctt ggg ggt aat att gtg gtt ctc tct tat caa      192
   Glu Leu Cys Lys Ile Leu Gly Gly Asn Ile Val Val Leu Ser Tyr Gln
       50                  55                  60
   ggg aag ccc gct ggt cca cgg gga gtc gag gtg cca gat ctt tcc gag      240
   Gly Lys Pro Ala Gly Pro Arg Gly Val Glu Val Pro Asp Leu Ser Glu
   65                  70                  75                  80
   gaa gcc cta att caa gct ctt gca ttg att cgg tct aca tat gga gtt      288
   Glu Ala Leu Ile Gln Ala Leu Ala Leu Ile Arg Ser Thr Tyr Gly Val
                   85                  90                  95
   cca att ggt ttt att tgt cag caa gtg tct aat gtg agc acc aag gca      336
   Pro Ile Gly Phe Ile Cys Gln Gln Val Ser Asn Val Ser Thr Lys Ala
               100                 105                 110
   cag ctt tgt tgg gca ctc ctc gca gcg aag cat ctc aag aag gat ttg      384
   Gln Leu Cys Trp Ala Leu Leu Ala Ala Lys His Leu Lys Lys Asp Leu
           115                 120                 125
   aat gct gtc tta ccc gat tca aga tcc ttc ttc gtc gga gtt gta cgc      432
   Asn Ala Val Leu Pro Asp Ser Arg Ser Phe Phe Val Gly Val Val Arg
       130                 135                 140
   ttg aac ggg aaa ctt gga act ttc gaa aac atc agc gac ttc tct aaa      480
   Leu Asn Gly Lys Leu Gly Thr Phe Glu Asn Ile Ser Asp Phe Ser Lys
   145                 150                 155                 160
   ttt gat ttg acg aaa gcc cta gat tac gga cag cgt ggt tct ctc tta      528
   Phe Asp Leu Thr Lys Ala Leu Asp Tyr Gly Gln Arg Gly Ser Leu Leu
                   165                 170                 175
   ggc ctg tgc aag tca cta gac tta gaa tgg gaa cag gtg ttt tgc cgt      576
   Gly Leu Cys Lys Ser Leu Asp Leu Glu Trp Glu Gln Val Phe Cys Arg
               180                 185                 190
   gga ata gat ctt gcg tgt gat ctt atg cca ctc cag gcc gca agg ata      624
   Gly Ile Asp Leu Ala Cys Asp Leu Met Pro Leu Gln Ala Ala Arg Ile
           195                 200                 205
   ctc aga aat gag ctt cag tgt ccc aat atg cgc ctt cgc gag gtt ggg      672
   Leu Arg Asn Glu Leu Gln Cys Pro Asn Met Arg Leu Arg Glu Val Gly
       210                 215                 220
   tac gat att tct ggc gcc agg tac acc att tca acc gat gac ctg cta      720
   Tyr Asp Ile Ser Gly Ala Arg Tyr Thr Ile Ser Thr Asp Asp Leu Leu
   225                 230                 235                 240
   tgt gga ccc tcg aag gct aaa gta gag gcc gca gac ttg ttt ctt gtg      768
   Cys Gly Pro Ser Lys Ala Lys Val Glu Ala Ala Asp Leu Phe Leu Val
                   245                 250                 255
   aca ggt ggc gca cga ggt att aca cct cat tgt gtt cgt gag att gca      816
   Thr Gly Gly Ala Arg Gly Ile Thr Pro His Cys Val Arg Glu Ile Ala
               260                 265                 270
   agt cga tcc ccc gga acc aca ttt gtg ctg gtt gga aga agc gaa atg      864
   Ser Arg Ser Pro Gly Thr Thr Phe Val Leu Val Gly Arg Ser Glu Met
           275                 280                 285
   tcc gac gag cct gac tgg gct gtt ggc cac tac aat aaa gac ctg gac      912
   Ser Asp Glu Pro Asp Trp Ala Val Gly His Tyr Asn Lys Asp Leu Asp
       290                 295                 300
   caa agc aca atg aaa cac ttg aaa gca acg cat gct gct gga ggg gta      960
   Gln Ser Thr Met Lys His Leu Lys Ala Thr His Ala Ala Gly Gly Val
   305                 310                 315                 320
   aaa cct acg cct aaa gca cat cgt gca ctt gtg aac agg gtc act ggc     1008
   Lys Pro Thr Pro Lys Ala His Arg Ala Leu Val Asn Arg Val Thr Gly
                   325                 330                 335
   tca cgg gag gta cga gaa tct ctt aga gca atc cag gag gca ggg gca     1056
   Ser Arg Glu Val Arg Glu Ser Leu Arg Ala Ile Gln Glu Ala Gly Ala
               340                 345                 350
   aat gtc gaa tat atc gcc tgt gat gtt tcg gat gaa aac aag gtc cgc     1104
   Asn Val Glu Tyr Ile Ala Cys Asp Val Ser Asp Glu Asn Lys Val Arg
           355                 360                 365
   caa ctt gtg caa aga gtg gag caa aag tat ggc tgt gaa ata act ggg     1152
   Gln Leu Val Gln Arg Val Glu Gln Lys Tyr Gly Cys Glu Ile Thr Gly
       370                 375                 380
   att tgg cat gca agc ggg gtt ctt cgt gac aaa ctt gtc gag caa aag     1200
   Ile Trp His Ala Ser Gly Val Leu Arg Asp Lys Leu Val Glu Gln Lys
   385                 390                 395                 400
   act aca gac gac ttt gag gca gtt ttt ggg acc aag gtg act ggc ctt     1248
   Thr Thr Asp Asp Phe Glu Ala Val Phe Gly Thr Lys Val Thr Gly Leu
                   405                 410                 415
   gta aac atc gtg tca caa gtc aat atg tct aag cta cga cac ttc atc     1296
   Val Asn Ile Val Ser Gln Val Asn Met Ser Lys Leu Arg His Phe Ile
               420                 425                 430
   ctc ttc agt tct ttg gct gga ttt cat ggg aac aag ggc caa acg gat      1344
   Leu Phe Ser Ser Leu Ala Gly Phe His Gly Asn Lys Gly Gln Thr Asp
           435                 440                 445
   tat gca att gct aat gaa gcc ttg aac aaa atc gcg cat act ctc tca      1392
   Tyr Ala Ile Ala Asn Glu Ala Leu Asn Lys Ile Ala His Thr Leu Ser
       450                 455                 460
   gcg ttt ttg ccc aaa ctg aat gca aag gtg cta gac ttc ggt ccg tgg      1440
   Ala Phe Leu Pro Lys Leu Asn Ala Lys Val Leu Asp Phe Gly Pro Trp
   465                 470                 475                 480
   gta ggt tca gga atg gta acc gaa aca ctt gag aag cat ttt aaa gct      1488
   Val Gly Ser Gly Met Val Thr Glu Thr Leu Glu Lys His Phe Lys Ala
                   485                 490                 495
   atg ggg gtt cag act att cct ctc gag cca gga gca cgg act gtt gcg      1536
   Met Gly Val Gln Thr Ile Pro Leu Glu Pro Gly Ala Arg Thr Val Ala
               500                 505                 510
   caa atc att ttg gca agt tcg cca ccg caa tcg ctt ttg ggg aac tgg      1584
   Gln Ile Ile Leu Ala Ser Ser Pro Pro Gln Ser Leu Leu Gly Asn Trp
           515                 520                 525
   ggc ttt cca gcc acc aaa ccg cta caa cgc tct aat gta gtc acg ggc      1632
   Gly Phe Pro Ala Thr Lys Pro Leu Gln Arg Ser Asn Val Val Thr Gly
       530                 535                 540
   aca ctc tct ccg gaa gag ata gaa ttc atc gca gac cac aaa att caa      1680
   Thr Leu Ser Pro Glu Glu Ile Glu Phe Ile Ala Asp His Lys Ile Gln
   545                 550                 555                 560
   ggc cgc aag gtg ctt ccc atg atg gct gca atc ggg ttc atg gcc tct      1728
   Gly Arg Lys Val Leu Pro Met Met Ala Ala Ile Gly Phe Met Ala Ser
                   565                 570                 575
   att gcg gaa gga ctc tac ccg ggg tac aat ctg caa ggc gtg gaa aat      1776
   Ile Ala Glu Gly Leu Tyr Pro Gly Tyr Asn Leu Gln Gly Val Glu Asn
               580                 585                 590
   gct cag ctc ttt caa ggc ttg act atc aac caa gag aca aaa ttt caa      1824
   Ala Gln Leu Phe Gln Gly Leu Thr Ile Asn Gln Glu Thr Lys Phe Gln
           595                 600                 605
   atc act ctc att gag gag cac aac tct gag gaa aac ctg gat gtc ctg      1872
   Ile Thr Leu Ile Glu Glu His Asn Ser Glu Glu Asn Leu Asp Val Leu
       610                 615                 620
   aca tcc ctt ggt gta atg ttg gaa agc ggg aag gtg ctt ccc gct tac      1920
   Thr Ser Leu Gly Val Met Leu Glu Ser Gly Lys Val Leu Pro Ala Tyr
   625                 630                 635                 640
   cga tgt gtt gta tgc ttg aat aca acc cag cag cag ccc aag cta tct      1968
   Arg Cys Val Val Cys Leu Asn Thr Thr Gln Gln Gln Pro Lys Leu Ser
                   645                 650                 655
   cca aaa att ctt aac ttg gaa gtt gac cct gca tgc gag gtt aac ccc      2016
   Pro Lys Ile Leu Asn Leu Glu Val Asp Pro Ala Cys Glu Val Asn Pro
               660                 665                 670
   tat gat gga aag tcg ttg ttc cac ggt ccg ctt ttg caa ttc gtt caa      2064
   Tyr Asp Gly Lys Ser Leu Phe His Gly Pro Leu Leu Gln Phe Val Gln
           675                 680                 685
   caa gtg ttg cac tca agt acc aaa ggc ctc gtt gcc aag tgc cgc gcg      2112
   Gln Val Leu His Ser Ser Thr Lys Gly Leu Val Ala Lys Cys Arg Ala
       690                 695                 700
   ctt cca atc aaa gaa gcc atc cga ggg cca ttt atc aag caa aca ctc      2160
   Leu Pro Ile Lys Glu Ala Ile Arg Gly Pro Phe Ile Lys Gln Thr Leu
   705                 710                 715                 720
   cat gat cca att cta gac gac gtc att ttt cag cta atg ctc gtg tgg      2208
   His Asp Pro Ile Leu Asp Asp Val Ile Phe Gln Leu Met Leu Val Trp
                   725                 730                 735
   tgt cgt aat gct cta gga agt gca tcg cta ccc aac aga att gaa aag      2256
   Cys Arg Asn Ala Leu Gly Ser Ala Ser Leu Pro Asn Arg Ile Glu Lys
               740                 745                 750
   atg tca tac ttt ggg aat gtc tca gaa ggt agc act ttc ttt gcc tca      2304
   Met Ser Tyr Phe Gly Asn Val Ser Glu Gly Ser Thr Phe Phe Ala Ser
           755                 760                 765
   gtt aca cct gtg gga cca aga gta cca aag gat ccc gtg atc aaa atg      2352
   Val Thr Pro Val Gly Pro Arg Val Pro Lys Asp Pro Val Ile Lys Met
       770                 775                 780
   cag ttt ctt ctc caa gat gaa tcc ggc aac aca ttt tca tcg ggg gag      2400
   Gln Phe Leu Leu Gln Asp Glu Ser Gly Asn Thr Phe Ser Ser Gly Glu
   785                 790                 795                 800
   ggc tcg gtt gtg ctt agt gac gaa ctc gtc ttt                          2433
   Gly Ser Val Val Leu Ser Asp Glu Leu Val Phe
                   805                 810
<210> 50
   <211> 811
   <212> PRT
   <213> Thraustochytrium sp.
<400> 50
   Lys Ser Val Lys Ala Ser Gly Cys Glu Asn Val Asp Thr Arg Phe Ala
   1               5                   10                  15
   Lys Val Val Gln Ile Ser Leu Pro Ser Lys Leu Lys Ser Thr Val Ser
               20                  25                  30
   His Asp Arg Pro Val Ile Val Val Asp Asp Gly Thr Pro Leu Thr Thr
           35                  40                  45
   Glu Leu Cys Lys Ile Leu Gly Gly Asn Ile Val Val Leu Ser Tyr Gln
       50                  55                  60
   Gly Lys Pro Ala Gly Pro Arg Gly Val Glu Val Pro Asp Leu Ser Glu
   65                  70                  75                  80
   Glu Ala Leu Ile Gln Ala Leu Ala Leu Ile Arg Ser Thr Tyr Gly Val
                   85                  90                  95
   Pro Ile Gly Phe Ile Cys Gln Gln Val Ser Asn Val Ser Thr Lys Ala
               100                 105                 110
   Gln Leu Cys Trp Ala Leu Leu Ala Ala Lys His Leu Lys Lys Asp Leu
           115                 120                 125
   Asn Ala Val Leu Pro Asp Ser Arg Ser Phe Phe Val Gly Val Val Arg
       130                 135                 140
   Leu Asn Gly Lys Leu Gly Thr Phe Glu Asn Ile Ser Asp Phe Ser Lys
   145                 150                 155                 160
   Phe Asp Leu Thr Lys Ala Leu Asp Tyr Gly Gln Arg Gly Ser Leu Leu
                   165                 170                 175
   Gly Leu Cys Lys Ser Leu Asp Leu Glu Trp Glu Gln Val Phe Cys Arg
               180                 185                 190
   Gly Ile Asp Leu Ala Cys Asp Leu Met Pro Leu Gln Ala Ala Arg Ile
           195                 200                 205
   Leu Arg Asn Glu Leu Gln Cys Pro Asn Met Arg Leu Arg Glu Val Gly
       210                 215                 220
   Tyr Asp Ile Ser Gly Ala Arg Tyr Thr Ile Ser Thr Asp Asp Leu Leu
   225                 230                 235                 240
   Cys Gly Pro Ser Lys Ala Lys Val Glu Ala Ala Asp Leu Phe Leu Val
                   245                 250                 255
   Thr Gly Gly Ala Arg Gly Ile Thr Pro His Cys Val Arg Glu Ile Ala
               260                 265                 270
   Ser Arg Ser Pro Gly Thr Thr Phe Val Leu Val Gly Arg Ser Glu Met
           275                 280                 285
   Ser Asp Glu Pro Asp Trp Ala Val Gly His Tyr Asn Lys Asp Leu Asp
       290                 295                 300
   Gln Ser Thr Met Lys His Leu Lys Ala Thr His Ala Ala Gly Gly Val
   305                 310                 315                 320
   Lys Pro Thr Pro Lys Ala His Arg Ala Leu Val Asn Arg Val Thr Gly
                   325                 330                 335
   Ser Arg Glu Val Arg Glu Ser Leu Arg Ala Ile Gln Glu Ala Gly Ala
               340                 345                 350
   Asn Val Glu Tyr Ile Ala Cys Asp Val Ser Asp Glu Asn Lys Val Arg
           355                 360                 365
   Gln Leu Val Gln Arg Val Glu Gln Lys Tyr Gly Cys Glu Ile Thr Gly
       370                 375                 380
   Ile Trp His Ala Ser Gly Val Leu Arg Asp Lys Leu Val Glu Gln Lys
   385                 390                 395                 400
   Thr Thr Asp Asp Phe Glu Ala Val Phe Gly Thr Lys Val Thr Gly Leu
                   405                 410                 415
   Val Asn Ile Val Ser Gln Val Asn Met Ser Lys Leu Arg His Phe Ile
               420                 425                 430
   Leu Phe Ser Ser Leu Ala Gly Phe His Gly Asn Lys Gly Gln Thr Asp
           435                 440                 445
   Tyr Ala Ile Ala Asn Glu Ala Leu Asn Lys Ile Ala His Thr Leu Ser
   450 455 460
   Ala Phe Leu Pro Lys Leu Asn Ala Lys Val Leu Asp Phe Gly Pro Trp
   465 470 475 480
   Val Gly Ser Gly Met Val Thr Glu Thr Leu Glu Lys His Phe Lys Ala
   485 490 495
   Met Gly Val Gln Thr Ile Pro Leu Glu Pro Gly Ala Arg Thr Val Ala
   500 505 510
   Gln Ile Ile Leu Ala Ser Ser Pro Pro Gln Ser Leu Leu Gly Asn Trp
   515 520 525
   Gly Phe Pro Ala Thr Lys Pro Leu Gln Arg Ser Asn Val Val Thr Gly
   530 535 540
   Thr Leu Ser Pro Glu Glu Ile Glu Phe Ile Ala Asp His Lys Ile Gln
   545 550 555 560
   Gly Arg Lys Val Leu Pro Met Met Ala Ala Ile Gly Phe Met Ala Ser
   565 570 575
   Ile Ala Glu Gly Leu Tyr Pro Gly Tyr Asn Leu Gln Gly Val Glu Asn
   580 585 590
   Ala Gln Leu Phe Gln Gly Leu Thr Ile Asn Gln Glu Thr Lys Phe Gln
   595 600 605
   Ile Thr Leu Ile Glu Glu His Asn Ser Glu Glu Asn Leu Asp Val Leu
   610 615 620
   Thr Ser Leu Gly Val Met Leu Glu Ser Gly Lys Val Leu Pro Ala Tyr
   625 630 635 640
   Arg Cys Val Val Cys Leu Asn Thr Thr Gln Gln Gln Pro Lys Leu Ser
   645 650 655
   Pro Lys Ile Leu Asn Leu Glu Val Asp Pro Ala Cys Glu Val Asn Pro
   660 665 670
   Tyr Asp Gly Lys Ser Leu Phe His Gly Pro Leu Leu Gln Phe Val Gln
   675 680 685
   Gln Val Leu His Ser Ser Thr Lys Gly Leu Val Ala Lys Cys Arg Ala
   690 695 700
   Leu Pro Ile Lys Glu Ala Ile Arg Gly Pro Phe Ile Lys Gln Thr Leu
   705 710 715 720
   His Asp Pro Ile Leu Asp Asp Val Ile Phe Gln Leu Met Leu Val Trp
   725 730 735
   Cys Arg Asn Ala Leu Gly Ser Ala Ser Leu Pro Asn Arg Ile Glu Lys
               740                 745                 750
   Met Ser Tyr Phe Gly Asn Val Ser Glu Gly Ser Thr Phe Phe Ala Ser
           755                 760                 765
   Val Thr Pro Val Gly Pro Arg Val Pro Lys Asp Pro Val Ile Lys Met
       770                 775                 780
   Gln Phe Leu Leu Gln Asp Glu Ser Gly Asn Thr Phe Ser Ser Gly Glu
   785                 790                 795                 800
   Gly Ser Val Val Leu Ser Asp Glu Leu Val Phe
                   805                 810
<210> 51
   <211> 5808
   <212> DNA
   <213> Thraustochytrium sp.
<220>
   <221> CDS
   <222> (1)..(5805)
<220>
   <221> misc_feature
   <222> (1)..(5808)
   <223> n = a c t or g
<400> 51
   atg caa ctt cct cca gcg cat tct gcc gat gag aat cgc atc gcg gtc      48
   Met Gln Leu Pro Pro Ala His Ser Ala Asp Glu Asn Arg Ile Ala Val
   1               5                   10                  15
   gtg ggc atg gcc gtc aaa tat gcg ggc tgt gac aat aaa gaa gag ttt      96
   Val Gly Met Ala Val Lys Tyr Ala Gly Cys Asp Asn Lys Glu Glu Phe
               20                  25                  30
   tgg aag act ttg atg aat ggt agt atc aat acc aag tcg att tcg gca     144
   Trp Lys Thr Leu Met Asn Gly Ser Ile Asn Thr Lys Ser Ile Ser Ala
           35                  40                  45
   gca agg ttg ggc agc aat aag cgt gac gaa cac tat gtt cct gaa cga     192
   Ala Arg Leu Gly Ser Asn Lys Arg Asp Glu His Tyr Val Pro Glu Arg
       50                  55                  60
   tcg aaa tat gca gat acg ttc tgt aac gaa agg tac ggt tgt atc cag     240
   Ser Lys Tyr Ala Asp Thr Phe Cys Asn Glu Arg Tyr Gly Cys Ile Gln
   65                  70                  75                  80
   caa ggt acg gat aat gag cat gac ctc ctc cta ggt ctt gct caa gaa     288
   Gln Gly Thr Asp Asn Glu His Asp Leu Leu Leu Gly Leu Ala Gln Glu
                   85                  90                  95
   gct ctc gct gac gct gcc ggg cgg atg gag aaa caa cct tcg gag gcg     336
   Ala Leu Ala Asp Ala Ala Gly Arg Met Glu Lys Gln Pro Ser Glu Ala
               100                 105                 110
   ttc gat ctg gaa aat act ggc atc gtg agt ggg tgc tta tct ttt cca     384
   Phe Asp Leu Glu Asn Thr Gly Ile Val Ser Gly Cys Leu Ser Phe Pro
           115                 120                 125
   atg gat aac ctg caa gga gag ttg ttg aac ttg tat caa agc cat gtg     432
   Met Asp Asn Leu Gln Gly Glu Leu Leu Asn Leu Tyr Gln Ser His Val
       130                 135                 140
   gag aaa caa ctt cca cct agt gcc ttg gta gaa gcc gtg aag ctt tgg      480
   Glu Lys Gln Leu Pro Pro Ser Ala Leu Val Glu Ala Val Lys Leu Trp
   145                 150                 155                 160
   tct gag cga cag aaa tct acg aaa gca cat gca ggg gac aag cgc cgg      528
   Ser Glu Arg Gln Lys Ser Thr Lys Ala His Ala Gly Asp Lys Arg Arg
                   165                 170                 175
   ttc att gac cca gct tct ttt gta gct gat aaa ctg aac cta ggc cca      576
   Phe Ile Asp Pro Ala Ser Phe Val Ala Asp Lys Leu Asn Leu Gly Pro
               180                 185                 190
   cta cat tat gcg atc gat gca gca tgc gct tct gca ttg tac gtg tta      624
   Leu His Tyr Ala Ile Asp Ala Ala Cys Ala Ser Ala Leu Tyr Val Leu
           195                 200                 205
   aaa tta gct caa gac cac ctt gtt tca ggt gcc gtt gat atg atg tta      672
   Lys Leu Ala Gln Asp His Leu Val Ser Gly Ala Val Asp Met Met Leu
       210                 215                 220
   tgt gga gcg acg tgc ttc cca gaa cca ttc ttc atc ttg tct ggg ttc      720
   Cys Gly Ala Thr Cys Phe Pro Glu Pro Phe Phe Ile Leu Ser Gly Phe
   225                 230                 235                 240
   tcg act ttt caa gcg atg cct gnt ggg gca gat gga gtc tca cta cct      768
   Ser Thr Phe Gln Ala Met Pro Xaa Gly Ala Asp Gly Val Ser Leu Pro
                   245                 250                 255
   ctc cat aaa acg agt gct ggg ctc act cca ggt gaa ggg ggg tcc att      816
   Leu His Lys Thr Ser Ala Gly Leu Thr Pro Gly Glu Gly Gly Ser Ile
               260                 265                 270
   atg gtg ctc aag cga ctg aaa gac gct atc aga gat gga aat cac att      864
   Met Val Leu Lys Arg Leu Lys Asp Ala Ile Arg Asp Gly Asn His Ile
           275                 280                 285
   tat ggt gtg ctc ctt gaa gca aat tta agt aac gca ggt tgt ggg ctt      912
   Tyr Gly Val Leu Leu Glu Ala Asn Leu Ser Asn Ala Gly Cys Gly Leu
       290                 295                 300
   cca ctc agc ccg cac tta ccg agc gaa gaa tca tgt att cgt gat acc      960
   Pro Leu Ser Pro His Leu Pro Ser Glu Glu Ser Cys Ile Arg Asp Thr
   305                 310                 315                 320
   tac cgc cgt gct gga gtt gct gca gat caa agt att cag tat att gag     1008
   Tyr Arg Arg Ala Gly Val Ala Ala Asp Gln Ser Ile Gln Tyr Ile Glu
                   325                 330                 335
   tgc cac gct acg gga acc cct cga ggg gat gtc gtg gaa att gag gcg     1056
   Cys His Ala Thr Gly Thr Pro Arg Gly Asp Val Val Glu Ile Glu Ala
               340                 345                 350
   gtt gaa aga gtt ttc aag aaa aac gtt cca cgc tta ggc tcg acg aaa     1104
   Val Glu Arg Val Phe Lys Lys Asn Val Pro Arg Leu Gly Ser Thr Lys
           355                 360                 365
   gga aat ttt ggt cac tcg tta gtt gcg gct ggt ttc gca ggt atg gca     1152
   Gly Asn Phe Gly His Ser Leu Val Ala Ala Gly Phe Ala Gly Met Ala
       370                 375                 380
   aag ctt ctt ctt gca atg gaa cat gga gtg att cct ccc aca cca ggt     1200
   Lys Leu Leu Leu Ala Met Glu His Gly Val Ile Pro Pro Thr Pro Gly
   385                 390                 395                 400
   ctt gat gct tcg aac cag gca agt gag cac gtt gtg aca aag gct atc     1248
   Leu Asp Ala Ser Asn Gln Ala Ser Glu His Val Val Thr Lys Ala Ile
                   405                 410                 415
   act tgg cct gag aca cat ggg gct cca aaa cga gct ggc ctt tca gca     1296
   Thr Trp Pro Glu Thr His Gly Ala Pro Lys Arg Ala Gly Leu Ser Ala
               420                 425                 430
   ttt gga ttt ggt ggg act aat gcg cat gca ctc ttc gaa gag ttt aat      1344
   Phe Gly Phe Gly Gly Thr Asn Ala His Ala Leu Phe Glu Glu Phe Asn
           435                 440                 445
   gcc gag ggc ata agt tat cgc cct gga aag cct cca gtc gaa tcg aat      1392
   Ala Glu Gly Ile Ser Tyr Arg Pro Gly Lys Pro Pro Val Glu Ser Asn
       450                 455                 460
   acc cgt cct tcc gtc gta ata act ggg atg gac tgt acc ttt ggg agc      1440
   Thr Arg Pro Ser Val Val Ile Thr Gly Met Asp Cys Thr Phe Gly Ser
   465                 470                 475                     480
   ctt gaa ggg att gat gcg ttc gag act gcc ctg tac gag ggg cgt gac      1488
   Leu Glu Gly Ile Asp Ala Phe Glu Thr Ala Leu Tyr Glu Gly Arg Asp
                   485                 490                 495
   gca gct cgt gac tta ccc gcc aaa cgt tgg agg ttc cta ggt gag gac      1536
   Ala Ala Arg Asp Leu Pro Ala Lys Arg Trp Arg Phe Leu Gly Glu Asp
               500                 505                 510
   ttg gag ttt ctc cga gcc atc agg ctc aag gaa aag cct agg ggt tgt      1584
   Leu Glu Phe Leu Arg Ala Ile Arg Leu Lys Glu Lys Pro Arg Gly Cys
           515                 520                 525
   ttt gtg gag agt gtt gac gtt aac ttt aga cgg ctg aaa acg ccc ttg      1632
   Phe Val Glu Ser Val Asp Val Asn Phe Arg Arg Leu Lys Thr Pro Leu
       530                 535                 540
   aca cca gaa gat atg ttg cgg ccc caa caa ctc ttg gcg gtt tct acg      1680
   Thr Pro Glu Asp Met Leu Arg Pro Gln Gln Leu Leu Ala Val Ser Thr
   545                 550                 555                     560
   atg gac cga gca att atc gat gca ggt cta aag aag ggc caa cat gta      1728
   Met Asp Arg Ala Ile Ile Asp Ala Gly Leu Lys Lys Gly Gln His Val
                   565                 570                 575
   gca gtt ctt gtt ggc cta gga act gac ctg gaa ctt tac cgt cat cga      1776
   Ala Val Leu Val Gly Leu Gly Thr Asp Leu Glu Leu Tyr Arg His Arg
               580                 585                 590
   gca aga gtc gcg ctt aaa gag gtt ttg cac ccg agc tta aag tca gac      1824
   Ala Arg Val Ala Leu Lys Glu Val Leu His Pro Ser Leu Lys Ser Asp
           595                 600                 605
   act gca att ctc cag aaa ata atg caa tat gtg aat gat gca gga act      1872
   Thr Ala Ile Leu Gln Lys Ile Met Gln Tyr Val Asn Asp Ala Gly Thr
       610                 615                 620
   tcg act tca tac aca tct tac att gga aac ctc gtt gcc acg cgt att      1920
   Ser Thr Ser Tyr Thr Ser Tyr Ile Gly Asn Leu Val Ala Thr Arg Ile
   625                 630                 635                     640
   tcg tct cag tgg gga ttc aca ggg ccg tcc ttt act gtc aca gaa gga      1968
   Ser Ser Gln Trp Gly Phe Thr Gly Pro Ser Phe Thr Val Thr Glu Gly
                   645                 650                 655
   aat aat tcc gtg tac aga tgt gca caa cta gcc aaa gat atg ctt cag      2016
   Asn Asn Ser Val Tyr Arg Cys Ala Gln Leu Ala Lys Asp Met Leu Gln
               660                 665                 670
   gtt aac cga gtt gat gct gtc gtc atc gca ggc gtt gat ctc aac gga      2064
   Val Asn Arg Val Asp Ala Val Val Ile Ala Gly Val Asp Leu Asn Gly
           675                 680                 685
   agc gcc gaa agt ttt ttt gtc cga gca aat cgt caa aag ata tcc aag      2112
   Ser Ala Glu Ser Phe Phe Val Arg Ala Asn Arg Gln Lys Ile Ser Lys
       690                 695                 700
   cta agt cat cca tgt gca agc ttc gac aga gat gca gat gga ttt ttc      2160
   Leu Ser His Pro Cys Ala Ser Phe Asp Arg Asp Ala Asp Gly Phe Phe
   705                 710                 715                     720
   gca ggt gag ggc tgt ggt gcc cta gtt ttc aag agg tta gaa gac tgt      2208
   Ala Gly Glu Gly Cys Gly Ala Leu Val Phe Lys Arg Leu Glu Asp Cys
                       725                 730                 735
   gct cct cag gaa aaa att tat gct agt ata gac tct atc gca ata gat      2256
   Ala Pro Gln Glu Lys Ile Tyr Ala Ser Ile Asp Ser Ile Ala Ile Asp
                   740                 745                 750
   aaa gag cct act agc tca gct gtg aaa gct gtc tac caa agt gat tcg      2304
   Lys Glu Pro Thr Ser Ser Ala Val Lys Ala Val Tyr Gln Ser Asp Ser
               755                 760                 765
   agt ctc tcc gat att gag ctg tta gaa atc agt gga gac tcc aaa cgg      2352
   Ser Leu Ser Asp Ile Glu Leu Leu Glu Ile Ser Gly Asp Ser Lys Arg
           770                 775                 780
   ttt gca gca ttc gaa ggc gct gtg gaa att caa tca agt gtg gaa gcc      2400
   Phe Ala Ala Phe Glu Gly Ala Val Glu Ile Gln Ser Ser Val Glu Ala
   785                 790                 795                 800
   cag cta aaa gga ctt tcc aaa gtc ctt gaa cct gca aaa ggc caa ggc      2448
   Gln Leu Lys Gly Leu Ser Lys Val Leu Glu Pro Ala Lys Gly Gln Gly
                   805                 810                 815
   gta gcg gtg gga agt act cga gca acc gtt ggg gat ata ggg tat gct      2496
   Val Ala Val Gly Ser Thr Arg Ala Thr Val Gly Asp Ile Gly Tyr Ala
               820                 825                 830
   aca gga gcg gca agc ctg att aaa act gca ctc tgc tta tat aat cgc      2544
   Thr Gly Ala Ala Ser Leu Ile Lys Thr Ala Leu Cys Leu Tyr Asn Arg
           835                 840                 845
   tac ctt ccg gca tta gca aac tgg agt ggc cca tgt gaa cag tcc gcc      2592
   Tyr Leu Pro Ala Leu Ala Asn Trp Ser Gly Pro Cys Glu Gln Ser Ala
       850                 855                 860
   tgg ggc tca aac atg ttc gtt tgc cat gaa aca cgg ccg tgg atg aaa      2640
   Trp Gly Ser Asn Met Phe Val Cys His Glu Thr Arg Pro Trp Met Lys
   865                 870                 875                 880
   aac cag aat gaa aag aga tgt gcc ctc att tct gga aca gat cca tct      2688
   Asn Gln Asn Glu Lys Arg Cys Ala Leu Ile Ser Gly Thr Asp Pro Ser
                   885                 890                 895
   cat aca tgc ttt tcc ctc gta cta tcg gat act ggg tgt tat gaa gag      2736
   His Thr Cys Phe Ser Leu Val Leu Ser Asp Thr Gly Cys Tyr Glu Glu
               900                 905                 910
   cac aat cga acg tgc ttt gat gtg caa gcg cca cag cta gtt ctg ata      2784
   His Asn Arg Thr Cys Phe Asp Val Gln Ala Pro Gln Leu Val Leu Ile
           915                 920                 925
   cac gga ttc gat gga aaa act att gtg cgg cga ctt gaa gga tat ctc      2832
   His Gly Phe Asp Gly Lys Thr Ile Val Arg Arg Leu Glu Gly Tyr Leu
       930                 935                 940
   ctt gaa ctt gtt gaa ggg cat gca agc cct tca gag tat ttc cac aaa      2880
   Leu Glu Leu Val Glu Gly His Ala Ser Pro Ser Glu Tyr Phe His Lys
   945                 950                 955                 960
   ctg att gga caa agt cta ctt gag aac tcg aaa gaa agt aaa ctc aca      2928
   Leu Ile Gly Gln Ser Leu Leu Glu Asn Ser Lys Glu Ser Lys Leu Thr
                   965                 970                 975
   ctt tcg ctt gtg tgc aat ccg aac cag ctc caa aag gag ctc atg ctt      2976
   Leu Ser Leu Val Cys Asn Pro Asn Gln Leu Gln Lys Glu Leu Met Leu
               980                 985                 990
   gct atc aaa gga gta caa cga agc  atg tta aca ggg aag  gat tgg gtc    3024
   Ala Ile Lys Gly Val Gln Arg Ser  Met Leu Thr Gly Lys  Asp Trp Val
           995                 1000                1005
   agt cca  tca gga agt tgt ttt  gcc cca aat ccg tta  tca agc gca    3069
   Ser Pro  Ser Gly Ser Cys Phe  Ala Pro Asn Pro Leu  Ser Ser Ala
       1010                 1015                 1020
   aaa gtg  gca ttc atg tac gga  gaa ggc cga agc ccg  tac tgt ggt    3114
   Lys Val  Ala Phe Met Tyr Gly  Glu Gly Arg Ser Pro  Tyr Cys Gly
       1025                 1030                 1035
   gta ggc  ttg ggt cta cat cgt  ttg tgg ccc ggt ctc  cat gaa aat    3159
   Val Gly  Leu Gly Leu His Arg  Leu Trp Pro Gly Leu  His Glu Asn
       1040                 1045                 1050
   gtg aac  aat aag aca gtc gat  tta tgg acg gaa gga  gat ggt tgg    3204
   Val Asn  Asn Lys Thr Val Asp  Leu Trp Thr Glu Gly  Asp Gly Trp
       1055                 1060                 1065
   tta tat  cct cga acg ttg aca  cga gaa gag cat aca  aaa gcc atc    3249
   Leu Tyr  Pro Arg Thr Leu Thr  Arg Glu Glu His Thr  Lys Ala Ile
       1070                 1075                 1080
   gaa tct  ttc aac gca aat caa  att gaa atg ttt cgc  gct ggg att    3294
   Glu Ser  Phe Asn Ala Asn Gln  Ile Glu Met Phe Arg  Ala Gly Ile
       1085                 1090                 1095
   ttc atc  tca atg tgt cag aca  gac tat gtc atg aat  gtt ctc ggt    3339
   Phe Ile  Ser Met Cys Gln Thr  Asp Tyr Val Met Asn  Val Leu Gly
       1100                 1105                 1110
   gtc cag  cct aag gcc gga ttt  ggg ctg agc ttg gga  gaa att tca    3384
   Val Gln  Pro Lys Ala Gly Phe  Gly Leu Ser Leu Gly  Glu Ile Ser
       1115                 1120                 1125
   atg ctc  ttt gcg atg tca aag  gag aac tgc agg cag  tca cag gaa    3429
   Met Leu  Phe Ala Met Ser Lys  Glu Asn Cys Arg Gln  Ser Gln Glu
       1130                 1135                 1140
   atg acc  aat cgt ttg cgc ggt  tct cca gtg tgg tct  aac gag ctt    3474
   Met Thr  Asn Arg Leu Arg Gly  Ser Pro Val Trp Ser  Asn Glu Leu
       1145                 1150                 1155
   gct atc  aac ttc aat gca att  cgc aag tta tgg aaa  atc ccc cga    3519
   Ala Ile  Asn Phe Asn Ala Ile  Arg Lys Leu Trp Lys  Ile Pro Arg
       1160                 1165                 1170
   gga gct  ccc tta gaa tcc ttt  tgg caa gga tac ttg  gtt cac ggc    3564
   Gly Ala  Pro Leu Glu Ser Phe  Trp Gln Gly Tyr Leu  Val His Gly
       1175                 1180                 1185
   aca aga  gaa gaa gta gag cat  gct att ggt ctt tct  gag cct tat    3609
   Thr Arg  Glu Glu Val Glu His  Ala Ile Gly Leu Ser  Glu Pro Tyr
       1190                 1195                 1200
   gta cgt  ctg ctt att gtg aac  gat tca agg agt gcc  ttg att gct    3654
   Val Arg  Leu Leu Ile Val Asn  Asp Ser Arg Ser Ala  Leu Ile Ala
       1205                 1210                 1215
   gga aaa  cca gac gcc tgt cag  gca gta atc agt aga  cta aac tcc    3699
   Gly Lys  Pro Asp Ala Cys Gln  Ala Val Ile Ser Arg  Leu Asn Ser
       1220                 1225                 1230
   aag ttc  cct tct ctg ccg gta  aag caa gga atg att  ggt cat tgc    3744
   Lys Phe  Pro Ser Leu Pro Val  Lys Gln Gly Met Ile  Gly His Cys
       1235                 1240                 1245
   cca gaa  gtt cgt gcg ttc atc  aaa gat att ggg tac  atc cat gaa    3789
   Pro Glu  Val Arg Ala Phe Ile  Lys Asp Ile Gly Tyr  Ile His Glu
       1250                 1255                 1260
   aca ctc  cga att tcc aat gac  tat tcg gat tgt cag  ctt ttc tca    3834
   Thr Leu  Arg Ile Ser Asn Asp  Tyr Ser Asp Cys Gln  Leu Phe Ser
       1265                 1270                 1275
   gcg gta  acc aag ggc gca ctt  gac agc tcc aca atg  gaa atc aaa       3879
   Ala Val  Thr Lys Gly Ala Leu  Asp Ser Ser Thr Met  Glu Ile Lys
       1280                 1285                 1290
   cac ttt  gtg gga gag gtc tac  tcc cgg atc gca gac  ttt cct caa       3924
   His Phe  Val Gly Glu Val Tyr  Ser Arg Ile Ala Asp  Phe Pro Gln
       1295                 1300                 1305
   atc gtc  aac acg gtg cat tcg  gct ggt tat gac gta  ttt ctt gag       3969
   Ile Val  Asn Thr Val His Ser  Ala Gly Tyr Asp Val  Phe Leu Glu
       1310                 1315                 1320
   ctt ggc  tgt gat gct tct aga  tct gca gca gtt caa  aac att ctt       4014
   Leu Gly  Cys Asp Ala Ser Arg  Ser Ala Ala Val Gln  Asn Ile Leu
       1325                 1330                 1335
   ggt ggt  caa gga aag ttc ttg  tct aca gct att gac  aaa aaa gga       4059
   Gly Gly  Gln Gly Lys Phe Leu  Ser Thr Ala Ile Asp  Lys Lys Gly
       1340                 1345                 1350
   cac tcc  gcc tgg tca caa gta  ctt cgg gct acc gca  tca tta gct       4104
   His Ser  Ala Trp Ser Gln Val  Leu Arg Ala Thr Ala  Ser Leu Ala
       1355                 1360                 1365
   gca cat  cga gta ccg gga atc  tca att ttg gat ttg  ttt cac cca       4149
   Ala His  Arg Val Pro Gly Ile  Ser Ile Leu Asp Leu  Phe His Pro
       1370                 1375                 1380
   aat ttc  cga gaa atg tgc tgt  aca atg gca acc aca  cct aaa gtg       4194
   Asn Phe  Arg Glu Met Cys Cys  Thr Met Ala Thr Thr  Pro Lys Val
       1385                 1390                 1395
   gaa gat  aag ttc ctg cgc acg  att caa atc aat ggt  cgg ttt gaa       4239
   Glu Asp  Lys Phe Leu Arg Thr  Ile Gln Ile Asn Gly  Arg Phe Glu
       1400                 1405                 1410
   aaa gaa  atg att cac cta gaa  gat aca aca tta agt  tgc tta ccc       4284
   Lys Glu  Met Ile His Leu Glu  Asp Thr Thr Leu Ser  Cys Leu Pro
       1415                 1420                 1425
   gct cca  agt gaa gca aat atc  gca gct att caa tct  cgg tca att       4329
   Ala Pro  Ser Glu Ala Asn Ile  Ala Ala Ile Gln Ser  Arg Ser Ile
       1430                 1435                 1440
   cga tct  gct gcg gcg cgt tct  gga caa tcc cat gat  tgt gca tcc       4374
   Arg Ser  Ala Ala Ala Arg Ser  Gly Gln Ser His Asp  Cys Ala Ser
       1445                 1450                 1455
   cat agc  cat gaa gaa aat aag  gat tca tgc cct gaa  aag ctg aag       4419
   His Ser  His Glu Glu Asn Lys  Asp Ser Cys Pro Glu  Lys Leu Lys
       1460                 1465                 1470
   ctt gat  tct gtg tcc gtc gcc  ata aat ttc gac aat  gat gac cgc       4464
   Leu Asp  Ser Val Ser Val Ala  Ile Asn Phe Asp Asn  Asp Asp Arg
       1475                 1480                 1485
   att cag  ctt ggg cac gcg ggt  ttt cgg gag atg tac  aat aca aga       4509
   Ile Gln  Leu Gly His Ala Gly  Phe Arg Glu Met Tyr  Asn Thr Arg
       1490                 1495                 1500
   tat agc  ttg tac aca ggg gcg  atg gca aag gga att  gca tct gca       4554
   Tyr Ser  Leu Tyr Thr Gly Ala  Met Ala Lys Gly Ile  Ala Ser Ala
       1505                 1510                 1515
   gat ctt  gtc att gcc gct ggg  aaa gag ggc atc cta  gct tcc tat       4599
   Asp Leu  Val Ile Ala Ala Gly  Lys Glu Gly Ile Leu  Ala Ser Tyr
       1520                 1525                 1530
   gga gct  gga gga cta cct ctt  gct act gtt cga aag  gga ata gac       4644
   Gly Ala  Gly Gly Leu Pro Leu  Ala Thr Val Arg Lys  Gly Ile Asp
       1535                 1540                 1545
   aaa att  caa caa gcc ttg cca  agt ggc cca tat gct  gta aat ctt       4689
   Lys Ile  Gln Gln Ala Leu Pro  Ser Gly Pro Tyr Ala  Val Asn Leu
       1550                 1555                 1560
   att cac  tct ccc ttt gac ggc  aac ttg gag cag gga  aac gtc gat       4734
   Ile His  Ser Pro Phe Asp Gly  Asn Leu Glu Gln Gly  Asn Val Asp
       1565                 1570                 1575
   ttg ttc  ttg gaa aag aac gtc  cgc gtg gcg gaa tgt  tcc gcg ttt       4779
   Leu Phe  Leu Glu Lys Asn Val  Arg Val Ala Glu Cys  Ser Ala Phe
       1580                 1585                 1590
   aca acg  cta aca gtg cca gta  gta cac tat cgt gct  gca ggg ctt       4824
   Thr Thr  Leu Thr Val Pro Val  Val His Tyr Arg Ala  Ala Gly Leu
       1595                 1600                 1605
   gtt cgg  cgc caa gat gga agc  att ttg atc aag aac  cga atc att       4869
   Val Arg  Arg Gln Asp Gly Ser  Ile Leu Ile Lys Asn  Arg Ile Ile
       1610                 1615                 1620
   gct aaa  gta tct agg aca gaa  ctc gct gag atg ttc  ctt cgt ccg       4914
   Ala Lys  Val Ser Arg Thr Glu  Leu Ala Glu Met Phe  Leu Arg Pro
       1625                 1630                 1635
   gca cct  caa atc atc ctc gaa  aaa ctg gta gca gca  gaa atc att       4959
   Ala Pro  Gln Ile Ile Leu Glu  Lys Leu Val Ala Ala  Glu Ile Ile
       1640                 1645                 1650
   tca tct  gac caa gcg cgt atg  gca gcc aaa gtt ccc  atg gcg gac       5004
   Ser Ser  Asp Gln Ala Arg Met  Ala Ala Lys Val Pro  Met Ala Asp
       1655                 1660                 1665
   gac atc  gca gtc gaa gcc gac  tct ggt ggg cac acg  gat aat cgg       5049
   Asp Ile  Ala Val Glu Ala Asp  Ser Gly Gly His Thr  Asp Asn Arg
       1670                 1675                 1680
   cct atg  cac gtc att ttg ccc  ctg ata att caa ctc  cgc aat act       5094
   Pro Met  His Val Ile Leu Pro  Leu Ile Ile Gln Leu  Arg Asn Thr
       1685                 1690                 1695
   ata ctt  gca gag tat ggc tgt  gcc acg gct ttt cgt  acc cgt ata       5139
   Ile Leu  Ala Glu Tyr Gly Cys  Ala Thr Ala Phe Arg  Thr Arg Ile
       1700                 1705                 1710
   ggc gct  gga gga ggc att ggt  tgt cct tca gcg gcc  ctc gca gcc       5184
   Gly Ala  Gly Gly Gly Ile Gly  Cys Pro Ser Ala Ala  Leu Ala Ala
       1715                 1720                 1725
   ttt gat  atg ggt gcg agt ttt  gtc gtg act gga agc  ata aat caa       5229
   Phe Asp  Met Gly Ala Ser Phe  Val Val Thr Gly Ser  Ile Asn Gln
       1730                 1735                 1740
   att tgc  cgc gag gca ggg act  tgc gat act gtt cgg  gag cta ctt       5274
   Ile Cys  Arg Glu Ala Gly Thr  Cys Asp Thr Val Arg  Glu Leu Leu
       1745                 1750                 1755
   gcc aac  tca agc tac tcg gac  gtg acg atg gcg cca  gca gca gac       5319
   Ala Asn  Ser Ser Tyr Ser Asp  Val Thr Met Ala Pro  Ala Ala Asp
       1760                 1765                 1770
   atg ttt  gac caa ggt gtg aaa  ctc caa gtc tta aaa  cga gga acg       5364
   Met Phe  Asp Gln Gly Val Lys  Leu Gln Val Leu Lys  Arg Gly Thr
       1775                 1780                 1785
   atg ttt  cca agc aga gca aat  aaa ctc cgg aag ctc  ttt gtg aac       5409
   Met Phe  Pro Ser Arg Ala Asn  Lys Leu Arg Lys Leu  Phe Val Asn
       1790                 1795                 1800
   tac gaa  tct cta gaa aca ctc  ccg tcg aaa gag ttg  aaa tac ctg       5454
   Tyr Glu  Ser Leu Glu Thr Leu  Pro Ser Lys Glu Leu  Lys Tyr Leu
       1805                 1810                 1815
   gaa aac  atc ata ttc aag caa  gca gta gac cag gtg  tgg gag gaa        5499
   Glu Asn  Ile Ile Phe Lys Gln  Ala Val Asp Gln Val  Trp Glu Glu
       1820                 1825                 1830
   aca aag  cgc ttt tac tgt gaa  aaa ctg aac aat cca  gat aaa att        5544
   Thr Lys  Arg Phe Tyr Cys Glu  Lys Leu Asn Asn Pro  Asp Lys Ile
       1835                 1840                 1845
   gca agg  gcc atg aaa gat cct  aaa ttg aag atg tcg  ctt tgc ttt        5589
   Ala Arg  Ala Met Lys Asp Pro  Lys Leu Lys Met Ser  Leu Cys Phe
       1850                 1855                 1860
   cgg tgg  tat ctc tcc aag agc  tct ggg tgg gcc aac  gca gga att        5634
   Arg Trp  Tyr Leu Ser Lys Ser  Ser Gly Trp Ala Asn  Ala Gly Ile
       1865                 1870                 1875
   aaa tct  cgt gca ctc gac tac  cag atc tgg tgt ggc  ccg gca atg        5679
   Lys Ser  Arg Ala Leu Asp Tyr  Gln Ile Trp Cys Gly  Pro Ala Met
       1880                 1885                 1890
   ggc tcg  ttc aac aat ttc gcc  agc ggc aca tcc ctc  gat tgg aaa        5724
   Gly Ser  Phe Asn Asn Phe Ala  Ser Gly Thr Ser Leu  Asp Trp Lys
       1895                 1900                 1905
   gtg act  ggg gtt ttc cct ggc  gtt gcg gaa gta aac  atg gcc att        5769
   Val Thr  Gly Val Phe Pro Gly  Val Ala Glu Val Asn  Met Ala Ile
       1910                 1915                 1920
   tta gat  ggc gcg cga gaa cta  gct gct aaa cga aat  taa                5808
   Leu Asp  Gly Ala Arg Glu Leu  Ala Ala Lys Arg Asn
       1925                 1930                 1935
<210> 52
   <211> 1935
   <212> PRT
   <213> Thraustochytrium sp.
<220>
   <221> misc_feature
   <222> (248)..(248)
   <223> The 'Xaa' at location 248 stands for Asp, Gly, Ala, or Val.
<400> 52
   Met Gln Leu Pro Pro Ala His Ser Ala Asp Glu Asn Arg Ile Ala Val
   1               5                   10                  15
   Val Gly Met Ala Val Lys Tyr Ala Gly Cys Asp Asn Lys Glu Glu Phe
               20                  25                  30
   Trp Lys Thr Leu Met Asn Gly Ser Ile Asn Thr Lys Ser Ile Ser Ala
           35                  40                  45
   Ala Arg Leu Gly Ser Asn Lys Arg Asp Glu His Tyr Val Pro Glu Arg
       50                  55                  60
   Ser Lys Tyr Ala Asp Thr Phe Cys Asn Glu Arg Tyr Gly Cys Ile Gln
   65                  70                  75                  80
   Gln Gly Thr Asp Asn Glu His Asp Leu Leu Leu Gly Leu Ala Gln Glu
                   85                  90                  95
   Ala Leu Ala Asp Ala Ala Gly Arg Met Glu Lys Gln Pro Ser Glu Ala
               100                 105                 110
   Phe Asp Leu Glu Asn Thr Gly Ile Val Ser Gly Cys Leu Ser Phe Pro
   115 120 125
   Met Asp Asn Leu Gln Gly Glu Leu Leu Asn Leu Tyr Gln Ser His Val
   130 135 140
   Glu Lys Gln Leu Pro Pro Ser Ala Leu Val Glu Ala Val Lys Leu Trp
   145 150 155 160
   Ser Glu Arg Gln Lys Ser Thr Lys Ala His Ala Gly Asp Lys Arg Arg
   165 170 175
   Phe Ile Asp Pro Ala Ser Phe Val Ala Asp Lys Leu Asn Leu Gly Pro
   180 185 190
   Leu His Tyr Ala Ile Asp Ala Ala Cys Ala Ser Ala Leu Tyr Val Leu
   195 200 205
   Lys Leu Ala Gln Asp His Leu Val Ser Gly Ala Val Asp Met Met Leu
   210 215 220
   Cys Gly Ala Thr Cys Phe Pro Glu Pro Phe Phe Ile Leu Ser Gly Phe
   225 230 235 240
   Ser Thr Phe Gln Ala Met Pro Xaa Gly Ala Asp Gly Val Ser Leu Pro
   245 250 255
   Leu His Lys Thr Ser Ala Gly Leu Thr Pro Gly Glu Gly Gly Ser Ile
   260 265 270
   Met Val Leu Lys Arg Leu Lys Asp Ala Ile Arg Asp Gly Asn His Ile
   275 280 285
   Tyr Gly Val Leu Leu Glu Ala Asn Leu Ser Asn Ala Gly Cys Gly Leu
   290 295 300
   Pro Leu Ser Pro His Leu Pro Ser Glu Glu Ser Cys Ile Arg Asp Thr
   305 310 315 320
   Tyr Arg Arg Ala Gly Val Ala Ala Asp Gln Ser Ile Gln Tyr Ile Glu
   325 330 335
   Cys His Ala Thr Gly Thr Pro Arg Gly Asp Val Val Glu Ile Glu Ala
   340 345 350
   Val Glu Arg Val Phe Lys Lys Asn Val Pro Arg Leu Gly Ser Thr Lys
   355 360 365
   Gly Asn Phe Gly His Ser Leu Val Ala Ala Gly Phe Ala Gly Met Ala
   370 375 380
   Lys Leu Leu Leu Ala Met Glu His Gly Val Ile Pro Pro Thr Pro Gly
   385 390 395 400
   Leu Asp Ala Ser Asn Gln Ala Ser Glu His Val Val Thr Lys Ala Ile
   405 410 415
   Thr Trp Pro Glu Thr His Gly Ala Pro Lys Arg Ala Gly Leu Ser Ala
   420 425 430
   Phe Gly Phe Gly Gly Thr Asn Ala His Ala Leu Phe Glu Glu Phe Asn
   435 440 445
   Ala Glu Gly Ile Ser Tyr Arg Pro Gly Lys Pro Pro Val Glu Ser Asn
   450 455 460
   Thr Arg Pro Ser Val Val Ile Thr Gly Met Asp Cys Thr Phe Gly Ser
   465 470 475 480
   Leu Glu Gly Ile Asp Ala Phe Glu Thr Ala Leu Tyr Glu Gly Arg Asp
   485 490 495
   Ala Ala Arg Asp Leu Pro Ala Lys Arg Trp Arg Phe Leu Gly Glu Asp
   500 505 510
   Leu Glu Phe Leu Arg Ala Ile Arg Leu Lys Glu Lys Pro Arg Gly Cys
   515 520 525
   Phe Val Glu Ser Val Asp Val Asn Phe Arg Arg Leu Lys Thr Pro Leu
   530 535 540
   Thr Pro Glu Asp Met Leu Arg Pro Gln Gln Leu Leu Ala Val Ser Thr
   545 550 555 560
   Met Asp Arg Ala Ile Ile Asp Ala Gly Leu Lys Lys Gly Gln His Val
   565 570 575
   Ala Val Leu Val Gly Leu Gly Thr Asp Leu Glu Leu Tyr Arg His Arg
   580 585 590
   Ala Arg Val Ala Leu Lys Glu Val Leu His Pro Ser Leu Lys Ser Asp
   595 600 605
   Thr Ala Ile Leu Gln Lys Ile Met Gln Tyr Val Asn Asp Ala Gly Thr
   610 615 620
   Ser Thr Ser Tyr Thr Ser Tyr Ile Gly Asn Leu Val Ala Thr Arg Ile
   625 630 635 640
   Ser Ser Gln Trp Gly Phe Thr Gly Pro Ser Phe Thr Val Thr Glu Gly
   645 650 655
   Asn Asn Ser Val Tyr Arg Cys Ala Gln Leu Ala Lys Asp Met Leu Gln
   660 665 670
   Val Asn Arg Val Asp Ala Val Val Ile Ala Gly Val Asp Leu Asn Gly
   675 680 685
   Ser Ala Glu Ser Phe Phe Val Arg Ala Asn Arg Gln Lys Ile Ser Lys
       690                 695                 700
   Leu Ser His Pro Cys Ala Ser Phe Asp Arg Asp Ala Asp Gly Phe Phe
   705                 710                 715                 720
   Ala Gly Glu Gly Cys Gly Ala Leu Val Phe Lys Arg Leu Glu Asp Cys
                   725                 730                 735
   Ala Pro Gln Glu Lys Ile Tyr Ala Ser Ile Asp Ser Ile Ala Ile Asp
               740                 745                 750
   Lys Glu Pro Thr Ser Ser Ala Val Lys Ala Val Tyr Gln Ser Asp Ser
           755                 760                 765
   Ser Leu Ser Asp Ile Glu Leu Leu Glu Ile Ser Gly Asp Ser Lys Arg
       770                 775                 780
   Phe Ala Ala Phe Glu Gly Ala Val Glu Ile Gln Ser Ser Val Glu Ala
   785                 790                 795                 800
   Gln Leu Lys Gly Leu Ser Lys Val Leu Glu Pro Ala Lys Gly Gln Gly
                   805                 810                 815
   Val Ala Val Gly Ser Thr Arg Ala Thr Val Gly Asp Ile Gly Tyr Ala
               820                 825                 830
   Thr Gly Ala Ala Ser Leu Ile Lys Thr Ala Leu Cys Leu Tyr Asn Arg
           835                 840                 845
   Tyr Leu Pro Ala Leu Ala Asn Trp Ser Gly Pro Cys Glu Gln Ser Ala
       850                 855                 860
   Trp Gly Ser Asn Met Phe Val Cys His Glu Thr Arg Pro Trp Met Lys
   865                 870                 875                 880
   Asn Gln Asn Glu Lys Arg Cys Ala Leu Ile Ser Gly Thr Asp Pro Ser
                   885                 890                 895
   His Thr Cys Phe Ser Leu Val Leu Ser Asp Thr Gly Cys Tyr Glu Glu
               900                 905                 910
   His Asn Arg Thr Cys Phe Asp Val Gln Ala Pro Gln Leu Val Leu Ile
           915                 920                 925
   His Gly Phe Asp Gly Lys Thr Ile Val Arg Arg Leu Glu Gly Tyr Leu
       930                 935                 940
   Leu Glu Leu Val Glu Gly His Ala Ser Pro Ser Glu Tyr Phe His Lys
   945                 950                 955                 960
   Leu Ile Gly Gln Ser Leu Leu Glu Asn Ser Lys Glu Ser Lys Leu Thr
                   965                 970                 975
   Leu Ser Leu Val Cys Asn Pro Asn Gln Leu Gln Lys Glu Leu Met Leu
   980 985 990
   Ala Ile Lys Gly Val Gln Arg Ser Met Leu Thr Gly Lys Asp Trp Val
   995 1000 1005
   Ser Pro Ser Gly Ser Cys Phe Ala Pro Asn Pro Leu Ser Ser Ala
   1010 1015 1020
   Lys Val Ala Phe Met Tyr Gly Glu Gly Arg Ser Pro Tyr Cys Gly
   1025 1030 1035
   Val Gly Leu Gly Leu His Arg Leu Trp Pro Gly Leu His Glu Asn
   1040 1045 1050
   Val Asn Asn Lys Thr Val Asp Leu Trp Thr Glu Gly Asp Gly Trp
   1055 1060 1065
   Leu Tyr Pro Arg Thr Leu Thr Arg Glu Glu His Thr Lys Ala Ile
   1070 1075 1080
   Glu Ser Phe Asn Ala Asn Gln Ile Glu Met Phe Arg Ala Gly Ile
   1085 1090 1095
   Phe Ile Ser Met Cys Gln Thr Asp Tyr Val Met Asn Val Leu Gly
   1100 1105 1110
   Val Gln Pro Lys Ala Gly Phe Gly Leu Ser Leu Gly Glu Ile Ser
   1115 1120 1125
   Met Leu Phe Ala Met Ser Lys Glu Asn Cys Arg Gln Ser Gln Glu
   1130 1135 1140
   Met Thr Asn Arg Leu Arg Gly Ser Pro Val Trp Ser Asn Glu Leu
   1145 1150 1155
   Ala Ile Asn Phe Asn Ala Ile Arg Lys Leu Trp Lys Ile Pro Arg
   1160 1165 1170
   Gly Ala Pro Leu Glu Ser Phe Trp Gln Gly Tyr Leu Val His Gly
   1175 1180 1185
   Thr Arg Glu Glu Val Glu His Ala Ile Gly Leu Ser Glu Pro Tyr
   1190 1195 1200
   Val Arg Leu Leu Ile Val Asn Asp Ser Arg Ser Ala Leu Ile Ala
   1205 1210 1215
   Gly Lys Pro Asp Ala Cys Gln Ala Val Ile Ser Arg Leu Asn Ser
   1220 1225 1230
   Lys Phe Pro Ser Leu Pro Val Lys Gln Gly Met Ile Gly His Cys
   1235 1240 1245
   Pro Glu Val Arg Ala Phe Ile Lys Asp Ile Gly Tyr Ile His Glu
   1250 1255 1260
   Thr Leu Arg Ile Ser Asn Asp Tyr Ser Asp Cys Gln Leu Phe Ser
   1265 1270 1275
   Ala Val Thr Lys Gly Ala Leu Asp Ser Ser Thr Met Glu Ile Lys
   1280 1285 1290
   His Phe Val Gly Glu Val Tyr Ser Arg Ile Ala Asp Phe Pro Gln
   1295 1300 1305
   Ile Val Asn Thr Val His Ser Ala Gly Tyr Asp Val Phe Leu Glu
   1310 1315 1320
   Leu Gly Cys Asp Ala Ser Arg Ser Ala Ala Val Gln Asn Ile Leu
   1325 1330 1335
   Gly Gly Gln Gly Lys Phe Leu Ser Thr Ala Ile Asp Lys Lys Gly
   1340 1345 1350
   His Ser Ala Trp Ser Gln Val Leu Arg Ala Thr Ala Ser Leu Ala
   1355 1360 1365
   Ala His Arg Val Pro Gly Ile Ser Ile Leu Asp Leu Phe His Pro
   1370 1375 1380
   Asn Phe Arg Glu Met Cys Cys Thr Met Ala Thr Thr Pro Lys Val
   1385 1390 1395
   Glu Asp Lys Phe Leu Arg Thr Ile Gln Ile Asn Gly Arg Phe Glu
   1400 1405 1410
   Lys Glu Met Ile His Leu Glu Asp Thr Thr Leu Ser Cys Leu Pro
   1415 1420 1425
   Ala Pro Ser Glu Ala Asn Ile Ala Ala Ile Gln Ser Arg Ser Ile
   1430 1435 1440
   Arg Ser Ala Ala Ala Arg Ser Gly Gln Ser His Asp Cys Ala Ser
   1445 1450 1455
   His Ser His Glu Glu Asn Lys Asp Ser Cys Pro Glu Lys Leu Lys
   1460 1465 1470
   Leu Asp Ser Val Ser Val Ala Ile Asn Phe Asp Asn Asp Asp Arg
   1475 1480 1485
   Ile Gln Leu Gly His Ala Gly Phe Arg Glu Met Tyr Asn Thr Arg
   1490 1495 1500
   Tyr Ser Leu Tyr Thr Gly Ala Met Ala Lys Gly Ile Ala Ser Ala
   1505 1510 1515
   Asp Leu Val Ile Ala Ala Gly Lys Glu Gly Ile Leu Ala Ser Tyr
   1520 1525 1530
   Gly Ala Gly Gly Leu Pro Leu Ala Thr Val Arg Lys Gly Ile Asp
   1535 1540 1545
   Lys Ile Gln Gln Ala Leu Pro Ser Gly Pro Tyr Ala Val Asn Leu
   1550 1555 1560
   Ile His Ser Pro Phe Asp Gly Asn Leu Glu Gln Gly Asn Val Asp
   1565 1570 1575
   Leu Phe Leu Glu Lys Asn Val Arg Val Ala Glu Cys Ser Ala Phe
   1580 1585 1590
   Thr Thr Leu Thr Val Pro Val Val His Tyr Arg Ala Ala Gly Leu
   1595 1600 1605
   Val Arg Arg Gln Asp Gly Ser Ile Leu Ile Lys Asn Arg Ile Ile
   1610 1615 1620
   Ala Lys Val Ser Arg Thr Glu Leu Ala Glu Met Phe Leu Arg Pro
   1625 1630 1635
   Ala Pro Gln Ile Ile Leu Glu Lys Leu Val Ala Ala Glu Ile Ile
   1640 1645 1650
   Ser Ser Asp Gln Ala Arg Met Ala Ala Lys Val Pro Met Ala Asp
   1655 1660 1665
   Asp Ile Ala Val Glu Ala Asp Ser Gly Gly His Thr Asp Asn Arg
   1670 1675 1680
   Pro Met His Val Ile Leu Pro Leu Ile Ile Gln Leu Arg Asn Thr
   1685 1690 1695
   Ile Leu Ala Glu Tyr Gly Cys Ala Thr Ala Phe Arg Thr Arg Ile
   1700 1705 1710
   Gly Ala Gly Gly Gly Ile Gly Cys Pro Ser Ala Ala Leu Ala Ala
   1715 1720 1725
   Phe Asp Met Gly Ala Ser Phe Val Val Thr Gly Ser Ile Asn Gln
   1730 1735 1740
   Ile Cys Arg Glu Ala Gly Thr Cys Asp Thr Val Arg Glu Leu Leu
   1745 1750 1755
   Ala Asn Ser Ser Tyr Ser Asp Val Thr Met Ala Pro Ala Ala Asp
   1760 1765 1770
   Met Phe Asp Gln Gly Val Lys Leu Gln Val Leu Lys Arg Gly Thr
   1775 1780 1785
   Met Phe  Pro Ser Arg Ala Asn  Lys Leu Arg Lys Leu  Phe Val Asn
       1790                 1795                 1800
   Tyr Glu  Ser Leu Glu Thr Leu  Pro Ser Lys Glu Leu  Lys Tyr Leu
       1805                 1810                 1815
   Glu Asn  Ile Ile Phe Lys Gln  Ala Val Asp Gln Val  Trp Glu Glu
       1820                 1825                 1830
   Thr Lys  Arg Phe Tyr Cys Glu  Lys Leu Asn Asn Pro  Asp Lys Ile
       1835                 1840                 1845
   Ala Arg  Ala Met Lys Asp Pro  Lys Leu Lys Met Ser  Leu Cys Phe
       1850                 1855                 1860
   Arg Trp  Tyr Leu Ser Lys Ser  Ser Gly Trp Ala Asn  Ala Gly Ile
       1865                 1870                 1875
   Lys Ser  Arg Ala Leu Asp Tyr  Gln Ile Trp Cys Gly  Pro Ala Met
       1880                 1885                 1890
   Gly Ser  Phe Asn Asn Phe Ala  Ser Gly Thr Ser Leu  Asp Trp Lys
       1895                 1900                 1905
   Val Thr  Gly Val Phe Pro Gly  Val Ala Glu Val Asn  Met Ala Ile
       1910                 1915                 1920
   Leu Asp  Gly Ala Arg Glu Leu  Ala Ala Lys Arg Asn
       1925                 1930                 1935
<210> 53
   <211> 1500
   <212> DNA
   <213> Thraustochytrium sp.
<220>
   <221> CDS
   <222> (1)..(1500)
<220>
   <221> misc_feature
   <222> (1)..(1500)
   <223> n = a c t or g
<400> 53
   atg caa ctt cct cca gcg cat tct gcc gat gag aat cgc atc gcg gtc        48
   Met Gln Leu Pro Pro Ala His Ser Ala Asp Glu Asn Arg Ile Ala Val
   1               5                   10                  15
   gtg ggc atg gcc gtc aaa tat gcg ggc tgt gac aat aaa gaa gag ttt        96
   Val Gly Met Ala Val Lys Tyr Ala Gly Cys Asp Asn Lys Glu Glu Phe
               20                  25                  30
   tgg aag act ttg atg aat ggt agt atc aat acc aag tcg att tcg gca       144
   Trp Lys Thr Leu Met Asn Gly Ser Ile Asn Thr Lys Ser Ile Ser Ala
           35                  40                  45
   gca agg ttg ggc agc aat aag cgt gac gaa cac tat gtt cct gaa cga       192
   Ala Arg Leu Gly Ser Asn Lys Arg Asp Glu His Tyr Val Pro Glu Arg
       50                  55                  60
   tcg aaa tat gca gat acg ttc tgt aac gaa agg tac ggt tgt atc cag      240
   Ser Lys Tyr Ala Asp Thr Phe Cys Asn Glu Arg Tyr Gly Cys Ile Gln
   65                  70                  75                  80
   caa ggt acg gat aat gag cat gac ctc ctc cta ggt ctt gct caa gaa      288
   Gln Gly Thr Asp Asn Glu His Asp Leu Leu Leu Gly Leu Ala Gln Glu
                   85                  90                  95
   gct ctc gct gac gct gcc ggg cgg atg gag aaa caa cct tcg gag gcg      336
   Ala Leu Ala Asp Ala Ala Gly Arg Met Glu Lys Gln Pro Ser Glu Ala
               100                 105                 110
   ttc gat ctg gaa aat act ggc atc gtg agt ggg tgc tta tct ttt cca      384
   Phe Asp Leu Glu Asn Thr Gly Ile Val Ser Gly Cys Leu Ser Phe Pro
           115                 120                 125
   atg gat aac ctg caa gga gag ttg ttg aac ttg tat caa agc cat gtg      432
   Met Asp Asn Leu Gln Gly Glu Leu Leu Asn Leu Tyr Gln Ser His Val
       130                 135                 140
   gag aaa caa ctt cca cct agt gcc ttg gta gaa gcc gtg aag ctt tgg      480
   Glu Lys Gln Leu Pro Pro Ser Ala Leu Val Glu Ala Val Lys Leu Trp
   145                 150                 155                 160
   tct gag cga cag aaa tct acg aaa gca cat gca ggg gac aag cgc cgg      528
   Ser Glu Arg Gln Lys Ser Thr Lys Ala His Ala Gly Asp Lys Arg Arg
                   165                 170                 175
   ttc att gac cca gct tct ttt gta gct gat aaa ctg aac cta ggc cca      576
   Phe Ile Asp Pro Ala Ser Phe Val Ala Asp Lys Leu Asn Leu Gly Pro
               180                 185                 190
   cta cat tat gcg atc gat gca gca tgc gct tct gca ttg tac gtg tta      624
   Leu His Tyr Ala Ile Asp Ala Ala Cys Ala Ser Ala Leu Tyr Val Leu
           195                 200                 205
   aaa tta gct caa gac cac ctt gtt tca ggt gcc gtt gat atg atg tta      672
   Lys Leu Ala Gln Asp His Leu Val Ser Gly Ala Val Asp Met Met Leu
       210                 215                 220
   tgt gga gcg acg tgc ttc cca gaa cca ttc ttc atc ttg tct ggg ttc      720
   Cys Gly Ala Thr Cys Phe Pro Glu Pro Phe Phe Ile Leu Ser Gly Phe
   225                 230                 235                 240
   tcg act ttt caa gcg atg cct gnt ggg gca gat gga gtc tca cta cct      768
   Ser Thr Phe Gln Ala Met Pro Xaa Gly Ala Asp Gly Val Ser Leu Pro
                   245                 250                 255
   ctc cat aaa acg agt gct ggg ctc act cca ggt gaa ggg ggg tcc att      816
   Leu His Lys Thr Ser Ala Gly Leu Thr Pro Gly Glu Gly Gly Ser Ile
               260                 265                 270
   atg gtg ctc aag cga ctg aaa gac gct atc aga gat gga aat cac att      864
   Met Val Leu Lys Arg Leu Lys Asp Ala Ile Arg Asp Gly Asn His Ile
           275                 280                 285
   tat ggt gtg ctc ctt gaa gca aat tta agt aac gca ggt tgt ggg ctt      912
   Tyr Gly Val Leu Leu Glu Ala Asn Leu Ser Asn Ala Gly Cys Gly Leu
       290                 295                 300
   cca ctc agc ccg cac tta ccg agc gaa gaa tca tgt att cgt gat acc      960
   Pro Leu Ser Pro His Leu Pro Ser Glu Glu Ser Cys Ile Arg Asp Thr
   305                 310                 315                 320
   tac cgc cgt gct gga gtt gct gca gat caa agt att cag tat att gag     1008
   Tyr Arg Arg Ala Gly Val Ala Ala Asp Gln Ser Ile Gln Tyr Ile Glu
                   325                 330                 335
   tgc cac gct acg gga acc cct cga ggg gat gtc gtg gaa att gag gcg     1056
   Cys His Ala Thr Gly Thr Pro Arg Gly Asp Val Val Glu Ile Glu Ala
               340                 345                 350
   gtt gaa aga gtt ttc aag aaa aac gtt cca cgc tta ggc tcg acg aaa      1104
   Val Glu Arg Val Phe Lys Lys Asn Val Pro Arg Leu Gly Ser Thr Lys
           355                 360                 365
   gga aat ttt ggt cac tcg tta gtt gcg gct ggt ttc gca ggt atg gca      1152
   Gly Asn Phe Gly His Ser Leu Val Ala Ala Gly Phe Ala Gly Met Ala
       370                 375                 380
   aag ctt ctt ctt gca atg gaa cat gga gtg att cct ccc aca cca ggt      1200
   Lys Leu Leu Leu Ala Met Glu His Gly Val Ile Pro Pro Thr Pro Gly
   385                 390                 395                 400
   ctt gat gct tcg aac cag gca agt gag cac gtt gtg aca aag gct atc      1248
   Leu Asp Ala Ser Asn Gln Ala Ser Glu His Val Val Thr Lys Ala Ile
                   405                 410                 415
   act tgg cct gag aca cat ggg gct cca aaa cga gct ggc ctt tca gca      1296
   Thr Trp Pro Glu Thr His Gly Ala Pro Lys Arg Ala Gly Leu Ser Ala
               420                 425                 430
   ttt gga ttt ggt ggg act aat gcg cat gca ctc ttc gaa gag ttt aat      1344
   Phe Gly Phe Gly Gly Thr Asn Ala His Ala Leu Phe Glu Glu Phe Asn
           435                 440                 445
   gcc gag ggc ata agt tat cgc cct gga aag cct cca gtc gaa tcg aat      1392
   Ala Glu Gly Ile Ser Tyr Arg Pro Gly Lys Pro Pro Val Glu Ser Asn
       450                 455                 460
   acc cgt cct tcc gtc gta ata act ggg atg gac tgt acc ttt ggg agc      1440
   Thr Arg Pro Ser Val Val Ile Thr Gly Met Asp Cys Thr Phe Gly Ser
   465                 470                 475                 480
   ctt gaa ggg att gat gcg ttc gag act gcc ctg tac gag ggg cgt gac      1488
   Leu Glu Gly Ile Asp Ala Phe Glu Thr Ala Leu Tyr Glu Gly Arg Asp
                   485                 490                 495
   gca gct cgt gac                                                      1500
   Ala Ala Arg Asp
               500
<210> 54
   <211> 500
   <212> PRT
   <213> Thraustochytrium sp.
<220>
   <221> misc_feature
   <222> (248)..(248)
   <223> The 'Xaa' at location 248 stands for Asp, Gly, Ala, or Val.
<400> 54
   Met Gln Leu Pro Pro Ala His Ser Ala Asp Glu Asn Arg Ile Ala Val
   1               5                   10                  15
   Val Gly Met Ala Val Lys Tyr Ala Gly Cys Asp Asn Lys Glu Glu Phe
               20                  25                  30
   Trp Lys Thr Leu Met Asn Gly Ser Ile Asn Thr Lys Ser Ile Ser Ala
           35                  40                  45
   Ala Arg Leu Gly Ser Asn Lys Arg Asp Glu His Tyr Val Pro Glu Arg
       50                  55                  60
   Ser Lys Tyr Ala Asp Thr Phe Cys Asn Glu Arg Tyr Gly Cys Ile Gln
   65                  70                  75                  80
   Gln Gly Thr Asp Asn Glu His Asp Leu Leu Leu Gly Leu Ala Gln Glu
   85 90 95
   Ala Leu Ala Asp Ala Ala Gly Arg Met Glu Lys Gln Pro Ser Glu Ala
   100 105 110
   Phe Asp Leu Glu Asn Thr Gly Ile Val Ser Gly Cys Leu Ser Phe Pro
   115 120 125
   Met Asp Asn Leu Gln Gly Glu Leu Leu Asn Leu Tyr Gln Ser His Val
   130 135 140
   Glu Lys Gln Leu Pro Pro Ser Ala Leu Val Glu Ala Val Lys Leu Trp
   145 150 155 160
   Ser Glu Arg Gln Lys Ser Thr Lys Ala His Ala Gly Asp Lys Arg Arg
   165 170 175
   Phe Ile Asp Pro Ala Ser Phe Val Ala Asp Lys Leu Asn Leu Gly Pro
   180 185 190
   Leu His Tyr Ala Ile Asp Ala Ala Cys Ala Ser Ala Leu Tyr Val Leu
   195 200 205
   Lys Leu Ala Gln Asp His Leu Val Ser Gly Ala Val Asp Met Met Leu
   210 215 220
   Cys Gly Ala Thr Cys Phe Pro Glu Pro Phe Phe Ile Leu Ser Gly Phe
   225 230 235 240
   Ser Thr Phe Gln Ala Met Pro Xaa Gly Ala Asp Gly Val Ser Leu Pro
   245 250 255
   Leu His Lys Thr Ser Ala Gly Leu Thr Pro Gly Glu Gly Gly Ser Ile
   260 265 270
   Met Val Leu Lys Arg Leu Lys Asp Ala Ile Arg Asp Gly Asn His Ile
   275 280 285
   Tyr Gly Val Leu Leu Glu Ala Asn Leu Ser Asn Ala Gly Cys Gly Leu
   290 295 300
   Pro Leu Ser Pro His Leu Pro Ser Glu Glu Ser Cys Ile Arg Asp Thr
   305 310 315 320
   Tyr Arg Arg Ala Gly Val Ala Ala Asp Gln Ser Ile Gln Tyr Ile Glu
   325 330 335
   Cys His Ala Thr Gly Thr Pro Arg Gly Asp Val Val Glu Ile Glu Ala
   340 345 350
   Val Glu Arg Val Phe Lys Lys Asn Val Pro Arg Leu Gly Ser Thr Lys
   355 360 365
   Gly Asn Phe Gly His Ser Leu Val Ala Ala Gly Phe Ala Gly Met Ala
       370                 375                 380
   Lys Leu Leu Leu Ala Met Glu His Gly Val Ile Pro Pro Thr Pro Gly
   385                 390                 395                 400
   Leu Asp Ala Ser Asn Gln Ala Ser Glu His Val Val Thr Lys Ala Ile
                   405                 410                 415
   Thr Trp Pro Glu Thr His Gly Ala Pro Lys Arg Ala Gly Leu Ser Ala
               420                 425                 430
   Phe Gly Phe Gly Gly Thr Asn Ala His Ala Leu Phe Glu Glu Phe Asn
           435                 440                 445
   Ala Glu Gly Ile Ser Tyr Arg Pro Gly Lys Pro Pro Val Glu Ser Asn
       450                 455                 460
   Thr Arg Pro Ser Val Val Ile Thr Gly Met Asp Cys Thr Phe Gly Ser
   465                 470                 475                 480
   Leu Glu Gly Ile Asp Ala Phe Glu Thr Ala Leu Tyr Glu Gly Arg Asp
                   485                 490                 495
   Ala Ala Arg Asp
               500
<210> 55
   <211> 1500
   <212> DNA
   <213> Thraustochytrium sp.
<220>
   <221> CDS
   <222> (1)..(1500)
<400> 55
   tta ccc gcc aaa cgt tgg agg ttc cta ggt gag gac ttg gag ttt ctc      48
   Leu Pro Ala Lys Arg Trp Arg Phe Leu Gly Glu Asp Leu Glu Phe Leu
   1               5                   10                  15
   cga gcc atc agg ctc aag gaa aag cct agg ggt tgt ttt gtg gag agt      96
   Arg Ala Ile Arg Leu Lys Glu Lys Pro Arg Gly Cys Phe Val Glu Ser
               20                  25                  30
   gtt gac gtt aac ttt aga cgg ctg aaa acg ccc ttg aca cca gaa gat     144
   Val Asp Val Asn Phe Arg Arg Leu Lys Thr Pro Leu Thr Pro Glu Asp
           35                  40                  45
   atg ttg cgg ccc caa caa ctc ttg gcg gtt tct acg atg gac cga gca     192
   Met Leu Arg Pro Gln Gln Leu Leu Ala Val Ser Thr Met Asp Arg Ala
       50                  55                  60
   att atc gat gca ggt cta aag aag ggc caa cat gta gca gtt ctt gtt     240
   Ile Ile Asp Ala Gly Leu Lys Lys Gly Gln His Val Ala Val Leu Val
   65                  70                  75                  80
   ggc cta gga act gac ctg gaa ctt tac cgt cat cga gca aga gtc gcg     288
   Gly Leu Gly Thr Asp Leu Glu Leu Tyr Arg His Arg Ala Arg Val Ala
                   85                  90                  95
   ctt aaa gag gtt ttg cac ccg agc tta aag tca gac act gca att ctc      336
   Leu Lys Glu Val Leu His Pro Ser Leu Lys Ser Asp Thr Ala Ile Leu
               100                 105                 110
   cag aaa ata atg caa tat gtg aat gat gca gga act tcg act tca tac      384
   Gln Lys Ile Met Gln Tyr Val Asn Asp Ala Gly Thr Ser Thr Ser Tyr
           115                 120                 125
   aca tct tac att gga aac ctc gtt gcc acg cgt att tcg tct cag tgg      432
   Thr Ser Tyr Ile Gly Asn Leu Val Ala Thr Arg Ile Ser Ser Gln Trp
       130                 135                 140
   gga ttc aca ggg ccg tcc ttt act gtc aca gaa gga aat aat tcc gtg      480
   Gly Phe Thr Gly Pro Ser Phe Thr Val Thr Glu Gly Asn Asn Ser Val
   145                 150                 155                 160
   tac aga tgt gca caa cta gcc aaa gat atg ctt cag gtt aac cga gtt      528
   Tyr Arg Cys Ala Gln Leu Ala Lys Asp Met Leu Gln Val Asn Arg Val
                   165                 170                 175
   gat gct gtc gtc atc gca ggc gtt gat ctc aac gga agc gcc gaa agt      576
   Asp Ala Val Val Ile Ala Gly Val Asp Leu Asn Gly Ser Ala Glu Ser
               180                 185                 190
   ttt ttt gtc cga gca aat cgt caa aag ata tcc aag cta agt cat cca      624
   Phe Phe Val Arg Ala Asn Arg Gln Lys Ile Ser Lys Leu Ser His Pro
           195                 200                 205
   tgt gca agc ttc gac aga gat gca gat gga ttt ttc gca ggt gag ggc      672
   Cys Ala Ser Phe Asp Arg Asp Ala Asp Gly Phe Phe Ala Gly Glu Gly
       210                 215                 220
   tgt ggt gcc cta gtt ttc aag agg tta gaa gac tgt gct cct cag gaa      720
   Cys Gly Ala Leu Val Phe Lys Arg Leu Glu Asp Cys Ala Pro Gln Glu
   225                 230                 235                 240
   aaa att tat gct agt ata gac tct atc gca ata gat aaa gag cct act      768
   Lys Ile Tyr Ala Ser Ile Asp Ser Ile Ala Ile Asp Lys Glu Pro Thr
                   245                 250                 255
   agc tca gct gtg aaa gct gtc tac caa agt gat tcg agt ctc tcc gat      816
   Ser Ser Ala Val Lys Ala Val Tyr Gln Ser Asp Ser Ser Leu Ser Asp
               260                 265                 270
   att gag ctg tta gaa atc agt gga gac tcc aaa cgg ttt gca gca ttc      864
   Ile Glu Leu Leu Glu Ile Ser Gly Asp Ser Lys Arg Phe Ala Ala Phe
           275                 280                 285
   gaa ggc gct gtg gaa att caa tca agt gtg gaa gcc cag cta aaa gga      912
   Glu Gly Ala Val Glu Ile Gln Ser Ser Val Glu Ala Gln Leu Lys Gly
       290                 295                 300
   ctt tcc aaa gtc ctt gaa cct gca aaa ggc caa ggc gta gcg gtg gga      960
   Leu Ser Lys Val Leu Glu Pro Ala Lys Gly Gln Gly Val Ala Val Gly
   305                 310                 315                 320
   agt act cga gca acc gtt ggg gat ata ggg tat gct aca gga gcg gca     1008
   Ser Thr Arg Ala Thr Val Gly Asp Ile Gly Tyr Ala Thr Gly Ala Ala
                   325                 330                 335
   agc ctg att aaa act gca ctc tgc tta tat aat cgc tac ctt ccg gca     1056
   Ser Leu Ile Lys Thr Ala Leu Cys Leu Tyr Asn Arg Tyr Leu Pro Ala
               340                 345                 350
   tta gca aac tgg agt ggc cca tgt gaa cag tcc gcc tgg ggc tca aac     1104
   Leu Ala Asn Trp Ser Gly Pro Cys Glu Gln Ser Ala Trp Gly Ser Asn
           355                 360                 365
   atg ttc gtt tgc cat gaa aca cgg ccg tgg atg aaa aac cag aat gaa     1152
   Met Phe Val Cys His Glu Thr Arg Pro Trp Met Lys Asn Gln Asn Glu
       370                 375                 380
   aag aga tgt gcc ctc att tct gga aca gat cca tct cat aca tgc ttt      1200
   Lys Arg Cys Ala Leu Ile Ser Gly Thr Asp Pro Ser His Thr Cys Phe
   385                 390                 395                 400
   tcc ctc gta cta tcg gat act ggg tgt tat gaa gag cac aat cga acg      1248
   Ser Leu Val Leu Ser Asp Thr Gly Cys Tyr Glu Glu His Asn Arg Thr
                   405                 410                 415
   tgc ttt gat gtg caa gcg cca cag cta gtt ctg ata cac gga ttc gat      1296
   Cys Phe Asp Val Gln Ala Pro Gln Leu Val Leu Ile His Gly Phe Asp
               420                 425                 430
   gga aaa act att gtg cgg cga ctt gaa gga tat ctc ctt gaa ctt gtt      1344
   Gly Lys Thr Ile Val Arg Arg Leu Glu Gly Tyr Leu Leu Glu Leu Val
           435                 440                 445
   gaa ggg cat gca agc cct tca gag tat ttc cac aaa ctg att gga caa      1392
   Glu Gly His Ala Ser Pro Ser Glu Tyr Phe His Lys Leu Ile Gly Gln
       450                 455                 460
   agt cta ctt gag aac tcg aaa gaa agt aaa ctc aca ctt tcg ctt gtg      1440
   Ser Leu Leu Glu Asn Ser Lys Glu Ser Lys Leu Thr Leu Ser Leu Val
   465                 470                 475                 480
   tgc aat ccg aac cag ctc caa aag gag ctc atg ctt gct atc aaa gga      1488
   Cys Asn Pro Asn Gln Leu Gln Lys Glu Leu Met Leu Ala Ile Lys Gly
                   485                 490                 495
   gta caa cga agc                                                      1500
   Val Gln Arg Ser
               500
<210> 56
   <211> 500
   <212> PRT
   <213> Thraustochytrium sp.
<400> 56
   Leu Pro Ala Lys Arg Trp Arg Phe Leu Gly Glu Asp Leu Glu Phe Leu
   1               5                   10                  15
   Arg Ala Ile Arg Leu Lys Glu Lys Pro Arg Gly Cys Phe Val Glu Ser
               20                  25                  30
   Val Asp Val Asn Phe Arg Arg Leu Lys Thr Pro Leu Thr Pro Glu Asp
           35                  40                  45
   Met Leu Arg Pro Gln Gln Leu Leu Ala Val Ser Thr Met Asp Arg Ala
       50                  55                  60
   Ile Ile Asp Ala Gly Leu Lys Lys Gly Gln His Val Ala Val Leu Val
   65                  70                  75                  80
   Gly Leu Gly Thr Asp Leu Glu Leu Tyr Arg His Arg Ala Arg Val Ala
                   85                  90                  95
   Leu Lys Glu Val Leu His Pro Ser Leu Lys Ser Asp Thr Ala Ile Leu
               100                 105                 110
   Gln Lys Ile Met Gln Tyr Val Asn Asp Ala Gly Thr Ser Thr Ser Tyr
           115                 120                 125
   Thr Ser Tyr Ile Gly Asn Leu Val Ala Thr Arg Ile Ser Ser Gln Trp
       130                 135                 140
   Gly Phe Thr Gly Pro Ser Phe Thr Val Thr Glu Gly Asn Asn Ser Val
   145                 150                 155                 160
   Tyr Arg Cys Ala Gln Leu Ala Lys Asp Met Leu Gln Val Asn Arg Val
                   165                 170                 175
   Asp Ala Val Val Ile Ala Gly Val Asp Leu Asn Gly Ser Ala Glu Ser
               180                 185                 190
   Phe Phe Val Arg Ala Asn Arg Gln Lys Ile Ser Lys Leu Ser His Pro
           195                 200                 205
   Cys Ala Ser Phe Asp Arg Asp Ala Asp Gly Phe Phe Ala Gly Glu Gly
       210                 215                 220
   Cys Gly Ala Leu Val Phe Lys Arg Leu Glu Asp Cys Ala Pro Gln Glu
   225                 230                 235                 240
   Lys Ile Tyr Ala Ser Ile Asp Ser Ile Ala Ile Asp Lys Glu Pro Thr
                   245                 250                 255
   Ser Ser Ala Val Lys Ala Val Tyr Gln Ser Asp Ser Ser Leu Ser Asp
               260                 265                 270
   Ile Glu Leu Leu Glu Ile Ser Gly Asp Ser Lys Arg Phe Ala Ala Phe
           275                 280                 285
   Glu Gly Ala Val Glu Ile Gln Ser Ser Val Glu Ala Gln Leu Lys Gly
       290                 295                 300
   Leu Ser Lys Val Leu Glu Pro Ala Lys Gly Gln Gly Val Ala Val Gly
   305                 310                 315                 320
   Ser Thr Arg Ala Thr Val Gly Asp Ile Gly Tyr Ala Thr Gly Ala Ala
                   325                 330                 335
   Ser Leu Ile Lys Thr Ala Leu Cys Leu Tyr Asn Arg Tyr Leu Pro Ala
               340                 345                 350
   Leu Ala Asn Trp Ser Gly Pro Cys Glu Gln Ser Ala Trp Gly Ser Asn
           355                 360                 365
   Met Phe Val Cys His Glu Thr Arg Pro Trp Met Lys Asn Gln Asn Glu
       370                 375                 380
   Lys Arg Cys Ala Leu Ile Ser Gly Thr Asp Pro Ser His Thr Cys Phe
   385                 390                 395                 400
   Ser Leu Val Leu Ser Asp Thr Gly Cys Tyr Glu Glu His Asn Arg Thr
                   405                 410                 415
   Cys Phe Asp Val Gln Ala Pro Gln Leu Val Leu Ile His Gly Phe Asp
   420 425 430
   Gly Lys Thr Ile Val Arg Arg Leu Glu Gly Tyr Leu Leu Glu Leu Val
   435 440 445
   Glu Gly His Ala Ser Pro Ser Glu Tyr Phe His Lys Leu Ile Gly Gln
   450 455 460
   Ser Leu Leu Glu Asn Ser Lys Glu Ser Lys Leu Thr Leu Ser Leu Val
   465 470 475 480
   Cys Asn Pro Asn Gln Leu Gln Lys Glu Leu Met Leu Ala Ile Lys Gly
   485 490 495
   Val Gln Arg Ser
   500
<210> 57
   <211> 1500
   <212> DNA
   <213> Thraustochytrium sp.
<220>
   <221> CDS
   <222> (1)..(1500)
<400> 57
   atg tta aca ggg aag gat tgg gtc agt cca tca gga agt tgt ttt gcc      48
   Met Leu Thr Gly Lys Asp Trp Val Ser Pro Ser Gly Ser Cys Phe Ala
   1               5                   10                  15
   cca aat ccg tta tca agc gca aaa gtg gca ttc atg tac gga gaa ggc      96
   Pro Asn Pro Leu Ser Ser Ala Lys Val Ala Phe Met Tyr Gly Glu Gly
               20                  25                  30
   cga agc ccg tac tgt ggt gta ggc ttg ggt cta cat cgt ttg tgg ccc     144
   Arg Ser Pro Tyr Cys Gly Val Gly Leu Gly Leu His Arg Leu Trp Pro
           35                  40                  45
   ggt ctc cat gaa aat gtg aac aat aag aca gtc gat tta tgg acg gaa     192
   Gly Leu His Glu Asn Val Asn Asn Lys Thr Val Asp Leu Trp Thr Glu
       50                  55                  60
   gga gat ggt tgg tta tat cct cga acg ttg aca cga gaa gag cat aca     240
   Gly Asp Gly Trp Leu Tyr Pro Arg Thr Leu Thr Arg Glu Glu His Thr
   65                  70                  75                  80
   aaa gcc atc gaa tct ttc aac gca aat caa att gaa atg ttt cgc gct     288
   Lys Ala Ile Glu Ser Phe Asn Ala Asn Gln Ile Glu Met Phe Arg Ala
                   85                  90                  95
   ggg att ttc atc tca atg tgt cag aca gac tat gtc atg aat gtt ctc     336
   Gly Ile Phe Ile Ser Met Cys Gln Thr Asp Tyr Val Met Asn Val Leu
               100                 105                 110
   ggt gtc cag cct aag gcc gga ttt ggg ctg agc ttg gga gaa att tca     384
   Gly Val Gln Pro Lys Ala Gly Phe Gly Leu Ser Leu Gly Glu Ile Ser
           115                 120                 125
   atg ctc ttt gcg atg tca aag gag aac tgc agg cag tca cag gaa atg     432
   Met Leu Phe Ala Met Ser Lys Glu Asn Cys Arg Gln Ser Gln Glu Met
       130                 135                 140
   acc aat cgt ttg cgc ggt tct cca gtg tgg tct aac gag ctt gct atc      480
   Thr Asn Arg Leu Arg Gly Ser Pro Val Trp Ser Asn Glu Leu Ala Ile
   145                 150                 155                 160
   aac ttc aat gca att cgc aag tta tgg aaa atc ccc cga gga gct ccc      528
   Asn Phe Asn Ala Ile Arg Lys Leu Trp Lys Ile Pro Arg Gly Ala Pro
               165                 170                 175
   tta gaa tcc ttt tgg caa gga tac ttg gtt cac ggc aca aga gaa gaa      576
   Leu Glu Ser Phe Trp Gln Gly Tyr Leu Val His Gly Thr Arg Glu Glu
           180                 185                 190
   gta gag cat gct att ggt ctt tct gag cct tat gta cgt ctg ctt att      624
   Val Glu His Ala Ile Gly Leu Ser Glu Pro Tyr Val Arg Leu Leu Ile
       195                 200                 205
   gtg aac gat tca agg agt gcc ttg att gct gga aaa cca gac gcc tgt      672
   Val Asn Asp Ser Arg Ser Ala Leu Ile Ala Gly Lys Pro Asp Ala Cys
       210                 215                 220
   cag gca gta atc agt aga cta aac tcc aag ttc cct tct ctg ccg gta      720
   Gln Ala Val Ile Ser Arg Leu Asn Ser Lys Phe Pro Ser Leu Pro Val
   225                 230                 235                 240
   aag caa gga atg att ggt cat tgc cca gaa gtt cgt gcg ttc atc aaa      768
   Lys Gln Gly Met Ile Gly His Cys Pro Glu Val Arg Ala Phe Ile Lys
               245                 250                 255
   gat att ggg tac atc cat gaa aca ctc cga att tcc aat gac tat tcg      816
   Asp Ile Gly Tyr Ile His Glu Thr Leu Arg Ile Ser Asn Asp Tyr Ser
           260                 265                 270
   gat tgt cag ctt ttc tca gcg gta acc aag ggc gca ctt gac agc tcc      864
   Asp Cys Gln Leu Phe Ser Ala Val Thr Lys Gly Ala Leu Asp Ser Ser
       275                 280                 285
   aca atg gaa atc aaa cac ttt gtg gga gag gtc tac tcc cgg atc gca      912
   Thr Met Glu Ile Lys His Phe Val Gly Glu Val Tyr Ser Arg Ile Ala
       290                 295                 300
   gac ttt cct caa atc gtc aac acg gtg cat tcg gct ggt tat gac gta      960
   Asp Phe Pro Gln Ile Val Asn Thr Val His Ser Ala Gly Tyr Asp Val
   305                 310                 315                 320
   ttt ctt gag ctt ggc tgt gat gct tct aga tct gca gca gtt caa aac     1008
   Phe Leu Glu Leu Gly Cys Asp Ala Ser Arg Ser Ala Ala Val Gln Asn
               325                 330                 335
   att ctt ggt ggt caa gga aag ttc ttg tct aca gct att gac aaa aaa     1056
   Ile Leu Gly Gly Gln Gly Lys Phe Leu Ser Thr Ala Ile Asp Lys Lys
           340                 345                 350
   gga cac tcc gcc tgg tca caa gta ctt cgg gct acc gca tca tta gct     1104
   Gly His Ser Ala Trp Ser Gln Val Leu Arg Ala Thr Ala Ser Leu Ala
       355                 360                 365
   gca cat cga gta ccg gga atc tca att ttg gat ttg ttt cac cca aat     1152
   Ala His Arg Val Pro Gly Ile Ser Ile Leu Asp Leu Phe His Pro Asn
       370                 375                 380
   ttc cga gaa atg tgc tgt aca atg gca acc aca cct aaa gtg gaa gat     1200
   Phe Arg Glu Met Cys Cys Thr Met Ala Thr Thr Pro Lys Val Glu Asp
   385                 390                 395                 400
   aag ttc ctg cgc acg att caa atc aat ggt cgg ttt gaa aaa gaa atg     1248
   Lys Phe Leu Arg Thr Ile Gln Ile Asn Gly Arg Phe Glu Lys Glu Met
               405                 410                 415
   att cac cta gaa gat aca aca tta agt tgc tta ccc gct cca agt gaa     1296
   Ile His Leu Glu Asp Thr Thr Leu Ser Cys Leu Pro Ala Pro Ser Glu
           420                 425                 430
   gca aat atc gca gct att caa tct cgg tca att cga tct gct gcg gcg       1344
   Ala Asn Ile Ala Ala Ile Gln Ser Arg Ser Ile Arg Ser Ala Ala Ala
           435                 440                 445
   cgt tct gga caa tcc cat gat tgt gca tcc cat agc cat gaa gaa aat       1392
   Arg Ser Gly Gln Ser His Asp Cys Ala Ser His Ser His Glu Glu Asn
       450                 455                 460
   aag gat tca tgc cct gaa aag ctg aag ctt gat tct gtg tcc gtc gcc       1440
   Lys Asp Ser Cys Pro Glu Lys Leu Lys Leu Asp Ser Val Ser Val Ala
   465                 470                 475                 480
   ata aat ttc gac aat gat gac cgc att cag ctt ggg cac gcg ggt ttt       1488
   Ile Asn Phe Asp Asn Asp Asp Arg Ile Gln Leu Gly His Ala Gly Phe
                   485                 490                 495
   cgg gag atg tac                                                       1500
   Arg Glu Met Tyr
               500
<210> 58
   <211> 500
   <212> PRT
   <213> Thraustochytrium sp.
<400> 58
   Met Leu Thr Gly Lys Asp Trp Val Ser Pro Ser Gly Ser Cys Phe Ala
   1               5                   10                  15
   Pro Asn Pro Leu Ser Ser Ala Lys Val Ala Phe Met Tyr Gly Glu Gly
               20                  25                  30
   Arg Ser Pro Tyr Cys Gly Val Gly Leu Gly Leu His Arg Leu Trp Pro
           35                  40                  45
   Gly Leu His Glu Asn Val Asn Asn Lys Thr Val Asp Leu Trp Thr Glu
       50                  55                  60
   Gly Asp Gly Trp Leu Tyr Pro Arg Thr Leu Thr Arg Glu Glu His Thr
   65                  70                  75                  80
   Lys Ala Ile Glu Ser Phe Asn Ala Asn Gln Ile Glu Met Phe Arg Ala
                   85                  90                  95
   Gly Ile Phe Ile Ser Met Cys Gln Thr Asp Tyr Val Met Asn Val Leu
               100                 105                 110
   Gly Val Gln Pro Lys Ala Gly Phe Gly Leu Ser Leu Gly Glu Ile Ser
           115                 120                 125
   Met Leu Phe Ala Met Ser Lys Glu Asn Cys Arg Gln Ser Gln Glu Met
       130                 135                 140
   Thr Asn Arg Leu Arg Gly Ser Pro Val Trp Ser Asn Glu Leu Ala Ile
   145                 150                 155                 160
   Asn Phe Asn Ala Ile Arg Lys Leu Trp Lys Ile Pro Arg Gly Ala Pro
                   165                 170                 175
   Leu Glu Ser Phe Trp Gln Gly Tyr Leu Val His Gly Thr Arg Glu Glu
               180                 185                 190
   Val Glu His Ala Ile Gly Leu Ser Glu Pro Tyr Val Arg Leu Leu Ile
           195                 200                 205
   Val Asn Asp Ser Arg Ser Ala Leu Ile Ala Gly Lys Pro Asp Ala Cys
       210                 215                 220
   Gln Ala Val Ile Ser Arg Leu Asn Ser Lys Phe Pro Ser Leu Pro Val
   225                 230                 235                 240
   Lys Gln Gly Met Ile Gly His Cys Pro Glu Val Arg Ala Phe Ile Lys
                   245                 250                 255
   Asp Ile Gly Tyr Ile His Glu Thr Leu Arg Ile Ser Asn Asp Tyr Ser
               260                 265                 270
   Asp Cys Gln Leu Phe Ser Ala Val Thr Lys Gly Ala Leu Asp Ser Ser
           275                 280                 285
   Thr Met Glu Ile Lys His Phe Val Gly Glu Val Tyr Ser Arg Ile Ala
       290                 295                 300
   Asp Phe Pro Gln Ile Val Asn Thr Val His Ser Ala Gly Tyr Asp Val
   305                 310                 315                 320
   Phe Leu Glu Leu Gly Cys Asp Ala Ser Arg Ser Ala Ala Val Gln Asn
                   325                 330                 335
   Ile Leu Gly Gly Gln Gly Lys Phe Leu Ser Thr Ala Ile Asp Lys Lys
               340                 345                 350
   Gly His Ser Ala Trp Ser Gln Val Leu Arg Ala Thr Ala Ser Leu Ala
           355                 360                 365
   Ala His Arg Val Pro Gly Ile Ser Ile Leu Asp Leu Phe His Pro Asn
       370                 375                 380
   Phe Arg Glu Met Cys Cys Thr Met Ala Thr Thr Pro Lys Val Glu Asp
   385                 390                 395                 400
   Lys Phe Leu Arg Thr Ile Gln Ile Asn Gly Arg Phe Glu Lys Glu Met
                   405                 410                 415
   Ile His Leu Glu Asp Thr Thr Leu Ser Cys Leu Pro Ala Pro Ser Glu
               420                 425                 430
   Ala Asn Ile Ala Ala Ile Gln Ser Arg Ser Ile Arg Ser Ala Ala Ala
           435                 440                 445
   Arg Ser Gly Gln Ser His Asp Cys Ala Ser His Ser His Glu Glu Asn
       450                 455                 460
   Lys Asp Ser Cys Pro Glu Lys Leu Lys Leu Asp Ser Val Ser Val Ala
   465                 470                 475                 480
   Ile Asn Phe Asp Asn Asp Asp Arg Ile Gln Leu Gly His Ala Gly Phe
                   485                 490                 495
   Arg Glu Met Tyr
               500
<210> 59
   <211> 1305
   <212> DNA
   <213> Thraustochytrium sp.
<220>
   <221> CDS
   <222> (1)..(1305)
<400> 59
   aat aca aga tat agc ttg tac aca ggg gcg atg gca aag gga att gca      48
   Asn Thr Arg Tyr Ser Leu Tyr Thr Gly Ala Met Ala Lys Gly Ile Ala
   1               5                   10                  15
   tct gca gat ctt gtc att gcc gct ggg aaa gag ggc atc cta gct tcc      96
   Ser Ala Asp Leu Val Ile Ala Ala Gly Lys Glu Gly Ile Leu Ala Ser
               20                  25                  30
   tat gga gct gga gga cta cct ctt gct act gtt cga aag gga ata gac     144
   Tyr Gly Ala Gly Gly Leu Pro Leu Ala Thr Val Arg Lys Gly Ile Asp
           35                  40                  45
   aaa att caa caa gcc ttg cca agt ggc cca tat gct gta aat ctt att     192
   Lys Ile Gln Gln Ala Leu Pro Ser Gly Pro Tyr Ala Val Asn Leu Ile
       50                  55                  60
   cac tct ccc ttt gac ggc aac ttg gag cag gga aac gtc gat ttg ttc     240
   His Ser Pro Phe Asp Gly Asn Leu Glu Gln Gly Asn Val Asp Leu Phe
   65                  70                  75                  80
   ttg gaa aag aac gtc cgc gtg gcg gaa tgt tcc gcg ttt aca acg cta     288
   Leu Glu Lys Asn Val Arg Val Ala Glu Cys Ser Ala Phe Thr Thr Leu
                   85                  90                  95
   aca gtg cca gta gta cac tat cgt gct gca ggg ctt gtt cgg cgc caa     336
   Thr Val Pro Val Val His Tyr Arg Ala Ala Gly Leu Val Arg Arg Gln
               100                 105                 110
   gat gga agc att ttg atc aag aac cga atc att gct aaa gta tct agg     384
   Asp Gly Ser Ile Leu Ile Lys Asn Arg Ile Ile Ala Lys Val Ser Arg
           115                 120                 125
   aca gaa ctc gct gag atg ttc ctt cgt ccg gca cct caa atc atc ctc     432
   Thr Glu Leu Ala Glu Met Phe Leu Arg Pro Ala Pro Gln Ile Ile Leu
       130                 135                 140
   gaa aaa ctg gta gca gca gaa atc att tca tct gac caa gcg cgt atg     480
   Glu Lys Leu Val Ala Ala Glu Ile Ile Ser Ser Asp Gln Ala Arg Met
   145                 150                 155                 160
   gca gcc aaa gtt ccc atg gcg gac gac atc gca gtc gaa gcc gac tct     528
   Ala Ala Lys Val Pro Met Ala Asp Asp Ile Ala Val Glu Ala Asp Ser
                   165                 170                 175
   ggt ggg cac acg gat aat cgg cct atg cac gtc att ttg ccc ctg ata     576
   Gly Gly His Thr Asp Asn Arg Pro Met His Val Ile Leu Pro Leu Ile
               180                 185                 190
   att caa ctc cgc aat act ata ctt gca gag tat ggc tgt gcc acg gct      624
   Ile Gln Leu Arg Asn Thr Ile Leu Ala Glu Tyr Gly Cys Ala Thr Ala
           195                 200                 205
   ttt cgt acc cgt ata ggc gct gga gga ggc att ggt tgt cct tca gcg      672
   Phe Arg Thr Arg Ile Gly Ala Gly Gly Gly Ile Gly Cys Pro Ser Ala
       210                 215                 220
   gcc ctc gca gcc ttt gat atg ggt gcg agt ttt gtc gtg act gga agc      720
   Ala Leu Ala Ala Phe Asp Met Gly Ala Ser Phe Val Val Thr Gly Ser
   225                 230                 235                 240
   ata aat caa att tgc cgc gag gca ggg act tgc gat act gtt cgg gag      768
   Ile Asn Gln Ile Cys Arg Glu Ala Gly Thr Cys Asp Thr Val Arg Glu
                   245                 250                 255
   cta ctt gcc aac tca agc tac tcg gac gtg acg atg gcg cca gca gca      816
   Leu Leu Ala Asn Ser Ser Tyr Ser Asp Val Thr Met Ala Pro Ala Ala
               260                 265                 270
   gac atg ttt gac caa ggt gtg aaa ctc caa gtc tta aaa cga gga acg      864
   Asp Met Phe Asp Gln Gly Val Lys Leu Gln Val Leu Lys Arg Gly Thr
           275                 280                 285
   atg ttt cca agc aga gca aat aaa ctc cgg aag ctc ttt gtg aac tac      912
   Met Phe Pro Ser Arg Ala Asn Lys Leu Arg Lys Leu Phe Val Asn Tyr
       290                 295                 300
   gaa tct cta gaa aca ctc ccg tcg aaa gag ttg aaa tac ctg gaa aac      960
   Glu Ser Leu Glu Thr Leu Pro Ser Lys Glu Leu Lys Tyr Leu Glu Asn
   305                 310                 315                 320
   atc ata ttc aag caa gca gta gac cag gtg tgg gag gaa aca aag cgc     1008
   Ile Ile Phe Lys Gln Ala Val Asp Gln Val Trp Glu Glu Thr Lys Arg
                   325                 330                 335
   ttt tac tgt gaa aaa ctg aac aat cca gat aaa att gca agg gcc atg     1056
   Phe Tyr Cys Glu Lys Leu Asn Asn Pro Asp Lys Ile Ala Arg Ala Met
               340                 345                 350
   aaa gat cct aaa ttg aag atg tcg ctt tgc ttt cgg tgg tat ctc tcc     1104
   Lys Asp Pro Lys Leu Lys Met Ser Leu Cys Phe Arg Trp Tyr Leu Ser
           355                 360                 365
   aag agc tct ggg tgg gcc aac gca gga att aaa tct cgt gca ctc gac     1152
   Lys Ser Ser Gly Trp Ala Asn Ala Gly Ile Lys Ser Arg Ala Leu Asp
       370                 375                 380
   tac cag atc tgg tgt ggc ccg gca atg ggc tcg ttc aac aat ttc gcc     1200
   Tyr Gln Ile Trp Cys Gly Pro Ala Met Gly Ser Phe Asn Asn Phe Ala
   385                 390                 395                 400
   agc ggc aca tcc ctc gat tgg aaa gtg act ggg gtt ttc cct ggc gtt     1248
   Ser Gly Thr Ser Leu Asp Trp Lys Val Thr Gly Val Phe Pro Gly Val
                   405                 410                 415
   gcg gaa gta aac atg gcc att tta gat ggc gcg cga gaa cta gct gct     1296
   Ala Glu Val Asn Met Ala Ile Leu Asp Gly Ala Arg Glu Leu Ala Ala
               420                 425                 430
   aaa cga aat                                                         1305
   Lys Arg Asn
           435
<210> 60
   <211> 435
   <212> PRT
   <213> Thraustochytrium sp.
<400> 60
   Asn Thr Arg Tyr Ser Leu Tyr Thr Gly Ala Met Ala Lys Gly Ile Ala
   1               5                   10                  15
   Ser Ala Asp Leu Val Ile Ala Ala Gly Lys Glu Gly Ile Leu Ala Ser
               20                  25                  30
   Tyr Gly Ala Gly Gly Leu Pro Leu Ala Thr Val Arg Lys Gly Ile Asp
           35                  40                  45
   Lys Ile Gln Gln Ala Leu Pro Ser Gly Pro Tyr Ala Val Asn Leu Ile
       50                  55                  60
   His Ser Pro Phe Asp Gly Asn Leu Glu Gln Gly Asn Val Asp Leu Phe
   65                  70                  75                  80
   Leu Glu Lys Asn Val Arg Val Ala Glu Cys Ser Ala Phe Thr Thr Leu
                   85                  90                  95
   Thr Val Pro Val Val His Tyr Arg Ala Ala Gly Leu Val Arg Arg Gln
               100                 105                 110
   Asp Gly Ser Ile Leu Ile Lys Asn Arg Ile Ile Ala Lys Val Ser Arg
           115                 120                 125
   Thr Glu Leu Ala Glu Met Phe Leu Arg Pro Ala Pro Gln Ile Ile Leu
       130                 135                 140
   Glu Lys Leu Val Ala Ala Glu Ile Ile Ser Ser Asp Gln Ala Arg Met
   145                 150                 155                 160
   Ala Ala Lys Val Pro Met Ala Asp Asp Ile Ala Val Glu Ala Asp Ser
                   165                 170                 175
   Gly Gly His Thr Asp Asn Arg Pro Met His Val Ile Leu Pro Leu Ile
               180                 185                 190
   Ile Gln Leu Arg Asn Thr Ile Leu Ala Glu Tyr Gly Cys Ala Thr Ala
           195                 200                 205
   Phe Arg Thr Arg Ile Gly Ala Gly Gly Gly Ile Gly Cys Pro Ser Ala
       210                 215                 220
   Ala Leu Ala Ala Phe Asp Met Gly Ala Ser Phe Val Val Thr Gly Ser
   225                 230                 235                 240
   Ile Asn Gln Ile Cys Arg Glu Ala Gly Thr Cys Asp Thr Val Arg Glu
                   245                 250                 255
   Leu Leu Ala Asn Ser Ser Tyr Ser Asp Val Thr Met Ala Pro Ala Ala
               260                 265                 270
   Asp Met Phe Asp Gln Gly Val Lys Leu Gln Val Leu Lys Arg Gly Thr
           275                 280                 285
   Met Phe Pro Ser Arg Ala Asn Lys Leu Arg Lys Leu Phe Val Asn Tyr
   290 295 300
   Glu Ser Leu Glu Thr Leu Pro Ser Lys Glu Leu Lys Tyr Leu Glu Asn
   305 310 315 320
   Ile Ile Phe Lys Gln Ala Val Asp Gln Val Trp Glu Glu Thr Lys Arg
   325 330 335
   Phe Tyr Cys Glu Lys Leu Asn Asn Pro Asp Lys Ile Ala Arg Ala Met
   340 345 350
   Lys Asp Pro Lys Leu Lys Met Ser Leu Cys Phe Arg Trp Tyr Leu Ser
   355 360 365
   Lys Ser Ser Gly Trp Ala Asn Ala Gly Ile Lys Ser Arg Ala Leu Asp
   370 375 380
   Tyr Gln Ile Trp Cys Gly Pro Ala Met Gly Ser Phe Asn Asn Phe Ala
   385 390 395 400
   Ser Gly Thr Ser Leu Asp Trp Lys Val Thr Gly Val Phe Pro Gly Val
   405 410 415
   Ala Glu Val Asn Met Ala Ile Leu Asp Gly Ala Arg Glu Leu Ala Ala
   420 425 430
   Lys Arg Asn
   435
<210> 61
   <211> 4410
   <212> DNA
   <213> Thraustochytrium sp.
<220>
   <221> CDS
   <222> (1)..(4410)
<400> 61
   atg ggc ccg cga gtg gcg tca ggc aag gtg ccg gct tgg gag atg agc      48
   Met Gly Pro Arg Val Ala Ser Gly Lys Val Pro Ala Trp Glu Met Ser
   1               5                   10                  15
   aag tcc gag ctg tgt gat gac cgc acg gta gtc ttt gac tat gag gag      96
   Lys Ser Glu Leu Cys Asp Asp Arg Thr Val Val Phe Asp Tyr Glu Glu
               20                  25                  30
   ctg ctg gag ttc gct gag ggc gat atc agt aag gtt ttt ggg ccg gag     144
   Leu Leu Glu Phe Ala Glu Gly Asp Ile Ser Lys Val Phe Gly Pro Glu
           35                  40                  45
   ttc aaa gtg gtg gac ggg ttt agg cgc agg gtg agg ttg ccc gct cga     192
   Phe Lys Val Val Asp Gly Phe Arg Arg Arg Val Arg Leu Pro Ala Arg
       50                  55                  60
   gag tac ctg ctg gtg acc cgg gtt acg ctg atg gat gcc gag gtg ggc     240
   Glu Tyr Leu Leu Val Thr Arg Val Thr Leu Met Asp Ala Glu Val Gly
   65                  70                  75                  80
   aac ttt cga gtg gga gca cgt atg gtg aca gag tat gac gta cct gtg      288
   Asn Phe Arg Val Gly Ala Arg Met Val Thr Glu Tyr Asp Val Pro Val
                   85                  90                  95
   aac gga gag ctc tcg gaa ggg gga gat gtg ccg tgg gct gtg ttg gtg      336
   Asn Gly Glu Leu Ser Glu Gly Gly Asp Val Pro Trp Ala Val Leu Val
               100                 105                 110
   gaa gcc ggg cag tgc gac ttg ctg cta att tct tac atg ggc atc gat      384
   Glu Ala Gly Gln Cys Asp Leu Leu Leu Ile Ser Tyr Met Gly Ile Asp
           115                 120                 125
   ttc cag tgc aaa gga gag cgg gtc tac cgg ctg ctg aac acc acc ttg      432
   Phe Gln Cys Lys Gly Glu Arg Val Tyr Arg Leu Leu Asn Thr Thr Leu
       130                 135                 140
   acg ttt ttt ggc gtc gcg aaa gaa ggg gaa acg ctt gtg tac gat att      480
   Thr Phe Phe Gly Val Ala Lys Glu Gly Glu Thr Leu Val Tyr Asp Ile
   145                 150                 155                 160
   cgc gtc acg ggt ttc gcc aag agg ccg gac gga gat atc tcc atg ttc      528
   Arg Val Thr Gly Phe Ala Lys Arg Pro Asp Gly Asp Ile Ser Met Phe
                   165                 170                 175
   ttt ttc gaa tat gat tgc tac tgc aat ggc aag ctt ctc atc gaa atg      576
   Phe Phe Glu Tyr Asp Cys Tyr Cys Asn Gly Lys Leu Leu Ile Glu Met
               180                 185                 190
   cga gat ggc tct gca ggc ttc ttc acg gac gaa gag ctc gct gcc ggc      624
   Arg Asp Gly Ser Ala Gly Phe Phe Thr Asp Glu Glu Leu Ala Ala Gly
           195                 200                 205
   aaa gga gtg gtc gtc act cgt gca cag caa aac atg cgg gac aaa att      672
   Lys Gly Val Val Val Thr Arg Ala Gln Gln Asn Met Arg Asp Lys Ile
       210                 215                 220
   gta cgg cag tcc att gag cct ttt gca ctg gcg gct tgc acg cac aaa      720
   Val Arg Gln Ser Ile Glu Pro Phe Ala Leu Ala Ala Cys Thr His Lys
   225                 230                 235                 240
   acg act ctg aac gag agt gac atg cag tcc ctt gtg gag cga aac tgg      768
   Thr Thr Leu Asn Glu Ser Asp Met Gln Ser Leu Val Glu Arg Asn Trp
                   245                 250                 255
   gca aac gtt ttt ggc acc agt aac aag atg gcg gag ctc aac tat aaa      816
   Ala Asn Val Phe Gly Thr Ser Asn Lys Met Ala Glu Leu Asn Tyr Lys
               260                 265                 270
   att tgc gcc agg aaa atg ctc atg atc gac agg gtt acc cac att gac      864
   Ile Cys Ala Arg Lys Met Leu Met Ile Asp Arg Val Thr His Ile Asp
           275                 280                 285
   cac cac ggt ggg gcg tat ggc ctc gga cta ctt gtt gga gag aag atc      912
   His His Gly Gly Ala Tyr Gly Leu Gly Leu Leu Val Gly Glu Lys Ile
       290                 295                 300
   ttg gat cga aac cat tgg tac ttt cct tgt cac ttt gtc aat gat caa      960
   Leu Asp Arg Asn His Trp Tyr Phe Pro Cys His Phe Val Asn Asp Gln
   305                 310                 315                 320
   gtc atg gca ggg tca ctg gtc agc gat ggt tgc agc cag ctc tta aaa     1008
   Val Met Ala Gly Ser Leu Val Ser Asp Gly Cys Ser Gln Leu Leu Lys
                   325                 330                 335
   ctc tat atg atc tgg ctt ggc ctc cac ctg aaa atg gag gaa ttt gat     1056
   Leu Tyr Met Ile Trp Leu Gly Leu His Leu Lys Met Glu Glu Phe Asp
               340                 345                 350
   ttt ctc cca gtt agc ggc cac aaa aac aag gtg cga tgc agg gga caa     1104
   Phe Leu Pro Val Ser Gly His Lys Asn Lys Val Arg Cys Arg Gly Gln
           355                 360                 365
   att tca ccg cat aaa ggc aag ctt gtc tac gtc atg gaa atc aaa aag      1152
   Ile Ser Pro His Lys Gly Lys Leu Val Tyr Val Met Glu Ile Lys Lys
       370                 375                 380
   atg ggt tac gat caa gca tct gga agc cca tac gcc atc gcg gac gtt      1200
   Met Gly Tyr Asp Gln Ala Ser Gly Ser Pro Tyr Ala Ile Ala Asp Val
   385                 390                 395                 400
   gat atc att gac gtc aac gaa gag ctg ggt caa agt ttt gac atc aac      1248
   Asp Ile Ile Asp Val Asn Glu Glu Leu Gly Gln Ser Phe Asp Ile Asn
                   405                 410                 415
   gac ctt gcg agc tac gga aaa ggt gac ctg agc aaa aaa atc gtg gtt      1296
   Asp Leu Ala Ser Tyr Gly Lys Gly Asp Leu Ser Lys Lys Ile Val Val
               420                 425                 430
   gac ttc aaa gga att gct ttg cag ctc aaa ggc cgc gct ttt tca cgc      1344
   Asp Phe Lys Gly Ile Ala Leu Gln Leu Lys Gly Arg Ala Phe Ser Arg
           435                 440                 445
   atg agt tcc agc tcg tcc ttg aac gaa gga tgg caa tgt gtt cca aaa      1392
   Met Ser Ser Ser Ser Ser Leu Asn Glu Gly Trp Gln Cys Val Pro Lys
       450                 455                 460
   cca agc cag aga atg gaa cac gaa cag ccc cct gct cac tgc ctt gca      1440
   Pro Ser Gln Arg Met Glu His Glu Gln Pro Pro Ala His Cys Leu Ala
   465                 470                 475                 480
   agc gac ccc gaa gcc cct tca act gtg acc tgg cac cca atg tca aag      1488
   Ser Asp Pro Glu Ala Pro Ser Thr Val Thr Trp His Pro Met Ser Lys
                   485                 490                 495
   ctt cct ggc aac cct acg ccg ttc ttc tcc cct tca tct tac cct ccg      1536
   Leu Pro Gly Asn Pro Thr Pro Phe Phe Ser Pro Ser Ser Tyr Pro Pro
               500                 505                 510
   agg gca att tgc ttc atc cct ttc ccg ggc aat ccc ctt gac aac aac      1584
   Arg Ala Ile Cys Phe Ile Pro Phe Pro Gly Asn Pro Leu Asp Asn Asn
           515                 520                 525
   tgc aag gct gga gaa atg ccc ctg aac tgg tac aac atg tca gag ttc      1632
   Cys Lys Ala Gly Glu Met Pro Leu Asn Trp Tyr Asn Met Ser Glu Phe
       530                 535                 540
   atg tgt ggc aag gtt tct aac tgc ttg ggc cca gaa ttc gca cgc ttt      1680
   Met Cys Gly Lys Val Ser Asn Cys Leu Gly Pro Glu Phe Ala Arg Phe
   545                 550                 555                 560
   gac aag tcg aac acc agc cgg agc cct gct ttt gac ttg gct ctg gtg      1728
   Asp Lys Ser Asn Thr Ser Arg Ser Pro Ala Phe Asp Leu Ala Leu Val
                   565                 570                 575
   acc cga gtt gtt gaa gtc aca aac atg gaa cac ggc aag ttt cta aac      1776
   Thr Arg Val Val Glu Val Thr Asn Met Glu His Gly Lys Phe Leu Asn
               580                 585                 590
   gtt gat tgc aat cca agc aaa ggc aca atg gtg ggg gag ttt gac tgt      1824
   Val Asp Cys Asn Pro Ser Lys Gly Thr Met Val Gly Glu Phe Asp Cys
           595                 600                 605
   ccc caa gac gcg tgg ttc ttt gat ggt tcg tgc aac gac ggc cat atg      1872
   Pro Gln Asp Ala Trp Phe Phe Asp Gly Ser Cys Asn Asp Gly His Met
       610                 615                 620
   ccg tat tcc att atc atg gaa atc gga ctg caa acc tca ggt gtt ctc      1920
   Pro Tyr Ser Ile Ile Met Glu Ile Gly Leu Gln Thr Ser Gly Val Leu
   625                 630                 635                 640
   acc tcg gtg ttg aag gca ccg ctg act atg gac aag gat gac att ctc      1968
   Thr Ser Val Leu Lys Ala Pro Leu Thr Met Asp Lys Asp Asp Ile Leu
                   645                 650                 655
   ttt cga aac ctc gat gca agt gct gaa atg gtg cgt cca gac gtg gat      2016
   Phe Arg Asn Leu Asp Ala Ser Ala Glu Met Val Arg Pro Asp Val Asp
               660                 665                 670
   gtt cgc ggc aaa acg att cga aac gtg acc aag tgt acc ggc tat gca      2064
   Val Arg Gly Lys Thr Ile Arg Asn Val Thr Lys Cys Thr Gly Tyr Ala
           675                 680                 685
   atg ttg gga aag atg ggg att cac cgg ttc acg ttt gag ttg agc gtt      2112
   Met Leu Gly Lys Met Gly Ile His Arg Phe Thr Phe Glu Leu Ser Val
       690                 695                 700
   gac ggc gtg gta ttt tat aaa gga tcc act tcc ttt gga tgg ttc act      2160
   Asp Gly Val Val Phe Tyr Lys Gly Ser Thr Ser Phe Gly Trp Phe Thr
   705                 710                 715                 720
   ccc gag gtg ttt gct cag caa gct gga ctc gac aac ggg aaa aag acg      2208
   Pro Glu Val Phe Ala Gln Gln Ala Gly Leu Asp Asn Gly Lys Lys Thr
                   725                 730                 735
   gag ccc tgg tgc aag act aac aac acc tcg gtt cga aga gtt gaa atc      2256
   Glu Pro Trp Cys Lys Thr Asn Asn Thr Ser Val Arg Arg Val Glu Ile
               740                 745                 750
   gca tcc gcc aaa gga aaa gag cag ctg act gag aag ctt ccc gac gca      2304
   Ala Ser Ala Lys Gly Lys Glu Gln Leu Thr Glu Lys Leu Pro Asp Ala
           755                 760                 765
   act aat gct caa gtt ctt cgg cgt tca gag cag tgt gaa tac ctc gat      2352
   Thr Asn Ala Gln Val Leu Arg Arg Ser Glu Gln Cys Glu Tyr Leu Asp
       770                 775                 780
   tac ctc aat att gcc cct gac tct ggg ctg cat ggg aag ggc tac gcc      2400
   Tyr Leu Asn Ile Ala Pro Asp Ser Gly Leu His Gly Lys Gly Tyr Ala
   785                 790                 795                 800
   cac gga cac aaa gac gtt aac ccg caa gac tgg ttc ttc tct tgc cac      2448
   His Gly His Lys Asp Val Asn Pro Gln Asp Trp Phe Phe Ser Cys His
                   805                 810                 815
   ttt tgg ttc gat cct gta atg cca gga tct tta gga att gaa tca atg      2496
   Phe Trp Phe Asp Pro Val Met Pro Gly Ser Leu Gly Ile Glu Ser Met
               820                 825                 830
   ttc cag ctt atc gag gcc ttt gcg gtg gac caa aac att cct gga gag      2544
   Phe Gln Leu Ile Glu Ala Phe Ala Val Asp Gln Asn Ile Pro Gly Glu
           835                 840                 845
   tac aac gta tcc aat ccg acc ttt gcc cat gca cca ggc aaa acg gcg      2592
   Tyr Asn Val Ser Asn Pro Thr Phe Ala His Ala Pro Gly Lys Thr Ala
       850                 855                 860
   tgg aaa tac cga ggc cag ctc aca cca aag aac cgt gcg atg gac tgc      2640
   Trp Lys Tyr Arg Gly Gln Leu Thr Pro Lys Asn Arg Ala Met Asp Cys
   865                 870                 875                 880
   gag gtg cat atc gtt tca att acc gcc tcc ccc gag aac ggg ggc tac      2688
   Glu Val His Ile Val Ser Ile Thr Ala Ser Pro Glu Asn Gly Gly Tyr
                   885                 890                 895
   gtt gac atc gtg gcc gat gga gcg ctt tgg gta gat gga ctt cgc gtg      2736
   Val Asp Ile Val Ala Asp Gly Ala Leu Trp Val Asp Gly Leu Arg Val
               900                 905                 910
   tac gaa gcc aaa gag ctt cga gtt cgt gtc gtt tcg gca aaa cct caa      2784
   Tyr Glu Ala Lys Glu Leu Arg Val Arg Val Val Ser Ala Lys Pro Gln
           915                 920                 925
   gca att ccg gat gta caa caa cag cca cct agc gca aag gcg gac ccg      2832
   Ala Ile Pro Asp Val Gln Gln Gln Pro Pro Ser Ala Lys Ala Asp Pro
       930                 935                 940
   ggg aaa aca gga gtt gca ctt tcg ccc act cag cta cgc gac gtc ctg      2880
   Gly Lys Thr Gly Val Ala Leu Ser Pro Thr Gln Leu Arg Asp Val Leu
   945                 950                 955                 960
   ctt gaa gtg gac aat cca ttg tat ctt ggt gta gag aac tcc aat ttg      2928
   Leu Glu Val Asp Asn Pro Leu Tyr Leu Gly Val Glu Asn Ser Asn Leu
                   965                 970                 975
   gtg cag ttt gag tcg aaa cct gca act tct tca cgt atc gtt tcg atc      2976
   Val Gln Phe Glu Ser Lys Pro Ala Thr Ser Ser Arg Ile Val Ser Ile
               980                 985                 990
   aaa ccg tgc tcg att agt gac ctt  ggc gat aag tct ttt  atg gaa acg    3024
   Lys Pro Cys Ser Ile Ser Asp Leu  Gly Asp Lys Ser Phe  Met Glu Thr
           995                 1000                 1005
   tac aac  gtg tca gca cct ctg  tat act gga gca atg  gcc aag ggc      3069
   Tyr Asn  Val Ser Ala Pro Leu  Tyr Thr Gly Ala Met  Ala Lys Gly
       1010                 1015                 1020
   att gca  tcc gcc gac ttg gtc  att gct gct ggg aaa  cgc aag ata      3114
   Ile Ala  Ser Ala Asp Leu Val  Ile Ala Ala Gly Lys  Arg Lys Ile
       1025                 1030                 1035
   ctt gga  tcg ttt ggt gcg gga  ggg ctg cct att tcc  ata gtc cgt      3159
   Leu Gly  Ser Phe Gly Ala Gly  Gly Leu Pro Ile Ser  Ile Val Arg
       1040                 1045                 1050
   gaa gca  ctg gag aaa att caa  caa cac ctg ccc cac  ggc ccc tac      3204
   Glu Ala  Leu Glu Lys Ile Gln  Gln His Leu Pro His  Gly Pro Tyr
       1055                 1060                 1065
   gct gtt  aac ctc att cac tcg  cct ttc gac agc aac  ttg gaa aag      3249
   Ala Val  Asn Leu Ile His Ser  Pro Phe Asp Ser Asn  Leu Glu Lys
       1070                 1075                 1080
   ggc aac  gtt gac ctc ttt ctc  gag atg ggc gtg aca  gtg gta gaa      3294
   Gly Asn  Val Asp Leu Phe Leu  Glu Met Gly Val Thr  Val Val Glu
       1085                 1090                 1095
   tgc agc  gcg ttc atg gaa ctc  acg gcc cag gtt gtc  cgg tac cgc      3339
   Cys Ser  Ala Phe Met Glu Leu  Thr Ala Gln Val Val  Arg Tyr Arg
       1100                 1105                 1110
   gcg tct  ggt cta agc aaa agt  gcg gac ggt tcg att  cgc att gct      3384
   Ala Ser  Gly Leu Ser Lys Ser  Ala Asp Gly Ser Ile  Arg Ile Ala
       1115                 1120                 1125
   cac cgt  att att ggc aag gtt  tcc aga acc gag ctg  gca gaa atg      3429
   His Arg  Ile Ile Gly Lys Val  Ser Arg Thr Glu Leu  Ala Glu Met
       1130                 1135                 1140
   ttt att  cgt cca gca cca cag  cac ctc ctc caa aaa  ctc gta gcc      3474
   Phe Ile  Arg Pro Ala Pro Gln  His Leu Leu Gln Lys  Leu Val Ala
       1145                 1150                 1155
   tcc ggc  gag ctg aca gct gag  caa gcc gag ctt gca  aca cag gtt      3519
   Ser Gly  Glu Leu Thr Ala Glu  Gln Ala Glu Leu Ala  Thr Gln Val
       1160                 1165                 1170
   ccg gtg  gcg gat gac att gcg  gtc gaa gcc gac tcg  ggg ggg cat      3564
   Pro Val  Ala Asp Asp Ile Ala  Val Glu Ala Asp Ser  Gly Gly His
       1175                 1180                 1185
   acc gac  aac agg cct att cac  gtc att ctt cct cta  atc atc aac      3609
   Thr Asp  Asn Arg Pro Ile His  Val Ile Leu Pro Leu  Ile Ile Asn
       1190                 1195                 1200
   cta cgc  aac cgt ttg cat aaa  gag ctt gac tac cct  tcg cat ctc      3654
   Leu Arg  Asn Arg Leu His Lys  Glu Leu Asp Tyr Pro  Ser His Leu
       1205                 1210                 1215
   cgg gta cgt gtg ggt gct ggt ggt ggt att gga tgt cct caa gcc    3699
   Arg Val Arg Val Gly Ala Gly Gly Gly Ile Gly Cys Pro Gln Ala
       1220                1225                1230
   gct ctt gca gca ttt caa atg ggg gca gcg ttt tta atc act gga    3744
   Ala Leu Ala Ala Phe Gln Met Gly Ala Ala Phe Leu Ile Thr Gly
       1235                1240                1245
   acg gtg aac cag ctt gct cgt gaa agt ggc act tgt gac aac gtc    3789
   Thr Val Asn Gln Leu Ala Arg Glu Ser Gly Thr Cys Asp Asn Val
       1250                1255                1260
   cgg tta cag ctc tca aag gcc acg tat agc gac gtg tgt atg gct    3834
   Arg Leu Gln Leu Ser Lys Ala Thr Tyr Ser Asp Val Cys Met Ala
       1265                1270                1275
   cct gct gcc gat atg ttt gac caa ggc gtg gag ctg caa gta ttg    3879
   Pro Ala Ala Asp Met Phe Asp Gln Gly Val Glu Leu Gln Val Leu
       1280                1285                1290
   aag aaa ggc acg ctg ttc cca agt cgt gct aag aag ctg tac gag    3924
   Lys Lys Gly Thr Leu Phe Pro Ser Arg Ala Lys Lys Leu Tyr Glu
       1295                1300                1305
   ctg ttc tgc aag tat gac tcg ttt gag gca atg ccg gct gaa gaa    3969
   Leu Phe Cys Lys Tyr Asp Ser Phe Glu Ala Met Pro Ala Glu Glu
       1310                1315                1320
   ttg caa cgg gtt gaa aag cgg att ttt caa aag tcg ctt gct gaa    4014
   Leu Gln Arg Val Glu Lys Arg Ile Phe Gln Lys Ser Leu Ala Glu
       1325                1330                1335
   gtt tgg cag gag acc agt gac ttt tac att cat cgt atc aag aac    4059
   Val Trp Gln Glu Thr Ser Asp Phe Tyr Ile His Arg Ile Lys Asn
       1340                1345                1350
   cct gag aaa atc aat cgt gct gca agc gat ggc aaa ctg aaa atg    4104
   Pro Glu Lys Ile Asn Arg Ala Ala Ser Asp Gly Lys Leu Lys Met
       1355                1360                1365
   tcg ctt tgc ttt cgc tgg tac ctt ggg ctt tcc tca ttt tgg gcc    4149
   Ser Leu Cys Phe Arg Trp Tyr Leu Gly Leu Ser Ser Phe Trp Ala
       1370                1375                1380
   aac tct ggg gca caa gat cgc gtc atg gac tat caa att tgg tgt    4194
   Asn Ser Gly Ala Gln Asp Arg Val Met Asp Tyr Gln Ile Trp Cys
       1385                1390                1395
   ggc cct gct att ggc gct ttc aat gat ttt acc aag ggc acg tac    4239
   Gly Pro Ala Ile Gly Ala Phe Asn Asp Phe Thr Lys Gly Thr Tyr
       1400                1405                1410
   ctt gac gtg act gtt gca aag agt tac cct tgt gtg gca cag atc    4284
   Leu Asp Val Thr Val Ala Lys Ser Tyr Pro Cys Val Ala Gln Ile
       1415                1420                1425
   aat ttg caa att ttg caa gga gct gcg tat ctg aaa cgc ctt ggt    4329
   Asn Leu Gln Ile Leu Gln Gly Ala Ala Tyr Leu Lys Arg Leu Gly
       1430                1435                1440
   gtc att cgt ttt gac cgc atg ctg ctg cag gcc gtc gat atc gac    4374
   Val Ile Arg Phe Asp Arg Met Leu Leu Gln Ala Val Asp Ile Asp
       1445                1450                1455
   gat cct gta ttt act tac gtg ccg acc cag cca ctt                4410
   Asp Pro Val Phe Thr Tyr Val Pro Thr Gln Pro Leu
       1460                1465                1470
<212> PRT
   <213> Thraustochytrium sp.
<400> 62
<210> 63
   <211> 1500
   <212> DNA
   <213> Thraustochytrium sp.
<220>
   <221> CDS
   <222> (1)..(1500)
<400> 63
   atg ggc ccg cga gtg gcg tca ggc aag gtg ccg gct tgg gag atg agc       48
   Met Gly Pro Arg Val Ala Ser Gly Lys Val Pro Ala Trp Glu Met Ser
   1               5                   10                  15
   aag tcc gag ctg tgt gat gac cgc acg gta gtc ttt gac tat gag gag       96
   Lys Ser Glu Leu Cys Asp Asp Arg Thr Val Val Phe Asp Tyr Glu Glu
               20                  25                  30
   ctg ctg gag ttc gct gag ggc gat atc agt aag gtt ttt ggg ccg gag      144
   Leu Leu Glu Phe Ala Glu Gly Asp Ile Ser Lys Val Phe Gly Pro Glu
           35                  40                  45
   ttc aaa gtg gtg gac ggg ttt agg cgc agg gtg agg ttg ccc gct cga      192
   Phe Lys Val Val Asp Gly Phe Arg Arg Arg Val Arg Leu Pro Ala Arg
       50                  55                  60
   gag tac ctg ctg gtg acc cgg gtt acg ctg atg gat gcc gag gtg ggc      240
   Glu Tyr Leu Leu Val Thr Arg Val Thr Leu Met Asp Ala Glu Val Gly
   65                  70                  75                  80
   aac ttt cga gtg gga gca cgt atg gtg aca gag tat gac gta cct gtg      288
   Asn Phe Arg Val Gly Ala Arg Met Val Thr Glu Tyr Asp Val Pro Val
                   85                  90                  95
   aac gga gag ctc tcg gaa ggg gga gat gtg ccg tgg gct gtg ttg gtg      336
   Asn Gly Glu Leu Ser Glu Gly Gly Asp Val Pro Trp Ala Val Leu Val
               100                 105                 110
   gaa gcc ggg cag tgc gac ttg ctg cta att tct tac atg ggc atc gat      384
   Glu Ala Gly Gln Cys Asp Leu Leu Leu Ile Ser Tyr Met Gly Ile Asp
           115                 120                 125
   ttc cag tgc aaa gga gag cgg gtc tac cgg ctg ctg aac acc acc ttg      432
   Phe Gln Cys Lys Gly Glu Arg Val Tyr Arg Leu Leu Asn Thr Thr Leu
       130                 135                 140
   acg ttt ttt ggc gtc gcg aaa gaa ggg gaa acg ctt gtg tac gat att      480
   Thr Phe Phe Gly Val Ala Lys Glu Gly Glu Thr Leu Val Tyr Asp Ile
   145                 150                 155                 160
   cgc gtc acg ggt ttc gcc aag agg ccg gac gga gat atc tcc atg ttc       528
   Arg Val Thr Gly Phe Ala Lys Arg Pro Asp Gly Asp Ile Ser Met Phe
                   165                 170                 175
   ttt ttc gaa tat gat tgc tac tgc aat ggc aag ctt ctc atc gaa atg       576
   Phe Phe Glu Tyr Asp Cys Tyr Cys Asn Gly Lys Leu Leu Ile Glu Met
               180                 185                 190
   cga gat ggc tct gca ggc ttc ttc acg gac gaa gag ctc gct gcc ggc       624
   Arg Asp Gly Ser Ala Gly Phe Phe Thr Asp Glu Glu Leu Ala Ala Gly
           195                 200                 205
   aaa gga gtg gtc gtc act cgt gca cag caa aac atg cgg gac aaa att       672
   Lys Gly Val Val Val Thr Arg Ala Gln Gln Asn Met Arg Asp Lys Ile
       210                 215                 220
   gta cgg cag tcc att gag cct ttt gca ctg gcg gct tgc acg cac aaa       720
   Val Arg Gln Ser Ile Glu Pro Phe Ala Leu Ala Ala Cys Thr His Lys
   225                 230                 235                 240
   acg act ctg aac gag agt gac atg cag tcc ctt gtg gag cga aac tgg       768
   Thr Thr Leu Asn Glu Ser Asp Met Gln Ser Leu Val Glu Arg Asn Trp
                   245                 250                 255
   gca aac gtt ttt ggc acc agt aac aag atg gcg gag ctc aac tat aaa       816
   Ala Asn Val Phe Gly Thr Ser Asn Lys Met Ala Glu Leu Asn Tyr Lys
               260                 265                 270
   att tgc gcc agg aaa atg ctc atg atc gac agg gtt acc cac att gac       864
   Ile Cys Ala Arg Lys Met Leu Met Ile Asp Arg Val Thr His Ile Asp
           275                 280                 285
   cac cac ggt ggg gcg tat ggc ctc gga cta ctt gtt gga gag aag atc       912
   His His Gly Gly Ala Tyr Gly Leu Gly Leu Leu Val Gly Glu Lys Ile
       290                 295                 300
   ttg gat cga aac cat tgg tac ttt cct tgt cac ttt gtc aat gat caa       960
   Leu Asp Arg Asn His Trp Tyr Phe Pro Cys His Phe Val Asn Asp Gln
   305                 310                 315                 320
   gtc atg gca ggg tca ctg gtc agc gat ggt tgc agc cag ctc tta aaa      1008
   Val Met Ala Gly Ser Leu Val Ser Asp Gly Cys Ser Gln Leu Leu Lys
                   325                 330                 335
   ctc tat atg atc tgg ctt ggc ctc cac ctg aaa atg gag gaa ttt gat      1056
   Leu Tyr Met Ile Trp Leu Gly Leu His Leu Lys Met Glu Glu Phe Asp
               340                 345                 350
   ttt ctc cca gtt agc ggc cac aaa aac aag gtg cga tgc agg gga caa      1104
   Phe Leu Pro Val Ser Gly His Lys Asn Lys Val Arg Cys Arg Gly Gln
           355                 360                 365
   att tca ccg cat aaa ggc aag ctt gtc tac gtc atg gaa atc aaa aag      1152
   Ile Ser Pro His Lys Gly Lys Leu Val Tyr Val Met Glu Ile Lys Lys
       370                 375                 380
   atg ggt tac gat caa gca tct gga agc cca tac gcc atc gcg gac gtt      1200
   Met Gly Tyr Asp Gln Ala Ser Gly Ser Pro Tyr Ala Ile Ala Asp Val
   385                 390                 395                 400
   gat atc att gac gtc aac gaa gag ctg ggt caa agt ttt gac atc aac      1248
   Asp Ile Ile Asp Val Asn Glu Glu Leu Gly Gln Ser Phe Asp Ile Asn
                   405                 410                 415
   gac ctt gcg agc tac gga aaa ggt gac ctg agc aaa aaa atc gtg gtt      1296
   Asp Leu Ala Ser Tyr Gly Lys Gly Asp Leu Ser Lys Lys Ile Val Val
               420                 425                 430
   gac ttc aaa gga att gct ttg cag ctc aaa ggc cgc gct ttt tca cgc      1344
   Asp Phe Lys Gly Ile Ala Leu Gln Leu Lys Gly Arg Ala Phe Ser Arg
           435                 440                 445
   atg agt tcc agc tcg tcc ttg aac gaa gga tgg caa tgt gtt cca aaa        1392
   Met Ser Ser Ser Ser Ser Leu Asn Glu Gly Trp Gln Cys Val Pro Lys
       450                 455                 460
   cca agc cag aga atg gaa cac gaa cag ccc cct gct cac tgc ctt gca        1440
   Pro Ser Gln Arg Met Glu His Glu Gln Pro Pro Ala His Cys Leu Ala
   465                 470                 475                 480
   agc gac ccc gaa gcc cct tca act gtg acc tgg cac cca atg tca aag        1488
   Ser Asp Pro Glu Ala Pro Ser Thr Val Thr Trp His Pro Met Ser Lys
                   485                 490                 495
   ctt cct ggc aac                                                        1500
   Leu Pro Gly Asn
               500
<210> 64
   <211> 500
   <212> PRT
   <213> Thraustochytrium sp.
<400> 64
   Met Gly Pro Arg Val Ala Ser Gly Lys Val Pro Ala Trp Glu Met Ser
   1 5 10 15
   Lys Ser Glu Leu Cys Asp Asp Arg Thr Val Val Phe Asp Tyr Glu Glu
   20 25 30
   Leu Leu Glu Phe Ala Glu Gly Asp Ile Ser Lys Val Phe Gly Pro Glu
   35 40 45
   Phe Lys Val Val Asp Gly Phe Arg Arg Arg Val Arg Leu Pro Ala Arg
   50 55 60
   Glu Tyr Leu Leu Val Thr Arg Val Thr Leu Met Asp Ala Glu Val Gly
   65 70 75 80
   Asn Phe Arg Val Gly Ala Arg Met Val Thr Glu Tyr Asp Val Pro Val
   85 90 95
   Asn Gly Glu Leu Ser Glu Gly Gly Asp Val Pro Trp Ala Val Leu Val
   100 105 110
   Glu Ala Gly Gln Cys Asp Leu Leu Leu Ile Ser Tyr Met Gly Ile Asp
   115 120 125
   Phe Gln Cys Lys Gly Glu Arg Val Tyr Arg Leu Leu Asn Thr Thr Leu
   130 135 140
   Thr Phe Phe Gly Val Ala Lys Glu Gly Glu Thr Leu Val Tyr Asp Ile
   145 150 155 160
   Arg Val Thr Gly Phe Ala Lys Arg Pro Asp Gly Asp Ile Ser Met Phe
   165 170 175
   Phe Phe Glu Tyr Asp Cys Tyr Cys Asn Gly Lys Leu Leu Ile Glu Met
   180 185 190
   Arg Asp Gly Ser Ala Gly Phe Phe Thr Asp Glu Glu Leu Ala Ala Gly
   195 200 205
   Lys Gly Val Val Val Thr Arg Ala Gln Gln Asn Met Arg Asp Lys Ile
   210 215 220
   Val Arg Gln Ser Ile Glu Pro Phe Ala Leu Ala Ala Cys Thr His Lys
   225 230 235 240
   Thr Thr Leu Asn Glu Ser Asp Met Gln Ser Leu Val Glu Arg Asn Trp
   245 250 255
   Ala Asn Val Phe Gly Thr Ser Asn Lys Met Ala Glu Leu Asn Tyr Lys
   260 265 270
   Ile Cys Ala Arg Lys Met Leu Met Ile Asp Arg Val Thr His Ile Asp
   275 280 285
   His His Gly Gly Ala Tyr Gly Leu Gly Leu Leu Val Gly Glu Lys Ile
   290 295 300
   Leu Asp Arg Asn His Trp Tyr Phe Pro Cys His Phe Val Asn Asp Gln
   305 310 315 320
   Val Met Ala Gly Ser Leu Val Ser Asp Gly Cys Ser Gln Leu Leu Lys
   325 330 335
   Leu Tyr Met Ile Trp Leu Gly Leu His Leu Lys Met Glu Glu Phe Asp
   340 345 350
   Phe Leu Pro Val Ser Gly His Lys Asn Lys Val Arg Cys Arg Gly Gln
   355 360 365
   Ile Ser Pro His Lys Gly Lys Leu Val Tyr Val Met Glu Ile Lys Lys
   370 375 380
   Met Gly Tyr Asp Gln Ala Ser Gly Ser Pro Tyr Ala Ile Ala Asp Val
   385 390 395 400
   Asp Ile Ile Asp Val Asn Glu Glu Leu Gly Gln Ser Phe Asp Ile Asn
   405 410 415
   Asp Leu Ala Ser Tyr Gly Lys Gly Asp Leu Ser Lys Lys Ile Val Val
   420 425 430
   Asp Phe Lys Gly Ile Ala Leu Gln Leu Lys Gly Arg Ala Phe Ser Arg
   435 440 445
   Met Ser Ser Ser Ser Ser Leu Asn Glu Gly Trp Gln Cys Val Pro Lys
   450 455 460
   Pro Ser Gln Arg Met Glu His Glu Gln Pro Pro Ala His Cys Leu Ala
   465 470 475 480
   Ser Asp Pro Glu Ala Pro Ser Thr Val Thr Trp His Pro Met Ser Lys
   485 490 495
   Leu Pro Gly Asn
   500
<210> 65
   <211> 1500
   <212> DNA
   <213> Thraustochytrium sp.
<220>
   <221> CDS
   <222> (1)..(1500)
<400> 65
   cct acg ccg ttc ttc tcc cct tca tct tac cct ccg agg gca att tgc      48
   Pro Thr Pro Phe Phe Ser Pro Ser Ser Tyr Pro Pro Arg Ala Ile Cys
   1               5                   10                  15
   ttc atc cct ttc ccg ggc aat ccc ctt gac aac aac tgc aag gct gga      96
   Phe Ile Pro Phe Pro Gly Asn Pro Leu Asp Asn Asn Cys Lys Ala Gly
               20                  25                  30
   gaa atg ccc ctg aac tgg tac aac atg tca gag ttc atg tgt ggc aag     144
   Glu Met Pro Leu Asn Trp Tyr Asn Met Ser Glu Phe Met Cys Gly Lys
           35                  40                  45
   gtt tct aac tgc ttg ggc cca gaa ttc gca cgc ttt gac aag tcg aac     192
   Val Ser Asn Cys Leu Gly Pro Glu Phe Ala Arg Phe Asp Lys Ser Asn
       50                  55                  60
   acc agc cgg agc cct gct ttt gac ttg gct ctg gtg acc cga gtt gtt     240
   Thr Ser Arg Ser Pro Ala Phe Asp Leu Ala Leu Val Thr Arg Val Val
   65                  70                  75                  80
   gaa gtc aca aac atg gaa cac ggc aag ttt cta aac gtt gat tgc aat     288
   Glu Val Thr Asn Met Glu His Gly Lys Phe Leu Asn Val Asp Cys Asn
                   85                  90                  95
   cca agc aaa ggc aca atg gtg ggg gag ttt gac tgt ccc caa gac gcg     336
   Pro Ser Lys Gly Thr Met Val Gly Glu Phe Asp Cys Pro Gln Asp Ala
               100                 105                 110
   tgg ttc ttt gat ggt tcg tgc aac gac ggc cat atg ccg tat tcc att     384
   Trp Phe Phe Asp Gly Ser Cys Asn Asp Gly His Met Pro Tyr Ser Ile
           115                 120                 125
   atc atg gaa atc gga ctg caa acc tca ggt gtt ctc acc tcg gtg ttg     432
   Ile Met Glu Ile Gly Leu Gln Thr Ser Gly Val Leu Thr Ser Val Leu
       130                 135                 140
   aag gca ccg ctg act atg gac aag gat gac att ctc ttt cga aac ctc     480
   Lys Ala Pro Leu Thr Met Asp Lys Asp Asp Ile Leu Phe Arg Asn Leu
   145                 150                 155                 160
   gat gca agt gct gaa atg gtg cgt cca gac gtg gat gtt cgc ggc aaa     528
   Asp Ala Ser Ala Glu Met Val Arg Pro Asp Val Asp Val Arg Gly Lys
                   165                 170                 175
   acg att cga aac gtg acc aag tgt acc ggc tat gca atg ttg gga aag     576
   Thr Ile Arg Asn Val Thr Lys Cys Thr Gly Tyr Ala Met Leu Gly Lys
               180                 185                 190
   atg ggg att cac cgg ttc acg ttt gag ttg agc gtt gac ggc gtg gta     624
   Met Gly Ile His Arg Phe Thr Phe Glu Leu Ser Val Asp Gly Val Val
           195                 200                 205
   ttt tat aaa gga tcc act tcc ttt gga tgg ttc act ccc gag gtg ttt     672
   Phe Tyr Lys Gly Ser Thr Ser Phe Gly Trp Phe Thr Pro Glu Val Phe
       210                 215                 220
   gct cag caa gct gga ctc gac aac ggg aaa aag acg gag ccc tgg tgc     720
   Ala Gln Gln Ala Gly Leu Asp Asn Gly Lys Lys Thr Glu Pro Trp Cys
   225                 230                 235                 240
   aag act aac aac acc tcg gtt cga aga gtt gaa atc gca tcc gcc aaa     768
   Lys Thr Asn Asn Thr Ser Val Arg Arg Val Glu Ile Ala Ser Ala Lys
                   245                 250                 255
   gga aaa gag cag ctg act gag aag ctt ccc gac gca act aat gct caa     816
   Gly Lys Glu Gln Leu Thr Glu Lys Leu Pro Asp Ala Thr Asn Ala Gln
               260                 265                 270
   gtt ctt cgg cgt tca gag cag tgt gaa tac ctc gat tac ctc aat att     864
   Val Leu Arg Arg Ser Glu Gln Cys Glu Tyr Leu Asp Tyr Leu Asn Ile
           275                 280                 285
   gcc cct gac tct ggg ctg cat ggg aag ggc tac gcc cac gga cac aaa     912
   Ala Pro Asp Ser Gly Leu His Gly Lys Gly Tyr Ala His Gly His Lys
       290                 295                 300
   gac gtt aac ccg caa gac tgg ttc ttc tct tgc cac ttt tgg ttc gat     960
   Asp Val Asn Pro Gln Asp Trp Phe Phe Ser Cys His Phe Trp Phe Asp
   305                 310                 315                 320
   cct gta atg cca gga tct tta gga att gaa tca atg ttc cag ctt atc    1008
   Pro Val Met Pro Gly Ser Leu Gly Ile Glu Ser Met Phe Gln Leu Ile
                   325                 330                 335
   gag gcc ttt gcg gtg gac caa aac att cct gga gag tac aac gta tcc    1056
   Glu Ala Phe Ala Val Asp Gln Asn Ile Pro Gly Glu Tyr Asn Val Ser
               340                 345                 350
   aat ccg acc ttt gcc cat gca cca ggc aaa acg gcg tgg aaa tac cga    1104
   Asn Pro Thr Phe Ala His Ala Pro Gly Lys Thr Ala Trp Lys Tyr Arg
           355                 360                 365
   ggc cag ctc aca cca aag aac cgt gcg atg gac tgc gag gtg cat atc    1152
   Gly Gln Leu Thr Pro Lys Asn Arg Ala Met Asp Cys Glu Val His Ile
       370                 375                 380
   gtt tca att acc gcc tcc ccc gag aac ggg ggc tac gtt gac atc gtg    1200
   Val Ser Ile Thr Ala Ser Pro Glu Asn Gly Gly Tyr Val Asp Ile Val
   385                 390                 395                 400
   gcc gat gga gcg ctt tgg gta gat gga ctt cgc gtg tac gaa gcc aaa    1248
   Ala Asp Gly Ala Leu Trp Val Asp Gly Leu Arg Val Tyr Glu Ala Lys
                   405                 410                 415
   gag ctt cga gtt cgt gtc gtt tcg gca aaa cct caa gca att ccg gat    1296
   Glu Leu Arg Val Arg Val Val Ser Ala Lys Pro Gln Ala Ile Pro Asp
               420                 425                 430
   gta caa caa cag cca cct agc gca aag gcg gac ccg ggg aaa aca gga    1344
   Val Gln Gln Gln Pro Pro Ser Ala Lys Ala Asp Pro Gly Lys Thr Gly
           435                 440                 445
   gtt gca ctt tcg ccc act cag cta cgc gac gtc ctg ctt gaa gtg gac    1392
   Val Ala Leu Ser Pro Thr Gln Leu Arg Asp Val Leu Leu Glu Val Asp
       450                 455                 460
   aat cca ttg tat ctt ggt gta gag aac tcc aat ttg gtg cag ttt gag    1440
   Asn Pro Leu Tyr Leu Gly Val Glu Asn Ser Asn Leu Val Gln Phe Glu
   465                 470                 475                 480
   tcg aaa cct gca act tct tca cgt atc gtt tcg atc aaa ccg tgc tcg    1488
   Ser Lys Pro Ala Thr Ser Ser Arg Ile Val Ser Ile Lys Pro Cys Ser
                   485                 490                 495
   att agt gac ctt                                                      1500
   Ile Ser Asp Leu
               500
<210> 66
   <211> 500
   <212> PRT
   <213> Thraustochytrium sp.
<400> 66
   Pro Thr Pro Phe Phe Ser Pro Ser Ser Tyr Pro Pro Arg Ala Ile Cys
   1               5                   10                  15
   Phe Ile Pro Phe Pro Gly Asn Pro Leu Asp Asn Asn Cys Lys Ala Gly
               20                  25                  30
   Glu Met Pro Leu Asn Trp Tyr Asn Met Ser Glu Phe Met Cys Gly Lys
           35                  40                  45
   Val Ser Asn Cys Leu Gly Pro Glu Phe Ala Arg Phe Asp Lys Ser Asn
       50                  55                  60
   Thr Ser Arg Ser Pro Ala Phe Asp Leu Ala Leu Val Thr Arg Val Val
   65                  70                  75                  80
   Glu Val Thr Asn Met Glu His Gly Lys Phe Leu Asn Val Asp Cys Asn
                   85                  90                  95
   Pro Ser Lys Gly Thr Met Val Gly Glu Phe Asp Cys Pro Gln Asp Ala
               100                 105                 110
   Trp Phe Phe Asp Gly Ser Cys Asn Asp Gly His Met Pro Tyr Ser Ile
           115                 120                 125
   Ile Met Glu Ile Gly Leu Gln Thr Ser Gly Val Leu Thr Ser Val Leu
       130                 135                 140
   Lys Ala Pro Leu Thr Met Asp Lys Asp Asp Ile Leu Phe Arg Asn Leu
   145                 150                 155                 160
   Asp Ala Ser Ala Glu Met Val Arg Pro Asp Val Asp Val Arg Gly Lys
                   165                 170                 175
   Thr Ile Arg Asn Val Thr Lys Cys Thr Gly Tyr Ala Met Leu Gly Lys
               180                 185                 190
   Met Gly Ile His Arg Phe Thr Phe Glu Leu Ser Val Asp Gly Val Val
           195                 200                 205
   Phe Tyr Lys Gly Ser Thr Ser Phe Gly Trp Phe Thr Pro Glu Val Phe
       210                 215                 220
   Ala Gln Gln Ala Gly Leu Asp Asn Gly Lys Lys Thr Glu Pro Trp Cys
   225                 230                 235                 240
   Lys Thr Asn Asn Thr Ser Val Arg Arg Val Glu Ile Ala Ser Ala Lys
                   245                 250                 255
   Gly Lys Glu Gln Leu Thr Glu Lys Leu Pro Asp Ala Thr Asn Ala Gln
               260                 265                 270
   Val Leu Arg Arg Ser Glu Gln Cys Glu Tyr Leu Asp Tyr Leu Asn Ile
           275                 280                 285
   Ala Pro Asp Ser Gly Leu His Gly Lys Gly Tyr Ala His Gly His Lys
       290                 295                 300
   Asp Val Asn Pro Gln Asp Trp Phe Phe Ser Cys His Phe Trp Phe Asp
   305                 310                 315                 320
   Pro Val Met Pro Gly Ser Leu Gly Ile Glu Ser Met Phe Gln Leu Ile
                   325                 330                 335
   Glu Ala Phe Ala Val Asp Gln Asn Ile Pro Gly Glu Tyr Asn Val Ser
               340                 345                 350
   Asn Pro Thr Phe Ala His Ala Pro Gly Lys Thr Ala Trp Lys Tyr Arg
           355                 360                 365
   Gly Gln Leu Thr Pro Lys Asn Arg Ala Met Asp Cys Glu Val His Ile
       370                 375                 380
   Val Ser Ile Thr Ala Ser Pro Glu Asn Gly Gly Tyr Val Asp Ile Val
   385                 390                 395                 400
   Ala Asp Gly Ala Leu Trp Val Asp Gly Leu Arg Val Tyr Glu Ala Lys
                   405                 410                 415
   Glu Leu Arg Val Arg Val Val Ser Ala Lys Pro Gln Ala Ile Pro Asp
               420                 425                 430
   Val Gln Gln Gln Pro Pro Ser Ala Lys Ala Asp Pro Gly Lys Thr Gly
           435                 440                 445
   Val Ala Leu Ser Pro Thr Gln Leu Arg Asp Val Leu Leu Glu Val Asp
       450                 455                 460
   Asn Pro Leu Tyr Leu Gly Val Glu Asn Ser Asn Leu Val Gln Phe Glu
   465                 470                 475                 480
   Ser Lys Pro Ala Thr Ser Ser Arg Ile Val Ser Ile Lys Pro Cys Ser
                   485                 490                 495
   Ile Ser Asp Leu
               500
<210> 67
   <211> 1410
   <212> DNA
   <213> Thraustochytrium sp.
<220>
   <221> CDS
   <222> (1)..(1410)
<400> 67
   ggc gat aag tct ttt atg gaa acg tac aac gtg tca gca cct ctg tat      48
   Gly Asp Lys Ser Phe Met Glu Thr Tyr Asn Val Ser Ala Pro Leu Tyr
   1               5                   10                  15
   act gga gca atg gcc aag ggc att gca tcc gcc gac ttg gtc att gct      96
   Thr Gly Ala Met Ala Lys Gly Ile Ala Ser Ala Asp Leu Val Ile Ala
               20                  25                  30
   gct ggg aaa cgc aag ata ctt gga tcg ttt ggt gcg gga ggg ctg cct     144
   Ala Gly Lys Arg Lys Ile Leu Gly Ser Phe Gly Ala Gly Gly Leu Pro
           35                  40                  45
   att tcc ata gtc cgt gaa gca ctg gag aaa att caa caa cac ctg ccc     192
   Ile Ser Ile Val Arg Glu Ala Leu Glu Lys Ile Gln Gln His Leu Pro
       50                  55                  60
   cac ggc ccc tac gct gtt aac ctc att cac tcg cct ttc gac agc aac     240
   His Gly Pro Tyr Ala Val Asn Leu Ile His Ser Pro Phe Asp Ser Asn
   65                  70                  75                  80
   ttg gaa aag ggc aac gtt gac ctc ttt ctc gag atg ggc gtg aca gtg     288
   Leu Glu Lys Gly Asn Val Asp Leu Phe Leu Glu Met Gly Val Thr Val
                   85                  90                  95
   gta gaa tgc agc gcg ttc atg gaa ctc acg gcc cag gtt gtc cgg tac     336
   Val Glu Cys Ser Ala Phe Met Glu Leu Thr Ala Gln Val Val Arg Tyr
               100                 105                 110
   cgc gcg tct ggt cta agc aaa agt gcg gac ggt tcg att cgc att gct     384
   Arg Ala Ser Gly Leu Ser Lys Ser Ala Asp Gly Ser Ile Arg Ile Ala
           115                 120                 125
   cac cgt att att ggc aag gtt tcc aga acc gag ctg gca gaa atg ttt     432
   His Arg Ile Ile Gly Lys Val Ser Arg Thr Glu Leu Ala Glu Met Phe
       130                 135                 140
   att cgt cca gca cca cag cac ctc ctc caa aaa ctc gta gcc tcc ggc     480
   Ile Arg Pro Ala Pro Gln His Leu Leu Gln Lys Leu Val Ala Ser Gly
   145                 150                 155                 160
   gag ctg aca gct gag caa gcc gag ctt gca aca cag gtt ccg gtg gcg     528
   Glu Leu Thr Ala Glu Gln Ala Glu Leu Ala Thr Gln Val Pro Val Ala
                   165                 170                 175
   gat gac att gcg gtc gaa gcc gac tcg ggg ggg cat acc gac aac agg     576
   Asp Asp Ile Ala Val Glu Ala Asp Ser Gly Gly His Thr Asp Asn Arg
               180                 185                 190
   cct att cac gtc att ctt cct cta atc atc aac cta cgc aac cgt ttg     624
   Pro Ile His Val Ile Leu Pro Leu Ile Ile Asn Leu Arg Asn Arg Leu
           195                 200                 205
   cat aaa gag ctt gac tac cct tcg cat ctc cgg gta cgt gtg ggt gct     672
   His Lys Glu Leu Asp Tyr Pro Ser His Leu Arg Val Arg Val Gly Ala
       210                 215                 220
   ggt ggt ggt att gga tgt cct caa gcc gct ctt gca gca ttt caa atg     720
   Gly Gly Gly Ile Gly Cys Pro Gln Ala Ala Leu Ala Ala Phe Gln Met
   225                 230                 235                 240
   ggg gca gcg ttt tta atc act gga acg gtg aac cag ctt gct cgt gaa     768
   Gly Ala Ala Phe Leu Ile Thr Gly Thr Val Asn Gln Leu Ala Arg Glu
                   245                 250                 255
   agt ggc act tgt gac aac gtc cgg tta cag ctc tca aag gcc acg tat      816
   Ser Gly Thr Cys Asp Asn Val Arg Leu Gln Leu Ser Lys Ala Thr Tyr
               260                 265                 270
   agc gac gtg tgt atg gct cct gct gcc gat atg ttt gac caa ggc gtg      864
   Ser Asp Val Cys Met Ala Pro Ala Ala Asp Met Phe Asp Gln Gly Val
           275                 280                 285
   gag ctg caa gta ttg aag aaa ggc acg ctg ttc cca agt cgt gct aag      912
   Glu Leu Gln Val Leu Lys Lys Gly Thr Leu Phe Pro Ser Arg Ala Lys
       290                 295                 300
   aag ctg tac gag ctg ttc tgc aag tat gac tcg ttt gag gca atg ccg      960
   Lys Leu Tyr Glu Leu Phe Cys Lys Tyr Asp Ser Phe Glu Ala Met Pro
   305                 310                 315                 320
   gct gaa gaa ttg caa cgg gtt gaa aag cgg att ttt caa aag tcg ctt     1008
   Ala Glu Glu Leu Gln Arg Val Glu Lys Arg Ile Phe Gln Lys Ser Leu
                   325                 330                 335
   gct gaa gtt tgg cag gag acc agt gac ttt tac att cat cgt atc aag     1056
   Ala Glu Val Trp Gln Glu Thr Ser Asp Phe Tyr Ile His Arg Ile Lys
               340                 345                 350
   aac cct gag aaa atc aat cgt gct gca agc gat ggc aaa ctg aaa atg     1104
   Asn Pro Glu Lys Ile Asn Arg Ala Ala Ser Asp Gly Lys Leu Lys Met
           355                 360                 365
   tcg ctt tgc ttt cgc tgg tac ctt ggg ctt tcc tca ttt tgg gcc aac     1152
   Ser Leu Cys Phe Arg Trp Tyr Leu Gly Leu Ser Ser Phe Trp Ala Asn
       370                 375                 380
   tct ggg gca caa gat cgc gtc atg gac tat caa att tgg tgt ggc cct     1200
   Ser Gly Ala Gln Asp Arg Val Met Asp Tyr Gln Ile Trp Cys Gly Pro
   385                 390                 395                 400
   gct att ggc gct ttc aat gat ttt acc aag ggc acg tac ctt gac gtg     1248
   Ala Ile Gly Ala Phe Asn Asp Phe Thr Lys Gly Thr Tyr Leu Asp Val
                   405                 410                 415
   act gtt gca aag agt tac cct tgt gtg gca cag atc aat ttg caa att     1296
   Thr Val Ala Lys Ser Tyr Pro Cys Val Ala Gln Ile Asn Leu Gln Ile
               420                 425                 430
   ttg caa gga gct gcg tat ctg aaa cgc ctt ggt gtc att cgt ttt gac     1344
   Leu Gln Gly Ala Ala Tyr Leu Lys Arg Leu Gly Val Ile Arg Phe Asp
           435                 440                 445
   cgc atg ctg ctg cag gcc gtc gat atc gac gat cct gta ttt act tac     1392
   Arg Met Leu Leu Gln Ala Val Asp Ile Asp Asp Pro Val Phe Thr Tyr
       450                 455                 460
   gtg ccg acc cag cca ctt                                             1410
   Val Pro Thr Gln Pro Leu
   465                 470
<210> 68
   <211> 470
   <212> PRT
   <213> Thraustochytrium sp.
<400> 68
   Gly Asp Lys Ser Phe Met Glu Thr Tyr Asn Val Ser Ala Pro Leu Tyr
   1               5                   10                  15
   Thr Gly Ala Met Ala Lys Gly Ile Ala Ser Ala Asp Leu Val Ile Ala
               20                  25                  30
   Ala Gly Lys Arg Lys Ile Leu Gly Ser Phe Gly Ala Gly Gly Leu Pro
   35 40 45
   Ile Ser Ile Val Arg Glu Ala Leu Glu Lys Ile Gln Gln His Leu Pro
   50 55 60
   His Gly Pro Tyr Ala Val Asn Leu Ile His Ser Pro Phe Asp Ser Asn
   65 70 75 80
   Leu Glu Lys Gly Asn Val Asp Leu Phe Leu Glu Met Gly Val Thr Val
   85 90 95
   Val Glu Cys Ser Ala Phe Met Glu Leu Thr Ala Gln Val Val Arg Tyr
   100 105 110
   Arg Ala Ser Gly Leu Ser Lys Ser Ala Asp Gly Ser Ile Arg Ile Ala
   115 120 125
   His Arg Ile Ile Gly Lys Val Ser Arg Thr Glu Leu Ala Glu Met Phe
   130 135 140
   Ile Arg Pro Ala Pro Gln His Leu Leu Gln Lys Leu Val Ala Ser Gly
   145 150 155 160
   Glu Leu Thr Ala Glu Gln Ala Glu Leu Ala Thr Gln Val Pro Val Ala
   165 170 175
   Asp Asp Ile Ala Val Glu Ala Asp Ser Gly Gly His Thr Asp Asn Arg
   180 185 190
   Pro Ile His Val Ile Leu Pro Leu Ile Ile Asn Leu Arg Asn Arg Leu
   195 200 205
   His Lys Glu Leu Asp Tyr Pro Ser His Leu Arg Val Arg Val Gly Ala
   210 215 220
   Gly Gly Gly Ile Gly Cys Pro Gln Ala Ala Leu Ala Ala Phe Gln Met
   225 230 235 240
   Gly Ala Ala Phe Leu Ile Thr Gly Thr Val Asn Gln Leu Ala Arg Glu
   245 250 255
   Ser Gly Thr Cys Asp Asn Val Arg Leu Gln Leu Ser Lys Ala Thr Tyr
   260 265 270
   Ser Asp Val Cys Met Ala Pro Ala Ala Asp Met Phe Asp Gln Gly Val
   275 280 285
   Glu Leu Gln Val Leu Lys Lys Gly Thr Leu Phe Pro Ser Arg Ala Lys
   290 295 300
   Lys Leu Tyr Glu Leu Phe Cys Lys Tyr Asp Ser Phe Glu Ala Met Pro
   305 310 315 320
   Ala Glu Glu Leu Gln Arg Val Glu Lys Arg Ile Phe Gln Lys Ser Leu
   325 330 335
   Ala Glu Val Trp Gln Glu Thr Ser Asp Phe Tyr Ile His Arg Ile Lys
   340 345 350
   Asn Pro Glu Lys Ile Asn Arg Ala Ala Ser Asp Gly Lys Leu Lys Met
   355 360 365
   Ser Leu Cys Phe Arg Trp Tyr Leu Gly Leu Ser Ser Phe Trp Ala Asn
   370 375 380
   Ser Gly Ala Gln Asp Arg Val Met Asp Tyr Gln Ile Trp Cys Gly Pro
   385 390 395 400
   Ala Ile Gly Ala Phe Asn Asp Phe Thr Lys Gly Thr Tyr Leu Asp Val
   405 410 415
   Thr Val Ala Lys Ser Tyr Pro Cys Val Ala Gln Ile Asn Leu Gln Ile
   420 425 430
   Leu Gln Gly Ala Ala Tyr Leu Lys Arg Leu Gly Val Ile Arg Phe Asp
   435 440 445
   Arg Met Leu Leu Gln Ala Val Asp Ile Asp Asp Pro Val Phe Thr Tyr
   450 455 460
   Val Pro Thr Gln Pro Leu
   465 470
<210> 69
   <211> 20
   <212> DNA
   <213> Artificial sequence
<220>
   <223> primer
<400> 69
   ggyatgmtgr ttggtgaagg 20
<210> 70
   <211> 22
   <212> DNA
   <213> Artificial sequence
<220>
   <223> primer
<400> 70
   trttsasrta ytgygaacct tg 22
<210> 71
   <211> 20
   <212> DNA
   <213> Artificial sequence
<220>
   <223> primer
<220>
   <221> misc feature
   <222> (1)..(20)
   <223> n = a c g or t
<400> 71
   atgkcngaag gttgtggcca 20
<210> 72
   <211> 23
   <212> DNA
   <213> Artificial sequence
<220>
   <223> primer
<400> 72
   ccwgaratra agccrttdgg ttg 23
<210> 73
   <211> 30
   <212> DNA
   <213> Artificial Sequence
<220>
   <223> primer
<400> 73
   cggggtaccc gggagccgcc ttggctttgt 30
<210> 74
   <211> 35
   <212> DNA
   <213> Artificial Sequence
<220>
   <223> primer
<400> 74
   aaactgcagc ccgggtccag ctggcaggca ccctg 35
<210> 75
   <211> 798
   <212> DNA
   <213> Shewanella olleyana
<400> 75
   atggcagaag gttgtggcca attattgcag ttcttcatgc tgcacattgg tatgcacacc   60
   ttagttgaaa acggacgttt ccagccttta gaaaatgctt cacaaaaagt acgttgtcgt  120
   ggccaagtac tgccacaaca tggtgaactg acgtaccgca tggaagtcac agaaattggt  180
   actcaccctc gcccatacgc caaagccaat attgaaatat tgctcaatgg taaagcggtc  240
   gtggacttcc aaaatcttgg ggtgatgatt aaagaagaag gtgaatgtac tcgttacact  300
   gccgactcta ctgaaacaca tacaacctca ggcacagtcc aaaaaaacaa cagccacaac  360
   acaccagcat cattaaatgc accgttaatg gcacaagtgc cagacttaag tgaaccagcc  420
   aataaaggcg ttatcccgct gcaacatgtt gaagcgccta tgctgccaga ctacccaaat  480
   cgaacccctg atacgctgcc gttcaccgcg taccatatgt ttgagtttgc aacaggtgac  540
   atcgaaaact gttttggacc tgactttagt atttaccggg gctttattcc gccgcgcacg  600
   ccatgtggtg acttacagct aacaacccgt gttgttgata ttcaaggtaa acgtggcgag  660
   cttaaaaaac cgtcatcgtg tatcgctgaa tatgaagtgc caaccgatgc gtggtatttt  720
   gctaaaaaca gtcacgcttc agtgatgcct tactcggtat taatggaaat atcactgcaa  780
   cccaacggct tcatctca                                                 798
<210> 76
   <211> 1042
   <212> DNA
   <213> Shewanella olleyana
<400> 76
   ggcatgatgg ttggtgaagg tatcggcatg gtagcactta agcgcctaga agatgctgag      60
   cgtgatggcg accgtattta ttctgtgatt aaaggtgtcg gcgcttcatc agacggtaaa     120
   tttaagagta tttatgcacc gcgccctgaa ggccaagcaa aagcattaaa acgagcttat     180
   gatgacgctg gttttgcccc tgaaacagtt ggcttaatcg aagctcacgg tacgggtact     240
   gctgcaggtg atgtagccga atttaacggc cttaaatctg tatttggtga aaacgatcca     300
   actaagcaac acatcgcttt aggttcagtg aaatcacaag tgggtcacac gaaatcaacc     360
   gctggtactg ctggcgtgat taaagctgcc cttgccctgc accataaagt attgccaccg     420
   accattaacg tctctaagcc aaaccctaag cttaatgttg aggattcacc gtttttcgtt     480
   aataccgaaa cacgcccatg gatgcctcgc cctgacggca ctcctcgccg tgctggtatt     540
   agctcgttcg gttttggtgg aactaacttc cacttagtat tagaagaata cacccctgag     600
   cacagccatg atgagaaata ccgtcaacgc caagtggctc aaagcttatt aatgagtgct     660
   gataataaag cagccttgat tgcagaagtg aataagctaa ctgcagacat cagcgcgctt     720
   aaaggcacag ataacagcag cattgaacaa gctgaacttg ctcgcattgc taaactatat     780
   gctgttcgca ccatagatac ttcagcagcc cgtttaggtc ttgtggtatc aagccttaat     840
   gaattaacca ctcagcttgg tttagcgtta aagcagctta ataatgatgt tgatgcatgg     900
   caactgccat cagggactag ctaccgctct tcagcactca tcacgattaa tgcaaaccaa     960
   aaggcgacta aaggtaaaaa agcgactaac gcaccgaaag ttgcagcatt gtttgcaggt    1020
   caaggttcac aatacctcaa ca                                             1042
<210> 77
   <211> 806
   <212> DNA
   <213> Shewanella japonica
<400> 77
   atggcggaag gttgtggcca attactgcaa ttctttatgc tgcacattgg tatgcacacg      60
   ctcgttgaaa atggccgttt ccaaccactt gaaaatgctt cacaaaaagt gcgttgtcgt     120
   ggtcaagttc tgccgcagca cggtgaactg acttaccgga tggaaatcac tgaaattggc     180
   attcaccctc gcccatatgc caaagcgaat attgatattt tgcttaacgg taaagcggtt     240
   gtcgacttcc aaaacttagg tgtcatgatc aaagaagaaa gcgaatgtac gcgctacctt     300
   aatgatacgc ccgctgtcga tgcctcagct gatcgaatta attcagcaac caataatatt     360
   ctatacccag cggcttcaac caatgcgcca ctcatggctc aactgcctga tttgaatgcc     420
   ccaacgaata aaggcgttat cccactgcaa catgttgaag cgccgataat tccagattat 480
   ccaaatcgta ctcctgatac cctgccattc acggcgtatc acatgttcga atttgccact 540
   ggcaatattg aaaactgctt tggaccggac tttagtattt accgtggttt cattccaccg 600
   cgcacaccat gtggcgactt acagctaacg actcgtattg ttgatattca aggtaaacgt 660
   ggcgaattga aaaagccatc atcgtgtatc gcagaatatg aagtgccaac tgatgcatgg 720
   tatttcgcta aaaacagcca cgcctcggtc ataccttatt cagtgttgat ggaaatttca 780
   ctgcaaccca atggcttcat ctcagg 806
<210> 78
   <211> 1042
   <212> DNA
   <213> Shewanella japonica
<400> 78
   ggtatgctgg ttggtgaagg cattggcatg gtggcattaa aacgtcttga agatgctgag 60
   cgtgacggtg accgtattta ctcagtcatt aaaggggtcg gcgcttcatc tgatggtaag 120
   ttcaaatcaa tttatgcacc tcgacctgaa ggccaagcta aagcgctgaa gcgtgcttat 180
   gatgacgccg gctttgcacc tgaaaccgtt ggcttaattg aagctcacgg aacaggcact 240
   gcagcgggtg atgtggcaga atttaatggt cttaaatctg tatttggtga gaatgactca 300
   acaaagcaac acattgcttt aggttcagtt aagtcacaag tgggccatac taaatcaact 360
   gcgggaaccg cgggtgtgat taaagcggcg ttagcactgc atcataaagt gctgccgcca 420
   accatcaacg tctctaagcc taaccctaag cttaatgttg aggattcacc gtttttcatt 480
   aacactgaaa ctcgcccttg gatgcctcgc cctgatggca caccacgccg agctggtata 540
   agttcgttcg gttttggtgg cacaaacttc cacttagtac tagaagaata cagcccagag 600
   cacagccgtg atgagaaata tcgtcagcgc caagtagcac aaagcttatt gattagcgct 660
   gacaataaag ctgagctcat tgcagaaatc aacaagctta acgctgacat cagcgcgctt 720
   aaaggcacag ataacagcag catcgaacaa gctgaacttg cccgcattgc taaactatat 780
   gctgttcgca ctttagatac ttcagcagtc cgtttgggtc ttgtggtctc aagccttaat 840
   gaattaacca ctcaacttgg tttagcgtta aagcagctaa gtaacgacgc tgaagcatgg 900
   caattaccat caggtacgag ctatcgctca tctgcgctca tcacgattaa tgccaaccaa 960
   aagacgacta aaggtaaaaa agcagctaac acaccgaaag tagcagcatt atttgcaggt 1020
   caaggttcgc aatatctcaa ca 1042
<210> 79
   <211> 6
   <212> DNA
   <213> Artificial Sequence
<220>
   <223> restriction enzyme site
<400> 79
   catatg 6
<210> 80
   <211> 192
   <212> DNA
   <213> Schizochytrium sp.
<220>
   <221> misc_feature
   <222> (1)..(7)
   <223> BspHI restriction site
<220>
   <221> misc_feature
   <222> (188)..(192)
   <223> SacII restriction site
<400> 80
   tcatgaagcc ggttgctccg aagttctacg cgcgtctcaa cattgacgag caggacgaga     60
   cccgtgatcc gatcctcaac aaggacaacg cgccgtcttc cagctctagc tcctcttcca    120
   gctcttccag ctcttccagc ccgtcgccag ctccgtccgc cccagtgcaa aagaaggctg    180
   ctccggccgc gg                                                        192
<210> 81
   <211> 717
   <212> DNA
   <213> Nostoc sp.
<220>
   <221> CDS
   <222> (4)..(714)
<400> 81
   taa ttg ttg cag cat act tgg cta cca aaa ccc cca aat tta acc tta      48
       Leu Leu Gln His Thr Trp Leu Pro Lys Pro Pro Asn Leu Thr Leu
       1               5                   10                  15
   ttg tca gat gaa gtt cat ctc tgg cgc att ccc ctt gac caa cca gaa      96
   Leu Ser Asp Glu Val His Leu Trp Arg Ile Pro Leu Asp Gln Pro Glu
                   20                  25                  30
   tca cag cta cag gat tta gcc gct acc tta tct agt gac gaa tta gcc     144
   Ser Gln Leu Gln Asp Leu Ala Ala Thr Leu Ser Ser Asp Glu Leu Ala
               35                  40                  45
   cgt gca aac aga ttt tat ttt ccc gaa cat cgc cgg cgt ttt act gct     192
   Arg Ala Asn Arg Phe Tyr Phe Pro Glu His Arg Arg Arg Phe Thr Ala
           50                  55                  60
   ggt cgt ggt att ctc cgc agt atc ttg ggg ggc tat ttg ggt gtg gaa     240
   Gly Arg Gly Ile Leu Arg Ser Ile Leu Gly Gly Tyr Leu Gly Val Glu
       65                  70                  75
   cca ggg caa gtt aaa ttt gat tat gaa tcc cgt ggt aaa cca ata tta     288
   Pro Gly Gln Val Lys Phe Asp Tyr Glu Ser Arg Gly Lys Pro Ile Leu
   80                  85                  90                  95
   ggc gat cgc ttt gcc gag agt ggt tta tta ttt aac ttg tca cac tcc     336
   Gly Asp Arg Phe Ala Glu Ser Gly Leu Leu Phe Asn Leu Ser His Ser
                   100                 105                 110
   cag aac ttg gcc ttg tgt gca gtc aat tac acg cgc caa atc ggc atc     384
   Gln Asn Leu Ala Leu Cys Ala Val Asn Tyr Thr Arg Gln Ile Gly Ile
               115                 120                 125
   gat tta gaa tat ctc cgc ccc aca tct gat tta gaa tcc ctt gcc aaa     432
   Asp Leu Glu Tyr Leu Arg Pro Thr Ser Asp Leu Glu Ser Leu Ala Lys
           130                 135                 140
   agg ttc ttt tta ccg cga gaa tat gaa tta ttg cga tcg cta ccc gat     480
   Arg Phe Phe Leu Pro Arg Glu Tyr Glu Leu Leu Arg Ser Leu Pro Asp
       145                 150                 155
   gag caa aaa caa aaa att ttc ttt cgt tac tgg act tgt aaa gag gct     528
   Glu Gln Lys Gln Lys Ile Phe Phe Arg Tyr Trp Thr Cys Lys Glu Ala
   160                 165                 170                 175
   tat ctt aaa gca acg ggt gac ggc atc gct aaa tta gag gaa att gaa     576
   Tyr Leu Lys Ala Thr Gly Asp Gly Ile Ala Lys Leu Glu Glu Ile Glu
                   180                 185                 190
   ata gca cta act ccc aca gaa cca gct aag tta cag aca gct cca gcg     624
   Ile Ala Leu Thr Pro Thr Glu Pro Ala Lys Leu Gln Thr Ala Pro Ala
               195                 200                 205
   tgg agt ctc cta gag cta gtg cca gat gat aat tgt gtt gct gct gtt     672
   Trp Ser Leu Leu Glu Leu Val Pro Asp Asp Asn Cys Val Ala Ala Val
           210                 215                 220
   gcc gtg gcg ggt ttt ggc tgg cag cca aaa ttc tgg cat tat tga         717
   Ala Val Ala Gly Phe Gly Trp Gln Pro Lys Phe Trp His Tyr
       225                 230                 235
<210> 82
   <211> 237
   <212> PRT
   <213> Nostoc sp.
<400> 82
   Leu Leu Gln His Thr Trp Leu Pro Lys Pro Pro Asn Leu Thr Leu Leu
   1 5 10 15
   Ser Asp Glu Val His Leu Trp Arg Ile Pro Leu Asp Gln Pro Glu Ser
   20 25 30
   Gln Leu Gln Asp Leu Ala Ala Thr Leu Ser Ser Asp Glu Leu Ala Arg
   35 40 45
   Ala Asn Arg Phe Tyr Phe Pro Glu His Arg Arg Arg Phe Thr Ala Gly
   50 55 60
   Arg Gly Ile Leu Arg Ser Ile Leu Gly Gly Tyr Leu Gly Val Glu Pro
   65 70 75 80
   Gly Gln Val Lys Phe Asp Tyr Glu Ser Arg Gly Lys Pro Ile Leu Gly
   85 90 95
   Asp Arg Phe Ala Glu Ser Gly Leu Leu Phe Asn Leu Ser His Ser Gln
   100 105 110
   Asn Leu Ala Leu Cys Ala Val Asn Tyr Thr Arg Gln Ile Gly Ile Asp
   115 120 125
   Leu Glu Tyr Leu Arg Pro Thr Ser Asp Leu Glu Ser Leu Ala Lys Arg
   130 135 140
   Phe Phe Leu Pro Arg Glu Tyr Glu Leu Leu Arg Ser Leu Pro Asp Glu
   145 150 155 160
   Gln Lys Gln Lys Ile Phe Phe Arg Tyr Trp Thr Cys Lys Glu Ala Tyr
   165 170 175
   Leu Lys Ala Thr Gly Asp Gly Ile Ala Lys Leu Glu Glu Ile Glu Ile
   180 185 190
   Ala Leu Thr Pro Thr Glu Pro Ala Lys Leu Gln Thr Ala Pro Ala Trp
   195 200 205
   Ser Leu Leu Glu Leu Val Pro Asp Asp Asn Cys Val Ala Ala Val Ala
   210 215 220
   Val Ala Gly Phe Gly Trp Gln Pro Lys Phe Trp His Tyr
   225 230 235